(19) Europäisches Patentamt
European Patent Office
Office européen des brevets

(11) EP 2 419 540 B1

(12) **EUROPEAN PATENT SPECIFICATION**

(45) Date of publication and mention of the grant of the patent:
**17.05.2017 Bulletin 2017/20**

(21) Application number: **10765254.7**

(22) Date of filing: **16.04.2010**

(51) Int Cl.:
***C12Q 1/68*** *(2006.01)*

(86) International application number:
**PCT/US2010/031384**

(87) International publication number:
**WO 2010/121123 (21.10.2010 Gazette 2010/42)**

(54) **METHODS AND GENE EXPRESSION SIGNATURE FOR ASSESSING RAS PATHWAY ACTIVITY**

VERFAHREN UND GENEXPRESSIONSSIGNATUREN ZUR BEURTEILUNG DER AKTIVITÄT DES RAS-SIGNALWEGES

PROCÉDÉS ET SIGNATURE D'EXPRESSION GÉNÉTIQUE POUR ÉVALUER L'ACTIVITÉ DE LA VOIE RAS

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **18.04.2009 US 212987 P**

(43) Date of publication of application:
**22.02.2012 Bulletin 2012/08**

(73) Proprietor: **Merck Sharp & Dohme Corp.**
**Rahway, NJ 07065-0907 (US)**

(72) Inventors:
- **LOBODA, Andrey**
  **Rahway**
  **New Jersey 07065 (US)**
- **NEBOZHYN, Michael**
  **Rahway**
  **New Jersey 07065-0907 (US)**
- **ZHANG, Theresa**
  **Rahway**
  **New Jersey 07065-0907 (US)**
- **WATTERS, James, W.**
  **Rahway**
  **New Jersey 07065-0907 (US)**
- **HUANG, Pearl, S.**
  **Rahway**
  **New Jersey 07065-0907 (US)**
- **CHASTAIN, Michael**
  **Rahway**
  **New Jersey 07065-0907 (US)**
- **KLINGHOFFER, Richard, A.**
  **Rahway**
  **New Jersey 07065-0907 (US)**

(74) Representative: **Hussain, Deeba et al**
**Merck & Co., Inc.**
**Patent Department**
**Hertford Road**
**Hoddesdon**
**Hertfordshire EN11 9BU (GB)**

(56) References cited:
**WO-A1-2006/124836**
**US-A1- 2003 229 455**
**US-A1- 2006 160 109**
**US-A1- 2007 009 530**
**US-A1- 2007 099 209**
**US-A1- 2008 064 055**
**US-A1- 2008 292 546**

- **ROMAN ROUZIER ET AL: "microtubule associated protein tau: A marker of paclitaxel sensitivity in breast cancer", PNAS, vol. 102, no. 23, 7 June 2005 (2005-06-07) , pages 8315-8320, XP55044887,**
- **BILD ANDREA H ET AL: "Oncogenic pathway signatures in human cancers as a guide to targeted therapies", NATURE: INTERNATIONAL WEEKLY JOURNAL OF SCIENCE, NATURE PUBLISHING GROUP, UNITED KINGDOM, vol. 439, no. 7074, 19 January 2006 (2006-01-19), pages 353-357, XP002460134, ISSN: 0028-0836, DOI: 10.1038/NATURE04296**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 419 540 B1



(Cont. next page)

- DOWNWARD JULIAN: "Targeting RAS signalling pathways in cancer therapy", NATURE REVIEWS. CANCER, NATUR PUBLISHING GROUP, LONDON, GB, vol. 3, no. 1, 1 January 2003 (2003-01-01) , pages 11-22, XP009146312, ISSN: 1474-175X, DOI: 10.1038/NRC969
- GOLUB T R ET AL: "Molecular classification of cancer: Class discovery and class prediction by gene expression monitoring", SCIENCE, AMERICAN ASSOCIATION FOR THE ADVANCEMENT OF SCIENCE, WASHINGTON, DC; US, vol. 286, no. 5439, 15 October 1999 (1999-10-15), pages 531-537, XP002207658, ISSN: 0036-8075, DOI: 10.1126/SCIENCE.286.5439.531

SEQUENCE LISTING

<110> Merck Sharp & Dohme Corp.
Loboda, Andrey
Nebozhyn, Michael
Zhang, Theresa
Watters, James W.
Huang, Pearl S.
Chastain, Michael
Klinghoffer, Richard A.

<120> Methods and Gene Expression Signature
for Assessing Ras Pathway Activity

<130> ROS-ONC-00003

<150> 61/212,987
<151> 2009-04-18

<160> 294

<170> FastSEQ for Windows Version 4.0

<210> 1
<211> 3280
<212> DNA
<213> Homo sapiens

<400> 1

agaccgtgtc ccgcggggct gtcacctccg cctctgctcc ccgacccggc catgcgcggc 60
ctcgggctct ggctgctggg cgcgatgatg ctgcctgcga ttgcccccag ccggccctgg 120
gccctcatgg agcagtatga ggtcgtgttg ccgtggcgtc tgccaggccc ccgagtccgc 180
cgagctctgc cctcccactt gggcctgcac ccagagaggg tgagctacgt ccttggggcc 240
acagggcaca acttcaccct ccacctgcgg aagaacaggg acctgctggg ctccggctac 300
acagagacct atacggctgc caatggctcc gaggtgacgg agcagcctcg cgggcaggac 360
cactgcttct accagggcca cgtagagggg tacccggact cagccgccag cctcagcacc 420
tgtgccggcc tcaggggttt cttccaggtg gggtcagacc tgcacctgat cgagcccctg 480
gatgaaggtg gcgagggcgg acggcacgcc gtgtaccagg ctgagcacct gctgcagacg 540
gccgggacct gcggggtcag cgacgacagc ctgggcagcc tcctgggacc ccggacggca 600
gccgtcttca ggcctcggcc cggggactct ctgccatccc gagagacccg ctacgtggag 660
ctgtatgtgg tcgtggacaa tgcagagttc cagatgctgg ggagcgaagc agccgtgcgt 720
catcgggtgc tggaggtggt gaatcacgtg gacaagctat atcagaaact caacttccgt 780
gtggtcctgg tgggcctgga gatttggaat agtcaggaca ggttccacgt cagcccccgac 840
cccagtgtca cactggagaa cctcctgacc tggcaggcac ggcaacggac acggcggcac 900
ctgcatgaca acgtacagct catcacgggt gtcgacttca ccgggactac cgtggggttt 960
gccagggtgt ccgccatgtg ctcccacagc tcaggggctg tgaaccagga ccacagcaag 1020
aaccccgtgg gcgtggcctg taccatggcc catgagatgg gccacaacct gggcatggac 1080
catgatgaga acgtccaggg ctgccgctgc caggaacgct tcgaggccgg ccgctgcatc 1140
atggcgggca gcattggctc cagtttcccc aggatgttca gtgactgcag ccaggcctac 1200
ctggagagct tttggagcg gccgcagtcg gtgtgcctcg ccaacgcccc tgacctcagc 1260
cacctggtgg gcggccccgt gtgtgggaac ctgtttgtgg agcgtgggga gcagtgcgac 1320
tgcggccccc ccgaggactg ccggaaccgc tgctgcaact ctaccacctg ccagctggct 1380
gaggggggccc agtgtgcgca cggtacctgc tgccaggagt gcaaggtgaa gccggctggt 1440
gagctgtgcc gtcccaagaa ggacatgtgt gacctcgagg agttctgtga cggccggcac 1500
cctgagtgcc cggaagacgc cttccaggag aacggcacgc cctgctccgg gggctactgc 1560
tacaacgggg cctgtcccac actggcccag cagtgccagg ccttctgggg gccaggtggg 1620
caggctgccg aggagtcctg cttctcctat gacatcctac caggctgcaa ggccagccgg 1680
tacagggctg acatgtgtgg cgttctgcag tgcaagggtg ggcagcagcc cctggggcgt 1740
gccatctgca tcgtggatgt gtgccacgcg ctcaccacag aggatggcac tgcgtatgaa 1800
ccagtgcccg agggcacccg gtgtggacca gagaaggttt gctggaaagg acgttgccag 1860
gacttacacg tttacagatc cagcaactgc tctgcccagt gccacaacca tggggtgtgc 1920
aaccacaagc aggagtgcca ctgccacgcg ggctgggccc cgccccactg cgcgaagctg 1980
ctgactgagg tgcacgcagc gtccgggagc ctccccgtct tcgtggtggt ggttctggtg 2040

ctcctggcag ttgtgctggt caccctggca ggcatcatcg tctaccgcaa agcccggagc 2100
cgcatcctga gcaggaacgt ggctcccaag accacaatgg ggcgctccaa ccccctgttc 2160
caccaggctg ccagccgcgt gccggccaag ggcggggctc cagccccatc caggggcccc 2220
caagagctgg tccccaccac ccacccgggc cagcccgccc gacacccggc ctcctcggtg 2280
gctctgaaga ggccgccccc tgctcctccg gtcactgtgt ccagcccacc cttcccagtt 2340
cctgtctaca cccggcaggc accaaagcag gtcatcaagc caacgttcgc acccccagtg 2400
cccccagtca aacccggggc tggtgcggcc aaccctggtc cagctgaggg tgctgttggc 2460
ccaaaggttg ccctgaagcc ccccatccag aggaagcaag gagccgagct cccacagcac 2520
cctagggggg cacctgcgcc tgtgtggaaa tttggagaag ttgcggcaga gaagccatgc 2580
gttccagcat tccacggtcc agctagtgcc gctcagccct agaccctgac tttgcaggct 2640
cagctgctgt tctaacctca ggaatgcatc tacctgagag gctcctgctg tccacgccct 2700
cagccaattc cttctccccg ccttggccac gtgtagcccc agctgtctgc aggcaccagg 2760
ctgggatgag ctgtgtgctt gcgggtgcgt gtgtgtgtac gtgtctccag gtggccgctg 2820
gtctcccgct gtgttcagga ggccacatat acagcccctc ccagccacac ctgcccctgc 2880
tctggggcct gctgagccgg ctgccctggg cacccggttc caggcagcac agacgtgggg 2940
catccccaga aagactccat cccaggacca ggttcccctg cgtgctcttc gagagggtgt 3000
cagtgagcag actgcacccc aagctcccga ctccaggtcc cctgatcttg ggcctgttt 3060
cccatgggat tcaagaggga cagccccagc tttgtgtgtg tttaagctta ggaatcgcct 3120
ttatggaaag ggctatgtgg gagagtcagc tatcttgtct ggttttcttg agacctcaga 3180
tgtgtgttca gcagggctga aagcttttat tctttaataa tgagaaatgt atatttact 3240
aataaattat tgaccgagtt ctgtaaaaaa aaaaaaaaaa 3280

<210> 2
<211> 2033
<212> DNA
<213> Homo sapiens

<400> 2

```
gcacataacg ggcagaacgc actgcgaagc ggcttcttca gagcacgggc tggaactggc 60
aggcaccgcg agcccctagc acccgacaag ctgagtgtgc aggacgagtc cccaccacac 120
ccacaccaca gccgctgaat gaggcttcca ggcgtccgct cgcggcccgc agagccccgc 180
cgtgggtccg cccgctgagg cgcccccagc cagtgcgctc acctgccaga ctgcgcgcca 240
tggggcaacc cgggaacggc agcgccttct tgctggcacc caatagaagc catgcgccgg 300
accacgacgt cacgcagcaa agggacgagg tgtgggtggt gggcatgggc atcgtcatgt 360
ctctcatcgt cctggccatc gtgtttggca atgtgctggt catcacagcc attgccaagt 420
tcgagcgtct gcagacggtc accaactact tcatcacttc actggcctgt gctgatctgg 480
tcatgggcct ggcagtggtg ccctttgggg ccgcccatat tcttatgaaa atgtggactt 540
ttggcaactt ctggtgcgag ttttggactt ccattgatgt gctgtgcgtc acggccagca 600
ttgagaccct gtgcgtgatc gcagtggatc gctactttgc cattacttca cctttcaagt 660
accagagcct gctgaccaag aataaggccc gggtgatcat tctgatggtg tggattgtgt 720
caggccttac ctccttcttg cccattcaga tgcactggta ccgggccacc caccaggaag 780
ccatcaactg ctatgccaat gagacctgct gtgacttctt cacgaaccaa gcctatgcca 840
ttgcctcttc catcgtgtcc ttctacgttc ccctggtgat catggtcttc gtctactcca 900
gggtctttca ggaggccaaa aggcagctcc agaagattga caaatctgag ggccgcttcc 960
atgtccagaa ccttagccag gtggagcagg atgggcggac ggggcatgga ctccgcagat 1020
cttccaagtt ctgcttgaag gagcacaaag ccctcaagac gttaggcatc atcatgggca 1080
ctttcaccct ctgctggctg cccttcttca tcgttaacat tgtgcatgtg atccaggata 1140
acctcatccg taaggaagtt tacatcctcc taaattggat aggctatgtc aattctggtt 1200
tcaatcccct tatctactgc cggagcccag atttcaggat tgccttccag gagcttctgt 1260
gcctgcgcag gtcttctttg aaggcctatg ggaatggcta ctccagcaac ggcaacacag 1320
gggagcagag tggatatcac gtggaacagg agaaagaaaa taaactgctg tgtgaagacc 1380
tcccaggcac ggaagacttt gtgggccatc aaggtactgt gcctagcgat aacattgatt 1440
cacaagggag gaattgtagt acaaatgact cactgctgta aagcagtttt tctactttta 1500
aagacccccc cccccaacag aacactaaac agactattta acttgagggt aataaactta 1560
gaataaaatt gtaaaattgt atagagatat gcagaaggaa gggcatcctt ctgccttttt 1620
tatttttta agctgtaaaa agagagaaaa cttatttgag tgattatttg ttatttgtac 1680
agttcagttc ctctttgcat ggaatttgta agtttatgtc taaagagctt tagtcctaga 1740
ggacctgagt ctgctatatt ttcatgactt ttccatgtat ctacctcact attcaagtat 1800
taggggtaat atattgctgc tggtaatttg tatctgaagg agattttcct tcctacaccc 1860
ttggacttga ggattttgag tatctcggac ctttcagctg tgaacatgga ctcttccccc 1920
actcctctta tttgctcaca cggggtattt taggcaggga tttgaggagc agcttcagtt 1980
gttttcccga gcaaagtcta aagtttacag taaataaatt gtttgaccat gcc        2033
```

<210> 3
<211> 1967
<212> DNA
<213> Homo sapiens

<400> 3

```
ataaaaaccg tcctcgggcg cggcggggag aagccgagct gagcggatcc tcacacgact 60
gtgatccgat tctttccagc ggcttctgca accaagcggg tcttaccccc ggtcctccgc 120
gtctccagtc ctcgcacctg gaaccccaac gtccccgaga gtccccgaat ccccgctccc 180
aggctaccta agaggatgag cggtgctccg acggccgggg cagccctgat gctctgcgcc 240
gccaccgccg tgctactgag cgctcagggc ggacccgtgc agtccaagtc gccgcgcttt 300
gcgtcctggg acgagatgaa tgtcctggcg cacggactcc tgcagctcgg ccaggggctg 360
cgcgaacacg cggagcgcac ccgcagtcag ctgagcgcgc tggagcggcg cctgagcgcg 420
tgcgggtccg cctgtcaggg aaccgagggg tccaccgacc tcccgttagc cctgagagc 480
cgggtggacc ctgaggtcct tcacagcctg cagacacaac tcaaggctca gaacagcagg 540
atccagcaac tcttccacaa ggtggcccag cagcagcggc acctggagaa gcagcacctg 600
cgaattcagc atctgcaaag ccagtttggc ctcctggacc acaagcacct agaccatgag 660
gtggccaagc ctgcccgaag aaagaggctg cccgagatgg cccagccagt tgacccggct 720
cacaatgtca gccgcctgca ccggctgccc agggattgcc aggagctgtt ccaggttggg 780
gagaggcaga gtggactatt tgaaatccag cctcaggggt ctccgccatt tttggtgaac 840
tgcaagatga cctcagatgg aggctggaca gtaattcaga ggcgccacga tggctcagtg 900
gacttcaacc ggccctggga agcctacaag gcggggtttg ggatccccca cggcgagttc 960
tggctgggtc tggagaaggt gcatagcatc acgggggacc gcaacagccg cctggccgtg 1020
cagctgcggg actgggatgg caacgccgag ttgctgcagt tctccgtgca cctgggtggc 1080
gaggacacgg cctatagcct gcagctcact gcacccgtgg ccggccagct gggcgccacc 1140
accgtcccac ccagcggcct ctccgtaccc ttctccactt gggaccagga tcacgacctc 1200
cgcagggaca agaactgcgc caagagcctc tctggaggct ggtggtttgg cacctgcagc 1260
cattccaacc tcaacggcca gtacttccgc tccatcccac agcagcggca gaagcttaag 1320
aagggaatct tctggaagac ctggcggggc cgctactacc cgctgcaggc caccaccatg 1380
ttgatccagc ccatggcagc agaggcagcc tcctagcgtc ctggctgggc ctggtcccag 1440
gcccacgaaa gacggtgact cttggctctg cccgaggatg tggccgttcc ctgcctgggc 1500
aggggctcca aggagggcc atctggaaac ttgtggacag agaagaagac cacgactgga 1560
gaagcccct ttctgagtgc agggggctg catgcgttgc ctcctgagat cgaggctgca 1620
ggatatgctc agactctaga ggcgtggacc aaggggcatg gagcttcact ccttgctggc 1680
cagggagttg gggactcaga gggaccactt ggggccagcc agactggcct caatggcgga 1740
ctcagtcaca ttgactgacg gggaccaggg cttgtgtggg tcgagagcgc cctcatggtg 1800
ctggtgctgt tgtgtgtagg tcccctgggg acacaagcag gcgccaatgg tatctgggcg 1860
gagctcacag agttcttgga ataaaagcaa cctcagaaca cttaaaaaaa aaaaaaaaaa 1920
aaaaaaaaaa aaaaaaaaaa aaaaaaaaaa aaaaaaaaaa aaaaaaa              1967
```

<210> 4
<211> 1930
<212> DNA
<213> Homo sapiens

<400> 4

gaccaagtgg ctcctgcgat ggcggcggaa gaggaggctg cggcgggagg taaagtgttg 60
agagaggaga accagtgcat tgctcctgtg gtttccagcc gcgtgagtcc agggacaaga 120
ccaacagcta tggggtcttt cagctcacac atgacagagt ttccacgaaa acgcaaagga 180
agtgattcag acccatccca gtcaggaatc atgacagaaa aagtggtgga aaagctttct 240
cagaatcccc ttacctatct tctttcaaca aggatagaaa tatcagcctc cagtggcagc 300
agagtggaag atggtgaaca ccaagttaaa atgaaggcct tcagagaagc tcatagccaa 360
actgaaaagc ggaggagaga taaaatgaat aacctgattg aagaactgtc tgcaatgatc 420
cctcagtgca accccatggc gcgtaaactg gacaaactta cagttttaag aatggctgtt 480
caacacttga gatctttaaa aggcttgaca aattcttatg tgggaagtaa ttatagacca 540
tcatttcttc aggataatga gctcagacat ttaatcctta agactgcaga aggcttctta 600
tttgtggttg gatgtgaaag aggaaaaatt ctcttcgttt ctaagtcagt ctccaaaata 660
cttaattatg atcaggctag tttgactgga caaagcttat ttgacttctt acatccaaaa 720
gatgttgcca aagtaaagga acaactttct tcttttgata tttcaccaag agaaaagcta 780
atagatgcca aaactggttt gcaagttcac agtaatctcc acgctggaag gacacgtgtg 840
tattctggct caagacgatc ttttttctgt cggataaaga gttgtaaaat ctctgtcaaa 900
gaagagcatg gatgcttacc caactcaaag aagaaagagc acagaaaatt ctatactatc 960
cattgcactg gttacttgag aagctggcct ccaaatattg ttggaatgga agaagaaagg 1020
aacagtaaga aagacaacag taattttacc tgccttgtgg ccattggaag attacagcca 1080
tatattgttc cacagaacag tggagagatt aatgtgaaac caactgaatt tataacccgg 1140
tttgcagtga atggaaaatt tgtctatgta gatcaaaggg caacagcgat tttaggatat 1200
ctgcctcagg aacttttggg aacttcttgt tatgaatatt ttcatcaaga tgaccacaat 1260

aatttgactg acaagcacaa agcagttcta cagagtaagg agaaaatact tacagattcc 1320
tacaaattca gagcaaaaga tggctctttt gtaactttaa aaagccaatg gtttagtttc 1380
acaaatcctt ggacaaaaga actggaatat attgtatctg tcaacacttt agttttggga 1440
catagtgagc ctggagaagc atcattttta ccttgtagct ctcaatcatc agaagaatcc 1500
tctagacagt cctgtatgag tgtacctgga atgtctactg gaacagtact tggtgctggt 1560
agtattggaa cagatattgc aaatgaaatt ctggatttac agaggttaca gtcttcttca 1620
taccttgatg attcgagtcc aacaggttta atgaaagata ctcatactgt aaactgcagg 1680
agtatgtcaa ataaggagtt gtttccacca agtccttctg aaatggggga gctagaggct 1740
accaggcaaa accagagtac tgttgctgtc cacagccatg agccactcct cagtgatggt 1800
gcacagttgg atttcgatgc cctatgtgac aatgatgaca cagccatggc tgcatttatg 1860
aattacttag aagcagaggg gggcctggga gaccctgggg acttcagtga catccagtgg 1920
accctctagc 1930

<210> 5
<211> 1067
<212> DNA
<213> Homo sapiens

<400> 5

```
ggcggacgct ccacgtgtcc ctcgccgcgc cccgtctacc cgcccctgcc ctgaggaccc 60
tagtccaaca tggcggcgcc cagcggaggg tggaacggcg tcggcgcgag cttgtgggcc 120
gcgctgctcc taggggccgt ggcgctgagg ccggcggagg cggtgtccga gcccacgacg 180
gtggcgtttg acgtgcggcc cggcggcgtc gtgcattcct tctcccataa cgtgggcccg 240
ggggacaaat atacgtgtat gttcacttac gcctctcaag gagggaccaa tgagcaatgg 300
cagatgagtc tggggaccag cgaagaccac cagcacttca cctgcaccat ctggaggccc 360
caggggaagt cctatctgta cttcacacag ttcaaggcag aggtgcgggg cgctgagatt 420
gagtacgcca tggcctactc taaagccgca tttgaaaggg aaagtgatgt ccctctgaaa 480
actgaggaat ttgaagtgac caaaacagca gtggctcaca ggcccggggc attcaaagct 540
gagctgtcca agctggtgat tgtggccaag gcatcgcgca ctgagctgtg accagcagcc 600
ctgttgcggg tggcaccttc tcatctccgg tgaagctgaa ggggcctgtg tccctgaaag 660
ggccagcaca tcactggttt tctaggaggg actcttaagt tttctacctg ggctgacgtt 720
gccttgtccg gaggggcttg cagggtggct gaagccctgg ggcagagaac agagggtcca 780
gggccctcct ggctcccaac agcttctcag ttcccacttc ctgctgagct cttctggact 840
caggatcgca gatccggggc acaaagaggg tggggaacat gggggctatg ctggggaaag 900
cagccatgct ccccccgacc tccagccgag catccttcat gagcctgcag aactgctttc 960
ctatgtttac ccaggggacc tcctttcaga tgaactggga agagatgaaa tgtttttca 1020
tatttaaata aataagaaca ttaaaaagca aaaaaaaaa aaaaaaa 1067
```

<210> 6
<211> 5167
<212> DNA
<213> Homo sapiens

<400> 6

```
ctggagtctc ccctggtcgg gagcctcagc cttctggaga ctgacaccac cctcttactc 60
agagacgaat cgttttgtcg ttgccttcac ctccctgacc acaagtgctc cggggctctt 120
tctgagggag gcagctgttc taggcgggag gctggagtct cctgggcctg gacgcacccc 180
ggggtgtgag tgatgggtat gcctgaaagg aggggaagtg gcgcggcttt aatcatctgg 240
gctagtcccc ggcgggcctg ggggaacagg gaaactggag gcggccttaa agcgtcagga 300
tgagacatcg caagaggagc tgcagatact gagcgtgcgc cccgggttct cgccgccttc 360
tctccgccga gcagcccttc ggccacccct tgcccttaaa aatctgcaga ctgcgcctcc 420
tctccgcggg agcgagacct agcaggcccg gggctgggcg tgccctcgcc tgccacgctg 480
cgcgctgccc tcagccgggc cgctggggcc gtgcagtgca ccgggcacgc cgcgccaggc 540
tgggggcagg caccgagcct ccgtgggagg tcccgaggca gcttcgcctg ctcgccctgg 600
ctccagccct cacctgccgc agccttagct gagcagccgc cgccactggg cgccccccgc 660
tccccacttc gccagcgccc gctcctcggc tcggcccggg gtagtttgta gggacgcagc 720
tctccacgtg cgcgactgcg aggctggacg ctacgggctc ctggaaagga gacaccagca 780
tttgccacaa tgctgtcatc cactgacttt acatttgctt cctgggagct tgtggtccgc 840
gttgaccatc ccaatgaaga gcagcagaaa gacgtcacac tgagagtatc tggagacctt 900
catgttggag gagtgatgct caagttagta gaacagatca atatatccca agactggtca 960
gactttgctc tttggtggga acagaagcat tgctggcttc tgaaaaccca ctggaccctg 1020
gacaaatatg gggtccaggc agatgcaaag cttctcttca cccctcagca taaaatgctg 1080
cgccttcgtc tgccgaattt gaagatggtg aggttgcgag tcagcttctc agctgtggtt 1140
tttaaagctg tcagtgatat ctgcaaaatc ctgaatatta gaagatcaga agagctttcc 1200
ttgttaaagc cgtctggtga ctattttaag aagaagaaga aaaaagacaa aaataataag 1260
```

```
gaacccataa ttgaagatat tctaaacctg gagagttctc caacagcttc aggttcatca 1320
gtaagtcctg gtttatacag taaaaccatg acccctatat atgaccccat caatggaaca 1380
ccagcatcat ccaccatgac ttggttcagt gacagccctt tgacggaaca aaactgcagc 1440
atcctcgcat tcagccaacc cccccagtcc ccagaagcac ttgcggatat gtaccagcct 1500
cggtctctgg ttgataaagc caagctcaat gcaggttggc tagactcctc acgctccctt 1560
atggaacaag gcatccaaga ggatgagcag ctgctcttac gatttaaata ttattctttc 1620
ttcgacttga atcctaaata tgatgctgtc cgaataaacc aactctatga gcaagccagg 1680
tgggccattc tcttagaaga aattgattgc acagaggaag aaatgttgat ctttgcagct 1740
ctacagtacc acattagcaa actgtcgttg tctgctgaaa cacaggattt tgcaggcgag 1800
tccgaggttg atgaaataga agcggcgctt tctaatttgg aagtaaccct agaaggtgga 1860
aaagcggaca gccttttgga ggacattact gatatcccta aacttgcaga taatctcaaa 1920
ttatttaggc ccaagaagtt actaccaaaa gctttcaaac aatattggtt tatctttaaa 1980
gacacatcca tagcatactt taaaaataag gaacttgaac aaggagaacc actagaaaaa 2040
ctaaatctta gaggctgcga agttgtgccc gatgtaaatg tagcaggaag aaaatttgga 2100
atcaagttac taatccctgt tgccgatggt atgaatgaaa tgtatttgag atgtgaccat 2160
gagaatcaat acgcccaatg gatggctgcc tgcatgttgg catcgaaggg caaaaccatg 2220
gcagacagct cctaccagcc agaggtcctc aacatccttt catttctgag gatgaaaaac 2280
aggaactctg catctcaggt ggcttccagt ctcgaaaaca tggatatgaa cccagaatgt 2340
tttgtgtcac cacggtgtgc aaaaagacac aaatccaaac agctggccgc ccggatcctg 2400
gaggcgcacc agaacgtggc ccagatgccc ctggtcgaag ccaagctgcg gttcatccag 2460
gcgtggcagt cactgcctga gtttggcctc acctactacc ttgtcagatt taaaggaagc 2520
aaaaaagatg acattctggg agtttcatat aacaggttga ttaaaattga tgcagccacc 2580
gggattccag tgacaacatg gagattcaca aatatcaaac agtggaatgt aaactgggaa 2640
acccggcagg tggtcatcga gtttgaccaa aacgtcttta ctgctttcac ctgcctgagt 2700
gcagattgca agattgtgca cgagtacatt ggcggctaca ttttcttgtc cacccgctcc 2760
aaggaccaga atgaaacact cgatgaggac ttgttccaca aattgaccgg cggtcaggat 2820
tgaaacaagc acgcgtgctc ggctcacacc aacaaggcaa gccaaaggcg cccctcccca 2880
gagggatccc taacgtgccc agcatgtaga ttctggacta acagacaaca tacattcacc 2940
gctggtcacc cagatcctca ttcaaaccca ctgctggcac atccctttcc ttactttgcc 3000
ctgtgctacc agccacggaa ggagcctctc ttgtttttttc tataaaatgg gtaggcagga 3060
gaaaagcagg tgccctaaga ttgctctaag gcccagcatg tggttacagt tctctgactt 3120
gcagaacctg ccaggtgtat ggctacaagt tatcctcgtg ctgatctgtc tcattactaa 3180
gtcaatggag aagacagaaa ggtaaaaatc acgtgtagca agaacaactc ttatttcaca 3240
aactcaggta tgaaacgaaa cgcctgtcct tcatggaact gcttttagct cctgtctttt 3300
caaaatggca gagggagttc ctacacacac tttttccctg gaggccaagg tctaggggta 3360
gaaaggggag gggtggggct accaggtagc agttgacaac ccaaggtcag aggagtggcc 3420
ctcagtgtca tctgtccaca gtgatacctg ccaagatgac cactgaccca catctggtct 3480
tagtcattgg tctcctcaga tttctggggc cacctgcaag ccccattcca ttcctacaga 3540
tctctcagcc acctgtaagt cctttgtgaa gatgtgggtg acacaggggg acaggaaaac 3600
ccatttctca acccagatcc atgtctccac tgcttctact ctgggttggg attcaggaag 3660
acaggcacag tcctctctgt tcatagaaac acctgccagt gtcaaggatt ccagtcaggt 3720
gtctatccca actggtcagg gagagaaggg cagacccatt ctcaaagacc accatgtcca 3780
aggtctgaca gctccccact ggctgccccc acaggggctt taggctggtc tgggtcatgg 3840
ggaagcgtcc ctcttatcgc tggtctgtgt tctcctggat ttggtatcta tgttggtacg 3900
actcctggcc ttttatctaa aggactttgg cttttgtaaa tcacaagcca ataatagact 3960
tttttctccc cctctgtttt ttgctgtgtc atctctgcct tgagactgcc ttgagacagt 4020
gcttgccttg agagagtgag ccaattaaca gctgcctgaa ttgtcatttt ccattttggt 4080
ttgttagagg tgggaggggt gggttttgag aaggtcaaaa gcaataccag aagtaaaggg 4140
aaatatcaga caatatttta ttattttttc atagatgttc tgccacacaa agaacttggg 4200
gtgtaaggat aaggcaaaag ctccaatccc atttttcagt tctcctagga tgcacccctc 4260
agggagcctg gccagagttc cgaggcccgt gagcgtcagc tgttgcttta ttttccatca 4320
aagccctctg agaagtgaga cctcagcaat tccgggagcc acatagagac agacttggca 4380
agggaccccc tggttctgag ccagtagctg ccatctggaa attcctcttt tagcctctcc 4440
ttagaggtga atgtgaatga agcctcccag gcacccgctg aatttctgag gccttgctta 4500
aagctcagaa gtggtttagg catttggaaa atctggttca catcataaag aacttgattt 4560
gaaatgtttt ctatagaaac aagtgctaag tgtaccgtat atacttgatg ttggtcattt 4620
ctcagtccta tttctcagtt ctattatttt agaacctagt cagttcttta agattataac 4680
tggtcctaca ttaaaataat gcttctcgat gtcagatttt acctgtttgc tgctgagaac 4740
atctctgcct aatttaccaa agccagacct tcagttcaac atgcttcctt agcttttcat 4800
agttgtctga catttccatg aaaacaaagg aaccaacttt gttttaacca aactttgttt 4860
ggttacagtt ttcaggggag cgtttcttcc atgacacaca gcaacatccc aaagaaataa 4920
acaagtgtga caaaaaaaaa aaaaaacaaa cctaaatgct actgttccaa agagcaactt 4980
gatggttttt tttaatactg agtgcaaaag gtcacccaaa ttcctatgat gaaattttaa 5040
attaatgggc acctttcaac atcatttgct tccttatcta cagttgattc agaaatctgc 5100
atttttttatt cttttatatg acttttaagt aaaagattta tatggatttg aaaaaaaaaa 5160
```

aaaaaaa 5167

<210> 7
<211> 1128
<212> DNA
<213> Homo sapiens

<400> 7

```
agtccgagtg gagagagcga gctgagtggt tgtgtggtcg cgtctcggaa accggtagcg 60
cttgcagcat ggctgaccaa ctgactgaag agcagattgc agaattcaaa gaagcttttt 120
cactatttga caaagatggt gatggaacta taacaacaaa ggaattggga actgtaatga 180
gatctcttgg gcagaatccc acagaagcag agttacagga catgattaat gaagtagatg 240
ctgatggtaa tggcacaatt gacttccctg aatttctgac aatgatggca agaaaaatga 300
aagacacaga cagtgaagaa gaaattagag aagcattccg tgtgtttgat aaggatggca 360
atggctatat tagtgctgca gaacttcgcc atgtgatgac aaaccttgga gagaagttaa 420
cagatgaaga agttgatgaa atgatcaggg aagcagatat tgatggtgat ggtcaagtaa 480
actatgaaga gtttgtacaa atgatgacag caaagtgaag accttgtaca gaatgtgtta 540
aatttcttgt acaaaattgt ttatttgcct ttttctttgtt tgtaacttat ctgtaaaagg 600
tttctcccta ctgtcaaaaa aatatgcatg tatagtaatt aggacttcat tcctccatgt 660
tttcttccct tatcttactg tcattgtcct aaaaccttat tttagaaaat tgatcaagta 720
acatgttgca tgtggcttac tctggatata tctaagccct tctgcacatc taaacttaga 780
tggagttggt caaatgaggg aacatctggg ttatgccttt tttaaagtag ttttctttag 840
gaactgtcag catgttgttg ttgaagtgtg gagttgtaac tctgcgtgga ctatggacag 900
tcaacaatat gtacttaaaa gttgcactat tgcaaaacgg gtgtattatc caggtactcg 960
tacactattt ttttgtactg ctggtcctgt accagaaaca ttttctttta ttgttacttg 1020
ctttttaaac tttgtttagc cacttaaaat ctgcttatgg cacaatttgc ctcaaaatcc 1080
attccaagtt gtatatttgt tttccaataa aaaaattaca atttaccc              1128
```

<210> 8
<211> 3320
<212> DNA
<213> Homo sapiens

<400> 8

```
gtgggtgagc ggcggccacg gcatcctgtg ctgtgggggc tacgaggaaa gatctaatta 60
tcatggacct gcgacagttt cttatgtgcc tgtccctgtg cacagccttt gccttgagca 120
aacccacaga aaagaaggac cgtgtacatc atgagcctca gctcagtgac aaggttcaca 180
atgatgctca gagttttgat tatgaccatg atgccttctt gggtgctgaa gaagcaaaga 240
cctttgatca gctgacacca gaagagagca aggaaaggct tggaaagatt gtaagtaaaa 300
tagatggcga caaggacggg tttgtcactg tggatgagct caaagactgg attaaatttg 360
cacaaaagcg ctggatttac gaggatgtag agcgacagtg gaaggggcat gacctcaatg 420
aggacggcct cgtttcctgg gaggagtata aaaatgccac ctacggctac gttttagatg 480
atccagatcc tgatgatgga tttaactata aacagatgat ggttagagat gagcggaggt 540
ttaaaatggc agacaaggat ggagacctca ttgccaccaa ggaggagttc acagctttcc 600
tgcaccctga ggagtatgac tacatgaaag atatagtagt acaggaaaca atggaagata 660
tagataagaa tgctgatggt ttcattgatc tagaagagta tattggtgac atgtacagcc 720
atgatgggaa tactgatgag ccagaatggg taaagacaga gcgagagcag tttgttgagt 780
ttcgggataa gaaccgtgat gggaagatgg acaaggaaga gaccaaagac tggatccttc 840
cctcagacta tgatcatgca gaggcagaag ccaggcacct ggtctatgaa tcagaccaaa 900
acaaggatgg caagcttacc aaggaggaga tcgttgacaa gtatgactta tttgttggca 960
gccaggccac agattttggg gaggccttag tacggcatga tgagttctga gctgcggagg 1020
aaccctcatt tcctcaaaag taatttattt ttacagcttc tggtttcaca tgaaattgtt 1080
tgcgctactg agactgttac tacaaacttt ttaagacatg aaaaggcgta atgaaaacca 1140
tcccgtcccc attcctcctc ctctctgagg gactggaggg aagccgtgct tctgaggaac 1200
aactctaatt agtacacttg tgtttgtaga tttacacttt gtattatgta ttaacatggc 1260
gtgtttattt ttgtattttt ctctggttgg gagtatgata tgaaggatca agatcctcca 1320
ctcacacatg tagacaaaca ttagctcttt actctttctc aaccccttat atgattttaa 1380
taattctcac ttcactaatt ttgtaagcct gagatcaata agaaatgttc aggagagagg 1440
aaagaaaaaa tatatatgct ccacaattta tatttagaga gagaacactt agtcttgcct 1500
gtcaaaaagt ccaacatttc ataggtagta ggggccacat attacattca gttgctatag 1560
gtccagcaac tgaacctgcc attacctggg caaggaaaga tccctttgct ctaggaaagc 1620
ttggcccaaa ttgattttct tctttttccc cctgtaggac tgactgttgg ctaattttgt 1680
caagcacagc tgtggtggga agagttaggg ccagtgtctt gaaaatcaat caagtagtga 1740
atgtgatctc tttgcagagc tatagataga aacagctgga aaactaaagg aaaaatacaa 1800
atgttttcgg ggcatacatt ttttttctgg gtgtgcatct gttgaaatgc tcaagactta 1860

attatttgcc ttttgaaatc actgtaaatg cccccatccg gttcctcttc ttcccaggtg 1920
tgccaaggaa ttaatcttgg tttcactaca attaaaattc actcctttcc aatcatgtca 1980
ttgaaagtgc ctttaacgaa agaaatggtc actgaatggg aattctctta agaaaccctg 2040
agattaaaaa aagactattt ggataactta taggaaagcc tagaacctcc cagtagagtg 2100
gggatttttt tcttcttccc tttctctttt ggacaatagt taaattagca gtattagtta 2160
tgagtttggt tgcagtgttc ttatcttgtg ggctgatttc caaaaaccac atgctgctga 2220
atttaccagg gatcctcata cctcacaatg caaaccactt actaccaggc ctttttctgt 2280
gtccactgga gagcttgagc tcacactcaa agatcagagg acctacagag agggctcttt 2340
ggtttgagga ccatggctta cctttcctgc ctttgaccca tcacacccca tttcctcctc 2400
tttccctctc cccgctgcca aaaaaaaaaa aaaggaaacg tttatcatga atcaacaggg 2460
tttcagtcct tatcaaagag agatgtggaa agagctaaag aaaccaccct ttgttcccaa 2520
ctccacttta cccatatttt atgcaacaca aacactgtcc ttttgggtcc ctttcttaca 2580
gatggacctc ttgagaagaa ttatcgtatt ccacgttttt agccctcagg ttaccaagat 2640
aaatatatgt atatataacc tttattattg ctatatcttt gtggataata cattcaggtg 2700
gtgctgggtg atttattata atctgaacct aggtatatcc tttggtcttc cacagtcatg 2760
ttgaggtggg ctccctggta tggtaaaaag ccaggtataa tgtaacttca ccccagcctt 2820
tgtactaagc tcttgatagt ggatatactc ttttaagttt agccccaata tagggtaatg 2880
gaaatttcct gccctctggg ttccccattt ttactattaa gaagaccagt gataatttaa 2940
taatgccacc aactctggct tagttaagtg agagtgtgaa ctgtgtggca agagagcctc 3000
acacctcact aggtgcagag agcccaggcc ttatgttaaa atcatgcact tgaaaagcaa 3060
accttaatct gcaaagacag cagcaagcat tatacggtca tcttgaatga tccctttgaa 3120
attttttttt tgtttgtttg tttaaatcaa gcctgaggct ggtgaacagt agctacacac 3180
ccatattgtg tgttctgtga atgctagctc tcttgaattt ggatattggt tattttttat 3240
agagtgtaaa ccaagtttta tattctgcaa tgcgaacagg tacctatctg tttctaaata 3300
aaactgttta cattcaaaaa                                             3320
```

<210> 9
<211> 2758

<212> DNA
<213> Homo sapiens

<400> 9

```
agcagctctt aaagctttgg attcggctgt cgtggagata acgggcacca gaaaaacttg 60
taggagtagc aacagctatt tggtcggatg gaggccagaa gccaagaata atcgaaacac 120
gctgccagcg cccattagat ggatgggaaa tgagtgtaac tcattgggaa agatattaga 180
cttcaaatca cctaaggcaa accctacttc ctggggcgcg ggcgcgggcg ctggcgttag 240
gagacgtcac tcccgcgcat aactgacatg gggccctctt ggtcggcgtt tccgggcggg 300
tccttccgac ggccgccgcg gtgattccat cactcggctt tcttcccggc ctgcctcgcg 360
cccgtagccg ggctgggcca gaacagccca agatggccga cttcgatgat cgtgtgtcgg 420
atgaggagaa ggtacgcata gctgctaaat tcatcactca tgcacccccca ggggaattta 480
atgaagtatt caatgacgtt cggctactac ttaataatga caatctcctc agggaagggg 540
cagcacatgc atttgcccag tataacatgg atcagttcac gcctgtgaag atagaaggat 600
atgaagatca ggtcttaatt acagagcacg gtgacctggg taatagcaga tttttagatc 660
caagaaacaa aatttccttt aaatttgacc acttacggaa agaagcaagt gaccccccagc 720
cagaagaagc agatggaggt ctgaagtctt ggagagaatc ctgtgacagt gctttaagag 780
cctatgtgaa agaccattat tccaacggct tctgtactgt ttatgctaaa actatcgatg 840
ggcaacagac tattattgca tgtattgaaa gccaccagtt tcagcctaaa aacttctgga 900
atggtcgttg gagatcagag tggaagttca ccatcacacc acctacagcc caggtggttg 960
gcgtgcttaa gattcaggtt cactattatg aagatggcaa tgttcagttg gttagtcata 1020
aagatgtaca ggattcacta actgtttcga atgaagccca aactgccaag gagtttatta 1080
aaatcataga gaatgcagaa aatgagtatc agacagcaat tagtgaaaac tatcaaacaa 1140
tgtcagatac cacattcaag gccttgcgcc ggcagcttcc agttacccgc accaaaatcg 1200
actggaacaa gatactcagc tacaagattg gcaaagaaat gcagaatgct taaaggctga 1260
atgtaggatt cttcagtatg tggaaagaca aggattcaac gtgtggtcat atgataaata 1320
agtgatttat aaacaagagt gatattttgc tagggctttc aaagttaacc ggttttctag 1380
cctcatggaa tactgttgaa cctatagcgt tgtcttgatt cttttgtgtt ctctgccttg 1440
taatttctg ttactgctat atctacgtgt aaatcttttt ttcttttttt ttttttttt 1500
ttggttaatt ctgccacatt taatgttggt gagagagtga tctatcctaa tgacatttta 1560
ctgtttaaaa aagtttccta gccatgaagc cctgctactg atttagacaa ggtattatgg 1620
tcattacttt gtacccctat ccttccaagc acttctggta cttcagtcgt ttttactgat 1680
ccaccaacac ctaaagaggc tatgctacag tctctagcta aatggaagac acattcatcc 1740
ttctccctct gactgctttg atcatcattt attgcatctc ataactaatt ttctaaagtt 1800
tggattggga cttttcaggt cctttttgga gggcaaagga agtgccagct tctctgggga 1860
acttgttttt aaatccaaag acttgaacca cattccctgc acatgaacat gtttgctttt 1920
atcccttctc tcattgtctc cttcccatct tagtaccatt gtagttataa acatctgcat 1980

tttttagaag cattttaccc atttattttt ttaaacattc aagaactgct gacgtactgt 2040
ggatgtagag tataaaactt gaaaaatgca gatgttgaag gaataatagg tatcttgtgc 2100
tttaatactt tatggcagga ttgtactata agcaaatgaa ttaaacagct atgtaaatca 2160
taaagaaaaa ctaaaaatga accaaagtga aaggataact tccaggcagt atctttctat 2220
tgtaacctgt tatttaagga aatactagtg atttcttcta aataggatgt aaaacttctt 2280
tcaaattact cttcctcagt cctgcctgcc aagaactcaa gtgtaactgt gataaaataa 2340
cctttcccag gtatattggc aggtatgtgt gtaatctcag aatacacagg tgacatagat 2400
atgatatgac aactggtaat ggtggattca tttacattgt ttacacttct atgaccaggc 2460
cttaagggaa ggtcagtttt ttaaaaaacc aagtagtgtc ttcctaccta tctccagata 2520
catgtcaaaa aaagaaaggt gtttgtgctt ccgttttgtt tctgctcagt aatatagtca 2580
agcaagtttg ttccaggtga cccattgagc tgtgtatgca tttttgttta tttcaataaa 2640
atatatttgt attatttgtc cttcatacta tccatccata ccacactatc ttctgtatca 2700
ggtagtctaa tagaaatata cctgttttgt tctaaaattg aaaaaaaaaa aaaaaaa   2758
```

<210> 10
<211> 799
<212> DNA
<213> Homo sapiens

<400> 10

cactcccaaa gaactgggta ctcaacactg agcagatctg ttctttgagc taaaaaccat 60
gtgctgtacc aagagtttgc tcctggctgc tttgatgtca gtgctgctac tccacctctg 120
cggcgaatca gaagcagcaa gcaactttga ctgctgtctt ggatacacag accgtattct 180
tcatcctaaa tttattgtgg gcttcacacg gcagctggcc aatgaaggct gtgacatcaa 240
tgctatcatc tttcacacaa agaaaaagtt gtctgtgtgc gcaaatccaa aacagacttg 300
ggtgaaatat attgtgcgtc tcctcagtaa aaaagtcaag aacatgtaaa aactgtggct 360
tttctggaat ggaattggac atagcccaag aacagaaaga accttgctgg ggttggaggt 420
ttcacttgca catcatggag ggtttagtgc ttatctaatt tgtgcctcac tggacttgtc 480
caattaatga agttgattca tattgcatca tagtttgctt tgtttaagca tcacattaaa 540
gttaaactgt attttatgtt atttatagct gtaggttttc tgtgtttagc tatttaatac 600
taattttcca taagctattt tggtttagtg caaagtataa aattatattt ggggggggaat 660
aagattatat ggactttctt gcaagcaaca agctattttt taaaaaaact atttaacatt 720
cttttgttta tattgttttg tctcctaaat tgttgtaatt gcattataaa ataagaaaaa 780
cattaataag acaaatatt 799

<210> 11
<211> 1553
<212> DNA
<213> Homo sapiens

<400> 11

cgaggctccg caccagccgc gcttctgtcc gcctgcaggg cattccagaa agatgaggat 60
atttgctgtc tttatattca tgacctactg gcatttgctg aacgcattta ctgtcacggt 120
tcccaaggac ctatatgtgg tagagtatgg tagcaatatg acaattgaat gcaaattccc 180
agtagaaaaa caattagacc tggctgcact aattgtctat tgggaaatgg aggataagaa 240
cattattcaa tttgtgcatg gagaggaaga cctgaaggtt cagcatagta gctacagaca 300
gagggcccgg ctgttgaagg accagctctc cctgggaaat gctgcacttc agatcacaga 360
tgtgaaattg caggatgcag gggtgtaccg ctgcatgatc agctatggtg gtgccgacta 420
caagcgaatt actgtgaaag tcaatgcccc atacaacaaa atcaaccaaa gaattttggt 480
tgtggatcca gtcacctctg aacatgaact gacatgtcag gctgagggct accccaaggc 540
cgaagtcatc tggacaagca gtgaccatca agtcctgagt ggtaagacca ccaccaccaa 600
ttccaagaga gaggagaagc ttttcaatgt gaccagcaca ctgagaatca acacaacaac 660
taatgagatt ttctactgca cttttaggag attagatcct gaggaaaacc atacagctga 720
attggtcatc ccagaactac ctctggcaca tcctccaaat gaaaggactc acttggtaat 780
tctgggagcc atcttattat gccttggtgt agcactgaca ttcatcttcc gtttaagaaa 840
agggagaatg atggatgtga aaaaatgtgg catccaagat acaaactcaa agaagcaaag 900
tgatacacat ttggaggaga cgtaatccag cattggaact tctgatcttc aagcagggat 960
tctcaacctg tggtttaggg gttcatcggg gctgagcgtg acaagaggaa ggaatgggcc 1020
cgtgggatgc aggcaatgtg ggacttaaaa ggcccaagca ctgaaaatgg aacctggcga 1080
aagcagagga ggagaatgaa gaaagatgga gtcaaacagg gagcctggag ggagaccttg 1140
atactttcaa atgcctgagg ggctcatcga cgcctgtgac agggagaaag gatacttctg 1200
aacaaggagc ctccaagcaa atcatccatt gctcatccta ggaagacggg ttgagaatcc 1260
ctaatttgag ggtcagttcc tgcagaagtg ccctttgcct ccactcaatg cctcaatttg 1320
ttttctgcat gactgagagt ctcagtgttg gaacgggaca gtatttatgt atgagttttt 1380
cctatttatt ttgagtctgt gaggtcttct tgtcatgtga gtgtggttgt gaatgatttc 1440

ttttgaagat atattgtagt agatgttaca attttgtcgc caaactaaac ttgctgctta 1500
atgatttgct cacatctagt aaaacatgga gtatttgtaa aaaaaaaaaa aaa 1553

<210> 12
<211> 6017
<212> DNA
<213> Homo sapiens

<400> 12

gggcggggct cgggccggtc cgcccgcgcg caggtgagtg agccagggcg gagcgcagct 60
gcgccgggct tgggcgcctg gggccgccgc tccccaccgt cgttttcccc accgaggccg 120
aggcgtcccg gagtcatggc cggcctgaac tgcggggtct ctatcgcact gctaggggtt 180
ctgctgctgg gtgcggcgcg cctgccgcgc ggggcagaag cttttgagat tgctctgcca 240
cgagaaagca acattacagt tctcataaag ctggggaccc cgactctgct ggcaaaaccc 300
tgttacatcg tcatttctaa aagacatata accatgttgt ccatcaagtc tggagaaaga 360
atagtcttta cctttagctg ccagagtcct gagaatcact ttgtcataga gatccagaaa 420
aatattgact gtatgtcagg cccatgtcct tttggggagg ttcagcttca gccctcgaca 480
tcgttgttgc ctaccctcaa cagaactttc atctgggatg tcaaagctca taagagcatc 540
ggtttagagc tgcagttttc catccctcgc ctgaggcaga tcggtccggg tgagagctgc 600
ccagacggag tcactcactc catcagcggc cgaatcgatg ccaccgtggt caggatcgga 660
accttctgca gcaatggcac tgtgtcccgg atcaagatgc aagaaggagt gaaaatggcc 720
ttacacctcc catggttcca ccccagaaat gtctccggct tcagcattgc aaaccgctca 780
tctataaaac gtctgtgcat catcgagtct gtgtttgagg gtgaaggctc agcaaccctg 840
atgtctgcca actacccaga aggcttccct gaggatgagc tcatgacgtg gcagtttgtc 900
gttcctgcac acctgcgggc cagcgtctcc ttcctcaact tcaacctctc caactgtgag 960
aggaaggagg agcgggttga atactacatc ccgggctcca ccaccaaccc cgaggtgttc 1020
aagctggagg acaagcagcc tgggaacatg gcggggaact tcaacctctc tctgcaaggc 1080
tgtgaccaag atgcccaaag tccagggatc ctccggctgc agttccaagt tttggtccaa 1140
catccacaaa atgaaagcaa taaaatctac gtggttgact tgagtaatga gcgagccatg 1200
tcactcacca tcgagccacg gcccgtcaaa cagagccgca agtttgtccc tggctgtttc 1260
gtgtgtctag aatctcggac ctgcagtagc aacctcaccc tgacatctgg ctccaaacac 1320
aaaatctcct tcctttgtga tgatctgaca cgtctgtgga tgaatgtgga aaaaaccata 1380
agctgcacag accaccggta ctgccaaagg aaatcctact cactccaggt gcccagtgac 1440
atcctccacc tgcctgtgga gctgcatgac ttctcctgga agctgctggt gcccaaggac 1500
aggctcagcc tggtgctggt gccagcccag aagctgcagc agcatacaca cgagaagccc 1560
tgcaacacca gcttcagcta cctcgtggcc agtgccatac ccagccagga cctgtacttc 1620
ggctccttct gcccgggagg ctctatcaag cagatccagg tgaagcagaa catctcggtg 1680
acccttcgca cctttgcccc cagcttccaa caagaggcct ccaggcaggg tctgacggtg 1740
tcctttatac cttatttcaa agaggaaggc gttttcacgg tgacccctga cacaaaaagc 1800
aaggtctacc tgaggacccc caactgggac cggggcctgc catccctcac ctctgtgtcc 1860
tggaacatca gcgtgcccag agaccaggtg gcctgcctga ctttctttaa ggagcggagc 1920
ggcgtggtct gccagacagg gcgcgcattc atgatcatcc aggagcagcg gacccgggct 1980
gaggagatct tcagcctgga cgaggatgtg ctccccaagc caagcttcca ccatcacagc 2040
ttctgggtca acatctctaa ctgcagcccc acgagcggca agcagctaga cctgctcttc 2100
tcggtgacac ttaccccaag gactgtggac ttgactgtca tcctcatcgc agcggtggga 2160
ggtggagtct tactgctgtc tgccctcggg ctcatcattt gctgtgtgaa aaagaagaaa 2220
aagaagacaa acaagggccc cgctgtgggt atctacaatg acaacatcaa tactgagatg 2280
ccgaggcagc caaaaaagtt tcagaaaggg cgaaaggaca atgactccca tgtgtatgca 2340
gtcatcgagg acaccatggt atatgggcat ctgctacagg attccagcgg ctccttcctg 2400
cagccagagg tggacaccta ccggccgttc cagggcacca tggggggtctg tcctccctcc 2460
ccacccacca tatgctccag ggccccaact gcaaagttgg ccactgagga gccacctcct 2520
cgctcccctc ctgagtctga gagtgaaccg tacaccttct cccatcccaa caatggggat 2580
gtaagcagca aggacacaga cattccctta ctgaacactc aggagcccat ggagccagca 2640
gaataacttg atccattcca gacgctttgc tgagtttcat aaagcagggc actgagacac 2700
ccgtccgtgt tcctaaccag aaatcctaaa gaagaggaat tatacagaag gaacagcagg 2760
aggttttcct ggacaccgcc aacttcacat tgctcagtgg actcattcta agggcaagac 2820
attgaaaatg atgaattcca atctggatac agtcatgaca gctcatgtgc tcctcaactt 2880
aggctgtgcg gttagccagc ctgtaatgag aggagagagg cctgagtcac ctagcatagg 2940
gttgcagcaa gccctggatt cagagtgtta aacagaggct tgccctcttc aggacaacag 3000
ttccaattcc aaggagccta cctgaggtcc ctactctcac tggggtcccc aggatgaaaa 3060
cgacaatgtg ccttttttatt attatttatt tggtggtcct gtgttattta agagatcaaa 3120
tgtataacca cctagctctt ttcacctgac ttagtaataa ctcatactaa ctggtttgga 3180
tgcctgggtt gtgacttcta ctgaccgcta gataaacgtg tgcctgtccc ccaggtggtg 3240
ggaataattt acaatctgtc caaccagaaa agaatgtgtg tgtttgagca gcattgacac 3300
atatctgctt tgataagaga cttcctgatt ctctaggtcg gttcgtggtt atcccattgt 3360

```
ggaaattcat cttgaatccc attgtcctat agtcctagca ataagagaaa tttcctcaag 3420
tttccatgtg cggttctcct agctgcagca atactttgac atttaaagag aaatttagag 3480
aatattctca tcctctaaaa atgtttaaat atataccaaa cagtggcccc ctgcattagt 3540
tttctgttgc cactgcaacc tattacttgg tagcttaaaa acaacacatt agcttatagt 3600
cctggggatc agaattccaa aatggatgtc cctgaatgaa aatcaaggtg tcagcagagc 3660
tgtgctcctt ctgaaggctc tagggagaag ccggttcctt gccatttcaa gcttctagag 3720
gctggctgca ttcccaggct ccagtggctg gtcaagcttt tctcacatgg catcactgtg 3780
acactggccc tcccacttcc ctctttgact tacaaagccc accaggaaga tccaggataa 3840
tctctccatc taaagttcct tcatcatcct ggaagagcct tttgccatgc aagacaacat 3900
agccacaggt ggggattagg accagaacat ctttggggtg ctgttattct gcctaccaca 3960
ccttcctgcc actgactccc acaggagagg ctacaaaatg atctggcgca cagggatgtt 4020
ttgtttagct tgcggactct aacacttaaa aaaaaaccca gatcagaaga tctggccatg 4080
ctggggctca cattctcacc tagcaacaac tggctggagc tgggcaccag ctctgccttt 4140
agaaggggtg tccacttcac caggtcacca cagcccacac tacgccctat cacttcccac 4200
aatgaggctg agtgtttgtt tctactgatc aatgcccctg caggttgcat ttattgtaat 4260
gaaaaagaaa gactgggatt aatctctaat caggtgagta gaccatgaga ccaatgtgtg 4320
ctcacattac cctttttctt tttttctttt ttctttttct tttttttttt aatgtgagac 4380
aggatctcat tctgttgcct aggctggagt gcagtggcgc aatctcggct cactgcaacc 4440
tctgcctcct gggctcaagc aattctccca cctcagcctc ccaaatagct gggatcactg 4500
gcacaaacca ccatgcccag ctaattttgt atttttgta gagacagggt ttcaccatgt 4560
tgcccaggct ggtctcaacc tcctgggctc aagcaatcct cctgcctcgg cctcccaaag 4620
tgctgggatt acagatgtga gccaccgcat ccagccccac accctcattt ataccaatta 4680
cctgcccagt aactgtggac ttttgcttcc tcacccctgc tctgatctgg aaggagaggg 4740
attatgttat agcttgtcag cacagtccca agttcaatat ttctgcggca aaaacttcct 4800
tcaaaaaata aatgtacttc attgtattca atgaattcac cttggaaatg caccgcctca 4860
acttgttcac atggcataaa tgaaaggaat tttatagtct cctaaatggc gtgtactgca 4920
agacctcttg aacactttcc agaggatagg atatttaagt catgcccttg gcgttgccta 4980
tggcaccttt cccttctgaa agtctggttc ctgcccagtg acccttggcc ttgtgagccg 5040
agatgctgac cctgcataaa gggccaaagg agggctgcgg cttccttccc tcactgaaga 5100
gcccttattt gaattcactg tgtggagccc tagccctcca ttctcgacat tccccaacct 5160
cccagcccct tccaagcagg actaggtgcc ctgcattcca cccaaggtgg gattggcctt 5220
ccttaggctg gctacttgtc accatcaccg acatcactgt tgcctgcaag gacaccacgt 5280
ggccattttc cttcaactga gggctcaaaa ctcctggaca agttgctggc tcctgagacc 5340
agtatttcct ggagctgtgc ctcagtgaag gggcccagcc tgaggaaccc tggctctttt 5400
ctttaaagcc caggccccac ttacgtaaaa catttcaggg tcactggaaa cagtgaagtg 5460
ccatttgttg aagcctactg catgccagcc cactgctcat ccacgtggtc tgccatgcct 5520
acgaggaagg ccagcgcatg caggactggt ctctaatgct gtggtcattg cacagaaggg 5580
aaaggtctca aggaagagtc aactggaaca agcacaagcc caccggacat ggccttggta 5640
aaggttagca gactggtgtg tgtggatctg cagtgcttca ctggaaataa tttattcatt 5700
gcagatactt tttaggtggc attttattca tttcctgtgc tttaaataaa caaatgtacc 5760
aaaaaacaag tatcaagctg tttaagtgct tcggctactt gtcccctggt tcagtagagg 5820
ccccggtttc ccagttgttg actgtgacag gctcagcatg ggctcagcag atgctgtctt 5880
aatttgtgga tgatacagaa agccaggctt tgggatacaa gttctttcct cttcatttga 5940
tgccgtgcac tgtgtgaagc agatgttttt gtccggaaat aaaaataata gtcttggagt 6000
ctcgccaaaa aaaaaaa                                                6017
```

<210> 13
<211> 1860
<212> DNA
<213> Homo sapiens

<400> 13

acgtcagaaa aaaaaaaaaa aagtttgcag aatgtcccac accgaaaaaa aaaaaaaatc 60
cgaaaccgag cgaaaaaaac ctgcgagtgg gcctggcgga tgggattatt aaagcttcgc 120
cggagccgcg gctcgccctc ccactccgcc agcctccggg agaggagccg cacccggccg 180
gcccggcccc agccccatgg acctccgagc aggggactcg tgggggatgt tagcgtgcct 240
gtgcacggtg ctctggcacc tccctgcagt gccagctctc aatcgcacag gggacccagg 300
gcctggcccc tccatccaga aaacctatga cctcacccgc tacctggagc accaactccg 360
cagcttggct gggacctatc tgaactacct gggccccct ttcaacgagc cagacttcaa 420
ccctccccgc ctgggggcag agactctgcc cagggccact gttgacttgg aggtgtggcg 480
aagcctcaat gacaaactgc ggctgaccca gaactacgag gcctacagcc accttctgtg 540
ttacttgcgt ggcctcaacc gtcaggctgc cactgctgag ctgcgccgca gcctggccca 600
cttctgcacc agcctccagg gcctgctggg cagcattgcg ggcgtcatgg cagctctggg 660
ctacccactg ccccagccgc tgcctgggac tgaacccact tggactcctg gccctgccca 720
cagtgacttc ctccagaaga tggacgactt ctggctgctg aaggagctgc agacctggct 780

gtggcgctcg gccaaggact tcaaccggct caagaagaag atgcagcctc cagcagctgc 840
agtcaccctg cacctggggg ctcatggctt ctgacttctg accttctcct cttcgctccc 900
ccttcaaacc ctgctcccac tttgtgagag ccagccctgt atgccaacac ctgttgagcc 960
aggagacaga agctgtgagc ctctggccct ttcctggacc ggctgggcgt gtgatgcgat 1020
cagccctgtc tcctccccac ctcccaaagg tctaccgagc tggggaggag gtacagtagg 1080
ccctgtcctg tcctgtttct acaggaagtc atgctcgagg gagtgtgaag tggttcaggt 1140
tggtgcagag gcgctcatgg cctcctgctt cttgcctacc acttggccag tgcccaccca 1200
gcccctcagg tggcacatct ggagggcagg ggttgagggg ccaccaccac acatgccttt 1260
ctggggtgaa gccctttggc tgccccactc tccttggatg ggtgttgctc ccttatcccc 1320
aaatcactct atacatccaa ttcaggaaac aaacatggtg gcaattctac acaaaaagag 1380
atgagattaa cagtgcaggg ttggggtctg cattggaggt gccctataaa ccagaagaga 1440
aaatactgaa agcacagggg cagggacaga ccagaccaga cccaggagtc tccaaagcac 1500
agagtggcaa acaaaacccg agctgagcat caggaccttg cctcgaattg tcttccagta 1560
ttacggtgcc tcttctctgc cccctttccc agggtatctg tgggttgcca ggctggggag 1620
ggcaaccata gccacaccac aggatttcct gaaagtttac aatgcagtag cattttgggg 1680
tgtagggtgg cagctcccca aggccctgcc ccccagcccc acccactcat gactctaagt 1740
gtgttgtatt aatatttatt tatttggaga tgttatttat tagatgatat ttattgcaga 1800
atttctattc ttgtattaac aaataaaatg cttgccccag aaaaaaaaaa aaaaaaaaaa 1860

<210> 14
<211> 2106
<212> DNA
<213> Homo sapiens

<400> 14

gcgcgatggc ggcggctcct ttaggcagct gaaaggggat ttaggcccgg aagatccgag 60
tccatccgcg gcggggagag ggcaagcggg accggtaggg gccggagcag cggcggcggc 120
gctcggactg tcccatccgc cccgtattga ggcgctggga gcggcggggc gacaggaaag 180
cgatggtgaa agcggggccg tgaggggggc ggagccggga gccggacccg cagtagcggc 240
agcagcggcg ccgcctccca gagttcagac ccaggaagcg gccgggaggg caggagcgaa 300
tcgggccgcc gccgccatgg agctgagagt cgggaacagg taccggctgg gccggaagat 360
cggcagcggc tccttcggag acatctatct cggtacggac attgctgcag gagaagaggt 420
tgccatcaag cttgaatgtg tcaaaaccaa acaccctcag ctccacattg agagcaaaat 480
ctacaagatg atgcagggag gagtgggcat ccccaccatc agatggtgcg gggcagaggg 540
ggactacaac gtcatggtga tggagctgct ggggccaagc ctggaggacc tcttcaactt 600
ctgctccagg aaattcagcc tcaaaaccgt cctgctgctt gctgaccaaa tgatcagtcg 660
catcgaatac attcattcaa agaacttcat ccaccgggat gtgaagccag acaacttcct 720
catgggcctg gggaagaagg gcaacctggt gtacatcatc gacttcgggc tggccaagaa 780
gtaccgggat gcacgcaccc accagcacat cccctatcgt gagaacaaga acctcacggg 840
gacggcgcgg tacgcctcca tcaacacgca ccttggaatt gaacaatccc gaagagatga 900
cttggagtct ctgggctacg tgctaatgta cttcaacctg ggctctctcc cctggcaggg 960
gctgaaggct gccaccaaga gacagaaata cgaaaggatt agcgagaaga aaatgtccac 1020
ccccatcgaa gtgttgtgta aggctaccc ttccgaattt gccacatacc tgaatttctg 1080
ccgttccttg cgttttgacg acaagcctga ctactcgtac ctgcggcagc ttttccggaa 1140
tctgttccat cgccagggct tctcctatga ctacgtgttc gactggaaca tgctcaaatt 1200
tggtgccagc cgggccgccg atgacgccga gcgggagcgc agggaccgag aggagcggct 1260
gagacactcg cggaacccgg ctacccgcgg cctcccttcc acagcctccg gccgcctgcg 1320
ggggacgcag gaagtggctc cccccacacc cctcacccct acctcacaca cggctaacac 1380
ctccccccgg cccgtctccg gcatggagag agagcggaaa gtgagtatgc ggctgcaccg 1440
cggggccccc gtcaacatct cctcgtccga cctcacaggc cgacaagata cctctcgcat 1500
gtccacctca cagaatagca ttcctttcga acaccacggc aagtagctgc tcgtctccca 1560
tcggaaggca gcactggatt cctggtcggg tggcttccag tggtcttcag tctgtcgtgc 1620
accgatgaga actctcctta ttgctgtgaa gggcagacaa tgcatggctg atctactctg 1680
ttaccaatgg ctttactagt gacacgtccc ccggtctagg atcgaaatgt taacaccggg 1740
agctctccag gccactcacc cagcgacgct cgtgggggaa acatactaaa cggacagact 1800
ccaagagctg ccaccgctgg ggctgcactg cggcccccca cgtgaactcg gttgtaacgg 1860
ggctgggaag aaaagcagag agagaattgc agagaatcag actccttttc cagggcctca 1920
gctccctcca gtggtggccg ccctgtactc cctgacgatt ccactgtaac taccaatctt 1980
ctacttggtt aagacagttt tgtatcattt tgctaaaaat tattggctta aatctgtgta 2040
aagaaaaaaa aaaaaaaaaa aaaaaaaaaa aaaaaaaaaa aaaaaaaaaa aaaaaaaaaa 2100
aaaaaa 2106

<210> 15
<211> 1103
<212> DNA
<213> Homo sapiens

<400> 15

```
cacagagccc gggccgcagg cacctcctcg ccagctcttc cgctcctctc acagccgcca 60
gacccgcctg ctgagcccca tggcccgcgc tgctctctcc gccgccccca gcaatccccg 120
gctcctgcga gtggcactgc tgctcctgct cctggtagcc gctggccggc gcgcagcagg 180
agcgtccgtg gccactgaac tgcgctgcca gtgcttgcag accctgcagg gaattcaccc 240
caagaacatc caaagtgtga acgtgaagtc ccccggaccc cactgcgccc aaaccgaagt 300
catagccaca ctcaagaatg ggcggaaagc ttgcctcaat cctgcatccc ccatagttaa 360
gaaaatcatc gaaaagatgc tgaacagtga caaatccaac tgaccagaag ggaggaggaa 420
gctcactggt ggctgttcct gaaggaggcc ctgcccttat aggaacagaa gaggaaagag 480
agacacagct gcagaggcca cctggattgt gcctaatgtg tttgagcatc gcttaggaga 540
agtcttctat ttatttattt attcattagt tttgaagatt ctatgttaat attttaggtg 600
taaaataatt aagggtatga ttaactctac ctgcacactg tcctattata ttcattcttt 660
ttgaaatgtc aaccccaagt tagttcaatc tggattcata tttaatttga aggtagaatg 720
ttttcaaatg ttctccagtc attatgttaa tatttctgag gagcctgcaa catgccagcc 780
actgtgatag aggctggcgg atccaagcaa atggccaatg agatcattgt gaaggcaggg 840
gaatgtatgt gcacatctgt tttgtaactg tttagatgaa tgtcagttgt tatttattga 900
aatgatttca cagtgtgtgg tcaacatttc tcatgttgaa actttaagaa ctaaaatgtt 960
ctaaatatcc cttggacatt ttatgtcttt cttgtaaggc atactgcctt gtttaatggt 1020
agttttacag tgtttctggc ttagaacaaa ggggcttaat tattgatgtt ttcatagaga 1080
atataaaaat aaagcactta tag                                         1103
```

<210> 16
<211> 1234
<212> DNA
<213> Homo sapiens

<400> 16

```
gagctccggg aatttccctg gcccgggact ccgggctttc cagccccaac catgcataaa 60
aggggttcgc cgttctcgga gagccacaga gcccgggcca caggcagctc cttgccagct 120
ctcctcctcg cacagccgct cgaaccgcct gctgagcccc atggcccgcg ccacgctctc 180
cgccgccccc agcaatcccc ggctcctgcg ggtggcgctg ctgctcctgc tcctggtggc 240
cgccagccgg cgcgcagcag gagcgcccct ggccactgaa ctgcgctgcc agtgcttgca 300
gaccctgcag ggaattcacc tcaagaacat ccaaagtgtg aaggtgaagt cccccggacc 360
ccactgcgcc caaaccgaag tcatagccac actcaagaat gggcagaaag cttgtctcaa 420
ccccgcatcg cccatggtta agaaaatcat cgaaaagatg ctgaaaaatg gcaaatccaa 480
ctgaccagaa ggaaggagga agcttattgg tggctgttcc tgaaggaggc cctgccctta 540
caggaacaga agaggaaaga gagacacagc tgcagaggcc acctggattg cgcctaatgt 600
gtttgagcat cacttaggag aagtcttcta tttatttatt tatttattta tttgtttgtt 660
ttagaagatt ctatgttaat attttatgtg taaaataagg ttatgattga atctacttgc 720
acactctccc attatattta ttgtttattt taggtcaaac ccaagttagt tcaatcctga 780
ttcatattta atttgaagat agaaggtttg cagatattct ctagtcattt gttaatattt 840
cttcgtgatg acatatcaca tgtcagccac tgtgatagag gctgaggaat ccaagaaaat 900
ggccagtgag atcaatgtga cggcagggaa atgtatgtgt gtctattttg taactgtaaa 960
gatgaatgtc agttgttatt tattgaaatg atttcacagt gtgtggtcaa catttctcat 1020
gttgaagctt taagaactaa aatgttctaa atatcccttg gacattttat gtctttcttg 1080
taaggcatac tgccttgttt aatgttaatt atgcagtgtt tccctctgtg ttagagcaga 1140
gaggtttcga tatttattga tgttttcaca aagaacagga aaataaaata tttaaaaata 1200
taaaaaaaaa aaaaaaaaaa aaaaaaaaaa aaaa                             1234
```

<210> 17
<211> 1166
<212> DNA
<213> Homo sapiens

<400> 17

gctccgggaa tttccctggc ccggccgctc cgggctttcc agtctcaacc atgcataaaa 60
agggttcgcc gatcttgggg agccacacag cccgggtcgc aggcacctcc ccgccagctc 120
tcccgcttct cgcacagctt cccgacgcgt ctgctgagcc ccatggccca cgccacgctc 180
tccgccgccc ccagcaatcc ccggctcctg cgggtggcgc tgctgctcct gctcctggtg 240
gccgccagcc ggcgcgcagc aggagcgtcc gtggtcactg aactgcgctg ccagtgcttg 300
cagacactgc agggaattca cctcaagaac atccaaagtg tgaatgtaag gtcccccgga 360
ccccactgcg cccaaaccga agtcatagcc acactcaaga atgggaagaa agcttgtctc 420

aaccccgcat cccccatggt tcagaaaatc atcgaaaaga tactgaacaa ggggagcacc 480
aactgacagg agagaagtaa gaagcttatc agcgtatcat tgacacttcc tgcagggtgg 540
tccctgccct taccagagct gaaaatgaaa aagagaacag cagctttcta gggacagctg 600
gaaaggactt aatgtgtttg actatttctt acgagggttc tacttattta tgtatttatt 660
tttgaaagct tgtattttaa tattttacat gctgttattt aaagatgtga gtgtgtttca 720
tcaaacatag ctcagtcctg attatttaat tggaatatga tgggttttaa atgtgtcatt 780
aaactaatat ttagtgggag accataatgt gtcagccacc ttgataaatg acagggtggg 840
gaactggagg gtggggggat tgaaatgcaa gcaattagtg gatcactgtt agggtaaggg 900
aatgtatgta cacatctatt ttttatactt tttttttaaa aaaagaatgt cagttgttat 960
ttattcaaat tatctcacat tatgtgttca acatttttat gctgaagttt cccttagaca 1020
ttttatgtct tgcttgtagg gcataatgcc ttgtttaatg tccattctgc agcgtttctc 1080
tttcccttgg aaaagagaat ttatcattac tgttacattt gtacaaatga catgataata 1140
aaagttttat gaaaaaaaaa aaaaaa 1166

<210> 18
<211> 2475
<212> DNA
<213> Homo sapiens

<400> 18

```
gtgcagaagg cacgaggaag ccacagtgct ccggatcctc caatcttcgc tcctccaatc 60
tccgctcctc cacccagttc aggaacccgc gaccgctcgc agcgctctct tgaccactat 120
gagcctcctg tccagccgcg cggcccgtgt ccccggtcct tcgagctcct tgtgcgcgct 180
gttggtgctg ctgctgctgc tgacgcagcc agggcccatc gccagcgctg gtcctgccgc 240
tgctgtgttg agagagctgc gttgcgtttg tttacagacc acgcaaggag ttcatcccaa 300
aatgatcagt aatctgcaag tgttcgccat aggcccacag tgctccaagg tggaagtggt 360
agcctccctg aagaacggga aggaaatttg tcttgatcca gaagcccctt ttctaaagaa 420
agtcatccag aaaattttgg acggtggaaa caaggaaaac tgattaagag aaatgagcac 480
gcatggaaaa gtttcccagt cttcagcaga gaagttttct ggaggtctct gaacccaggg 540
aagacaagaa ggaaagattt tgttgttgtt tgtttatttg tttttccagt agttagcttt 600
cttcctggat tcctcacttt gaagagtgtg aggaaaacct atgtttgccg cttaagcttt 660
cagctcagct aatgaagtgt ttagcatagt acctctgcta tttgctgtta ttttatctgc 720
tatgctattg aagttttggc aattgactat agtgtgagcc aggaatcact ggctgttaat 780
ctttcaaagt gtcttgaatt gtaggtgact attatatttc caagaaatat tccttaagat 840
attaactgag aaggctgtgg atttaatgtg gaaatgatgt ttcataagaa ttctgttgat 900
ggaaatacac tgttatcttc acttttataa gaaataggaa atattttaat gtttcttggg 960
gaatatgtta gagaatttcc ttactcttga ttgtgggata ctatttaatt atttcacttt 1020
agaaagctga gtgtttcaca ccttatctat gtagaatata tttccttatt cagaatttct 1080
aaaagtttaa gttctatgag ggctaatatc ttatcttcct ataattttag acattcttta 1140
tctttttagt atggcaaact gccatcattt acttttaaac tttgatttta tatgctattt 1200
attaagtatt ttattaggag taccataatt ctggtagcta aatatatatt ttagatagat 1260
gaagaagcta gaaaacaggc aaattcctga ctgctagttt atatagaaat gtattctttt 1320
agtttttaaa gtaaaggcaa acttaacaat gacttgtact ctgaaagttt tggaaacgta 1380
ttcaaacaat ttgaatataa atttatcatt tagttataaa aatatatagc gacatcctcg 1440
aggccctagc atttctcctt ggataggga ccagagagag cttggaatgt taaaaacaaa 1500
acaaaacaaa aaaaaacaag gagaagttgt ccaagggatg tcaatttttt atccctctgt 1560
atgggttaga ttttccaaaa tcataatttg aagaaggcca gcatttatgg tagaatatat 1620
aattatatat aaggtggcca cgctggggca agttccctcc ccactcacag ctttggcccc 1680
tttcacagag tagaacctgg gttagaggat tgcagaagac gagcggcagc ggggagggca 1740
gggaagatgc ctgtcgggtt tttagcacag ttcatttcac tgggattttg aagcatttct 1800
gtctgaatgt aaagcctgtt ctagtcctgg tgggacacac tggggttggg ggtgggggaa 1860
gatgcggtaa tgaaaccggt tagtcagtgt tgtcttaata tccttgataa tgctgtaaag 1920
tttatttttta caaatatttc tgtttaagct atttcacctt tgtttggaaa tccttccctt 1980
ttaaagagaa aatgtgacac ttgtgaaaag gcttgtagga aagctcctcc cttttttttct 2040
ttaaaccttt aaatgacaaa cctaggtaat taatggttgt gaatttctat ttttgctttg 2100
tttttaatga acatttgtct ttcagaatag gattctgtga taatatttaa atggcaaaaa 2160
caaaacataa ttttgtgcaa ttaacaaagc tactgcaaga aaaataaaac atttcttggt 2220
aaaaacgtat gtatttatat attatatatt tatatataat atatattata tatttagcat 2280
tgctgagctt tttagatgcc tattgtgtat cttttaaagg ttttgaccat tttgttatga 2340
gtaattacat atatattaca ttcactatat taaaattgta ctttttttact atgtgtctca 2400
ttggttcata gtctttattt tgtcctttga ataaacatta aaagatttct aaacttcaaa 2460
aaaaaaaaaa aaaaa                                                  2475
```

<210> 19
<211> 3974
<212> DNA
<213> Homo sapiens

<400> 19

ggggctaggg ggtggggctg cagccgggac ggggccttcg gcagggcctg cgggggcggg 60
acggagcgga cccgagtgat acccgggaga ctagcttggc cacaggagac aacgttgagg 120
tacagacagg attattcaag atcaggttgt agccattttc aacaaggatc actgatcaaa 180
attttctttt tacaaagaga tttacaagtt aaacatgaag ggaaaatact ggaatcattt 240
ggattctgac aggataaaga atacaatatt tcacatttac ttgtaatgga acctttgtaa 300
gattcatcac ttgtcattca cccagttggg aacatggatg ttggtttttc tcgtactact 360
gttcagacac tgtcaagaag ccactgcaaa aacatcaaac aaaaaatttc tcaatgggaa 420
ggaagggcta atggtatatc taatccagaa aagtggtgtc caaaggactt tggagtgaga 480
tataactgtc accaagagat ccgtcttaag aaaaatccta tagctgagag aaagagcaaa 540
aacttggatg taaccagccg tgaaaatgtg ggtctagata taaatgaaaa taccaaaagc 600
catgatcaaa gtgagaatga aaataagaaa catgaatatg acgatacaca cttctttaaa 660
aatgaatcag aatccaactg ggtatgttct cgggtcaaag aaattgaaag ctgtaaagaa 720
gatgtcttag atccagagac ttcattacct ccaggaaact tctatacctc acaaatactg 780
tggaagaaaa tagaagcact tcccccagat aaactcttaa atttggcttt agaacattgt 840
gactcttcag aaaaagaact gaacttcaga gttctggata gttcatacgg aataaccaag 900
agcttagaaa atatttactc tgaacctgag gggcaagaat gtggaccttc cataaatcct 960
ttgccaaaac ctcgtaggac attcagatat ttatccgaat ctggtgttac gccgtataaa 1020
gaaagaaact gtgacaaaaa atactgtgaa aataactctt gtgcacaatc ttctttggcc 1080
tcttctcagg aacctgaacc aaagaaatat ggtggaaaaa tcagaggaag atctaaaagg 1140
aaatcctttg aatttgagga tattcagcac tttcgaaatc ggaactcaca gacgattcgt 1200
gaagaacttg gaagaaattc tgggtcagca ctttattaca cacagtctga ggacaatatc 1260
tatgaagata tcatatatcc caccaaagaa aatccatatg aagatattcc agtgcagcct 1320
ttacctatgt ggagatcccc ttcagcatgg aagctaccac ccgctaaaag tgcttttaaa 1380
gcacccaagc ttgtattgaa aatagatgac atatttgaat ctaaaagagg gaagaagaag 1440
gtaaagttac attcttacac tggaaaggaa ttacctccga caaaaggtga aaccagtggg 1500
aacgaaagtg atgccgagta tctgccaaag aatcgccata aacgcttagc acaactgcaa 1560
ccgtcttcca agaggaatcc tcactaccag accttggagc gggatcttat tgaattacag 1620
gagcagcagc tgtttgaact tttgtggtg gtgtctctac agaagaaacc atcaggaata 1680
agctatattc cccaggtcat acaacaattc cctggcaagg atgatcatgg ctataagcag 1740
tccaaagaca tggaagagag acttaaagtt attccaaaat tttgttttcc tgattcaaag 1800
gactggatgc caacctcaga actcaagagt gaaacattct cctttgtctt gactggtgaa 1860
gatggaagcc ggtggtttgg ttactgtaag aagctcttgc cagtaggcaa aggaaagcga 1920
ctccctgagg tatactgcat ggttagtcgc ctaggctgct tcaatctttt ttcaaagatt 1980
ctggatgaag tagagaagag aagagaaatg tctccagccc ttgtttaccc attcatgcga 2040
agtgtcatgg aagctccttt cccagctcct ggacgcacca tcacagttaa gagttacctc 2100
cctggggctg gagatgagtc cattgaactc tgccgaccac tagattcccg attggaacat 2160
gttgatttta aatgtctctt taagtgcctg agtgtgtgtc atctcatccg ggtctgtgcc 2220
tctctccttt tggagcgtag ggtaatcttt gttgccaaca gcctaagcac cctgtcaaaa 2280
tgtggccatg ctgtggtagc tacactgtat ccgttcacct ggcagcatac ctatatccca 2340
gtcctgccag catctatgat tgacatcgtg tgctcaccta caccattcct tattggaatc 2400
ctgtcttgct ccttaccaca gctccaggac ctacccattg aagaggtgct gatagttgat 2460
ctctgtgcag acaagttctt acaggaggta tctgatgagg atgaaattct accaccaaaa 2520
cttcaagctg ccctgatgca gattttggaa gaacgaaatg aaatcttgac tcaggagcag 2580
aatttttcac aagatgtgac actcaactct ctggtgtccg aagcatttgt caggtttttt 2640
gtggagttgg taggacatta ttctttgaac atgactgtca ctgagcgtgg ggagcgtgtt 2700
ttccaaaggg aaccattccg taagtcccac acctcccgaa gtgtacgcca cttcctggat 2760
ctcttcatgg aaactcagat gtttgcagga ttcatccagg accgagagct tcggaaaagt 2820
ggagttaaag gtttgtttga gatccgggcc atccagtatt tggaaacaat tcctgagtcg 2880
gagcccagtg ggatgaatag aattttgcgg agtcttggaa gcaaaatgaa atttctgcaa 2940
aagaaatgaa atctctgatt gtctccatcc tgaatataca acagaaagtg ccaaagaaaa 3000
tatttttcta agggtcagga tcccatgaag tattctacaa aattcttgaa atttaaaaag 3060
gttaaagagc gagtcaggct gttagaggag gattctcctg ttgctgctgt agtaatcact 3120
ggagaattgt tgggaatggt ctggcaccat gccatcttta ctctgtctcc attttttttt 3180
tatagcagcc ttttctatgt gattcttggg ctcattctct gttttgtgct ttgtctgtga 3240
aagtagttgt gtttcacaag gaaaagcctt gttgttgtta catttaaaaa atagctcaaa 3300
gatgagaata atgagagaaa tgctgagttt aaggtagtag agagacatga tggaaatgga 3360
gaacactagc cttatacaca taattctcat cactgaacta tcatggcccc tttaaaccca 3420
tgggctgttt gacctgggct aaggaataga ctcatcacat atctccattg ttttgcctta 3480
tattatatct aggggaatct cacttaggag gaagccctac ttgtaatatt ataaggtact 3540
gttattaagt accttataaa attagacatc ttgcaactcc tcctccaaat tatgtggcat 3600
ttgagtagtt tgcttgtgcc tcacatgtca acattaaact ttgtggaggc tggatgcagt 3660

ggctcacacc tgtaatctcc accactttgg gaggctaagg caggagtttg agggtgggag 3720
tttgagacta gcctgggcaa catagcaaga tcccatctct acaaaaacat tttaaaaata 3780
agtaaataaa tttaaacttt gtgaaattat agctaatatg aaaatgtcct ttcaagggca 3840
tgggagccac ctttattttt tagtgtccac catgtgccat gcactgtggt atatgtatgc 3900
atatcatctc aaccaatcct taaatcaacc ttatgagata gatatttgca tctccatttt 3960
actaataaac tgag 3974

<210> 20
<211> 2015
<212> DNA
<213> Homo sapiens

<400> 20

tcgctgcgaa ggacatttgg gctgtgtgtg cgacgcgggt cggaggggca gtcgggggaa 60
ccgcgaagaa gccgaggagc ccggagcccc gcgtgacgct cctctctcag tccaaaagcg 120
gcttttggtt cggcgcagag agacccgggg gtctagcttt tcctcgaaaa gcgccgccct 180
gcccttggcc ccgagaacag acaaagagca ccgcagggcc gatcacgctg gggcgctga 240
ggccggccat ggtcatggaa gtgggcaccc tggacgctgg aggcctgcgg gcgctgctgg 300
gggagcgagc ggcgcaatgc ctgctgctgg actgccgctc cttcttcgct ttcaacgccg 360
gccacatcgc cggctctgtc aacgtgcgct tcagcaccat cgtgcggcgc cgggccaagg 420
gcgccatggg cctggagcac atcgtgccca acgccgagct ccgcggccgc ctgctggccg 480
gcgcctacca cgccgtggtg ttgctggacg agcgcagcgc cgccctggac ggcgccaagc 540
gcgacggcac cctggccctg gcggccggcg cgctctgccg cgaggcgcgc gccgcgcaag 600
tcttcttcct caaaggagga tacgaagcgt tttcggcttc ctgcccggag ctgtgcagca 660
aacagtcgac cccccatgggg ctcagccttc ccctgagtac tagcgtccct gacagcgcgg 720
aatctgggtg cagttcctgc agtaccccac tctacgatca gggtggcccg gtggaaatcc 780
tgccctttct gtacctgggc agtgcgtatc acgcttcccg caaggacatg ctggatgcct 840
tgggcataac tgccttgatc aacgtctcag ccaattgtcc caaccatttt gagggtcact 900
accagtacaa gagcatccct gtggaggaca accacaaggc agacatcagc tcctggttca 960
acgaggccat tgacttcata gactccatca agaatgctgg aggaagggtg tttgtccact 1020
gccaggcagg catttcccgg tcagccacca tctgccttgc ttaccttatg aggactaatc 1080
gagtcaagct ggacgaggcc tttgagtttg tgaagcagag gcgaagcatc atctctccca 1140
acttcagctt catgggccag ctgctgcagt ttgagtccca ggtgctggct ccgcactgtt 1200
cggcagaggc tgggagcccc gccatggctg tgctcgaccg aggcacctcc accaccaccg 1260
tgttcaactt ccccgtctcc atccctgtcc actccacgaa cagtgcgctg agctaccttc 1320
agagccccat tacgacctct cccagctgct gaaaggccac gggaggtgag gctcttcaca 1380
tcccattggg actccatgct ccttgagagg agaaatgcaa taactctggg aggggctcga 1440
gagggctggt ccttatttat ttaacttcac ccgagttcct ctgggtttct aagcagttat 1500
ggtgatgact tagcgtcaag acatttgctg aactcagcac attcgggacc aatatatagt 1560
gggtacatca agtccatctg acaaaatggg gcagaagaga aaggactcag tgtgtgatcc 1620
ggtttctttt tgctcgcccc tgttttttgt agaatctctt catgcttgac atacctacca 1680
gtattattcc cgacgacaca tatacatatg agaatatacc ttatttattt ttgtgtaggt 1740
gtctgccttc acaaatgtca ttgtctactc ctagaagaac caaatacctc aatttttgtt 1800
tttgagtact gtactatcct gtaaatatat cttaagcagg tttgttttca gcactgatgg 1860
aaaataccag tgttgggttt ttttttagtt gccaacagtt gtatgtttgc tgattattta 1920
tgacctgaaa taatatattt cttcttctaa gaagacattt tgttacataa ggatgacttt 1980
tttatacaat ggaataaatt atggcatttc tattg 2015

<210> 21
<211> 2498
<212> DNA
<213> Homo sapiens

<400> 21

gctgagcgcc ggaggagcgt aggcagggca gcgctggcgc cagtggcgac aggagccgcg 60
cgaccggcaa aaatacacgg gaggccgtcg ccgaaaagag tccgcggtcc tctctcgtaa 120
acacactctc ctccaccggc gcctccccct ccgctctgcg cgccgcccgg ctgggcgccc 180
gaggccgctc cgactgctat gtgaccgcga ggctgcggga ggaaggggac agggaagaag 240
aggctctccc gcgggagccc ttgaggacca agtttgcggc cacttctgca ggcgtccctt 300
cttagctctc gcccgcccct ttctgcagcc taggcggccc gggttctctt ctcttcctcg 360
cgcgcccagc cgcctcggtt cccggcgacc atggtgacga tggaggagct gcgggagatg 420
gactgcagtg tgctcaaaag gctgatgaac cgggacgaga atggcggcgg cgcgggcggc 480
agcggcagcc acggcaccct ggggctgccg agcggcggca agtgcctgct gctggactgc 540
agaccgttcc tggcgcacag cgcgggctac atcctaggtt cggtcaacgt gcgctgtaac 600
accatcgtgc ggcggcgggc taagggctcc gtgagcctgg agcagatcct gcccgccgag 660

gaggaggtac gcgcccgctt gcgctccggc ctctactcgg cggtcatcgt ctacgacgag 720
cgcagcccgc gcgccgagag cctccgcgag gacagcaccg tgtcgctggt ggtgcaggcg 780
ctgcgccgca acgccgagcg caccgacatc tgcctgctca aaggcggcta tgagaggttt 840
tcctccgagt acccagaatt ctgttctaaa accaaggccc tggcagccat cccacccccg 900
gttcccccca gtgccacaga gcccttggac ctgggctgca gctcctgtgg gaccccacta 960
cacgaccagg ggggtcctgt ggagatcctt cccttcctct acctcggcag tgcctaccat 1020
gctgcccgga gagacatgct ggacgccctg ggcatcacgg ctctgttgaa tgtctcctcg 1080
gactgcccaa accactttga aggacactat cagtacaagt gcatcccagt ggaagataac 1140
cacaaggccg acatcagctc ctggttcatg gaagccatag agtacatcga tgccgtgaag 1200
gactgccgtg ggcgcgtgct ggtgcactgc caggcgggca tctcgcggtc ggccaccatc 1260
tgcctggcct acctgatgat gaagaaacgg gtgaggctgg aggaggcctt cgagttcgtt 1320
aagcagcgcc gcagcatcat ctcgcccaac ttcagcttca tggggcagct gctgcagttc 1380
gagtcccagg tgctggccac gtcctgtgct gcggaggctg ctagcccctc gggacccctg 1440
cgggagcggg gcaagacccc cgccaccccc acctcgcagt tcgtcttcag ctttccggtc 1500
tccgtgggcg tgcactcggc ccccagcagc ctgccctacc tgcacagccc catcaccacc 1560
tctcccagct gttagagccg ccctgggggc cccagaacca gagctggctc ccagcaaggg 1620
taggacgggc cgcatgcggg cagaaagttg ggactgagca gctgggagca ggcgaccgag 1680
ctccttcccc atcatttctc cttggccaac gacgaggcca gccagaatgg caataaggac 1740
tccgaataca taataaaagc aaacagaaca ctccaactta gagcaataac ggctgccgca 1800
gcagccaggg aagaccttgg tttggtttat gtgtcagttt cacttttccg atagaaattt 1860
cttacctcat ttttttaagc agtaaggctt gaagtgatga aacccacaga tcctagcaaa 1920
tgtgcccaac cagctttact aaagggggag gaagggaggg caaagggatg agaagacaag 1980
tttcccagaa gtgcctggtt ctgtgtactt gtccctttgt tgtcgttgtt gtagttaaag 2040
gaatttcatt ttttaaaaga aatcttcgaa ggtgtggttt tcatttctca gtcaccaaca 2100
gatgaataat tatgcttaat aataaagtat ttattaagac tttcttcaga gtatgaaagt 2160
acaaaaagtc tagttacagt ggatttagaa tatatttatg ttgatgtcaa acagctgagc 2220
accgtagcat gcagatgtca aggcagttag gaagtaaatg gtgtcttgta gatatgtgca 2280
aggtagcatg atgagcaact tgagtttgtt gccactgaga agcaggcggg ttgggtggga 2340
ggaggaagaa agggaagaat taggtttgaa ttgcttttta aaaaaaaaag aaaagaaaaa 2400
gacagcatct cactatgttg ccaaggctca tcttgagaag caggcgggtt gggtgggagg 2460
aggaagaaag ggaagaatta ggtttgaatt gcttttt 2498

<210> 22
<211> 2545
<212> DNA
<213> Homo sapiens

<400> 22

actcattcac ataaaacgct gcgcggccgg cggaatcccc ggcttctagg gcggcgagcg 60
gccgggctgg ctatcgagcg agcggggcgg gaacgcggag ttgcgccgcc gctcgggcgc 120
cgggctccgt cgcggccgca gccccgcggg tcgccctccc gtgcctcgcc cgcggacacc 180
ctggccgtgg acaccctggc cgtgggcacc cgcggggcgc gcggcgcggg gccgctggcc 240
ggcggcggcg gcggcatgaa ggtcacgtcg ctcgacgggc gccagctgcg caagatgctc 300
cgcaaggagg cggcggcgcg ctgcgtggtg ctcgactgcc ggccctatct ggccttcgct 360
gcctcgaacg tgcgcggctc gctcaacgtc aacctcaact cggtggtgct gcggcgggcc 420
cggggcggcg cggtgtcggc gcgctacgtg ctgcccgacg aggcggcgcg cgcgcggctc 480
ctgcaggagg gcggcggcgg cgtcgcggcc gtggtggtgc tggaccaggg cagccgccac 540
tggcagaagc tgcgagagga gagcgccgcg cgtgtcgtcc tcacctcgct actcgcttgc 600
ctacccgccg gcccgcgggt ctacttcctc aaaggggggat atgagacttt ctactcggaa 660
tatcctgagt gttgcgtgga tgtaaaaccc atttcacaag agaagattga gagtgagaga 720
gccctcatca gccagtgtgg aaaaccagtg gtaaatgtca gctacaggcc agcttatgac 780
cagggtggcc cagttgaaat ccttcccttc ctctaccttg gaagtgccta ccatgcatcc 840
aagtgcgagt tcctcgccaa cctgcacatc acagccctgc tgaatgtctc ccgacggacc 900
tccgaggcct gcgcgaccca cctacactac aaatggatcc ctgtggaaga cagccacacg 960
gctgacatta gctcccactt tcaagaagca atagacttca ttgactgtgt cagggaaaag 1020
ggaggcaagg tcctggtcca ctgtgaggct gggatctccc gttcacccac catctgcatg 1080
gcttacctta tgaagaccaa gcagttccgc ctgaaggagg ccttcgatta catcaagcag 1140
aggaggagca tggtctcgcc caactttggc ttcatgggcc agctcctgca gtacgaatct 1200
gagatcctgc cctccacgcc caaccccccag cctccctcct gccaagggga ggcagcaggc 1260
tcttcactga taggccattt gcagacactg agccctgaca tgcagggtgc ctactgcaca 1320
ttccctgcct cggtgctggc accggtgcct acccactcaa cagtctcaga gctcagcaga 1380
agccctgtgg caacggccac atcctgctaa aactgggatg gaggaatcgg cccagcccca 1440
agagcaactg tgatttttgt ttttaagact catggacatt tcatacctgt gcaatactga 1500
agacctcatt ctgtcatgct gccccagtga gatagtgagt ggtcaccagg cttgcaaatg 1560
aacttcagac ggacctcagg gtaggttctc gggactgaag gaaggccaag ccattacggg 1620

agcacagcat gtgctgacta ctgtacttcc agacccctgc cctcttggga ctgcccagtc 1680
cttgcacctc agagttcgcc ttttcatttc aagcataagg caataaatac ctgcagcaac 1740
gtgggagaaa gaagttgctg gaccaggaga aaaggcagtt atgaagccaa ttcattttga 1800
aggaagcaca atttccacct tattttttga actttggcag tttcaatgtc tgtctctgtt 1860
gcttcggggc ataagctgat caccgtctag ttgggaaagt aaccctacag ggtttgtagg 1920
gacatgatca gcatcctgat ttgaaccctg aaatgttgtg tagacaccct cttgggtcca 1980
atgaggtagt tggttgaagt agcaagatgt tggcttttct ggattttttt tgccatgggt 2040
tcttcactga ccttggactt tggcatgatt cttagtcata cttgaacttg tctcattcca 2100
cctcttctca gagcaactct tcctttggga aaagagttct tcagatcata gaccaaaaaa 2160
gtcatacctt cgaggtggta gcagtagatt ccaggaggag aagggtactt gctaggtatc 2220
ctgggtcagt ggcggtgcaa actggtttcc tcagctgcct gtccttctgt gtgcttatgt 2280
ctcttgtgac aattgttttc ctccctgccc ctggaggttg tcttcaagct gtggacttct 2340
gggatttgca gattttgcaa cgtggtacta cttttttttt ctttttgtct gttagttatt 2400
tctccagggg aaaaggcaat aattttctaa gacccgtgtg aatgtgaaga aaagcagtat 2460
gttactggtt gttgttgttg ttcttgtttt ttatagtgta aaataaaaat agtaaaagga 2520
gaaaagcaaa aaaaaaaaaa aaaaa 2545

<210> 23
<211> 2404
<212> DNA
<213> Homo sapiens

<400> 23

```
gagacgctcg ctgtttgtat ccattgagga gctgcctcgc gcagggggtg tgcgaggctg 60
agtccaagag atagcaaatc gagtcttaaa taatccgggg agaaagacgc ccgggtagat 120
ttgaggtgca gccttggagg gagggattag aagccgctag actttttttc ctcccctctc 180
agtagcacgg agtccgaatt aattggattt cattcactgg ggaggaacaa aaactatctg 240
ggcagcttca ttgagagaga ttcattgaca ctaagagcca gcggctgcag ctgggtgcag 300
agagaacctc cggctttact tctgtctcgt ctgccccaac cgctagcctc ggcttgggta 360
aggcgaggcg gaattaaacc ccgctccgag agcggcagct tcgcgcgcgg tgcgctcggc 420
ctatgcctgc cccgaggggc gtctggtagg caccccgccc tctcccgcag ctcgaccccc 480
atgatagata cgctcagacc cgtgcccttc gcgtcggaaa tggcgatcag caagacggtg 540
gcgtggctca acgagcagct ggagctgggc aacgagcggc tgctgctgat ggactgccgg 600
ccgcaggagc tatacgagtc gtcgcacatc gagtcggcca tcaacgtggc catcccgggc 660
atcatgctgc ggcgcctgca gaagggtaac ctgccggtgc gcgcgctctt cacgcgcggc 720
gaggaccggg accgcttcac ccggcgctgt ggcaccgaca cagtggtgct ctacgacgag 780
agcagcagcg actggaacga gaatacgggc ggcgagtcgg tgctcgggct gctgctcaag 840
aagctcaagg acgagggctg ccgggcgttc tacctggaag atgaagcccg gggcaagaac 900
tgtggtgtct tggtacattg cttggctggc attagccgct cagtcactgt gactgtggct 960
taccttatgc agaagctcaa tctgtcgatg aacgatgcct atgacattgt caaaatgaaa 1020
aaatccaaca tatcccctaa cttcaacttc atgggtcagc tgctggactt cgagaggacg 1080
ctgggactca gcagcccatg tgacaacagg gttccagcac agcagctgta ttttaccacc 1140
ccttccaacc agaatgtata ccaggtggac tctctgcaat ctacgtgaaa gaccccacac 1200
ccctccttgc tggaatgtgt ctggcccttc agcagtttct cttggcagca tcagctgggc 1260
tgctttcttt gtgtgtggcc ccaggtgtca aaatgacacc agctgtctgt actagacaag 1320
gttaccaagt gcggaattgg ttaatactaa cagagagatt tgctccattc tctttggaat 1380
aacaggacat gctgtataga tacaggcagt aggtttgctc tgtacccatg tgtacagcct 1440
acccatgcag ggactgggat tcgaggactt ccaggcgcat agggtagaac caaatgatag 1500
ggtaggagca tgtgttcttt agggccttgt aaggctgttt ccttttgcat ctggaactga 1560
ctatataatt gtcttcaatg aagactaatt caattttgca tatagaggag ccaaagagag 1620
atttcagctc tgtatttgtg gtatcagttt ggaaaaaaaa atctgatact ccatttgatt 1680
attgtaaata tttgatcttg aatcacttga cagtgtttgt ttgaattgtg tttgtttttt 1740
cctttgatgg gcttaaaaga aattatccaa agggagaaag agcagtatgc cacttcttaa 1800
aacagaacaa aacaaaaaaa gaaaattgtg ctcttttcta atccaaaggg tatatttgca 1860
gcatgcttga ctttaccaat tctgatgaca tctttacgga cactattatc actaagacct 1920
tgttatggcg aagtctttag tctttttcat gtattttcct catgattttt tctctttatg 1980
tagtttgact atgccttacc tttgtaaata tttttgcttg tgttgtcgca aaggggataa 2040
tctgggaaag acaccaaatc atgggctcac tttaaaaaaa gaaagaataa aaaaaccttc 2100
agctgtgcta aacagtatat tacctctgta taaaattctt cagggagtgt cacctcaaat 2160
gcaatacttt gggttggttt ctttccttta aaaaaatttg tataaaactg gaagtgtgtg 2220
tgtgtgagca tgggtaccca tttgataaga gaaatgcatt tgattgtgaa gaagggagag 2280
ttaaattctc cattatgttc gtggtgtaaa gtttagagct ggaatttatt ataagaatgt 2340
aaaaccttaa attattaata aataactatt ttggctattg aaaaaaaaaa aaaaaaaaaa 2400
aaaa                                                              2404
```

<210> 24
<211> 3346
<212> DNA
<213> Homo sapiens

<400> 24

```
gagtagacag cacagcggca gcggagggag tctatgcgag ctggacagca gtgggaggtt 60
tgtgaggctc gcactggccg cagaccctcg ggctcgatcg cccgggagcc aggactcggc 120
gacgcgaggc tgccgggcta cccggccgag gcttcggggg cgcaaactaa tgggactggc 180
tcgctcggca gcatctcccc gctcttctaa gtacactgag cagggcccgc gctgaagtag 240
aagctgtccg gggcgcgta gcccggagtc ccagtgtggc ccggaggaac ggagcccgtg 300
ccagggcggc ccagtcggga gcccggggac cgagcttgtg ctgtggggaa acccccactt 360
cttccaaggg acagcgatcc cgggacggtc gaggcgtcgg ggcggtcacc gagacctctg 420
cgggaagacc ccgtcgggga gagggcgcgc agccccgaag cgtctcggga agtcgagcgg 480
aatcgggcgg gatcacccgg gggcgcagag cccccgtcgc gcctcgtgcg gcagcggaga 540
gcccaggaga acgagccctc gggggccgaa gcccatgccc gggttggggg cggctgccca 600
gtgagtcctc ctggccggcc gggcggagaa gagcgacacc gaagccggcg ggaggggagc 660
acttcaaggc cggcggctgc ggaggatggg cgcctgagcg gctccgagcg cagcgcggca 720
gaggaaggcg aggcgagctt tggtgaggag gcgccaaggg atcccgaagt gcagtctgcc 780
cccgggaaga tggctcggcc tgggcagcgt tggctcggca agtggcttgt ggcgatggtc 840
gtgtgggcgc tgtgccggct cgcccacaccg ctggccaaga acctggagcc cgtatcctgg 900
agctccctca accccaagtt cctgagtggg aagggcttgg tgatctatcc gaaaattgga 960
gacaagctgg acatcatctg cccccgagca gaagcagggc ggccctatga gtactacaag 1020
ctgtacctgg tgcggcctga gcaggcagct gcctgtagca cagttctcga ccccaacgtg 1080
ttggtcacct gcaataggcc agagcaggaa atacgcttta ccatcaagtt ccaggagttc 1140
agccccaact acatgggcct ggagttcaag aagcaccatg attactacat tacctcaaca 1200
tccaatggaa gcctggaggg gctggaaaac cgggagggcg gtgtgtgccg cacacgcacc 1260
atgaagatca tcatgaaggt tgggcaagat cccaatgctg tgacgcctga gcagctgact 1320
accagcaggc ccagcaagga ggcagacaac actgtcaaga tggccacaca ggcccctggt 1380
agtcggggct ccctgggtga ctctgatggc aagcatgaga ctgtgaacca ggaagagaag 1440
agtggcccag gtgcaagtgg gggcagcagc ggggaccctg atggcttctt caactccaag 1500
gtggcattgt tcgcggctgt cggtgccggt tgcgtcatct tcctgctcat catcatcttc 1560
ctgacggtcc tactactgaa gctacgcaag cggcaccgca agcacacaca gcagcgggcg 1620
gctgccctct cgctcagtac cctggccagt cccaaggggg gcagtggcac agcgggcacc 1680
gagcccagcg acatcatcat tcccttacgg actacagaga acaactactg cccccactat 1740
gagaaggtga gtggggacta cgggcaccct gtctacatcg tccaagagat gccgccccag 1800
agcccggcga acatctacta caaggtctga gtgcccggca cggcctcagg cccccgaggg 1860
acagtcggcc tggaccggac ctctcctttc gcccccacac cccctcccct tgccagctgt 1920
gcccaccttt gtatttagtt ttgtagtttc ttggctttta taatccccct ttttccctgc 1980
cccctgggct tcggaggggg gtgcttgtgc ccctaacccc catgctcttg tgccttcccc 2040
ctctggccag gcctctgggc tccgtggggg cgccccttct tggaaggcag ggctggacac 2100
tgatggacag caggcaggga gacagtcccc tggccctgcc cctccctcgc ccccttgcc 2160
accttcccag gactgcttgt ccgctatcat cactgttttt aatgcttttg tgttcatttt 2220
ttagctgtca actcattttc atctgttttt tgaagaaaaa tggaaaaatg taaaaggcag 2280
cccctcccca ggctttgtga gcctggccca agccagtaca agagggcctg ggcacgatg 2340
tggtcagcca ggaagcatag gatgccattt cttttataga ttccttggta tttctggtgg 2400
ggtaaggggc aggccagggc tgttcacgcc catgagggaa gaggaaagtg ccactgggca 2460
aggtgtccca ccctcccctc ctgaccctcc tacgaggctt atcctggcaa tggggtagtc 2520
actgccaccc ttccacacac acacacacac acacacacac aaaaaaaaat cccttccttg 2580
tgggattctt gggcatctcc tgcctccctc actctcacgg taattaatgt cttaattggc 2640
tgttgcctgg ggaacaggag agctgctgca ggcagatgac ctcatggggg gtggagggag 2700
gtgaggtgcc caggtggcta tttgccctgc agagctggga gtttcacccc caccccccac 2760
cctgttctct ccttaccttt ggcatccttt ggcctggtgg ggaaacagag gcccagggtg 2820
gagacctaag cgggtataag accaggtggc ctgctccttt tctgggccct agcacaggtg 2880
ggtaaccccc acccaaccca gctcctgctg ctgtcccagt cttgggctgg ggcctggaaa 2940
gaggaagagg ctgcctgggg ctgggccagc ccgctgtgca ctttgacccc agttccttgc 3000
cagcacggct gctaacagac tgccacttga gtgcgccttg caggcactcc cagagcagcc 3060
atggaaggag ctggccctca caccatccac ctccacactg cctcctggcc agctgcccac 3120
cccagtgcca ggtgggagag ggagcagaac agccagcccc ttccaggtgg cagtcggaag 3180
ggtttttgtt tttgtttctg ttgccatttg tgtaaatact agtctttttg gaaaaaaaat 3240
aatgtaaaga tgttttgtat aaactctgaa ttattttctt gttgcttttt tcttagaaaa 3300
aaatgagaac taaaaaaaaa aaattaacca catggaaaaa aaaaaa                3346
```

<210> 25
<211> 3136
<212> DNA
<213> Homo sapiens

<400> 25

```
gcgcagaact tggggagccg ccgccgccat ccgccgccgc agccagcttc cgccgccgca 60
ggaccggccc ctgccccagc ctccgcagcc gcggcgcgtc cacgcccgcc cgcgcccagg 120
gcgagtcggg gtcgccgcct gcacgcttct cagtgttccc cgcgccccgc atgtaacccg 180
gccaggcccc cgcaactgtg tcccctgcag ctccagcccc gggctgcacc cccccgcccc 240
gacaccagct ctccagcctg ctcgtccagg atggccgcgg ccaaggccga gatgcagctg 300
atgtccccgc tgcagatctc tgacccgttc ggatcctttc ctcactcgcc caccatggac 360
aactacccta agctggagga gatgatgctg ctgagcaacg gggctcccca gttcctcggc 420
gccgccgggg ccccagaggg cagcggcagc aacagcagca gcagcagcag cgggggcggt 480
ggaggcggcg gggcggcag caacagcagc agcagcagca gcaccttcaa ccctcaggcg 540
gacacgggcg agcagcccta cgagcacctg accgcagagt cttttcctga catctctctg 600
aacaacgaga aggtgctggt ggagaccagt taccccagcc aaaccactcg actgcccccc 660
atcacctata ctggccgctt ttccctggag cctgcaccca acagtggcaa caccttgtgg 720
cccgagcccc tcttcagctt ggtcagtggc ctagtgagca tgaccaaccc accggcctcc 780
tcgtcctcag caccatctcc agcggcctcc tccgcctccg cctcccagag cccacccctg 840
agctgcgcag tgccatccaa cgacagcagt cccatttact cagcggcacc caccttcccc 900
acgccgaaca ctgacatttt ccctgagcca caaagccagg ccttcccggg ctcggcaggg 960
acagcgctcc agtacccgcc tcctgcctac cctgccgcca agggtggctt ccaggttccc 1020
atgatccccg actacctgtt tccacagcag caggggggatc tgggcctggg caccccagac 1080
cagaagccct tccagggcct ggagagccgc acccagcagc cttcgctaac ccctctgtct 1140
actattaagg cctttgccac tcagtcgggc tcccaggacc tgaaggccct caataccagc 1200
taccagtccc agctcatcaa acccagccgc atgcgcaagt accccaaccg gcccagcaag 1260
acgccccccc acgaacgccc ttacgcttgc ccagtggagt cctgtgatcg ccgcttctcc 1320
cgctccgacg agctcacccg ccacatccgc atccacacag gccagaagcc cttccagtgc 1380
cgcatctgca tgcgcaactt cagccgcagc gaccacctca ccacccacat ccgcacccac 1440
acaggcgaaa agcccttcgc ctgcgacatc tgtggaagaa agtttgccag gagcgatgaa 1500
cgcaagaggc ataccaagat ccacttgcgg cagaaggaca agaaagcaga caaaagtgtt 1560
gtggcctctt cggccacctc ctctctctct tcctacccgt ccccggttgc tacctcttac 1620
ccgtccccgg ttactacctc ttatccatcc ccggccacca cctcataccc atcccctgtg 1680
cccacctcct tctcctctcc cggctcctcg acctacccat cccctgtgca cagtggcttc 1740
ccctccccgt cggtggccac cacgtactcc tctgttcccc ctgctttccc ggcccaggtc 1800
agcagcttcc cttcctcagc tgtcaccaac tccttcagcg cctccacagg gctttcggac 1860
atgacagcaa ccttttctcc caggacaatt gaaatttgct aaagggaaag gggaaagaaa 1920
gggaaaaggg agaaaaagaa acacaagaga cttaaaggac aggaggagga gatggccata 1980
ggagaggagg gttcctctta ggtcagatgg aggttctcag agccaagtcc tccctctcta 2040
ctggagtgga aggtctattg gccaacaatc ctttctgccc acttcccctt ccccaattac 2100
tattcccttt gacttcagct gcctgaaaca gccatgtcca agttcttcac ctctatccaa 2160
agaacttgat ttgcatggat tttggataaa tcatttcagt atcatctcca tcatatgcct 2220
gacccccttgc tcccttcaat gctagaaaat cgagttggca aatggggtt tgggcccctc 2280
agagccctgc cctgcaccct tgtacagtgt ctgtgccatg gatttcgttt ttcttggggt 2340
actcttgatg tgaagataat ttgcatattc tattgtatta tttggagtta ggtcctcact 2400
tgggggaaaa aaaaaaaaga aaagccaagc aaaccaatgg tgatcctcta ttttgtgatg 2460
atgctgtgac aataagtttg aaccttttttt tttgaaacag cagtcccagt attctcagag 2520
catgtgtcag agtgttgttc cgttaacctt tttgtaaata ctgcttgacc gtactctcac 2580
atgtggcaaa atatggtttg gtttttcttt tttttttttt ttgaaagtgt tttttcttcg 2640
tcctttttggt ttaaaaagtt tcacgtcttg gtgccttttg tgtgatgcgc cttgctgatg 2700
gcttgacatg tgcaattgtg agggacatgc tcacctctag ccttaagggg ggcagggagt 2760
gatgatttgg gggaggcttt gggagcaaaa taaggaagag ggctgagctg agcttcggtt 2820
ctccagaatg taagaaaaca aaatctaaaa caaaatctga actctcaaaa gtctattttt 2880
ttaactgaaa atgtaaattt ataaatatat tcaggagttg gaatgttgta gttacctact 2940
gagtaggcgg cgatttttgt atgttatgaa catgcagttc attattttgt ggttctattt 3000
tactttgtac ttgtgtttgc ttaaacaaag tgactgtttg gcttataaac acattgaatg 3060
cgctttattg cccatgggat atgtggtgta tatccttcca aaaaattaaa acgaaaataa 3120
agtagctgcg attggg 3136
```

<210> 26
<211> 3482
<212> DNA
<213> Homo sapiens

<400> 26

**ccctaccgcc cccaattccg ccctgccccc gccgcggcgg cgctagccgc cactgaggga 60**

```
ccgaccctat aaaggccgct ccgcgagggg tgcgcagcat tcggcagagg gcgcttcgac 120
gggctgggct gtgcgcctgc gcagtgtggg tcgctcccga ttccctgccc cggccggccc 180
cgcctcggct ccgcaccctc gccccgctct cagccgccgc tctgccccgc agcagccagc 240
cccgtgtccg gcagtatgtt cagctgggtc agcaaggatg cccgccgcaa gaaggagccg 300
gagctcttcc agacggtggc tgaggggctg cggcagctgt acgcgcagaa gctgctaccc 360
ctggaggagc actaccgctt ccacgagttc cactcgcccg cgctggagga cgctgacttc 420
gacaacaagc ctatggtgct cctcgtgggg cagtacagca cgggcaagac caccttcatc 480
cgacacctga tcgagcagga cttcccgggg atgcgcatcg ggcccgagcc caccaccgac 540
tccttcatcg ccgtcatgca cggccccact gagggcgtgg tgccgggcaa cgcgctcgtg 600
gtggacccgc ggcgcccctt ccgcaagctc aacgcgtttg gcaacgcttt cctcaacagg 660
ttcatgtgtg cccagctgcc caaccccgtc ctggacagca tcagcatcat cgacaccccc 720
gggatcctgt ctggagagaa gcagcggatc agcagaggct atgactttgc agccgtcctg 780
gagtggttcg cggagcgtgt ggaccgcatc atcctgctct tcgacgccca caagctggac 840
atctccgatg agttctcgga agtgatcaag gctctgaaga accatgagga caagatccgc 900
gtggtgctga acaaggcaga ccagatcgag acgcagcagc tgatgcgggt gtacggggcc 960
ctcatgtggt ccctgggcaa gatcatcaac acccccgagg tggtcagggt ctacatcggc 1020
tccttctggt cccacccgct cctcatcccc gacaaccgca agctctttga ggccgaggag 1080
caggacctct tcaaggacat ccagtcactg ccccgaaacg ccgccctcag gaagctcaat 1140
gacctgatca agcgggcacg gctggccaag gttcacgcct acatcatcag ctccctcaag 1200
aaagagatgc ccaatgtctt tggtaaagag agcaaaaaga aagagctggt gaacaacctg 1260
ggagagatct accagaagat tgagcgcgag caccagatct cccctgggga cttcccgagc 1320
ctccgcaaga tgcaggaact cctgcagacc caggacttca gcaagttcca ggcgctgaag 1380
cccaagctgc tggacacggt ggatgacatg ctggccaacg acatcgcgcg gctgatggtg 1440
atggtgcggc aggaggagtc cctgatgcct tcccaggtgg tcaagggcgg cgcctttgac 1500
ggcaccatga acgggccgtt cgggcacggc tacggcgagg gggccggcga gggcatcgac 1560
gacgtggagt gggtggtggg caaggacaag cccacctacg acgagatctt ctacacgctg 1620
tcccctgtca acggcaagat cacgggcgcc aacgccaaga aggagatggt gaagtccaag 1680
ctccccaaca ccgtgctagg gaagatctgg aagctggccg acgtggacaa ggacgggctg 1740
ctggacgacg aggagttcgc gctggccaac cacctcatca aggtcaagct ggagggccac 1800
gagctgcccg ccgacctgcc cccgcacctg gtgccgccct ccaagcgcag acatgagtga 1860
tggcgcccgg ccccgcacct gccatttgca cgcccggccg ggaggcagag acggggggag 1920
gggaagcctc accatttctc aaggtccata aagactgagc ggatgtttcc tcgcctctcg 1980
aaaaggaaaa ccaccatctt tcttttaagg ctgttcctgg gcctggcggg ggaggcaggg 2040
gtgagaggat ggaattgtgt gcacaagaac tgtggctatt ttaatatata acgttagagg 2100
ctgcgttctt tgtcgccgcc tcccctgtgt gccagccctg tgtgcacggc ctctgccccc 2160
cggcctttgc tgtggctgga gctggacagt gcagcgactg cgaccgtggg ggagccaggt 2220
cgccctttttg gcagctgcta ggctgaggct gcatggacag gaacaccagg caccctccgt 2280
gtgcttctga gctgaggttg cttcacggga ccgtggcttc cttcctcacc tggctctgcc 2340
tccccgtgc tctcggggcga agtgggttct tgtgccttcc cctcccgggc ccaggctccc 2400
cgtgcgcggg ccctgccctt tcctcccgcg ccccaccggc tccgacgcgc aaccccgctc 2460
agcagtcaca gaagcagggc ccagccacct tggtcttttt ttgggagttc aggggagtag 2520
gagaatgtct tccagaaaaa tacataagct agtttctgtt ctgtaaagtg atatctttca 2580
tacttgacca aagttcccaa taacttccca accactgttc aaaagctgtg atttttgtct 2640
ccccttccca ccctccagcc aaggagcagc cctgcccagg gggcattagg tgtgggtacc 2700
cggggagcac cccgttcctg gaccccagtg ttgcatttcc tggctgagga agggtggtca 2760
tcccagctcc tgccctaccc tctcacttaa ctggagcttt gggacgcacc ctccacagtg 2820
ggaggtggtg gtgggtggcg gtggcggggc ctcacgacag cttggtgctg gtaagaggaa 2880
gcccgtggtt ctggctaggc tctcatgtcc agacagcggg gaccagggga aaacccagcc 2940
ccttctgtaa tccccccttca tttcctacct tccttcctcc tctgtttagc aaaggagggc 3000
agctcacttg gatgtcctta caacgcccct ggcccccagg ttgagcaata agaaaccaga 3060
accttgcggc ccagtggccc gggccagttc aggccgcctc cccctcctct gcctggggcc 3120
attgagccca gcctccaggg cccgggcgcg tttgcaggcc agtggccact gtccgggctg 3180
tgatggcacc aaggcaggtg gagcaccagg taccacacag ctgggcttcc caccaggctt 3240
tcccgcgggg gtctcaggga gcttctcccc agcgctgctc ggagtctgca ggaactggcc 3300
ttgttctcct tagcccgtca ctccatacag tattaggtga ggatggatgc gggcgctgtc 3360
cttgccggga agtcactgtt gaagttgcag tggcttgttc acacctgtgg gaagagaagt 3420
gaagactttc tccttgcatt aaaaagtctg aactgtgaaa aaaaaaaaaa aaaaaaaaaa 3480
aa                                                                3482
```

<210> 27
<211> 2180

<212> DNA
<213> Homo sapiens

<400> 27

```
gggcggaaaa gcctgtttac acagactgca caccgcctgg ggaataatgc agtaaaggaa 60
gtgagccggc tcggcctgac tgctccaact tcctgctctc acacacacca gaggggaaaa 120
aaaaagagga gcgagagaaa gaaaaaaagg gggaaaaatc aggatctcat tacaagagcc 180
acagaccgtc tgcagacgcc tgtcagcatg gaaagtcggg ggctttcgcc cgggtcctcc 240
tagaaattcc ccccgaagaa gactccccca catctgggta tggagagtgc aatcacgctg 300
tggcagttcc tgttgcagtt gctgctggat cagaaacatg agcatttgat ctgctggacc 360
tcgaacgatg gtgaattcaa gctcctcaaa gcagaagaag tggccaagct gtggggactc 420
cgaaaaaaca aaacaaatat gaactatgat aagctgagca gagccctgcg atactattat 480
gacaagaaca tcatcaagaa ggtgatcggg cagaagtttg tgtacaagtt tgtctctttc 540
ccggagatcc tgaagatgga tcctcacgcg gtggagatca gccgggagag ccttctgctg 600
caggacagcg actgcaaggc gtctccggag ggccgcgagg cccacaaaca cggcctggcc 660
gccctcagaa gcacgagccg caacgaatac atccactcag gcctgtactc gtccttcacc 720
attaattccc tgcagaaccc accagacgcc ttcaaggcca tcaagacgga gaagctggag 780
gagccgcccg aagacagccc ccccgtggaa gaagtcagga ctgtgatcag gtttgtgacc 840
aataaaaccg acaagcacgt caccaggccg gtggtgtccc tgccttccac gtcagaggct 900
gcggcggcgt ccgccttcct ggcctcgtcc gtctcggcca agatctcctc tttaatgttg 960
ccaaacgctg ccagtatttc atccgcctca cccttctcat ctcggtcccc gtccctgtcc 1020
cccaactcac ccctcccttc tgaacacaga agcctcttcc tggaggccgc ctgccatgac 1080
tccgattccc tggagccctt gaacctgtca tcgggctcca agaccaagtc tccatctctt 1140
cccccaaagg ccaaaaaacc caaaggcttg gaaatctcag cgcccccgct ggtgctctcc 1200
ggcaccgaca tcggctccat cgccctcaac agcccagccc tcccctcggg atccctcacc 1260
ccagccttct tcaccgcaca gacaccaaat ggattgcttc tgactccgag tccactgctc 1320
tccagcatac atttctggag cagccttagt ccagttgctc cgctgagtcc tgccaggctg 1380
caagggccaa gcacgctgtt ccagttcccc acactgctta atggccacat gccagtgcca 1440
atccccagtc tggacagagc tgcttctcca gtactgcttt cttcaaactc tcagaaatcc 1500
tgatgacgtc tggccacaat taaggactca ttaactgatg aaacaaattt gtccccacgg 1560
gctagtttac ctgtgtcgtg agaaggacat tgtgaaactc ttgttaattt ggtttgcact 1620
tttcataaca tggatagtct agatttatgt tagcatttta aaaactgttt ttgatatatt 1680
caagtatata tgaaaatctg tttggcatta agtgaatttt aatgtttttg tttttatatc 1740
cttttagctc ttaagtgttg aacactgttg acagtgaaga acttttctta atggttttca 1800
gtataactaa taaggatgtg aagctttttt ctctttagtt ctgagtatgc taaactgtgt 1860
gcttatatag actataacca gttgtgcctt cctttgcatt taatgtaaat gaatgattta 1920
tatatttttt agtattaaga ggaaatgttt gaaagatgaa aattagtatc aaacagctct 1980
ctagtagaat ttcattattt ttcaccagtg ggcaatatga aagcatatat cacgttttgt 2040
tttactttca attgtataag aattgcctta gaacctcttt tgaactgaaa ttcagtaaat 2100
gtccaagtaa tgtttttata ataaactaag ccatatttag acaataaaca tcgaaaaaaa 2160
aaaaaaaaaa aaaaaaaaaa                                             2180
```

<210> 28
<211> 4628
<212> DNA
<213> Homo sapiens

<400> 28

```
tcacttgcct gatatttcca gtgtcagagg gacacagcca acgtggggtc ccttctaggc 60
tgacagccgc tctccagcca ctgccgcgag cccgtctgct cccgccctgc ccgtgcactc 120
tccgcagccg ccctccgcca agccccagcg cccgctccca tcgccgatga ccgcggggag 180
gaggatggag atgctctgtg ccggcagggt ccctgcgctg ctgctctgcc tgggtttcca 240
tcttctacag gcagtcctca gtacaactgt gattccatca tgtatcccag gagagtccag 300
tgataactgc acagctttag ttcagacaga agacaatcca cgtgtggctc aagtgtcaat 360
aacaaagtgt agctctgaca tgaatggcta ttgtttgcat ggacagtgca tctatctggt 420
ggacatgagt caaaactact gcaggtgtga agtgggttat actggtgtcc gatgtgaaca 480
cttcttttta accgtccacc aacctttaag caaagaatat gtggctttga ccgtgattct 540
tattattttg tttcttatca cagtcgtcgg ttccacatat tatttctgca gatggtacag 600
aaatcgaaaa agtaaagaac caaagaagga atatgagaga gttacctcag gggatccaga 660
gttgccgcaa gtctgaatgg cgccatcaaa cttatgggca gggataacag tgtgcctggt 720
taatattaat attcccattt tattaataat atttatgttg ggtcaagtgt taggtcaata 780
acactgtatt ttaatgtact tgaaaaatgt ttttattttt gttttatttt tgacagacta 840
tttgctaatg tataatgtgc agaaaatatt taatatcaaa agaaaattga tatttttata 900
caagtaattt cctgagctaa atgcttcatt gaaagcttca aagtttatat gcctggtgca 960
cagtgcttag aagtaagcaa ttcccaggtc atagctcaag aattgttagc aaatgacaga 1020
tttctgtaag cctatatata tagtcaaatc gatttagtaa gtatgttttt tatgttcctc 1080
aaatcagtga taattggttt gactgtacca tggtttgata tgtagttggc accatggtat 1140
catatattaa aacaataatg caattagaat ttgggagaag caaatatagg tcctgtgtta 1200
aacactacac atttgaaaca agctaaccct ggggagtcta tggtctcttc actcaggtct 1260
```

```
cagctataat tctgttatat gaggggcagt ggacagttcc ctatgccaac tcacgactcc 1320
tacaggtact agtcactcat ctaccagatt ctgcctatgt aaaatgaatt gaaaaacaat 1380
tttctgtaat cttttattta agtagtgggc atttcatagc ttcacaatgt tcctttttttg 1440
tatattacaa catttatgtg aggtaattat tgctcaacag acaattagaa aaaagtccac 1500
acttgaagcc taaatttgtg ctttttaaga atatttttag actatttctt tttatagggg 1560
ctttgctgaa ttctaacatt aaatcacagc ccaaaatttg atggactaat tattatttta 1620
aaatatatga agacaataat tctacatgtt gtcttaagat ggaaatacag ttatttcatc 1680
ttttattcaa ggaagtttta actttaatac agctcagtaa atggcttctt ctagaatgta 1740
aagttatgta tttaaagttg tatcttgaca caggaaatgg gaaaaaactt aaaaattaat 1800
atggtgtatt tttccaaatg aaaaatctca attgaaagct tttaaaatgt agaaacttaa 1860
acacaccttc ctgtggaggc tgagatgaaa actagggctc attttcctga catttgttta 1920
ttttttggaa gagacaaaga tttcttctgc actctgagcc cataggtctc agagagttaa 1980
taggagtatt tttgggctat tgcataagga gccactgctg ccaccacttt tggattttat 2040
gggaggctcc ttcatcgaat gctaaacctt tgagtagagt ctccctggat cacataccag 2100
gtcagggagg atctgttctt cctctacgtt tatcctggca tgtgctaggg taaacgaagg 2160
cataataagc catggctgac ctctggagca ccaggtgcca ggacttgtct ccatgtgtat 2220
ccatgcatta tataccctgg tgcaatcaca cgactgtcat ctaaagtcct ggccctggcc 2280
cttactatta ggaaaataaa cagacaaaaa caagtaaata tatatggtca tatacatatt 2340
gtatatatat tcatatacaa acatgtatgt atacatgacc ttaatggatc atagaattgc 2400
agtcatttgg tgctctgcta accatttata taaaacttaa aaacaagaga aaagaaaaat 2460
caattagatc taaacagtta tttctgtttc ctatttaata cagctgaagt caaaatatgt 2520
aagaacacat tttaaatact ctacttacag ttggccctct gtggttagtt ccacatctgt 2580
ggattcaacc aaccaaggac ggaaaatgct taaaaaataa tacaacaaca acaaaaaata 2640
cattataaca actatttact tttttttttt tctttttgag atggagtctc gctctgttgc 2700
ccaggttgga gtgcagtggc acgatctcgg ctcactgcaa cctcacctcc cgggttcaag 2760
agatcctcct gcctcagcct cctgagcagc tgggactaca ggcgcatgcc accatgccca 2820
gctaattttt gtatttttag tagaggcggg gtttcaccat gttggccagg atggtctcaa 2880
tctcctaacc ttgagatcca ccctccacag cctcccaaac tgctgggatt acaggtgtga 2940
gccaccgcac gtagcattta cattaggtat tacaagtaat gtaaagatga tttaagtata 3000
caggaggatg tgaataggtt atatgcaagc actatgccct tttatataag tgacttgaac 3060
atctgtgccc gattttagta tgtgcagggg ggcgatctgg gaatcagtcc cctgtggata 3120
ccaaggtaca actgtattta ttaacgctta ctagatgtga ggagagtctg aatattttca 3180
gtgatcttgg ctgtttcaaa aaaatctatt gacttttcaa taaatcagct gcaatccatt 3240
tatttcattt acaaaagatt tattgtaagc atctcaatct tggtttgtca gtttatctta 3300
agcatgtcaa ttcataaaaa caagtcattt ttgtattttt catctttaag aatgcttaaa 3360
aaagctaatc cctaaaatag ttagatcttt gtaaatgcat attaaataat aaagtatgac 3420
ccacattact ttttatgggt gaaaataaga caaaaataat agttttagtg aggatggtgc 3480
tgagtaaaca taaaaactga tttgctctca gctgatgtgt cctgtacaca gtgggaagat 3540
tttagttcac acttagtcta actcccccat tttacagatt tctcactata tatatttcta 3600
gaagggggcta tgcatattca atgtattgag aaccaaagca accacaaatg cataaatgca 3660
taatttatgg tcttcaacca aggccacata ataacccagt taacttactc tttaaccagg 3720
aatattaagt tctataacta gtactcaagg tttaacctta aaattaagat ttccttaacc 3780
ttaaccttaa aattgatatt atattaaaca tacataatac aatgtaactc cactgttctc 3840
ctgaatattt tttgctctaa tctctctgcc gaaagtcaaa gtgatgggag aattggtata 3900
ctggtatgac tacgtcttaa gtcagatttt tatttatgag tctttgagac taaattcaat 3960
caccaccagg tatcaaatca acttttatgc agcaaatata tgattctagt gtctgacttt 4020
tgttaaattc agtaatgcag tttttaaaaa cctgtatctg acccactttg taatttttgc 4080
tccaatatcc attctgtaga cttttgaaaa aaaagttttt aatttgatgc ccaatatatt 4140
ctgaccgtta aaaaattctt gttcatatgg gagaaggggg agtaatgact tgtacaaaca 4200
gtatttctgg tgtatatttt aatgttttta aaaagagtaa tttcatttaa atatctgtta 4260
ttcaaatttg atgatgttaa atgtaatata atgtattttc tttttatttt gcactctgta 4320
attgcacttt ttaagtttga agagccattt tggtaaacgg tttttattaa agatgctatg 4380
gaacataaag ttgtattgca tgcaatttga agtaacttat ttgactatga atgttatcgg 4440
attactgaat tgtatcaatt tgtttgtgtt caatatcagc tttgataatt gtgtacctta 4500
agatattgaa ggagaaaata gataatttac aagatattat taatttttat ttatttttct 4560
tgggaattga aaaaaattga aataaataaa aatgcattga acatcttgca ttcaaaatct 4620
tcactgac                                                          4628
```

<210> 29
<211> 2084
<212> DNA

<213> Homo sapiens

<400> 29

aaccgcatct gcagcgagca actgagaagc caagactgag ccggcggccg cggcgcagcg 60

aacgagcagt gaccgtgctc ctacccagct ctgcttcaca gcgcccacct gtctccgccc 120
ctcggcccct cgcccggctt tgcctaaccg ccacgatgat gttctcgggc ttcaacgcag 180
actacgaggc gtcatcctcc cgctgcagca gcgcgtcccc ggccggggat agcctctctt 240
actaccactc acccgcagac tccttctcca gcatgggctc gcctgtcaac gcgcaggact 300
tctgcacgga cctggccgtc tccagtgcca acttcattcc cacggtcact gccatctcga 360
ccagtccgga cctgcagtgg ctggtgcagc ccgccctcgt ctcctctgtg gccccatcgc 420
agaccagagc ccctcaccct ttcggagtcc ccgccccctc cgctggggct tactccaggg 480
ctggcgttgt gaagaccatg acaggaggcc gagcgcagag cattggcagg aggggcaagg 540
tggaacagtt atctccagaa gaagaagaga aaaggagaat ccgaagggaa aggaataaga 600
tggctgcagc caaatgccgc aaccggagga gggagctgac tgatacactc caagcggaga 660
cagaccaact agaagatgag aagtctgctt tgcagaccga gattgccaac ctgctgaagg 720
agaaggaaaa actagagttc atcctggcag ctcaccgacc tgcctgcaag atccctgatg 780
acctgggctt cccagaagag atgtctgtgg cttcccttga tctgactggg ggcctgccag 840
aggttgccac ccccggagtct gaggaggcct tcaccctgcc tctcctcaat gaccctgagc 900
ccaagccctc agtggaacct gtcaagagca tcagcagcat ggagctgaag accgagccct 960
ttgatgactt cctgttccca gcatcatcca ggcccagtgg ctctgagaca gcccgctccg 1020
tgccagacat ggacctatct gggtccttct atgcagcaga ctgggagcct ctgcacagtg 1080
gctccctggg gatggggccc atggccacag agctggagcc cctgtgcact ccggtggtca 1140
cctgtactcc cagctgcact gcttacacgt cttccttcgt cttcacctac cccgaggctg 1200
actccttccc cagctgtgca gctgcccacc gcaagggcag cagcagcaat gagccttcct 1260
ctgactcgct cagctcaccc acgctgctgg ccctgtgagg gggcagggaa ggggaggcag 1320
ccggcaccca caagtgccac tgcccgagct ggtgcattac agagaggaga aacacatctt 1380
ccctagaggg ttcctgtaga cctagggagg accttatctg tgcgtgaaac acaccaggct 1440
gtgggcctca aggacttgaa agcatccatg tgtggactca agtccttacc tcttccggag 1500
atgtagcaaa acgcatggag tgtgtattgt tcccagtgac acttcagaga gctggtagtt 1560
agtagcatgt tgagccaggc ctgggtctgt gtctcttttc tctttctcct tagtcttctc 1620
atagcattaa ctaatctatt gggttcatta ttggaattaa cctggtgctg gatattttca 1680
aattgtatct agtgcagctg attttaacaa taactactgt gttcctggca atagtgtgtt 1740
ctgattagaa atgaccaata ttatactaag aaaagatacg actttatttt ctggtagata 1800
gaaataaata gctatatcca tgtactgtag tttttcttca acatcaatgt tcattgtaat 1860
gttactgatc atgcattgtt gaggtggtct gaatgttctg acattaacag ttttccatga 1920
aaacgtttta ttgtgttttt aatttattta ttaagatgga ttctcagata tttatatttt 1980
tattttattt ttttctacct tgaggtcttt tgacatgtgg aaagtgaatt tgaatgaaaa 2040
atttaagcat tgtttgctta ttgttccaag acattgtcaa taaa 2084

<210> 30
<211> 2338
<212> DNA
<213> Homo sapiens

<400> 30

```
ctcaacgtcg aaccgaaggc gacacccacc caactcctcc ctctccgccc catagtccac 60
ccaacacttg cagcccctcc agagaaaaag cccagcggaa gaggactccg gaaaagtgac 120
tatctcggaa gaccagggta gagctcccgg gagaaaaagg cgaagatccc cggccccgga 180
tcgccatccc cttccctggc accagcttct ctaggctgcg cgaaggaaga gggtacgacg 240
ccggggaggg actgggtgcg cgggcatgaa gttggaggtg ttcgtccctc gcgcggccca 300
cggggacaag cagggcagtg acctggaggg cgcgggcggc agcgacgcgc cgtccccgct 360
gtcggcggcg ggagacgact ccctgggctc agatggggac tgcgcggcca acagcccggc 420
cgcgggcggc ggcgccagag atacgcaggg cgacggcgaa cagagtgcgg gaggcgggcc 480
gggcgcggag gaggcgatcc cggcagcagc tgctgcagcg gtggtggcgg agggcgcgga 540
ggccgggcg gcggggccag gcgcgggcgg cgcggggagc ggcgagggtg cacgcagcaa 600
gccatatacg cggcggccca agcccccta ctcgtacatc gcgctcatcg ccatggccat 660
ccgcgactcg gcgggcgggc gcttgacgct ggcggagatc aacgagtacc tcatgggcaa 720
gttcccctt ttccgcggca gctacacggg ctggcgcaac tccgtgcgcc acaacctttc 780
gctcaacgac tgcttcgtca aggtgctgcg cgacccctcg cggccctggg gcaaggacaa 840
ctactggatg ctcaacccca acagcgagta caccttcgcc gacggggtct tccgccgccg 900
ccgcaagcgc ctcagccacc gcgcgccggt ccccgcgccc gggctgcggc ccgaggaggc 960
cccgggcctc cccgccgccc cgccgcccgc gcccgccgcc ccggcctcgc cccgcatgcg 1020
ctcgcccgcc cgccaggagg agcgcgccag ccccgcgggc aagttctcca gctccttcgc 1080
catcgacagc atcctgcgca agcccttccg cagccgccgc ctcagggaca cggcccccgg 1140
gacgacgctt cagtggggcg ccgcgccctg cccgccgctg cccgcgttcc ccgcgctcct 1200
ccccgcggcg ccctgcaggg ccctgctgcc gctctgcgcg tacggcgcgg gcgagccggc 1260
gcggctgggc gcgcgcgagg ccgaggtgcc accgaccgcg ccgcccctcc tgcttgcacc 1320
tctcccggcg gcggcccccg ccaagccact ccgaggcccg gcggccggcg gcgcgcacct 1380
gtactgcccc ctgcggctgc ccgcagccct gcaggcggcc tcagtccgcc gccctggccc 1440

gcacctgccg tacccggtgg agacgctcct agcctgagta gcgccgcggg gcccgggaac 1500
gccgagtgcg cgccgtcggg gaggcgggaa cgcgggcccg cgcgcggact ttgcactttg 1560
aatccagagg aagatgaatt tttaaaatct ccatcaaacg tgccttaaag ctaaagcatt 1620
ttgacaggag tatttaaact tagtccagga tattttcctt tgtgttttat aactttacag 1680
aggaccaaag caattcttgt tttagtttct ttgcgaagcc tgctcctcct tcccttcata 1740
aggacttttt ttgtctttct ttaacgccca ggcttcgtct tatttctact gtttttgtcg 1800
caacttccat tgatttatgt cccttccctc cccctaagt acatcaggga acctttccac 1860
actataaatg atatgactac tgtttggggt ttctgggccc ccatccgtgt acgtatgtgg 1920
catttccagg tatgactgag tgtgagagac atgtcagagg ctcttcagtg atttcttgct 1980
attgaccgat gcttcactgt gccaaaagag aaaaaaaatg ttgggttttg taattaaatt 2040
atttatatat ttttgaaacc cgaattgaaa atgttgcagg caacgggcta cagctttatt 2100
agtggttctc taactgtggt ctccttgggc caagcaattt ctttaaagga aaagttgatt 2160
atgtatgtgg ggtgccagga ccactgcctt gaaagcaagt gtgattttta tttttaatat 2220
tattttattt gtgtctgtgt acatattcat gtataaattt tatgaaaccc aagcatagtg 2280
cttatttttt aataaaacaa ctgacttaac acattgtgaa aaaaaaaaaa aaaaaaa   2338
```

<210> 31
<211> 879
<212> DNA
<213> Homo sapiens

<400> 31

gcggagtctc caactgggag agctgcagct gccgagagga ggagaacgct gaggtcggtc 60
ggaccaacgg acgcgctgac cgctgccaac tgcagctcgc gctgcctcct gctcgcgccg 120
tgccactaag gtcactcccg cctccgagag cccagagccg agatggaaac ggtccaggag 180
ctgatccccc tggccaagga gatgatggcc cagaagcgca aggggaagat ggtgaagctg 240
tacgtgctgg gcagcgtgct ggccctcttc ggcgtggtgc tcggcctgat ggagactgtg 300
tgcagcccct tcacggccgc cagacgtctg cgggaccagg aggcagccgt ggcggagctg 360
caggccgccc tggagcgaca ggctctccag aagcaagccc tgcaggagaa aggcaagcag 420
caggacacgg tcctcggcgg ccgggccctg tccaaccggc agcacgcctc ctaggaactg 480
tgggagacca gcggagtggg agggagacgc agtagacaga gacagaccga gaaggaaggg 540
agagacagag gggcgcgcg cacaggagcc tgactccgct gggagagtgc aggagcacgt 600
gctgtttttt atttggactt aacttcagag aaaccgctga catctagaac tgacctacca 660
caagcatcca ccaaaggagt ttgggattga gttttgctgc tgtgcagcac tgcattgtca 720
tgacatttcc aacactgtgt gaattatcta aatgcgtcta ccattttgca ctagggagga 780
aggataaatg ctttttatgt tattattatt aattattaca atgaccacca ttttgcattt 840
tgaaataaaa aactttttat accaaaaaaa aaaaaaaaa 879

<210> 32
<211> 1220
<212> DNA
<213> Homo sapiens

<400> 32

agtcccagct cagagccgca acctgcacag ccatgcccgg gcaagaactc aggacggtga 60
atggctctca gatgctcctg gtgttgctgg tgctctcgtg gctgccgcat gggggcgccc 120
tgtctctggc cgaggcgagc cgcgcaagtt tcccgggacc ctcagagttg cactccgaag 180
actccagatt ccgagagttg cggaaacgct acgaggacct gctaaccagg ctgcgggcca 240
accagagctg ggaagattcg aacaccgacc tcgtcccggc ccctgcagtc cggatactca 300
cgccagaagt gcggctggga tccggcggcc acctgcacct gcgtatctct cgggccgccc 360
ttcccgaggg gctccccgag gcctcccgcc ttcaccgggc tctgttccgg ctgtccccga 420
cggcgtcaag gtcgtgggac gtgacacgac cgctgcggcg tcagctcagc cttgcaagac 480
cccaggcgcc cgcgctgcac ctgcgactgt cgccgccgcc gtcgcagtcg gaccaactgc 540
tggcagaatc ttcgtccgca cggccccagc tggagttgca cttgcggccg caagccgcca 600
gggggcgccg cagagcgcgt gcgcgcaacg gggaccactg tccgctcggg cccgggcgtt 660
gctgccgtct gcacacggtc cgcgcgtcgc tggaagacct gggctgggcc gattgggtgc 720
tgtcgccacg ggaggtgcaa gtgaccatgt gcatcggcgc gtgcccgagc cagttccggg 780
cggcaaacat gcacgcgcag atcaagacga gcctgcaccg cctgaagccc gacacggtgc 840
cagcgccctg ctgcgtgccc gccagctaca atcccatggt gctcattcaa aagaccgaca 900
ccggggtgtc gctccagacc tatgatgact tgttagccaa agactgccac tgcatatgag 960
cagtcctggt ccttccactg tgcacctgcg cggaggacgc gacctcagtt gtcctgccct 1020
gtggaatggg ctcaaggttc ctgagacacc cgattcctgc ccaaacagct gtatttatat 1080
aagtctgtta tttattatta atttattggg gtgaccttct tggggactcg gggctggtc 1140
tgatggaact gtgtatttat ttaaaactct ggtgataaaa ataaagctgt ctgaactgtt 1200
aaaaaaaaaa aaaaaaaaaa 1220

<210> 33
<211> 2371
<212> DNA
<213> Homo sapiens

<400> 33

```
ccggagcctg cggcgggcgg ctgtgacggc cccggcgctg acggcggcgg ccgggggggc 60
ggtgtcgacg cccgcggcgc cggctggggc atcaccgcgg gcctcgaccc cgaaatggcg 120
ctgctggccg aacacttgct gaagccgctg cccgcggaca agcagatcga gaccgggccc 180
ttcctcgagg cggtgtccca cctgccgccc ttcttcgatt gccttgggtc cccagtgttt 240
actcccatca aggcagacat aagcggcaac atcacgaaaa tcaaagctgt gtacgacacc 300
aacccagcca agttccggac cctgcagaac atcctggagg tggagaaaga aatgtatgga 360
gcagagtggc ccaaagtagg ggccacactg gcgctgatgt ggctgaaaag aggcctccgc 420
ttcatccagg tcttcctcca gagcatctgc gacggggagc gggacgagaa ccaccccaac 480
ctcatccgtg tcaacgccac caaggcctac gagatggccc tcaagaagta ccatggctgg 540
atcgtgcaga agatcttcca ggcagcactg tacgcagcac cctataagtc tgacttcctg 600
aaagcgctct ccaaggggca gaatgttacg gaggaggagt gcctggagaa gatccgcctc 660
ttcctagtca actacacggc gaccatcgat gtcatctacg agatgtacac ccagatgaac 720
gctgagctta actacaaggt gtaggcatgc ccactgctgg acacgtcccc gactcgtggc 780
cacatggaga aacaggcaaa ccagaatcac tgtgaatcaa gtcggtgaac tgcccctggc 840
ctgtgtcccc cagagcagcc cagggtcctg ccagagtctg caggggacag ccctcgtttt 900
ttaagtcttt tttttttttt tttttggtct attctaaagg accagtaaat aatgatctta 960
cttccaaatc tccttggaat ttcacgacag cacagactga ctttatacct tcatttcagc 1020
gtggtaaaaa ccgattaaca cttctaatga gtcaagtcct agggtttttt ggttttgttt 1080
tgttgccaac gaggaacaca gctctggggg aatggtgtca tccacctcgc tttaaaaata 1140
agcacatgat ggctgggcac cgtggctcac gcctgtaatc ccagcacttt gggaggctga 1200
ggcgggtgga tcacctgagg tcgggagttt gagaccagcc tggccaacat ggtgaaaccc 1260
catcgctact aaaaatataa aaaattagct gggcatggtg gcgcacgcct gtagttccag 1320
ctactcagga ggctgaggca ggagaatcgc ttgaacccgg gaggtggagg ttgcagtgag 1380
ctgagatcgc accattgcac tccagcctgg gcaacaagag cgaaactctg tctcaaaaaa 1440
aaaaaaaaga ggtgggtgga ttacttgagg tcagggtttg agatcagcct gaccaacatg 1500
gtgaaaccct atctctacta aaaatataga attagccagg catggtagcg cacgcctgta 1560
atcccatctt cttgggaggc tgaggcagga gaatcgctag aaactgggag gtggaggtta 1620
cagtagccga gatcgcgcca ctgcattcca gcctgggcaa caaaagcgaa actctgtctc 1680
aaaaaagaaa aaaaagaaaa aaaagcacgt gaccttatga ggctctcgct gtattgttat 1740
tttaaggact ataaagagtt tgatttaaaa ttatgcaggg cccctatgtg ggatttttta 1800
aaaagcaaac tggtgtgtat tctcatgtgg tttgcacagc ccagcctcac agcactattg 1860
taaaccctgc tctttctgtc tcgctagaca gatttttttg tttgttttct tttttctggt 1920
tgtttttttgt tgttgttgtt gttgttttac agctgaaacc aaccagcaag cccttgatga 1980
ccaagaggcg tttctttcaa agctataggg cacaaacaat tgaccataga tgactccgtt 2040
tgcattcttc tgcagaatta tttccttcag ggacagattt tccaacctaa gaaactacct 2100
accgtgtgta ttctcttgac ggggagagat gaacccttca gctgctaaga tccaagaaaa 2160
cgcctcactg ccttaacctt aactgttctt cctggcgcta aaaagagctg tatttttttaa 2220
agtgctgggg caaacaaagc aaccccaaaa gagttgatgt gtgttttaaa agaaaaaacc 2280
caatgaggaa caattggaga tttttatgca gaaactaaat aatccttaat aaataaatct 2340
ctattttgga atcacaaaaa aaaaaaaaaa a                                2371
```

<210> 34
<211> 2360
<212> DNA
<213> Homo sapiens

<400> 34

```
gctacgcggg ccacgctgct ggctggcctg acctaggcgc gcggggtcgg gcggccgcgc 60
gggcgggctg agtgagcaag acaagacact caagaagagc gagctgcgcc tgggtcccgg 120
ccaggcttgc acgcagaggc gggcggcaga cggtgcccgg cggaatctcc tgagctccgc 180
cgcccagctc tggtgccagc gcccagtggc cgccgcttcg aaagtgactg gtgcctcgcc 240
gcctcctctc ggtgcgggac catgaagctg ctgccgtcgg tggtgctgaa gctctttctg 300
gctgcagttc tctcggcact ggtgactggc gagagcctgg agcggcttcg gagagggcta 360
gctgctggaa ccagcaaccc ggaccctccc actgtatcca cggaccagct gctacccccta 420
ggaggcggcc gggaccggaa agtccgtgac ttgcaagagg cagatctgga cctttttgaga 480
gtcactttat cctccaagcc acaagcactg gccacaccaa acaaggagga gcacgggaaa 540
agaaagaaga aaggcaaggg gctagggaag aagagggacc catgtcttcg gaaatacaag 600
gacttctgca tccatggaga atgcaaatat gtgaaggagc tccgggctcc ctcctgcatc 660
```

```
tgccacccgg gttaccatgg agagaggtgt catgggctga gcctcccagt ggaaaatcgc 720
ttatatacct atgaccacac aaccatcctg gccgtggtgg ctgtggtgct gtcatctgtc 780
tgtctgctgg tcatcgtggg gcttctcatg tttaggtacc ataggagagg aggttatgat 840
gtggaaaatg aagagaaagt gaagttgggc atgactaatt cccactgaga gagacttgtg 900
ctcaaggaat cggctgggga ctgctacctc tgagaagaca caaggtgatt tcagactgca 960
gaggggaaag acttccatct agtcacaaag actccttcgt ccccagttgc cgtctaggat 1020
tgggcctccc ataattgctt tgccaaaata ccagagcctt caagtgccaa acagagtatg 1080
tccgatggta tctgggtaag aagaaagcaa aagcaaggga ccttcatgcc cttctgattc 1140
ccctccacca aaccccactt cccctcataa gtttgtttaa acacttatct tctggattag 1200
aatgccggtt aaattccata tgctccagga tctttgactg aaaaaaaaaa agaagaagaa 1260
gaaggagagc aagaaggaaa gatttgtgaa ctggaagaaa gcaacaaaga ttgagaagcc 1320
atgtactcaa gtaccaccaa gggatctgcc attgggaccc tccagtgctg gatttgatga 1380
gttaactgtg aaataccaca agcctgagaa ctgaattttg ggacttctac ccagatggaa 1440
aaataacaac tatttttgtt gttgttgttt gtaaatgcct cttaaattat atatttattt 1500
tattctatgt atgttaattt atttagtttt taacaatcta acaataatat ttcaagtgcc 1560
tagactgtta ctttggcaat ttcctggccc tccactcctc atccccacaa tctggcttag 1620
tgccacccac ctttgccaca aagctaggat ggttctgtga cccatctgta gtaatttatt 1680
gtctgtctac atttctgcag atcttccgtg gtcagagtgc cactgcggga gctctgtatg 1740
gtcaggatgt aggggttaac ttggtcagag ccactctatg agttggactt cagtcttgcc 1800
taggcgattt tgtctaccat ttgtgttttg aaagcccaag gtgctgatgt caaagtgtaa 1860
cagatatcag tgtctccccg tgtcctctcc ctgccaagtc tcagaagagg ttgggcttcc 1920
atgcctgtag ctttcctggt ccctcacccc catggcccca ggccacagcg tgggaactca 1980
ctttcccttg tgtcaagaca tttctctaac tcctgccatt cttctggtgc tactccatgc 2040
aggggtcagt gcagcagagg acagtctgga gaaggtatta gcaaagcaaa aggctgagaa 2100
ggaacaggga acattggagc tgactgttct tggtaactga ttacctgcca attgctaccg 2160
agaaggttgg aggtggggaa ggctttgtat aatcccaccc acctcaccaa aacgatgaag 2220
gtatgctgtc atggtccttt ctggaagttt ctggtgccat ttctgaactg ttacaacttg 2280
tatttccaaa cctggttcat atttatactt tgcaatccaa ataaagataa cccttattcc 2340
ataaaaaaaa aaaaaaaaaa                                             2360
```

<210> 35
<211> 1254
<212> DNA
<213> Homo sapiens

<400> 35

```
ctcacttggc cttacactcc gctcggctca ccatgtgtca ctctcgcagc tgccacccga 60
ccatgaccat cctgcaggcc ccgaccccgg ccccctccac catcccggga ccccggcggg 120
gctccggtcc tgagatcttc accttcgacc ctctcccgga gcccgcagcg gcccctgccg 180
ggcgccccag cgcctctcgc gggcaccgaa agcgcagccg cagggttctc taccctcgag 240
tggtccggcg ccagctgcca gtcgaggaac cgaacccagc caaaaggctt ctctttctgc 300
tgctcaccat cgtcttctgc cagatcctga tggctgaaga gggtgtgccg gcgcccctgc 360
ctccagagga cgcccctaac gccgcatccc tggcgcccac ccctgtgtcc gccgtcctcg 420
agccctttaa tctgacttcg gagccctcgg actacgctct ggacctcagc actttcctcc 480
agcaacaccc ggccgccttc taactgtgac tccccgcact ccccaaaaag aatccgaaaa 540
accacaaaga aacaccaggc gtacctggtg cgcgagagcg tatccccaac tgggacttcc 600
gaggcaactt gaactcagaa cactacagcg gagacgccac ccggtgcttg aggcgggacc 660
gaggcgcaca gagaccgagg cgcatagaga ccgaggcaca gcccagctgg ggctaggccc 720
ggtgggaagg agagcgtcgt taatttattt cttattgctc ctaattaata tttatatgta 780
tttatgtacg tcctcctagg tgatggagat gtgtacgtaa tatttatttt aacttatgca 840
agggtgtgag atgttccccc tgctgtaaat gcaggtctct tggtatttat tgagctttgt 900
gggactggtg gaagcaggac acctggaact gcggcaaagt aggagaagaa atggggagga 960
ctcgggtggg ggaggacgtc ccggctggga tgaagtctgg tggtgggtcg taagtttagg 1020
aggtgactgc atcctccagc atctcaactc cgtctgtcta ctgtgtgaga cttcggcgga 1080
ccattaggaa tgagatccgt gagatccttc catcttcttg aagtcgcctt tagggtggct 1140
gcgaggtaga gggttggggg ttggtgggct gtcacggagc gactgtcgag atcgcctagt 1200
atgttctgtg aacacaaata aaattgattt actgtctgca aaaaaaaaaa aaaa       1254
```

<210> 36

<211> 1376
<212> DNA
<213> Homo sapiens

<400> 36

```
gtaagaacac tctcgtgagt ctaacggtct tccggatgaa ggctatttga agtcgccata 60

acctggtcag aagtgtgcct gtcggcgggg agagaggcaa tatcaaggtt ttaaatctcg 120
gagaaatggc tttcgtttgc ttggctatcg gatgcttata tacctttctg ataagcacaa 180
catttggctg tacttcatct tcagacaccg agataaaagt taaccctcct caggattttg 240
agatagtgga tcccggatac ttaggttatc tctatttgca atggcaaccc ccactgtctc 300
tggatcattt taaggaatgc acagtggaat atgaactaaa ataccgaaac attggtagtg 360
aaacatggaa gaccatcatt actaagaatc tacattacaa agatgggttt gatcttaaca 420
agggcattga agcgaagata cacacgcttt taccatggca atgcacaaat ggatcagaag 480
ttcaaagttc ctgggcagaa actacttatt ggatatcacc acaaggaatt ccagaaacta 540
aagttcagga tatggattgc gtatattaca attggcaata tttactctgt tcttggaaac 600
ctggcatagg tgtacttctt gataccaatt acaacttgtt ttactggtat gagggcttgg 660
atcatgcatt acagtgtgtt gattacatca aggctgatgg acaaaatata ggatgcagat 720
ttccctattt ggaggcatca gactataaag atttctatat ttgtgttaat ggatcatcag 780
agaacaagcc tatcagatcc agttatttca cttttcagct tcaaaatata gttaaacctt 840
tgccgccagt ctatcttact tttactcggg agagttcatg tgaaattaag ctgaaatgga 900
gcataccttt gggacctatt ccagcaaggt gttttgatta tgaaattgag atcagagaag 960
atgatactac cttggtgact gctacagttg aaaatgaaac atacaccttg aaaacaacaa 1020
atgaaacccg acaattatgc tttgtagtaa gaagcaaagt gaatatttat tgctcagatg 1080
acggaatttg gagtgagtgg agtgataaac aatgctggga aggtgaagac ctatcgaaga 1140
aaactttgct acgtttctgg ctaccatttg gtttcatctt aatattagtt atatttgtaa 1200
ccggtctgct tttgcgtaag ccaaacacct acccaaaaat gattccagaa tttttctgtg 1260
atacatgaag actttccata tcaagagaca tggtattgac tcaacagttt ccagtcatgg 1320
ccaaatgttc aatatgagtc tcaataaact gaatttttct tgcgaatgtt gaaaaa     1376
```

<210> 37
<211> 2943
<212> DNA
<213> Homo sapiens

<400> 37

```
accaggcaac accattgaag gctcatatgt aaaaatccat gccttccttt ctcccaatct 60
ccattcccaa acttagccac tggcttctgg ctgaggcctt acgcatacct cccggggctt 120
gcacacacct tcttctacag aagacacacc ttgggcatat cctacagaag accaggcttc 180
tctctggtcc ttggtagagg gctactttac tgtaacaggg ccagggtgga gagttctctc 240
ctgaagctcc atcccctcta taggaaatgt gttgacaata ttcagaagag taagaggatc 300
aagacttctt tgtgctcaaa taccactgtt ctcttctcta ccctgcccta accaggagct 360
tgtcacccca aactctgagg tgatttatgc cttaatcaag caaacttccc tcttcagaaa 420
agatggctca ttttccctca aaagttgcca ggagctgcca agtattctgc caattcaccc 480
tggagcacaa tcaacaaatt cagccagaac acaactacag ctactattag aactattatt 540
attaataaat tcctctccaa atctagcccc ttgacttcgg atttcacgat ttctcccttc 600
ctcctagaaa cttgataagt ttcccgcgct tcccttttc taagactaca tgtttgtcat 660
cttataaagc aaaggggtga ataaatgaac caaatcaata acttctggaa tatctgcaaa 720
caacaataat atcagctatg ccatctttca ctattttagc cagtatcgag ttgaatgaac 780
atagaaaaat acaaaactga attcttccct gtaaattccc cgttttgacg acgcacttgt 840
agccacgtag ccacgcctac ttaagacaat tacaaaaggc gaagaagact gactcaggct 900
taagctgcca gccagagagg gagtcatttc attggcgttt gagtcagcaa agaagtcaag 960
atggccaaag ttccagacat gtttgaagac ctgaagaact gttacagtga aaatgaagaa 1020
gacagttcct ccattgatca tctgtctctg aatcagaaat ccttctatca tgtaagctat 1080
ggcccactcc atgaaggctg catggatcaa tctgtgtctc tgagtatctc tgaaacctct 1140
aaaacatcca agcttacctt caaggagagc atggtggtag tagcaaccaa cgggaaggtt 1200
ctgaagaaga gacggttgag tttaagccaa tccatcactg atgatgacct ggaggccatc 1260
gccaatgact cagaggaaga aatcatcaag cctaggtcag caccttttag cttcctgagc 1320
aatgtgaaat acaactttat gaggatcatc aaatacgaat tcatcctgaa tgacgccctc 1380
aatcaaagta taattcgagc caatgatcag tacctcacgg ctgctgcatt acataatctg 1440
gatgaagcag tgaaatttga catgggtgct tataagtcat caaaggatga tgctaaaatt 1500
accgtgattc taagaatctc aaaaactcaa ttgtatgtga ctgcccaaga tgaagaccaa 1560
ccagtgctgc tgaaggagat gcctgagata cccaaaacca tcacaggtag tgagaccaac 1620
ctcctcttct tctgggaaac tcacggcact aagaactatt tcacatcagt tgcccatcca 1680
aacttgttta ttgccacaaa gcaagactac tgggtgtgct tggcaggggg gccaccctct 1740
atcactgact ttcagatact ggaaaaccag gcgtaggtct ggagtctcac ttgtctcact 1800
tgtgcagtgt tgacagttca tatgtaccat gtacatgaag aagctaaatc ctttactgtt 1860
agtcatttgc tgagcatgta ctgagccttg taattctaaa tgaatgttta cactctttgt 1920
aagagtggaa ccaacactaa catataatgt tgttatttaa agaacaccct atattttgca 1980
tagtaccaat cattttaatt attattcttc ataacaattt taggaggacc agagctactg 2040
actatggcta ccaaaaagac tctacccata ttacagatgg gcaaattaag gcataagaaa 2100
actaagaaat atgcacaata gcagttgaaa caagaagcca cagacctagg atttcatgat 2160

ttcatttcaa ctgtttgcct tctactttta agttgctgat gaactcttaa tcaaatagca 2220
taagtttctg ggacctcagt tttatcattt tcaaaatgga gggaataata cctaagcctt 2280
cctgccgcaa cagtttttta tgctaatcag ggaggtcatt ttggtaaaat acttcttgaa 2340
gccgagcctc aagatgaagg caaagcacga aatgttattt tttaattatt atttatatat 2400
gtatttataa atatatttaa gataattata atatactata tttatgggaa ccccttcatc 2460
ctctgagtgt gaccaggcat cctccacaat agcagacagt gttttctggg ataagtaagt 2520
ttgatttcat taatacaggg cattttggtc caagttgtgc ttatcccata gccaggaaac 2580
tctgcattct agtacttggg agacctgtaa tcatataata aatgtacatt aattaccttg 2640
agccagtaat tggtccgatc tttgactctt ttgccattaa acttacctgg gcattcttgt 2700
ttcaattcca cctgcaatca agtcctacaa gctaaaatta gatgaactca actttgacaa 2760
ccatgagacc actgttatca aaactttctt ttctggaatg taatcaatgt ttcttctagg 2820
ttctaaaaat tgtgatcaga ccataatgtt acattattat caacaatagt gattgataga 2880
gtgttatcag tcataactaa ataaagcttg caacaaaatt ctctgacaaa aaaaaaaaaa 2940
aaa                                                               2943
```

<210> 38
<211> 1498
<212> DNA
<213> Homo sapiens

<400> 38

```
accaaacctc ttcgaggcac aaggcacaac aggctgctct gggattctct tcagccaatc 60
ttcattgctc aagtgtctga agcagccatg gcagaagtac ctgagctcgc cagtgaaatg 120
atggcttatt acagtggcaa tgaggatgac ttgttctttg aagctgatgg ccctaaacag 180
atgaagtgct ccttccagga cctggacctc tgccctctgg atggcggcat ccagctacga 240
atctccgacc accactacag caagggcttc aggcaggccg cgtcagttgt tgtggccatg 300
gacaagctga ggaagatgct ggttccctgc ccacagacct tccaggagaa tgacctgagc 360
accttctttc ccttcatctt tgaagaagaa cctatcttct tcgacacatg ggataacgag 420
gcttatgtgc acgatgcacc tgtacgatca ctgaactgca cgctccggga ctcacagcaa 480
aaaagcttgg tgatgtctgg tccatatgaa ctgaaagctc tccacctcca gggacaggat 540
atggagcaac aagtggtgtt ctccatgtcc tttgtacaag gagaagaaag taatgacaaa 600
atacctgtgg ccttgggcct caaggaaaag aatctgtacc tgtcctgcgt gttgaaagat 660
gataagccca ctctacagct ggagagtgta gatcccaaaa attacccaaa gaagaagatg 720
gaaaagcgat ttgtcttcaa caagatagaa atcaataaca agctggaatt tgagtctgcc 780
cagttcccca actggtacat cagcacctct caagcagaaa acatgcccgt cttcctggga 840
gggaccaaag gcggccagga tataactgac ttcaccatgc aatttgtgtc ttcctaaaga 900
gagctgtacc cagagagtcc tgtgctgaat gtggactcaa tccctagggc tggcagaaag 960
ggaacagaaa ggtttttgag tacggctata gcctggactt tcctgttgtc tacaccaatg 1020
cccaactgcc tgccttaggg tagtgctaag aggatctcct gtccatcagc caggacagtc 1080
agctctctcc tttcagggcc aatccccagc ccttttgttg agccaggcct ctctcacctc 1140
tcctactcac ttaaagcccg cctgacagaa accacggcca catttggttc taagaaaccc 1200
tctgtcattc gctcccacat tctgatgagc aaccgcttcc ctatttattt atttatttgt 1260
ttgtttgttt tattcattgg tctaatttat tcaaaggggg caagaagtag cagtgtctgt 1320
aaaagagcct agtttttaat agctatggaa tcaattcaat ttggactggt gtgctctctt 1380
taaatcaagt cctttaatta agactgaaaa tatataagct cagattattt aaatgggaat 1440
atttataaat gagcaaatat catactgttc aatggttctg aaataaactt cactgaag   1498
```

<210> 39
<211> 1666
<212> DNA
<213> Homo sapiens

<400> 39

```
ctccataagg cacaaacttt cagagacagc agagcacaca agcttctagg acaagagcca 60
ggaagaaacc accggaagga accatctcac tgtgtgtaaa catgacttcc aagctggccg 120
tggctctctt ggcagccttc ctgatttctg cagctctgtg tgaaggtgca gttttgccaa 180
ggagtgctaa agaacttaga tgtcagtgca taaagacata ctccaaacct ttccacccca 240
aatttatcaa agaactgaga gtgattgaga gtggaccaca ctgcgccaac acagaaatta 300
ttgtaaagct ttctgatgga agagagctct gtctggaccc caaggaaaac tgggtgcaga 360
gggttgtgga gaagtttttg aagagggctg agaattcata aaaaaattca ttctctgtgg 420
tatccaagaa tcagtgaaga tgccagtgaa acttcaagca aatctacttc aacacttcat 480
gtattgtgtg ggtctgttgt agggttgcca gatgcaatac aagattcctg gttaaatttg 540
aatttcagta aacaatgaat agtttttcat tgtaccatga aatatccaga acatacttat 600
atgtaaagta ttatttattt gaatctacaa aaaacaacaa ataattttta aatataagga 660
ttttcctaga tattgcacgg gagaatatac aaatagcaaa attgaggcca agggccaaga 720
```

```
gaatatccga actttaattt caggaattga atgggtttgc tagaatgtga tatttgaagc 780
atcacataaa aatgatggga caataaattt tgccataaag tcaaatttag ctggaaatcc 840
tggatttttt tctgttaaat ctggcaaccc tagtctgcta gccaggatcc acaagtcctt 900
gttccactgt gccttggttt ctcctttatt tctaagtgga aaaagtatta gccaccatct 960
tacctcacag tgatgttgtg aggacatgtg gaagcacttt aagtttttc atcataacat 1020
aaattatttt caagtgtaac ttattaacct atttattatt tatgtattta tttaagcatc 1080
aaatatttgt gcaagaattt ggaaaaatag aagatgaatc attgattgaa tagttataaa 1140
gatgttatag taaatttatt ttattttaga tattaaatga tgttttatta gataaatttc 1200
aatcagggtt tttagattaa acaaacaaac aattgggtac ccagttaaat tttcatttca 1260
gataaacaac aaataatttt ttagtataag tacattattg tttatctgaa attttaattg 1320
aactaacaat cctagtttga tactcccagt cttgtcattg ccagctgtgt tggtagtgct 1380
gtgttgaatt acggaataat gagttagaac tattaaaaca gccaaaactc cacagtcaat 1440
attagtaatt tcttgctggt tgaaacttgt ttattatgta caaatagatt cttataatat 1500
tatttaaatg actgcatttt taaatacaag gctttatatt tttaacttta agatgttttt 1560
atgtgctctc caaatttttt ttactgtttc tgattgtatg gaaatataaa agtaaatatg 1620
aaacatttaa aatataattt gttgtcaaag taaaaaaaaa aaaaaa                1666
```

<210> 40
<211> 7878
<212> DNA
<213> Homo sapiens

<400> 40

ttttccctgc tctcaccggg cgggggagag aagccctctg gacagcttct agagtgtgca 60
ggttctcgta tccctcggcc aagggtatcc tctgcaaacc tctgcaaacc cagcgcaact 120
acggtccccc ggtcagaccc aggatggggc cagaacggac aggggccgcg ccgctgccgc 180
tgctgctggt gttagcgctc agtcaaggca ttttaaattg ttgtttggcc tacaatgttg 240
gtctcccaga agcaaaaata ttttccggtc cttcaagtga acagtttggc tatgcagtgc 300
agcagtttat aaatccaaaa ggcaactggt tactggttgg ttcaccctgg agtggctttc 360
ctgagaaccg aatgggagat gtgtataaat gtcctgttga cctatccact gccacatgtg 420
aaaaactaaa tttgcaaact tcaacaagca ttccaaatgt tactgagatg aaaaccaaca 480
tgagcctcgg cttgatcctc accaggaaca tgggaactgg aggttttctc acatgtggtc 540
ctctgtgggc acagcaatgt gggaatcagt attacacaac gggtgtgtgt tctgacatca 600
gtcctgattt tcagctctca gccagcttct cacctgcaac tcagccctgc ccttccctca 660
tagatgttgt ggttgtgtgt gatgaatcaa atagtattta tccttgggat gcagtaaaga 720
atttttgga aaaatttgta caaggcctgg atataggccc cacaaagaca caggtggggt 780
taattcagta tgccaataat ccaagagttg tgtttaactt gaacacatat aaaaccaaag 840
aagaaatgat tgtagcaaca tcccagacat cccaatatgg tggggacctc acaaacacat 900
tcggagcaat tcaatatgca agaaaatatg cttattcagc agcttctggt gggcgacgaa 960
gtgctacgaa agtaatggta gttgtaactg acggtgaatc acatgatggt tcaatgttga 1020
aagctgtgat tgatcaatgc aaccatgaca atatactgag gtttggcata gcagttcttg 1080
ggtacttaaa cagaaacgcc cttgatacta aaaatttaat aaaagaaata aaagcaatcg 1140
ctagtattcc aacagaaaga tactttttca atgtgtctga tgaagcagct ctactagaaa 1200
aggctgggac attaggagaa caaattttca gcattgaagg tactgttcaa ggaggagaca 1260
actttcagat ggaaatgtca caagtgggat tcagtgcaga ttactcttct caaaatgata 1320
ttctgatgct gggtgcagtg ggagcttttg gctggagtgg gaccattgtc cagaagacat 1380
ctcatggcca tttgatcttt cctaaacaag cctttgacca aattctgcag gacagaaatc 1440
acagttcata tttaggttac tctgtggctg caatttctac tggagaaagc actcactttg 1500
ttgctggtgc tcctcgggca aattataccg gccagatagt gctatatagt gtgaatgaga 1560
atggcaatat cacggttatt caggctcacc gaggtgacca gattggctcc tattttggta 1620
gtgtgctgtg ttcagttgat gtggataaag acaccattac agacgtgctc ttggtaggtg 1680
caccaatgta catgagtgac ctaaagaaag aggaaggaag agtctacctg tttactatca 1740
aagagggcat tttgggtcag caccaatttc ttgaaggccc cgagggcatt gaaaacactc 1800
gatttggttc agcaattgca gctctttcag acatcaacat ggatggcttt aatgatgtga 1860
ttgttggttc accactagaa aatcagaatt ctggagctgt atacatttac aatggtcatc 1920
agggcactat ccgcacaaag tattcccaga aaatcttggg atccgatgga gcctttagga 1980
gccatctcca gtactttggg aggtccttgg atggctatgg agatttaaat ggggattcca 2040
tcaccgatgt gtctattggt gcctttggac aagtggttca actctggtca caaagtattg 2100
ctgatgtagc tatagaagct tcattcacac cagaaaaaat cactttggtc aacaagaatg 2160
ctcagataat tctcaaactc tgcttcagtg caaagttcag acctactaag caaaacaatc 2220
aagtggccat tgtatataac atcacacttg atgcagatgg attttcatcc agagtaacct 2280
ccaggggggtt atttaaagaa aacaatgaaa ggtgcctgca gaagaatatg gtagtaaatc 2340
aagcacagag ttgccccgag cacatcattt atatacagga gccctctgat gttgtcaact 2400
ctttggattt gcgtgtggac atcagtctgg aaaaccctgg cactagccct gcccttgaag 2460
cctattctga gactgccaag gtcttcagta ttcctttcca caaagactgt ggtgaggacg 2520

gactttgcat ttctgatcta gtcctagatg tccgacaaat accagctgct caagaacaac 2580
cctttattgt cagcaaccaa aacaaaaggt taacattttc agtaacgctg aaaaataaaa 2640
gggaaagtgc atacaacact ggaattgttg ttgatttttc agaaaacttg ttttttgcat 2700
cattctccct gccggttgat gggacagaag taacatgcca ggtggctgca tctcagaagt 2760
ctgttgcctg cgatgtaggc taccctgctt taaagagaga acaacaggtg acttttacta 2820
ttaactttga cttcaatctt caaaaccttc agaatcaggc gtctctcagt ttccaagcct 2880
taagtgaaag ccaagaagaa aacaaggctg ataatttggt caacctcaaa attcctctcc 2940
tgtatgatgc tgaaattcac ttaacaagat ctaccaacat aaatttttat gaaatctctt 3000
cggatgggaa tgttccttca atcgtgcaca gttttgaaga tgttggtcca aaattcatct 3060
tctccctgaa ggtaacaaca ggaagtgttc cagtaagcat ggcaactgta atcatccaca 3120
tccctcagta taccaaagaa aagaacccac tgatgtacct aactggggtg caaacagaca 3180
aggctggtga catcagttgt aatgcagata tcaatccact gaaaatagga caaacatctt 3240
cttctgtatc tttcaaaagt gaaaatttca ggcacaccaa agaattgaac tgcagaactg 3300
cttcctgtag taatgttacc tgctggttga aagacgttca catgaaagga gaatactttg 3360
ttaatgtgac taccagaatt tggaacggga ctttcgcatc atcaacgttc cagacagtac 3420
agctaacggc agctgcagaa atcaacacct ataaccctga gatatatgtg attgaagata 3480
acactgttac gattcccctg atgataatga aacctgatga gaaagccgaa gtaccaacag 3540
gagttataat aggaagtata attgctggaa tccttttgct gttagctctg gttgcaattt 3600
tatggaagct cggcttcttc aaaagaaaat atgaaaagat gaccaaaaat ccagatgaga 3660
ttgatgagac cacagagctc agtagctgaa ccagcagacc tacctgcagt gggaaccggc 3720
agcatcccag ccagggtttg ctgtttgcgt gaatggattt ctttttaaat cccatatttt 3780
ttttatcatg tcgtaggtaa actaacctgg tattttaaga gaaaactgca ggtcagtttg 3840
gaatgaagaa attgtggggg gtgggggagg tgcggggggc aggtagggaa ataataggga 3900
aaatacctat tttatatgat ggggggaaaa aagtaatctt taaactggct ggcccagagt 3960
ttacattcta atttgcattg tgtcagaaac atgaaatgct tccaagcatg acaactttta 4020
aagaaaaata tgatactctc agattttaag gggaaaaact gttctcttta aaatatttgt 4080
ctttaaacag caactacaga agtggaagtg cttgatatgt aagtacttcc acttgtgtat 4140
attttaatga atattgatgt taacaagagg ggaaaacaaa acacaggttt tttcaattta 4200
tgctgctcat ccaaagttgc cacagatgat acttccaagt gataatttta tttataaact 4260
aggtaaaatt tgttgttggt tccttttaga ccacggctgc cccttccaca ccccatcttg 4320
ctctaatgat caaaacatgc ttgaataact gagcttagag tatacctcct atatgtccat 4380
ttaagttagg agagggggcg atatagagaa taaggcacaa aattttgttt aaaactcaga 4440
atataacatg taaaatccca tctgctagaa gcccatcctg tgccagagga aggaaaagga 4500
ggaaatttcc tttctctttt aggaggcaca acagttctct tctaggattt gtttggctga 4560
ctggcagtaa cctagtgaat ttctgaaaga tgagtaattt ctttggcaac cttcctcctc 4620
ccttactgaa ccactctccc acctcctggt ggtaccatta ttatagaagc cctctacagc 4680
ctgactttct ctccagcggt ccaaagttat cccctccttt acccctcatc caaagttccc 4740
actccttcag gacagctgct gtgcattaga tattaggggg gaaagtcatc tgtttaattt 4800
acacacttgc atgaattact gtatataaac tccttaactt cagggagcta ttttcattta 4860
gtgctaaaca agtaagaaaa ataagctcga gtgaatttct aaatgttgga atgttatggg 4920
atgtaaacaa tgtaaagtaa gacatctcag gatttcacca gaagttacag atgaggcact 4980
ggaagccacc aaattagcag gtgcaccttc tgtggctgtc ttgtttctga agtacttaaa 5040
cttccacaag agtgaatttg acctaggcaa gtttgttcaa aaggtagatc ctgagatgat 5100
ttggtcagat tgggataagg cccagcaatc tgcattttaa caagcacccc agtcactagg 5160
atgcagatgg accacacttt gagaaacacc acccatttct actttttgca ccttattttc 5220
tctgttcctg agcccccaca ttctctagga gaaacttaga ggaaaagggc acagacacta 5280
catatctaaa gctttggaca agtccttgac ctctataaac ttcagagtcc tcattataaa 5340
atgggaagac tgagctggag ttcagcagtg atgcttttag ttttaaaagt ctatgatctg 5400
gacttcctat aatacaaata cacaatcctc caagaatttg acttggaaaa aaatgtcaaa 5460
ggaaaacagg ttatctgccc atgtgcatat ggacaacctt gactaccctg gcctggcccg 5520
tggtggcagt ccagggctat ctgtactgtt tacagaatta ctttgtagtt gacaacacaa 5580
aacaaacaaa aaaggcataa aatgccagcg gtttatagaa aaaacagcat ggtattctcc 5640
agttaggtat gccagagtcc aattctttta acagctgtga gaatttgctg cttcattcca 5700
acaaaatttt atttaaaaaa aaaaaaaaaa gactggagaa actagtcatt agcttgataa 5760
agaatattta acagctagtg gtgctggtgt gtacctgaag ctccagctac ttgagagact 5820
gagacaggaa gatcgcttga gcccaggagt tcaagtccag cctaagcaac atagcaagac 5880
cctgtctcaa aaaaatgact atttaaaaag acaatgtggc caggcacggt ggctcacacc 5940
tgtaatccca acactttggg aggctgaggc cggtggatca cgaggtcagg agtttgagac 6000
tagcctggcc aacatggtga aaccccatct ctaataatat aaaaattagc tgggcgtagt 6060
agcaggtgcc tgtaatccca gttactcggg aagctgaggc aggagaatca cttgaacccg 6120
ggaggcagag gtttcagtga gccgagatcg cgccactgca ctccagcctg ggtgacaggg 6180
caagactctg tctcaaacaa acaaacaaaa aaaaagttag tactgtatat gtaaatacta 6240
gcttttcaat gtgctataca aacaattata gcacatcctt ccttttactc tgtctcacct 6300
cctttaggtg agtacttcct taaataagtg ctaaacatac atatacggaa cttgaaagct 6360
ttggttagcc ttgccttagg taatcagcct agtttacact gtttccaggg agtagttgaa 6420

ttactataaa ccattagcca cttgtctctg caccatttat cacaccagga cagggtctct 6480
caacctgggc gctactgtca tttggggcca ggtgattctt ccttgcaggg gctgtcctgt 6540
accttgtagg acagcagccc tgtcctagaa ggtatgttta gcagcattcc tggcctctag 6600
ctacccgatg ccagagcatg ctccccccgc agtcatgaca atcaaaaaat gtctccagac 6660
attgtcaaat gcctcctggg gggcagtatt tctcaagcac ttttaagcaa aggtaagtat 6720
tcatacaaga aatttagggg gaaaaaacat tgtttaaata aaagctatgt gttcctattc 6780
aacaatattt ttgctttaaa agtaagtaga gggcataaaa gatgtcatat tcaaatttcc 6840
atttcataaa tggtgtacag acaaggtcta tagaatgtgg taaaaacttg actgcaacac 6900
aaggcttata aaatagtaag atagtaaaat agcttatgaa gaaactacag agatttaaaa 6960
ttgtgcatga ctcatttcag cagcaaaata agaactccta actgaacaga aatttttcta 7020
cctagcaatg ttattcttgt aaaatagtta cctattaaaa ctgtgaagag taaaactaaa 7080
gccaatttat tatagtcaca caagtgatta tactaaaaat tattataaag gttataattt 7140
tataatgtat ttacctgtcc tgatatatag ctataaccca atatatgaaa atctcaaaaa 7200
ttaagacatc atcatacaga aggcaggatt ccttaaactg agatccctga tccatcttta 7260
atatttcaat ttgcacacat aaaacaatgc ccttttgtgt acattcaggc atacccattt 7320
taatcaattt gaaaggttaa tttaaacctc tagaggtgaa tgagaaacat ggggggaaaag 7380
tatgaaatag gtgaaaatct taactatttc tttgaactct aaagactgaa actgtagcca 7440
ttatgtaaat aaagtttcat atgtacctgt ttattttggc agattaagtc aaaatatgaa 7500
tgtatatatt gcataactat gttagaattg tatatatttt aaagaaattg tcttggatat 7560
tttcctttat acataataga taagtctttt ttcaaatgtg gtgtttgatg tttttgatta 7620
aatgtgtttt gcctctttcc acaaaaactg taaaaataaa tgcatgtttg tacaaaaagt 7680
tgcagaattc atttgattta tgagaaacaa aaattaaatt gtagtcaaca gttagtagtt 7740
tttctcatat ccaagtataa caaacagaaa agtttcatta ttgtaaccca ctttttttcat 7800
accacattat tgaatattgt tacaattgtt ttgaaaataa agccattttc tttgggcttt 7860
tataagttaa aaaaaaaa 7878

<210> 41
<211> 9042
<212> DNA
<213> Homo sapiens

<400> 41

gcgcgccaag acgtgggcac ctcctcaccc ggaccccggg ccccgccgag ccgcctcctg 60
gctcccgtgg ccgccagccc gccccggccg caccgagcgt cgggatccga ggtgggagcg 120
tacccctccc cgctccccgg acgcctcagt cctccgcact gagcttggcc acgcgcccct 180
aggcgccccc cacgccctgg gccccggagg gccgcagcca tgagtgaaat gtccagcttt 240
cttcacatcg gggacatcgt ctccctgtac gccgagggct ccgtcaatgg cttcatcagc 300
actttggggc tggtggatga ccgctgtgtg gtggagcccg cggccgggga cctggacaac 360
ccccctaaga agttccgtga ctgcctcttc aaggtgtgcc ccatgaaccg ctactcggcc 420
cagaagcagt actggaaggc caagcagact aagcaggaca aggagaagat cgctgatgtg 480
gtgttgctgc agaagctgca gcatgcggcg cagatggagc agaagcaaaa tgacacggag 540
aacaagaagg tgcatgggga tgtcgtgaag tatggcagtg tgatccagct cctgcacatg 600
aagagcaaca agtacctgac agtgaacaag cggcttccgg ccttgctgga gaagaacgcc 660
atgcgggtga ctctggatgc cacaggcaac gagggttcct ggctcttcat ccagcccttc 720
tggaagctgc ggagcaacgg ggacaacgtg gtcgtggggg acaaggtgat cctgaatcct 780
gtcaatgccg ggcagcctct gcatgccagc aattacgagc tcagcgacaa cgccggctgc 840
aaggaggtca attctgtgaa ctgcaacacc agctggaaga tcaacctgtt tatgcagttt 900
cgggaccacc tggaggaggt gttgaaaggg ggagacgtgg tgcggctgtt ccatgcggag 960
caggagaagt tcctgacgtg tgacgagtac aagggcaagc tgcaggtgtt cctgcgaact 1020
acactgcgcc agtctgccac ctcggccacc agctccaatg ctctctggga ggtggaggtg 1080
gtccaccacg acccctgccg tggaggagct gggcactgga atggcttgta ccgcttcaag 1140
cacctggcta caggcaacta cctggctgct gaggagaacc ccagttacaa aggtgatgcc 1200
tcagatccca aggcagcagg aatgggggca cagggccgca caggccgcag gaatgctggg 1260
gagaagatca agtactgcct ggtggctgtg cctcatggca atgacatcgc ctctctcttt 1320
gagctggacc ccaccacctt gcagaaaacc gactctttcg tgccccggaa ctcgtacgtc 1380
cggctgcggc acctctgcac caacacgtgg attcagagca ccaatgtgcc cattgacatc 1440
gaggaggagc ggcccatccg gctcatgctg ggcacctgcc ccaccaagga ggacaaggag 1500
gcctttgcca tcgtgtcagt gcccgtgtct gagatccgag acctggactt tgccaatgac 1560
gccagctcca tgctggccag tgccgtggag aaactcaacg agggcttcat cagccagaat 1620
gaccgcaggt ttgtcatcca gctgctggaa gacctggtgt tctttgtcag cgatgtcccc 1680
aacaatgggc agaatgtcct ggacatcatg gtcactaagc ccaaccggga acggcagaag 1740
ctgatgaggg agcagaacat cctcaaacag gtctttggca ttctgaaggc cccgttccgt 1800
gagaaggggg gtgaaggtcc cctggtgcgg ctggaggagc tgtcagacca gaagaacgcc 1860
ccctaccagc acatgttccg cctgtgctac cgcgtgttgc ggcattccca ggaggactac 1920
cgcaagaacc aggagcacat tgccaagcag tttgggatga tgcagtccca gattggctac 1980

gacatcctgg ccgaggacac catcactgcc ctgctgcaca acaaccgcaa gctcctggaa 2040
aagcacatca ccaagaccga ggtggagacc ttcgtcagcc ttgtgcgcaa gaaccgggag 2100
cccaggttcc tggactacct ctctgacctg tgtgtgtcca accacatcgc catccccgtc 2160
acccaagagc tcatctgcaa gtgtgtgctg gaccccaaga acagtgacat tctcatccgg 2220
accgagcttc ggcccgtgaa ggagatggcc caatcccacg agtacctgag catcgagtac 2280
tcagaagagg aagtgtggct cacgtggact gacaagaata acgagcatca tgagaagagt 2340
gtgaggcagc tggcccagga ggcgcgggcc ggcaacgccc acgacgagaa tgtgctcagc 2400
tactacaggt accagctgaa gctctttgcc cgcatgtgct tggaccgcca gtacttggcc 2460
atcgacgaga tctcccagca gctgggcgtg gacctgattt tcctgtgcat ggcagacgag 2520
atgctgccct ttgacctgcg cgcctccttc tgccacctga tgctgcacgt gcacgtggac 2580
cgtgaccccc aggagctggt cacgccggtc aagtttgccc gtctctggac tgagatcccc 2640
acagccatca ccatcaagga ctatgattcc aacctcaacg cgtcccgaga tgacaagaag 2700
aacaagtttg ccaacaccat ggagttcgtg gaggactacc tcaacaatgt agtcagcgag 2760
gccgtgccct ttgccaacga ggagaagaac aagctcactt ttgaggtggt cagcctggcg 2820
cacaatctca tctacttcgg cttctacagc ttcagcgagc tgctgcggct cactcgcaca 2880
ctgctgggca tcatcgactg tgtgcagggg cccccggcca tgctgcaggc ctatgaggac 2940
cccggtggca agaatgtgcg gcggtccatc cagggcgtgg ggcacatgat gtccaccatg 3000
gtgctgagcc gcaagcagtc cgtcttcagt gcccccagcc tgtctgctgg ggccagtgct 3060
gctgagccgc tggacagaag caagtttgag gagaatgagg acattgtggt gatggagacc 3120
aagctgaaga tcctggaaat ccttcagttc atcctcaatg tccgcctgga ttaccgcata 3180
tcctacctgc tgtctgtctt caagaaggag tttgtggagg tgtttcccat gcaggacagt 3240
ggggctgatg gcacagcccc tgccttcgac tctaccactg ccaacatgaa cctggatcgc 3300
atcggggagc aggcggaggc catgtttgga gtggggaaga caagcagcat gctggaggtg 3360
gatgacgagg gcggccgcat gttcctgcgc gtgctcatcc acctcaccat gcacgactat 3420
gcgccgctgg tctcgggtgc cctgcagctg ctcttcaagc acttcagcca gcgccaggag 3480
gccatgcaca ccttcaagca ggttcagctg ctgatctcag cgcaggacgt ggagaactac 3540
aaggtgatca agtcggagct ggaccggctg cggaccatgg tggagaagtc agagctgtgg 3600
gtggacaaga agggcagtgg caagggtgag gaggtggagg caggcgccgc caaggacaag 3660
aaagagcgtc ccacggacga ggagggcttt ctgcacccac caggggagaa aagcagtgag 3720
aactaccaga tcgtcaaggg catcctggaa aggctgaaca agatgtgcgg ggttggggag 3780
caaatgagga agaagcagca acggctgctg aagaacatgg atgcccacaa ggtcatgctg 3840
gacctgctgc agatccccta tgacaagggt gatgccaaga tgatggagat cctgcgctac 3900
acgcaccagt tcctgcagaa gttctgtgca gggaaccccg gcaaccaggc cctgctgcac 3960
aaacacctgc acctcttcct cacgccaggg ctcctggagg cagagaccat gcagcacatc 4020
ttcctgaaca actatcagct ctgctccgag atcagcgagc ctgtgttgca gcacttcgtg 4080
cacctgctgg ccacgcacgg gcgccatgtg cagtacctgg acttcctgca caccgtcatt 4140
aaggccgagg gcaagtacgt caagaagtgc caggacatga tcatgactga gctgaccaat 4200
gcaggtgacg atgtggtcgt gttctacaat gataaggcat cgctggccca cctgctggac 4260
atgatgaagg ccgcccgcga cggcgtggag gaccacagcc ccctcatgta ccacatttcc 4320
ctggtggacc tgctggccgc ctgtgccgag ggcaaaaacg tctacactga gatcaagtgc 4380
acctccctgc tgccgctgga ggacgtggtg tctgtggtga cgcatgagga ctgcatcact 4440
gaggtgaaaa tggcctatgt gaacttcgtg aaccactgct acgtggacac ggaggtggag 4500
atgaaggaga tctacaccag caaccacatc tggacgctct ttgagaactt caccctggac 4560
atggctcggg tctgcagcaa gcgtgagaag cgcgtggctg accccacctt ggagaagtac 4620
gtgctgagcg ttgtgctgga caccatcaac gccttcttca gctccccatt ctctgagaac 4680
agcacttccc tgcagacaca ccagacgatt gtggtgcagc tgctgcagtc taccacacgc 4740
ctcctcgagt gtccgtggct acagcagcag cacaagggct ccgtggaggc ctgcatccgg 4800
accctcgcca tggtggccaa gggccgggcc atcttgctgc ccatggacct ggatgcccac 4860
atcagctcga tgctcagcag tggagccagc tgtgcagctg ccgcccagcg gaacgcctcc 4920
agctacaagg caaccacgcg ggccttcccc cgcgtcaccc ccaccgccaa ccagtgggac 4980
tacaagaaca tcattgagaa gctgcaggac atcatcacag ccctggagga gcggctgaag 5040
cccctggtac aggctgagct gtccgtgctg gtggatgtcc tgcactggcc tgagctgctc 5100
ttcctggagg gcagtgaggc ctaccagcgc tgcgagagtg gggcttcct gtccaagctg 5160
atccagcaca ccaaggacct catggagtcg gaggagaagc tgtgcatcaa ggtgctgcgg 5220
accctgcagc agatgctgct caagaagacc aagtacgggg accggggcaa ccagctgcgc 5280
aagatgctgc tgcaaaacta cctccagaac cggaagtcca cctcgcgggg ggaccttccc 5340
gaccccatag gcactggcct ggacccagac tggtcggcaa tcgcagccac ccagtgccgg 5400
ctggacaagg agggggccac caagttggta tgcgacctca tcaccagcac caagaacgag 5460
aagatcttcc aggagagcat cggcctggcc atccacctgc tggatggtgg caacacagag 5520
atccagaaat ccttccacaa cctgatgatg agtgacaaga agtcagagcg cttcttcaag 5580
gtgctgcacg accgcatgaa gcgggcccag caggagacca agtccacggt ggcagtcaac 5640
atgaatgacc tgggcagcca gccacatgag gaccgcgagc cagtcgaccc caccaccaaa 5700
ggccgcgtgg cctccttctc gatacctggc tcctcatccc gctactcgct gggccccagc 5760
ctgcgccggg ggcacgaggt gagcgaacgt gtgcagagca gtgagatggg cacatccgtg 5820
ctcatcatgc agcccatcct gcgctttctg cagctgctgt gtgagaacca caaccgggac 5880

```
ctgcagaact tcctgcgctg tcagaacaac aaaaccaact acaacttggt atgcgagacg 5940
ctgcagttcc tggacatcat gtgcggcagc accacgggcg gcctggggct gctggggctc 6000
tacatcaatg aggacaacgt gggcctcgtc atccagacct tggagaccct cactgagtac 6060
tgccagggcc cctgccatga gaaccagact tgcattgtga ctcacgagtc caatggcata 6120
gacatcatca ccgcactgat cctcaatgac atcagccccc tgtgcaagta ccgcatggat 6180
ctggtgctgc agctcaagga caatgcctcc aagctgctcc tggctctgat ggagagccgg 6240
catgacagtg aaaatgctga gcgaatcctc atcagcctgc ggccccagga gctggtggac 6300
gtcatcaaga aggcctacct gcaggaggaa gagcgtgaga actcggaggt gagcccacgt 6360
gaagtgggcc ataacatcta tatcctggcg ctgcagctct ccaggcacaa taaacagctg 6420
cagcacctgc tgaagccggt gaagcgcatt caagaggagg aggccgaggg tatctcttcc 6480
atgctcagcc tcaacaacaa gcagctgtca cagatgctca agtcctcagc gccagcacag 6540
gaggaggagg aagaccccct ggcctactat gagaaccaca cgtcccagat cgagattgtg 6600
cggcaggacc gcagcatgga gcagatcgtg ttcccagtgc ccggcatctg ccagttcctg 6660
acggaggaaa ccaagcaccg gctcttcacc actactgagc aggacgagca gggcagcaaa 6720
gtgagcgact tcttcgacca gtcctccttc ctgcacaacg agatggagtg gcagcgcaag 6780
ctccgcagca tgccgctgat ctactggttc tcccgccgca tgaccctgtg ggcagcatc 6840
tccttcaacc tggccgtgtt tatcaacatc atcattgcct tcttctaccc ttacatggag 6900
ggcgcgtcca caggcgtgct ggactcccct ctcatctcat tgctcttctg gatcctcatc 6960
tgcttctcca tcgcggccct gttcaccaag cgctacagca tccgccccct catcgtggcg 7020
ctcatcctgc gctccatcta ctatctgggc atcgggccca cactcaacat cctgggtgcc 7080
ctcaatctga ccaacaagat cgtgtttgtg gtgagcttcg tgggcaaccg tggcaccttc 7140
atccggggct ataaggccat ggtcatggac atggaattcc tctaccacgt gggctacatc 7200
ctgaccagtg tcctgggcct ctttgctcat gagctgttct acagcatcct gctctttgac 7260
ctcatctacc gcgaggagac gctgttcaac gtcatcaaga gtgtgacccg caatggccgc 7320
tccatcctgc tgacagccct gctggccctc atcctggtct acctcttctc catcgtcggc 7380
ttcctcttcc tcaaggatga cttcattctc gaggtcgacc ggctgcccaa caaccactcc 7440
acagccagcc ccctggggat gccacatgga gctgctgcat ttgtggacac ctgcagtggg 7500
gacaagatgg actgtgtctc agggctctcg gtgcctgagg tcctggaaga ggacagggag 7560
ctggacagca cagagcgggc ctgtgacact ctgttgatgt gcatcgtcac tgtcatgaac 7620
catgggctac gcaacggtgg tggcgtgggc gacattctcc gcaagccctc caaagatgag 7680
tctctcttcc cagcccgagt ggtctatgac ctcctgttct tcttcatcgt catcatcatt 7740
gtgctgaacc tcatctttgg ggtaatcatc gacaccttcg ctgacctgcg tagtgagaag 7800
cagaagaagg aggagattct taagacgaca tgcttcatct gtggtctgga gagggacaag 7860
tttgataaca agacagtgtc atttgaggaa cacatcaagc tggagcacaa catgtggaac 7920
tacttgtact tcattgtgct ggtccgcgtg aagaacaaga ccgactacac gggccctgag 7980
agctacgtgg cccagatgat caagaacaag aacctggact ggttcccccg gatgcgggcc 8040
atgtcccttg tcagcaatga gggcgagggg gagcagaatg agattcggat tctccaggac 8100
aagctcaact ccaccatgaa gctggtgtcc cacctcactg cccagctcaa cgagctcaag 8160
gagcagatga cggagcagcg gaaacgcagg caacgcctag gctttgtgga tgtccagaac 8220
tgcattagcc gctgaggaga gccaccgaag gccccaacag gggatgctca tcactggaga 8280
ctgcgactgg gaagaacact gccccctccc tcgggttggg tggcccagcc agctggccag 8340
cctccactcc cactctgcca gacaccctga cacccaccca ggctttgaag agcatggagg 8400
gggagcctca gagctgacag tcctgcttag agcccttaaa aagacttgaa agttcactgg 8460
gactcagttt accttaatgc cttagcagaa gataaatcct acctagagac ctttgttcct 8520
taaagcaata actgacaact ctttgtagtc ctccttgtgg gtagttaaga gtggggtcac 8580
ccctttaact ccaagcacta cattttggcg gctgccggcc tctgggggag gtggcagtta 8640
tgctgttact agtgatttta gggctttgtt atttaactta tttcaagggt gctgtgctca 8700
gccctgccca tggctgtgca gctccctccg tgcctcagat ctgctgtagc cagtgcagac 8760
ctcactgtcg tgtccatgcc acccccggca tggctccagg tggcctggtg actccatgat 8820
ggacgatctt gctcccagga cctgcctctt cccaggcttc ctggggaaga gttgtacgcc 8880
caggcaacaa gggctgagct gcgcttgcgt ggctgtttca tgaccgcttg tttttctcct 8940
tttggtgtaa tgttttacaa atcctttggc ctgagaacta atatgttaat tgccttaaat 9000
aaattaatag aaatctagtc cgttaaaaaa aaaaaaaaaa aa                   9042
```

<210> 42
<211> 1901
<212> DNA
<213> Homo sapiens

<400> 42

gggcaggaag acggcgctgc ccggaggagc ggggcgggcg ggcgcgcggg ggagcgggcg 60
gcgggcggga gccaggcccg ggcgggggcg ggggcggcgg ggccagaaga ggcggcgggc 120
cgcgctccgg ccggtctgcg gcgttggcct tggctttggc tttggcggcg gcggtggaga 180
agatgctgca gtccctggcc ggcagctcgt gcgtgcgcct ggtggagcgg caccgctcgg 240
cctggtgctt cggcttcctg gtgctgggct acttgctcta cctggtcttc ggcgcagtgg 300

tcttctcctc ggtggagctg ccctatgagg acctgctgcg ccaggagctg cgcaagctga 360
agcgacgctt cttggaggag cacgagtgcc tgtctgagca gcagctggag cagttcctgg 420
gccgggtgct ggaggccagc aactacggcg tgtcggtgct cagcaacgcc tcgggcaact 480
ggaactggga cttcacctcc gcgctcttct tcgccagcac cgtgctctcc accacaggtt 540
atggccacac cgtgcccttg tcagatggag gtaaggcctt ctgcatcatc tactccgtca 600
ttggcattcc cttcaccctc ctgttcctga cggctgtggt ccagcgcatc accgtgcacg 660
tcacccgcag gccggtcctc tacttccaca tccgctgggg cttctccaag caggtggtgg 720
ccatcgtcca tgccgtgctc cttgggtttg tcactgtgtc ctgcttcttc ttcatcccgg 780
ccgctgtctt ctcagtcctg gaggatgact ggaacttcct ggaatccttt tatttttgtt 840
ttatttccct gagcaccatt ggcctggggg attatgtgcc tggggaaggc tacaatcaaa 900
aattcagaga gctctataag attgggatca cgtgttacct gctacttggc cttattgcca 960
tgttggtagt tctggaaacc ttctgtgaac tccatgagct gaaaaaattc agaaaaatgt 1020
tctatgtgaa gaaggacaag gacgaggatc aggtgcacat catagagcat gaccaactgt 1080
ccttctcctc gatcacagac caggcagctg gcatgaaaga ggaccagaag caaaatgagc 1140
cttttgtggc cacccagtca tctgcctgcg tggatggccc tgcaaaccat tgagcgtagg 1200
atttgttgca ttatgctaga gcaccagggt cagggtgcaa ggaagaggct taagtatgtt 1260
catttttatc agaatgcaaa agcgaaaatt atgtcacttt aagaaatagc tactgtttgc 1320
aatgtcttat taaaaaacaa caaaaaaaga cacatggaac aaagaagctg tgaccccagc 1380
aggatgtcta atatgtgagg aaatgagatg tccacctaaa attcatatgt gacaaaatta 1440
tctcgacctt acataggagg agaatacttg aagcagtatg ctgctgtggt tagaagcaga 1500
ttttatactt ttaactggaa actttggggt ttgcatttag atcatttagc tgatggctaa 1560
atagcaaaat ttatatttag aagcaaaaaa aaaaagcata gagatgtgtt ttataaatag 1620
gtttatgtgt actggtttgc atgtacccac ccaaaatgat tatttttgga gaatctaagt 1680
caaactcact atttataatg cataggtaac cattaactat gtacatataa agtataaata 1740
tgtttatatt ctgtacatat ggtttaggtc accagatcct agtgtagttc tgaaactaag 1800
actatagata ttttgtttct tttgatttct ctttatacta aagaatccag agttgctaca 1860
ataaaataag gggaataata aacttgagag tgaataacca t 1901

<210> 43
<211> 2240
<212> DNA
<213> Homo sapiens

<400> 43

```
gtccttcggt gtctgggtgt ggtgagtaga ggtgtgtgtc acaaagtaca gaccattgtg 60
tgtgacaaag cccatcgtgt gtctgtgtgt gtctttatcc acgtggatgg acgtctcttt 120
cttgctctgc cccaagacac accctagccc ctccttattc tcaaaagggg gagctgggga 180
gcctccccct accctggggc ctcccctgcc cctccccgcc ctgcctggcc gtcaccactc 240
cccagagggc acagggctct gctgtgcctc agagcaaaag tcccagagcc agcagagcag 300
gctgacgacc tgcaagccac agtggctgcc ctgtgcgtgc tgcgaggtgg gggaccctgg 360
gcaggaagct ggctgagccc caagaccccg gggccatgg gcggggatct ggtgcttggc 420
ctgggggcct tgagacgccg aaagcgcttg ctggagcagg agaagtctct ggccggctgg 480
gcactggtgc tggcaggaac tggcattgga ctcatggtgc tgcatgcaga gatgctgtgg 540
ttcggggggt gctcgtgggc gctctacctg ttcctggtta aatgcacgat cagcatttcc 600
accttcttac tcctctgcct catcgtggcc tttcatgcca aagaggtcca gctgttcatg 660
accgacaacg ggctgcggga ctggcgcgtg gcgctgaccg ggcggcaggc ggcgcagatc 720
gtgctggagc tggtggtgtg tgggctgcac ccggcgcccg tgcggggccc gccgtgcgtg 780
caggatttag ggcgccgct gacctccccg cagccctggc cgggattcct gggccaaggg 840
gaagcgctgc tgtccctggc catgctgctg cgtctctacc tggtgccccg cgccgtgctc 900
ctgcgcagcg gcgtcctgct caacgcttcc taccgcagca tcggcgctct caatcaagtc 960
cgcttccgcc actggttcgt ggccaagctt tacatgaaca cgcaccctgg ccgcctgctg 1020
ctcggcctca cgcttggcct ctggctgacc accgcctggg tgctgtccgt ggccgagagg 1080
caggctgtta atgccactgg gcacctttca gacacacttt ggctgatccc catcacattc 1140
ctgaccatcg gctatggtga cgtggtgccg ggcaccatgt ggggcaagat cgtctgcctg 1200
tgcactggag tcatgggtgt ctgctgcaca gccctgctgg tggccgtggt ggcccggaag 1260
ctggagttta acaaggcaga gaagcacgtg cacaacttca tgatggatat ccagtatacc 1320
aaagagatga aggagtccgc tgcccgagtg ctacaagaag cctggatgtt ctacaaacat 1380
actcgcagga aggagtctca tgctgcccgc aggcatcagc gcaagctgct ggccgccatc 1440
aacgcgttcc gccaggtgcg gctgaaacac cggaagctcc gggaacaagt gaactccatg 1500
gtggacatct ccaagatgca catgatcctg tatgacctgc agcagaatct gagcagctca 1560
caccgggccc tggagaaaca gattgacacg ctggcgggga agctggatgc cctgactgag 1620
ctgcttagca ctgccctggg gccgaggcag cttccagaac ccagccagca gtccaagtag 1680
ctggacccac gaggaggaac caggctactt tccccagtac tgaggtggtg gacatcgtct 1740
ctgccactcc tgacccagcc ctgaacaaag cacctcaagt gcaaggacca aagggggccc 1800
tggcttggag tgggttggct tgctgatggc tgctggaggg gacgctggct aaagtgggta 1860

ggccttggcc cacctgaggc cccaggtggg aacatggtca cccccactct gcataccctc 1920
atcaaaaaca ctctcactat gctgctatgg acgacctcca gctctcagtt acaagtgcag 1980
gcgactggag gcaggactcc tgggtccctg ggaaagaggg tactaggggc ccggatccag 2040
gattctggga ggcttcagtt accgctggcc gagctgaaga actgggtatg aggctggggc 2100
ggggctggag gtggcgcccc ctggtgggac aacaaagagg acaccatttt tccagagctg 2160
cagagagcac ctggtgggga ggaagaagtg taactcacca gcctctgctc ttatctttgt 2220
aataaatgtt aaagccagaa                                             2240
```

<210> 44
<211> 3350
<212> DNA
<213> Homo sapiens

<400> 44

tagtcgcggg gcaggtacgt gcgctcgcgg ttctctcgcg gaggtcggcg gtggcgggag 60
cgggctccgg agagcctgag agcacggtgg ggcggggcgg gagaaagtgg ccgccccggag 120
gacgttggcg tttacgtgtg gaagagcgga agagttttgc ttttcgtgcg cgccttcgaa 180
aactgcctgc cgctgtctga ggagtccacc cgaaacctcc cctcctccgc cggcagcccc 240
gcgctgagct cgccgaccca agccagcgtg ggcgaggtgg gaagtgcgcc cgacccgcgc 300
ctggagctgc gcccccgagt gcccatggct acaagggtgc tgagcatgag cgcccgcctg 360
ggacccgtgc cccagccgcc ggcgccgcag gacgagccgg tgttcgcgca gctcaagccg 420
gtgctgggcg ccgcgaatcc ggcccgcgac gcggcgctct tccccggcga ggagctgaag 480
cacgcgcacc accgcccgca ggcgcagccc gcgcccgcgc aggccccgca gccggcccag 540
ccgcccgcca ccggcccgcg gctgcctcca gaggacctgg tccagacaag atgtgaaatg 600
gagaagtatc tgacacctca gcttcctcca gttcctataa ttccagagca taaaaagtat 660
agacgagaca gtgcctcagt cgtagaccag ttcttcactg acactgaagg gttaccttac 720
agtatcaaca tgaacgtctt cctccctgac atcactcacc tgagaactgg cctctacaaa 780
tcccagagac cgtgcgtaac acacatcaag acagaacctg ttgccatttt cagccaccag 840
agtgaaacga ctgcccctcc tccggccccg acccaggccc tccctgagtt caccagtata 900
ttcagctcac accagaccgc agctccagag gtgaacaata ttttcatcaa acaagaactt 960
cctacaccag atcttcatct ttctgtccct acccagcagg gccacctgta ccagctactg 1020
aatacaccgg atctagatat gcccagttct acaaatcaga cagcagcaat ggacactctt 1080
aatgtttcta tgtcagctgc catggcaggc cttaacacac acacctctgc tgttccgcag 1140
actgcagtga aacaattcca gggcatgccc ccttgcacat acacaatgcc aagtcagttt 1200
cttccacaac aggccactta ctttcccccg tcaccaccaa gctcagagcc tggaagtcca 1260
gatagacaag cagagatgct ccagaattta accccacctc catcctatgc tgctacaatt 1320
gcttctaaac tggcaattca caatccaaat ttacccacca ccctgccagt taactcacaa 1380
aacatccaac ctgtcagata caatagaagg agtaaccccg atttggagaa acgacgcatc 1440
cactactgcg attaccctgg ttgcacaaaa gtttatacca agtcttctca tttaaaagct 1500
cacctgagga ctcacactgg tgaaaagcca tacaagtgta cctgggaagg ctgcgactgg 1560
aggttcgcgc gatcggatga gctgacccgc cactaccgga agcacacagg cgccaagccc 1620
ttccagtgcg gggtgtgcaa ccgcagcttc tcgcgctctg accacctggc cctgcatatg 1680
aagaggcacc agaactgagc actgcccgtg tgacccgttc caggtcccct gggctccctc 1740
aaatgacaga cctaactatt cctgtgtaaa aacaacaaaa acaaacaaaa gcaagaaaac 1800
cacaactaaa actggaaatg tatattttgt atatttgaga aaacagggaa tacattgtat 1860
taataccaaa gtgtttggtc attttaagaa tctggaatgc ttgctgtaat gtatatggct 1920
ttactcaagc agatctcatc tcatgacagg cagccacgtc tcaacatggg taaggggtgg 1980
gggtggaggg gagtgtgtgc agcgttttta cctaggcacc atcatttaat gtgacagtgt 2040
tcagtaaaca aatcagttgg caggcaccag aagaagaatg gattgtatgt caagatttta 2100
cttggcattg agtagttttt ttcaatagta ggtaattcct tagagataca gtatacctgg 2160
caattcacaa atagccattg aacaaatgtg tgggttttta aaaattatat acatatatga 2220
gttgcctata tttgctattc aaaattttgt aaatatgcaa atcagcttta taggtttatt 2280
acaagttttt taggattctt ttggggaaga gtcataattc ttttgaaaat aaccatgaat 2340
acacttacag ttaggatttg tggtaaggta cctctcaaca ttaccaaaat catttcttta 2400
gagggaagga ataatcattc aaatgaactt taaaaaagca aatttcatgc actgattaaa 2460
ataggattat tttaaataca aaaggcattt tatatgaatt ataaactgaa gagcttaaag 2520
atagttacaa aatacaaaag ttcaacctct tacaataagc taaacgcaat gtcattttta 2580
aaaagaagga cttagggtgt cgttttcaca tatgacaatg ttgcatttat gatgcagttt 2640
caagtaccaa aacgttgaat tgatgatgca gttttcatat atcgagatgt tcgctcgtgc 2700
agtactgttg gttaaatgac aatttatgtg gattttgcat gtaatacaca gtgagacaca 2760
gtaattttat ctaaattaca gtgcagttta gttaatctat taatactgac tcagtgtctg 2820
cctttaaata taaatgatat gttgaaaact taaggaagca aatgctacat atatgcaata 2880
taaaatagta atgtgatgct gatgctgtta accaaagggc agaataaata agcaaaatgc 2940
caaaaggggt cttaattgaa atgaaaattt aattttgttt ttaaaatatt gtttatcttt 3000
atttattttg tggtaatata gtaagttttt ttagaagaca attttcataa cttgataaat 3060

tatagttttg tttgttagaa aagttgctct taaaagatgt aaatagatga caaacgatgt 3120
aaataatttt gtaagaggct tcaaaatgtt tatacgtgga aacacaccta catgaaaagc 3180
agaaatcggt tgctgttttg cttctttttc cctcttattt ttgtattgtg gtcatttcct 3240
atgcaaataa tggagcaaac agctgtatag ttgtagaatt ttttgagaga atgagatgtt 3300
tatatattaa cgacaatttt ttttttggaa aataaaaagt gcctaaaaga 3350

<210> 45
<211> 1598
<212> DNA

<213> Homo sapiens

<400> 45

```
gggggaggct ggcagcggag ctttgaatag ggaagttttg caggggttac gtttgcagtc 60
agtccggtgt ttgcaaatat tgtgtgggct ccgcgcgctg cgggctgcgg gagggtccgg 120
ccgggcgtct ctgcgagcct ggagtttgca tgaaactttc acctgcgctc cggggagact 180
ttcggctccg gctcccaccg cgcgcctcgc cgccctcgcg accgcgggct ccgtccaacc 240
cggcccgaca tggacgtgct ccccatgtgc agcatcttcc aggagctcca gatcgtgcac 300
gagaccggct acttctcggc gctgccgtct ctggaggagt actggcaaca gacctgccta 360
gagctggaac gttacctcca gagcgagccc tgctatgttt cagcctcaga aatcaaattt 420
gacagccagg aagatctgtg gaccaaaatc attctggctc gggagaaaaa ggaggaatcc 480
gaactgaaga tatcttccag tcctccagag gacactctca tcagcccgag cttttgttac 540
aacttagaga ccaacagcct gaactcagat gtcagcagcg aatcctctga cagctccgag 600
gaactttctc ccacggccaa gtttacctcc gaccccattg gcgaagtttt ggtcagctcg 660
ggaaaattga gctcctctgt cacctccacg cctccatctt ctccggaact gagcagggaa 720
ccttctcaac tgtggggttg cgtgcccggg gagctgccct cgccagggaa ggtgcgcagc 780
gggacttcgg ggaagccagg tgacaaggga aatggcgatg cctcccccga cggcaggagg 840
agggtgcacc ggtgccactt taacggctgc aggaaagttt acaccaaaag ctcccacttg 900
aaagcacacc agcggacgca cacaggagaa aagccttaca gatgctcatg ggaagggtgt 960
gagtggcgtt ttgcaagaag tgatgagtta accaggcact tccgaaagca caccggggcc 1020
aagcctttta aatgctccca ctgtgacagg tgtttttcca ggtctgacca cctggccctg 1080
cacatgaaga ggcacctctg agggagcaga gaggtggatc ctgtaggcta aaaggcttcc 1140
aggctgagag ccggccgtgg aaggagggat gcgtgttcca gccaaagcat gccgttctgc 1200
accctaccca gttgcctcca gggcctctcc ttggaaggtc ttttgagggc taaaaaggtc 1260
ctgtaagaag cggcatagca cccgtggtgc atggtatgtg ggtgaccctg gactcgccac 1320
tggtacccgc ccttccgagc ggcgcctaag cctttgccgt gagcatgcac actgagaatg 1380
ctaatggttg ggttgattgt atgttgagga tctattactg accgtatgat gaggccaact 1440
tttttttcctt gtggttagca agactgcaag agatggaaaa aaagtagttt gaatgttttg 1500
tgtgtaagga gtataccatg agatgagatg accaccaatc atttccttgg ggggaggggg 1560
tgtctgcacc ttagaaaaaa aaagaaaaat caaaaaaa                          1598
```

<210> 46
<211> 10511
<212> DNA
<213> Homo sapiens

<400> 46

```
gcaggtccgg gaggcgcagg cggagagcgg cggtgccccc gagcccctct gcggacggct 60
caggcgggag gaccccgcgc ggctggatgg cggcggccgc gcggcctcgg ggtcgggcac 120
tggggccagt actgccgccg acgccgctgc tcctgctggt actgcgggtg ctgccagcct 180
gcggggcgac cgctcgggat cccggggccg cggccgggct cagccttcac ccgacttact 240
tcaacctggc cgaggcggcg aggatttggg ccaccgccac ctgcggggag aggggacccg 300
gcgaggggag gccccagccc gagctctact gcaagttggt cgggggcccc accgccccag 360
gcagcggcca caccatccag ggccagttct gtgactattg caattctgaa gaccccagga 420
aagcacatcc tgtcaccaat gccatcgatg gatctgaacg ttggtggcaa agccctcccc 480
tgtcctcagg cacacagtac aacagagtca acctcacctt ggatctgggg cagctcttcc 540
atgtggccta tattttaatc aaatttgcaa attctcctcg ccctgatctt tgggtcttgg 600
aaagatctgt agactttgga agcacctact caccatggca atattttgct cattctaaag 660
tagactgttt aaaagaattt gggcgggagg caaatatggc tgtcacccgg gatgatgatg 720
tactttgtgt tactgaatat tcccgtattg tacctttgga aaatggtgag gttgtggtgt 780
ccttgataaa cggtcgtcca ggtgcaaaaa attttacttt ctctcacacc ctgagggagt 840
ttaccaaggc aacaaacatc cgcttgcgtt ttcttagaac caatacgctt cttggacacc 900
tcatctccaa agcccagcga gatccaactg tcactcggcg gtattattac agcataaagg 960
acatcagcat tggtgggcag tgtgtttgca atggccatgc tgaagtgtgc aatataaaca 1020
atcctgaaaa actgtttcgg tgtgaatgcc agcaccacac ctgtggggag acgtgtgatc 1080
gctgctgcac agggtacaat cagaggcgct ggcggcccgc cgcttgggag cagagccacg 1140
```

agtgtgaagc atgcaactgc cacggccatg ccagcaactg ttactatgat ccagatgttg 1200
agcggcagca ggcaagcttg aatacccagg gcatctatgc tggtggaggg gtctgcatta 1260
actgtcagca caacacagct ggagtaaact gtgaacagtg tgctaagggc tattaccgcc 1320
cttatggggt tccagtggat gcccctgatg gctgcatccc ctgcagctgt gaccctgagc 1380
atgcggatgg ctgtgaacag ggttcaggcc gctgtcactg caagccaaat ttccacggag 1440
acaactgtga gaagtgtgca attggatact acaatttccc attttgcttg agaattccca 1500
tttttcctgt ttctacacca agttcagaag atccagtagc tggagatata aaagggtgtg 1560
actgtaatct ggaaggtgtt ctccctgaaa tatgtgatgc ccacggacgg tgcctgtgcc 1620
gccctggggt tgagggccct cgatgtgata cctgccgctc tggtttctac tcattcccta 1680
tttgccaagc ctgctggtgt tcagcccttg gatcctacca gatgccctgc agctcagtga 1740
ctggacagtg tgaatgtcgg ccaggagtta caggacagcg gtgtgacagg tgtctctcag 1800
gagcttatga tttcccccac tgccaaggtt ccagcagtgc ttgtgaccca gctggtacca 1860
tcaactccaa tttggggtat tgccaatgca agcttcatgt tgaaggtcct acttgtagcc 1920
gctgcaaact gttatattgg aatctggaca aagaaaaccc cagtggatgt tcagaatgca 1980
agtgccataa ggcgggaaca gtgagtggaa ctggagagtg taggcaggga gatggtgact 2040
gtcactgcaa gtcccatgtg ggtggcgatt cctgcgacac ctgtgaagat ggatattttg 2100
ctttggaaaa gagcaattac tttgggtgtc aagggtgtca gtgtgacatt ggtggggcat 2160
tgtcctccat gtgcagtggg ccctcgggag tgtgccagtg ccgagagcat gtcgtgggaa 2220
aggtgtgcca gcggcctgaa aacaactact atttcccaga tttgcatcat atgaagtatg 2280
agattgaaga cggcagcaca cctaatggga gagaccttcg atttggattt gatccgctgg 2340
catttcctga gtttagctgg agaggatatg cccaaatgac ctcagtacag aatgatgtaa 2400
gaataacatt gaatgtaggg aagtcaagtg gctccttgtt tcgtgttatt ctgagatacg 2460
ttaaccctgg aactgaagca gtatctggcc atataactat ttatccatcc tggggtgctg 2520
ctcaaagcaa agagatcatc ttcctgccga gtaaggagcc agcctttgtc actgtccctg 2580
gaaatggttt tgcagaccca ttttcaatca caccaggaat atgggttgct tgtattaagg 2640
cagaaggagt ccttctggat tacctggtgc tgctccccag ggactactat gaagcctctg 2700
tactgcagct gccagtcaca gaaccatgtg cctacgcagg acctccccaa gaaaattgct 2760
tactctacca gcatttgcca gtgaccagat tcccctgtac cctggcttgt gaggccagac 2820
acttcctgct tgatggggag ccaagacccg tggcagtgag gcagcccaca cctgcacacc 2880
ctgtcatggt ggacctcagc gggagagagg tggaattgca tctgcggctg cgcatcccac 2940
aggttggcca ctacgtggtt gtggtcgagt attccacgga ggcagctcag ctgtttgtgg 3000
ttgatgtgaa tgtgaagagc tccgggtctg ttctggcagg ccaggtgaac atttacagct 3060
gcaactacag tgttctctgc cggagtgctg tgattgatca catgagccgc atcgccatgt 3120
atgagctatt ggcagatgca gacattcagc tcaagggaca catggcccga ttccttctgc 3180
atcaagtttg tatcatacct attgaagaat tctcagctga gtatgtgaga ccacaagtcc 3240
actgcattgc cagttatggg cgatttgtca atcaaagtgc cacctgtgtc tccttggccc 3300
atgaaactcc tccaacagca ttaattttgg atgttctaag tggcaggcct ttccctcacc 3360
tgccccagca gtcgtcacct tctgttgatg ttcttcctgg ggtcaccttg aaggcaccgc 3420
agaatcaagt gaccctgaga ggacgtgtac cacacctggg ccgatacgtc tttgtcatcc 3480
atttttacca agcagcgcac ccgacgtttc ccgcgcaggt gtcggtggat ggcgggtggc 3540
cacgggcagg ctccttccat gcctcttttt gcccccatgt gcttggctgc cgggatcaag 3600
tgattgccga aggccagatt gagtttgaca tctcagagcc tgaagtggcc gcaactgtga 3660
aggttccaga aggaaagtcc ttggttttgg tccgtgttct agtggtgcct gcagaaaact 3720
atgactacca aatacttcac aaaaaatcca tggacaagtc actcgagttt atcaccaatt 3780
gtggaaaaaa cagcttttac cttgaccccc agacagcctc cagattctgt aagaattccg 3840
ccaggtccct ggtggccttt taccacaagg gcgccctgcc ttgtgagtgc caccccactg 3900
gggccaccgg ccctcactgc agccctgagg gtgggcagtg cccatgccag cccaacgtca 3960
tcgggcggca gtgcacccgc tgtgcaacag gccactacgg attcccacgc tgcaagccgt 4020
gcagctgtgg tcggcgcctt tgtgaagaga tgacggggca gtgccgctgc cctccccgca 4080
cggtcaggcc ccagtgtgag gtgtgtgaga cacactcatt cagcttccac cccatggccg 4140
gctgcgaagg ctgcaactgt tccaggaggg gcaccatcga ggctgccatg ccggagtgtg 4200
accgggacag cgggcagtgc agatgcaagc ccagaatcac agggcggcag tgtgaccgat 4260
gtgcttccgg gttttaccgc tttcctgagt gtgttccctg caattgcaac agagatggga 4320
ctgagccagg agtgtgtgac ccagggaccg gggcttgcct ctgcaaggaa aatgtagaag 4380
gcacagagtg taatgtgtgt cgagaaggct cattccattt ggacccagcc aatctcaagg 4440
gttgtaccag ctgtttctgt tttggagtaa ataatcaatg tcacagctca cataagcgaa 4500
ggactaagtt tgtggatatg ctgggctggc acctggagac agcagacaga gtggacatcc 4560
ctgtctcttt caacccaggc agcaacagta tggtggcgga tctccaggag ctgcccgcaa 4620
ccatccacag cgcgtcctgg gtcgcaccca cctcctacct gggggacaag gtttcttcat 4680
atggtggtta cctcacttac caagccaagt cctttggctt gcctggcgac atggttcttc 4740
tggaaaagaa gccggatgta cagctcactg gtcagcacat gtccatcatc tatgaggaga 4800
caaacacccc acggccagac cggctgcatc atggacgagt gcacgtggtc gagggaaact 4860
tcagacatgc cagcagccgt gccccagtgt ctagggagga gctgatgaca gtgctgtcta 4920
gactggcaga tgtgcgcatc caaggcctct acttcacaga gactcaaagg ctcaccctga 4980
gcgaggtggg gctagaggaa gcctctgaca caggaagtgg gcgcatagca cttgctgtgg 5040

aaatctgtgc ctgcccccct gcctacgctg gtgactcttg tcagggttgt agccctggat 5100
actatcggga tcataaaggc ttgtataccg gacggtgtgt tccctgcaat tgcaacggac 5160
attcaaatca atgccaggat ggctcaggca tatgtgttaa ctgtcagcac aacaccgcgg 5220
gagagcactg tgaacgctgc caggagggct actatggcaa cgccgtccac ggatcctgca 5280
gggcctgccc atgtcctcac actaacagct ttgccactgg ctgtgtggtg aatgggggag 5340
acgtgcggtg ctcctgcaaa gctgggtaca caggaacaca gtgtgaaagg tgtgcaccgg 5400
gatatttcgg gaatccccag aaattcggag gtagctgcca accatgcagt tgtaacagca 5460
atggccagct gggcagctgt catcccctga ctggagactg cataaaccaa gaacccaaag 5520
atagcagccc tgcagaagaa tgtgatgatt gcgacagctg tgtgatgacc ctcctgaacg 5580
acctggccac catgggcgag cagctccgcc tggtcaagtc tcagctgcag ggcctgagtg 5640
ccagcgcagg gcttctggag cagatgaggc acatggagac ccaggccaag gacctgagga 5700
atcagttgct caactaccgt tctgccattt caaatcatgg atcaaaaata gaaggcctgg 5760
aaagagaact gactgatttg aatcaagaat ttgagacttt gcaagaaaag gctcaagtaa 5820
attccagaaa agcacaaaca ttaaacaaca atgttaatcg ggcaacacaa agcgcaaaag 5880
aactggatgt gaagattaaa aatgtcatcc ggaatgtgca cattctttta aagcagatct 5940
ctgggacaga tggagaggga aacaacgtgc cttcaggtga cttttccaga gagtgggctg 6000
aagcccagcg catgatgagg gaactgcgga acaggaactt tggaaagcac ctcagagaag 6060
cagaagctga taaaagggag tcgcagctct tgctgaaccg gataaggacc tggcagaaaa 6120
cccaccaggg ggagaacaat gggcttgcta acagtatccg ggattcttta aatgaatacg 6180
aagccaaact cagtgacctt cgtgctcggc tgcaggaggc agctgcccaa gccaagcagg 6240
caaatggctt gaaccaagaa aacgagagag ctttgggagc cattcagaga caagtgaaag 6300
aaataaattc cctgcagagt gatttcacca agtatctaac cactgcagac tcatctttgt 6360
tgcaaaccaa cattgcgctg cagctgatgg agaaaagcca gaaggaatat gaaaaattag 6420
ctgccagttt aaatgaagca agacaagaac taagtgacaa agtaagagaa ctttccagat 6480
ctgctggcaa aacatccctt gtggaggagg cagaaaagca cgcgcggtcc ttacaagagc 6540
tggcaaagca gctggaagag atcaagagaa acgccagcgg ggatgagctg gtgcgctgtg 6600
ctgtggatgc cgccaccgcc tacgagaaca tcctcaatgc catcaaagcg gccgaggacg 6660
cagccaacag ggctgccagt gcatctgaat ctgccctcca gacagtgata aaggaagatc 6720
tgccaagaaa agctaaaacc ctgagttcca acagtgataa actgttaaat gaagccaaga 6780
tgacacaaaa gaagctaaag caagaagtca gtccagctct caacaaccta cagcaaaccc 6840
tgaatattgt gacagttcag aaagaagtga tagacaccaa tctcacaact ctccgagatg 6900
gtcttcatgg gatacagaga ggtgatattg atgctatgat cagtagtgca aagagcatgg 6960
tcagaaaggc caacgacatc acagatgagg ttctggatgg gctcaacccc atccagacag 7020
atgtggaaag aattaaggac acctatggga ggacacagaa cgaagacttc aaaaaggctc 7080
tgactgatgc agataactcg gtgaataagt taaccaacaa actacctgat ctttggcgca 7140
agattgaaag tatcaaccaa cagctgttgc ccttgggaaa catctctgac aacatggaca 7200
gaatacgaga actaattcag caggccagag atgctgccag taaggttgct gtccccatga 7260
ggttcaatgg taaatctgga gtcgaagtcc gactgccaaa tgacctggaa gatttgaaag 7320
gatatacatc tctgtccttg tttctccaaa ggcccaactc aagagaaaat ggggggtactg 7380
agaatatgtt tgtgatgtac cttggaaata aagatgcctc ccgggactac atcggcatgg 7440
cagttgtgga tggccagctc acctgtgtct acaacctggg ggaccgtgag gctgaactcc 7500
aagtggacca gatcttgacc aagagtgaga ctaaggaggc agttatggat cgggtgaaat 7560
ttcagagaat ttatcagttt gcaaggctta attacaccaa aggagccaca tccagtaaac 7620
cagaaacacc cggagtctat gacatggatg gtagaaatag caatacactc cttaatttgg 7680
atcctgaaaa tgttgtattt tatgttggag gttacccacc tgattttaaa cttcccagtc 7740
gactaagttt ccctccatac aaaggttgta ttgaattaga tgacctcaat gaaaatgttc 7800
tgagcttgta caacttcaaa aaaacattca atctcaacac aactgaagtg gagccttgta 7860
gaaggaggaa ggaagagtca gacaaaaatt attttgaagg tacgggctat gctcgagttc 7920
caactcaacc acatgctccc atcccaacct ttggacagac aattcagacc accgtggata 7980
gaggcttgct gttctttgca gaaaacgggg atcgcttcat atctctaaat atagaagatg 8040
gcaagctcat ggtgagatac aaactgaatt cagagctacc aaaagagaga ggagttggag 8100
acgccataaa caacggcaga gaccattcga ttcagatcaa aattggaaaa ctccaaaagc 8160
gtatgtggat aaatgtggac gttcaaaaca ctataattga tggtgaagta tttgatttca 8220
gcacatatta tctgggagga attccaattg caatcaggga aagatttaac atttctacgc 8280
ctgctttccg aggctgcatg aaaaatttga agaaaaccag tggtgtcgtt agattgaatg 8340
atactgtggg agtaaccaaa aagtgctcgg aagactggaa gcttgtgcga tctgcctcat 8400
tctccagagg aggacaattg agtttcactg atttgggctt accacctact gaccacctcc 8460
aggcctcatt tggatttcag acctttcaac ccagtggcat attattagat catcagacat 8520
ggacaaggaa cctgcaggtc actctggaag atggttacat tgaattgagc accagcgata 8580
gcggcagccc aatttttaaa tctccacaga cgtatatgga tggtttactg cattatgtat 8640
ctgtaataag cgacaactct ggactacggc ttctcatcga tgaccagctt ctgagaaata 8700
gcaaaaggct aaaacacatt tcaagttccc ggcagtctct gcgtctgggc gggagcaatt 8760
ttgagggttg tattagcaat gtttttgtcc agaggttatc actgagtcct gaagtcctag 8820
atttgaccag taactctctc aagagagatg tgtccctggg aggctgcagt ttaaacaaac 8880
cacctttttct aatgttgctt aaaggttcta ccaggtttaa caagaccaag acttttcgta 8940

```
tcaaccagct gttgcaggac acaccagtgg cctccccaag gagcgtgaag gtgtggcaag 9000
atgcttgctc accacttccc aagacccagg ccaatcatgg agccctccag tttggggaca 9060
ttcccaccag ccacttgcta ttcaagcttc ctcaggagct gctgaaaccc aggtcacagt 9120
ttgctgtgga catgcagaca acatcctcca gaggactggt gtttcacacg ggcactaaga 9180
actcctttat ggctctttat ctttcaaaag gacgtctggt ctttgcactg gggacagatg 9240
ggaaaaaatt gaggatcaaa agcaaggaga aatgcaatga tgggaaatgg cacacggtgg 9300
tgtttggcca tgatggggaa aaggggcgct tggttgtgga tggactgagg gcccgggagg 9360
gaagtttgcc tggaaactcc accatcagca tcagagcgcc agtttacctg ggatcacctc 9420
catcagggaa accaaagagc ctccccacaa acagctttgt gggatgcctg aagaactttc 9480
agctggattc aaaacccttg tataccccctt cttcaagctt cggggtgtct tcctgcttgg 9540
gtggtccttt ggagaaaggc atttatttct ctgaagaagg aggtcatgtc gtcttggctc 9600
actctgtatt gttggggcca gaatttaagc ttgttttcag catccgccca agaagtctca 9660
ctgggatcct aatacacatc ggaagtcagc ccgggaagca cttatgtgtt tacctggagg 9720
caggaaaggt cacggcctct atggacagtg gggcaggtgg gacctcaacg tcggtcacac 9780
caaagcagtc tctgtgtgat ggacagtggc actcggtggc agtcaccata aaacaacaca 9840
tcctgcacct ggaactggac acagacagta gctacacagc tggacagatc cccttcccac 9900
ctgccagcac tcaagagcca ctacaccttg gaggtgctcc agccaatttg acgacactga 9960
ggatccctgt gtggaaatca ttctttggct gtctgaggaa tattcatgtc aatcacatcc 10020
ctgtccctgt cactgaagcc ttggaagtcc aggggcctgt cagtctgaat ggttgtcctg 10080
accagtaacc caagcctatt tcacagcaag gaaattcacc ttcaaaagca ctgattaccc 10140
aatgcacctc cctccccagc tcgagatcat tcttcactca ggacacaaac cagacaggtt 10200
taatagcgaa tctaattttg aattctgacc atggataccc atcactttgg cattcagtgc 10260
tacatgtgta ttttatataa aaatcccatt tcttgaagat aaaaaaattg ttattcaaat 10320
tgttatgcac agaatgtttt tggtaatatt aatttccact aaaaaattaa atgtctttta 10380
agaaacattc ttttccactt gttaaaaaaa ttaaatatat tttaaagcac tttaagaata 10440
tgaaactttc atatatgtta aaggattata atttatggaa ttaaaaaatg cagtgtagtc 10500
cttaaaaaaa a                                                      10511
```

<210> 47
<211> 5175
<212> DNA
<213> Homo sapiens

<400> 47

```
gccccgagtg caatcgcggg aagccagggt ttccagctag gacacagcag gtcgtgatcc 60
gggtcgggac actgcctggc agaggctgcg agcatggggc cctggggctg gaaattgcgc 120
tggaccgtcg ccttgctcct cgccgcggcg gggactgcag tgggcgacag atgtgaaaga 180
aacgagttcc agtgccaaga cgggaaatgc atctcctaca agtgggtctg cgatggcagc 240
gctgagtgcc aggatggctc tgatgagtcc caggagacgt gcttgtctgt cacctgcaaa 300
tccggggact tcagctgtgg gggccgtgtc aaccgctgca ttcctcagtt ctggaggtgc 360
gatggccaag tggactgcga caacggctca gacgagcaag gctgtccccc caagacgtgc 420
tcccaggacg agtttcgctg ccacgatggg aagtgcatct ctcggcagtt cgtctgtgac 480
tcagaccggg actgcttgga cggctcagac gaggcctcct gcccggtgct cacctgtggt 540
cccgccagct tccagtgcaa cagctccacc tgcatccccc agctgtgggc ctgcgacaac 600
gaccccgact gcgaagatgg ctcggatgag tggccgcagc gctgtagggg tctttacgtg 660
ttccaagggg acagtagccc ctgctcggcc ttcgagttcc actgcctaag tggcgagtgc 720
atccactcca gctggcgctg tgatggtggc cccgactgca aggacaaatc tgacgaggaa 780
aactgcgctg tggccacctg tcgccctgac gaattccagt gctctgatgg aaactgcatc 840
catggcagcc ggcagtgtga ccgggaatat gactgcaagg acatgagcga tgaagttggc 900
tgcgttaatg tgacactctg cgagggaccc aacaagttca agtgtcacag cggcgaatgc 960
atcaccctgg acaaagtctg caacatggct agagactgcc gggactggtc agatgaaccc 1020
atcaaagagt gcgggaccaa cgaatgcttg gacaacaacg gcggctgttc ccacgtctgc 1080
aatgacctta agatcggcta cgagtgcctg tgccccgacg gcttccagct ggtggcccag 1140
cgaagatgcg aagatatcga tgagtgtcag gatcccgaca cctgcagcca gctctgcgtg 1200
aacctggagg gtggctacaa gtgccagtgt gaggaaggct tccagctgga cccccacacg 1260
aaggcctgca aggctgtggg ctccatcgcc tacctcttct tcaccaaccg gcacgaggtc 1320
aggaagatga cgctggaccg gagcgagtac accagcctca tccccaacct gaggaacgtg 1380
gtcgctctgg acacggaggt ggccagcaat agaatctact ggtctgacct gtcccagaga 1440
atgatctgca gcacccagct tgacagagcc cacggcgtct cttcctatga caccgtcatc 1500
agcagggaca tccaggcccc cgacgggctg gctgtggact ggatccacag caacatctac 1560
tggaccgact ctgtcctggg cactgtctct gttgcggata ccaagggcgt gaagaggaaa 1620
acgttattca gggagaacgg ctccaagcca agggccatcg tggtggatcc tgttcatggc 1680
ttcatgtact ggactgactg gggaactccc gccaagatca agaaaggggg cctgaatggt 1740
gtggacatct actcgctggt gactgaaaac attcagtggc ccaatggcat caccctagat 1800
ctcctcagtg gccgcctcta ctgggttgac tccaaacttc actccatctc aagcatcgat 1860
```

```
gtcaatgggg gcaaccggaa gaccatcttg gaggatgaaa agaggctggc ccacccccttc 1920
tccttggccg tctttgagga caaagtattt tggacagata tcatcaacga agccattttc 1980
agtgccaacc gcctcacagg ttccgatgtc aacttgttgg ctgaaaacct actgtcccca 2040
gaggatatgg tcctcttcca caacctcacc cagccaagag gagtgaactg gtgtgagagg 2100
accaccctga gcaatggcgg ctgccagtat ctgtgcctcc ctgccccgca gatcaacccc 2160
cactcgccca agtttacctg cgcctgcccg gacggcatgc tgctggccag ggacatgagg 2220
agctgcctca cagaggctga ggctgcagtg gccacccagg agacatccac cgtcaggcta 2280
aaggtcagct ccacagccgt aaggacacag cacacaacca cccggcctgt tcccgacacc 2340
tcccggctgc ctggggccac ccctgggctc accacggtgg agatagtgac aatgtctcac 2400
caagctctgg gcgacgttgc tggcagagga aatgagaaga agcccagtag cgtgagggct 2460
ctgtccattg tcctccccat cgtgctcctc gtcttccttt gcctgggggt cttccttcta 2520
tggaagaact ggcggcttaa gaacatcaac agcatcaact ttgacaaccc cgtctatcag 2580
aagaccacag aggatgaggt ccacatttgc cacaaccagg acggctacag ctacccctcg 2640
agacagatgg tcagtctgga ggatgacgtg gcgtgaacat ctgcctggag tcccgcccct 2700
gcccagaacc cttcctgaga cctcgccggc cttgttttat tcaaagacag agaagaccaa 2760
agcattgcct gccagagctt tgttttatat atttattcat ctgggaggca gaacaggctt 2820
cggacagtgc ccatgcaatg gcttgggttg ggattttggt ttcttccttt cctgtgaagg 2880
ataagagaaa caggcccggg gggaccagga tgacacctcc atttctctcc aggaagtttt 2940
gagtttctct ccaccgtgac acaatcctca aacatggaag atgaaagggc aggggatgtc 3000
aggcccagag aagcaagtgg ctttcaacac acaacagcag atggcaccaa cgggaccccc 3060
tggccctgcc tcatccacca atctctaagc caaaccccta aactcaggag tcaacgtgtt 3120
tacctcttct atgcaagcct tgctagacag ccaggttagc ctttgccctg tcacccccga 3180
atcatgaccc acccagtgtc tttcgaggtg ggtttgtacc ttccttaagc caggaaaggg 3240
attcatggcg tcggaaatga tctggctgaa tccgtggtgg caccgagacc aaactcattc 3300
accaaatgat gccacttccc agaggcagag cctgagtcac cggtcaccct taatatttat 3360
taagtgcctg agacacccgg ttaccttggc cgtgaggaca cgtggcctgc acccaggtgt 3420
ggctgtcagg acaccagcct ggtgcccatc ctcccgaccc ctacccactt ccattcccgt 3480
ggtctccttg cactttctca gttcagagtt gtacactgtg tacatttggc atttgtgtta 3540
ttattttgca ctgttttctg tcgtgtgtgt tgggatggga tcccaggcca gggaaagccc 3600
gtgtcaatga atgccgggga cagagagggg caggttgacc gggacttcaa agccgtgatc 3660
gtgaatatcg agaactgcca ttgtcgtctt tatgtccgcc cacctagtgc ttccacttct 3720
atgcaaatgc ctccaagcca ttcacttccc caatcttgtc gttgatgggt atgtgtttaa 3780
aacatgcacg gtgaggccgg gcgcagtggc ctcacgcctg taatcccagc actttgggag 3840
gccgaggcgg gtggatcatg aggtcaggag atcgagacca tcctggctaa caaggtgaaa 3900
ccccgtctct actaaaaata caaaaaatta gccgggcgcg gtggtgggca cctgtagtcc 3960
cagctactcg ggaggctgag gcaggagaat ggtgtgaacc cgggaagcgg agcttgcagt 4020
gagccgagat tgcgccactg cagtccgcag tctggcctgg gcgacagagc gagactccgt 4080
ctcaaaaaaa acaaaacaaa aaaaaaccat gcatggtgca tcagcagccc atggcctctg 4140
gccaggcatg gcgaggctga ggtgggagga tggtttgagc tcaggcattt gaggctgtcg 4200
tgagctatga ttatgccact gctttccagc ctgggcaaca tagtaagacc ccatctctta 4260
aaaaatgaat ttggccagac acaggtgcct cacgcctgta atcccagcac tttgggaggc 4320
tgagctggat cacttgagtt caggagttgg agaccaggcc tgagcaacaa agcgagatcc 4380
catctctaca aaaaccaaaa agttaaaaat cagctgggta tggtggcacg tgcctgtgat 4440
cccagctact tgggaggctg aggcaggagg atcgcctgag cccaggaggt ggaggttgca 4500
gtgagccatg atcgagccac tgcactccag cctgggcaac agatgaagac cctatttcag 4560
aaatacaact ataaaaaaaa taaataaatc ctccagtctg gatcgtttga cgggacttca 4620
ggttctttct gaaatcgccg tgttactgtt gcactgatgt ccggagagac agtgacagcc 4680
tccgtcagac tcccgcgtga agatgtcaca agggattggc aattgtcccc agggacaaaa 4740
cactgtgtcc cccccagtgc agggaaccgt gataagcctt tctggtttcg gagcacgtaa 4800
atgcgtccct gtacagatag tggggatttt ttgttatgtt tgcactttgt atattggttg 4860
aaactgttat cacttatata tatatataca cacatatata taaaatctat ttatttttgc 4920
aaaccctggt tgctgtattt gttcagtgac tattctcggg gccctgtgta gggggttatt 4980
gcctctgaaa tgcctcttct ttatgtacaa agattatttg cacgaactgg actgtgtgca 5040
acgcttttttg ggagaatgat gtccccgttg tatgtatgag tggcttctgg gagatgggtg 5100
tcactttttta aaccactgta tagaaggttt ttgtagcctg aatgtcttac tgtgatcaat 5160
taaatttctt aaatg                                                  5175
```

<210> 48
<211> 5061
<212> DNA
<213> Homo sapiens

<400> 48

```
gggcagggcg gctcggccgc ctctttctct cgctgccccc gcccctcctc ctcctccctc 60
tccccgagtg ctagcccggg cggccgccga gcttggcgct ctctcgcctc gctgcctcct 120
```

cccccgcgcc gcggaccctc tcccctccct tgcgttgccc ccctctcccg ccgcctcctc 180
ccgctctcct ctcgctctcc tcccgcctcc ttcttccagg cgcggccagc gggggcaaga 240
gcgaggtggc tgcgaggagg cgtcactgaa agccctgact tggggaaggc tgcgggctcg 300
gagccggaga cgccgagctg gggccgggtg agttgggaat cagactcttg agttgacagc 360
tggcctatgg tttctgcatt ttgttaagaa gcacctcatg caagcgaggg acctgggact 420
gctcacagcc aggtggcatt tttctgaagt tgttcattaa gacttcccag cattcctaca 480
gatataaata ggtgaggccg caggcggtgc tctgggtccg ggagcgctgt ccccagcatg 540
aacgcggccg gcggcgggag tgaatgactg cagctgcgac ttccttcccg ggccgcccga 600
gcctccttcc ccaccgactt tcttgttttg attaactccg tggactcctg actctttctt 660
cgcccggaac atcaatatgt gtcatgtcat tgtcacctgt cgctcgatgc tctggacctt 720
gctgagtatt gtggtggctt ttgccgagct cattgccttc atgagtgcag actggctgat 780
cgggaaagcg aggagccgcg gcggcgtgga gccggcgggc ccgggcgggg gctccccgga 840
gccctaccac cccaccctgg gcatctacgc ccgctgcatc cggaacccag ggggtgcagca 900
cttccagcgg gacacgctgt gcgggcccta cgccgagagc ttcggcgaga tcgccagcgg 960
cttctggcag gccacagcta ttttcctggc tgtgggaatc tttattctct gcatggtggc 1020
cttggtgtcc gtcttcacca tgtgtgtaca gagcatcatg aagaaaagca tcttcaatgt 1080
ctgtgggctg ttgcaaggaa ttgcaggtct attccttatc ctcggtttga tactctaccc 1140
tgctggctgg ggttgccaga aggccataga ctactgtgga cattatgcat ctgcctacaa 1200
acctggagac tgctccttgg gctgggcctt ttataccgcc attgggggca cagtcctcac 1260
tttcatctgt gctgtcttct ctgcacaagc agaaattgca acctctagtg acaaagtaca 1320
ggaagaaatt gaagagggga aaaatctgat ctgcctcctt tagtttggaa gagacaatgc 1380
cattttctcc cttgagtaat cttgtgaaac agtccacagt ttcatcattt gagtcaagtg 1440
gagaactaac ctttacctac caaagccacg ttccacggcc cgaggcttaa acaggaccaa 1500
tgagaggcca catccagcta cgcaaagtta ctggacatgc ggtctgcagt gcacattata 1560
aggaatggaa catgaaaata gtatataatc ctagacctgg agttgccaag ttctgtcaga 1620
ctccatctcc cccaggttca atgaaggata ataatctaaa tcattagggc agcagtttct 1680
ctggtaacgg aagagaccgt ccgccagatc tgcaggctgt ttctgctcca acactgcttg 1740
cttgtgagca tctctgcctc agaatggggt tttgggttgg agttcttgtt ttcctctgtt 1800
ctttcaagtt gtctccaacg aacagaaaac tataaactta ctggggacag gatgtgtgct 1860
aaagggcaca gcaagacact gtcttttgct tagctgacca aaggggtcag cagggatggc 1920
gtggagtcat gctgtggaac ttattctagg ctgaatccta gggtaaggtg gatcaactga 1980
actgtcactc cagagatttt agaaatttga gtaaagaaac aataaggacc tatacaatca 2040
tatgagaaca aaaatatgaa atcttgctag tgaagacgta ttttttcttc ttcccagcag 2100
ccaggctagc accagttctg gcccagtctc ctcttcttct ggagatcaca tgtttttctt 2160
ctaaggttag gattgtgctt tgactgcgaa aggaaacctc actgtttcct ccttccaggg 2220
actgaggtct ccaagctagc tgtggcttat gcagatgttc actgggagga cctgccagaa 2280
tctcggcact tggggggaga cctttactcc cagtttggtg accatgctgt agtcagctct 2340
atttccaatc ccgacagtag cagaatggca ttctacaaca aaaagaagct agttatggga 2400
gttaagtttt tgtagttact ggtgttgatc ctgaaagcag actgagataa cattaaattg 2460
ctgcaactga agaactgcag ccaagacctt aattccagga aagcacagag gacaaagtta 2520
attcaaaaag aggcgctaga tcaaggtcac agcactgcct acacctgttt acaaaaagaa 2580
tcaaatacca ctatgaataa ggattcaggg gtttttaatc tactttccat aaattaccaa 2640
tatcactgat tcaggaagat agtatctcag aatgaccaga gcagcacaga aacaagctac 2700
tctgacatta tgggagcttc aaaattgtat catgatacag aaacactcct tagcacttta 2760
agaaagtgag atggaactgc cagatttctg gaaggagaaa aagtgtaggt atttgggttc 2820
attaatctgc tcacttgagg actttgtttt gaaaaagtac cttctgtgga caaggtattg 2880
tgctaccagc tatacaaccc tgacttcaga gtttgcaacc ttgccctgag tgaatcatgt 2940
taaagctgtc tgagtctaaa gcaccgtatc ttggtgcaga acagataatt atacagagat 3000
ggaatgggac aaccgcagtt ttactacatt ctggtgtttg gcctatatga gaaaccatct 3060
tctcacagat taagggctaa gggcaaaagg ggtgggaggt gtggaactag ccttaatgag 3120
tttcccattc ctgaaccaaa attcaaagtg agtgagatgt aaatcctgtg attttggtga 3180
agaaaaaaac gggtatcttc atagcagcct aggaaacctt aaccatatct ctaacaccac 3240
acagaaagag gctggaggag ccactggaca aagcttctgt ctctgtgtgt acatttataa 3300
tgttctaacc aagtctcaaa ccttgatgaa aacacaaaa tttttccata aacttatcag 3360
aagactcact tttctttctt tcttggatag agaaaccatt ttctgacact aggtttacaa 3420
tctcagtgtc cttacaagtt aagtcctaag ctcacaggat cctccgagca tgtccatcac 3480
ctgctctttg gctaaggtgg cagtgtacct ctagatcaac ctgggaacag tcacaaggga 3540
gtgtgacttc ttggccataa taaactcact cgatagtgtt tatgttatta atctgaatgc 3600
aacagaagac aaaagcacag gcatgcacac acacagaacc ccaaaccact aaaaactacc 3660
taaacactga cttagtaaat agtaaaaagg taatgttggg acttttaaac cttgaatcca 3720
ttagccaggc ttgggatgaa aggaccatct aaaatcatgc tagtctaaac catgctcttc 3780
cacacagctg tttaaaaacc actgggtatg aggaatatgc tagaaagaaa tgttaaaaat 3840
agattgttgg ctcacactta tttttctaat aaataggacc attattacta ccaggaaagt 3900
cttatttatt ttgcctgaaa ttggcttaaa gaaagtctca tgacgggatg ggatgggctg 3960
cgcttctcaa tgaactctga ggcagaaata tttgccttgg attctgtgga ttctttaaac 4020

ctgtgtgcta ataattcaaa caatgttgca ttaattgtat aagggttttt gtatagtttt 4080
caaacatctg tggtgtaatg atctttgtta aacatatatt ctgtaaagtg ccatagtctt 4140
tttttatgtg tagcatattt aaaaatatat atgtatatta tacatacaca agtttgtgtg 4200
aaagatgtgc aataacaaag gtgtatgtat gttttgttgt tttgttttgg aaactggaca 4260
ggagtcaaaa cagggatgtt tgtttctgtt ttggcaagga gagttccaca tttttgcctt 4320
catggcttat tcagtaaccc ataattttaa tgctacacaa atcttatgtg aagaaaagac 4380
tggtatgaaa tcatttttc ctgggtctaa aataatcgct agtgttatgt caaagttaag 4440
cccgcacgcc aggcccagtt aatgctagtc tttcatgtga aatgtgaagc tgccatgttg 4500
ccttttctct tagtaggata actagtagct ggtacataat cactgaggag ctatttctta 4560
acatgctttt atagaccatg ctaatgctag accagtattt aagggctaat ctcacacctc 4620
cttagctgta agagtctggc ttagaacaga cctctctgtg caataacttg tggccactgg 4680
aaatccctgg gccggcattt gtattggggt tgcaatgact cccaagggcc aaaagagtta 4740
aaggcacgac tgggatttct tctgagactg tggtgaaact ccttccaagg ctgagggggt 4800
cagtaggtgc tctggaggga ctcggcacca cttgatattc aacagccact tgagccaaat 4860
ataaaattgt atttacagct gatggactca atttgagcct tcaaacttgt agttatccta 4920
ttatattgta aactaataca ttgtctagca ttgatttggt tcctgtgcat atgtattttc 4980
actatgtgct cccctcccca gatcttaatt aaaccagatt ttgcaattca ttcttattct 5040
ttcaaaaaaa aaaaaaaaaa a 5061

<210> 49
<211> 3868
<212> DNA
<213> Homo sapiens

<400> 49

```
atgaacctct gaaaactgcc ggcatctgag gtttcctcca aggccctctg aagtgcagcc 60
cataatgaag gtcttggcgg caggagttgt gcccctgctg ttggttctgc actggaaaca 120
tggggcgggg agccccctcc ccatcacccc tgtcaacgcc acctgtgcca tacgccaccc 180
atgtcacaac aacctcatga accagatcag gagccaactg gcacagctca atggcagtgc 240
caatgccctc tttattctct attacacagc ccagggggag ccgttcccca acaacctgga 300
caagctatgt ggccccaacg tgacggactt cccgcccttc cacgccaacg gcacggagaa 360
ggccaagctg gtggagctgt accgcatagt cgtgtacctt ggcacctccc tgggcaacat 420
cacccgggac cagaagatcc tcaaccccag tgccctcagc ctccacagca agctcaacgc 480
caccgccgac atcctgcgag gcctccttag caacgtgctg tgccgcctgt gcagcaagta 540
ccacgtgggc catgtggacg tgacctacgg ccctgacacc tcgggtaagg atgtcttcca 600
gaagaagaag ctgggctgtc aactcctggg gaagtataag cagatcatcg ccgtgttggc 660
ccaggccttc tagcaggagg tcttgaagtg tgctgtgaac cgagggatct caggagttgg 720
gtccagatgt gggggcctgt ccaagggtgg ctggggccca gggcatcgct aaacccaaat 780
gggggctgct ggcagacccc gagggtgcct ggccagtcca ctccactctg ggctgggctg 840
tgatgaagct gagcagagtg gaaacttcca tagggaggga gctagaagaa ggtgcccctt 900
cctctgggag attgtggact ggggagcgtg ggctggactt ctgcctctac ttgtcccttt 960
ggccccttgc tcactttgtg cagtgaacaa actacacaag tcatctacaa gagccctgac 1020
cacagggtga gacagcaggg cccaggggag tggaccagcc cccagcaaat tatcaccatc 1080
tgtgcctttg ctgcccctta ggttgggact taggtgggcc agaggggcta ggatcccaaa 1140
ggactccttg tcccctagaa gtttgatgag tggaagatag agaggggcct ctgggatgga 1200
aggctgtctt cttttgagga tgatcagaga acttgggcat aggaacaatc tggcagaagt 1260
ttccagaagg aggtcacttg gcattcaggc tcttggggag gcagagaagc caccttcagg 1320
cctgggaagg aagacactgg gaggaggaga ggcctggaaa gctttggtag gttcttcgtt 1380
ctcttccccg tgatcttccc tgcagcctgg gatggccagg gtctgatggc tggacctgca 1440
gcaggggttt gtggaggtgg gtagggcagg ggcaggttgc taagtcaggt gcagaggttc 1500
tgagggaccc aggctcttcc tctgggtaaa ggtctgtaag aaggggctgg ggtagctcag 1560
agtagcagct cacatctgag gccctgggag gtcttgtgag gtcacacaga ggtacttgag 1620
ggggactgga ggccgtctct ggtccccagg gcaagggaac agcagaactt agggtcaggg 1680
tctcagggaa ccctgagctc caagcgtgct gtgcgtctga cctggcatga tttctattta 1740
ttatgatatc ctatttatat taacttattg gtgctttcag tggccaagtt aattcccctt 1800
tccctggtcc ctactcaaca aaatatgatg atggctcccg acacaagcgc cagggccagg 1860
gcttagcagg gcctggtctg gaagtcgaca atgttacaag tggaataagc ttacgggtga 1920
agctcagaga agggtcggat ctgagagaat ggggaggcct gagtgggagt gggggccctt 1980
gctccacccc catcccctac tgtgacttgc tttagcgtgt cagggtccag gctgcagggg 2040
ctgggccaat ttgtggagag gccgggtgcc tttctgtctt gcttccaggg ggctggttca 2100
cactgttctt gggcgcccca gcattgtgtt gtgaggcgca ctgttcctgg cagatattgt 2160
gccccctgga gcagtgggca agacagtcct tgtggcccac cctgtccttg tttctgtgtc 2220
cccatgctgc ctctgaaata gcgccctgga acaaccctgc ccctgcaccc agcatgctcc 2280
gacacagcag ggaagctcct cctgtggccc ggacacccat agacggtgcg gggggcctgg 2340
ctgggccaga ccccaggaag gtggggtaga ctggggggat cagctgccca ttgctcccaa 2400
```

```
gaggaggaga gggaggctgc agacgcctgg gactcagacc aggaagctgt gggccctcct 2460
gctccacccc catcccactc ccacccatgt ctgggctccc aggcagggaa cccgatctct 2520
tcctttgtgc tggggccagg cgagtggaga aacgccctcc agtctgagag caggggaggg 2580
aaggaggcag cagagttggg gcagctgctc agagcagtgt tctggcttct tctcaaaccc 2640
tgagcgggct gccggcctcc aagttcctcc gacaagatga tggtactaat tatggtactt 2700
ttcactcact ttgcaccttt ccctgtcgct ctctaagcac tttacctgga tggcgcgtgg 2760
gcagtgtgca ggcaggtcct gaggcctggg gttggggtgg agggtgcggc ccggagttgt 2820
ccatctgtcc atcccaacag caagacgagg atgtggctgt tgagatgtgg gccacactca 2880
cccttgtcca ggatgcaggg actgccttct ccttcctgct tcatccggct tagcttgggg 2940
ctggctgcat tcccccagga tgggcttcga gaaagacaaa cttgtctgga aaccagagtt 3000
gctgattcca cccgggggc ccggctgact cgcccatcac ctcatctccc tgtggacttg 3060
ggagctctgt gccaggccca ccttgcggcc ctggctctga gtcgctctcc cacccagcct 3120
ggacttggcc ccatgggacc catcctcagt gctccctcca gatcccgtcc ggcagcttgg 3180
cgtccaccct gcacagcatc actgaatcac agagcctttg cgtgaaacag ctctgccagg 3240
ccgggagctg ggtttctctt ccctttttat ctgctggtgt ggaccacacc tgggcctggc 3300
cggaggaaga gagagtttac caagagagat gtctccgggc ccttatttat tatttaaaca 3360
tttttttaaa aagcactgct agtttacttg tctctcctcc ccatcgtccc catcgtcctc 3420
cttgtccctg acttggggca cttccaccct gacccagcca gtccagctct gccttgccgg 3480
ctctccagag tagacatagt gtgtggggtt ggagctctgg cacccgggga ggtagcattt 3540
ccctgcagat ggtacagatg ttcctgcctt agagtcatct ctagttcccc acctcaatcc 3600
cggcatccag ccttcagtcc cgcccacgtg ctagctccgt gggcccaccg tgcggcctta 3660
gaggtttccc tccttccttt ccactgaaaa gcacatggcc ttgggtgaca aattcctctt 3720
tgatgaatgt accctgtggg gatgtttcat actgacagat tatttttatt tattcaatgt 3780
catatttaaa atatttattt tttataccaa atgaatcact ttttttttta agaaaaaaaa 3840
gagaaatgaa taaagaatct actcttcg                                    3868
```

<210> 50
<211> 2575
<212> DNA
<213> Homo sapiens

<400> 50

```
aaagattcct tagctggaaa ggtctaggag gagactcccg ctctgctccc tcctgcacca 60
gcgctgtgcc ccccgccggc caggcagagc catccgatgc cgctgggccg cccactgagg 120
atctgctggc tgcagcgagt ggaaggacct gctcggctgg aacgtttttt ttttttttcc 180
ctcccaggcg acgtccgatg ggtgtgtcgg gcaggaggtg atatttgaca ggctgcgcgc 240
gggcgagctg ccgcggagca cccggcaggg gctgacagca tggcctcgcc cgacccgccc 300
gccaccagct acgccccgtc cgacgtgccc tcgggggtcg cgctgttcct caccatccct 360
ttcgccttct tcctgcccga gctgatattt gggttcttgg tctggaccat ggtagccgcc 420
acccacatag tatacccctt gctgcaagga tgggtgatgt atgtctcgct cacctcgttt 480
ctcatctcct tgatgttcct gttgtcttac ttgtttggat tttacaaaag atttgaatcc 540
tggagagttc tggacagcct gtaccacggg accactggca tcctgtacat gagcgctgcc 600
gtcctacaag tacatgccac gattgtttct gagaaactgc tggacccaag aatttactac 660
attaattcgg cagcctcgtt cttcgccttc atcgccacgc tgctctacat tctccatgcc 720
ttcagcatct attaccactg atgcacaggc gccaggccaa gggggaaatg ctctttgaaa 780
gctccaatta ttggtcccca aaagcagctt ccaacgtttg ccatctggat gacaaacgga 840
agatccacta aaacgtccac gggattaaca gaacgtcctt gcagactgag cgatgacacc 900
acactttgtt tggacattta aattcactct gctgaatagg aggaagcttt tctttttcct 960
gggaaaacaa ctgtctcttg gaattatctg accatgaact tgctcttcta gacaactcac 1020
atcaaagccc tcactccact aatggagaat cctagcccca ctaatgccaa gtctgtttgg 1080
ggattttgcc tcagctatgg gcttccctag agtaggtcta ggggaatact cagtctgatc 1140
tttttttTgt ttgttttatt ttgttttttt tgagacggag tctcgctctt cctccaaggc 1200
tggagtgcag tgacgcgatc tccactcact gcaggctccg cctcccgggt tcccgccatt 1260
ctcctgcctc agcctcccga gtagccggga ctacaggcgc ccaccaccat gcccggctaa 1320
tttagttgta tttttagtag agatggggtt tcaccgtatt agccaggatg gtctcgatct 1380
cctgacctcg tgatccgccc gcctcggcct cccaaagtgc tgggattaca ggcgtgagcc 1440
accgcgcccg gcctgattct cttaaaattg aagaggtgct gccaaggcct tcagatctaa 1500
cgcagatgca tagaccttgt tcctggtact tgttcagcct gtgctgggga gccgtggtcc 1560
cgagttccct gggaggctga cagggtcaag ccaccctgcc caccaccctc ccacttcccc 1620
tcccctttcc tctccagcat taggattcaa gggaaatctg catgaagcca attttgaggg 1680
tagacgtgtg gggaaaataa atcattatac agtaagacct ggggcttgag gggtggggaa 1740
tggggaggga agggcatagc ctgctcctcc atgagtctga catctcggaa actgagcagc 1800
tgccggacgc ctgggtcagg aatccaagac cccacctctt aaggactggt tcctcagaaa 1860
gcaccctcag ggaaaaaggt gaaaacatta catccgtgga ttctcctgcc acaaccgcat 1920
tggaagaaaa ggctgccgca acatctcagc gaggagtgaa ggacccatgt cccaggaacc 1980

gcgctgcgcc acctgcactc acccccctca cattctctta agcacccggt ggccctccga 2040
ggcctggcgg aatggtggtg cccacggggt tgggcaaggg ctcaccagga cctcaacggg 2100
caaagttgtg cacactaaaa tatcaaatca aggtgcttgg ttttaaagta aatgtttttc 2160
taaagaaagc tgtgctcttc tgttgaccca gacgaatagg gcacagccct gtaactgcac 2220
gtgccttctg tcattgggaa tgaaataaat tattacgaga aagggacttg tcctaactgg 2280
tttgaggcct tacagttttg tatctacatt tttcccctcc tggggtttgc ggggacaggg 2340
acagaactac aggagtcatg ggaaagaaaa ttctggcttc actactgctc actgctcact 2400
ttctgatcac tctgatactt tttttttttt tttttttttg caacctgata ccttgaaaag 2460
cttctatgtg tctctccttt tgttgcctgg cagctgtcta ggatgatcac tgattactat 2520
ttactaagta gccacatgca aataaaagtt gtttggtaaa aaggaaaaaa aaaaa      2575
```

<210> 51
<211> 758
<212> DNA
<213> Homo sapiens

<400> 51

acggccacag tcggattcgc tgccgcagca gccgccaccc ccaggagccg ccgggaccct 60
cgcgtcgtcg ccgccgcggc ccagatcccc acaccatgcc gtcggagaag accttcaagc 120
agcggcgcac cttcgaacaa agagtagaag atgtccgact tattcgagag cagcatccaa 180
ccaaaatccc ggtgataata gaacgataca agggtgagaa gcagcttcct gttctggata 240
aaacaaagtt ccttgtacct gaccatgtca acatgagtga gctcatcaag ataattagaa 300
ggcgcttaca gctcaatgct aatcaggcct tcttcctgtt ggtgaacgga cacagcatgg 360
tcagcgtctc cacaccaatc tcagaggtgt atgagagtga gaaagatgaa gatggattcc 420
tgtacatggt ctgtgcctcc caggagacgt tcgggatgaa attgtcagtg taaaaccaga 480
aaaaatgcat ctcttctaga atttttttaaa cccttaccaa ggaaaaaaaa agggatgtta 540
ccaactgaga tcgatcagtt catctaatca cagatcatca aacagtagtg ttcccaccta 600
ggagtgttag gaagttgtgt ttgtgtttca agcagaaaaa ctgagctcca agtgagcaca 660
ttcagctttg gaaactatat tatttaatgt aggctagctt gttttcaaat tttaaaagtt 720
taaaaataaa atactttgca ttctaaaaaa aaaaaaaa 758

<210> 52
<211> 476
<212> DNA
<213> Homo sapiens

<400> 52

aaagaagaaa aacatgtgaa tacgatacag gaccaggccc cacatcctga taacatgcag 60
tgcctcttca ccagccatgc cttttgcctc accctttctg ttgctatgtt ctctatcctg 120
cgttggacaa agagtgacag gcgaggtttt ctaggcatct ccatcaaaat gagaaggaaa 180
aacctccatc tctcagcact tattaaccta aaagataagt tgcactttct actttttctta 240
ggtggggatt acatgagccc caccagtcat cacggacaca tcgtttgtac ctatcagatg 300
tatcagtaga gatggggaca ttaatttgtg taggggtgtc agcagtgctc tggggagaaa 360
gagatattgg catacacagt ggtctggagc atgtcttcac aaaatagtgt ttgtggggag 420
ccagagattc ctaaaagaac agtgaatagc acagtctcat taaaaaaaaa aaaaaa 476

<210> 53
<211> 3558
<212> DNA
<213> Homo sapiens

<400> 53

cagaccccag ttcgccgact aagcagaaga aagatcaaaa accggaaaag aggagaagag 60
caaacaggca ctttgaggaa caatcccctt taactccaag ccgacagcgg tctaggaatt 120
caagttcagt gcctaccgaa gacaaaggcg ccccgaggga gtggcggtgc gaccccaggg 180
cgtgggcccg gccgcggagc ccacactgcc cggctgaccc ggtggtctcg gaccatgtct 240
cccgccccaa gacccccccg ttgtctcctg ctccccctgc tcacgctcgg caccgcgctc 300
gcctccctcg gctcggccca aagcagcagc ttcagccccg aagcctggct acagcaatat 360
ggctacctgc ctcccgggga cctacgtacc cacacacagc gctcacccca gtcactctca 420
gcggccatcg ctgccatgca gaagttttac ggcttgcaag taacaggcaa agctgatgca 480
gacaccatga aggccatgag gcgcccccga tgtggtgttc cagacaagtt tggggctgag 540
atcaaggcca atgttcgaag gaagcgctac gccatccagg gtctcaaatg gcaacataat 600
gaaatcactt tctgcatcca gaattacacc cccaaggtgg gcgagtatgc cacatacgag 660
gccattcgca aggcgttccg cgtgtgggag agtgccacac cactgcgctt ccgcgaggtg 720
ccctatgcct acatccgtga gggccatgag aagcaggccg acatcatgat cttctttgcc 780

gagggcttcc atggcgacag cacgcccttc gatggtgagg gcggcttcct ggcccatgcc 840
tacttcccag gccccaacat tggaggagac acccactttg actctgccga gccttggact 900
gtcaggaatg aggatctgaa tggaaatgac atcttcctgg tggctgtgca cgagctgggc 960
catgccctgg ggctcgagca ttccagtgac ccctcggcca tcatggcacc cttttaccag 1020
tggatggaca cggagaattt tgtgctgccc gatgatgacc gccggggcat ccagcaactt 1080
tatgggggtg agtcagggtt ccccaccaag atgccccctc aacccaggac tacctcccgg 1140
ccttctgttc ctgataaacc caaaaacccc acctatgggc ccaacatctg tgacgggaac 1200
tttgacaccg tggccatgct ccgaggggag atgtttgtct tcaaggagcg ctggttctgg 1260
cgggtgagga ataaccaagt gatggatgga tacccaatgc ccattggcca gttctggcgg 1320
ggcctgcctg cgtccatcaa cactgcctac gagaggaagg atggcaaatt cgtcttcttc 1380
aaaggagaca agcattgggt gtttgatgag gcgtccctgg aacctggcta ccccaagcac 1440
attaaggagc tgggccgagg gctgcctacc gacaagattg atgctgctct cttctggatg 1500
cccaatggaa agacctactt cttccgtgga aacaagtact accgtttcaa cgaagagctc 1560
agggcagtgg atagcgagta ccccaagaac atcaaagtct gggaagggat ccctgagtct 1620
cccagagggt cattcatggg cagcgatgaa gtcttcactt acttctacaa ggggaacaaa 1680
tactggaaat tcaacaacca gaagctgaag gtagaaccgg gctaccccaa gtcagccctg 1740
agggactgga tgggctgccc atcgggaggc cggccggatg aggggactga ggaggagacg 1800
gaggtgatca tcattgaggt ggacgaggag ggcggcgggg cggtgagcgc ggctgccgtg 1860
gtgctgcccg tgctgctgct gctcctggtg ctggcggtgg gccttgcagt cttcttcttc 1920
agacgccatg ggacccccag gcgactgctc tactgccagc gttccctgct ggacaaggtc 1980
tgacgcccac cgccggcccg cccactccta ccacaaggac tttgcctctg aaggccagtg 2040
gcagcaggtg gtggtgggtg ggctgctccc atcgtcccga gccccctccc cgcagcctcc 2100
ttgcttctct ctgtcccctg gctggcctcc ttcaccctga ccgcctccct ccctcctgcc 2160
ccggcattgc atcttcccta gataggtccc ctgagggctg agtgggaggg cggccctttc 2220
cagcctctgc ccctcagggg aaccctgtag ctttgtgtct gtccagcccc atctgaatgt 2280
gttgggggct ctgcacttga aggcaggacc ctcagacctc gctggtaaag gtcaaatggg 2340
gtcatctgct ccttttccat cccctgacat accttaacct ctgaactctg acctcaggag 2400
gctctgggca ctccagccct gaaagcccca ggtgtaccca attggcagcc tctcactact 2460
ctttctggct aaaaggaatc taatcttgtt gagggtagag accctgagac agtgtgaggg 2520
ggtggggact gccaagccac cctaagacct tgggaggaaa actcagagag ggtcttcgtt 2580
gctcagtcag tcaagttcct cggagatctg cctctgcctc acctacccca gggaacttcc 2640
aaggaaggag cctgagccac tggggactaa gtgggcagaa gaaacccttg gcagccctgt 2700
gcctctcgaa tgttagcctt ggatggggct ttcacagtta gaagagctga aaccaggggt 2760
gcagctgtca ggtagggtgg ggccggtggg agaggcccgg gtcagagccc tgggggtgag 2820
cctgaaggcc acagagaaag aaccttgccc aaactcaggc agctggggct gaggcccaaa 2880
ggcagaacag ccagaggggg caggagggga ccaaaaagga aaatgaggac gtgcagcagc 2940
attggaaggc tggggccggg caggccaggc caagccaagc agggggccac agggtgggct 3000
gtggagctct caggaagggc cctgaggaag gcacacttgc tcctgttggt ccctgtcctt 3060
gctgcccagg cagcgtggag gggaagggta gggcagccag agaaaggagc agagaaggca 3120
cacaaacgag gaatgagggg cttcacgaga ggccacaggg cctggctggc cacgctgtcc 3180
cggcctgctc accatctcag tgagggcag gagctggggc tcgcttaggc tgggtccacg 3240
cttccctggt gccagcaccc ctcaagcctg tctcaccagt ggcctgccct ctcgctcccc 3300
cacccagccc acccattgaa gtctccttgg gccaccaaag gtggtggcca tggtaccggg 3360
gacttgggag agtgagaccc agtggaggga gcaagaggag agggatgtcg gggggtggg 3420
gcacggggta ggggaaatgg ggtgaacggt gctggcagtt cggctagatt tctgtcttgt 3480
ttgttttttt gttttgttta atgtatattt ttattataat tattatatat gaattccaaa 3540
aaaaaaaaaa aaaaaaa 3558

<210> 54
<211> 5587
<212> DNA
<213> Homo sapiens

<400> 54

```
gagtcgggga taaacactcc gcggcggtgg cggctgctgc tctgctccgg gttctgtcac 60
tgtgtcggcg gtgcccagct cactggcccc ctccctctct tgtcgagcgt ggttgccaga 120
gaggctccct cagccctgct ccgcggggtc cacagcgggc tccacagcgg gctccatagc 180
gggctccaca gcggtccggc ggcggcagcg agcccgtggg cagtgggggt tggtcccgtg 240
gctccggccc ccggtgcaga atggcggcgg cggttcggat gaacatccag atgctgctgg 300
aggcggccga ctatctggag cggcgggaga gagaagctga acatggttat gcctccatgt 360
taccatacaa taacaaggac agagatgcct taaaacggag gaacaaatcc aaaaagaata 420
acagcagtag cagatcaact cacaatgaaa tggagaagaa tagacgggct catcttcgct 480
tgtgcctgga gaagttgaag gggctggtgc cacttggacc cgaatcaagt cgacacacta 540
cgttgagttt attaacaaaa gccaaattgc acataaagaa acttgaagat tgtgacagaa 600
aagccgttca ccaaatcgac cagcttcagc gagagcagcg acacctgaag aggcagctgg 660
```

agaagctggg cattgagagg atccggatgg acagcatcgg ctccaccgtc tcctcggagc 720
gctccgactc cgacagggaa gaaatcgacg ttgacgtgga gagcacggac tatctcacag 780
gtgatctgga ctggagcagc agcagtgtga gcgactctga cgagcggggc agcatgcaga 840
gcctcggcag tgatgagggc tattccagca ccagcatcaa gagaataaag ctgcaggaca 900
gtcacaaggc gtgtcttggt ctctaagaga gtgggcactg cggctgtctc cttgaaggtt 960
ctccctgttg gttctgatta ggtaacgtat tggacctgcc cacaactccc ttgcacgtaa 1020
acttcagtgt cccaccttga ccaaaatcag ctttgtaact gttttcaagg aggtgcttag 1080
gattgtgggt ttctgattgc atccactagc ttctcttttt ctcgccataa aaatttgtct 1140
ctgagagact atacattcca atcaatttga agcatccaag aattcttaga ccgaataagc 1200
aatgtccaca tctcagcaat ccccctcttt gctctcctcg tgtcctctcc cagacctttc 1260
ttccagtttt ctggcttact gtgttacttg cctaggagga atgtccaagt gtgtcttgta 1320
cttaggaaac tgaaggaaac aaactttga aattgagatc ctgatctcag aactccaaag 1380
taagctttga aagcagcatt taagagcact taaccatgga cctcaccacc agtgaggaag 1440
tcaggaatac ctctagaaaa cacgcccttc acaccgcgca tgctcattcc cccgacgcgc 1500
cgcgtgtgga tgggagcagt atttctcact ttaaaatgga caccttgata gtgtgtctct 1560
ggggttgcac agctcatcaa agttccaaat tgtttgttct cacaaacaaa acggtacaat 1620
ctatttttgt gcgatgttct tgggacctcg ctgtgttctg ctatctcgag gcacattctc 1680
ccctccaact ttgttaatgc tacttgtctc tggaacattt ttttttccta cctcagagat 1740
aaatgagatc tccatttccc acttaacaca gtgattatcc ttttttcccc ccccaaaata 1800
gtgacatttg gtccaattat aatctatttt cctattgaac tttcagcaat aactgagctg 1860
tggcactagc agagctagta gccattatca gtgaaagaaa aagtccacat ttctactctc 1920
tgttgcaggt gaacaggaga ggatggtaca ctgttactag aactcctctc cttattgatt 1980
atttttggac acacccagaa acttctttat ggaggctttt gggttgatag tttaaaaggc 2040
tgatttttct ttttggttat gatttctccc ttaagtggtc tccgactttg tagcattttt 2100
atttaagcta aaacagagca catgtatatg tacataagac acattaaatc tataaatact 2160
atttattcat tttatataaa ctaatgtaat ggaaaacaaa ttcttatgac tttgtggttt 2220
tatagatgtt ctagaaactt tgtatgtagg tatctacaaa attagttcat tcccctgaat 2280
atttttgcat tcatattttt gaggtcttga tgttttcagc ctctggcgaa tctttttcat 2340
tgaatttgaa ccatttgtaa aatctgtgat gctgaagcag agtgtgtcac aaagtgatga 2400
gaacattact aaaatccacg gacgcactgc gacctaaggg ctcaacggct gactcggcag 2460
cgggcagcca ccccacgctc ccctgcggtc actcgcacac cacagcctga agctccccca 2520
gcgcctgcac ctcgcacaca gctaaggtca aagttcaaac gcactccaca cggaagctca 2580
ttctataccc gaagagcagt ctcagaaagc aagattactt ttgtgttttt taaaaaatga 2640
ttctttaatg tatttttcta aacattctga ttggaagtag tggattccta aatgattcca 2700
aagtcacctg taattcttct gtttttgttt tgttctgtct tttcttcatt ttggctttgg 2760
gtgggggag gggcaggtga cacaaaggat tttttttttt ttttttaat ttttggaatc 2820
ttttccaata accagctaaa gatttgcact gaaatacaac ttgtatgcct tttgcatttt 2880
taaagcctgc ttcctggatt taagcagagt gatagtgttc aaagagccag ttcagcctgt 2940
aacatatttg aaaaagatat gtctgcactt tgaggtccct tttgaatgcc attcactaga 3000
cctctcaagc attttgtttc attgctacat ccaagcgcct cacaagtcca caatgcggga 3060
cagcatcaaa agctcaagac tttggaaaaa gcttgtgggc ttgcactggg ggagggaagg 3120
gaacaaaatt tgtgtacttc tttgtttaat ttagaaataa ggcatccaag agatgccatt 3180
attttctgtg tttcaattgt tgtgcctttg agttaaactg catttttgtc ttttggttga 3240
aatctgaaat gtactgtccc aatataaaac agtaattatt tgacctttgc actgtttgtc 3300
tggtcctttt cagtttgatt gcatataaat gtggaacttg atagatctct atatttttaa 3360
tgcacttgtg ataaactggc agcagggtta gacattactt tcaaagcttg aggtagaccg 3420
agtcagcatg ctagacaggc ttctctctct aaccaaaact gtaatcttca ggaccagcaa 3480
actcagccca aggcagctaa tccccccaac cccatcctcc gcgccccgtg cggctgatcg 3540
gcagccctga ttcgccaatt tgtcctctct cattcactga tccaccagcc tgactgctaa 3600
gagctatagt ctttttagtt gttttgtctt tttaagcaag atgaaaacct ttctattagg 3660
gattttgggg ttgggagggg atgggcagag atataaaccc cagcctttaa gactttgaca 3720
attgtacgta aatacagatg tgtataaata taggcacatg catattttta tgtgaaagtt 3780
gattttaaaa aactaaaaaa atctaaactg cactcttatt gataccatca taacgcaagt 3840
gggaaaaata agagtacgca gtctaattta atttcatgca gtgggaaaat atatatgtgt 3900
gcttctgtaa catcctgaaa acatagcttt ccatcccctg ttggctttga atggtgggcc 3960
gagcacccag gtcgtctgta ttttggtttt cttttgctaa gcagagatct tgaattcttc 4020
aaggtgctga tagcaactgc tggctccttt ctgtagtcac acacctaatg ctagtttagt 4080
gattcaaaat gcatcacatt tttaggcagg acctagattt tccctgtcaa cctaagatga 4140
aaataatttc agtgttgatt cagaactgaa cattaagtag gccctcgtcc tgcagttggc 4200
cacttgagtg ttttgttttg tttttatttt ttaaggtggg cattttcctt taacctctac 4260
tttttcaaaa gcaacaaagg ggcctcaacc tgagtttcct atgggcctct cttctgcatc 4320
cccaaagcgg ccaagagcaa atcctggggg ataagaaaaa agtgtaaact aggtaggatt 4380
gtgatgctca aaataaccat ctagcaatat cttggagctt gagaatagat tttgtgggct 4440
tatttcttct tgcctcttcc ccatcctttc aagagagaac ttatttttga aaagtatcta 4500
tatatacaca cacacacaca cacacacata ttattattta ggtttttata ccatactgta 4560

```
ttggcgagaa taccactatc attgtccttt acagtctatt tcttccccca agtcttggtc 4620
tttttttatt ttctattttt tcatgaacca cacaggagac tttaacatcc tggtcttttc 4680
tgtttcttct ttgtttcccc aagtttgtct gtccccctttt gccttccctg agtgttgaac 4740
atcaggtagt aaaaggctaa acgcaatttc ttgcatgtca atctattctt tttctatgtt 4800
tgactctgat gcagtgtgtt tagcgtgtct agtagctggc tactcctatt taaaaactct 4860
tcctggtaga agacaaccca aagacccttt tcgatgaggt ggtttctcat tctacatcct 4920
ctgatctcta tagactgtag gatgctttgc tttcaaagat aactgggtta gagggtgggg 4980
tgtgcaatag gtgatttatc atggtttttt tcattatcaa tattacatgg atgattttct 5040
cagattcttc tgaaagaaga aattgacagg cactgctaga ttcagctatt gaatggctga 5100
agagattgag tatttgacct tctctcaaaa tcataaagtg agaattcata aggcacccaa 5160
tgttaagatt tatccagatt tttacatttt gatttcttct ctctgtgggg tggcaagttg 5220
agggagcatt cttcatttta gcttttacct gacaaccaaa cttgccttta ccccatccct 5280
agaattggtg ctcttggaat attgctgtta ccatcatttt tgggggggcca tcttcctaat 5340
gctacacaca gcctgacagg ggagcagcag atgaaagggt atgctattct gtttccagat 5400
gtttctttat gtaaatatga cgccaatgta aatcctgtgt caagatcata gagaatggtg 5460
ctttttacta cagttagcac atgcattttt agaaactact acatgtttta gagaatcttt 5520
gctgtgtata tgtaaactgt attgttcaac tgttaacaaa taataaatta tttcattatt 5580
aaagaaa                                                           5587
```

<210> 55
<211> 9758
<212> DNA
<213> Homo sapiens

<400> 55

agatgcaggg aagttttgcc tcttcctgaa aattatatta ttagcttttt aaaaatcagg 60
atgactgcta gttttgttta aagtatttgt tctggaaata ctaaagttgg agtctaccag 120
actgaggtta gaagcatttt ctttggcagc aagaagataa ttttatagaa gccatgcctg 180
ttcttggggt tgcctcaaaa ctgaggcagc cagctgttgg gtcaaagcct gtgcatactg 240
ctcttccgat accaaatctt ggcactactg ggtcacagca ctgttcttca agacctttgg 300
aacttactga aacagagagc tccatgcttt cttgtcagct tgcgttaaaa tcaacctgtg 360
aatttggaga gaagaaaccc ctccaaggaa aagccaagga gaaagaagac agcaagattt 420
acactgactg ggccaaccac tacctagcaa aatcaggcca caagcggctg atcaaggact 480
tgcaacaaga cattgcagat ggagtactcc tagcagaaat catccagatt attgcaaatg 540
aaaaagttga agatatcaat ggatgtccta gaagtcagtc tcagatgatt gaaaatgttg 600
atgtctgcct tagttttcta gcagccagag gggtaaatgt tcaaggtcta tctgctgaag 660
aaataagaaa tggaaactta aaagccattc tagggctgtt tttcagttta tctcgctaca 720
agcagcaaca acaccatcaa caacagtact atcagtcctt ggtggaactt cagcagcgag 780
ttactcacgc ttcccctcca tcggaagcca gccaggccaa aacccagcaa gatatgcagt 840
ccagtctggc agccagatat gcaactcagt ctaatcacag tggaattgca accagtcaaa 900
aaaagcctac taggcttcca gggccctcta gggtgcctgc tgcaggaagc agcagcaagg 960
tccagggagc ctctaattta aataggagaa gtcagagctt taacagcatt gacaaaaaca 1020
agcctccaaa ttatgcaaat ggaaacgaaa aagattcctc caaaggacct caatcgtctt 1080
caggtgtaaa tggtaacgtg cagcctccca gtactgctgg gcagcctcct gcctctgcca 1140
tcccttctcc aagtgccagc aagccctggc gcagcaagtc catgaatgtc aaacacagtg 1200
ccacctccac catgttgact gtaaagcagt caagtacagc cacctccccc acaccatctt 1260
cagacagact gaagccacct gtctcagaag gggtcaaaac tgctccctca ggacagaaat 1320
ccatgcttga gaaattcaag ctagtcaatg cccggactgc tttacgcccc ccgcagcctc 1380
ccagttcagg acctagtgat ggtgggaagg atgatgatgc cttttctgaa tctggtgaaa 1440
tggaaggttt taacagtggt ctgaatagtg gtggctcaac aaatagcagt cccaaagtgt 1500
cacctaagtt ggcccctcca aaagctggaa gcaaaaatct cagcaataaa aagtctttgc 1560
tacagccaaa ggaaaaagaa gaaaagaaca gggacaaaaa taaagtttgc actgaaaaac 1620
cagtcaaaga agagaaggat caggtgacag agatggctcc aaaaaagacc tccaaaattg 1680
caagcttgat ccctaagggc agcaagacaa cagcagctaa gaaggaaagc ttaattccgt 1740
cttccagtgg tattccaaaa ccaggctcta aagttccaac agtaaagcaa accatttcac 1800
ctggcagcac agcaagcaaa gagtctgaga aattcaggac taccaagggg agcccttccc 1860
agtccttatc taagcctata accatggaga aagcaagtgc ttctagttgt cctgcccctt 1920
tggaaggaag ggaagctggc caagcttctc cttctggttc ctgtaccatg acagtggcac 1980
aaagcagtgg gcagagcaca ggaaatggtg ctgtccaact ccctcaacag cagcaacata 2040
gccacccgaa taccgcgaca gtggcaccat tcatttacag ggcacattca gaaaatgaag 2100
gtaccgcttt accatcggct gactcctgta ccagtcctac aaagatggac ttatcatata 2160
gtaagactgc taagcagtgc ctggaggaga tatctggtga agaccctgaa acaagaagaa 2220
tgagaacagt taaaaacata gcagacttga ggcagaattt agaagagact atgtccagtc 2280
ttcgtgggac tcagataagc cacagcaccc tggagacaac atttgacagc actgtgacaa 2340
cagaagttaa tggaaggacc atacccaact tgacaagtcg acccaccccc atgacctgga 2400

ggttgggcca ggcatgtccg cgacttcagg cgggagatgc tccctccctg ggtgctggct 2460
atcctcgcag tggtaccagt cgattcatcc acacagaccc ctcgaggttc atgtatacca 2520
cgcctctccg tcgagctgct gtctctaggc tgggaaacat gtcacagatt gacatgagtg 2580
agaaagcaag cagtgacctg gacatgtctt ctgaggtcga tgtgggtgga tatatgagtg 2640
atggtgatat ccttgggaaa agtctcagga ctgatgacat caacagtggg tacatgacag 2700
atggaggact taacctatat actagaagtc tgaaccgaat accagacaca gcaacttccc 2760
gggacatcat ccagagaggg gttcacgatg tgacagtgga tgcagacagc tgggatgaca 2820
gcagttcagt gagcagtggt ctcagtgaca cccttgataa catcagcact gatgacctga 2880
acaccacatc ctctgtcagc tcttactcca acatcaccgt cccctctagg aagaatactc 2940
agctgaggac agattcagag aaacgctcca ccacagacga gacctgggat agtcctgagg 3000
aactgaaaaa accagaagaa gattttgaca gccatgggga tgctggtggc aagtggaaga 3060
ctgtgtcctc tggacttcct gaagaccccg agaaggcagg gcagaaagct tccctgtctg 3120
tttcacagac aggttcctgg agaagaggca tgtctgccca aggagggggcg ccatctaggc 3180
agaaagctgg aacaagtgca ctcaaaacac ccgggaaaac cgatgatgcc aaagcttctg 3240
agaaaggaaa agctccccta aaaggatcat ctctacaaag atctccttca gatgcaggaa 3300
aaagcagtgg agatgaaggg aaaaagcccc cctcaggcat tggaagatcg actgccacca 3360
gctcctttgg ctttaagaaa ccaagtggag tagggtcatc tgccatgatc accagcagtg 3420
gagcaaccat aacaagtggc tctgcaacac tgggtaaaat tccaaaatct gctgccattg 3480
gcgggaagtc aaatgcaggg agaaaaacca gtttggacgg ttcacagaat caggatgatg 3540
ttgtgctgca tgttagctca aagactaccc tacaatatcg cagcttgccc cgcccttcaa 3600
aatccagcac cagtggcatt cctggccgag gaggccacag atccagtacc agcagtattg 3660
attccaacgt cagcagcaag tctgctgggg ccaccacctc gaaactgaga gaaccaacta 3720
aaattgggtc agggcgctcg agtcctgtca ccgtcaacca aacagacaag gaaaaggaaa 3780
aagtagcagt ctcagattca gaaagtgttt ctttgtcagg ttcccccaaa tccagcccca 3840
cctctgccag cgcctgtggt gcacaaggtc tcaggcagcc aggatccaag tatccagata 3900
ttgcctcacc cacatttcga aggttgtttg gtgccaaggc aggtggcaaa tctgcctctg 3960
cacctaatac tgagggtgtg aaatcttcct cagtaatgcc cagccctagt accacattag 4020
cgcggcaagg cagtctggag tcaccgtcgt ccggtacggg cagcatgggc agtgctggtg 4080
ggctaagcgg cagcagcagc cctctcttca ataaaccctc agacttaact acagatgtta 4140
taagcttaag tcactcgttg gcctccagcc cagcatcggt tcactctttc acatcaggtg 4200
gtctcgtgtg ggctgccaat atgagcagtt cctctgcagg cagcaaggat actccgagct 4260
accagtccat gactagcctc cacacgagct ctgagtccat tgacctcccc ctcagccatc 4320
atggctcctt gtctggactg accacaggca ctcacgaggt ccagagcctg ctcatgagaa 4380
cgggtagtgt gagatctact ctctcagaaa gcatgcagct tgacagaaat acactaccca 4440
aaaagggact aagatatacc ccatcatctc ggcaggccaa ccaagaagag ggcaaagagt 4500
ggttgcgttc tcattctact ggagggcttc aggacactgg caaccagtca cctctggttt 4560
ccccttctgc catgtcatct tctgcagctg gaaaatacca cttttctaac ttggtgagcc 4620
caacaaattt gtctcagttt aaccttcccg ggcccagcat gatgcgctca aacagcatcc 4680
cagcccaaga ctcttccttc gatctctatg atgactccca gctttgtggg agtgccactt 4740
ctctggagga aagacctcgt gccatcagtc attcgggctc attcagagac agcatggaag 4800
aagttcatgg ctcttcatta tcactggtgt ccagcacttc ttctctttac tctacagctg 4860
aagaaaaggc tcattcagag caaatccata aactgcggag agagctggtt gcatcacaag 4920
aaaaagttgc taccctcaca tctcagcttt cagcaaatgc tcaccttgta gcagcttttg 4980
aaaagagctt agggaatatg actggccgat tgcaaagtct aactatgaca gcggaacaaa 5040
aggaatctga acttatagaa ctaagagaaa ccattgaaat gctgaaggct cagaattctg 5100
ctgcccaggc ggctattcag ggagcactga atggtccaga ccatcctccc aaagatcttc 5160
gcatcagaag acagcattcc tctgaaagtg tttctagtat caacagtgcc acaagccatt 5220
ccagtattgg cagtggtaat gatgccgact ccaagaagaa gaaaaagaaa aactgggtga 5280
actctagagg aagtgagctg agaagttctt tcaaacaagc ctttgggaag aaaaagtcca 5340
ccaagcctcc ttcatcacat tctgacattg aagagcttac tgattcatcc cttccggcat 5400
cccccaagtt accccataat gctggtgact gtggctcagc atccatgaag ccctcacaat 5460
ctgcttcagc gatctgtgaa tgcacagaag ctgaggcaga gataattctg cagctgaaga 5520
gcgagctcag agaaaaggaa ttaaaattaa cggatattcg gctggaggcc ctcagctctg 5580
ctcatcatct tgatcagatc cgggaagcca tgaaccggat gcagaatgaa attgaaatac 5640
tgaaagctga aaatgaccgg ttgaaggcag aaactggtaa cacagctaag cctactcggc 5700
caccgtcaga atcctcaagc agcacctcct cttcatcttc caggcagtca ttaggacttt 5760
ctctaaacaa tttgaacatc acagaggctg ttagctcaga tattttgcta gatgatgctg 5820
gtgatgcaac tggacataaa gatggccgca gtgtgaaaat tatagtctcc ataagcaagg 5880
gctatggtcg agcaaaggac caaaaatctc aggcatattt gataggatcc attggtgtta 5940
gtggaaaaac caagtgggat gtcttagatg gtgtaataag acgtctcttt aaggaatatg 6000
tattccgaat tgatacatcc actagccttg gtctgagctc tgactgcatt gctagctact 6060
gtataggaga cttaattaga tcccataacc tagaagtgcc tgaattgctg ccttgtggat 6120
accttgttgg agataataac atcatcactg tgaacctcaa aggggtagaa gaaaatagtt 6180
tggacagttt tgtttttgat acgctgattc ctaaaccaat tacccaaagg tactttaact 6240
tgttgatgga gcatcacaga attatactct caggaccgag tggtactgga aagacctatt 6300

```
tggcaaacaa acttgctgaa tatgtaataa ccaaatctgg aaggaaaaaa acagaggatg 6360
caattgccac ttttaatgtg gaccacaagt caagtaagga attgcaacaa tatctagcta 6420
acctggctga acagtgcagt gctgataata atggagtgga gctcccagtt gtaataattc 6480
ttgataatct tcatcatgtg ggctctctga gtgatatctt caatggtttt ctcaattgta 6540
aatacaacaa atgtccatat attattggaa caatgaatca gggagtttct tcatcaccaa 6600
atctagagct gcatcacaat ttcaggtggg tattatgtgc aaatcataca gaaccagtga 6660
aaggcttttt aggcagatat cttcgaagaa aactcataga gatagaaatt gaaaggaaca 6720
ttcgcaataa tgacctagtc aaaattatag attggattcc gaagacgtgg catcatctca 6780
acagtttttt ggaaacacac agttcttctg acgttaccat tggtccccga ctattccttc 6840
cttgccccat ggatgtagaa ggttctagag tatggttcat ggatctctgg aactattctt 6900
tagtacctta tattctggag gcagtgagag agggtcttca gatgtatggg aaacgcacac 6960
catgggaaga tccttcaaag tgggtgcttg acacatatcc atggagctca gcaactctgc 7020
ctcaggagag cccagcctta cttcagctgc gaccagaaga tgttgggtat gaaagctgca 7080
catccactaa ggaagccaca acctcaaagc acattccgca aactgacaca gaaggagatc 7140
ccctgatgaa tatgctaatg aaactccaag aagcagccaa ttactcgagc acacaaagct 7200
gcgacagcga aagcaccagc caccatgaag acattttgga ttcatctctt gaatctaccc 7260
tctagagggt gaaaaaagtt aagggaaaag actttgcttt taaaaaaatg tttcaaaaga 7320
aaggtatttt cactaaacca ctgccagtat aaaagcaccc tgtcaagggc cctgacccag 7380
agttgtggtc tccaaggagg cagcagaact aagtctgaac cgccaagatg ctaaattgca 7440
atggaagctt aactttagtt tatttctaag catttttat atctgtggag taatagaaag 7500
ctccattact caactggaaa ggaccctaat gacagggcaa ctgaacagat tgcacatggg 7560
atagccaaac tggactttct ttgtttcctc tttaaaagtt tacaatgcag accattttt 7620
gtcccttcct tttgtttcct ctgaggggct gttcgcccca ggcagggtcc atctttctga 7680
tctgtccaac ctcctttgtg ccacacggtg ctggtcacag ggcttcagta gtgtttgtgt 7740
tgtgcgctca ccccattcca gaacaaatcc aagaggccag tcctccataa gcacaaatgg 7800
aattgtgcaa ccaccagaaa aacactactg tggcaaactg gagaagtgcc aatttaattc 7860
taactgccac gttctcatga tgtgctccac caacttttta gtatatgagt cactggtttt 7920
ataaggttgt ttttaccaca gtggtctttt taaaccacct gcccactccc ttaacaagag 7980
ttttatacca attattagtc aacactgata aaaggctttt ttagggcttt atttgtttga 8040
gccttttcag tgaaagaagg aacatttcct atggtgctgt ctcactgcct taaaacagat 8100
ttctatgaca gtttaacagt tggtttaaat cctaaaccat tggtaatttc cactgtcttt 8160
tcatttacaa ccaagcaaca ccagttaaca tagtagcctc atctctatat atctttctct 8220
tttttttttt tttgaagaaa tggataggag aaagatcagt atttttagcc ttgtgaatag 8280
atcgctttgc ctatcctcca aaatattaaa ataacccaga aatgctcttt gaccgtcact 8340
taaaacctaa gacatgtggc gaaattccat ccagttctaa gtgaaagagt ttcagaaggc 8400
agaagatttt gaattattat ccagcagggc tggaagcact agatgcagca tgagcacaac 8460
tattcggctt tccttcccta ttgtttttgt ttttttaatg agttttgacg catgttgttt 8520
tgattgctat tgttgtacat gagaaattca gcattaaaga acactgaagc ggtaaggtca 8580
ctgtggaaga ggaagcgttt atactgtaaa agaaggttag atttgcacag tctactgggt 8640
aggtattgta aataataatt tttaaaactt gcacaaatca aaacaaacac aaacaaaatt 8700
gtattttatc ctgttggtgt taagaggtgt ttcacttgct gagatttcct gtacattgca 8760
aacaaataca gaatgcaaac cctcaaagct gtattatctg gtgtgtttgt cctgtattta 8820
cagttgtttt tgactatgca ggagctatca gtgctagagt gagcatgctt caaaactgta 8880
catgaagcca atatattttt ggataagtaa aactgtctga aagtacatct gtcatggcag 8940
gctttaaaga gagtgcatga aaactgatca gtcattggag aagttaccac cacacacaaa 9000
ggacaggttt taagtttatg aaacccaagg gctaggccat ggtatagact tcttctatga 9060
gtgtgtgaaa atgtgttact tttaggacgt gtatttggtg ctactctctg tgaccaccaa 9120
tgggtcagtt gctatagaac aacaacacca cgaaacatct gtgcagtttt cagagtgtca 9180
caaagtcaat aggtccttac acggtgctat tgccctaagg gaaatccgaa ctgaatttat 9240
gcacatagaa ttgtcaccct gactttgaag cctcaaacat ggatcaaatc tgttgtgaaa 9300
catcaatata tgtagctgga tgagtgacta gtttcccttg tataatatgt gatctaagaa 9360
aattgctaat ctttccctgc cattttgaga aacacagtcc aaacatgagc ataaacagaa 9420
tttcctgcaa tacatcccag taggtccacc tagtttacaa cttaaactag tttgtgaaac 9480
atttgtctgt atacatttta tattttgtac attttgatgt aacatatcat gtaaataggc 9540
agaaacagtg aaataaatca tctgaaaagt tttgtagtct ttgtaaagcc ccaacaataa 9600
gtacttggtg tcaatggact taactggatg atgtattttc tattggttta ttgttcctct 9660
agcttgtaaa ccagcttgca tatatttttt tgcaaatgtg caccctgtat ctgtctaaat 9720
tattactttg ccattaaagt ggaattattt attgacaa                         9758
```

<210> 56
<211> 3074

<212> DNA
<213> Homo sapiens

<400> 56

```
aacaaacctc gcctggctcc cagctggtgc tgaagctcgt cagttcacca tccgccctcg 60
gcttccgcgg ggcgctgggc cgccagcctc ggcaccgtcc tttcctttct ccctcgcgtt 120
aggcaggtga cagcagggac atgtctcggg agatgcagga tgtagacctc gctgaggtga 180
agcctttggt ggagaaaggg gagaccatca ccggcctcct gcaagagttt gatgtccagg 240
agcaggacat cgagacttta catggctctg ttcacgtcac gctgtgtggg actcccaagg 300
gaaaccggcc tgtcatcctc acctaccatg acatcggcat gaaccacaaa acctgctaca 360
acccctctt caactacgag gacatgcagg agatcaccca gcactttgcc gtctgccacg 420
tggacgcccc tggccagcag gacggcgcag cctccttccc cgcagggtac atgtacccct 480
ccatggatca gctggctgaa atgcttcctg gagtccttca acagtttggg ctgaaaagca 540
ttattggcat gggaacagga gcaggcgcct acatcctaac tcgatttgct ctaaacaacc 600
ctgagatggt ggagggcctt gtccttatca acgtgaaccc ttgtgcggaa ggctggatgg 660
actgggccgc ctccaagatc tcaggatgga cccaagctct gccggacatg gtggtgtccc 720
acctttttgg gaaggaagaa atgcagagta acgtggaagt ggtccacacc taccgccagc 780
acattgtgaa tgacatgaac cccggcaacc tgcacctgtt catcaatgcc tacaacagcc 840
ggcgcgacct ggagattgag cgaccaatgc cgggaaccca cacagtcacc ctgcagtgcc 900
ctgctctgtt ggtggttggg gacagctcgc ctgcagtgga tgccgtggtg gagtgcaact 960
caaaattgga cccaacaaag accactctcc tcaagatggc ggactgtggc ggcctcccgc 1020
agatctccca gccggccaag ctcgctgagg ccttcaagta cttcgtgcag ggcatgggat 1080
acatgccctc ggctagcatg acccgcctga tgcggtcccg cacagcctct ggttccagcg 1140
tcacttctct ggatggcacc cgcagccgct cccacaccag cgagggcacc cgaagccgct 1200
cccacaccag cgagggcacc cgcagccgct cgcacaccag cgagggggcc cacctggaca 1260
tcaccccaa ctcgggtgct gctgggaaca gcgccgggcc caagtccatg gaggtctcct 1320
gctaggcggc ctgcccagct gccgccccg gactctgatc tctgtagtgg cccctcctc 1380
cccggcccct tttcgccccc tgcctgccat actgcgccta actcggtatt aatccaaagc 1440
ttattttgta agagtgagct ctggtggaga caaatgaggt ctattacgtg ggtgccctct 1500
ccaaaggcgg ggtggcggtg gaccaaagga aggaagcaag catctccgca tcgcatcctc 1560
ttccattaac cagtggccgg ttgccactct cctcccctcc ctcagagaca ccaaactgcc 1620
aaaaacaaga cgcgtagcag cacacacttc acaaagccaa gcctaggccg ccctgagcat 1680
cctggttcaa acgggtgcct ggtcagaagg ccagccgccc acttcccgtt tcctctttaa 1740
ctgaggagaa gctgatccag tttccggaaa caaaatcctt ttctcatttg gggagggggg 1800
taatagtgac atgcaggcac ctcttttaaa caggcaaaac aggaaggggg aaaaggtggg 1860
attcatgtcg aggctagagg catttggaac aacaaatcta cgtagttaac ttgaagaaac 1920
cgatttttaa agttggtgca tctagaaagc tttgaatgca gaagcaaaca agcttgattt 1980
ttctagcatc ctcttaatgt gcagcaaaag caggcgacaa aatctcctgg ctttacagac 2040
aaaaatattt cagcaaacgt tgggcatcat ggtttttgaa ggctttagtt ctgctttctg 2100
cctctcctcc acagccccaa cctcccaccc ctgatacatg agccagtgat tattcttgtt 2160
cagggagaag atcatttaga tttgttttgc attccttaga atggagggca acattccaca 2220
gctgccctgg ctgtgatgag tgtccttgca ggggccggag taggagcact ggggtggggg 2280
tggaattggg gttactcgat gtaagggatt ccttgttgtt gtgttgagat ccagtgcagt 2340
tgtgatttct gtggatccca gcttggttcc aggaattttg tgtgattggc ttaaatccag 2400
ttttcaatct tcgacagctg ggctggaacg tgaactcagt agctgaacct gtctgacccg 2460
gtcacgttct tggatcctca gaactctttg ctcttgtcgg ggtgggggtg ggaactcacg 2520
tggggagcgg tggctgagaa aatgtaagga ttctggaata catattccat gggactttcc 2580
ttccctctcc tgcttcctct tttcctgctc cctaaccttt cgccgaatgg ggcagcacca 2640
ctgacgtttc tgggcggcca gtgcggctgc caggttcctg tactactgcc ttgtactttt 2700
cattttggct caccgtggat tttctcatag gaagtttggt cagagtgaat tgaatattgt 2760
aagtcagcca ctgggacccg aggatttctg ggaccccgca gttgggagga ggaagtagtc 2820
cagccttcca ggtggcgtga gaggcaatga ctcgttacct gccgcccatc accttggagg 2880
ccttccctgg ccttgagtag aaaagtcggg gatcggggca agagaggctg agtacggatg 2940
ggaaactatt gtgcacaagt ctttccagag gagtttctta atgagatatt tgtatttatt 3000
tccagaccaa taaatttgta actttgcagc ggaaaaaaaa aaaaaaaaaa aaaaaaaaaa 3060
aaaaaaaaaa aaaa                                                   3074
```

<210> 57
<211> 3938
<212> DNA

<213> Homo sapiens

<400> 57

```
gtactggccc gcgccgtccg cccgccgaca gctccctgag ccagcccggg aggcagccgc 60
gcgcagcgag ccggtggcgc aggtgtcggg gtcctcgagc gcccagcctg ggagcatgat 120
tgtggacaag ctgctggacg acagccgcgg cggagagggg ctgcgggacg cggcgggcgg 180
ctgcggcctc atgaccagcc cgctcaacct gagctacttc tacggcgcgt cgccgcccgc 240
cgccgccccg ggcgcctgcg acgccagctg ctcggtcttg ggccccctcgg cgcccggctc 300
gcccggctcc gactcctccg acttctcctc tgcctcgtcg gtgtcctcct gcggcgccgt 360
```

```
ggagtcccgg tcgagaggcg gcgcccgcgc cgagcgccag ccagttgagc cccatatggg 420
ggttggcagg cagcagagag gcccctttca aggtgttcgg gtaaagaact cagtgaagga 480
actcctgttg cacatccgaa gtcataaaca gaaggcttct ggccaagctg tggatgattt 540
taagacacaa ggtgtgaaca tagaacagtt cagagaattg aagaacacag tatcatacag 600
tgggaaaagg aaagggcccg attcgttgtc tgatggacct gcttgcaaaa ggccagctct 660
gttgcattcc caatttttga caccacctca aacaccaacg cccggggaga gcatggaaga 720
tgttcatctc aatgaaccca aacaggagag cagtgctgat ctgcttcaga acattatcaa 780
cattaagaat gaatgcagcc ccgtttccct gaacacagtt caagttagct ggctgaaccc 840
cgtggtggtc cctcagagct cccccgcaga gcagtgtcag gacttccatg gagggcaggt 900
cttttctcca cctcagaaat gccaaccatt ccaagtcagg ggctcccaac aaatgataga 960
ccaggcttcc ctgtaccagt attctccaca gaaccagcat gtagagcagc agccacacta 1020
cacccacaaa ccaactctgg aatacagtcc ttttcccata cctcccccagt cccccgctta 1080
tgaaccaaac ctctttgatg gtccagaatc acagttttgc ccaaaccaaa gcttagtttc 1140
ccttcttggt gatcaaaggg aatctgagaa tattgctaat cccatgcaga cttcctccag 1200
tgttcagcag caaaatgatg ctcacttgca cagcttcagc atgatgccca gcagcgcctg 1260
tgaggccatg gtggggcacg agatggcctc tgactcttca aacacttcac tgccattctc 1320
aaacatggga aatccaatga acaccacaca gttagggaaa tcacttttc agtggcaggt 1380
ggagcaggaa gaaagcaaat tggcaaatat ttcccaagac cagtttcttt caaaggatgc 1440
agatggtgac acgttccttc atattgctgt tgcccaaggg agaagggcac tttcctatgt 1500
tcttgcaaga aagatgaatg cacttcacat gctggatatt aaagagcaca atggacagag 1560
tgcctttcag gtggcagtgg ctgccaatca gcatctcatt gtgcaggatc tggtgaacat 1620
cggggcacag gtgaacacca cagactgctg gggaagaaca cctctgcatg tgtgtgctga 1680
gaagggccac tcccaggtgc ttcaggcgat tcagaaggga gcagtgggaa gtaatcagtt 1740
tgtggatctt gaggcaacta actatgatgg cctgactccc cttcactgtg cagtcatagc 1800
ccacaatgct gtggtccatg aactccagag aaatcaacag cctcattcac ctgaagttca 1860
ggagctttta ctgaagaata agagtctggt tgataccatt aagtgcctaa ttcaaatggg 1920
agcagcggtg gaagcgaagg atcgcaaaag tggccgcaca gccctgcatt tggcagctga 1980
agaagcaaat ctggaactca ttcgcctctt tttggagctg cccagttgcc tgtcttttgt 2040
gaatgcaaag gcttacaatg gcaacactgc cctccatgtt gctgccagct tgcagtatcg 2100
gttgacacaa ttagatgctg tccgcctgtt gatgaggaag ggagcagacc caagtactcg 2160
gaacttggag aacgaacagc cagtgcattt ggttcccgat ggccctgtgg gagaacagat 2220
ccgacgtatc ctgaagggaa agtccattca gcagagagct ccaccgtatt agctccatta 2280
gcttggagcc tggctagcaa cactcactgt cagttaggca gtcctgatgt atctgtacat 2340
agaccatttg ccttatattg gcaaatgtaa gttgtttcta tgaaacaaac atatttagtt 2400
cactattata tagtgggtta tattaaaaga aaagaagaaa aatatctaat ttctcttggc 2460
agatttgcat atttcatacc caggtatctg ggatctagac atctgaattt gatctcaatg 2520
gtaacattgc cttcaattaa cagtagcttt tgagtaggaa aggactttga tttgtggcac 2580
aaaacattat taatatagct attgacagtt tcaaagcagg taaattgtaa atgtttcttt 2640
aagaaaaagc atgtgaaagg aaaaaggtaa atacagcatt gaggcttcat ttggccttag 2700
tccctgggag ttactggcgt tggacaggct tcagtcattg gactagatga aaggtgtcca 2760
tggttagaat ttgatctttg caaactgtat ataattgtta tttttgtcct taaaaatatt 2820
gtacatactt ggttgttaac atggtcatat ttgaaatgta taagtccata aaatagaaaa 2880
gaacaagtga attgttgcta tttaaaaaaa ttttacaatt cttactaagg agtttttatt 2940
gtgtaatcac taagtctttg tagataaagc agatggggag ttacggagtt gttcctttac 3000
tggctgaaag atatattcga attgtaaaga tgctttttct catgcattga aattatacat 3060
tatttgtagg gaattgcatg cttttttttt tttttctccc gagacagggt cttgctctgg 3120
cgcccaggct ggagtacagt ggcatgatct tggctcactt cagccttgac ttgggctcaa 3180
gtgatcctcc tacctgagcc ttctgagtaa ctggaactac aggtgtgcac tcctcgcctg 3240
gctaattttt tatttttttgt acaggcagga tcttgccacc ttgcccaggc tggtcttgaa 3300
ctcctgagct catgccatct gcctgcctta gtctcccaaa atgctgggat tacaggagtg 3360
agccaccatg cccggctggc agttgcatgg aagagaacac ctctttatgg cttaccctct 3420
agaatttcta atttatgtgt tctgttgaaa tttttgtttt tttaccttta ttgaaacaac 3480
aaaaagtcag tattgaaaca tatcttcctg ttttctgttg tcaaatgatg ataatgtgcc 3540
atgatgtttt atatatatca ttcagaaaaa gttttatttt ttaataacat tctattaaca 3600
ttattttgct tgccgctggc atgcctgagg aatgtatttg gctttgatta cacactaagt 3660
ttttgtaata aatttgactc attaaaaacc tttttttttt aaaaaaaaaa aaaaagaaaa 3720
tctcattagt gaacttatct ttgcagctga gtacttaaat tctttttaaa aagataccct 3780
ttggattgat cacattgttt gacccagtat gtcttgtaga cacgttagtt ataatcacct 3840
tgtatctcta aatatggtgt gatatgaacc agtccattca cattggaaaa actgatggtt 3900
ttaaataaac taattcacta ataaaaaaaa aaaaaaa                         3938
```

<210> 58
<211> 2204
<212> DNA
<213> Homo sapiens

<400> 58
gggtgcgtgt ggagaggccc aggtgaggag caagcgcccg cgttccggaa gcccgctccc 60
ggggccatgg gggcacaggt gaggctgccg cccggagagc cctgccgaga aggatatgtg 120
ctgtctctgg tctgtccaaa ctcctcccag gcttggtgtg agatcacaaa tgtgtcacag 180
ctgctggctt ctcctgtgct ctacacggac ctgaattaca gcataaacaa cttgagcatt 240
tcagcaaatg tagaaaacaa atacagtctt tatgtgggct tggtactggc agtaagctca 300
agtattttta ttggctccag cttcatactg aaaaagaagg gcctcttgca actggccagc 360
aagggcttta ctagagctgg acaaggtgga cattcttacc tgaaggaatg gctctggtgg 420
gtaggattgc tgtcaatggg agcaggagag gctgcaaatt ttgctgctta tgcttttgca 480
cctgccacct tggtcacccc tctgggtgct ttgagtgttc tcataagtgc aatattatct 540
tcctactttt taaacgagca cttgaacatt catgggaaaa taggctgcat attaagtata 600
ttggggtcaa ctgtgatggt tatccatgcc ccacaagaag aggaagtcac atctttgcat 660
gaaatggaaa tgaaattgag agacccaggg tttatttcct ttgctgtgat cataactgtg 720
atctccttgg tgctgatttt gattgtggct cccaagaaag gacagaccaa tatattggtt 780
tatatttcaa tctgttcctt gattggagcg ttttcagttt cttctgtgaa aggcctggga 840
attgccatta aggagctgat agaatggaag ccagtttaca aacatccgct ggtctttgtt 900
ttgctggctg tacttgtgct ttcagtaact acacagatta actatctcaa caaggcactg 960
gacaccttta atacctctct tgtgacaccc atttattatg tattcttcac atccatggta 1020
gtgacttgct ctgccatctt attccaagag tggtatggca tgacagctgg agatatcatt 1080
gggaccctga gtggattctt cactattatc attggcatct tccttctaca tgcttttaaa 1140
aatactgaca ttacttggag tgagcttaca tccactgcta agaaagaagc cgtctctctg 1200
aatgtcaatg aaaacaatta tgttttacta gagaacttgg agtgttcagc cccaggatac 1260
aatgatgacg ttaccttgtt tagtagaact gatgactgaa gtctctagaa acactgagtt 1320
ttaaccaata tgagacacac gaagaggaaa atgaatgctt gcttcttgga agaactgcta 1380
ggaaagttag catttttgca gttctaacta atttagatgt gaggccaagt aaaaatgcca 1440
ttttttttggc cattgaattt gaaaatcaaa ttgattatcc tccagaattt ctacaaactt 1500
tgaaatactc tctaaggatc aaagaagtca aagagctatg tgtgtctcag aataatctcc 1560
ttcttctggt cacagtatct ttgtctctgc caggtggctc ttcatttctt tggtagctat 1620
ttttagactt acagtcattc accaatatac agttagcagt gttccgatag acacagtatc 1680
agtcatattc tccgttgagt cataatttca aactcaacaa gatttcagaa aggtataatc 1740
atgtaatttc tcatattaaa actgagaaga gaggccaggc atggtggttc acacctgtaa 1800
tcctagcagt ttgggaggcc aaggcggaca gatcacttga gctcaggagt tcgagaccac 1860
cttgggccac aaggcgagac cctgtctcta caaaaaatac aaaaattagc caggcgtggt 1920
ggcacatgcc tgtagttcca gccacccggg agcctgaggt gggaggattg cttgagtctg 1980
cgaggttggg gctgccgtga gcccagattg cgccattgtg ctccagcctg ggtgacaaag 2040
caaggccctg tctcaaaaaa gaagaagaga aagggccagg ggcttttgct ttaaaaaact 2100
ttttttaaaa cagaagtgaa accacgggtg ctttgttctt ccttctgcac ctccttctta 2160
aactagatag ctaattagtt attttaagaa aaaaaaaaaa aaaa 2204

<210> 59
<211> 3547
<212> DNA
<213> Homo sapiens

<400> 59

gcactcgccc ggctcgcccg ctttcgcacc cagttcacgc gccacagcta tgtgtccccg 60
agccgcgcgg gcgcccgcga cgctactcct cgccctgggc gcggtgctgt ggcctgcggc 120
tggcgcctgg gagcttacga ttttgcacac caacgacgtg cacagccggc tggagcagac 180
cagcgaggac tccagcaagt gcgtcaacgc cagccgctgc atgggtggcg tggctcggct 240
cttcaccaag gttcagcaga tccgccgcgc cgaacccaac gtgctgctgc tggacgccgg 300
cgaccagtac cagggcacta tctggttcac cgtgtacaag ggcgccgagg tggcgcactt 360
catgaacgcc ctgcgctacg atgccatggc actgggaaat catgaatttg ataatggtgt 420
ggaaggactg atcgagccac tcctcaaaga ggccaaattt ccaattctga gtgcaaacat 480
taaagcaaag gggccactag catctcaaat atcaggactt tatttgccat ataaagttct 540
tcctgttggt gatgaagttg tgggaatcgt tggatacact tccaaagaaa ccccttttct 600
ctcaaatcca gggacaaatt tagtgtttga agatgaaatc actgcattac aacctgaagt 660
agataagtta aaaactctaa atgtgaacaa aattattgca ctgggacatt cgggttttga 720
aatggataaa ctcatcgctc agaaagtgag ggtgtggac gtcgtggtgg gaggacactc 780
caacacattt ctttacacag gcaatccacc ttccaaagag gtgcctgctg ggaagtaccc 840
attcatagtc acttctgatg atgggcggaa ggttcctgta gtccaggcct atgcttttgg 900
caaataccta ggctatctga agatcgagtt tgatgaaaga ggaaacgtca tctcttccca 960
tggaaatccc attcttctaa acagcagcat tcctgaagat ccaagcataa aagcagacat 1020
taacaaatgg aggataaaat tggataatta ttctacccag gaattaggga aaacaattgt 1080
ctatctggat ggctcctctc aatcatgccg ctttagagaa tgcaacatgg gcaacctgat 1140

ttgtgatgca atgattaaca acaacctgag acacacggat gaaatgttct ggaaccacgt 1200
atccatgtgc attttaaatg gaggtggtat ccggtcgccc attgatgaac gcaacaatgg 1260
cacaattacc tgggagaacc tggctgctgt attgcccttt ggaggcacat ttgacctagt 1320
ccagttaaaa ggttccaccc tgaagaaggc ctttgagcat agcgtgcacc gctacggcca 1380
gtccactgga gagttcctgc aggtgggcgg aatccatgtg gtgtatgatc tttcccgaaa 1440
acctggagac agagtagtca aattagatgt tctttgcacc aagtgtcgag tgcccagtta 1500
tgaccctctc aaaatggacg aggtatataa ggtgatcctc ccaaacttcc tggccaatgg 1560
tggagatggg ttccagatga taaaagatga attattaaga catgactctg gtgaccaaga 1620
tatcaacgtg gtttctacat atatctccaa aatgaaagta atttatccag cagttgaagg 1680
tcggatcaag ttttccacag gaagtcactg ccatggaagc ttttctttaa tatttctttc 1740
actttgggca gtgatctttg ttttatacca atagccaaaa attctccttg cctttaatgt 1800
gtgaaactgc atttttttcaa gtgagattca aatctgcctt ttaggacctg gctttgtgac 1860
agcaaaaacc atctttacag gctcctagaa gctgaaggtt agagcattat aaaatgaaga 1920
gacagacatg attactcagg gtcagcaacc tagtgagtta gaaaaaaaat taacataggg 1980
ccctataagg agaaagccaa ctatgttaag tttacgtgtc caaattttaa tgaaatttta 2040
ctaacaattt taaaccatat ttttcttctt catatccatt tctaatccat caaacagctt 2100
atgtttacat aaaattttat cattcacaag gaagttttaa gcacactgtc tcatttgata 2160
tccacaactt atttttggta ggaaagagag atgtttttcc cacctgtcag atgaaaaaac 2220
tgaagctcaa aaagggttga cttgaccata cagctaatgc tgacagatcc aagacctaga 2280
cctaggtctt ttgaactcaa gtccagcatt ctcaactata tcaagttact gttcagaata 2340
cttaatatct cctctcttca taattatcaa tagccccaag ctcatggatg acaaatctct 2400
gctttatttc ttgtctctat tttttcactt tatagctcct gttataatag caagtttaat 2460
ggtataaaca caggatacca tcctctcttg caacacccat gtgcctttga tgagtcaggt 2520
agcaagctgt agtagataat gagaaaggcc agaggctgca aagacagtca aaggacacga 2580
gagaaaggaa ggggaagaac aggactccag gactgtttta tattatagaa aagcaagagc 2640
taaagagcat ttacacatgt taaacagata cttgttaagc atagtgcctg acacacggca 2700
ttagctgtta ttttatgaga ttccatcagc tctgcctctg tcctctttct tctaacatga 2760
aggtatcatg agaagagaac cttctaacat aagctgtaat tctaaacctg cacttgtccc 2820
tctccagcaa gaggctagca ctgaattcat tctactcata ctacacaccc agttatggaa 2880
tgtccagagt tctcgaagaa aataaatgac tttaggaaga ggtatacatt ttttaagtcg 2940
ctctgcctcc aaatctgaac agtcactgta aatcattctt aagcccagat atgagaactt 3000
ctgctggaaa gtgggaccct ctgagtgggt ggtcagaaaa tacccatgct gatgaaatga 3060
cctatgccca aagaacaaat acttaacgtg ggagtggaac cacatgagcc tgctcagctc 3120
tgcataagta attcaagaaa tgggaggctt caccttaaaa acagtgtgca aatggcagct 3180
agaggttttg ataggaagta tgtttgtttc ttagtgttta caaatattaa gtactcttga 3240
tacaaaatat acttttaaac ttcataacct ttttataaaa gttgttgcag caaaataata 3300
gcctcggttc tatgcatata tggattgcta taaaaaatgt caataagatt gtacaaggaa 3360
aattagagaa agtcacattt agggtttatt ttttacactt ggccagtaaa atagggtaaa 3420
tcctattaga aattttttaa agaacttttt ttaagtttcc taaatctgtg tgtgtattgt 3480
gaagtggtat aagaaatgac tttgaaccac tttgcaattg tagattccca acaataaaat 3540
tgaagat 3547

<210> 60
<211> 4525
<212> DNA
<213> Homo sapiens

<400> 60

```
ttggcccgtg cgtggggctg gggtggaggg cgggacagag gggctgggcc caggcaaagc 60
ttctgtcgct gctgctgcgg aggccgagga caggacgtgg gctgggccgg gcagtgccgg 120
actcggagga gcaggaggcg aggtccgccg gagctctgag cccccgctgc tctgccgcgc 180
ggtgaccccg cgccccggcg ccgtccgacc cgtggctgtt ccgagacgat tggtgggggc 240
gcggcggcgg cggcggcggc tgttgggggt ggggagacgc gcggcgagga gacgagcgag 300
gtcagcgagt ttgagggagg accgcgagcc gctgccgccg tcatgtccga ggactcgagc 360
gccctgccct ggtccatcaa cagggacgat tacgagctgc aggaggtgat cgggagtgga 420
gcaactgctg tagtccaagc agcttattgt gcccctaaaa aggagaaagt ggcaatcaaa 480
cggataaacc ttgagaaatg tcaaactagc atggatgaac tcctgaaaga aattcaagcc 540
atgagtcaat gccatcatcc taatattgta tcttactaca catcttttgt ggtaaaagat 600
gagctgtggc ttgtcatgaa gctgctaagt ggaggttctg ttctggatat tattaagcac 660
attgtggcaa aaggggaaca caaaagtgga gtcctagatg aatctaccat tgctacgata 720
ctccgagaag tactggaagg gctggaatat ctgcataaaa atggacagat ccacagagat 780
gtgaaagctg gaaacattct tcttggagaa gatggctcag tacagattgc agactttggg 840
gttagtgctt ttttagcaac tggtggtgat attacccgaa ataaagtgag aaagaccttt 900
gttggcaccc cttgttggat ggcacctgaa gttatggaac aggtccgtgg ttatgatttc 960
aaagctgata tttggagttt tggaattaca gcaattgaat tggctacagg ggcggctcct 1020
```

tatcataaat atccaccaat gaaggtttta atgctgacac tgcagaacga tcctccttct 1080
ttggaaactg gtgttcaaga taaagaaatg ctgaaaaaat atggaaaatc atttagaaaa 1140
atgatttcat tgtgccttca aaaagatcca gaaaaaagac caacagcagc agaactatta 1200
aggcacaaat ttttccagaa agcaaagaat aaagaatttc ttcaagaaaa aacattgcag 1260
agagcaccaa ccatttctga aagagcaaaa aaggttcgga gagtaccagg ttccagtggg 1320
cgtcttcata agacagagga tggaggctgg gagtggagtg atgatgaatt tgatgaagaa 1380
agtgaggaag ggaaagcagc aatttcacaa ctcaggtctc cccgagtgaa agaatcaata 1440
tcaaattctg agctctttcc aacaactgat cctgtgggta ctttgctcca agttccagaa 1500
cagatctctg ctcatctacc tcagccagct gggcagattg ctacacagcc aactcaagtc 1560
tctctcccac ccaccgcaga gccagcaaaa acagctcagg ctttgtcttc aggatcaggt 1620
tcacaagaaa ccaagatccc aatcagtcta gtactaagat taaggaattc caaaaaagaa 1680
ctaaatgata ttcgatttga atttactcct gggagagata cagcagaggg tgtctctcag 1740
gaactcattt ctgctggcct ggtcgacgga agggatttag taatagtggc agctaatttg 1800
cagaaaattg tggaagaacc tcagtcaaat cgatctgtca ctttcaaact ggcatctggt 1860
gtcgaaggct cagatattcc tgatgatggt aaactgatag gatttgccca gctcagcatc 1920
agctaaacca caaccctgga agaggcggcc taaggagatt ccacacatgc gtatctctgt 1980
tgcttctatt ggcctaaacc cactactgcc aaagaaccca gcaacaaacc tcccggctag 2040
gagctttaga agtctttatg ttcttcctgc catcattcct cctttttccca cagggaaaga 2100
aaagttggat cactagtggc cagcatcccc agagttccgt tagtaaactt acttcatatg 2160
tcccctgtct tcctccatct gagaagtggc ccatgtgctt caaggcccag gagggagatc 2220
tgtcagctca ttcttgcctt actccaatga tggcccaggt ggaaaagtag cagctgtatc 2280
gggcttcctc atcctgcctg ttccccccaca cctgccagga tatggacatc ttgggatatc 2340
tctttaccac tgaagtagaa ttgattgttc agctggagcc cagagaattt aatttaatgt 2400
tttttctttg tacctgatgt gaattctagc aacctttgtt aggaaaaagc acagcctcag 2460
atggaggcag cctaaactgt gttcttgttt tgttcatggt gtttctaagc gttttgctga 2520
agctgctctc aggcaccccc ttcttcattg ctctctccag aaagggttgc tagccttaac 2580
ttcagctggt gcaaaacatc tgactgtagc cgaacttcag ccatcagatc cttcaaagtg 2640
gaactttgga ttgtttttac agacaacatc gagtaatggc ttgtaaatgt gaattttgcc 2700
agaggtggtt tttgaacagg aaaatcataa ttcatatcat tggagaagta tttattttca 2760
aatatcaaat tgaagaaaaa ctcaatcctc ccatgaaaat cagttcgcct ggcctccaag 2820
tcgtgaggaa atgggtatgc aaggctgaga tttctacagc aataaaggag acacacactg 2880
ggccagagag gcctgccttc tgcctgctct cctgcactga ccctttggag gggtctctg 2940
tgtgctgaag ctaactcaag atggaaagtg aaaccacatg tgccgtgacc tttaggtttt 3000
atgagtagac agtgttcatt tgattttcta cagaaataat ataaattatt ctttaggttt 3060
aaaaaagagc actcataatg caatatgtga ataatcagtg aggttgattt ttcttttttc 3120
ctaccgtttc atagtctttg tctaactgct agtaacccta ccgagtttta tatatgagtg 3180
ggatactcaa tctggcctta aaaagataca caaagatggg ctgtgggtcc ctggaaaggg 3240
ggagagttgc cctttacaga atcactcgag ccctttccag cactgttggt ctgatgaaca 3300
aggttgtttt accttatttt ctcttggaac atatctgaaa accttcccca caaataactt 3360
gtcacacctt ttgtttcatt ctgagtcttt agttttagtc atgggctttc ttcacctgct 3420
ctaggtgcaa aggcatgttg ggaaagagat ggatgttggg gaggaagaga ggagatggat 3480
ttcagttggg agttaggagg agagtaggtg agatgatcag acaccggagt tcaacgtccc 3540
agcagtcttg gtaaaaggag ggagcctgct gagccaggag ggagaaaaga agattgacca 3600
gcttgctaga aaaatactta gcttttcttt ttcttttttt gtggaggggg gacggagagg 3660
aacaaggatg gggaggtagg aatgaggtat agaaaagaga tagcatcttc tttggcacaa 3720
gactagtggc ttaccgctta ccttagagtt ttgttttttt tttttcaaac ccatcaaaat 3780
ctacttattt atgaatccaa ggggtggcag catcactctg ttctagcatt ctttgtggag 3840
atggtctggt gcctagctgg gagtgagcag cagcccatcc cctgttcact ttctctagcc 3900
catcattacc tgtgaactgc agtggggcag tcatggcaaa tagaattggg ctggggtttc 3960
tccttctttt cagttcattg tttgccctgc taggaattag aagacagaca ccatgtccca 4020
ggacagtgtt acttcttctg catgatgtgt ggtagactcc ctttgctggc ttgtgcagtg 4080
atactgagaa aatacatgaa cagaaactgc ccaggtggaa cagcacgtaa cctagtgagt 4140
gactgtactc ctttctagga atgctgattc agagtgcacc tctttgacta ggtcccagga 4200
tccccttgtc cctggagtag ggactaacta tagcacaaag taatatgtgc caatgctatt 4260
tgtgaaatgt ttggtctttc taaacgacta aaggatttgt tgggtttttg cttaagtttt 4320
gaaccaaatc ctagagccag ctgataatat ttaataatct ggaggagaga ataatgatgt 4380
accaataagt ggagattcct ccttatgatg tatgctaggt tatggaagat gtaaaatatt 4440
caacttttttc ctccttttttt tggactttgt attttactgc atgttttctt catttttaat 4500
caataaagag taaattgtcc ttaaa 4525

<210> 61

<211> 4593
<212> DNA
<213> Homo sapiens

<400> 61

gctgccgcgc cccgcccttt ctcggccccc ggagggtgac ggggtgaagg cgggggaacc 60
gaggtgggga gtccgccaga gctcccagac tgcgagcacg cgagccgccg cagccgtcac 120
ccgcgccgcg tcacggctcc cgggcccgcc ctcctctgac ccctcccctc tctccgtttc 180
cccctctccc cctcctccgc cgaccgagca gtgacttaag caacggagcg cggtgaagct 240
catttttctc cttcctcgca gccgcgccag ggagctcgcg gcgcgcggcc cctgtcctcc 300
ggcccgagat gaatcctgcg gcagaagccg agttcaacat cctcctggcc accgactcct 360
acaaggttac tcactataaa caatatccac ccaacacaag caaagtttat tcctactttg 420
aatgccgtga aaagaagaca gaaaactcca aattaaggaa ggtgaaatat gaggaaacag 480
tattttatgg gttgcagtac attcttaata agtacttaaa aggtaaagta gtaaccaaag 540
agaaaatcca ggaagccaaa gatgtctaca aagaacattt ccaagatgat gtctttaatg 600
aaaagggatg gaactacatt cttgagaagt atgatgggca tcttccaata gaaataaaag 660
ctgttcctga gggctttgtc attcccagag gaaatgttct cttcacggtg gaaaacacag 720
atccagagtg ttactggctt acaaattgga ttgagactat tcttgttcag tcctggtatc 780
caatcacagt ggccacaaat tctagagagc agaagaaaat attggccaaa tatttgttag 840
aaacttctgg taacttagat ggtctggaat acaagttaca tgattttggc tacagaggag 900
tctcttccca agagactgct ggcataggag catctgctca cttggttaac ttcaaaggaa 960
cagatacagt agcaggactt gctctaatta aaaaatatta tggaacgaaa gatcctgttc 1020
caggctattc tgttccagca gcagaacaca gtaccataac agcttggggg aaagaccatg 1080
aaaaagatgc ttttgaacat attgtaacac agttttcatc agtgcctgta tctgtggtca 1140
gcgatagcta tgacatttat aatgcgtgtg agaaaatatg ggtgaagat ctaagacatt 1200
taatagtatc aagaagtaca caggcaccac taataatcag acctgattct ggaaaccctc 1260
ttgacactgt gttaaaggtt ttggagattt taggtaagaa gtttcctgtt actgagaact 1320
caaagggtta caagttgctg ccaccttatc ttagagttat tcaaggggat ggagtagata 1380
ttaatacctt acaagagatt gtagaaggca tgaaacaaaa aatgtggagt attgaaaata 1440
ttgccttcgg ttctggtgga ggtttgctac agaagttgac aagagatctc ttgaattgtt 1500
ccttcaagtg tagctatgtt gtaactaatg gccttgggat taacgtcttc aaggacccag 1560
ttgctgatcc caacaaaagg tccaaaaagg gccgattatc tttacatagg acgccagcag 1620
ggaattttgt tacactggag gaaggaaaag gagaccttga ggaatatggt caggatcttc 1680
tccatactgt cttcaagaat ggcaaggtga caaaaagcta ttcatttgat gaaataagaa 1740
aaaatgcaca gctgaatatt gaactggaag cagcacatca ttaggcttta tgactgggtg 1800
tgtgttgtgt gtatgtaata cataatgttt attgtacaga tgtgtggggt ttgtgtttta 1860
tgatacatta cagccaaatt atttgttggt ttatggacat actgcccttt cattttttt 1920
cttttccagt gtttaggtga tctcaaatta ggaaatgcat ttaaccatgt aaaagatgag 1980
tgctaaagta agctttttag ggccctttgc caataggtag tcattcaatc tggtattgat 2040
cttttcacaa ataacagaac tgagaaactt ttatatataa ctgatgatca cataaaacag 2100
atttgcataa aattaccatg attgctttat gtttatattt aacttgtatt tttgtacaaa 2160
caagattgtg taagatatat ttgaagtttc agtgatttaa cagtctttcc aacttttcat 2220
gatttttatg agcacagact ttcaagaaaa tacttgaaaa taaattacat tgccttttgt 2280
ccattaatca gcaaataaaa catggcctta acaaagttgt ttgtgttatt gtacaatttg 2340
aaaattatgt cgggacatac cctatagaat tactaacctt actgcccctt gtagaatatg 2400
tattaatcat tctacattaa agaaaataat ggttcttact ggaatgtcta ggcactgtac 2460
agttattata tatcttggtt gttgtattgt accagtgaaa tgccaaattt gaaaggcctg 2520
tactgcaatt ttatatgtca gagattgcct gtggctctaa tatgcacctc aagattttaa 2580
ggagataatg tttttagaga gaatttctgc ttccactata gaatatatac ataaatgtaa 2640
aatacttaca aaagtggaag tagtgtattt taaagtaatt acacttctga atttattttt 2700
catattctat agttggtatg acttaaatga attactggag tgggtagtga gtgtacttaa 2760
atgtttcaat tctgttatat tttttattaa gtttttaaaa aattaaattg gatattaaat 2820
tgtatggaca tcatttatta attttaaact gaatgccctc aataagtaat actgaagcac 2880
attcttaaat gaagataaat tatctccaat gaaaagcatg acatgtgttt caatagaaga 2940
atcttaagtt ggctaaattc aaagtgcttg acatcaaaat gttctagagt gattagctac 3000
tagattctga atcatacatc acatctgact agagaccagt ttctttcgaa tgattctttt 3060
atgtatgtag atctgttctt ctgaggcagc ggttggccaa ctatagccca aaggccaaat 3120
ttggacttct ttttataaat gcagattgtc tatggctgct ttcccactac tccagcctaa 3180
ggtaaacagc tgcaatagaa gccaaatgag aatcgcaaag cccaaaatgt ttattaacct 3240
gccctttaca caaaattaca caaaaagttt cctgatctct gttctaagaa aaggagtgtg 3300
ccttgcattt aaaaggaaat gttggtttct agggaaggga ggaggctaaa taattgatac 3360
ggaattttcc tcttttgtct tctttttctt cacttaagaa tccgatactg gaagactgat 3420
ttagaaaagt ttttaacatg acattaaatg tgaaatttta aaaattgaaa agccataaat 3480
catctgtttt aaatagttac atgagaaaat gatcactaga ataacctaat tagaagtgtt 3540
atcttcatta aatgtttttt gtaagtggta ttagaaagaa tatgtttttc agatggttct 3600
ttaaacatgt agtgagaaca ataagcatta ttcactttta gtaagtcttc tgtaatccat 3660
gatataaaat aattttaaaa tgatttttta atgtatttga gtaaagatga gtagtattaa 3720
gaaaaacaca catttcttca caaaatgtgc taaggggcgt gtaaagaatc aaaagaaact 3780
attaccaata atagttttga taatcaccca taattttgtg tttaaacatt gaaattatag 3840

```
tacagacagt attctctgtg ttctgtgaat ttcagcagct tcagaataga gtttaattta 3900
gaaatttgca gtgaaaaaag ctatctcttt gttcacaacc ataaatcagg agatggagat 3960
taattctatt ggctcttagt cacttggaac tgattaattc tgactttctg tcactaagca 4020
cttggtattt ggccatctcc attctgagca ccaaacggtt aacacgaatg tccactagaa 4080
ctctgctgtg tgtcaccctt aaatcagtct aaatcttcca gacaaaagca aatggcattt 4140
atggatttaa gtcattagat tttcaactga cattaattaa tccctcttga ttgattatat 4200
catcaagtat ttatatctta aataggaggt aggatttctg tgttaagact cttatttgta 4260
ccctataatt aaagtaaaat gtttttttatg agtatccctt gttttccctt cttaaattgt 4320
tatcaaacaa tttttataat gaaatctatc ttggaaaatt agaaagaaaa atggcaaggt 4380
atttattgtt ctgtttgcca taatttagaa ctcacactta agtattttgt agttttacat 4440
tcctttttaa cccattcagt ggagaatgtc agcttttctc ccaagttgta tgttaagtct 4500
attctaatat gtactcaaca tcaagttata aacatgtaat aaacatggaa ataaagttta 4560
gctctattag tgaagtgtta aaaaaaaaaa aaa                             4593
```

<210> 62
<211> 2110
<212> DNA
<213> Homo sapiens

<400> 62

```
gttcccctgg aaggcgaccc ggacccgagc cccggaaact cctctcctct acccagggcg 60
atcgcccaag gagccctggc tgcctgacta ctgccgacgc tcacagggcg ctgcgaggcc 120
atcccccgcg tcccgcttct ccccggcccg cgggactctc tgctaacagc ctcagaaagt 180
gaggacgggg gcgcggaggc gggggcagcc tccaggcagt gctgcctctc ggttcttggg 240
aattagcccg ggggattcgc gctgggctcc gagagcccgc agaaccagcg tcgtgtcctg 300
ggcgagctgg gaggccgggg ccgagtcccg cgagtcccgc gagtgtgcct cccccggacg 360
cctaggtaaa caactgagcg cacagaggcc gagaggacat cgtgtcttcc cacgcatatt 420
cgctcacggt cactaagtag ctcttcttgg aaagagggcg attgttaaag caatagtgcg 480
actgtcgggc ttgccacggg tggagaggcg gacccacgtc aggtcgcatc tcgcctgctt 540
ttctcgaccc cgaccgcagg cgctaacccg tcttcggctt tcttcttgac ccgtacggtt 600
tgcaacgtct cttaaaacag taactcaact ctctacaaac cggggtaata tccggtgctg 660
agatcccaca ctgcaaggcg cagttccaaa cgtctggtcg gcctcgcagg tccctctttc 720
caaggcctag ccagatcttg gttttggaat ctcatttatc tgtgacccgg ggaatgcaat 780
accccccagct ccctcctact cctacattta aacaatgcct atagaagccg cttcctggga 840
agtaaggcat aaccgagacc tagcctcccg gtagggaagt tcatccgttt agtaaacagc 900
cccaactacg tgcggaccct cacggggata cgaagaattc aaccagggct ggacaagtca 960
gagtgggcca aggtaagagt tacggctgct actgtgccca cgttggaaca gatagattag 1020
ttcatctgct agttgtgtgg caagagcaaa gaattcaaga ggggaagaga aagtgagagg 1080
agcttagaga gctaagcaag aatcgaatcc tttgtgtgtc atggtaagaa attcgaactt 1140
aataccaagg tagtcagaac aaaggacaac ttttatacac gagggcgtcc tgaatattca 1200
tctaagaaac aatgtctgat caaacgggac aatccagcca gcaatcctga gtcccaccgg 1260
attatgtttg gccagctttc tagccttcac attgttaatt tattagcaat cttttcagga 1320
gaaaggcaat tattaaaatt atgtggactt ctagtaaaat tatctcgaag atttacaatt 1380
atgtatacca ataacaaaaa aactacatgt tttctgctcc cctgcctttg taggacaagt 1440
gagcattcca gagaatttct acatgaccag ctgttcattc ttttaagagg atgacattct 1500
tcagctcatc tggaaaggat acttccttta agtctgttat tcacagtctc caagggcagg 1560
tgtagttggg aaagacattt catatggaaa aacaaacttg atagggtaaa aaaaaaaaaa 1620
aaatgagatg taatctggag gcccaagtca ttttcttagg agaaactcaa atgtgtttgg 1680
tgttatctaa ccttctgcag tttatcagtc tgaaacaaat gcattctatt ctcatttgaa 1740
aatgtctgaa cagccaccaa ttttcaaaat cacccattta actagagact ttaaagccca 1800
gaatttgcag ttacgtattt cagaatgatt tagtaatttt gtgagcagct cagaggttaa 1860
gtctacaatg tctgactacg gtgcattatt gaaaatatca gtagtgtaag gatcccgtag 1920
attggtgata actttgctct taatttgtta tttcaacagt taccaccaac agttggtgac 1980
ttaacaaatt aagagcatag ttgccatatg ctcatatttg gcaaatatgt caaaatggcc 2040
atatttgcca atttttaaga caataaattc acagaaaatg cattacagaa aataacttaa 2100
aaaaaaaaag                                                        2110
```

<210> 63
<211> 937

<212> DNA
<213> Homo sapiens

<400> 63

```
agagccggcg ccgtcaccgc ccgcattgcc gctcccagtc ccgcgctcgg cacgacatga 60
aatcccccga cgaggtgcta cgcgagggcg agttggagaa gcgcagcgac agcctcttcc 120

agctatggaa gaagaagcgc ggggtgctca cctccgaccg cctgagcctg ttccccgcca 180
gcccccgcgc gcgccccaag gagctgcgct tccactccat cctcaaggtg gactgcgtgg 240
agcgcacggg caagtacgtg tacttcacca tcgtcaccac cgaccacaag gagatcgact 300
tccgctgcgc gggcgagagc tgctggaacg cggccatcgc gctggcgctc atcgatttcc 360
agaaccgccg cgccctgcag gactttcgca gccgccagga acgcaccgca cccgccgcac 420
ccgccgagga cgccgtggct gccgcggccg ccgcaccctc cgagccctcg gagccctcca 480
ggccatcccc gcagcccaaa ccccgcacgc catgagcccg ccgcgggcca tacgctggac 540
gagtcggacc gaggctagga cgtggccggc gctctccagc cctgcagcag aagaacttcc 600
cgtgcgcgcg gatcctcgct ccgttgcacg ggcgccttaa gttattggac tatctaatat 660
ctatgtattt atttcgctgg ttctttgtag tcacatattt tatagtctta atatcttgtt 720
tttgcatcac tgtgcccatt gcaaataaat cacttggcca gtttgctttt ctaccatccg 780
gctgtggctc agtgagactc ctgctgggag ggtggaggcc caggaatggg cgggcaggac 840
accctcatcc agtcctgcgg ggctggtgtg aaaggcgctg ggaaccggct ttgaatgaat 900
aaatgaatcg tgtcatctgc aaaaaaaaaa aaaaaaa                         937
```

<210> 64
<211> 623
<212> DNA
<213> Homo sapiens

<400> 64

```
aggccaagct ggactgcata aagattggta tggccttagc tcttagccaa acaccttcct 60
gacaccatga gggccagcag cttcttgatc gtggtggtgt tcctcatcgc tgggacgctg 120
gttctagagg cagctgtcac gggagttcct gttaaaggtc aagacactgt caaaggccgt 180
gttccattca atggacaaga tcccgttaaa ggacaagttt cagttaaagg tcaagataaa 240
gtcaaagcgc aagagccagt caaaggtcca gtctccacta agcctggctc ctgccccatt 300
atcttgatcc ggtgcgccat gttgaatccc cctaaccgct gcttgaaaga tactgactgc 360
ccaggaatca agaagtgctg tgaaggctct tgcgggatgg cctgtttcgt tccccagtga 420
gagggagccg gtccttgctg cacctgtgcc gtccccagag ctacaggccc catctggtcc 480
taagtccctg ctgcccttcc ccttcccaca ctgtccattc ttcctcccat tcaggatgcc 540
cacggctgga gctgcctctc tcatccactt tccaataaag agttccttct gctccaaaaa 600
aaaaaaaaaa aaaaaaaaaa aaa                                        623
```

<210> 65
<211> 5411
<212> DNA
<213> Homo sapiens

<400> 65

ctcgcgccca gcgcagtcgc tccgagcggc cgcgagcaga gccgcccagc cctgccagct 60
gcgccgggac gataaggagt caggccaggg cgggatgaca ctcattgatt ctaaagcatc 120
tttaatctgc caggcggagg gggctttgct ggtctttctt ggactattcc agagaggaca 180
actgtcatct gggaagtaac aacgcaggat gccccctggg gtggactgcc ccatggaatt 240
ctggaccaag gaggagaatc agagcgttgt ggttgacttc ctgctgccca caggggtcta 300
cctgaacttc cctgtgtccc gcaatgccaa cctcagcacc atcaagcagc tgctgtggca 360
ccgcgcccag tatgagccgc tcttccacat gctcagtggc cccgaggcct atgtgttcac 420
ctgcatcaac cagacagcgg agcagcaaga gctggaggac gagcaacggc gtctgtgtga 480
cgtgcagccc ttcctgcccg tcctgcgcct ggtggcccgt gagggcgacc gcgtgaagaa 540
gctcatcaac tcacagatca gcctcctcat cggcaaaggc ctccacgagt ttgactcctt 600
gtgcgaccca gaagtgaacg actttcgcgc caagatgtgc caattctgcg aggaggcggc 660
cgcccgccgg cagcagctgg gctgggaggc ctggctgcag tacagtttcc ccctgcagct 720
ggagccctcg gctcaaacct gggggcctgg taccctgcgg ctcccgaacc gggcccttct 780
ggtcaacgtt aagtttgagg gcagcgagga gagcttcacc ttccaggtgt ccaccaagga 840
cgtgccgctg gcgctgatgg cctgtgccct gcggaagaag gccacagtgt tccggcagcc 900
gctggtggag cagccggaag actacacgct gcaggtgaac ggcaggcatg agtacctgta 960
tggcagctac ccgctctgcc agttccagta catctgcagc tgcctgcaca gtgggttgac 1020
ccctcacctg accatggtcc attcctcctc catcctcgcc atgcgggatg agcagagcaa 1080
ccctgccccc caggtccaga aaccgcgtgc caaaccacct cccattcctg cgaagaagcc 1140
ttcctctgtg tccctgtggt ccctggagca gccgttccgc atcgagctca tccagggcag 1200
caaagtgaac gccgacgagc ggatgaagct ggtggtgcag gccgggcttt tccacggcaa 1260
cgagatgctg tgcaagacgg tgtccagctc ggaggtgagc gtgtgctcgg agcccgtgtg 1320
gaagcagcgg ctggagttcg acatcaacat ctgcgacctg ccccgcatgg cccgtctctg 1380
ctttgcgctg tacgccgtga tcgagaaagc caagaaggct cgctccacca agaagaagtc 1440
caagaaggcg gactgcccca ttgcctgggc caacctcatg ctgtttgact acaaggacca 1500
gcttaagacc ggggaacgct gcctctacat gtggccctcc gtcccagatg agaagggcga 1560

```
gctgctgaac cccacgggca ctgtgcgcag taaccccaac acggatagcg ccgctgccct 1620
gctcatctgc ctgcccgagg tggccccgca ccccgtgtac taccccgccc tggagaagat 1680
cttggagctg ggcgacacac gcgagtgtgt gcatgtcacc gaggaggagc agctgcagct 1740
gcgggaaatc ctggagcggc gggggtctgg ggagctgtat gagcacgaga aggacctggt 1800
gtggaagctg cggcatgaag tccaggagca cttcccggag gcgctagccc ggctgctgct 1860
ggtcaccaag tggaacaagc atgaggatgt ggcccagatg ctctacctgc tgtgctcctg 1920
gccggagctg cccgtcctga gcgccctgga gctgctagac ttcagcttcc ccgattgcca 1980
cgtaggctcc ttcgccatca agtcgctgcg gaaactgacg gacgatgagc tgttccagta 2040
cctgctgcag ctggtgcagg tgctcaagta cgagtcctac ctggactgcg agctgaccaa 2100
attcctgctg gaccgggccc tggccaaccg caagatcggc cacttccttt tctggcacct 2160
ccgctccgag atgcacgtgc cgtcggtggc cctgcgcttc ggcctcatcc tggaggccta 2220
ctgcaggggc agcacccacc acatgaaggt gctgatgaag caggggggaag cactgagcaa 2280
actgaaggcc ctgaatgact tcgtcaagct gagctctcag aagaccccca agccccagac 2340
caaggagctg atgcacttgt gcatgcggca ggaggcctac ctagaggccc tctcccacct 2400
gcagtcccca ctcgacccca gcaccctgct ggctgaagtc tgcgtggagc agtgcacctt 2460
catggactcc aagatgaagc ccctgtggat catgtacagc aacgaggagg caggcagcgg 2520
cggcagcgtg ggcatcatct ttaagaacgg ggatgacctc cggcaggaca tgctgaccct 2580
gcagatgatc cagctcatgg acgtcctgtg gaagcaggag gggctggacc tgaggatgac 2640
cccctatggc tgcctcccca ccggggaccg cacaggcctc attgaggtgg tactccgttc 2700
agacaccatc gccaacatcc aactcaacaa gagcaacatg gcagccacag ccgccttcaa 2760
caaggatgcc ctgctcaact ggctgaagtc caagaacccg ggggaggccc tggatcgagc 2820
cattgaggag ttcaccctct cctgtgctgg ctattgtgtg gccacatatg tgctgggcat 2880
tggcgatcgg cacagcgaca acatcatgat ccgagagagt gggcagctgt tccacattga 2940
ttttggccac tttctgggga atttcaagac caagtttgga atcaaccgcg agcgtgtccc 3000
attcatcctc acctacgact ttgtccatgt gattcagcag gggaagacta ataatagtga 3060
gaaatttgaa cggttccggg gctactgtga aagggcctac accatcctgc ggcgccacgg 3120
gcttctcttc ctccacctct ttgccctgat gcgggcggca ggcctgcctg agctcagctg 3180
ctccaaagac atccagtatc tcaaggactc cctggcactg gggaaaacag aggaggaggc 3240
actgaagcac ttccgagtga agtttaacga agccctccgt gagagctgga aaaccaaagt 3300
gaactggctg gcccacaacg tgtccaaaga caacaggcag tagtggctcc tcccagccct 3360
gggcccaaga ggaggcggct gcgggtcgtg gggaccaagc acattggtcc taaaggggct 3420
gaagagcctg aactgcacct aacgggaaag aaccgacatg gctgcctttt gtttacactg 3480
gttatttatt tatgacttga aatagtttaa ggagctaaac agccataaac ggaaacgcct 3540
ccttcatgca gcggcggtgc tgggcccccc gaggctgcac ctggctctcg gctgaggatt 3600
gtcaccccaa gtcttccagc tggtggatct gggcccagca aagactgttc tcctcccgag 3660
ggaaccttct tcccaggcct cccgccagac tgcctgggtc ctggcgcctg gcggtcacct 3720
ggtgcctact gtccgacagg atgccttgat cctcgtgcga cccaccctgt gtatcctccc 3780
tagactgagt tctggcagct ccccgaggca gccggggtac cctctagatt cagggatgct 3840
tgctctccac ttttcaagtg ggtcttgggt acgagaattc cctcatcttt ctctactgta 3900
aagtgatttt gtttgcaggt aagaaaataa tagatgactc accacacctc tacggctggg 3960
gagatcaggc ccagccccat aaaggagaat ctacgctggt cctcaggacg tgttaaagag 4020
atctgggcct catgtagctc accccggtca cgcatgaagg caaaagcagg tcagaagcga 4080
atactctgcc attatctcaa aaatcttttt tttttttttg agatggggtc ttcctctgtt 4140
gcccaggctg gagtgcagtg gtgcaatctt ggctcactgt aacctccgcc tcccaggttc 4200
aagtgattct tctgcctcag cctcctgagt agctgggatt acaggtgtgc accaccgtac 4260
ccagctaatt tttgtatttt agtagagacg gggtttcac catgttggct gggctggtct 4320
cgaactcctg acctcaggtg atccacccgc ctgagcctcc caaagtgctg ggattacagg 4380
catgagccac cgcgcccggc ccactctgcc attgtctaag ccacctctga aagcaggttt 4440
taacaaaagg atgaggccag aactcttcca gaaccatcac ctttgggaac ctgctgtgag 4500
agtgctgagg taccagaagt gtgagaacga gggggcgtgc tgggatcttt ctctctgact 4560
atacttagtt tgaaatggtg caggcttagt cttaagcctc caaaggcctg gatttgagca 4620
gctttagaaa tgcaggttct agggcttctc ccagccttca gaagccaact aactctgcag 4680
atggggctag gactgtgggc ttttagcagc ccacaggtga tcctaacata tcaggccatg 4740
gactcaggac ctgcccggtg atgctgttga tttctcaaag gtcttccaaa actcaacaga 4800
gccagaagta gccgcccgct cagcggctca ggtgccagct ctgttctgat tcaccagggg 4860
tccgtcagta gtcattgcca cccgcggggc acctccctgg ccacacgcct gttcccagca 4920
agtgctgaaa ctcactagac cgtctgcctg tttcgaaatg gggaaagccg tgcgtgcgcg 4980
ttatttattt aagtgcgcct gtgtgcgcgg gtgtgggagc acactttgca aagccacagc 5040
gtttctggtt ttgggtgtac agtcttgtgt gcctggcgag aagaatattt tctatttttt 5100
taagtcattt catgtttctg tctggggaag gcaagttagt taagtatcac tgatgtgggt 5160
tgagaccagc actctgtgaa accttgaaat gagaagtaaa ggcagatgaa aagaaagaaa 5220
aagccttttt atgttctttt atgttctcgg ctcaaaaaga aacaagggag tgtaggttta 5280
aaaccaaaac aggagagaag acaaaccccg ctccggctgg agttagttag aaccagaact 5340
ttattgtagc ggatacactt tctgacctat catgagtata cacatctgcg aagggaaacc 5400
gcgcggcgac a                                                      5411
```

<210> 66
<211> 2684
<212> DNA
<213> Homo sapiens

<400> 66

```
cccgagagga gtcggtggca gcggcggcgg cgggaccggc agcagcagca gcagcagcag 60
cagcaaccac tagcctcctg ccccgcggcg ctgccgcacg agccccacga gccgctcacc 120
ccgccgttct cagcgctgcc cgaccccgct ggcgcgccct cccgccgcca gtcccggcag 180
cgccctcagt tgtcctccga ctcgccctcg gccttccgcg ccagccgcag ccacagccgc 240
aacgccaccc gcagccacag ccacagccac agccccaggc atagccttcg gcacagcccc 300
ggctccggct cctgcggcag ctcctctggg caccgtccct gcgccgacat cctggaggtt 360
gggatgctct tgtccaaaat caactcgctt gcccacctgc gcgccgcgcc ctgcaacgac 420
ctgcacgcca ccaagctggc gcccggcaag gagaaggagc ccctggagtc gcagtaccag 480
gtgggcccgc tactgggcag cggcggcttc ggctcggtct actcaggcat ccgcgtctcc 540
gacaacttgc cggtggccat caaacacgtg gagaaggacc ggatttccga ctggggagag 600
ctgcctaatg gcactcgagt gcccatggaa gtggtcctgc tgaagaaggt gagctcgggt 660
ttctccggcg tcattaggct cctggactgg ttcgagaggc ccgacagttt cgtcctgatc 720
ctggagaggc ccgagccggt gcaagatctc ttcgacttca tcacggaaag gggagccctg 780
caagaggagc tggcccgcag cttcttctgg caggtgctgg aggccgtgcg gcactgccac 840
aactgcgggg tgctccaccg cgacatcaag gacgaaaaca tccttatcga cctcaatcgc 900
ggcgagctca agctcatcga cttcgggtcg ggggcgctgc tcaaggacac cgtctacacg 960
gacttcgatg ggacccgagt gtatagccct ccagagtgga tccgctacca tcgctaccat 1020
ggcaggtcgg cggcagtctg gtccctgggg atcctgctgt atgatatggt gtgtggagat 1080
attcctttcg agcatgacga agagatcatc aggggccagg ttttcttcag gcagagggtc 1140
tcttcagaat gtcagcatct cattagatgg tgcttggccc tgagaccatc agataggcca 1200
accttcgaag aaatccagaa ccatccatgg atgcaagatg ttctcctgcc ccaggaaact 1260
gctgagatcc acctccacag cctgtcgccg gggcccagca aatagcagcc tttctggcag 1320
gtcctcccct ctcttgtcag atgcccgagg gaggggaagc ttctgtctcc agcttcccga 1380
gtaccagtga cacgtctcgc caagcaggac agtgcttgat acaggaacaa catttacaac 1440
tcattccaga tcccaggccc ctggaggctg cctcccaaca gtggggaaga gtgactctcc 1500
aggggtccta ggcctcaact cctcccatag atactctctt cttctcatag gtgtccagca 1560
ttgctggact ctgaaatatc ccgggggtgg ggggtggggg tgggtcagaa ccctgccatg 1620
gaactgtttt cttcatcatg agttctgctg aatgccgcga tgggtcaggt agggggggaaa 1680
caggttggga tgggatagga ctagcaccat tttaagtccc tgtcacctct tccgactctt 1740
tctgagtgcc ttctgtgggg actccggctg tgctgggaga aatacttgaa cttgcctctt 1800
ttacctgctg cttctccaaa aatctgcctg ggttttgttc cctatttttc tctcctgtcc 1860
tccctcaccc cctccttcat atgaaaggtg ccatggaaga ggctacaggg ccaaacgctg 1920
agccacctgc cctttttttct gcctccttta gtaaaactcc gagtgaactg gtcttccttt 1980
ttggttttta cttaactgtt tcaaagccaa gacctcacac acacaaaaaa tgcacaaaca 2040
atgcaatcaa cagaaaagct gtaaatgtgt gtacagttgg catggtagta tacaaaaaga 2100
ttgtagtgga tctaattttt aagaaatttt gcctttaagt tattttacct gtttttgttt 2160
cttgttttga aagatgcgca ttctaacctg gaggtcaatg ttatgtattt atttatttat 2220
ttatttggtt cccttcctat tccaagcttc catagctgct gccctagttt tctttcctcc 2280
tttcctcctc tgacttgggg accttttggg ggagggctgc gacgcttgct ctgtttgtgg 2340
ggtgacggga ctcaggcggg acagtgctgc agctccctgg cttctgtggg gcccctcacc 2400
tacttaccca ggtgggtccc ggctctgtgg gtgatgggga ggggcattgc tgactgtgta 2460
tataggataa ttatgaaaag cagttctgga tggtgtgcct tccagatcct ctctggggct 2520
gtgttttgag cagcaggtag cctgctggtt ttatctgagt gaaatactgt acaggggaat 2580
aaaagagatc ttattttttt ttttatactt ggcgtttttt gaataaaaac cttttgtctt 2640
aaaaaaaaaa aaaaaaaaaa aaaaaaaaaa aaaaaaaaaa aaaa                  2684
```

<210> 67
<211> 1437
<212> DNA
<213> Homo sapiens

<400> 67

```
cagggccgag ccagcccctt caccaccagc cggccgcgcc ccgggaaggg aagtttgtgg 60
cggaggaggt tcgtacggga ggagggggag gcgcccacgc atctggggct gactcgctct 120
ttcgcaaaac gtctgggagg agtccctggg gccacaaaac tgcctccttc ctgaggccag 180
aaggagagaa gacgtgcagg gaccccgcgc acaggagctg ccctcgcgac atgggtcacc 240
cgccgctgct gccgctgctg ctgctgctcc acacctgcgt cccagcctct tggggcctgc 300
ggtgcatgca gtgtaagacc aacggggatt gccgtgtgga agagtgcgcc ctgggacagg 360

acctctgcag gaccacgatc gtgcgcttgt gggaagaagg agaagagctg gagctggtgg 420
agaaaagctg tacccactca gagaagacca acaggaccct gagctatcgg actggcttga 480
agatcaccag ccttaccgag gttgtgtgtg ggttagactt gtgcaaccag ggcaactctg 540
gccgggctgt cacctattcc cgaagccgtt acctcgaatg catttcctgt ggctcatcag 600
acatgagctg tgagaggggc cggcaccaga gcctgcagtg ccgcagccct gaagaacagt 660
gcctggatgt ggtgacccac tggatccagg aaggtgaaga agggcgtcca aaggatgacc 720
gccacctccg tggctgtggc taccttcccg gctgcccggg ctccaatggt ttccacaaca 780
acgacacctt ccacttcctg aaatgctgca acaccaccaa atgcaacgag ggcccaatcc 840
tggagcttga aaatctgccg cagaatggcc gccagtgtta cagctgcaag ggggaacagca 900
cccatggatg ctcctctgaa gagactttcc tcattgactg ccgaggcccc atgaatcaat 960
gtctggtagc caccggcact cacgaacgct cactctgggg aagctggttg ccatgtaaaa 1020
gtactactgc cctgagacca ccatgctgtg aggaagccca agctactcat gtataaatgc 1080
catgtggaga tagagcccca gatgtttcag ccatctcagc ccaggcacca gacaagtggg 1140
tgaagaagcc accttggaca tgtagcccca gcagatgtga tatagagaag aaacaggaaa 1200
cttggctata ttagtttcct agggctgcct gtgataaatt attacaaact ttataaacta 1260
acacattgtg tgcctatatc aaaacatcat ggaaggacag gcacagtggc tcatgcctgt 1320
agtcctagca ctttgggagg gtgagaaagg aagatctctt gagctcagga gttcaagatc 1380
agcctgggca acacagtgag acctcatctc cactaaaaat aaaaaaaaat tggctgg    1437
```

<210> 68
<211> 4788
<212> DNA
<213> Homo sapiens

<400> 68

accgcccta gccccgcgct ccggaagtga agcggccaga ccaccagcta atggatgcgg 60
agcggagggc ccgctgaccg ctctccgcgc ctggagcagc ttggcttggc tggagctaag 120
agccagacac accactgtgt ggaggtgggt gatgtcttcc tgtgctaaaa ggtgaataaa 180
taagctcctc acctctcgcg gaacactcgg gaacacatca acaggggtcc aagccgccct 240
gctgggaggc ttctcttcaa gagttctggg tcccagagtg gaaggcattt tcccatcaac 300
tggagagaga cgaaacatca gagaccagga ggctgtggag aaagcagctg tcccaggtgc 360
ctcaactatc agagaagggt cagcgtcacg tggctgccag catctttgag aaaatcactg 420
gcaatcggac ttcagagctg cgggcacagg tgtggttaga actgagatac gacctgccca 480
cctgggtcag gcctaaagac aagaagtcct gagttcttgc cactgagtag gccagggtca 540
tttgtccaga aaactttgtg actgtctttg agtgacctag tctgggaccc attcattggt 600
gggttctaag gttagaagct catccaggat attttcaata ttaagtcagt gcatagctgc 660
accactaaca aattggtgcc tgtagagtca gagtgggtca attcttagga caatggcgct 720
ggcactgtta gaggactggt gcaggataat gagtgtggat gagcagaagt cactgatggt 780
tacggggata ccggcggact ttgaggaggc tgagattcag gaggtccttc aggagacttt 840
aaagtctctg ggcaggtata gactgcttgg caagatattc cggaagcagg agaatgccaa 900
tgctgtctta ctagagcttc tggaagatac tgatgtctcg gccattccca gtgaggtcca 960
gggaaagggg ggtgtctgga aggtgatctt taagacccct aatcaggaca ctgagtttct 1020
tgaaagattg aacctgtttc tagaaaaaga ggggcagacg gtctcgggta tgtttcgagc 1080
cctggggcag gagggcgtgt ctccagccac agtgccctgc atctcaccag aattactggc 1140
ccatttgttg ggacaggcaa tggcacatgc gcctcagccc ctgctaccca tgagataccg 1200
gaaactgcga gtattctcag ggagtgctgt cccagcccca gaggaagagt cctttgaggt 1260
ctggttggaa caggccacgg agatagtcaa agagtggcca gtaacagagg cagaaaagaa 1320
aaggtggctg gcggaaagcc tgcggggccc tgccctggac ctcatgcaca tagtgcaggc 1380
agacaacccg tccatcagtg tagaagagtg tttggaggcc tttaagcaag tgtttgggag 1440
cctagagagc cgcaggacag cccaggtgag gtatctgaag acctatcagg aggaaggaga 1500
gaaggtctca gcctatgtgt tacggctaga aaccctgctc cggagagcgg tggagaaacg 1560
cgccatccct cggcgtattg cggaccaggt ccgcctggag caggtcatgg ctggggccac 1620
tcttaaccag atgctgtggt gccggcttag ggagctgaag gatcagggcc cgccccccag 1680
cttccttgag ctaatgaagg taatacggga agaagaggag gaagaggcct cctttgagaa 1740
tgagagtatc gaagagccag aggaacgaga tggctatggc cgctggaatc atgagggaga 1800
cgactgaaaa ccacctgggg gcaggaccca cagccagtgg gctaagacct ttaaaaaatt 1860
tttttcttta atgtatggga ctgaaatcaa accatgaaag ccaattattg accttccttc 1920
cttccttcct tccctccctt cctccttctc tccttctctc ctcctctctc ctctcctctc 1980
ctctctttcc ttccttcctt cctttttttct ttttctcttt cttctttatt tcttgggtct 2040
cactctcatc acccaggcta gagtgcagtg gcacaaaaat ctcggctcac tgcagccttg 2100
acttcccagg ctcaggctca ggtgatcctc acaccttagc ctcccaagta cctgggacta 2160
caggcacgca ccaccatgcc tagctattct tttgtatttt tggtagagac agggttttgc 2220
tgtgttgctc aggctggtct ggaacccccta ggctcaaatg atgtgcccaa ctcggcctcc 2280
caaagtgctg ggattacagg catgaaccgc catgcctggc ccttgatttt tctttttaag 2340
aaaaaaatat ctaggagttt cttagaccct atgtagatta ttaatgaaca aaagattaaa 2400

```
ctccaaatat taaatagtaa gcctgaagga atctgaaaca cttgtacttc caattttctt 2460
taaataatcc caaatagacc agaattggcc cataccatag aagaaagaat tggcagtcaa 2520
aaaaaaaaat accttttgta atgtttgaaa aataaagctg tttgacttgt caggtgtttt 2580
cctttctcaa atcagcaaat tctctctgag tgcctggctt tgtgagacac tgtacaagga 2640
gttacaagac tacagctata acctgcagtt gagcagttat aaacctacaa aatgggccct 2700
gccctcagag aggttccagt ctagatgagg agctgatcta gacaggtaaa aggctaacta 2760
accctttgtg taaataagtt catcacccca gtaaaagtgt catcacccag tgaataggac 2820
cacctctgcc tgcagatttt tgttgttgtt gttgtcattg ttgttgttgt tttaacctgg 2880
gaagtgttct tcctgccttt ctgctaggtg tcagatagat ggtcccagag ctaggtgctg 2940
tgtcaggccc tgaagacaca gatgactcaa cctaagcttt actttccaga ggtccacagc 3000
ctgagaggtg tccccaaaga aagggggaca tgaggggact gcatgcttga gagcagggtt 3060
gtttagggca ggtttggatt tagtgagcag gctggtttgc ttagagaagg cttttagtgg 3120
caacaaagga tgaagaggag agaaaaggaa ctcacattta ttgagggcct actgtgtgca 3180
aagtgtttca tgtatatctc attgaatgta tacagccacc ctgttgtggt ataattttgc 3240
tctttataaa gagaaagacc gaagctcaga tgagttaagt ggtctcctca acaccaaaat 3300
gccaagaagt gatggagcct agacagaagc ccagaacttt ctgactcaca ctagtccatc 3360
ctctaccatc acgatgactt tcaaattgtg ctctgcagtt ctgcagattt tctagcagtg 3420
ccatctccaa aatgtgtttt aaactcttta ttttttttaat tattattagt attattttga 3480
gactgagtct tgctctatca cccaggctgg agtgcagtgg tgcaatctca gctcactgca 3540
acctccgcct cccaggttca agcgatttcg tgcctcagcc tcccgagtag ctgggattac 3600
aggcacccac caccacgccc agctaatttt tgtattttta gtagaaatgg ggtttcacca 3660
tgttggccag gctggtctcg aactcctgac ctcaagtgat ccactcacct cggcctccca 3720
aagtgctggg attacaggtg tgagccacca tgcctgggct aaactcttta agtctctagt 3780
aaatgcagct agattcaaat gggctgataa ccaaatttta acacatcagc attcaccacc 3840
aggtttactt ttattttcag attggctcat tttgtgcaga ccttagagca aagtttcctt 3900
tatggtatct gtgtacgtat ccaaacttct tttaattgtt cacagatttt aaaagcggta 3960
gcaccacatg gttgtgtaga tcagacctgt gtatttagat cagacctgtg tatcacgtaa 4020
gtgtgtgagt gcagtgcaga tgagcaccat ttagttatat gtgctaggca aatctccaac 4080
acagttgatg tgtagtcttg tggtagattt gtgcatactg taagcaaatt gcttagcttc 4140
tctagacatc agtttccaca tctgaaaaat aagaagatga gagtacacgg ttgttatgaa 4200
caaatgactt aatgcttttt aagcacgttg catgacatct ggaacacaga aagccctcaa 4260
tacattgaag ctcttaggat tttcacgatg ttcctgtctg ctcaatgcat gctttcttta 4320
ttgttctgac agttgtgtgg taacaagcta atatgcttcc agttgacttc cagtctaccc 4380
tggtgttaga aaccgtttca tctcttattg taaatttgag tgcttgttgt tttttatatt 4440
tgtgatgact cttccagcag ttgttgacaa ttgttagagg tttgactttt aaataattac 4500
ttattttttc tgattgtggt tcagtttaac tgaagaatat cctgagattg taagaaaagc 4560
atttttttaaa aggtatcact tgtgatcatt tatctttcta aattctattt ttaatactgt 4620
tccaccaaag tgatgcagtg gttaccatga caccctaatt tcatgtgttt ttgtatttat 4680
gaaaatagtt tcattgtcat ttattggcgg tatacaaagt aaaatgttat aaatgtgaag 4740
ttataaaata aatatatgct aataaaatcc tgagtttttc tgtttcct              4788
```

<210> 69
<211> 2399
<212> DNA
<213> Homo sapiens

<400> 69

```
ataaaagcct agtggccatt gtgttcgttg ctcttatcgg ttcccatccc agttgttgat 60
cttatgcaag acgctgcacg accccgcgcc cgcttgtcgc cacggcactt gaggcagccg 120
gagatactct gagttactcg gagcccgacg cctgagggtg agatgaacgc gctggcctcc 180
ctaaccgtcc ggacctgtga tcgcttctgg cagaccgaac cggcgctcct gcccccgggg 240
tgacgcgcag ctcccagccg cccagacaca tggccccagg ccaagcaccc catcaggcta 300
ccccgtggag ggatgcccac cctttcttcc tcctgtcccc agtgatgggc ctcctcagcc 360
gcgcctggag ccgcctgagg ggcctgggac ctctagagcc ctggctggtg gaagcagtaa 420
aaggagcagc tctggtagaa gctggcctgg agggagaagc taggactcct ctggcaatcc 480
cccatacccc ttggggcaga cgccctgaag aggaggctga agacagtgga ggccctggag 540
aggacagaga aacactgggg ctgaaaacca gcagttccct tcctgaagcc tggggacttt 600
tggatgatga tgatggcatg tatggtgagc gagaggcaac cagtgtccct agagggcagg 660
gaagtcaatt tgcagatggc cagcgtgctc ccctgtctcc cagccttctg ataaggacac 720
tgcaaggttc tgataagaac ccaggggagg agaaagccga ggaagaggga gttgctgaag 780
aggagggagt taacaagttc tcttatccac catcacaccg ggagtgttgt ccagccgtgg 840
aggaggagga cgatgaagaa gctgtaaaga aagaagctca cagaacctct acttctgcct 900
tgtctccagg atccaagccc agcacttggg tgtcttgccc aggggaggaa gagaatcaag 960
ccacggagga taaaagaaca gaaagaagta aaggagccag gaagacctcc gtgtcccccc 1020
gatcttcagg ctccgacccc aggtcctggg agtatcgttc aggagaggcg tccgaggaga 1080

aggaggaaaa ggcacacaaa gaaactggga aaggagaagc tgccccaggg ccgcaatcct 1140
cagccccagc ccagaggccc cagctcaagt cctggtggtg ccaacccagt gatgaagagg 1200
agggtgaggt caaggctttg gggggcagctg agaaggatgg agaagctgag tgtcctccct 1260
gcatcccccc accaagtgcc ttcctgaagg cctgggtgta ttggccagga gaggacacag 1320
aggaagagga agatgaggaa gaagatgagg acagtgactc tggatcagat gaggaagagg 1380
gagaagctga ggcttcctct tccactcctg ctacaggtgt cttcttgaag tcctgggtct 1440
atcagccagg agaggacaca gaggaggagg aagatgagga cagtgataca ggatcagccg 1500
aggatgaaag agaagctgag acttctgctt ccacaccccc tgcaagtgct ttcttgaagg 1560
cctgggtgta tcggccagga gaggacacgg aggaggagga agatgaggat gtggatagtg 1620
aggataagga agatgattca gaagcagcct tgggagaagc tgagtcagac ccacatccct 1680
cccacccgga ccagagggcc cacttcaggg gctggggata tcgacctgga aaagagacag 1740
aggaagagga agctgctgag gactggggag aagctgagcc ctgccccttc cgagtggcca 1800
tctatgtacc tggagagaag ccaccgcctc cctgggctcc tcctaggctg cccctccgac 1860
tgcaaaggcg gctcaagcgc ccagaaaccc ctactcatga tccggaccct gagactcccc 1920
taaaggccag aaaggtgcgc ttctccgaga aggtcactgt ccatttcctg gctgtctggg 1980
cagggccggc ccaggccgcc cgccagggcc cctgggagca gcttgctcgg gatcgcagcc 2040
gcttcgcacg ccgcatcacc caggcccagg aggagctgag cccctgcctc acccctgctg 2100
cccgggccag agcctgggca cgcctcagga acccaccttt agcccccatc cctgccctca 2160
cccagacctt gccttcctcc tctgtccctt cgtccccagt ccagaccacg cccttgagcc 2220
aagctgtggc cacaccttcc cgctcgtctg ctgctgcagc ggctgccctg gacctcagtg 2280
ggaggcgtgg ctgagaccaa ctggtttgcc tataatttat taactattta ttttttctaa 2340
gtgtgggttt atataaggaa taaagccttt tgatttgtag cgaaaaaaaa aaaaaaaaa  2399
```

<210> 70
<211> 2751
<212> DNA
<213> Homo sapiens

<400> 70

```
attaaagatg atttttacag tcaatgagcc acgtcaggga gcgatggcac ccgcaggcgg 60
tatcaactga tgcaagtgtt caagcgaatc tcaactcgtt ttttccggtg actcattccc 120
ggccctgctt ggcagcgctg caccctttaa cttaaacctc ggccggccgc ccgccggggg 180
cacagagtgt gcgccgggcc gcgcggcaat tggtccccgc gccgacctcc gcccgcgagc 240
gccgccgctt cccttccccg ccccgcgtcc ctccccctcg gccccgcgcg tcgcctgtcc 300
tccgagccag tcgctgacag ccgcggcgcc gcgagcttct cctctcctca cgaccgagag 360
cagtcattat ggcgaacctt ggctgctgga tgctggttct ctttgtggcc acatggagtg 420
acctgggcct ctgcaagaag cgcccgaagc ctggaggatg gaacactggg ggcagccgat 480
acccggggca gggcagccct ggaggcaacc gctacccacc tcagggcggt ggtggctggg 540
ggcagcctca tggtggtggc tgggggcagc ctcatggtgg tggctggggg cagccccatg 600
gtggtggctg ggggacagcct catggtggtg gctggggtca aggaggtggc acccacagtc 660
agtggaacaa gccgagtaag ccaaaaacca acatgaagca catggctggt gctgcagcag 720
ctggggcagt ggtgggggc cttggcggct acatgctggg aagtgccatg agcaggccca 780
tcatacattt cggcagtgac tatgaggacc gttactatcg tgaaaacatg caccgttacc 840
ccaaccaagt gtactacagg cccatggatg agtacagcaa ccagaacaac tttgtgcacg 900
actgcgtcaa tatcacaatc aagcagcaca cggtcaccac aaccaccaag ggggagaact 960
tcaccgagac cgacgttaag atgatggagc gcgtggttga gcagatgtgt atcacccagt 1020
acgagaggga atctcaggcc tattaccaga gaggatcgag catggtcctc ttctcctctc 1080
cacctgtgat cctcctgatc tctttcctca tcttcctgat agtgggatga ggaaggtctt 1140
cctgttttca ccatctttct aatctttttc cagcttgagg gaggcggtat ccacctgcag 1200
ccctttagt ggtggtgtct cactctttct tctctctttg tcccggatag gctaatcaat 1260
acccttggca ctgatgggca ctggaaaaca tagagtagac ctgagatgct ggtcaagccc 1320
cctttgattg agttcatcat gagccgttgc taatgccagg ccagtaaaag tataacagca 1380
aataaccatt ggttaatctg gacttatttt tggacttagt gcaacaggtt gaggctaaaa 1440
caaatctcag aacagtctga aatacctttg cctggatacc tctggctcct tcagcagcta 1500
gagctcagta tactaatgcc ctatcttagt agagatttca tagctattta gagatatttt 1560
ccattttaag aaaacccgac aacatttctg ccaggtttgt taggaggcca catgatactt 1620
attcaaaaaa atcctagaga ttcttagctc ttgggatgca ggctcagccc gctggagcat 1680
gagctctgtg tgtaccgaga actggggtga tgttttactt ttcacagtat gggctacaca 1740
gcagctgttc aacaagagta aatattgtca caacactgaa cctctggcta gaggacatat 1800
tcacagtgaa cataactgta acatatatga aaggcttctg ggacttgaaa tcaaatgttt 1860
gggaatggtg cccttggagg caacctccca ttttagatgt ttaaaggacc ctatatgtgg 1920
cattcctttc tttaaactat aggtaattaa ggcagctgaa aagtaaattg ccttctagac 1980
actgaaggca aatctccttt gtccatttac ctggaaacca gaatgatttt gacatacagg 2040
agagctgcag ttgtgaaagc accatcatca tagaggatga tgtaattaaa aaatggtcag 2100
tgtgcaaaga aaagaactgc ttgcatttct ttatttctgt ctcataattg tcaaaaacca 2160

gaattaggtc aagttcatag tttctgtaat tggcttttga atcaaagaat agggagacaa 2220
tctaaaaaat atcttaggtt ggagatgaca gaaatatgat tgatttgaag tggaaaaaga 2280
aattctgtta atgttaatta aagtaaaatt attccctgaa ttgtttgata ttgtcaccta 2340
gcagatatgt attacttttc tgcaatgtta ttattggctt gcactttgtg agtattctat 2400
gtaaaaatat atatgtatat aaaatatata ttgcatagga cagacttagg agttttgttt 2460
agagcagtta acatctgaag tgtctaatgc attaactttt gtaaggtact gaatacttaa 2520
tatgtgggaa acccttttgc gtggtcctta ggcttacaat gtgcactgaa tcgtttcatg 2580
taagaatcca aagtggacac cattaacagg tctttgaaat atgcatgtac tttatatttt 2640
ctatatttgt aactttgcat gttcttgttt tgttatataa aaaaattgta aatgtttaat 2700
atctgactga aattaaacga gcgaagatga gcaccaaaaa aaaaaaaaaa a          2751
```

<210> 71
<211> 4465
<212> DNA
<213> Homo sapiens

<400> 71

```
caattgtcat acgacttgca gtgagcgtca ggagcacgtc caggaactcc tcagcagcgc 60
ctccttcagc tccacagcca gacgccctca gacagcaaag cctacccccg cgccgcgccc 120
tgcccgccgc tcggatgctc gcccgcgccc tgctgctgtg cgcggtcctg gcgctcagcc 180
atacagcaaa tccttgctgt tcccacccat gtcaaaaccg aggtgtatgt atgagtgtgg 240
gatttgacca gtataagtgc gattgtaccc ggacaggatt ctatggagaa aactgctcaa 300
caccggaatt tttgacaaga ataaaattat ttctgaaacc cactccaaac acagtgcact 360
acatacttac ccacttcaag ggattttgga acgttgtgaa taacattccc ttccttcgaa 420
atgcaattat gagttatgtc ttgacatcca gatcacattt gattgacagt ccaccaactt 480
acaatgctga ctatggctac aaaagctggg aagccttctc taacctctcc tattatacta 540
gagcccttcc tcctgtgcct gatgattgcc cgactccctt gggtgtcaaa ggtaaaaagc 600
agcttcctga ttcaaatgag attgtggaaa aattgcttct aagaagaaag ttcatccctg 660
atccccaggg ctcaaacatg atgtttgcat tctttgccca gcacttcacg catcagtttt 720
tcaagacaga tcataagcga gggccagctt tcaccaacgg gctgggccat ggggtggact 780
taaatcatat ttacggtgaa actctggcta gacagcgtaa actgcgcctt ttcaaggatg 840
gaaaaatgaa atatcagata attgatggag agatgtatcc tcccacagtc aaagatactc 900
aggcagagat gatctaccct cctcaagtcc ctgagcatct acggtttgct gtggggcagg 960
aggtctttgg tctggtgcct ggtctgatga tgtatgccac aatctggctg cgggaacaca 1020
acagagtatg cgatgtgctt aaacaggagc atcctgaatg gggtgatgag cagttgttcc 1080
agacaagcag gctaatactg ataggagaga ctattaagat tgtgattgaa gattatgtgc 1140
aacacttgag tggctatcac ttcaaactga aatttgaccc agaactactt ttcaacaaac 1200
aattccagta ccaaaatcgt attgctgctg aatttaacac cctctatcac tggcatcccc 1260
ttctgcctga cacctttcaa attcatgacc agaaatacaa ctatcaacag tttatctaca 1320
acaactctat attgctggaa catggaatta cccagtttgt tgaatcattc accaggcaaa 1380
ttgctggcag ggttgctggt ggtaggaatg ttccacccgc agtacagaaa gtatcacagg 1440
cttccattga ccagagcagg cagatgaaat accagtcttt taatgagtac cgcaaacgct 1500
ttatgctgaa gccctatgaa tcatttgaag aacttacagg agaaaaggaa atgtctgcag 1560
agttggaagc actctatggt gacatcgatg ctgtggagct gtatcctgcc cttctggtag 1620
aaaagcctcg gccagatgcc atctttggtg aaaccatggt agaagttgga gcaccattct 1680
ccttgaaagg acttatgggt aatgttatat gttctcctgc ctactggaag ccaagcactt 1740
ttggtggaga agtgggtttt caaatcatca acactgcctc aattcagtct ctcatctgca 1800
ataacgtgaa gggctgtccc tttacttcat tcagtgttcc agatccagag ctcattaaaa 1860
cagtcaccat caatgcaagt tcttcccgct ccggactaga tgatatcaat cccacagtac 1920
tactaaaaga acgttcgact gaactgtaga agtctaatga tcatatttat ttatttatat 1980
gaaccatgtc tattaattta attatttaat aatatttata ttaaactcct tatgttactt 2040
aacatcttct gtaacagaag tcagtactcc tgttgcggag aaaggagtca tacttgtgaa 2100
gacttttatg tcactactct aaagattttg ctgttgctgt taagtttgga aaacagtttt 2160
tattctgttt tataaaccag agagaaatga gttttgacgt ctttttactt gaatttcaac 2220
ttatattata agaacgaaag taaagatgtt tgaatactta aacactatca caagatggca 2280
aaatgctgaa agtttttaca ctgtcgatgt ttccaatgca tcttccatga tgcattagaa 2340
gtaactaatg tttgaaattt taaagtactt ttggttattt ttctgtcatc aaacaaaaac 2400
aggtatcagt gcattattaa atgaatattt aaattagaca ttaccagtaa tttcatgtct 2460
actttttaaa atcagcaatg aaacaataat ttgaaatttc taaattcata gggtagaatc 2520
acctgtaaaa gcttgtttga tttcttaaag ttattaaact tgtacatata ccaaaaagaa 2580
gctgtcttgg atttaaatct gtaaaatcag atgaaatttt actacaattg cttgttaaaa 2640
tattttataa gtgatgttcc tttttcacca agagtataaa cctttttagt gtgactgtta 2700
aaacttcctt ttaaatcaaa atgccaaatt tattaaggtg gtggagccac tgcagtgtta 2760
tctcaaaata agaatatttt gttgagatat tccagaattt gtttatatgg ctggtaacat 2820
gtaaaatcta tatcagcaaa agggtctacc tttaaaataa gcaataacaa agaagaaaac 2880
```

caaattattg ttcaaattta ggtttaaact tttgaagcaa actttttttt atccttgtgc 2940
actgcaggcc tggtactcag attttgctat gaggttaatg aagtaccaag ctgtgcttga 3000
ataacgatat gttttctcag attttctgtt gtacagttta atttagcagt ccatatcaca 3060
ttgcaaaagt agcaatgacc tcataaaata cctcttcaaa atgcttaaat tcatttcaca 3120
cattaatttt atctcagtct tgaagccaat tcagtaggtg cattggaatc aagcctggct 3180
acctgcatgc tgttcctttt cttttcttct tttagccatt ttgctaagag acacagtctt 3240
ctcatcactt cgtttctcct attttgtttt actagtttta agatcagagt tcactttctt 3300
tggactctgc ctatattttc ttacctgaac ttttgcaagt tttcaggtaa acctcagctc 3360
aggactgcta tttagctcct cttaagaaga ttaaaagaga aaaaaaaagg ccctttttaaa 3420
aatagtatac acttatttta agtgaaaagc agagaatttt atttatagct aattttagct 3480
atctgtaacc aagatggatg caaagaggct agtgcctcag agagaactgt acggggtttg 3540
tgactggaaa aagttacgtt cccattctaa ttaatgccct ttcttattta aaaacaaaac 3600
caaatgatat ctaagtagtt ctcagcaata ataataatga cgataatact tcttttccac 3660
atctcattgt cactgacatt taatggtact gtatattact taatttattg aagattatta 3720
tttatgtctt attaggacac tatggttata aactgtgttt aagcctacaa tcattgattt 3780
tttttttgtta tgtcacaatc agtatatttt ctttggggtt acctctctga atattatgta 3840
aacaatccaa agaaatgatt gtattaagat ttgtgaataa atttttagaa atctgattgg 3900
catattgaga tatttaaggt tgaatgtttg tccttaggat aggcctatgt gctagcccac 3960
aaagaatatt gtctcattag cctgaatgtg ccataagact gaccttttaa aatgttttga 4020
gggatctgtg gatgcttcgt taatttgttc agccacaatt tattgagaaa atattctgtg 4080
tcaagcactg tgggttttaa tatttttaaa tcaaacgctg attacagata atagtattta 4140
tataaataat tgaaaaaaat tttcttttgg gaagagggag aaaatgaaat aaatatcatt 4200
aaagataact caggagaatc ttctttacaa ttttacgttt agaatgttta aggttaagaa 4260
agaaatagtc aatatgcttg tataaaacac tgttcactgt tttttttaaa aaaaaaactt 4320
gatttgttat taacattgat ctgctgacaa aacctgggaa tttgggttgt gtatgcgaat 4380
gtttcagtgc ctcagacaaa tgtgtattta acttatgtaa aagataagtc tggaaataaa 4440
tgtctgttta tttttgtact attta 4465

<210> 72
<211> 1331
<212> DNA
<213> Homo sapiens

<400> 72

cctgcatctt tttggaagga ttctttttat aaatcagaaa gtgttcgagg ttcaaaggtt 60
tgcctcggag cgtgtgaaca ttcctccgct cggttttcaa ctcgcctcca acctgcgccg 120
cccggccagc atgtctcccc gcccgtgaag cggggctgcc gcctccctgc cgctccggct 180
gccactaacg acccgccctc gccgccacct ggccctcctg atcgacgaca cacgcacttg 240
aaacttgttc tcagggtgtg tggaatcaac tttccggaag caaccagccc accagaggag 300
gtcccgagcg cgagcggaga cgatgcagcg gagactggtt cagcagtgga gcgtcgcggt 360
gttcctgctg agctacgcgg tgccctcctg cgggcgctcg gtggagggtc tcagccgccg 420
cctcaaaaga gctgtgtctg aacatcagct cctccatgac aaggggaagt ccatccaaga 480
tttacggcga cgattcttcc ttcaccatct gatcgcagaa atccacacag ctgaaatcag 540
agctacctcg gaggtgtccc ctaactccaa gccctctccc aacacaaaga accaccccgt 600
ccgatttggg tctgatgatg agggcagata cctaactcag gaaactaaca aggtggagac 660
gtacaaagag cagccgctca agacacctgg gaagaaaaag aaaggcaagc ccgggaaacg 720
caaggagcag gaaaagaaaa aacggcgaac tcgctctgcc tggttagact ctggagtgac 780
tgggagtggg ctagaagggg accacctgtc tgacacctcc acaacgtcgc tggagctcga 840
ttcacggagg cattgaaatt ttcagcagag accttccaag gacatattgc aggattctgt 900
aatagtgaac atatggaaag tattagaaat atttattgtc tgtaaatact gtaaatgcat 960
tggaataaaa ctgtctcccc cattgctcta tgaaactgca cattggtcat tgtgaatatt 1020
ttttttttgc caaggctaat ccaattatta ttatcacatt taccataatt tattttgtcc 1080
attgatgtat ttattttgta aatgtatctt ggtgctgctg aatttctata ttttttgtaa 1140
cataatgcac tttagatata catatcaagt atgttgataa atgacacaat gaagtgtctc 1200
tattttgtgg ttgattttaa tgaatgccta aatataatta tccaaattga ttttcctttg 1260
tgcatgtaaa aataacagta ttttaaattt gtaaagaatg tctaataaaa tataatctaa 1320
ttacatcatg a 1331

<210> 73

<211> 5376
<212> DNA
<213> Homo sapiens

<400> 73

agccggagct ggagccgagg cggcggcggg acgcggccgg ccggacaaat ttcctgctag 60

gctgcggaca gcgggcggca ggagccggcg cgagcggctt caggaaccca cggcctctgc 120
gcgtccccgc gacccttctt cgcgcccggc gaagacagcc gggcgccccg gagggcggcg 180
ggcaggcgcc cgggagatgc ggagcctccg ctgcagcgcg atctgcgcga ccagaccggc 240
ccccccgaga ctatagcctt cactttccct cggtccacca tggagccctt gtgtccactc 300
ctgctggtgg gttttagctt gccgctcgcc aggctctca ggggcaacga gaccactgcc 360
gacagcaacg agacaaccac gacctcaggc cctccggacc cgggcgcctc ccagccgctg 420
ctggcctggc tgctactgcc gctgctgctc ctcctcctcg tgctccttct cgccgcctac 480
ttcttcaggt tcaggaagca gaggaaagct gtggtcagca ccagcgacaa gaagatgccc 540
aacggaatct tggaggagca agagcagcaa agggtgatgc tgctcagcag gtcaccctca 600
gggcccaaga agtattttcc catccccgtg gagcacctgg aggaggagat ccgtatcaga 660
tccgccgacg actgcaagca gtttcgggag gagttcaact cattgccatc tggacacata 720
caaggaactt ttgaactggc aaataaagaa gaaaacagag aaaaaaacag atatcccaac 780
atccttccca atgaccattc tagggtgatt ctgagccaac tggatggaat tccctgttca 840
gactacatca atgcttccta catagatggt tacaaagaga agaataaatt catagcagct 900
caaggtccca aacaggaaac ggttaacgac ttctggagaa tggtctggga gcaaaagtct 960
gcgaccatcg tcatgttaac aaacttgaaa gaaaggaaag aggaaaagtg ccatcagtac 1020
tggcccgacc aaggctgctg gacctatgga aacatccggg tgtgcgtgga ggactgcgtg 1080
gttttggtcg actacaccat ccggaagttc tgcatacagc cacagctccc cgacggctgc 1140
aaagccccca ggctggtctc acagctgcac ttcaccagct ggcccgactt cggagtgcct 1200
tttaccccca ttgggatgct gaagttcctc aagaaagtaa agacgctcaa ccccgtgcac 1260
gctgggccca tcgtggtcca ctgtagcgcg ggcgtgggcc ggacgggcac cttcattgtg 1320
atcgatgcca tgatggccat gatgcacgcg gagcagaagg tggatgtgtt tgaatttgtg 1380
tctcgaatcc gtaatcagcg ccctcagatg gttcaaacgg atatgcagta cacgttcatc 1440
taccaagcct tactcgagta ctacctctac ggggacacag agctggacgt gtcctccctg 1500
gagaagcacc tgcagaccat gcacggcacc accaccccact tcgacaagat cgggctggag 1560
gaggagttca ggaaattgac aaatgtccgg atcatgaagg agaacatgag gacgggcaac 1620
ttgccggcaa acatgaagaa ggccagggtc atccagatca tcccgtatga cttcaaccga 1680
gtgatccttt ccatgaaaag gggtcaagaa tacacagact acatcaacgc atccttcata 1740
gacggctacc gacagaagga ctatttcatc gccacccagg ggccactggc acacacggtt 1800
gaggacttct ggaggatgat ctgggaatgg aaatcccaca ctatcgtgat gctgacggag 1860
gtgcaggaga gagagcagga taaatgctac cagtattggc caaccgaggg ctcagttact 1920
catggagaaa taacgattga gataaagaat gatacccttt cagaagccat cagtatacga 1980
gactttctgg tcactctcaa tcagccccag gcccgccagg aggagcaggt ccgagtagtg 2040
cgccagtttc acttccacgg ctggcctgag atcgggattc ccgccgaggg caaaggcatg 2100
attgacctca tcgcagccgt gcagaagcag cagcagcaga caggcaacca ccccatcacc 2160
gtgcactgca gtgccggagc tgggcgaaca ggtacattca tagccctcag caacattttg 2220
gagcgagtaa aagccgaggg acttttagat gtatttcaag ctgtgaagag tttacgactt 2280
cagagaccac atatggtgca aaccctggaa cagtatgaat tctgctacaa agtggtacaa 2340
gattttattg atatattttc tgattatgct aatttcaaat gaagattcct gccttaaaat 2400
attttttaat ttaatggtca gtatattttg taaaaatcat gttaatttat ttcatagttg 2460
acattaatat cttccctaat ttctttgtat atattttgtt atgccttaaa ggccacctgc 2520
tatacagttg ttaaatctta aatatgcttt ttaaaaattg gaataatgta ttaaggtcaa 2580
ataatatccc ataaaatata tatttctgct aatattagta aatatcttaa tttttcatta 2640
gattcatatc atttaatttc acatattcaa cacctttaaa tgttgtaatc ttaatatgcg 2700
aagtgtgcct ctgcaagata ctaacacaaa gctcatgtta agaaaacagt tgaggactca 2760
gaagtcagtt gaaaatgcac tttcctaaca gtgaattcac aaccctgaac agcagcattt 2820
ttggaaggca aactgttcgt gatggtacaa tgtaaatggg gacttctgta aagttctcag 2880
tttcggtcca tgtggtttat ctttacattt taaagatcaa agaagtcttt acaacctgaa 2940
tccaggtcta aacacacta gagtagctgg tgactataaa taatatttta aaatgctgtg 3000
tctacaccat caagactgtg tctacactat cttggctgaa cgagaagaga tgtaaatgct 3060
gggtggtccc gttgacccac ggcgttgggt acaacaaaac cagccatcgg agttacaccc 3120
caaagcacca tttgctgtcc agctgcctgt cgtttggccc agaccaccct cagaaaaaaa 3180
ccagctgcct ctcccattct cccctcccgt tctgccacag cggcctgggc tggtccagtg 3240
ctatgcctgg aggctcaaca caaaacttcc catccaaaca ttcagatgaa ctgagcgtct 3300
tacacacgca gtacagagga gcacacatta ggatagaaac agtagaataa ccacgggcaa 3360
ttaaacttta aattttctga gcagcatttt ggtatttaaa catttcttgt aaaaagctga 3420
gacagtttgt aagaaaagaa tccttaaaat ctagatttat accatttttt aaagtcccac 3480
ctttcaatgt ttaataaaac aaaaagagaa atccttaatc taaaagctaa attattttg 3540
aatggaaata ctactgagac cattgacact ggataacagt aatgatccca ttaccagata 3600
agattgactg acggggaaaa aaaaaaaaaa agaatggggt gtgaatgtac caacactgaa 3660
tcttacagca gttatctttc tatggccatt aggtacctag cagatgtgca caatataaac 3720
aaaaagatat ctggcctacc ttactactaa aagcatttaa cacgtgcatt tttggtactt 3780
ttttttttgt tttctaaaag ctacataaag gccttatttg acattttcac tgataactga 3840
tcgcaccctc atgttgcagt gttcgtcccc tattcattaa cagtgtgtgc aaatgcaacc 3900
ccaggacaca ttattgttct gatagatgct cacagggaat caagcttctc gcccactcca 3960

```
cggctctgag ccccatccaa gggcaagact tggtgcccag ctggaaggac gaaagcacac 4020
tttgtgaccg ccatcctcac cagctgcatg cctgggctgc acactgctga acgtgctcct 4080
ctctccttct ctgtacaatg attcagcatc tcggcggaag aggaaaatgg agctttttga 4140
ggctcgccag gtccctttttg ttttcaccat taaaattcca aacccaaagc ctttgtttga 4200
ctgaaggaga agagagggaa gtaagcttct gttcagcacc tgaaccctag aaaaagagcc 4260
agtttgctac gatgaaggtg acatttctct ggtcatttat ttgagagttc gaagtcaaag 4320
tcgaggggca ccggctttgg ttcatgtcta ggagccctct gtcagaatcc ttgaagccct 4380
ttaatggtct aactggcatc tcttgtatca agaagtacct ttaaggtaga ccttttcagg 4440
gtgccctcag gaaagggccc tgctcatgtt tttttcctgt ccccttagac caaccccagg 4500
tgtccactgc aggggttctg cctgttccca aactttttcc attccaggaa caaaggagaa 4560
gccactttcc ccaggacgca agactctccc ctccactgtc cgggacagcg ttcgcccttt 4620
agcggggagg tcattacagc ctcatggcct ctaccaaggc cccagatcac aggatctcct 4680
gggccttgga gcacctcacg ctgggggaat caatccctga gggactcaga atcttctccg 4740
tgcaacctgg aaagttcatc tcttgtttcc ttcagtcaaa gaaagtccat tgtacataac 4800
aaaacagccc ccaaacagcc cagtgccgac accattgttc ctttcacact ttcctttgtt 4860
gcatgcagtt gggttcaaat gccaaatagt gattagaaga cgaccattct gatctgtgtg 4920
tgatctggtc actatgtgac tgcctttacg gtttctctcc atgtgctata tgaatgaaga 4980
atgcatacca gtgttttaaa aggtattttt atgtgttttt aaacactttt ttaaatgagc 5040
ctgacacctg tgtttcagca tttggagaca tccccatgtt attcttttaa gtgtataatt 5100
actgatactt ttttgtttgt ttgtttaact aagttgtgtt taacttatgt gcagtcttta 5160
taatgtatgt atgttattac agtttcaact atcatatttt ctttgattac atttataatt 5220
tgatcttgct ctgattataa tgccagtgaa tgttgctgaa ctctttgtat atgcaaattg 5280
caagatttaa accattctga tgcaaggata aacctttact ttgactacca gcctgtgttt 5340
ttgtctttaa atctcttaat ttcattcctc tgcaaa                            5376
```

<210> 74
<211> 1908
<212> DNA
<213> Homo sapiens

<400> 74

```
catttattaa ggactctctg ctccagcctc tcactctcac tctcctccgc tcaaactcag 60
ctcacttgag agtctcctcc cgccagctgt ggaaagaact ttgcgtctct ccagcaatgc 120
atctccttgc gattctgttt tgtgctctct ggtctgcagt gttggccgag aactcggatg 180
attatgatct catgtatgtg aatttggaca acgaaataga caatggactc catcccactg 240
aggaccccac gccgtgcgac tgcggtcagg agcactcgga atgggacaag ctcttcatca 300
tgctggagaa ctcgcagatg agagagcgca tgctgctgca agccacggac gacgtcctgc 360
ggggcgagct gcagaggctg cgggaggagc tgggccggct cgcggaaagc ctggcgaggc 420
cgtgcgcgcc gggggctccc gcagaggcca ggctgaccag tgctctggac gagctgctgc 480
aggcgacccg cgacgcgggc cgcaggctgg cgcgtatgga gggcgcggag gcgcagcgcc 540
cagaggaggc ggggcgcgcc ctggccgcgg tgctagagga gctgcggcag acgcgagccg 600
acctgcacgc ggtgcagggc tgggctgccc ggagctggct gccggcaggt tgtgaaacag 660
ctattttatt cccaatgcgt tccaagaaga tttttggaag cgtgcatcca gtgagaccaa 720
tgaggcttga gtcttttagt gcctgcattt gggtcaaagc cacagatgta ttaaacaaaa 780
ccatcctgtt ttcctatggc acaaagagga atccatatga aatccagctg tatctcagct 840
accaatccat agtgtttgtg gtgggtggag aggagaacaa actggttgct gaagccatgg 900
tttccctggg aaggtggacc cacctgtgcg gcacctggaa ttcagaggaa gggctcacat 960
ccttgtgggt aaatggtgaa ctggcggcta ccactgttga gatggccaca ggtcacattg 1020
ttcctgaggg aggaatcctg cagattggcc aagaaaagaa tggctgctgt gtgggtggtg 1080
gctttgatga aacattagcc ttctctggga gactcacagg cttcaatatc tgggatagtg 1140
ttcttagcaa tgaagagata agagagaccg gaggagcaga gtcttgtcac atccggggga 1200
atattgttgg gtggggagtc acagagatcc agccacatgg aggagctcag tatgtttcat 1260
aaatgttgtg aaactccact tgaagccaaa gaaagaaact cacacttaaa acacatgcca 1320
gttgggaagg tctgaaaact cagtgcataa taggaacact tgagactaat gaaagagaga 1380
gttgagacca atctttattt gtactggcca aatactgaat aaacagttga aggaaagaca 1440
ttggaaaaag cttttgagga taatgttact agactttatg ccatggtgct ttcagtttaa 1500
tgctgtgtct ctgtcagata aactctcaaa taattaaaaa ggactgtatt gttgaacaga 1560
gggacaattg ttttactttt ctttggttaa ttttgttttg gccagagatg aattttacat 1620
tggaagaata acaaaataag atttgttgtc cattgttcat tgttattggt atgtacctta 1680
ttacaaaaaa aatgatgaaa acatatttat actacaaggt gacttaacaa ctataaatgt 1740
agtttatgtg ttataatcga atgtcacgtt tttgagaaga tagtcatata agttatattg 1800
caaaagggat ttgtattaat ttaagactat ttttgtaaag ctctactgta aataaaatat 1860
tttataaaac taaaaaaaaa aaaaaaaaaa aaaaaaaaaa aaaaaaaa             1908
```

<210> 75
<211> 3215
<212> DNA
<213> Homo sapiens

<400> 75

```
cttgaagaag tgggtattcc ccttcccacc ccaggcactg gaggagcggc cccccgggga 60
ttccaggacc tgagctccgg gagctggact cgcagcgacc gcggcagagc gagctggcgc 120
cgggaagcga ggagacgccc gcgggaggcc cagctgctcg gagcaactgg catggcccga 180
gccatggccg ccgcgtggcc gctgctgctg gtggcgctac tggtgctgtc ctggccaccc 240
ccaggaaccg gggacgtcgt cgtgcaggcg cccacccagg tgcccggctt cttgggcgac 300
tccgtgacgc tgccctgcta cctacaggtg cccaacatgg aggtgacgca tgtgtcacag 360
ctgacttggg cgcggcatgg tgaatctggc agcatggccg tcttccacca aacgcagggc 420
cccagctatt cggagtccaa acggctggaa ttcgtggcag ccagactggg cgcggagctg 480
cggaatgcct cgctgaggat gttcgggttg cgcgtagagg atgaaggcaa ctacacctgc 540
ctgttcgtca cgttcccgca gggcagcagg agcgtggata tctggctccg agtgcttgcc 600
aagccccaga acacagctga ggttcagaag gtccagctca ctggagagcc agtgcccatg 660
gcccgctgcg tctccacagg gggtcgcccg ccagcccaaa tcacctggca ctcagacctg 720
ggcgggatgc ccaatacgag ccaggtgcca gggttcctgt ctggcacagt cactgtcacc 780
agcctctgga tattggtgcc ctcaagccag gtggacggca agaatgtgac ctgcaaggtg 840
gagcacgaga gctttgagaa gcctcagctg ctgactgtga acctcaccgt gtactacccc 900
ccagaggtat ccatctctgg ctatgataac aactggtacc ttggccagaa tgaggccacc 960
ctgacctgcg atgctcgcag caacccagag cccacaggct ataattggag cacgaccatg 1020
ggtcccctgc cacccttttgc tgtggcccag ggcgcccagc tcctgatccg tcctgtggac 1080
aaaccaatca acacaacttt aatctgcaac gtcaccaatg ccctaggagc tcgccaggca 1140
gaactgaccg tccaggtcaa agagggacct cccagtgagc actcaggcat gtcccgtaac 1200
gccatcatct tcctggttct gggaatcctg gtttttctga tcctgctggg gatcgggatt 1260
tatttctatt ggtccaaatg ttcccgtgag gtcctttggc actgtcatct gtgtccctcg 1320
agtacagagc atgccagcgc ctcagctaat gggcatgtct cctattcagc tgtgagcaga 1380
gagaacagct cttcccagga tccacagaca gagggcacaa ggtgacagcg tcgggactga 1440
gaggggagag agactggagc tggcaaggac gtgggcctcc agagttggac ccgaccccaa 1500
tggatgaaga ccccctccaa agagaccagc ctccctccct gtgccagacc tcaaaacgac 1560
gggggcaggt gcaagttcat aggtctccaa gaccaccctc ctttcatttg ctagaaggac 1620
tcactagact caggaaagct gttaggctca cagttacagt ttattacagt aaaaggacag 1680
agattaagat cagcaaaggg aggaggtgca cagcacacgt tccacgacag atgaggcgac 1740
ggcttccatc tgccctctcc cagtggagcc atataggcag cacctgattc tcacagcaac 1800
atgtgacaac atgcaagaag tactgccaat actgccaacc agagcagctc actcgagatc 1860
tttgtgtcca gagtttttttg tttgtcttga gacagggtct ggctctgttg gcagactaga 1920
gtacagtggt gagatcacag ttcattgcag ccttgacttc tcaacgccaa gtcatcctcc 1980
cacctcagcc tcctgagtag ctatgactac aggtatgtgc caccacgtct ggctaatctt 2040
tttattattt gtaaagtcga ggtttccctg tgttgcccag gctggtcttg aactcttggc 2100
tccaagtgat acttctgcct tggcctccca aagtgctgaa ttaagcagct caccatccac 2160
acggctgacc tcatacatca agccaatacc gtgtggccca agacccccac cataaatcac 2220
atcattagca tgaaccaccc agagtggccc aagactccca gatcagctac caggcaggat 2280
attccaaggg cttagagatg aatgcccagg agctgaggat aaagggcccg atctttcttt 2340
gggcaaggtt aagcctttac tgcatagcag accacacaga agggtgtggg ccaccagaga 2400
attttggtaa aaatttggcc tctggccttg agcttctaaa tctctgtatc cgtcagatct 2460
ctgtggttac aagaaacagc cactgaccct ggtcaccaga ggctgcaatt caggccgcaa 2520
gcagctgcct aggggggtgtc caaggagcag agaaaactac tagatgtgaa cttgaagaag 2580
gttgtcagct gcagccactt tctgccagca tctgcagcca ctttctgcca gcatctgcag 2640
ccagcaagct gggactggca ggaaataacc cacaaaagaa gcaaatgcaa tttccaacac 2700
aagggggaag ggatgcaggg ggaggcagcg ctgcagttgc tcaggacacg ctcctatagg 2760
accaagatgg atgcgaccca agacccagga ggcccagctg ctcagtgcaa ctgacaagtt 2820
aaaaaggtct atgatcttga gggcagacag cagaattcct cttataaaga aaactgtttg 2880
ggaaaatacg ttgagggaga gaagaccttg ggccaagatg ctaaatggga atgcaaagct 2940
tgagctgctc tgcaagagaa aataagcagg acagaggatt tgctctggac agagatggaa 3000
gagccgggaa cagagaagtg tggggaagag ataggaacca gcaggatggc aggggcaaag 3060
ggctcaaggg tgaggaggcc agtgggaccc cacagagttg gggagataaa ggaacattgg 3120
ttgctttggt ggcacgtaag ctccttgtct gtctccagca cccagaatct cattaaagct 3180
tatttattgt acctccaaaa aaaaaaaaaa aaaaa                            3215
```

<210> 76

<211> 3357

<212> DNA

<213> Homo sapiens

<400> 76

```
gggttcgcga ccgcagccga gagaccccgg gcgaccggca cctccgagac tcgcgggcca 60
cctgcctcga ccttccccgg agcgcccccg cctccgagtc gctacttgcc gggccgggcc 120
gggccgggcg tgatgcgccg cgggacccct gtcctggcca ctggccgccg ccgccgccgc 180
cgctgagacc ccgagacccc cagtgacgcc gcagccatgg agagcggccc gcgtgcggag 240
ctgggggcgg gcgcaccccc agctgtggtc gccaggaccc ccccagagcc aagaccttct 300
ccagaaggtg acccttcccc cccaccacca ccaatgtcag ccctggtccc cgacactccc 360
ccggacaccc ctcctgccat gaagaatgcc actagctcta agcagctccc actggaacca 420
gagagcccct cagggcaggt cgggcctagg ccagcccccc cgcaggaaga gtccccttcc 480
tctgaagcaa agagcagagg acccacccca ccagccatgg gcccacggga tgccagacct 540
cctcgaagga gcagccagcc atctccaaca gcagtgccag cctccgacag ccctcccacc 600
aagcaagagg tgaagaaggc aggagagaga cacaagctgg caaaggagcg gcgagaagag 660
cgggccaagt acctggcggc caagaaggca gtgtggctgg agaaggagga gaaggccaag 720
gcgctgcggg agaagcagct ccaggagcgc cggcgccggc tggaggagca acgtcttaaa 780
gccgagcaac gccgtgcagc cctggaggaa cggcagcggc agaagctcga gaaaaacaag 840
gagcgctatg aagcagccat ccaacggtca gtgaagaaga cgtgggccga aatccggcag 900
cagcgctggt cctgggcagg ggccctgcac cacagctctc caggacataa gaccagtggg 960
agcaggtgct ccgtgtcggc agttaacctg cccaaacacg tggactctat aatcaacaag 1020
cggctctcaa agtcctctgc cacgctctgg aactccccca gtagaaatcg cagcctgcag 1080
ctgagcgcat gggagagcag catcgtggat cgtctgatga cgcccactct ctccttcctt 1140
gctcggagtc gcagcgcggt cacactgccc cgcaacggcc gggaccaggg taggggctgc 1200
gaccctggga gaggccccac gtggggccgg gcaggggcca gcctggcgcg cgggccgcaa 1260
cccgaccgca ctcatccctc tgcagccgtg ccggtgtgcc cgcgctcggc ctccgccagc 1320
cccctgacgc cgtgcagcgt cacccgaagc gtgcaccgct gcgcccccgc cggtgagcgc 1380
ggggagcgcc gcaagcccaa cgccgggggc agccccgctc cggtgcgccg ccggccggag 1440
gcctcgccgg tgcagaaaaa ggagaagaag gacaaggagc gggaaaacga gaaggagaag 1500
agtgccctag cccgggagcg cagcctcaag aagcgccagt cgctgcccgc ctccccacgt 1560
gcccgcctct ccgccagcac cgcctctgag ctcagcccca aatccaaggc caggccatcc 1620
tctccctcca catcctggca caggcctgcc tccccctgcc ccagcccagg gccaggccac 1680
actctgcctc caaagccacc gtcccccga ggcaccactg catcccccaa ggggcgggtt 1740
cggaggaagg aggaggcaaa ggagagcccc agcgccgcag ggcccgagga caagagccag 1800
agcaagcgca gggccagtaa cgagaaggag tcagcagccc cagcctcacc ggcaccttcg 1860
ccggcgccct cgcccacccc agccccgccc cagaaggagc agccccccgc ggagacccct 1920
acagacgctg ctgtcttgac ctcaccccca gcccctgctc cccggtgac ccctagcaaa 1980
ccaatggccg gcaccacaga ccgagaagaa gccactcggc tcttggctga gaagcggcgc 2040
caggcccggg agcagcggga gcgcgaggag caggagcgga ggctgcaggc agaaagggac 2100
aagcgaatgc gagaggagca gctggcacgg gaggccgagg cccgggcgga gcgggaggcg 2160
gaggcccgga ggcgggagga gcaggaggca cgagagaagg cgcaggccga gcaggaggag 2220
caggagcggc tgcagaagca gaaagaggag gccgaagctc ggtcgcggga agaggcggag 2280
cggcagcgtc tggagcggga aaagcacttc cagcagcagg agcaagagcg gcaagagcgc 2340
agaaagcgtc tggaggagat catgaagagg actcggaagt cagaagtttc tgaaaccaag 2400
cagaagcagg acagcaagga ggccaacgcc aacggttcca gcccagagcc tgtgaaagct 2460
gtggaggctc ggtccccagg gctgcagaag gaggctgtgc agaaagagga gcccatccca 2520
caggagcctc agtggagtct cccaagcaag gagttgccag cgtccctggt gaatggcctg 2580
cagcctctcc cagcacacca ggagaatggc ttctccacca acggaccctc tggggacaag 2640
agtctgagcc gaacaccaga gacactcctg ccctttgcag aggcagaagc cttcctcaag 2700
aaagctgtgg tgcagtcccc gcaggtcaca gaagtccttt aagagggttt gccttggatc 2760
cgggcacagt tgtgagggct cctctgcatc acctaccagg atgtctggag gagaaaaaga 2820
cagaacaaag atggaagtgg cctgggcccc tgggggtggg tcctctctgt tgtttttaat 2880
ctgcacctta tagactgatg tctctttggc cggagccaga tctgcccctc agtgcattcg 2940
tgtgctcgca cgcgcagaca tcccttctcc cccatacaca catatacact cacagcctct 3000
ctggcctctt cccttgggga ggggccacct gtagtatttg ccttgatttg gtggggtaca 3060
gtggatgtga atactgtaaa tagcttgtgc tcagactcct ctgcgtggag agggtgggtg 3120
caggaggcag accctccccc caaagccccc tggggagatc ttcctctctc tatttaactg 3180
taactgaggg ggatcccagg tctggggatg ggggacacct tgggccacag gatactggtt 3240
gcttcagggg tacccatgcc ccctgccctc gcctggaatc agtgttactg catctgatta 3300
aatgtctcca gaaataaaga ataaaaaaaa aaaaaaaaaa aaaaaaaaaa aaaaaaa    3357
```

<210> 77
<211> 683
<212> DNA
<213> Homo sapiens

<400> 77

```
ctgcgcagat gaggggagac tcgtcaccag gcgtgcagtg ggcactgctg ggctccccca 60

tcccgtccta acccggaaca gccccgggca ggaggcgtgg aaagtcgagg gggtaaaccg 120
cgaatgtgcg ttgtgtaagc cacggcgcag ggtggggcgc gggcgggact tgggcgggcg 180
gggtgggctt ggccgagctg gcctccgggg caccgaccgc tataaggcca gtcggactgc 240
gacacagccc atcccctcga ccgctcgcgt cgcatttggc cgcctcccta ccgctccaag 300
cccagccctc agccatggca tgccccctgg atcaggccat tggcctcctc gtggccatct 360
tccacaagta ctccggcagg gagggtgaca agcacaccct gagcaagaag gagctgaagg 420
agctgatcca gaaggagctc accattggct cgaagctgca ggatgctgaa attgcaaggc 480
tgatggaaga cttggaccgg aacaaggacc aggaggtgaa cttccaggag tatgtcacct 540
tcctgggggc cttggctttg atctacaatg aagccctcaa gggctgaaaa taaataggga 600
agatggagac accctctggg ggtcctctct gagtcaaatc cagtggtggg taattgtaca 660
ataaattttt tttggtcaaa ttt                                          683
```

<210> 78
<211> 3217
<212> DNA
<213> Homo sapiens

<400> 78

ggccgggaga cctggcggag ctgggggtgg ggggccagtt tttgcaacgg ctaaggaagg 60
gcctgtgggt ttattataag gcggagctcg gcgggagagg tgcgggccga atccgagccg 120
agcggagagg aatccggcag tagagagcgg actccagccg gcggaccctg cagccctcgc 180
ctgggacagc ggcgcgctgg gcaggcgccc aagagagcat cgagcagcgg aacccgcgaa 240
gccggcccgc agccgcgacc cgcgcagcct gccgctctcc cgccgccggt ccgggcagca 300
tgaggcgcgc ggcgctctgg ctctggctgt gcgcgctggc gctgagcctg cagccggccc 360
tgccgcaaat tgtggctact aatttgcccc ctgaagatca agatggctct ggggatgact 420
ctgacaactt ctccggctca ggtgcaggtg ctttgcaaga tatcaccttg tcacagcaga 480
ccccctccac ttggaaggac acgcagctcc tgacggctat tcccacgtct ccagaaccca 540
ccggcctgga ggctacagct gcctccacct ccaccctgcc ggctggagag ggcccaaagg 600
agggagaggc tgtagtcctg ccagaagtgg agcctggcct caccgcccgg gagcaggagg 660
ccacccccg acccagggag accacacagc tcccgaccac tcatcaggcc tcaacgacca 720
cagccaccac ggcccaggag cccgccacct cccaccccca cagggacatg cagcctggcc 780
accatgagac ctcaacccct gcaggaccca gccaagctga ccttcacact ccccacacag 840
aggatggagg tccttctgcc accgagaggg ctgctgagga tggagcctcc agtcagctcc 900
cagcagcaga gggctctggg gagcaggact tcacctttga aacctcgggg gagaatacgg 960
ctgtagtggc cgtggagcct gaccgccgga accagtcccc agtggatcag ggggccacgg 1020
gggcctcaca gggcctcctg gacaggaaag aggtgctggg aggggtcatt gccggaggcc 1080
tcgtggggct catctttgct gtgtgcctgg tgggtttcat gctgtaccgc atgaagaaga 1140
aggacgaagg cagctactcc ttggaggagc cgaaacaagc caacggcggg gcctaccaga 1200
agcccaccaa acaggaggaa ttctatgcct gacgcgggag ccatgcgccc cctccgccct 1260
gccactcact aggcccccac ttgcctcttc cttgaagaac tgcaggccct ggcctcccct 1320
gccaccaggc cacctcccca gcattccagc ccctctggtc gctcctgccc acggagtcgt 1380
ggggtgtgct gggagctcca ctctgcttct ctgacttctg cctggagact tagggcacca 1440
ggggtttctc gcataggacc tttccaccac agccagcacc tggcatcgca ccattctgac 1500
tcggtttctc caaactgaag cagcctctcc ccaggtccag ctctggaggg gagggggatc 1560
cgactgcttt ggacctaaat ggcctcatgt ggctggaaga tcctgcgggt ggggcttggg 1620
gctcacacac ctgtagcact tactggtagg accaagcatc ttgggggggt ggccgctgag 1680
tggcagggga caggagtcca ctttgtttcg tggggaggtc taatctagat atcgacttgt 1740
ttttgcacat gtttcctcta gttctttgtt catagcccag tagaccttgt tacttctgag 1800
gtaagttaag taagttgatt cggtatcccc ccatcttgct tccctaatct atggtcggga 1860
gacagcatca gggttaagaa gacttttttt tttttttttt aaactaggag aaccaaatct 1920
ggaagccaaa atgtaggctt agtttgtgtg ttgtctcttg agtttgtcgc tcatgtgtgc 1980
aacagggtat ggactatctg tctggtggcc ccgtttctgg tggtctgttg gcaggctggc 2040
cagtccaggc tgccgtgggg ccgccgcctc tttcaagcag tcgtgcctgt gtccatgcgc 2100
tcagggccat gctgaggcct gggccgctgc cacgttggag aagcccgtgt gagaagtgaa 2160
tgctgggact cagccttcag acagagagga ctgtagggag ggcggcaggg gcctggagat 2220
cctcctgcag accacgcccg tcctgcctgt ggcgccgtct ccaggggctg cttcctcctg 2280
gaaattgacg aggggtgtct tgggcagagc tggctctgag cgcctccatc caaggccagg 2340
ttctccgtta gctcctgtgg ccccaccctg ggccctgggc tggaatcagg aatattttcc 2400
aaagagtgat agtcttttgc ttttggcaaa actctactta atccaatggg tttttccctg 2460
tacagtagat tttccaaatg taataaactt taatataaag tagtcctgtg aatgccactg 2520
ccttcgcttc ttgcctctgt gctgtgtgtg acgtgaccgg acttttctgc aaacaccaac 2580
atgttgggaa acttggctcg aatctctgtg ccttcgtctt tcccatgggg agggattctg 2640
gttccagggt ccctctgtgt atttgctttt ttgttttggc tgaaattctc ctggaggtcg 2700
gtaggttcag ccaaggtttt ataaggctga tgtcaatttc tgtgttgcca agctccaagc 2760
cccatcttct aaatggcaaa ggaaggtgga tggccccagc acagcttgac ctgaggctgt 2820

ggtcacagcg gaggtgtgga gccgaggcct accccgcaga caccttggac atcctcctcc 2880
cacccggctg cagaggccag aggcccccag cccagggctc ctgcacttac ttgcttattt 2940
gacaacgttt cagcgactcc gttggccact ccgagaggtg ggccagtctg tggatcagag 3000
atgcaccacc aagccaaggg aacctgtgtc cggtattcga tactgcgact ttctgcctgg 3060
agtgtatgac tgcacatgac tcggggggtgg ggaaaggggt cggctgacca tgctcatctg 3120
ctggtccgtg ggacggtgcc caagccagag gctgggttca tttgtgtaac gacaataaac 3180
ggtacttgtc atttcgggca aaaaaaaaaa aaaaaaa 3217

<210> 79
<211> 2640
<212> DNA
<213> Homo sapiens

<400> 79

```
actcgccgca gcctgcgcgc cttctccagt ccgcggtgcc atggcccccg cccgtctgtt 60
cgcgctgctg ctgttcttcg taggcggagt cgccgagtcg atccgagaga ctgaggtcat 120
cgacccccag gacctcctag aaggccgata cttctccgga gccctaccag acgatgagga 180
tgtagtgggg cccgggcagg aatctgatga ctttgagctg tctggctctg gagatctgga 240
tgacttggaa gactccatga tcggccctga agttgtccat cccttggtgc ctctagataa 300
ccatatccct gagagggcag ggtctgggag ccaagtcccc accgaaccca agaaactaga 360
ggagaatgag gttatcccca agagaatctc acccgttgaa gagagtgagg atgtgtccaa 420
caaggtgtca atgtccagca ctgtgcaggg cagcaacatc tttgagagaa cggaggtcct 480
ggcagctctg attgtgggtg gcatcgtggg catcctcttt gccgtcttcc tgatcctact 540
gctcatgtac cgtatgaaga agaaggatga aggcagctat gacctgggca agaaacccat 600
ctacaagaaa gcccccacca atgagttcta cgcgtgaagc ttgcttgtgg gcactggctt 660
ggactttagc ggggagggaa gccaggggat tttgaagggt ggacattagg gtagggtgag 720
gtcaacctaa tactgacttg tcagtatctc cagctctgat tacctttgaa gtgttcagaa 780
gagacattgt cttctactgt tctgccaggt tcttcttgag ctttgggcct cagttgccct 840
ggcagaaaaa tggattcaac ttggcctttc tgaaggcaag actgggattg gatcacttct 900
taaacttcca gttaagaatc taggtccgcc ctcaagccca tactgaccat gcctcatcca 960
gagctcctct gaagccaggg ggctaacgga tgttgtgtgg agtcctggct ggaggtcctc 1020
ccccagtggc cttcctccct tcctttcaca gccggtctct ctgccaggaa atgggggaag 1080
gaactagaac cacctgcacc ttgagatgtt tctgtaaatg ggtacttgtg atcacactac 1140
gggaatctct gtggtatata cctggggcca ttctaggctc tttcaagtga cttttggaaa 1200
tcaacctttt ttatttgggg gggaggatgg ggaaaagagc tgagagttta tgctgaaatg 1260
gatttataga atatttgtaa atctattttt agtgtttgtt cgttttttta actgttcatt 1320
cctttgtgca gagtgtatat ctctgcctgg gcaagagtgt ggaggtgccg aggtgtcttc 1380
attctctcgc acatttccac agcacctgct aagtttgtat ttaatggttt ttgtttttgt 1440
ttttgtttgt ttcttgaaaa tgagagaaga gccggagaga tgatttttat taattttttt 1500
tttttttttt tttttttact atttatagct ttagataggg cctcccttcc cctcttcttt 1560
ctttgttctc tttcattaaa ccccttcccc agtttttttt ttatacttta aaccccgctc 1620
ctcatggcct tggccctttc tgaagctgct tcctcttata aaatagcttt tgccgaaaca 1680
tagttttttt ttagcagatc ccaaaatata atgaagggga tggtgggata tttgtgtctg 1740
tgttcttata atatattatt attcttcctt ggttctagaa aaatagataa atatattttt 1800
ttcaggaaat agtgtggtgt ttccagtttg atgttgctgg gtggttgagt gagtgaattt 1860
tcatgtggct gggtgggttt ttgccttttt ctcttgccct gttcctggtg ccttctgatg 1920
gggctggaat agttgaggtg gatggttcta ccctttctgc cttctgtttg ggacccagct 1980
ggtgttcttt ggtttgcttt cttcaggctc tagggctgtg ctatccaata cagtaaccac 2040
atgcggctgt ttaaagttaa gccaattaaa atcacataag attaaaaatt ccttcctcag 2100
ttgcactaac cacgtttcta gaggcgtcac tgtatgtagt tcatggctac tgtactgaca 2160
gcgagagcat gtccatctgt tggacagcac tattctagag aactaaactg gcttaacgag 2220
tcacagcctc agctgtgctg ggacgaccct tgtctccctg ggtagggggg ggggaatggg 2280
ggagggctga tgaggcccca gctggggcct gttgtctggg accctccctc tcctgagagg 2340
ggaggcctgg tggcttagcc tgggcaggtc gtgtctcctc ctgaccccag tggctgcggt 2400
gaggggaacc accctccctt gctgcaccag tggccattag ctcccgtcac cactgcaacc 2460
cagggtccca gctggctggg tcctcttctg cccccagtgc ccttcccctt gggctgtgtt 2520
ggagtgagca cctcctctgt aggcacctct cacactgttg tctgttactg attttttttg 2580
ataaaaagat aataaaacct ggtactttct aaaaaaaaaa aaaaaaaaaa aaaaaaaaaa 2640
```

<210> 80
<211> 655
<212> DNA
<213> Homo sapiens

<400> 80

```
tttttttga gacggattct ggctctgtct cccaggctgg agtgcagtgg cgcgatctca 60
gctcactgca acccgcgcct cccgggttca agcaattctc ccgcctcaga aaaagattag 120
aaggacagct gggatctgta tgtatagatc tctcttttc ttttttttaa ttaaaatttt 180
ttttgatgtg ttctcatctt tttttttaaa cagtgcacat gcatacatct gtgtaataaa 240
aaataaagga cagtaattct ggatacactc aggagacaga taagtcaacc aggcacgaag 300
gatgaaaaca ctacaaaaat cacagcatct accagccagt gatcatgaca gttaacattc 360
tggggtgtcc ctcctggcat acgcaccaaa actcacttgg tttttgtaaa aactgacacc 420
aggtgtatcc atatgtgaag caagaacact acaaacagga cagttgccaa tgttatataa 480
aatatatgaa tgtttggcca gacgcggtgg ctcatggctg taatcccggc actctgggag 540
cccaagatgg gtggatcatc tgaggtcaga agttcgagac cagcctggcc aacatggtga 600
aaccctgcct ccactataaa tacaaaaatt agctgggcat ggtggcgggc acctg 655
```

<210> 81
<211> 1310
<212> DNA
<213> Homo sapiens

<400> 81

```
gctcggagcc cggagcgtgc ctcggcggcc tgtcggtttt caccatggag cagctgagct 60
cagcaaacac ccgcttcgcc ttggacctgt tcctggcgtt gagtgagaac aatccggctg 120
gaaacatctt catctctccc ttcagcattt catctgctat ggccatggtt tttctgggga 180
ccagaggtaa cacggcagca cagctgtcca agactttcca tttcaacacg gttgaagagg 240
ttcattcaag attccagagt ctgaatgctg atatcaacaa acgtggagcg tcttatattc 300
tgaaacttgc taatagatta tatggagaga aaacttacaa tttccttcct gagttcttgg 360
tttcgactca gaaaacatat ggtgctgacc tggccagtgt ggattttcag catgcctctg 420
aagatgcaag gaagaccata aaccagtggg tcaaggaca gacagaagga aaaattccgg 480
aactgttggc ttcgggcatg gttgataaca tgaccaaact tgtgctagta aatgccatct 540
atttcaaggg aaactggaag gataaattca tgaaagaagc cacgacgaat gcaccattca 600
gattgaataa gaaagacaga aaaactgtga aaatgatgta tcagaagaaa aaatttgcat 660
atggctacat cgaggacctt aagtgccgtg tgctggaact gccttaccaa ggcgaggagc 720
tcagcatggt catcctgctg ccggatgaca ttgaggacga gtccacgggc ctgaagaaga 780
ttgaggaaca gttgactttg gaaaagttgc atgagtggac taaacctgag aatctcgatt 840
tcattgaagt taatgtcagc ttgcccaggt tcaaactgga agagagttac actctcaact 900
ccgacctcgc ccgcctaggt gtgcaggatc tctttaacag tagcaaggct gatctgtctg 960
gcatgtcagg agccagagat atttttatat caaaaattgt ccacaagtca tttgtggaag 1020
tgaatgaaga gggaacagag gcggcagctg ccacagcagg catcgcaact ttctgcatgt 1080
tgatgcccga agaaaatttc actgccgacc atccattcct tttctttatt cggcataatt 1140
cctcaggtag catcctattc ttggggagat ttcttcccc ttagaagaaa gagactgtag 1200
caatacaaaa atcaagctta gtgctttatt acctgagttt ttaatagagc caatatgtct 1260
tatatcttta ccaataaaac cactgtccag aaaaaaaaaa aaaaaaaaaa 1310
```

<210> 82
<211> 1900
<212> DNA
<213> Homo sapiens

<400> 82

```
acaactctca gaggagcatt gcccgtcaga cagcaactca gagaataacc agagaacaac 60
cagattgaaa caatggagga tctttgtgtg gcaaacacac tctttgccct caatttattc 120
aagcatctgg caaaagcaag ccccacccag aacctcttcc tctccccatg gagcatctcg 180
tccaccatgg ccatggtcta catgggctcc aggggcagca ccgaagacca gatggccaag 240
gtgcttcagt ttaatgaagt gggagccaat gcagttaccc ccatgactcc agagaacttt 300
accagctgtg ggttcatgca gcagatccag aagggtagtt atcctgatgc gattttgcag 360
gcacaagctg cagataaaat ccattcatcc ttccgctctc tcagctctgc aatcaatgca 420
tccacaggga attatttact ggaaagtgtc aataagctgt ttggtgagaa gtctgcgagc 480
ttccgggaag aatatattcg actctgtcag aaatattact cctcagaacc ccaggcagta 540
gacttcctag aatgtgcaga agaagctaga aaaaagatta attcctgggt caagactcaa 600
accaaaggca aaatcccaaa cttgttacct gaaggttctg tagatgggga taccaggatg 660
gtcctggtga atgctgtcta cttcaaagga aagtggaaaa ctccatttga gaagaaacta 720
aatgggcttt atcctttccg tgtaaactcg gctcagcgca cacctgtaca gatgatgtac 780
ttgcgtgaaa agctaaacat tggatacata gaagacctaa aggctcagat tctagaactc 840
ccatatgctg gagatgttag catgttcttg ttgcttccag atgaaattgc cgatgtgtcc 900
actggcttgg agctgctgga aagtgaaata acctatgaca aactcaacaa gtggaccagc 960
aaagacaaaa tggctgaaga tgaagttgag gtatacatac cccagttcaa attagaagag 1020

cattatgaac tcagatccat tctgagaagc atgggcatgg aggacgcctt caacaaggga 1080
cgggccaatt tctcagggat gtcggagagg aatgacctgt ttctttctga agtgttccac 1140
caagccatgg tggatgtgaa tgaggagggc actgaagcag ccgctggcac aggaggtgtt 1200
atgacaggga gaactggaca tggaggccca cagtttgtgg cagatcatcc ttttcttttt 1260
cttattatgc ataagataac caactgcatt ttatttttcg gcagattttc ctcaccctaa 1320
aactaagcgt gctgcttctg caaaagattt ttgtagatga gctgtgtgcc tcagaattgc 1380
tatttcaaat tgccaaaaat ttagagatgt ttctacatat tttctgctct tctgaacaac 1440
ttctgctacc cactaaataa aaacacagaa ataattagac aattgtctat tataacatga 1500
caaccctatt aatcatttgg tcttctaaaa tgggatcatg cccatttaga ttttccttac 1560
tatcagttta tttttataac attaactttt actttgttat ttattatttt atataatggt 1620
gagtttttaa attattgctc actgcctatt taatgtagct aataaagtta tagaagcaga 1680
tgatctgtta atttcctatc taataaatgc ctttaattgt tctcataatg aagaataagt 1740
aggtaccctc catgcccttc tgtaataaat atctggaaaa aacattaaac aataggcaaa 1800
tatatgttat gtgcatttct agaaatacat aacacatata tatgtctgta tcttatattc 1860
aattgcaagt atataataaa taaacctgct tccaaacaac                       1900
```

<210> 83
<211> 2557
<212> DNA
<213> Homo sapiens

<400> 83

```
ttgtgctcct cgcttgcctg ttccttttcc acgcattttc caggataact gtgactccag 60
gcccgcaatg gatgccctgc aactagcaaa ttcggctttt gccgttgatc tgttcaaaca 120
actatgtgaa aaggagccac tgggcaatgt cctcttctct ccaatctgtc tctccacctc 180
tctgtcactt gctcaagtgg gtgctaaagg tgacactgca aatgaaattg gacaggttct 240
tcattttgaa aatgtcaaag atataccctt tggatttcaa acagtaacat cggatgtaaa 300
caaacttagt tccttttact cactgaaact aatcaagcgg ctctacgtag acaaatctct 360
gaatctttct acagagttca tcagctctac gaagagaccc tatgcaaagg aattggaaac 420
tgttgacttc aaagataaat tggaagaaac gaaaggtcag atcaacaact caattaagga 480
tctcacagat ggccactttg agaacatttt agctgacaac agtgtgaacg accagaccaa 540
aatccttgtg gttaatgctg cctactttgt tggcaagtgg atgaagaaat ttcctgaatc 600
agaaacaaaa gaatgtcctt tcagactcaa caagacagac accaaaccag tgcagatgat 660
gaacatggag gccacgttct gtatgggaaa cattgacagt atcaattgta agatcataga 720
gcttcctttt caaaataagc atctcagcat gttcatccta ctacccaagg atgtggagga 780
tgagtccaca ggcttggaga agattgaaaa acaactcaac tcagagtcac tgtcacagtg 840
gactaatccc agcaccatgg ccaatgccaa ggtcaaactc tccattccaa aatttaaggt 900
ggaaaagatg attgatccca aggcttgtct ggaaaatcta gggctgaaac atatcttcag 960
tgaagacaca tctgatttct ctggaatgtc agagaccaag ggagtggccc tatcaaatgt 1020
tatccacaaa gtgtgcttag aaataactga agatggtggg gattccatag aggtgccagg 1080
agcacggatc ctgcagcaca aggatgaatt gaatgctgac catcccttta tttacatcat 1140
caggcacaac aaaactcgaa acatcatttt ctttggcaaa ttctgttctc cttaagtggc 1200
atagcccatg ttaagtcctc cctgactttt ctgtggatgc cgatttctgt aaactctgca 1260
tccagagatt cattttctag atacaataaa ttgctaatgt tgctggatca ggaagccgcc 1320
agtacttgtc atatgtagcc ttcacacaga tagacctttt ttttttttcca attctatctt 1380
ttgtttcctt ttttcccata agacaatgac atacgctttt aatgaaaagg aatcacgtta 1440
gaggaaaaat atttattcat tatttgtcaa attgtccggg gtagttggca gaaatacagt 1500
cttccacaaa gaaaattcct ataaggaaga tttggaagct cttcttccca gcactatgct 1560
ttccttcttt gggatagaga atgttccaga cattctcgct tccctgaaag actgaagaaa 1620
gtgtagtgca tgggacccac gaaactgccc tggctccagt gaaacttggg cacatgctca 1680
ggctactata ggtccagaag tccttatgtt aagccctggc aggcaggtgt ttattaaaat 1740
tctgaatttt ggggattttc aaaagataat attttacata cactgtatgt tatagaactt 1800
catggatcag atctggggca gcaacctata aatcaacacc ttaatatgct gcaacaaaat 1860
gtagaatatt cagacaaaat ggatacataa agactaagta gcccataagg ggtcaaaatt 1920
tgctgccaaa tgcgtatgcc accaacttac aaaaacactt cgttcgcaga gcttttcaga 1980
ttgtggaatg ttggataagg aattatagac ctctagtagc tgaaatgcaa gaccccaaga 2040
ggaagttcag atcttaatat aaattcactt tcatttttga tagctgtccc atctggtcat 2100
gtggttggca ctagactggt ggcaggggct tctagctgac tcgcacaggg attctcacaa 2160
tagccgatat cagaatttgt gttgaaggaa cttgtctctt catctaatat gatagcggga 2220
aaaggagagg aaactactgc ctttagaaaa tataagtaaa gtgattaaag tgctcacgtt 2280
accttgacac atagtttttc agtctatggg tttagttact ttagatggca agcatgtaac 2340
ttatattaat agtaatttgt aaagttgggt ggataagcta tccctgttgc cggttcatgg 2400
attacttctc tataaaaaat atatatttac caaaaaattt tgtgacattc cttctcccat 2460
ctcttccttg acatgcattg taaataggtt cttcttgttc tgagattcaa tattgaattt 2520
ctcctatgct attgacaata aaatattatt gaactac                          2557
```

<210> 84
<211> 3503
<212> DNA
<213> Homo sapiens

<400> 84

gctggtgtca gagcccggcg agcgctggca gttccgcggc ggggatgctg aggagcgctg 60
ggtccgggag cagccctggc ccctgcggac ttccgaggcc gtgaaaaccc ctgcgctgcg 120
gcccttccca ggcccccgag gccgttcgcc gttcccgaag cccgactggg ggaagagtcc 180
agcaccaaag cggccgttct cggattccgg agcgttctgg agccccgaga gacgccccgg 240
ggttctagaa gctccccggc ggcgcccagt cccggcttca ttcgggcgtc cctccgaaac 300
ccactcgggt gcacgggtcg tcggcgagcc gcgaccgggt cctggcgcgc accatgatcg 360
tggcggactc cgagtgccgc gcagagctca aggactacct gcggttcgcc ccgggcggcg 420
tcggcgactc gggccccgga gaggagcaga gggagagccg ggctcggcga ggccctcgag 480
ggcccagcgc cttcatcccc gtggaggagg tccttcggga gggggctgag agcctcgagc 540
agcacctggg gctggaggca ctgatgtcct ctgggcgagt agacaacctg gcagtggtga 600
tgggcctgca ccctgactac tttaccagct tctggcgcct gcactacctg ctgctgcaca 660
cggatggtcc cttggccagc tcctggcgcc actacattgc catcatggct gccgcccgcc 720
atcagtgttc ttacctggta ggctcccaca tggccgagtt tctgcagact ggtggtgacc 780
ctgagtggct gctgggcctc caccgggccc ccgagaagct gcgcaaactc agcgagatca 840
acaagttgct ggcgcatcgg ccatggctca tcaccaagga acacatccag gccttgctga 900
agaccggcga gcacacttgg tccctggccg agctcattca ggctctggtc ctgctcaccc 960
actgccactc gctctcctcc ttcgtgtttg gctgtggcat cctccctgag gggggatgcag 1020
atggcagccc tgccccccag gcacctacac cccctagtga acagagcagc cccccaagca 1080
gggacccgtt gaacaactct gggggctttg agtctgcccg cgacgtggag gcgctgatgg 1140
agcgcatgca gcagctgcag gagagcctgc tgcgggatga ggggacgtcc caggaggaga 1200
tggagagccg ctttgagctg gagaagtcag agagcctgct ggtgaccccc tcagctgaca 1260
tcctggagcc ctctccacac ccagacatgc tgtgctttgt ggaagaccct actttcggat 1320
atgaggactt cactcggaga ggggctcagg caccccctac cttccgggcc caggattata 1380
cctgggaaga ccatggctac tcgctgatcc agcggcttta ccctgagggt gggcagctgc 1440
tggatgagaa gttccaggca gcctatagcc tcacctacaa taccatcgcc atgcacagtg 1500
gtgtggacac ctccgtgctc cgcagggcca tctggaacta tatccactgc gtctttggca 1560
tcagatatga tgactatgat tatggggagg tgaaccagct cctggagcgg aacctcaagg 1620
tctatatcaa gacagtggcc tgctacccag agaagaccac ccgaagaatg tacaacctct 1680
tctggaggca cttccgccac tcagagaagg tccacgtgaa cttgctgctc ctggaggcgc 1740
gcatgcaagc cgctctgctg tacgccctcc gtgccatcac ccgctacatg acctgactcc 1800
tgagcaggac ctgggcccgg ttcagctccc cacaaggact tctctgtctg gagacagccc 1860
cagacccttt tgtgtcccat gcccaccctc cccacgctgc agtgggcttg tgtgtgatgt 1920
gcagtcccga agccacaccc tccctttcc tcactggaat ggacagttca ttgcactgac 1980
tctgggatct cagccctgct cctgggagct ggaagagcac ttggagatcc taagggacca 2040
caccccttcct ccttcccctg cccacagagg cagagggcac aggaaagaag ccgggccaag 2100
ctcggaatta atgtgccaca agtgttgtgg ccttcctgaa ctgggaagtc cctggctggc 2160
ccccgggggga gaggggcaaa tgcctccggg actgacactc caggcagctt tgccttctct 2220
cccctgtcat ttccagattt cattacctcc tacttgccat tcacccatca atgtgaaagt 2280
cagggtcaca gctggtctgt gtgtccagtt ccctaaaagc ctgttctgtt gggcagcctg 2340
aggctgttgc ccgaatccta gttcagtttt ttgacttcct ttgccctttt tccctttctct 2400
ccatgcttaa tggtgtgagg cgtcaggaga gaggccaagt acataaaaaa aaaaaaagca 2460
gattatctct agagagtttg agcctttgct ggtcacattg ccttctgaag aggagggagt 2520
attagattat aaatcctctt tattttggtc ctttatgctt gaggttccaa cctggagcca 2580
cagtgtgtga gaggaggagg agagggagaa ttctgttctc ccagagctgc acctgcctcg 2640
cagaggccag caccccactc tcctgcctcc agtggccctg ccgcagatgt ctcccaaaaa 2700
gttgagcctt tctagatggc ttaggtggca ccatggctca gcaggagggg cgggaggcac 2760
cagggttctt gtttggaccc tgcccctggg ccatggccag gtgaccatgg ctacattgcc 2820
aaacctctga ctgccacagc tgcagactga gagggtgggt ctgagtcccc acaatgtctg 2880
aagctgcccc tgggattctc aggccaacct gccaacagca agcggatttt cttgcaagat 2940
cagggacccc atttctgcag ccagtgtctc ctgggtgcct tctgaggact cccaccccca 3000
tcccagtatc tcatctgtcc cctctcctgg ggcttaagtg ggttgcttcc aggcagaagc 3060
agccaaggac cgattccagg cactttctgt agcaaatgac tgtgaattac gacttctctt 3120
gcccttcttc tagcagtctg tgcctcctct ctgaccagtt tggagggcac tgaagaaagg 3180
caagggccgt gctgctgctg ggcggggcag gagaggagcc tggccagtgt gccacattaa 3240
atacccgtgc aggcgcggag aagcaaccgg caccccccttc cggcctgaaa gccctccctg 3300
caagaaggtg tgcaggagag aagaggcccc ggcatgggga tctgggttct agagggcatg 3360
tgatgactgt aaatgttcac tgggtgggta gggagtggta tccagtgttc aagtgcagaa 3420
atctttggct ttgctaccag ttccatatga tgagaaataa acgttcgctg aggttttgtt 3480

tcataaaaaa aaaaaaaaaa aaa 3503

<210> 85
<211> 1336
<212> DNA
<213> Homo sapiens

<400> 85

```
gagagacaca gagtccggca ttggtcccag gcagcagtta gcccgccgcc cgcctgtgtg 60
tccccagagc catggagaga gccagtctga tccagaaggc caagctggca gagcaggccg 120
aacgctatga ggacatggca gccttcatga aaggcgccgt ggagaagggc gaggagctct 180
cctgcgaaga gcgaaacctg ctctcagtag cctataagaa cgtggtgggc ggccagaggg 240
ctgcctggag ggtgctgtcc agtattgagc agaaaagcaa cgaggagggc tcggaggaga 300
aggggcccga ggtgcgtgag taccgggaga aggtggagac tgagctccag ggcgtgtgcg 360
acaccgtgct gggcctgctg gacagccacc tcatcaagga ggccggggac gccgagagcc 420
gggtcttcta cctgaagatg aagggtgact actaccgcta cctggccgag gtggccaccg 480
gtgacgacaa gaagcgcatc attgactcag cccggtcagc ctaccaggag gccatggaca 540
tcagcaagaa ggagatgccg cccaccaacc ccatccgcct gggcctggcc ctgaactttt 600
ccgtcttcca ctacgagatc gccaacagcc ccgaggaggc catctctctg gccaagacca 660
ctttcgacga ggccatggct gatctgcaca ccctcagcga ggactcctac aaagacagca 720
ccctcatcat gcagctgctg cgagacaacc tgacactgtg gacggccgac aacgccgggg 780
aagagggggg cgaggctccc caggagcccc agagctgagt gttgcccgcc accgccccgc 840
cctgcccccct ccagtccccc accctgccga gaggactagt atggggtggg aggccccacc 900
cttctcccct aggcgctgtt cttgctccaa aggctccgt ggagagggac tggcagagct 960
gaggccacct ggggctgggg atcccactct tcttgcagct gttgagcgca cctaaccact 1020
ggtcatgccc ccacccctgc tctccgcacc cgcttcctcc cgaccccagg accaggctac 1080
ttctcccctc ctcttgcctc cctcctgccc ctgctgcctc tgatcgtagg aattgaggag 1140
tgtcccgcct tgtggctgag aactggacag tggcaggggc tggagatggg tgtgtgtgtg 1200
tgtgtgtgtg tgtgtgtgtg tgtgcgcgcg cgccagtgca agaccgagat tgagggaaag 1260
catgtctgct gggtgtgacc atgtttcctc tcaataaagt tcccctgtga cactcaaaaa 1320
aaaaaaaaaa aaaaaa 1336
```

<210> 86
<211> 2035
<212> DNA
<213> Homo sapiens

<400> 86

```
ttaaagtcgc ccccggcgcg gagcggaccg gcagaggcgg gcagaggcgg cgagaggcgg 60
cgagaggcgg gctgaggcgg cccagcggcg gcaggtgagg cggaaccaac cctcctggcc 120
atgggagggg ccgtggtgga cgagggcccc acaggcgtca aggcccctga cggcggctgg 180
ggctgggccg tgctcttcgg ctgtttcgtc atcactggct tctcctacgc cttccccaag 240
gccgtcagtg tcttcttcaa ggagctcata caggagtttg ggatcggcta cagcgacaca 300
gcctggatct cctccatcct gctggccatg ctctacggga caggtccgct ctgcagtgtg 360
tgcgtgaacc gctttggctg ccggcccgtc atgcttgtgg ggggtctctt tgcgtcgctg 420
ggcatggtgg ctgcgtcctt ttgccggagc atcatccagg tctacctcac cactggggtc 480
atcacggggt tgggtttggc actcaacttc cagccctcgc tcatcatgct gaaccgctac 540
ttcagcaagc ggcgccccat ggccaacggg ctggcggcag caggtagccc tgtcttcctg 600
tgtgccctga gcccgctggg gcagctgctg caggaccgct acggctggcg gggcggcttc 660
ctcatcctgg gcggcctgct gctcaactgc tgcgtgtgtg ccgcactcat gaggcccctg 720
gtggtcacgg cccagccggg ctcggggccg ccgcgaccct cccggcgcct gctagacctg 780
agcgtcttcc gggaccgcgg ctttgtgctt tacgccgtgg ccgcctcggt catggtgctg 840
gggctcttcg tcccgcccgt gttcgtggtg agctacgcca aggacctggg cgtgcccgac 900
accaaggccg ccttcctgct caccatcctg ggcttcattg acatcttcgc gcggccggcc 960
gcgggcttcg tggcggggct tgggaaggtg cggccctact ccgtctacct cttcagcttc 1020
tccatgttct tcaacggcct cgcggacctg gcgggctcta cggcgggcga ctacggcggc 1080
ctcgtggtct tctgcatctt ctttggcatc tcctacggca tggtgggggc cctgcagttc 1140
gaggtgctca tggccatcgt gggcacccac aagttctcca gtgccattgg cctggtgctg 1200
ctgatggagg cggtggccgt gctcgtcggg ccccccttcgg gaggcaaact cctggatgcg 1260
acccacgtct acatgtacgt gttcatcctg gcgggggccg aggtgctcac ctcctccctg 1320
attttgctgc tgggcaactt cttctgcatt aggaagaagc ccaaagagcc acagcctgag 1380
gtggcggccg cggaggagga gaagctccac aagcctcctg cagactcggg ggtggacttg 1440
cgggaggtgg agcatttcct gaaggctgag cctgagaaaa acggggaggt ggttcacacc 1500
ccggaaacaa gtgtctgagt ggctgggcgg ggccggcagg cacagggagg aggtacagaa 1560
gccggcaacg cttgctattt attttacaaa ctggactggc tcaggcaggg ccacggctgg 1620

gctccagctg ccggcccagc ggatcgtcgc ccgatcagtg ttttgagggg gaaggtggcg 1680
gggtgggaac cgtgtcattc cagagtggat ctgcggtgaa gccaagccgc aaggttacaa 1740
ggcatcctca ccagggggccc cgcctgctgc tcccaggtgg cctgcggcca ctgctatgct 1800
caaggacctg gaaacccatg cttcgagaca acgtgacttt aatgggaggg tgggtgggcc 1860
gcagacaggc tggcagggca ggtgctgcgt ggggccctct ccagcccgtc ctaccctggg 1920
ctcacatggg gcctgtgccc acccctcttg agtgtcttgg ggacagctct ttccacccct 1980
ggaagatgga aataaacctg cgtgtgggtg gagtgttagg accaacggtt tccta      2035
```

<210> 87
<211> 2195
<212> DNA
<213> Homo sapiens

<400> 87

```
ggctgcaggc ggggccggcg ggggcgcagc ggcccgggag gaagcagtac cttgaagaga 60
aattggagag ggagtcaatt cctaggatag cagagagatg gacaacagac agaatagatg 120
gagtttcaca atggtggcca tgtgtctgga attggtgggt tcttggtctc actgacttca 180
agaatgaagc cgcacaccct cgcagtcacc ccagctctga tctttgccat cacagttgct 240
acaatcggct ctttccagtt tggctacaac actggggtca tcaatgctcc tgagacgatc 300
ataaaggaat ttatcaataa aactttgacg gacaaggcaa atgcccctcc ctctgaggtg 360
ctgctcacga atctctggtc cttgtctgtg gccatatttt ccgtcggggg tatgatcggc 420
tccttttccg tcggactctt tgttaaccgc tttggcaggc gcaattcaat gctgattgtc 480
aacctgttgg ctgccactgg tggctgcctt atgggactgt gtaaaatagc tgagtcagtt 540
gaaatgctga tcctgggccg cttggttatt ggcctcttct gcggactctg cacaggtttt 600
gtgcccatgt acattggaga gatctcgcct actgccctga ggggtgcctt tggcactctc 660
aaccagctgg gcatagttat tggaattctg gtggcccaga tctttggtct ggaactcatc 720
cttgggtctg aagagctatg gccggtgcta ttaggcttta ccatccttcc agctatcctg 780
caaagtgcag cccttccatg ttgccctgaa agtcccagat tttgctcat taacagaaaa 840
aaagaggaga atgctacgcg gatcctccag cggttgtggg gcacccagga tgtatcccaa 900
gacatccagg agatgaaaga tgagagtgca aggatgtcac aagaaaagca agtcaccgtg 960
ctggagctct ttagagtgtc cagctaccga cagcccatca tcatttccat tgtgctccag 1020
ctctctcagc agctctctgg gatcaatgct gtgttctatt actcaacagg aatcttcaag 1080
gatgcaggtg ttcaacagcc catctatgcc accatcagcg cgggtgtggt taatactatc 1140
ttcactttac tttctctatt tctggtggaa agggcaggaa gaaggactct gcatatgata 1200
ggccttggag ggatggcttt ttgttccacg ctcatgactg tttctttgtt attaaagaat 1260
cactataatg ggatgagctt tgtctgtatt ggggctatct tggtctttgt ggcctgtttt 1320
gaaattggac caggccccat tccctggttt attgtggccg aactcttcag ccagggcccc 1380
cgcccagctg cgatggcagt ggccggctgc tccaactgga cctccaactt cctagtcgga 1440
ttgctcttcc cctctgctgc ttactattta ggagcctacg tttttattat cttcaccggc 1500
ttcctcatta ccttcttggc ctttaccttc ttcaaagtcc ctgagacccg tggcaggact 1560
tttgaggata tcacacgggc ctttgaaggg caggcacacg gtgcagatag atctgggaag 1620
gacggcgtca tggggatgaa cagcatcgag cctgctaagg agaccaccac caatgtctaa 1680
gtcatgcctc cttccacctc cctcccggca tgggaaagcc acctctccct caacaaggga 1740
gagactttat caggatgaac ccaggactgc ttctgaatgc tgctacttga tttctttctc 1800
atcccacgca ctccatgagc accccaaggc tgcagtttgt tggatcttca atggcttttt 1860
aaattttatt tcctggacat cctcttctgc ttaggagaga ccgagtgaac ctaccttcat 1920
ttcaggaggg attggccgct tggcacatga caactttgcc agcttttcct cccttgggtt 1980
ctgatattgc cgcactagag gatataggag aggaaaagta aggtgcagtt gccccaacct 2040
cagacttacc aggaagcaga tacatatgag tgtggaagcc ggagggtgtt tatgtaagag 2100
caccttcctc acttccatac agctctacgc ggcaaattaa cttgagtttt atttatctta 2160
tcctctggtt taattacata aatatttatt tttta                           2195
```

<210> 88
<211> 3915
<212> DNA
<213> Homo sapiens

<400> 88

```
gtggggtggg gtggggctgg gggcttgtcg ccctttcagg ctccaccctt tgcggagatt 60
ataaatagtc atgatcccag cgagacccag agatgcctgt aatggtgaga ctttggatcc 120
ttcctgagga cgtggagaaa actttctgct gagaaggaca ttttgaaggt tttgttggct 180
gaaaaagctg tttctggaat cacccctaga tctttcttga agacttgaat tagattacag 240
cgatggggac acagaaggtc accccagctc tgatatttgc catcacagtt gctacaatcg 300
gctctttcca atttggctac aacactgggg tcatcaatgc tcctgagaag atcataaagg 360
aatttatcaa taaaactttg acggacaagg gaaatgcccc accctctgag gtgctgctca 420
```

cgtctctctg gtccttgtct gtggccatat tttccgtcgg gggtatgatc ggctcctttt 480
ccgtcggact cttcgtcaac cgctttggca ggcgcaattc aatgctgatt gtcaacctgt 540
tggctgtcac tggtggctgc tttatgggac tgtgtaaagt agctaagtcg gttgaaatgc 600
tgatcctggg tcgcttggtt attggcctct tctgcggact ctgcacaggt tttgtgccca 660
tgtacattgg agagatctcg cctactgccc tgcggggtgc ctttggcact ctcaaccagc 720
tgggcatcgt tgttggaatt ctggtggccc agatctttgg tctggaattc atccttgggt 780
ctgaagagct atggccgctg ctactgggtt ttaccatcct tcctgctatc ctacaaagtg 840
cagcccttcc attttgccct gaaagtccca gatttttgct cattaacaga aaagaagagg 900
agaatgctaa gcagatcctc cagcggttgt ggggcaccca ggatgtatcc caagacatcc 960
aggagatgaa agatgagagt gcaaggatgt cacaagaaaa gcaagtcacc gtgctagagc 1020
tctttagagt gtccagctac cgacagccca tcatcatttc cattgtgctc cagctctctc 1080
agcagctctc tgggatcaat gctgtgttct attactcaac aggaatcttc aaggatgcag 1140
gtgttcaaga gcccatctat gccaccatcg gcgcgggtgt ggttaatact atcttcactg 1200
tagtttctct atttctggtg gaaagggcag gaagaaggac tctgcatatg ataggccttg 1260
gagggatggc tttttgttcc acgctcatga ctgtttcttt gttattaaag gataactata 1320
atgggatgag ctttgtctgt attggggcta tcttggtctt tgtagccttc tttgaaattg 1380
gaccaggccc cattccctgg tttattgtgg ccgaactctt cagccagggc cccgcccag 1440
ctgcgatggc agtggccggc tgctccaact ggacctccaa cttcctagtc ggattgctct 1500
tcccctccgc tgctcactat ttaggagcct acgtttttat tatcttcacc ggcttcctca 1560
ttaccttctt ggcttttacc ttcttcaaag tccctgagac ccgtggcagg acttttgagg 1620
atatcacacg ggcctttgaa gggcaggcac acggtgcaga tagatctgga aaggacggcg 1680
tcatggagat gaacagcatc gagcctgcta aggagaccac caccaatgtc taagtcgtgc 1740
ctccttccac ctccctcccg gcatgggaaa gccacctctc cctcaacaag ggagagacct 1800
catcaggatg aacccaggac gcttctgaat gctgctactt aattcctttc tcatcccacg 1860
cactccatga gcaccccaag gctgcggttt gttggatctt caatggcttt ttaaatttta 1920
tttcctggac atcctcttct gcttaggaga gaccgagtga acctaccttc atttcaggag 1980
ggattggccg cttggcacat gacaactttg ccagcttttc ctcccttggg ttctgatatt 2040
gccgcactag gggatatagg agaggaaaag taaggtgcag ttccccaac ctcagactta 2100
ccaggaagca gatacatatg agtgtggaag ccggagggtg tttatgtaag agcaccttcc 2160
tcacttccat acagctctac gtggcaaatt aacttgagtt ttatttattt tatcctctgg 2220
tttaattaca taattttttt tttttactt taagtttcag gatacatgtg ccgaatgtgc 2280
aggtttgtta cataggtata tatatgccat gatggaaata tttatttttt taagcgtaat 2340
tttgccaaat aataaaaaca gaaggaaatt gagattagag ggaggtgttt aaagagaggt 2400
tatagagtag aagatttgat gctggagagg ttaaggtgca ataagaattt agggagaaat 2460
gttgttcatt attggagggt aaatgatgtg gtgcctgagg tctgtacgtt acctcttaac 2520
aatttctgtc cttcagatgg aaactcttta acttctcgta aaagtcatat acctatataa 2580
taaagctact gatttccttg gagcttttt ctttaagata atagtttaca tgtagtagta 2640
cttgaaatct aggattatta actaatatgg gcattgtagt taatgatggt tgatgggttc 2700
taattttgga tggagtccag ggaagagaaa gtgatttcta gaaagcctgt tcccctcact 2760
ggatgaaata actccttctt gtagtagtct cattactttt gaagtaatcc cgccacctat 2820
ctcgtgggag agccatccaa ataagaaacc taaaataatt ggttcttggt agagattcat 2880
tatttttcca ctttgttctt taggagattt taggtgttga ttttctgttg tattttaact 2940
catacctta aaggaattcc ccaaagaatg tttatagcaa acttggaatt tgtaacctca 3000
gctctgggag aggatttttt tctgagcgat tattatctaa agtgtgttgt tgctttaggc 3060
tcacggcacg cttgcgtatg tctgttacca tgtcactgtg gtcctatgcc gaatgccctc 3120
aggggacttg aatctttcca ataaaccagg tttagacagt atgagtcaat gtgcagtgta 3180
gcccacactt gagaggatga atgtatgtgc actgtcactt tgctctgggt ggaagtacgt 3240
tattgttgac ttattttctc tgtgtttgtt cctacagccc ctttttcata tgttgctcag 3300
tctccctttc ccttcttggt gcttacacat ctcagaccct ttagccaaac ccttgtcagt 3360
gacagtattt tggttcttag ttctcactgt tccctctgct cctggagcct ttgaataaaa 3420
atgcacgtag ctgaggccgg atgcggtggc tcacgcctgt aatcccagca ctttgggagg 3480
cctaggcggg cggtcagggg ttcgagacca gtctggccaa catcgtgaaa ccctgtctct 3540
actaaaaatg caaaaattag ccgggcgtgg tggcgggcgc ctgtaatccc agctacttgg 3600
gaagctgagg cgggagaatc atgtgaaccc gggacgcagg ggttgcagtg agcggagatc 3660
gcatcattgc actctagcct gggccacagg gcgagactcc gtctcaaaaa aaaaaaaatg 3720
cacatagcta tcgagtgtgc tttagcttga aaaggtgacc ttgcaacttc atgtcaactt 3780
tctggctcct caaacagtag gttggcagta aggcagggtc ccatttctca ctgagaagat 3840
tgtgaatatt tccatatgga ttttctattg ttactctggt tctttgtttt aaaataaaaa 3900
ttctgaatgt acacg 3915

<210> 89

<211> 4516
<212> DNA
<213> Homo sapiens

<400> 89

```
ctcggcgcgc ctggacccct gcccctctct gggtggagaa gctcccggcc gcttcccggt 60
ttcactcctt ctcagcctgg gctcccagcc cctctctcct tttcctggac tggctctcac 120
ccccttcggt ccccttcctt tagctcaggc tccctacccc ttcctttagc ccacagccca 180
gagtcccagc tcctcagtca ctttcctcag ccaaaggtcc cagccttcct tcttcctttc 240
ctttgcacta tccctatcct gccccttcct ctatccctag ggctcagttt cccacatccg 300
tcctccccct tcccaggccc ggagttccag accttttggt ctcctttcgt ggtcgttcct 360
gggtccttgc cccctttccc cactttggag ttccagattg caaacccagc ctccctccac 420
ccccagaaaa ttgcttccat ggaaatgcct ctctaaaaca tgaacttttc ctagagacta 480
cgccagtctc tcttcccact tgctgaccct ttgctaccta tgtgcccggt tttactctca 540
tttgggtaag gtcgaggctg gctctggaag cagcaccatg gttctgcggt ctggcatctg 600
tggcctctct ccacatcgga tcttcccttc cttactcgtg gtggttgctt tggtggggct 660
gctgcctgtt ctcaggagcc atggcctcca gctcagccca actgccagca ccattcgaag 720
ctcagagcca ccacgagaac gctcgattgg ggatgtcacc accgctccac cggaggtcac 780
cccagagagc cgccctgtta atcattccgt cactgatcat ggcatgaagc cgcgcaaggc 840
ctttccagtc ctgggcatcg actacacaca cgtgcgcacc cccttcgaga tctccctctg 900
gatccttctg gcctgcctca tgaagatagg tttccatgtg atccccacta tctcaagcat 960
cgtcccggag agctgcctgc tgatcgtggt ggggctgctg gtgggggggcc tgatcaaggg 1020
tgtaggcgag acaccccccct tcctgcagtc cgacgtcttc ttcctcttcc tgctgccgcc 1080
catcatcctg gatgcgggct acttcctgcc actgcggcag ttcacagaaa acctgggcac 1140
catcctgatc tttgccgtgg tgggcacgct gtggaacgcc ttcttcctgg gcggcctcat 1200
gtacgccgtg tgcctggtgg gcggtgagca gatcaacaac atcggcctcc tggacaacct 1260
gctcttcggc agcatcatct cggccgtgga ccccgtggcg gttctggctg tctttgagga 1320
aattcacatc aatgagctgc tgcacatcct tgtttttggg gagtccttgc tcaatgacgc 1380
cgtcactgtg gtcctgtatc acctctttga ggagtttgcc aactacgaac acgtgggcat 1440
cgtggacatc ttcctcggct tcctgagctt cttcgtggtg gccctgggcg gggtgcttgt 1500
gggcgtggtc tacggggtca tcgcagcctt cacctcccga tttacctccc acatccgggt 1560
catcgagccg ctcttcgtct tcctctacag ctacatggcc tacttgtcag ccgagctctt 1620
ccacctgtca ggcatcatgg cgctcatagc ctcaggagtg gtgatgcgcc cctatgtgga 1680
ggccaacatc tcccacaagt cccacaccac catcaaatac ttcctgaaga tgtggagcag 1740
cgtcagcgag accctcatct tcatcttcct cggcgtctcc acggtggccg gctcccacca 1800
ctggaactgg accttcgtca tcagcaccct gctcttctgc ctcatcgccc gcgtgctggg 1860
ggtgctgggc ctgacctggt tcatcaacaa gttccgtatc gtgaagctga cccccaagga 1920
ccagttcatc atcgcctatg gggggcctgcg aggggccatc gccttctctc tgggctacct 1980
cctggacaag aagcacttcc ccatgtgtga cctgttcctc actgccatca tcactgtcat 2040
cttcttcacc gtctttgtgc agggcatgac cattcggccc ctggtagacc tgttggctgt 2100
gaagaaaaag caagagacga agcgctccat caacgaagag atccacacac agttcctgga 2160
ccaccttctg acaggcatcg aagacatctg tggccactac ggtcaccacc actggaagga 2220
caagctcaac cggtttaata agaaatatgt gaagaagtgt ctgatagctg gcgagcgctc 2280
caaggagccc cagctcattg ccttctacca caagatggag atgaagcagg ccatcgagct 2340
ggtggagagc gggggcatgg gcaagatccc ctctgccgtc tccaccgtct ccatgcagaa 2400
catccacccc aagtccctgc cttccgagcg catcctgcca gcactgtcca aggacaagga 2460
ggaggagatc cgcaaaatcc tgaggaacaa cttgcagaag accaggcagc ggctgcggtc 2520
ctacaacaga cacacgctgg tggcagaccc ctacgaggaa gcctggaacc agatgctgct 2580
ccggaggcag aaggcccggc agctggagca gaagatcaac aactacctga cggtgccagc 2640
ccacaagctg gactcaccca ccatgtctcg ggcccgcatc ggctcagacc cactggccta 2700
tgagccgaag gaggacctgc ctgtcatcac catcgacccg gcttccccgc agtcacccga 2760
gtctgtggac ctggtgaatg aggagctgaa gggcaaagtc ttagggttga gccgggatcc 2820
tgcaaaggtg gctgaggagg acgaggacga cgatgggggc atcatgatgc ggagcaagga 2880
gacttcgtcc ccaggaaccg acgatgtctt cacccccgcg cccagtgaca gccccagctc 2940
ccagaggata cagcgctgcc tcagtgaccc aggcccacac cctgagcctg ggggagggaga 3000
accgttcttc cccaaggggc agtaacgcca gggccagcag gcagcgcctg tcccctcaca 3060
gactcttcca ccagagcagg ggctgctggg ggctcccctt gcccttcctg acccggattg 3120
gccctgcccc tcccccctacc gcatggcagc tgggcccaca gcccccaccc cagcacagct 3180
cctcccctgc cgcctcccgg gaagcatcct ccccaccaga gctgcctccc caatccattt 3240
ggcagaactg ctggggctgg tgaggccggc cctgcccctc cctagatcca ggcttctccc 3300
ggacctggac tagggcctcg gaggctcctc cctctgcctc atcctcctcc tcattcagac 3360
caatcttagt ttctaaccaa agagtctctg gctcagctgt ggtcccaccc aggaagggag 3420
ggagctgagg cctcccttga gtaggccctg ctttatcagg ggacaaacca ggggtaccag 3480
gcacatggct gggggaggga ctgctgaccc accaaggtct cacactcctc ctgccagctc 3540
tgtcaccctg gccaccaccc aacctatcct tactcagagc tgcgggctga gggcatctct 3600
gagtgtctct gcctggagca ggggtggttt ctacggtgac agtgacgtga ctcagagctt 3660
ttcgaactgt gctcccacgg ggaccactgg gcccctcagg ggaagctgct aggggaagga 3720
ctggccgtgg ctccagaatg tgctgccttt ttaagttttg tttgttcaca ctcctatata 3780
tgattgtttg cacagagggc gctcctgttt ttaaaacatt ttgaaaaccc ctggctgaac 3840
```

agtgctctgc ctctaactcc ctcctcacac tccagaatta cccttcctca tctgtgcctg 3900
tctgtccaac ccctccccca cgtctctctg cctgctgggc tcttaactgt tgctcgaaga 3960
ctgtgacatc agaagtaact cccactccta atcaagagtc tctccagcct cacagatgct 4020
ggcctcttgg cacctgccta gctcttgggc ctgacctcca gtcctgctgg cctgctctta 4080
cttcccccac cctgggtttg gcccctggaa cctttccctt gtgtgtacca caccctgcct 4140
gctgtggagc ccattgtgga ggcggtgggg gggagaaggc ctcccctgag gatcccctgt 4200
cccctggggc tggtggattg ggcagaatcc tgggccccca gagacctttg cccacacaca 4260
ctccttcccc ttgtccctgg ggcactcccc caggattgtg caatagtcag agtgtccctt 4320
tttgcagggg actgggccat gggtcctcgg cccatctgtc catcctcctc tccatgcaag 4380
tgctgtttgg gcaggagtca ccatgcaagg gtgacatcga caaccacgta ccaagccacc 4440
gcagctgctg ccactctgct gcctgtacag aagaaactga atctttttca tattctaata 4500
aatcaatgtg agtttt 4516

<210> 90
<211> 4934
<212> DNA
<213> Homo sapiens

<400> 90

gcgagctgag ctgacagcgc ggagctggcg ctgtggagcg cagggagcct tgccggttcc 60
tccgaccggc gtctgcgagt acagcggcgg ctaacctgcc ccggcttcag gatttacaca 120
gacgtggggc gatgcttgtg accctgcagc tcctcaaacc agcctgtatt gagcggtttg 180
cagcctgatg ctcagccccc tccccacagg gcccctagaa gcctgtttct ccgtacagtc 240
caggacctcc agccccatgg agcccccgat cccacagagc gccccccttga ctcccaactc 300
agtcatggtc cagccccttc ttgacagccg gatgtcccac agccggctcc agcacccact 360
caccatccta cccattgacc aggtgaagac cagccatgtg gagaatgact acatagacaa 420
ccctagcctg gccctgacca ccggcccaaa gcggacccgg ggcggggccc cagagctggc 480
cccgacgccc gcccgctgtg accaggatgt cacccaccat tggatctcct tcagcgggcg 540
ccccagctct gtgagcagca gcagcagcac atcctctgac caacggctct tagaccacat 600
ggcaccacca cccgtggctg accaggcctc accaagggct gtgcgcatcc agcccaaggt 660
ggtccactgc cagccgctgg acctcaaggg cccggcggtc ccacccgagc tggacaagca 720
cttcttgctg tgcgaggcct gtgggaagtg taaatgcaag gagtgtgcat ccccccggac 780
gttgccttcc tgctgggtct gcaaccagga gtgcctgtgc tcagcccaga ctctggtcaa 840
ctatggcacg tgcatgtgtt tggtgcaggg catcttctac cactgcacga atgaggacga 900
tgagggctcc tgcgctgacc acccctgctc ctgctcccgc tccaactgct gcgcccgctg 960
gtccttcatg ggtgctctct ccgtggtgct gccctgcctg ctctgctacc tgcctgccac 1020
cggctgcgtg aagctggccc agcgtggcta cgaccgtctg cgccgccctg gttgccgctg 1080
caagcacacg aacagcgtca tctgcaaagc agccagcggg gatgccaaga ccagcaggcc 1140
cgacaagcct ttctgacagt ttgtgtcgaa gccccagtgc tctgcctgga aacctggttc 1200
tcttctgaca tctaagaaga ctgcagcaag gtcagaggtt ttagcctcct gaggctgacc 1260
ttgctagtct gcccactccc tacccccagc ttcggaaaat acagagacca ccaccacgta 1320
ccctgtattc cccaaggtga tgaagaagca ctttggggct ttttttcagg gtcctgaaac 1380
tttgtgtcaa acagacaatg caggggcagg gtgtggtttg gggggaaatt tttctttttc 1440
agaagacaga acacagatgt ggacacatat ccggaaactg cagctgcttg aatgccttcc 1500
cagcccctcc ttctccctcc ctccctccgc cccccccttc ctcttttcca ttgtctttgg 1560
ctctcacagg agctagctgc ctgggaggaa ttgttaactg agtaccaggg tacctttaaa 1620
gaagaccctt ggagtcttct ataccttctt ctccttcccc atctcactcc accccacttt 1680
gtccctgatg tcttggggaa ggtgtagaac accctagcag ttcctattgt atatacttgg 1740
gagccactga gaacagagga cggccagtga gtccaagcct cgttcctcct tctgcctccc 1800
cggagccaca ggatggattt aggagccact gctcagtgca cttctccctt ccaactgcat 1860
caactaactc tcggggtgt tctgctcacc acaccgtcct tcggttctta ctgagtcaca 1920
gactcgcctg cccactacgt gtcctgggtt ctctactcag atcccttcca gaaactttat 1980
atgggtagag gaagccaggg cggcaaatgc gagaccaaat atcattttgc caatgagtct 2040
gaggctgtgg tctctggatc cagtcattat gtttttatag aataattaaa ccggatgcta 2100
acggtgtttt aaaaaataat aataaaacaa cttgtttcct tttggccacc cccaggaagg 2160
gctgatttca aaatctgggg gcgagcaacc tcaaggaaca caatttccct ccctatcaac 2220
aagaggattt taacagcaaa gaagagaggc agcacctccc attggcagaa tgaccgctga 2280
gccaggctgg gtttgggttt cttctcttct gattctgctg ctcactgtca tagccttttg 2340
tgtatagtga tgtgtctgta tctttaatgt aaatagagag atgatgaaaa aagagtctat 2400
tttagtgtta ggaagcccca gcaggggagt cggaagagct tggaagagct ggggagaggg 2460
taggggaaag gtttttccag ggccactgg gtttgagccc tgcttctgtg cacagccaca 2520
ccaccctctc ccgacagccc tcaaagacgt agcaactctt tctctcaagg tgctaaagga 2580
ctcagaaggt gcagcacgtc cagtgggtag gtacttgttg catgcaaaag ctgtagtgta 2640
tctggtcctt cctccccagc ttttgtgtgg ggttcttgct ttgtgtggta ttttgttttc 2700
ccctctaatg agagggcatg gcctgagtca gaagagctac cccaggtgaa actggaagtg 2760

catgaggcag agcgtccgta gcatttccag tttgttctgt ataggacaga ggtgcctccg 2820
ggaaggaggc agcgaggtag gtagctatga taggcaccta atgcttctca aggacttatt 2880
ttttccttct tgaagactag tagtaacatc ttatgattta gagtaagttg attgtaacca 2940
taggtattta ttgattggag gaagggaggg tcatattatt ttcggcttta tttatgtaac 3000
atttgctagc ttataaaagg cgaatgtgaa atattgcatc tgcattttcc aaggctgatt 3060
cgtgtagcta cccttgccac agttgtgacg gatgtatgga tgttcttgaa catttcagaa 3120
ggagtggtag aaaaaaacac acattcagcc aaccacttat atgaattgaa tgtatcagaa 3180
gtgtactgaa gggactggag atggttttcc tcagatgagg gggccccaaa attgatagtg 3240
cacatctgca cgctttctgc gaggcctcag aacttttccc agggcccctc cctcaaattg 3300
tctccatggg aaacttgacc cagtggcaag ttgcactttg gtgatcttgg tggtctacac 3360
acccgttctg tggagagtcg atttacataa gctgtgtata cacacacaca cacacacaca 3420
cacacacaca cacacacacc cctaccccac actgactgtc taccgacaga gaccctattt 3480
cctggcaaac ggcctcctga accctgactt tttgtgtaca tacttgtaaa cacggatttt 3540
tctgggtttt ggtttgcttt ttcctttttt ccccctgccc ctgttctagc ttgttcttct 3600
tggtttgctt tcaacctgct tgatggatgt ctgcagagtg ctctctaaga gtccacctca 3660
gtgcctcgtg tgctcagtgg tcatgggaag gagcgaagga accatccttg gttctcccag 3720
cttggttgtg tagcaatccc tcagcattgt tttctcagc ttcttggcaa aaattaaaac 3780
aacaacaaca acaacaacaa caacaacaaa cagaaggata aactggcttg cctgtggacc 3840
ctccccggct ctggggccag tcgagagcca ctgagggacc cagcactcag agacacaaca 3900
cacatgtgta gctgcttctg gctgagtgtg tttcctgtca ccaatggcct gtttggctgg 3960
acgatgcctc ggcttgacct tttttgaaaa gtgctggtta gttcccgccc cctggtaaac 4020
ctggggtagg tgggggttct gtcttaactc gaggggcacc tgggatccag gacgcttcta 4080
gggggctctg gctgcccgtg ttaatgaagg acagcgcttc cgcgagcacc ctgggaactg 4140
ggtcttgggt agcaaagccc tcccagagaa aagatgggca caactaaggc tttcctgagc 4200
aggaaggggg tgaagaccaa tcccttcctt tggtcctttg gtacgcaccc cctcagagct 4260
gagatggaag acatggctag ttcttttcag ccttgtggag cctgtcagtc gccatcatac 4320
ctcgagtgag gcccagctag ataatgactt gtccaagatg gcacacgtgg aaagttgatc 4380
tgcaccagaa cccggatgac tgtcaccttg aagcgtcctg ttctccttct gtgctgtccc 4440
aggaagtgtc tggcgggcgt gggcagcaca gctctacact gtacgattca ctagggcatc 4500
ctgcgagcct cactagcctt ctggttcatg cctttgacaa gcatttttgt gccccctctg 4560
cttactgtga cagtcgatga tgaatcttgc gttgccattt tctgctgtgg gtaactgcgt 4620
gcagtgtctt gccttgcttt ctcttcttac tgtcccacag cttggtttca tgttacaaac 4680
agaaaagctc gaggctccca ccccgccaca tcccaacttc atttccccct cactgtagcc 4740
catttccacc ccaccacaaa gttgccacag gttttctttg tatagaatat ttattttgaa 4800
gctctatttt aatagtattt attttagaaa gtctactatt gtaagagttc ttctgtttgt 4860
gaagaaaaaa acaagttaaa aactgaatgt actgatttag aaaatatata taaatatata 4920
ttgttaaata taaa 4934

<210> 91
<211> 1203
<212> DNA
<213> Homo sapiens

<400> 91

```
agaaagccgc gcacctcctc ccgccaggcg ctttctcgga cgccttgccc agcgggccgc 60
ccgacccccct gcaccatgga ccccgctcgc cccctggggc tgtcgattct gctgcttttc 120
ctgacggagg ctgcactggg cgatgctgct caggagccaa caggaaataa cgcggagatc 180
tgtctcctgc ccctagacta cggaccctgc cgggccctac ttctccgtta ctactacgac 240
aggtacacgc agagctgccg ccagttcctg tacgggggct gcgagggcaa cgccaacaat 300
ttctacacct gggaggcttg cgacgatgct tgctggagga tagaaaaagt tcccaaagtt 360
tgccggctgc aagtgagtgt ggacgaccag tgtgaggggt ccacagaaaa gtatttcttt 420
aatctaagtt ccatgacatg tgaaaaattc ttttccggtg ggtgtcaccg gaaccggatt 480
gagaacaggt ttccagatga agctacttgt atgggcttct gcgcaccaaa gaaaattcca 540
tcattttgct acagtccaaa agatgaggga ctgtgctctg ccaatgtgac tcgctattat 600
tttaatccaa gatacagaac ctgtgatgct ttcacctata ctggctgtgg agggaatgac 660
aataactttg ttagcaggga ggattgcaaa cgtgcatgtg caaaagcttt gaaaaagaaa 720
aagaagatgc caaagcttcg ctttgccagt agaatccgga aaattcggaa gaagcaattt 780
taaacattct taatatgtca tcttgtttgt ctttatggct tatttgcctt tatggttgta 840
tctgaagaat aatatgacag catgaggaaa caaatcattg gtgatttatt caccagtttt 900
tattaataca agtcactttt tcaaaaattt ggattttttt atatataact agctgctatt 960
caaatgtgag tctaccattt ttaatttatg gttcaactgt ttgtgagact gaattcttgc 1020
aatgcataag atataaaagc aaatatgact cactcatttc ttggggtcgt attcctgatt 1080
tcagaagagg atcataactg aaacaacata agacaatata atcatgtgct tttaacatat 1140
ttgagaataa aaaggactag caaataaaac acaaaaaaaa aaaaaaaaaa aaaaaaaaaa 1200
aaa                                                               1203
```

<210> 92
<211> 4264
<212> DNA
<213> Homo sapiens

<400> 92

agccgccttc ctatttccgc ccggcgggca gcgctgcggg gcgagtgcca gcagagaggc 60
gctcggtcct ccctccgccc tcccgcgccg ggggcaggcc ctgcctagtc tgcgtctttt 120
tcccccgcac cgcggcgccg ctccgccact cgggcaccgc aggtagggca ggaggctgga 180
gagcctgctg cccgcccgcc cgtaaaatgg tcccctcggc tggacagctc gccctgttcg 240
ctctgggtat tgtgttggct gcgtgccagg ccttggagaa cagcacgtcc ccgctgagtg 300
cagacccgcc cgtggctgca gcagtggtgt cccattttaa tgactgccca gattcccaca 360
ctcagttctg cttccatgga acctgcaggt tttggtgca ggaggacaag ccagcatgtg 420
tctgccattc tgggtacgtt ggtgcacgct gtgagcatgc ggacctcctg gccgtggtgg 480
ctgccagcca gaagaagcag gccatcaccg ccttggtggt ggtctccatc gtggccctgg 540
ctgtccttat catcacatgt gtgctgatac actgctgcca ggtccgaaaa cactgtgagt 600
ggtgccgggc cctcatctgc cggcacgaga agcccagcgc cctcctgaag ggaagaaccg 660
cttgctgcca ctcagaaaca gtggtctgaa gagcccagag gaggagtttg gccaggtgga 720
ctgtggcaga tcaataaaga aaggcttctt caggacagca ctgccagaga tgcctgggtg 780
tgccacagac cttcctactt ggcctgtaat cacctgtgca gccttttgtg ggccttcaaa 840
actctgtcaa gaactccgtc tgcttggggt tattcagtgt gacctagaga agaaatcagc 900
ggaccacgat ttcaagactt gttaaaaaag aactgcaaag agacggactc ctgttcacct 960
aggtgaggtg tgtgcagcag ttggtgtctg agtccacatg tgtgcagttg tcttctgcca 1020
gccatggatt ccaggctata tatttctttt taatgggcca cctcccccaca acagaattct 1080
gcccaacaca ggagatttct atagttattg ttttctgtca tttgcctact ggggaagaaa 1140
gtgaaggagg ggaaactgtt taatatcaca tgaagaccct agctttaaga gaagctgtat 1200
cctctaacca cgagaccctc aaccagccca acatcttcca tggacacatg acattgaaga 1260
ccatcccaag ctatcgccac ccttggagat gatgtcttat ttattagatg gataatggtt 1320
ttatttttaa tctcttaagt caatgtaaaa agtataaaac cccttcagac ttctacatta 1380
atgatgtatg tgttgctgac tgaaaagcta tactgattag aaatgtctgg cctcttcaag 1440
acagctaagg cttgggaaaa gtcttccagg gtgcggagat ggaaccagag gctgggttac 1500
tggtaggaat aaaggtaggg gttcagaaat ggtgccattg aagccacaaa gccggtaaat 1560
gcctcaatac gttctgggag aaaacttagc aaatccatca gcagggatct gtcccctctg 1620
ttggggagag aggaagagtg tgtgtgtcta cacaggataa acccaataca tattgtactg 1680
ctcagtgatt aaatgggttc acttcctcgt gagccctcgg taagtatgtt tagaaataga 1740
acattagcca cgagccatag gcatttcagg ccaaatccat gaaagggggga ccagtcattt 1800
attttccatt ttgttgcttg gttggtttgt tgctttattt ttaaaaggag aagtttaact 1860
ttgctattta ttttcgagca ctaggaaaac tattccagta attttttttt cctcatttcc 1920
attcaggatg ccggctttat taacaaaaac tctaacaagt cacctccact atgtgggtct 1980
tcctttcccc tcaagagaag gagcaattgt tcccctgagc atctgggtcc atctgaccca 2040
tggggcctgc ctgtgagaaa cagtgggtcc cttcaaatac atagtggata gctcatccct 2100
aggaattttc attaaaattt ggaaacagag taatgaagaa ataatatata aactccttat 2160
gtgaggaaat gctactaata tctgaaaagt gaaagatttc tatgtattaa ctcttaagtg 2220
cacctagctt attacatcgt gaaaggtaca tttaaaatat gttaaattgg cttgaaattt 2280
tcagagaatt ttgtcttccc ctaattcttc ttccttggtc tggaagaaca atttctatga 2340
attttctctt tatttttttt tataattcag acaattctat gacccgtgtc ttcatttttg 2400
gcactcttat ttaacaatgc cacacctgaa gcacttggat ctgttcagag ctgaccccct 2460
agcaacgtag ttgacacagc tccaggtttt taaattacta aaataagttc aagtttacat 2520
cccttgggcc agatatgtgg gttgaggctt gactgtagca tcctgcttag agaccaatca 2580
acggacactg gtttttagac ctctatcaat cagtagttag catccaagag actttgcaga 2640
ggcgtaggaa tgaggctgga cagatggcgg aagcagaggt tccctgcgaa gacttgagat 2700
ttagtgtctg tgaatgttct agttcctagg tccagcaagt cacacctgcc agtgccctca 2760
tccttatgcc tgtaacacac atgcagtgag aggcctcaca tatacgcctc cctagaagtg 2820
ccttccaagt cagtcctttg gaaaccagca ggtctgaaaa agaggctgca tcaatgcaag 2880
cctggttgga ccattgtcca tgcctcagga tagaacagcc tggcttattt ggggattttt 2940
cttctagaaa tcaaatgact gataagcatt ggatccctct gccatttaat ggcaatggta 3000
gtctttggtt agctgcaaaa atactccatt tcaagttaaa aatgcatctt ctaatccatc 3060
tctgcaagct ccctgtgttt ccttgccctt tagaaaatga attgttcact acaattagag 3120
aatcatttaa catcctgacc tggtaagctg ccacacacct ggcagtgggg agcatcgctg 3180
tttccaatgg ctcaggagac aatgaaaagc cccccatttaa aaaaataaca aacatttttt 3240
aaaaggcctc caatactctt atggagcctg gatttttccc actgctctac aggctgtgac 3300
tttttttaag catcctgaca ggaaatgttt tcttctacat ggaaagatag acagcagcca 3360
accctgatct ggaagacagg gccccggctg gacacacgtg gaaccaagcc agggatgggc 3420
tggccattgt gtccccgcag gagagatggg cagaatggcc ctagagttct tttccctgag 3480

```
aaaggagaaa aagatgggat tgccactcac ccacccacac tggtaaggga ggagaatttg 3540
tgcttctgga gcttctcaag ggattgtgtt ttgcaggtac agaaaactgc ctgttatctt 3600
caagccaggt tttcgagggc acatgggtca ccagttgctt tttcagtcaa tttggccggg 3660
atggactaat gaggctctaa cactgctcag gagacccctg ccctctagtt ggttctgggc 3720
tttgatctct tccaacctgc ccagtcacag aaggaggaat gactcaaatg cccaaaacca 3780
agaacacatt gcagaagtaa gacaaacatg tatattttta aatgttctaa cataagacct 3840
gttctctcta gccattgatt taccaggctt tctgaaagat ctagtggttc acacagagag 3900
agagagagta ctgaaaaagc aactcctctt cttagtctta ataatttact aaaatggtca 3960
acttttcatt atctttatta taataaacct gatgcttttt tttagaactc cttactctga 4020
tgtctgtata tgttgcactg aaaaggttaa tatttaatgt tttaatttat tttgtgtggt 4080
aagttaattt tgatttctgt aatgtgttaa tgtgattagc agttattttc cttaatatct 4140
gaattatact taaagagtag tgagcaatat aagacgcaat tgtgtttttc agtaatgtgc 4200
attgttattg agttgtactg taccttattt ggaaggatga aggaatgaat ctttttttcc 4260
taaa                                                              4264
```

<210> 93
<211> 931
<212> DNA
<213> Homo sapiens

<400> 93

```
tttcgtcggc ccgccccttg gcttctgcac tgatggtggg tggatgagta atgcatccag 60
gaagcctgga ggcctgtggt ttccgcaccc gctgccaccc ccgcccctag cgtggacatt 120
tatcctctag cgctcaggcc ctgccgccat cgccgcagat ccagcgccca gagagacacc 180
agagaaccca ccatggcccc ctttgagccc ctggcttctg gcatcctgtt gttgctgtgg 240
ctgatagccc ccagcagggc ctgcacctgt gtcccacccc acccacagac ggccttctgc 300
aattccgacc tcgtcatcag ggccaagttc gtggggacac cagaagtcaa ccagaccacc 360
ttataccagc gttatgagat caagatgacc aagatgtata aagggttcca agccttaggg 420
gatgccgctg acatccggtt cgtctacacc cccgccatgg agagtgtctg cggatacttc 480
cacaggtccc acaaccgcag cgaggagttt ctcattgctg gaaaactgca ggatggactc 540
ttgcacatca ctacctgcag ttttgtggct ccctggaaca gcctgagctt agctcagcgc 600
cggggcttca ccaagaccta cactgttggc tgtgaggaat gcacagtgtt tccctgttta 660
tccatcccct gcaaactgca gagtggcact cattgcttgt ggacggacca gctcctccaa 720
ggctctgaaa agggcttcca gtcccgtcac cttgcctgcc tgcctcggga gccagggctg 780
tgcacctggc agtccctgcg gtcccagata gcctgaatcc tgcccggagt ggaagctgaa 840
gcctgcacag tgtccaccct gttcccactc ccatctttct tccggacaat gaaataaaga 900
gttaccaccc agcagaaaaa aaaaaaaaaa a                                931
```

<210> 94
<211> 2207
<212> DNA
<213> Homo sapiens

<400> 94

```
acgcacttgg cgcgcggcgc gggctgcaga cggctgcgag gcgctgggca caggtgtcct 60
gatggcaaat ttcaagggcc acgcgcttcc agggagtttc ttcctgatca ttgggctgtg 120
ttggtcagtg aagtacccgc tgaagtactt tagccacacg cggaagaaca gcccactaca 180
ttactatcag cgtctcgaga tcgtcgaagc cgcaattagg actttgtttt ccgtcactgg 240
gatcctggca gagcagtttg ttccggatgg gccccacctg cacctctacc atgagaacca 300
ctggataaag ttaatgaatt ggcagcacag caccatgtac ctattctttg cagtctcagg 360
aattgttgac atgctcacct atctggtcag ccacgttccc ttgggggtgg acagactggt 420
tatggctgtg gcagtattca tggaaggttt cctcttctac taccacgtcc acaaccggcc 480
tccgctggac cagcacatcc actcactcct gctgtatgct ctgttcggag ggtgtgttag 540
tatctcccta gaggtgatct tccgggacca cattgtgctg gaacttttcc gaaccagtct 600
catcattctt cagggaacct ggttctggca gattgggttt gtgctgttcc caccttttgg 660
aacacccgaa tgggaccaga aggatgatgc caacctcatg ttcatcacca tgtgcttctg 720
ctggcactac ctggctgccc tcagcattgt ggccgtcaac tattctcttg tttactgcct 780
tttgactcgg atgaagagac acggaagggg agaaatcatt ggaattcaga agctgaattc 840
agatgacact taccagaccg ccctcttgag tggctcagat gaggaatgag ccgagatgcg 900
gagggcgcag atgtcccact gcacagctgg aatgaatgga gttcatcccc tccacctgaa 960
tgcctgctgt ggtctgatct taagggtcta tatatttgca cctcctcatt caacacaggg 1020
ctggaggttc tacaacagga aatcaggcct acagcatcct gtgtatcttg cagttgggat 1080
ttttaaacat actataaagt ctgtgttggt atagtaccct tcataaggaa aaatgaagta 1140
atgcctataa gtagcaggcc tttgtgcctc agtgtcaaga gaaatcaaga gatgctaaaa 1200
gctttacaat ggaagtggcc tcatggatga atccggggta tgagcccagg agaacgtgct 1260

gcttttggta acttatccct ttttctctta agaaagcagg tactttctta ttagaaatat 1320
gttagaatgt gtaagcaaac gacagtgcct ttagaattac aattctaact tacatatttt 1380
ttgaaagtaa aataattcac aagctttggt attttaaaat tattgttaaa catatcataa 1440
ctaatcatac cagggtactg caataccact gtttataagt gacaaaatta ggccaaaggt 1500
gattttttt taaatcagga agctggttac tggctctact gagagttgga gccctgatgt 1560
tctgattctt caaagtcacc ctaaaagaag atctgacagg aaagctgtat aatgagatag 1620
aaaaacgtca ggtatggaag gctttcagtt ttaatatggc tgaaagcaaa ggataacgaa 1680
ttcagaatta gtaatgtaaa atcttgatac cctaatcttg cttctggatc tgttcttttt 1740
ttaaaaaaac ttccttcacc gcgcctataa tcctagcact ttgggaggcc gaggcaggca 1800
gatcacgggg tcaggagatc aagaccatcc tggctaacat ggtgaaaccc cgtctctact 1860
gaaaatacaa aaaattagcc gggtgtggtg gcgggcgcct gtagttccag ctactcggga 1920
ggctgaggca agagaatggc atgaacccgg tagggggagct tgcagtgagc ccagatcatg 1980
ccactgtact ccagcctagg tgacagagca agactctgtc tcaaaaacaa gcaaacagac 2040
ttccttcaac aaatatttat taaatatcca ctttgcaaca gcactgaaat ggctgtaagg 2100
actcctgaga tatgtgtcca gcaaggagtt tacagtcaaa caggagagac atgcctgtag 2160
ttacatccag tgtgatgggt gctgagaggc aagtacaaac cacgatg                2207
```

<210> 95
<211> 1723
<212> DNA
<213> Homo sapiens

<400> 95

actccgaatg cgaagttctg tcttgtcata gccaagcacg ctgcttcttg gattgacctg 60
gcaggatggc gccaccacca gctagagtac atctaggtgc gttcctggca gtgactccga 120
atcccgggag cgcagcgagt gggacagagg cagccgcggc cacacccagc aaagtgtggg 180
gctcttccgc ggggaggatt gaaccacgag gcgggggccg aggagcgctc cctacctcca 240
tgggacagca cggacccagt gcccgggccc gggcagggcg cgccccagga cccaggccgg 300
cgcgggaagc cagccctcgg ctccgggtcc acaagacctt caagtttgtc gtcgtcgggg 360
tcctgctgca ggtcgtacct agctcagctg caaccatcaa acttcatgat caatcaattg 420
gcacacagca atgggaacat agccctttgg gagagttgtg tccaccagga tctcatagat 480
cagaacatcc tggagcctgt aaccggtgca cagagggtgt gggttacacc aatgcttcca 540
acaatttgtt tgcttgcctc ccatgtacag cttgtaaatc agatgaagaa gagagaagtc 600
cctgcaccac gaccaggaac acagcatgtc agtgcaaacc aggaactttc cggaatgaca 660
attctgctga gatgtgccgg aagtgcagca gagggtgccc cagagggatg gtcaaggtca 720
aggattgtac gccctggagt gacatcgagt gtgtccacaa agaatcaggc aatggacata 780
atatatgggt gattttggtt gtgactttgg ttgttccgtt gctgttggtg gctgtgctga 840
ttgtctgttg ttgcatcggc tcaggttgtg gaggggaccc caagtgcatg gacagggtgt 900
gtttctggcg cttgggtctc ctacgagggc ctggggctga ggacaatgct cacaacgaga 960
ttctgagcaa cgcagactcg ctgtccactt tcgtctctga gcagcaaatg gaaagccagg 1020
agccggcaga tttgacaggt gtcactgtac agtccccagg ggaggcacag tgtctgctgg 1080
gaccggcaga agctgaaggg tctcagagga ggaggctgct ggttccagca aatggtgctg 1140
accccactga gactctgatg ctgttctttg acaagtttgc aaacatcgtg ccctttgact 1200
cctgggacca gctcatgagg cagctggacc tcacgaaaaa tgagatcgat gtggtcagag 1260
ctggtacagc aggcccaggg gatgccttgt atgcaatgct gatgaaatgg gtcaacaaaa 1320
ctggacggaa cgcctcgatc cacaccctgc tggatgcctt ggagaggatg gaagagagac 1380
atgcaaaaga gaagattcag gacctcttgg tggactctgg aaagttcatc tacttagaag 1440
atggcacagg ctctgccgtg tccttggagt gaaagactct ttttaccaga ggtttcctct 1500
taggtgttag gagttaatac atattaggtt tttttttttt ttaacatgta tacaaagtaa 1560
attcttagcc aggtgtagtg gctcatgcct gtaatcccag cactttggga ggctgaggcg 1620
ggtggatcac ttgaggtcag aagttcaaga ccagcctgac caacatcgtg aaatgccgtc 1680
tttacaaaaa aatacaaaaa ttaactggaa aaaaaaaaaa aaa 1723

<210> 96
<211> 4160
<212> DNA
<213> Homo sapiens

<400> 96

gcgcttgcgg aggattgcgt tgacgagact cttatttatt gtcaccaacc tgtggtggaa 60
tttgcagttg cacattggat ctgattcgcc ccgccccgaa tgacgcctgc ccggaggcag 120
tgaaagtaca gccgcgccgc cccaagtcag cctggacaca taaatcagca cgcggccgga 180
gaaccccgca atctctgcgc ccacaaaata caccgacgat gcccgatcta ctttaagggc 240
tgaaacccac gggcctgaga gactataaga gcgttcccta ccgccatgga acaacgggga 300
cagaacgccc cggccgcttc gggggcccgg aaaaggcacg gcccaggacc cagggaggcg 360

```
cggggagcca ggcctgggct ccgggtcccc aagacccttg tgctcgttgt cgccgcggtc 420
ctgctgttgg tctcagctga gtctgctctg atcacccaac aagacctagc tccccagcag 480
agagcggccc cacaacaaaa gaggtccagc cctcagagg gattgtgtcc acctggacac 540
catatctcag aagacggtag agattgcatc tcctgcaaat atggacagga ctatagcact 600
cactggaatg acctcctttt ctgcttgcgc tgcaccaggt gtgattcagg tgaagtggag 660
ctaagtccct gcaccacgac cagaaacaca gtgtgtcagt gcgaagaagg caccttccgg 720
gaagaagatt ctcctgagat gtgccggaag tgccgcacag ggtgtcccag agggatggtc 780
aaggtcggtg attgtacacc ctggagtgac atcgaatgtg tccacaaaga atcaggtaca 840
aagcacagtg gggaagcccc agctgtggag gagacggtga cctccagccc agggactcct 900
gcctctccct gttctctctc aggcatcatc ataggagtca cagttgcagc cgtagtcttg 960
attgtggctg tgtttgtttg caagtcttta ctgtggaaga aagtccttcc ttacctgaaa 1020
ggcatctgct caggtggtgg tggggaccct gagcgtgtgg acagaagctc acaacgacct 1080
ggggctgagg acaatgtcct caatgagatc gtgagtatct tgcagcccac ccaggtccct 1140
gagcaggaaa tggaagtcca ggagccagca gagccaacag gtgtcaacat gttgtccccc 1200
ggggagtcag agcatctgct ggaaccggca gaagctgaaa ggtctcagag gaggaggctg 1260
ctggttccag caaatgaagg tgatcccact gagactctga gacagtgctt cgatgacttt 1320
gcagacttgg tgccctttga ctcctgggag ccgctcatga ggaagttggg cctcatggac 1380
aatgagataa aggtggctaa agctgaggca gcgggccaca gggacacctt gtacacgatg 1440
ctgataaagt gggtcaacaa aaccgggcga gatgcctctg tccacaccct gctggatgcc 1500
ttggagacgc tgggagagag acttgccaag cagaagattg aggaccactt gttgagctct 1560
ggaaagttca tgtatctaga aggtaatgca gactctgcca tgtcctaagt gtgattctct 1620
tcaggaagtc agaccttccc tggtttacct tttttctgga aaaagcccaa ctggactcca 1680
gtcagtagga aagtgccaca attgtcacat gaccggtact ggaagaaact ctcccatcca 1740
acatcaccca gtggatggaa catcctgtaa cttttcactg cacttggcat tatttttata 1800
agctgaatgt gataataagg acactatgga aatgtctgga tcattccgtt tgtgcgtact 1860
ttgagatttg gtttgggatg tcattgtttt cacagcactt ttttatccta atgtaaatgc 1920
tttatttatt tatttgggct acattgtaag atccatctac acagtcgttg tccgacttca 1980
cttgatacta tatgatatga accttttttg ggtggggggt gcggggcagt tcactctgtc 2040
tcccaggctg gagtgcaatg gtgcaatctt ggctcactat agccttgacc tctcaggctc 2100
aagcgattct cccacctcag ccatccaaat agctgggacc acaggtgtgc accaccacgc 2160
ccggctaatt ttttgtattt tgtctagata taggggctct ctatgttgct cagggtggtc 2220
tcgaattcct ggactcaagc agtctgccca cctcagactc ccaaagcggt ggaattagag 2280
gcgtgagccc ccatgcttgg ccttaccttt ctacttttat aattctgtat gttattattt 2340
tatgaacatg aagaaacttt agtaaatgta cttgtttaca tagttatgtg aatagattag 2400
ataaacataa aaggaggaga catacaatgg gggaagaaga agaagtcccc tgtaagatgt 2460
cactgtctgg gttccagccc tccctcagat gtactttggc ttcaatgatt ggcaacttct 2520
acaggggcca gtcttttgaa ctggacaacc ttacaagtat atgagtatta tttataggta 2580
gttgtttaca tatgagtcgg gaccaaagag aactggatcc acgtgaagtc ctgtgtgtgg 2640
ctggtcccta cctgggcagt ctcatttgca cccatagccc ccatctatgg acaggctggg 2700
acagaggcag atgggttaga tcacacataa caatagggtc tatgtcatat cccaagtgaa 2760
cttgagccct gtttgggctc aggagataga agacaaaatc tgtctcccac gtctgccatg 2820
gcatcaaggg ggaagagtag atggtgcttg agaatggtgt gaaatggttg ccatctcagg 2880
agtagatggc ccggctcact tctggttatc tgtcaccctg agcccatgag ctgcctttta 2940
gggtacagat tgcctacttg aggaccttgg ccgctctgta agcatctgac tcatctcaga 3000
aatgtcaatt cttaaacact gtggcaacag gacctagaat ggctgacgca ttaaggtttt 3060
cttcttgtgt cctgttctat tattgtttta agacctcagt aaccatttca gcctctttcc 3120
agcaaaccct tctccatagt atttcagtca tggaaggatc atttatgcag gtagtcattc 3180
caggagtttt tggtcttttc tgtctcaagg cattgtgtgt tttgttccgg gactggtttg 3240
ggtgggacaa agttagaatt gcctgaagat cacacattca gactgttgtg tctgtggagt 3300
tttaggagtg gggggtgacc tttctggtct ttgcacttcc atcctctccc acttccatct 3360
ggcatcccac gcgttgtccc ctgcacttct ggaaggcaca gggtgctgct gcctcctggt 3420
ctttgccttt gctgggcctt ctgtgcagga cgctcagcct cagggctcag aaggtgccag 3480
tccggtccca ggtcccttgt cccttccaca gaggccttcc tagaagatgc atctagagtg 3540
tcagccttat cagtgtttaa gatttgtctt ttatttttaa ttttttttgag acagaatctc 3600
actctctcgc ccaggctgga gtgcaacggt acgatcttgg ctcagtgcaa cctccgcctc 3660
ctgggttcaa gcgattctcg tgcctcagcc tccggagtag ctgggattgc aggcacccgc 3720
caccacgcct ggctaatttt tgtattttta gtagagacgg ggtttcacca tgttggtcag 3780
gctggtctcg aactcctgac ctcaggtgat ccaccttggc ctccgaaagt gctgggatta 3840
caggcgtgag ccaccagcca ggccaagcta ttcttttaaa gtaagcttcc tgacgacatg 3900
aaataattgg gggttttgtt gtttagttac attaggcttt gctatatccc caggccaaat 3960
agcatgtgac acaggacagc catagtatag tgtgtcactc gtggttggtg tcctttcatg 4020
cttctgccct gtcaaaggtc cctatttgaa atgtgttata atacaaacaa ggaagcacat 4080
tgtgtacaaa atacttatgt atttatgaat ccatgaccaa attaaatatg aaaccttata 4140
taaaaaaaaa aaaaaaaaaa                                            4160
```

<210> 97
<211> 998
<212> DNA
<213> Homo sapiens

<400> 97

gcggcgggcg cagacagcgg cgggcgcagg acgtgcacta tggctcgggg ctcgctgcgc 60
cggttgctgc ggctcctcgt gctggggctc tggctggcgt tgctgcgctc cgtggccggg 120
gagcaagcgc caggcaccgc cccctgctcc cgcggcagct cctggagcgc ggacctggac 180
aagtgcatgg actgcgcgtc ttgcagggcg cgaccgcaca gcgacttctg cctgggctgc 240
gctgcagcac ctcctgcccc cttccggctg ctttggccca tccttggggg cgctctgagc 300
ctgaccttcg tgctggggct gctttctggc tttttggtct ggagacgatg ccgcaggaga 360
gagaagttca ccaccccccat agaggagacc ggcggagagg gctgcccagc tgtggcgctg 420
atccagtgac aatgtgcccc ctgccagccg gggctcgccc actcatcatt cattcatcca 480
ttctagagcc agtctctgcc tcccagacgc ggcgggagcc aagctcctcc aaccacaagg 540
ggggtggggg gcggtgaatc acctctgagg cctgggccca gggttcaggg gaaccttcca 600
aggtgtctgg ttgccctgcc tctggctcca gaacagaaag ggagcctcac gctggctcac 660
acaaaacagc tgacactgac taaggaactg cagcatttgc acaggggagg ggggtgccct 720
ccttccttag gacctggggg ccaggctgac ttgggggggca gacttgacac taggccccac 780
tcactcagat gtcctgaaat tccaccacgg gggtcaccct gggggggttag ggacctattt 840
ttaacactag gggctggccc actaggaggg ctggccctaa gatacagacc cccccaactc 900
cccaaagcgg ggaggagata tttattttgg ggagagtttg gaggggaggg agaatttatt 960
aataaaagaa tctttaactt taaaaaaaaa aaaaaaaa 998

<210> 98
<211> 4090
<212> DNA
<213> Homo sapiens

<400> 98

```
ggactctgag tcgtcttggt cccaggagcc agtagtgaag gcaacagtct gcccacctgt 60
ggacaccaga tcctgggagc tcctggttag caagtgagat ctctgggatg tcagtgaggc 120
tggttgaaga ccagaggtaa actgcagagg tcaccacccc caccatgtcc caggtgatgt 180
ccagcccact gctggcagga ggccatgctg tcagcttggc gccttgtgat gagcccagga 240
ggaccctgca cccagcaccc agccccagcc tgccacccca gtgttcttac tacaccacgg 300
aaggctgggg agcccaggcc ctgatggccc ccgtgccctg catggggccc cctggccgac 360
tccagcaagc cccacaggtg gaggccaaag ccacctgctt cctgccgtcc cctggtgaga 420
aggccttggg gaccccagag gaccttgact cctacattga cttctcactg gagagcctca 480
atcagatgat cctggaactg gaccccacct tccagctgct tcccccaggg actgggggct 540
cccaggctga gctggcccag agcaccatgt caatgagaaa gaaggaggaa tctgaagcct 600
tggacataaa gtacatcgag gtgacctccg ccagatcaag gtgccacgat ggcccccagc 660
actgctccag cccctctgtc accccgccct tcggctccct tcgcagtggt ggcctcctcc 720
tttccagaga cgtcccccga gagacacgaa gcagcagtga gagcctcatc ttctctggga 780
accagggcag ggggcaccag cgccctctgc ccccctcaga gggtctctcc cctcgacccc 840
caaattcccc cagcatctca atcccttgca tggggagcaa ggcctcgagc cccccatggtt 900
tgggctcccc gctggtggct tctccaagac tggagaagcg gctgggaggc ctggccccac 960
agcggggcag caggatctct gtgctgtcag ccagcccagt gtctgatgtc agctatatgt 1020
ttggaagcag ccagtccctc ctgcactcca gcaactccag ccatcagtca tcttccagat 1080
ccttggaaag tccagccaac tcttcctcca gcctccacag ccttggctca gtgtccctgt 1140
gtacaagacc cagtgacttc caggctccca gaaaccccac cctaaccatg ggccaaccca 1200
gaacacccca ctctccacca ctggccaaag aacatgccag cagctgcccc ccatccatca 1260
ccaactccat ggtggacata cccattgtgc tgatcaacgg ctgcccagaa ccagggtctt 1320
ctccacccca gcggaccca ggacaccaga actccgttca acctggagct gcttctccca 1380
gcaacccctg tccagccacc aggagcaaca gccagaccct gtcagatgcc cccttacca 1440
catgcccaga gggtcccgcc agggacatgc agcccaccat gaagttcgtg atggacacat 1500
ctaaatactg gtttaagcca aacatcaccc gagagcaagc aatcgagctg ctgaggaagg 1560
aggagccagg ggcttttgtc ataagggaca gctcttcata ccgaggctcc ttcggcctgg 1620
ccctgaaggt gcaggaggtt cccgcgtctg ctcagagtcg accaggtgag gacagcaatg 1680
acctcatccg acacttcctc atcgagtcgt ctgccaaagg agtgcatctc aaaggagcag 1740
atgaggagcc ctactttggg agcctctctg ccttcgtgtg ccagcattcc atcatggccc 1800
tggccctgcc ctgcaaactc accatcccac agagagaact gggaggtgca gatggggcct 1860
cggactctac agacagccca gcctcctgcc agaagaaatc tgcgggctgc cacaccctgt 1920
acctgagctc agtgagcgtg gagaccctga ctggagccct ggccgtgcag aaagccatct 1980
ccaccacctt tgagagggac atcctcccca cgcccaccgt ggtccacttc aaagtcacag 2040
agcagggcat cactctgact gatgtccaga ggaaggtgtt tttccggcgc cattacccac 2100
```

```
tcaccaccct ccgcttctgt ggtatggacc ctgagcaacg gaagtggcag aagtactgca 2160
aaccctcctg gatctttggg tttgtggcca agagccagac agagcctcag gagaacgtat 2220
gccacctctt tgcggagtat gacatggtcc agccagcctc gcaggtcatc ggcctggtga 2280
ctgctctgct gcaggacgca gaaaggatgt aggggagaga ctgcctgtgc acctaaccaa 2340
cacctccagg ggctcgctaa ggagcccccc tccacccccct gaatgggtgt ggcttgtggc 2400
catattgaca gaccaatcta tgggactagg gggattggca tcaagttgac acccttgaac 2460
ctgctatggc cttcagcagt caccatcatc cagacccccc gggcctcagt ttcctcaatc 2520
atagaagaag accaatagac aagatcagct gttcttagat gctggtgggc atttgaacat 2580
gctcctccat gattctgaag catgcacacc tctgaagacc cctgcatgaa aataacctcc 2640
aaggaccctc tgaccccatc gacctgggcc ctgcccacac aacagtctga gcaagagacc 2700
tgcagcccct gtttcgtggc agacagcagg tgcctggcgg tgacccacgg ggctcctggc 2760
ttgcagctgg tgatggtcaa gaactgacta caaaacagga atggatagac tctatttcct 2820
tccatatctg ttcctctgtt ccttttccca ctttctgggt ggctttttgg gtccacccag 2880
ccaggatgct gcaggccaag ctgggtgtgg tatttagggc agctcagcag gggggaacttg 2940
tccccatggt cagaggagac ccagctgtcc tgcacccccct tgcagatgag tatcacccca 3000
tcttttcttt ccacttggtt tttattttta tttttttttg agacagagtc tcactgtcac 3060
ccaggctgaa ctgcagtggt gtgatctagg ctcactgcaa cctccacctc ccaggttcaa 3120
gcaattatcc tgcctcaggc tcccaagtag ctgggattac aggcatgtgc aactcaccca 3180
gctaattttg tatttttagt agagacaggg tttcaccatg ttggccaggc tggtcttgaa 3240
ctcctgaccg caggtaatcc acctgcttcg gcctcccaaa gtgctgggat tacaggcgca 3300
agccacccag cccagcttct ttccattcct tgataggcga gtattccaaa gctggtatcg 3360
tagctgccct aatgttgcat attaggcggc gggggcagag ataagggcca tctctctgtg 3420
attctgcctc agctcctgtc ttgctgagcc ctccccccaac ccacgctcca acacacacac 3480
acacacacac acacacacac acacacacac acacacacac acacacacac gcccctctac 3540
tgctatgtgg cttcaaccag cctcacagcc acacgggggga agcagagagt caagaatgca 3600
aagaggccgc ttccctaaga ggcttggagg agctgggctc tatcccacac ccaccccccac 3660
cccaccccca cccagcctcc agaagctgga accatttctc ccgcaggcct gagttcctaa 3720
ggaaaccacc ctaccggggt ggaagggagg gtcagggaag aaacccactc ttgctctacg 3780
aggagcaagt gcctgccccc tcccagcagc cagccctgcc aaagttgcat tatctttggc 3840
caaggctggg cctgacggtt atgatttcag ccctgggcct gcaggagagg ctgagaccag 3900
cccacccagc cagtggtcga gcactgcccc gccgccaaag tctgcagaat gtgagatgag 3960
gttctcaagg tcacaggccc cagtcccagc ctgggggctg gcagaggccc ccatatactc 4020
tgctacagct cctatcatga aaaataaaat gtttgtcttt gcaaaacagt aaaaaaaaaa 4080
aaaaaaaaaa                                                        4090
```

<210> 99
<211> 3824
<212> DNA
<213> Homo sapiens

<400> 99

```
actgcctctc tcacagccct caagacacac catgggccca gaggcaggtt tgctacacag 60
cagcgacgac gcaggcggcg gccccagcga ctcgcaactg cctccctgac cacagcggcc 120
accgcccaac acccccgaga agccatcgcc accaccggca ggagaaccta gggtccataa 180
agccatcttc gcgatcgact aaagctacgt caacaactat ggcgggcgac gggcggcggg 240
cagaggcggt gcgggaagga tggggtgtgt acgtcacccc cagggccccc atccgagagg 300
gaaggggccg gctcgcccct caaaatggcg gcagcagcga tgcgcctgcg tacagaactc 360
ctccgtcgcg ccagggccgg cgggaagtga ggttctcgga cgagccgcca gaagtgtacg 420
gcgacttcga gcccctggtg gccaaagaaa ggtccccggt gggaaaacga acccggctag 480
aagagttccg gtccgattct gcgaaagagg aagtgagaga aagcgcgtac taccttcggt 540
ctaggcagcg gaggcagccg cgaccccagg aaaccgagga aatgaagacg cgaaggacta 600
cccgccttca gcagcagcac tcagagcagc ctccgctaca gccgtctcct gttatgacca 660
ggagagggct gcgggactct cattcctctg aagaggatga agcatcttcc caaactgatt 720
taagccaaac gatctcaaag aaaactgtca ggagcataca agaggctcca gtgagtgaag 780
atcttgtaat caggttacgt cgacccccctc taagataccc aagatatgaa gccaccagtg 840
tccaacagaa ggtcaatttc tctgaagaag gagaaactga agaagatgat caagacagct 900
ctcacagcag tgtcactact gttaaggcca gatccaggga ttctgatgaa tctggagata 960
aaaccaccag atcatctagt caatatatag aatcattttg gcagtcatca caaagtcaaa 1020
acttcacagc tcatgataag caaccttcag tgctaagctc aggatatcaa aaaactcccc 1080
aggaatgggc cccacaaact gcaagaataa ggaccaggat gcaaaatgac agcattctga 1140
aatcagagct tggaaaccag tcaccatcaa cctccagccg acaagtgact ggacaacccc 1200
aaaatgcatc ttttgtcaag aggaaccggt ggtggctact tcctctgata gctgctcttg 1260
cctctgggag tttttggttc tttagtactc ctgaggtaga aaccactgct gttcaagagt 1320
tccagaacca gatgaatcaa cttaagaata agtaccaagg tcaagatgag aagctgtgga 1380
aaaggagcca aacattcctg gaaaaacatc ttaatagctc ccatcctcgg tctcagcctg 1440
```

```
ctatcttact gctcactgct gcccgagatg ctgaagaagc acttaggtgt ctgagtgaac 1500
aaattgctga tgcctattct tcttttcgta gtgtccgtgc catccggatt gatgggacag 1560
ataaagctac tcaagacagt gatactgtca aactagaggt agaccaagaa ctgagcaatg 1620
gatttaagaa tggccagaat gcagctgtgg tacaccgctt tgagtcattt cccgcaggct 1680
ctactttgat cttctacaaa tattgtgacc atgaaaacgc ggccttcaaa gatgtagcct 1740
tagtcctgac tgtcttattg gaggaagaga cacttggaac aagtctaggc ctaaaggaag 1800
ttgaagaaaa agtaagagat tttcttaaag tcaagttcac caattctaac acacccaact 1860
cctacaatca tatggaccca gacaaactga atggcctctg gagccgtatt tctcacttag 1920
ttctgcctgt gcaacctgaa aatgccctga aaaggggcat ctgcttataa gaagtgagag 1980
agaagaaaaa tcatgtccca agttctgaga attgttcaca ctttctaacc agagacagaa 2040
ttcagagctc tttttgaaag aactagtttc tttttaaaga agttaagtgc ttacataaac 2100
atggaacata taaatcattc attcaaccta attatctgtg atatgagaga atcatttcag 2160
tttccattga gagctctgtt aaaggtatct taggagtgca gattatatgc agttccttag 2220
agaatctgtt ttgattctgg gctgagttat tacagttatg gtatgaccag tgagagggat 2280
ttgtgtcctt tcttatgaac cttcctgatt ttttaactta gatttctcac taagtttcct 2340
gagttattag taagattgct ccctaaatgt aaccagggat tttcccatta tgttcccttt 2400
tataccattt aggtgtgagt tccttagctt ctgcctatag gaacataagt aatgaaaggt 2460
catctaggtg tgtgtttgga atttttttct tttgtttttt ggaaaatgga aagaacaagg 2520
caaggaagga aaattaatgg ggaagctgaa gggaggaaat gttacagtaa tccactgaga 2580
tgtagtttag tcaaacatag gtatatgaac tgttaatctt ggtggattcc gttgggattt 2640
gtttttttaca tctccttttt ccttccttga agaaaaattc ttggctgtcc caaggattct 2700
tcatgtatat tgcaaaattt aatatatttg ttcacttgag tcttaagagt aggtcaggcc 2760
aaggcaggtg gatcacctga ggtcagaagt tcaagaccag cctggccaac atggtgaaac 2820
cccgtctcta ctaaaaatac aaaaattagc tgggcgtggt ggtgcatgcc tgaaatccca 2880
gctacttggg aggctgaggc aggagaattg cttgaacccg ggaggcggag gttgcagtaa 2940
gccgagacca tgcttattgc cctccagtct gggcaacaaa aatgaaactc cgtctcaaaa 3000
aaaaaaaagt aggtcaattc gtaacagttt attttggaca caccctgaag cttttgtttt 3060
gaattaagaa atgagtttca ggaattgaca aaccatttgt taaccagctg tcctgggcta 3120
ttgaagaaga tttcattttc cgcgcatcca cttgttgcag tccaagtcct ctagtgcaac 3180
gccatagcaa atataaagat ttactatgca cgtgatacat tttatggagt gcctcaagcc 3240
aagatagtga atttatatga agggtgtgaa atgtatttct ttactactat agtagtttag 3300
ttataacttt gcatctataa ttgagctttc tcatctgtca aatgctatgg ttttcttaaa 3360
atgttacaat tctagatcta tccaccttgt ttttttattg tctacaaacc attaaatgta 3420
aatattgttt ttagagtcag tcattggctt tgtcatttac cctttgagag ttccacaagt 3480
ggtagtagag tggtttaacg tctttcctct agtactacca gtattcataa atgtataccc 3540
cttactgtaa tttgttcctc ttagaagtca gatcatctga tttatagagg attatgaaaa 3600
ccagagtttt tgaaaaggct tattttatac atacgtatta tatagagaaa caaatgtttt 3660
tattaaatgt ttcattgacc caagtaattt aaaagtaata tgttacctat gtctttcagg 3720
aaaaataatt atagcagctg atctgtaaat gactttaaaa gctattttag taatttaaat 3780
aaatttactt tcttggttta tactctcaaa aaaaaaaaaa aaaa                  3824
```

<210> 100
<211> 1791
<212> DNA
<213> Homo sapiens

<400> 100

```
gagcccggct gcggatctgg gaagcgcctc ttcacggcac tgggatccgc atctgcctgg 60
gatcatcaag ccctagaagc tgggtttctt taaattaggg ctgccgtttt ctgtttctcc 120
ctgggctgcg gaaagccaga agattttatc tagcttatac aaggctgctg gtgttccctc 180
tttttttcca cgagggtgtt tttggctgca attgcatgaa atcccaatgg tgtagaccag 240
tggcgatgga tctaggagtt taccaactga gacatttttc aatttctttc ttgtcatcct 300
tgctggggac tgaaaacgct tctgtgagac ttgataatag ctcctctggt gcaagtgtgg 360
tagctattga caacaaaatc gagcaagcta tggatctagt gaaaagccat ttgatgtatg 420
cggtcagaga agaagtggag gtcctcaaag agcaaatcaa agaactaata gagaaaaatt 480
cccagctgga gcaggagaac aatctgctga agacactggc cagtcctgag cagcttgccc 540
agtttcaggc ccagctgcag actggctccc cccctgccac cacccagcca cagggcacca 600
cacagccccc cgcccagcca gcatcgcagg gctcaggacc aaccgcatag ctgcctatgc 660
ccccgcagaa ctggctgctg cgtgtgaact gaacagacgg agaagatgtg ctagggagaa 720
tctgcctcca cagtcaccca tttcattgct cgctgcgaaa gagacgtgag actgacatat 780
gccattatct cttttccagt attaaacact catatgctta tggcttggag aaatttctta 840
gttgggtgaa ttaaaggtta atccgagaat tagcatggat ataccgggac ctcatgcagc 900
ttggcagata tctgagaaat ggtttaattc atgctcagga gctgtgtgcc tttccatccc 960
ttccggctcc ctacccctca cttccaaggg ttctctctcc tgcttgcgct tagtgtccta 1020
catggggttg tgaagcgatg gagctcctca ctggactcgc ctctctcctc tcctcccccc 1080


aggaggaact tgaaaggagg gtaaaaagac taaaatgagg gggaacagag ttcactgtac 1140
aaatttgaca actgtcacca aaattcataa aaaacaatag tactgtgcct ctttcttctc 1200
aaacaatgga tgacacaaaa ctatgagagt gacaaaatgg tgacaggtag ctgggaccta 1260
ggctatctta ccatgaaggt tgttttgctt attgtatatt tgtgtatgta gtgtaactat 1320
tttgtacaat agaggactgt aactactatt taggttgtac agattgaaat ttagttgttt 1380
cattggctgt ctgaggaggt gtggactttt atatatagat ctacataaaa actgctacat 1440
gacaaaaacc acacctaaag aaattttaag aatttggcac agttactcac tttgtgtaat 1500
ctgaaatcta gctgctgaat acgctgaagt aaatccttgt tcactgaagt ctttcaattg 1560
agctggttga atactttgaa aaatgctcag ttctaactaa tgaaatggat ttcccagtag 1620
gggtttctgc atatcacctg tatagtagtt atatgcatat gtttctgtgc atgttctcta 1680
cacaattgta aggtgtcact gtatttaact gttgcacttg tcaactttca ataaagcata 1740
taaatgttga taaaaaaaaa aaaaaaaaaa aaaaaaaaaa aaaaaaaaaa a          1791
```

<210> 101
<211> 1514
<212> DNA
<213> Homo sapiens

<400> 101

agttctcact gagacctgtc accccgactc aacgtgagac gcaccgcccg gactcaccat 60
gcgtgaatgc atctcagtcc acgtggggca ggcaggtgtc cagatgggca atgcctgctg 120
ggagctctat tgcttggaac atgggattca gcctgatggg cagatgccca gtgacaagac 180
cattggtgga ggggacgact ccttcaccac cttcttctgt gaaactggtg ctggaaaaca 240
cgtaccccgg gcagtttttg tggatctgga gcctacggtc attgatgaga tccgaaatgg 300
cccataccga cagctcttcc acccagagca gctcatcact gggaaagagg atgctgccaa 360
caactatgcc cgtggtcact ataccattgg caaggagatc attgacccag tgctggatcg 420
gatccgcaag ctgtctgacc agtgcacagg acttcagggc ttcctggtgt tccacagctt 480
tggtgggggc actggctctg gcttcacctc actcctgatg gagcggctct ctgttgacta 540
tggcaagaaa tccaagctgg aattctccat ctacccagcc ccccaggtgt ctacagccgt 600
ggtcgagccc tacaactcta tcctgaccac ccacaccacc ctggagcact cagactgtgc 660
cttcatggtg gacaacgaag caatctatga catctgccgc cgcaacctag acatcgagcg 720
cccaacctac accaacctca atcgcctcat tagccaaatt gtctcctcca tcacagcttc 780
tctgcgcttt gacgggggccc tcaatgtgga cctgacagag ttccagacca acctggtgcc 840
ctaccctcgc atccacttcc ccctggccac ctatgcacca gtcatctctg cagaaaaggc 900
ataccacgag cagctgtcgg tggcagagat caccaatgcc tgctttgagc ctgccaacca 960
gatggtaaag tgtgatcccc ggcacggcaa gtacatggcc tgctgcctgc tgtaccgtgg 1020
agatgtggtg cccaaggatg tcaacgctgc cattgccgcc atcaagacca agcgcagcat 1080
tcagtttgtg gactggtgcc ccacaggctt caaggttggt atcaactacc agcctcccac 1140
tgtggtgcct gggggtgacc tggccaaggt gcagcgtgcc gtgtgcatgc tgagcaacac 1200
gaccgccatc gccgaggcct gggcccgcct ggaccacaag ttcgacctga tgtatgccaa 1260
gagggcgttt gtgcactggt atgtgggtga gggcatggag gagggtgagt tctccgaggc 1320
ccgtgaggat atggctgccc tggagaagga ttatgaggag gtgggcatcg actcctatga 1380
ggacgaggat gagggagaag aataaagcag ctgcctggag cctattcact atgtttattg 1440
caaaatcctt tcgaaataaa cagtttcctt gcacggttaa aaaaaaaaaa aaaaaaaaaa 1500
aaaaaaaaaa aaaa 1514

<210> 102
<211> 2344
<212> DNA
<213> Homo sapiens

<400> 102

cggccgcctc cgcgtccgcg tcgtcgtctg tgctcccggc gctgacgtgt ctgggcggtc 60
ggcttccact ccttcaggcg tcggcagcca ctagtcgtgg cgagaggggc ggggtggccg 120
gggctggcgc tccacttggc cccgctcccc ggcccgcccc gccgccgcgg ccccccggat 180
gagggtatat attcggagcg agcgcgggac gccgatgagt ggccgcgcgg aaggagctgg 240
agacggtcgt agctgcggtc gcgccgagaa aggtttacag gtacatacat tacaccccta 300
tttctacaaa gcttggctat tagagcatta tgaacattaa tgacctcaaa ctcacgttgt 360
ccaaagctgg gcaagagcac ctactacgtt tctggaatga gcttgaagaa gcccaacagg 420
tagaacttta tgcagagctc caggccatga actttgagga gctgaacttc tttttccaaa 480
aggccattga aggttttaac cagtcttctc accaaaagaa tgtggatgca cgaatggaac 540
ctgtgcctcg agaggtatta ggcagtgcta caagggatca agatcagctc caggcctggg 600
aaagtgaagg acttttccag atttctcaga ataaagtagc agttcttctt ctagctggtg 660
ggcaggggac aagactcggc gttgcatatc ctaaggggat gtatgatgtt ggtttgccat 720
cccgtaagac actttttcag attcaagcag agcgtatcct gaagctacag caggttgctg 780

```
aaaaatatta tggcaacaaa tgcattattc catggtatat aatgaccagt ggcagaacaa 840
tggaatctac aaaggagttc ttcaccaagc acaagtactt tggtttaaaa aaagagaatg 900
taatcttttt tcagcaagga atgctccccg ccatgagttt tgatgggaaa attattttgg 960
aagagaagaa caaagtttct atggctccag atgggaatgg tggtctttat cgggcacttg 1020
cagcccagaa tattgtggag gatatggagc aaagaggcat ttggagcatt catgtctatt 1080
gtgttgacaa catattagta aaagtggcag acccacggtt cattggattt tgcattcaga 1140
aaggagcaga ctgtggagca aaggtggtag agaaaacgaa ccctacagaa ccagttggag 1200
tggtttgccg agtggatgga gtttaccagg tggtagaata tagtgagatt tccctggcaa 1260
cagctcaaaa acgaagctca gacggacgac tgctgttcaa tgcggggaac attgccaacc 1320
atttcttcac tgtaccattt ctgagagatg ttgtcaatgt ttatgaacct cagttgcagc 1380
accatgtggc tcaaaagaag attccttatg tggataccca aggacagtta attaagccag 1440
acaaacccaa tggaataaag atggaaaaat ttgtctttga catcttccag tttgcaaaga 1500
agtttgtggt atatgaagta ttgcgagaag atgagttttc cccactaaag aatgctgata 1560
gtcagaatgg gaaagacaac cctactactg caaggcatgc tttgatgtcc cttcatcatt 1620
gctgggtcct caatgcaggg ggccatttca tagatgaaaa tggctctcgc cttccagcaa 1680
ttccccgctt gaaggatgcc aatgatgtac caatccaatg tgaaatctct cctcttatct 1740
cctatgctgg agaaggatta gaaagttatg tggcagataa agaattccat gcacctctaa 1800
tcatcgatga gaatggagtt catgagctgg tgaaaaatgg tatttgaacc agataccaag 1860
ttttgtttgc cacgatagga atagctttta tttttgatag accaactgtg aacctacaag 1920
acgtcttgga caactgaagt ttaaatatcc acagggtttt attttgcttg ttgaactctt 1980
agagctattg caaacttccc aagatccaga tgactgaatt tcagatagca tttttatgat 2040
tcccaactca ttgaaggtct tatttatata atttttttcca agccaaggag accattggcc 2100
atccaggaaa tttcgtacag ctgaaatata ggcaggatgt tcaacatcag tttacttgca 2160
gctggaagca tttgtttttg aagttgtaca tagtaataat atgtcattgt acatgttgaa 2220
aggtttctat ggtactaaaa gtttgtttta ttttatcaaa cattaagctt ttttaagaaa 2280
ataattgggc agtgaaataa atgtatcttc ttgtctctgg agtgtcaaaa aaaaaaaaaa 2340
aaaa                                                              2344
```

<210> 103
<211> 1857
<212> DNA
<213> Homo sapiens

<400> 103

```
caccgggcca gggctggttc cacgctgtca tttctctccg ctggccccag ccgcggggcc 60
accgccagac agacagaccg gggatcctgc ctggcggcgt gaccttgggt tagtcctaga 120
gccgcttcta gcccggtttc ctccgcccca ggttaggacc acgtgggtgg ttccctcatt 180
cgagtgctcc ggcgcacaga cccgcgcccc gccgtctgcg agcctcccga gagccgtccc 240
ttcgtccggc cctggagcat tgcgtttgtc gccggtgtcg cagtgcgagg atggcgccgc 300
gggtgtagcg gctctctgcg caggccgagt gggcccagag aagcgaggaa ctccgcagct 360
cgtcgacacg tctcgtctcc tgtcccaatt cagggcttgg tgaggtgact cgcggtcgcg 420
ggtgactcgc cggcaggaca ctgcctggaa cgcctggagc gcctcccact gcagacgtct 480
gtccgcctcc agccgctctc ctctgacggg tcctgcctca gttggcggaa tggcggccac 540
gggagccaat gcagagaaag ctgaaagtca caatgattgc cccgtcagac ttttaaatcc 600
aaacatagca aaaatgaaag aagatattct ctatcatttc aatctcacca ctagcagaca 660
caatttccca gccttgtttg gagatgtgaa gtttgtgtgt gttggtggaa gcccctcccg 720
gatgaaagcc ttcatcaggt gcgttggtgc agagctgggc cttgactgcc caggtagaga 780
ctatcccaac atctgtgcgg gaactgaccg ctatgccatg tataaagtag gaccggtgct 840
gtctgtcagt catggtatgg gcattccttc tatctcaatc atgttgcatg agctcataaa 900
gctgctgtac tatgcccggt gctccaacgt cactatcatc cgcattggca cttctggtgg 960
gataggtctg gagcccggca ctgtggtcat aacagagcag gcagtggata cctgcttcaa 1020
ggcagagttt gagcagattg tcctggggaa gcgggtcatc cggaaaacgg accttaacaa 1080
gaagctggtg caggagctgt tgctgtgttc tgcagagctg agcgagttca ccacagtggt 1140
ggggaacacc atgtgcacct tggacttcta tgaagggcaa ggccgtctgg atggggctct 1200
ctgctcctac acggagaagg acaagcaggc gtatctggag gcagcctatg cagccggcgt 1260
ccgcaatatc gagatggagt cctcggtgtt tgccgccatg tgcagcgcct gcggcctcca 1320
agcggccgtg gtgtgtgtca ccctcctgaa ccgcctggaa ggggaccaga tcagcagccc 1380
tcgcaatgtg ctcagcgagt accagcagag gccgcagcgg ctggtgagct acttcatcaa 1440
gaagaaactg agcaaggcct gagcgctgcc ctgcacctcc gcagacctgc tgtgatgact 1500
tgccattaaa agcattgtcc aaaatcccct gttgtgtgga ctttgagcac actttacaca 1560
agaatctaga aaatcagatc gcgattaaga gacagagaat cttggattaa ccgcatggga 1620
gatgttcttc cttttgaagt ttcattggag cattttcaat gatgttagcc tgatttgggg 1680
tttcttcaag aacattctac caaatttttg tactatttct agggaaattt ttcagacttt 1740
aaaattctaa tggtagtcag atttcatgtc actaaacaag aaatctgaca atagtgccag 1800
gaaactaatt tcctgataca ttaaaaaaat tccatgcaaa aaaaaaaaaa aaaaaaa    1857
```

<210> 104
<211> 3614
<212> DNA
<213> Homo sapiens

<400> 104

ggcttggggc agccgggtag ctcggaggtc gtggcgctgg gggctagcac cagcgctctg 60
tcgggaggcg cagcggttag gtggaccggt cagcggactc accggccagg gcgctcggtg 120
ctggaatttg atattcattg atccgggttt tatccctctt ctttttttctt aaacattttt 180
ttttaaaact gtattgtttc tcgttttaat ttatttttgc ttgccattcc ccacttgaat 240
cgggccgacg gcttggggag attgctctac ttccccaaat cactgtggat tttggaaacc 300
agcagaaaga ggaaagaggt agcaagagct ccagagagaa gtcgaggaag agagagacgg 360
ggtcagagag agcgcgcggg cgtgcgagca gcgaaagcga caggggcaaa gtgagtgacc 420
tgcttttggg ggtgaccgcc ggagcgcggc gtgagccctc cccttggga tcccgcagct 480
gaccagtcgc gctgacggac agacagacag acaccgcccc cagccccagc taccacctcc 540
tccccggccg gcggcggaca gtggacgcgg cggcgagccg cgggcagggg ccggagcccg 600
cgcccggagg cggggtggag ggggtcgggg ctcgcggcgt cgcactgaaa cttttcgtcc 660
aacttctggg ctgttctcgc ttcggaggag ccgtggtccg cgcgggggaa gccgagccga 720
gcggagccgc gagaagtgct agctcgggcc gggaggagcc gcagccggag gagggggagg 780
aggaagaaga gaaggaagag gagagggggc cgcagtggcg actcggcgct cggaagccgg 840
gctcatggac gggtgaggcg gcggtgtgcg cagacagtgc tccagccgcg cgcgctcccc 900
aggccctggc ccgggcctcg ggccggggag gaagagtagc tcgccgaggc gccgaggaga 960
gcgggccgcc ccacagcccg agccggagag ggagcgcgag ccgcgccggc cccggtcggg 1020
cctccgaaac catgaacttt ctgctgtctt gggtgcattg gagccttgcc ttgctgctct 1080
acctccacca tgccaagtgg tcccaggctg cacccatggc agaaggagga gggcagaatc 1140
atcacgaagt ggtgaagttc atggatgtct atcagcgcag ctactgccat ccaatcgaga 1200
ccctggtgga catcttccag gagtaccctg atgagatcga gtacatcttc aagccatcct 1260
gtgtgcccct gatgcgatgc gggggctgct gcaatgacga gggcctggag tgtgtgccca 1320
ctgaggagtc caacatcacc atgcagatta tgcggatcaa acctcaccaa ggccagcaca 1380
taggagagat gagcttccta cagcacaaca aatgtgaatg cagaccaaag aaagatagag 1440
caagacaaga aaaaaaatca gttcgaggaa agggaaaggg gcaaaaacga aagcgcaaga 1500
aatcccggta taagtcctgg agcgttccct gtgggccttg ctcagagcgg agaaagcatt 1560
tgtttgtaca agatccgcag acgtgtaaat gttcctgcaa aaacacagac tcgcgttgca 1620
aggcgaggca gcttgagtta aacgaacgta cttgcagatg tgacaagccg aggcggtgag 1680
ccgggcagga ggaaggagcc tccctcaggg tttcgggaac cagatctctc accaggaaag 1740
actgatacag aacgatcgat acagaaacca cgctgccgcc accacaccat caccatcgac 1800
agaacagtcc ttaatccaga aacctgaaat gaaggaagag gagactctgc gcagagcact 1860
ttgggtccgg agggcgagac tccggcggaa gcattcccgg gcgggtgacc cagcacggtc 1920
cctcttggaa ttggattcgc catttttattt ttcttgctgc taaatcaccg agcccggaag 1980
attagagagt tttatttctg ggattcctgt agacacaccc acccacatac atacatttat 2040
atatatatat attatatata tataaaaata aatatctcta ttttatatat ataaaatata 2100
tatattcttt ttttaaatta acagtgctaa tgttattggt gtcttcactg gatgtatttg 2160
actgctgtgg acttgagttg ggaggggaat gttcccactc agatcctgac agggaagagg 2220
aggagatgag agactctggc atgatctttt ttttgtccca cttggtgggg ccagggtcct 2280
ctcccctgcc caggaatgtg caaggccagg gcatgggggc aaatatgacc cagttttggg 2340
aacaccgaca aacccagccc tggcgctgag cctctctacc ccaggtcaga cggacagaaa 2400
gacagatcac aggtacaggg atgaggacac cggctctgac caggagtttg gggagcttca 2460
ggacattgct gtgctttggg gattccctcc acatgctgca cgcgcatctc gcccccaggg 2520
gcactgcctg gaagattcag gagcctgggc ggccttcgct tactctcacc tgcttctgag 2580
ttgcccagga gaccactggc agatgtcccg gcgaagagaa gagacacatt gttggaagaa 2640
gcagcccatg acagctcccc ttcctgggac tcgccctcat cctcttcctg ctccccttcc 2700
tggggtgcag cctaaaagga cctatgtcct cacaccattg aaaccactag ttctgtcccc 2760
ccaggagacc tggttgtgtg tgtgtgagtg gttgaccttc ctccatcccc tggtccttcc 2820
cttcccttcc cgaggcacag agagacaggg caggatccac gtgcccattg tggaggcaga 2880
gaaaagagaa agtgttttat atacggtact tatttaatat cccttttttaa ttagaaatta 2940
aaacagttaa tttaattaaa gagtagggtt ttttttcagt attcttggtt aatatttaat 3000
ttcaactatt tatgagatgt atcttttgct ctctcttgct ctcttatttg taccggtttt 3060
tgtatataaa attcatgttt ccaatctctc tctccctgat cggtgacagt cactagctta 3120
tcttgaacag atatttaatt ttgctaacac tcagctctgc cctccccgat cccctggctc 3180
cccagcacac attcctttga aataaggttt caatatacat ctacatacta tatatatatt 3240
tggcaacttg tatttgtgtg tatatatata tatatatgtt tatgtatata tgtgattctg 3300
ataaaataga cattgctatt ctgtttttta tatgtaaaaa caaaacaaga aaaaatagag 3360
aattctacat actaaatctc tctccttttt taattttaat atttgttatc atttatttat 3420
tggtgctact gtttatccgt aataattgtg gggaaaagat attaacatca cgtctttgtc 3480

```
tctagtgcag tttttcgaga tattccgtag tacatattta tttttaaaca acgacaaaga 3540
aatacagata tatcttaaaa aaaaaaaagc attttgtatt aaagaattta attctgatct 3600
caaaaaaaaa aaaa                                                   3614
```

<210> 105
<211> 1745
<212> DNA
<213> Homo sapiens

<400> 105

```
agcctgactt cagcgctccc actctcggcc gacacccctc atggccaacc gttacaccat 60
ggatctgact gccatctacg agagcctcct gtcgctgagc cctgacgtgc ccgtgccatc 120
cgaccatgga gggactgagt ccagcccagg ctggggctcc tcgggaccct ggagcctgag 180
cccctccgac tccagcccgt ctggggtcac ctcccgcctg cctggccgct ccaccagcct 240
agtggagggc cgcagctgtg gctgggtgcc cccacccct ggcttcgcac cgctggctcc 300
ccgcctgggc cctgagctgt caccctcacc cacttcgccc actgcaacct ccaccacccc 360
ctcgcgctac aagactgagc tatgtcggac cttctcagag agtgggcgct gccgctacgg 420
ggccaagtgc cagtttgccc atggcctggg cgagctgcgc caggccaatc gccaccccaa 480
atacaagacg gaactctgtc acaagttcta cctccagggc cgctgcccct acggctctcg 540
ctgccacttc atccacaacc ctagcgaaga cctggcggcc ccgggccacc ctcctgtgct 600
tcgccagagc atcagcttct ccggcctgcc ctctggccgc cggacctcac caccaccacc 660
aggcctggcc ggcccttccc tgtcctccag ctccttctcg ccctccagct ccccaccacc 720
acctggggac cttccactgt caccctctgc cttctctgct gcccctggca cccccctggc 780
tcgaagagac ccccaccccag tctgttgccc ctcctgccga agggccactc ctatcagcgt 840
ctgggggccc ttgggtggcc tggttcggac cccctctgta cagtccctgg gatccgaccc 900
tgatgaatat gccagcagcg gcagcagcct gggggctct gactctcccg tcttcgaggc 960
gggagttttt gcaccacccc agcccgtggc agccccccgg cgactcccca tcttcaatcg 1020
catctctgtt tctgagtgac aaagtgactg cccggtcaga tcagctggat ctcagcgggg 1080
agccacgtct cttgcactgt ggtctctgca tggaccccag ggctgtgggg acttggggga 1140
cagtaatcaa gtaatcccct tttccagaat gcattaaccc actcccctga cctcacgctg 1200
gggcaggtcc ccaagtgtgc aagctcagta ttcatgatgg tgggggatgg agtgtcttcc 1260
gaggttcttg gggaaaaaaa aattgtagca tatttaaggg aggcaatgaa ccctctcccc 1320
cacctcttcc ctgcccaaat ctgtctccta gaatcttatg tgctgtgaat aataggcctt 1380
cactgcccct ccagttttta tagacctgag gttccagtgt ctcctggtaa ctggaacctc 1440
tcctgagggg gaatcctggt gctcaaatta ccctccaaaa gcaagtagcc aaagccgttg 1500
ccaaacccca cccataaatc aatgggccct ttatttatga cgactttatt tattctaata 1560
tgattttata gtatttatat atattgggtc gtctgcttcc cttgtatttt tcttcctttt 1620
tttgtaatat tgaaaacgac gatataatta ttataagtag actataatat atttagtaat 1680
atatattatt accttaaaag tctatttttg tgttttgggc atttttaaat aaacaatctg 1740
agtgt                                                             1745
```

<210> 106
<211> 60
<212> DNA
<213> Homo sapiens

<400> 106
ctttatggaa agggctatgt gggagagtca gctatcttgt ctggttttct tgagacctca 60

<210> 107
<211> 60
<212> DNA
<213> Homo sapiens

<400> 107
cttcagttgt tttcccgagc aaaggtctaa agtttacagt aaataaaatg tttgaccatg 60

<210> 108

<211> 60
<212> DNA
<213> Homo sapiens

<400> 108
ggcgccaatg gtatctgggc ggagctcaca gagttcttgg aataaaagca acctcagaac 60

<210> 109
<211> 60
<212> DNA
<213> Homo sapiens

<400> 109
tatgtgacaa tgatgacaca gccatggctg catttatgaa ttacttagaa gcagaggggg 60

<210> 110
<211> 60
<212> DNA
<213> Homo sapiens

<400> 110
atgaactggg aagagatgaa atgttttttc atatttaaat aaataagaac attaaaaagc 60

<210> 111
<211> 60
<212> DNA
<213> Homo sapiens

<400> 111
gggcaccttt caacatcatt tgcttcctta tctacagttg attcagaaat ctgcattttt 60

<210> 112
<211> 60
<212> DNA
<213> Homo sapiens

<400> 112
ccacttaaaa tctgcttatg gcacaatttg cctcaaaatc cattccaagt tgtatatttg 60

<210> 113
<211> 60
<212> DNA
<213> Homo sapiens

<400> 113
aaatcaagcc tgaggctggt gaacagtagc tacacaccca tattgtgtgt tctgtgaatg 60

<210> 114
<211> 60
<212> DNA
<213> Homo sapiens

<400> 114
accacactat cttctgtatc aggtagtcta atagaaatat acctgttttg ttctaaaaaa 60

<210> 115
<211> 60
<212> DNA

<213> Homo sapiens

<400> 115
gtctcctaaa ttgttgtaat tgcattataa aataagaaaa acattaataa gacaaatatt 60

<210> 116
<211> 60
<212> DNA
<213> Homo sapiens

<400> 116
gccaaactaa acttgctgct taatgatttg ctcacatcta gtaaaacatg gagtatttgt 60

<210> 117
<211> 60
<212> DNA
<213> Homo sapiens

<400> 117
tgatgccgtg cactgtgtga agcagatgtt tttgtccgga aataaaaata atagtcttgg 60

<210> 118
<211> 60
<212> DNA
<213> Homo sapiens

<400> 118
tagatgatat ttattgcaga atttctattc ttgtattaac aaataaaatg cttgccccag 60

<210> 119
<211> 60
<212> DNA
<213> Homo sapiens

<400> 119
taagacagtt ttgtatcatt ttgctaaaaa ttattggctt aaatctgtgt aaagaaaaaa 60

<210> 120
<211> 60
<212> DNA
<213> Homo sapiens

<400> 120
gtaaggcata ctgccttgtt taatggtagt tttacagtgt ttctggctta gaacaaaggg 60

<210> 121
<211> 60
<212> DNA
<213> Homo sapiens

<400> 121
ttccctctgt gttagagcag agaggtttcg atatttattg atgttttcac aaagaacagg 60

<210> 122
<211> 60
<212> DNA
<213> Homo sapiens

<400> 122
ctgcagcgtt tctctttccc ttggaaaaga gaatttatca ttactgttac atttgtacaa 60

<210> 123
<211> 60
<212> DNA
<213> Homo sapiens

<400> 123
gttcatagtc tttattttgt cctttgaata aacattaaaa gatttctaaa cttcaaaaaa 60

<210> 124
<211> 60
<212> DNA
<213> Homo sapiens

<400> 124
aatcaagatt tgtataaaat gttgtttaca gatcttttaa tgaataaata cataaacccc 60

<210> 125
<211> 60
<212> DNA
<213> Homo sapiens

<400> 125
cattttgtta cataaggatg acttttttat acaatggaat aaattatggc atttctattg 60

<210> 126
<211> 60
<212> DNA
<213> Homo sapiens

<400> 126
cgggttgggt gggaggagga agaaagggaa gaattaggtt tgaattgctt ttttaaaaaa 60

<210> 127
<211> 60
<212> DNA
<213> Homo sapiens

<400> 127
ccgtgtgaat gtgaagaaaa gcagtatgtt actggttgtt gttgttgttc ttgtttttta 60

<210> 128
<211> 60
<212> DNA
<213> Homo sapiens

<400> 128
gaagggagag ttaaattctc cattatgttc gtggtgtaaa gtttagagct ggaatttatt 60

<210> 129
<211> 60
<212> DNA
<213> Homo sapiens

<400> 129
tgtaaagatg ttttgtataa actctgaatt attttcttgt tgcttttttc ttagaaaaaa 60

<210> 130
<211> 60
<212> DNA
<213> Homo sapiens

<400> 130
ggatatgtgg tgtatatcct tccaaaaaat taaaacgaaa ataaagtagc tgcgattggg 60

<210> 131
<211> 60
<212> DNA
<213> Homo sapiens

<400> 131
cacctgtggg aagagaagtg aagactttct ccttgcatta aaaagtctga actgtgaaaa 60

<210> 132
<211> 60
<212> DNA
<213> Homo sapiens

<400> 132
tgtccaagta atgtttttat aataaactaa gccatattta gacaataaac atcgaaaaaa 60

<210> 133
<211> 60
<212> DNA
<213> Homo sapiens

<400> 133
gaaaatagat aatttacaag atattattaa tttttattta tttttcttgg gaattgaaaa 60

<210> 134
<211> 60
<212> DNA
<213> Homo sapiens

<400> 134
tgaatttgaa tgaaaaattt aagcattgtt tgcttattgt tccaagacat tgtcaataaa 60

<210> 135
<211> 60
<212> DNA
<213> Homo sapiens

<400> 135
taaaggaaaa gttgattatg tatgtggggt gccaggacca ctgccttgaa agcaagtgtg 60

<210> 136
<211> 60
<212> DNA
<213> Homo sapiens

<400> 136
gaattatcta aatgcgtcta ccattttgca ctagggagga aggataaatg ctttttatgt 60

<210> 137
<211> 60

<212> DNA
<213> Homo sapiens

<400> 137
gactcggggg ctggtctgat ggaactgtgt atttatttaa aactctggtg ataaaaataa 60

<210> 138
<211> 60
<212> DNA
<213> Homo sapiens

<400> 138
ccccaaaaga gttgatgtgt gttttaaaag aaaaaaccca atgaggaaca attggagatt 60

<210> 139
<211> 60
<212> DNA
<213> Homo sapiens

<400> 139
gtgccatttc tgaactgtta caacttgtat ttccaaacct ggttcatatt tatactttgc 60

<210> 140
<211> 60
<212> DNA
<213> Homo sapiens

<400> 140
cgactgtcga gatcgcctag tatgttctgt gaacacaaat aaaattgatt tactgtctgc 60

<210> 141
<211> 60
<212> DNA
<213> Homo sapiens

<400> 141
gattccagaa tttttctgtg atacatgaag actttccata tcaagagaca tggtattgac 60

<210> 142
<211> 60
<212> DNA
<213> Homo sapiens

<400> 142
ccatgagacc actgttatca aaactttctt ttctggaatg taatcaatgt ttcttctagg 60

<210> 143
<211> 60
<212> DNA
<213> Homo sapiens

<400> 143
gcctagtttt taatagctat ggaatcaatt caatttggac tggtgtgctc tctttaaatc 60

<210> 144
<211> 60
<212> DNA
<213> Homo sapiens

<400> 144
atggaaatat aaaagtaaat atgaaacatt taaaatataa tttgttgtca aagtaaaaaa 60

<210> 145
<211> 60
<212> DNA
<213> Homo sapiens

<400> 145
tctgatctgg acttcctata atacaaatac acaatcctcc aagaatttga cttggaaaag 60

<210> 146
<211> 60
<212> DNA
<213> Homo sapiens

<400> 146
ctccttttgg tgtaatgttt tacaaatcct ttggcctgag aactaatatg ttaattgcct 60

<210> 147
<211> 60
<212> DNA
<213> Homo sapiens

<400> 147
agaatccaga gttgctacaa taaaataagg ggaataataa acttgagagt gaataaccat 60

<210> 148
<211> 60
<212> DNA
<213> Homo sapiens

<400> 148
ggaggaagaa gtgtaactca ccagcctctg ctcttatctt tgtaataaat gttaaagcca 60

<210> 149
<211> 60
<212> DNA
<213> Homo sapiens

<400> 149
ctgtatagtt gtagaatttt ttgagagaat gagatgttta tatattaacg acaatttttt 60

<210> 150
<211> 60
<212> DNA
<213> Homo sapiens

<400> 150
gaatgttttg tgtgaaagga gtataccatg agatgagatg accaccaatc atttccttgg 60

<210> 151
<211> 60
<212> DNA
<213> Homo sapiens

<400> 151
catatatgtt aaaggattat aatttatgga attaaaaaat gcagtgtagt ccttaaaaaa 60

<210> 152
<211> 60
<212> DNA
<213> Homo sapiens

<400> 152
gaatgatgtc cccgttgtat gtatgagtgg cttctgggag atgggtgtca ctttttaaac 60

<210> 153
<211> 60
<212> DNA
<213> Homo sapiens

<400> 153
aatacattgt ctagcattga tttggttcct gtgcatatgt attttcacta tgtgctcccc 60

<210> 154
<211> 60
<212> DNA
<213> Homo sapiens

<400> 154
tcctttccac tgaaaagcac atggccttgg gtgacaaatt cctctttgat gaatgtaccc 60

<210> 155
<211> 60
<212> DNA
<213> Homo sapiens

<400> 155
gctgtctagg atgatcactg attactattt actaagtagc cacatgcaaa taaaagttgt 60

<210> 156
<211> 60
<212> DNA
<213> Homo sapiens

<400> 156
ccaagtgagc acattcagct ttggaaacta tattatttaa tgtaggctag cttgttttca 60

<210> 157
<211> 60
<212> DNA
<213> Homo sapiens

<400> 157
aatagtgttt gtggggagcc agagattcct aaaagaacag tgaatagcac agtctcatta 60

<210> 158
<211> 60
<212> DNA
<213> Homo sapiens

<400> 158
cacggggtag gggaaatggg gtgaacggtg ctggcagttc ggctagattt ctgtcttgtt 60

<210> 159
<211> 60

<212> DNA
<213> Homo sapiens

<400> 159
gtaactgttt tcaaggaggt gcttaggatt gtgggtttct gattgcatca ctagcttctc 60

<210> 160
<211> 60
<212> DNA
<213> Homo sapiens

<400> 160
cagtgctaga gtgagcatgc ttcaaaactg tacatgaagc caatatattt ttggataagt 60

<210> 161
<211> 60
<212> DNA
<213> Homo sapiens

<400> 161
gagtacggat gggaaactat tgtgcacaag tctttccaga ggagtttctt aatgagatat 60

<210> 162
<211> 60
<212> DNA
<213> Homo sapiens

<400> 162
cattgtttga cccagtatgt cttgtagaca cgttagttat aatcaccttg tatctctaaa 60

<210> 163
<211> 60
<212> DNA
<213> Homo sapiens

<400> 163
aaccacgggt gctttgttct tccttctgca cctccttctt aaactagata gctaattagt 60

<210> 164
<211> 60
<212> DNA
<213> Homo sapiens

<400> 164
gtattgtgaa gtggtataag aaatgacttt gaaccacttt gcaattgtag attcccaaca 60

<210> 165
<211> 60
<212> DNA
<213> Homo sapiens

<400> 165
ccaataagtg gagattcctc cttatgatgt atgctaggtt atggaagatg taaaatattc 60

<210> 166
<211> 60
<212> DNA
<213> Homo sapiens

<400> 166
gttgtattgt accagtgaaa tgccaaattt gaaaggcctg tactgcaatt ttatatgtca 60

<210> 167
<211> 60
<212> DNA
<213> Homo sapiens

<400> 167
gttggtgact taacaaatta agagcatagt tgccatatgc tcatatttgg caaatatgtc 60

<210> 168
<211> 60
<212> DNA
<213> Homo sapiens

<400> 168
cctgcggggc tggtgtgaaa ggcgctggga accggctttg aatgaataaa tgaatcgtgt 60

<210> 169
<211> 60
<212> DNA
<213> Homo sapiens

<400> 169
ggctggagct gcctctctca tccactttcc aataaagagt tccttctgct ccaaaaaaaa 60

<210> 170
<211> 60
<212> DNA
<213> Homo sapiens

<400> 170
ggttgagacc agcactctgt gaaaccttga aatgagaagt aaaggcagat gaaaagaaaa 60

<210> 171
<211> 60
<212> DNA
<213> Homo sapiens

<400> 171
aggtagcctg ctggttttat ctgagtgaaa tactgtacag gggaataaaa gagatcttat 60

<210> 172
<211> 60
<212> DNA
<213> Homo sapiens

<400> 172
ggagttcaag atcagcctgg gcaacacagt gagacctcat ctccactaaa aataaaaaaa 60

<210> 173
<211> 60
<212> DNA
<213> Homo sapiens

<400> 173
ccaaagtgat gcagtggtta ccatgacacc ctaatttcat gtgtttttgt atttatgaaa 60

<210> 174
<211> 60
<212> DNA
<213> Homo sapiens

<400> 174
cctcagtggg aggcgtggct gagaccaact ggtttgccta taatttatta actatttatt 60

<210> 175
<211> 60
<212> DNA
<213> Homo sapiens

<400> 175
atgtacttta tattttctat atttgtaact ttgcatgttc ttgttttgtt atataaaaaa 60

<210> 176
<211> 60
<212> DNA
<213> Homo sapiens

<400> 176
gcgaatgttt cagtgcctca gacaaatgtg tatttaactt atgtaaaaga taagtctgga 60

<210> 177
<211> 60
<212> DNA
<213> Homo sapiens

<400> 177
ataacagtat tttaaatttg taaagaatgt ctaataaaat ataatctaat tacatcatga 60

<210> 178
<211> 60
<212> DNA
<213> Homo sapiens

<400> 178
gatgcaagga taaaccttta ctttgactac cagcctgtgt ttttgtcttt aaatctctta 60

<210> 179
<211> 60
<212> DNA
<213> Homo sapiens

<400> 179
aatttaagac tatttttgta aagctctact gtaaataaaa tattttataa aactaaaaaa 60

<210> 180
<211> 60
<212> DNA
<213> Homo sapiens

<400> 180
gtggcacgta agctccttgt ctgtctccag cacccagaat ctcattaaag cttatttatt 60

<210> 181
<211> 60

<212> DNA
<213> Homo sapiens

<400> 181
cctggaatca gtgttactgc atctgattaa atgtctccag aaataaagaa taattctgcc 60

<210> 182
<211> 60
<212> DNA
<213> Homo sapiens

<400> 182
tgatctacaa tgaagccctc aagggctgaa aataaatagg gaagatggag acaccctctg 60

<210> 183
<211> 60
<212> DNA
<213> Homo sapiens

<400> 183
caagccagag gctgggttca tttgtgtaac gacaataaac ggtacttgtc atttcgggca 60

<210> 184
<211> 60
<212> DNA
<213> Homo sapiens

<400> 184
gttggagtga gcacctcctc tgtaggcacc tctcacactg ttgtctgtta ctgatttttt 60

<210> 185
<211> 60
<212> DNA
<213> Homo sapiens

<400> 185
gatcatctga ggtcagaagt tcgagaccag cctggccaac atggtgaaac cctgcctcca 60

<210> 186
<211> 60
<212> DNA
<213> Homo sapiens

<400> 186
ccttagaaga aagagactgt agcaatacaa aaatcaagct tagtgcttta ttacctgagt 60

<210> 187
<211> 60
<212> DNA
<213> Homo sapiens

<400> 187
tatgtctgta tcttatattc aattgcaagt atataataaa taaacctgct tccaaacaac 60

<210> 188
<211> 60
<212> DNA
<213> Homo sapiens

<400> 188
tctgagattc aatattgaat ttctcctatg ctattgacaa taaaatatta ttgaactacc 60

<210> 189
<211> 60
<212> DNA
<213> Homo sapiens

<400> 189
ctttggcttt gctaccagtt ccatatgatg agaaataaac gttcgctgag gttttgtttc 60

<210> 190
<211> 60
<212> DNA
<213> Homo sapiens

<400> 190
gagggaaagc atgtctgctg ggtgtgacca tgtttcctct caataaagtt cccctgtgac 60

<210> 191
<211> 60
<212> DNA
<213> Homo sapiens

<400> 191
cctcttgagt gtcttgggga cagctctttc cacccctgga agatggaaat aaacctgcgt 60

<210> 192
<211> 60
<212> DNA
<213> Homo sapiens

<400> 192
attaacttga gttttattta tcttatcctc tggtttaatt acataaatat ttatttttta 60

<210> 193
<211> 60
<212> DNA
<213> Homo sapiens

<400> 193
aggttggcag taaggcaggg tcccatttct cactgagaag attgtgaata tttccatatg 60

<210> 194
<211> 60
<212> DNA
<213> Homo sapiens

<400> 194
ccactctgct gcctgtacag aagaaactga atctttttca tattctaata aatcaatgtg 60

<210> 195
<211> 60
<212> DNA
<213> Homo sapiens

<400> 195
aatagtattt attttagaaa gtctactatt gtaagagttc ttctgtttgt gaagaaaaaa 60

<210> 196
<211> 60
<212> DNA
<213> Homo sapiens

<400> 196
gggtcgtatt cctgatttca gaagaggatc ataactgaaa caacataaga caatataatc 60

<210> 197
<211> 60
<212> DNA
<213> Homo sapiens

<400> 197
cagtaatgtg cattgttatt gagttgtact gtaccttatt tggaaggatg aaggaatgaa 60

<210> 198
<211> 60
<212> DNA
<213> Homo sapiens

<400> 198
aatgcacagt gtttccctgt ttatccatcc cctgcaaact gcagagtggc actcattgct 60

<210> 199
<211> 60
<212> DNA
<213> Homo sapiens

<400> 199
gacatgcctg tagttacatc cagtgtgatg ggtgctgaga ggcaagtaca aaccacgatg 60

<210> 200
<211> 60
<212> DNA
<213> Homo sapiens

<400> 200
ctgaccaaca tcgtgaaatg ccgtctttac aaaaaaatac aaaaattaac tggaaaaaaa 60

<210> 201
<211> 60
<212> DNA
<213> Homo sapiens

<400> 201
ctgtcaaagg tccctatttg aaatgtgtta taatacaaac aaggaagcac attgtgtaca 60

<210> 202
<211> 60
<212> DNA
<213> Homo sapiens

<400> 202
tggggagagt ttggagggga gggagaattt attaataaaa gaatctttaa ctttaaaaaa 60

<210> 203
<211> 60

<212> DNA
<213> Homo sapiens

<400> 203
gggggctggc agaggccccc atatactctg ctacagctcc tatcatgaaa aataaaatgt 60

<210> 204
<211> 60
<212> DNA
<213> Homo sapiens

<400> 204
tttaaaagct attttagtaa tttaaataaa tttactttct tggtttatac tctcaaaaaa 60

<210> 205
<211> 60
<212> DNA
<213> Homo sapiens

<400> 205
catatgtttc tgtgcatgtt ctctacacaa ttgtaaggtg tcactgtatt taactgttgc 60

<210> 206
<211> 60
<212> DNA
<213> Homo sapiens

<400> 206
agcctattca ctatgtttat tgcaaaatcc tttcgaaata aacagtttcc ttgcacggtt 60

<210> 207
<211> 60
<212> DNA
<213> Homo sapiens

<400> 207
gcttttttaa gaaaataatt gggcagtgaa ataaatgtat cttcttgtct ctggaaaaaa 60

<210> 208
<211> 60
<212> DNA
<213> Homo sapiens

<400> 208
gatttcatgt cactaaacaa gaaatctgac aatagtgcca ggaaactaat ttcctgatac 60

<210> 209
<211> 60
<212> DNA
<213> Homo sapiens

<400> 209
aggcgaggca gcttgagtta aacgaacgta cttgcagatg tgacaagccg aggcggtgag 60

<210> 210
<211> 60
<212> DNA
<213> Homo sapiens

<400> 210
ttattacctt aaaagtctat ttttgtgttt tgggcatttt taaataaaca atctgagtgt 60

<210> 211
<211> 5851
<212> DNA
<213> Homo sapiens

<400> 211

```
cttgcgcggt gtggcggccg atgccgctat aaaggcttgt tttgctgcag ggctcatgct 60
cgggagcgtg gttgagcggc tggcgcggtt gtcctggagc aggggcgcag gaattctgat 120
gtgaaactaa cagtctgtga gccctggaac ctccactcag agaagatgaa ggatatcgac 180
ataggaaaag agtatatcat ccccagtcct gggtatagaa gtgtgaggga gagaaccagc 240
acttctggga cgcacagaga ccgtgaagat tccaagttca ggagaactcg accgttggaa 300
tgccaagatg ccttggaaac agcagcccga gccgagggcc tctctcttga tgcctccatg 360
cattctcagc tcagaatcct ggatgaggag catcccaagg gaaagtacca tcatggcttg 420
agtgctctga agcccatccg gactacttcc aaacaccagc acccagtgga caatgctggg 480
ctttttttcct gtatgacttt ttcgtggctt tcttctctgg cccgtgtggc ccacaagaag 540
ggggagctct caatggaaga cgtgtggtct ctgtccaagc acgagtcttc tgacgtgaac 600
tgcagaagac tagagagact gtggcaagaa gagctgaatg aagttgggcc agacgctgct 660
tccctgcgaa gggttgtgtg gatcttctgc cgcaccaggc tcatcctgtc catcgtgtgc 720
ctgatgatca cgcagctggc tggcttcagt ggaccagcct tcatggtgaa acacctcttg 780
gagtataccc aggcaacaga gtctaacctg cagtacagct tgttgttagt gctgggcctc 840
ctcctgacgg aaatcgtgcg gtcttggtcg cttgcactga cttgggcatt gaattaccga 900
accggtgtcc gcttgcgggg ggccatccta accatggcat ttaagaagat ccttaagtta 960
aagaacatta aagagaaatc cctgggtgag ctcatcaaca tttgctccaa cgatgggcag 1020
agaatgtttg aggcagcagc cgttggcagc ctgctggctg gaggacccgt tgttgccatc 1080
ttaggcatga tttataatgt aattattctg ggaccaacag gcttcctggg atcagctgtt 1140
tttatcctct tttacccagc aatgatgttt gcatcacggc tcacagcata tttcaggaga 1200
aaatgcgtgg ccgccacgga tgaacgtgtc cagaagatga atgaagttct tacttacatt 1260
aaatttatca aaatgtatgc ctgggtcaaa gcattttctc agagtgttca aaaaatccgc 1320
gaggaggagc gtcggatatt ggaaaaagct gggtacttcc agagcatcac tgtgggtgtg 1380
gctcccattg tggtggtgat tgccagcgtg gtgaccttct ctgttcatat gaccctgggc 1440
ttcgatctga cagcagcaca ggctttcaca gtggtgacag tcttcaattc catgactttt 1500
gctttgaaag taacaccgtt ttcagtaaag tccctctcag aagcctcagt ggctgttgac 1560
agatttaaga gtttgtttct aatggaagag gttcacatga taaagaacaa accagccagt 1620
cctcacatca agatagagat gaaaaatgcc accttggcat gggactcctc ccactccagt 1680
atccagaact cgcccaagct gacccccaaa atgaaaaaag acaagagggc ttccaggggc 1740
aagaaagaga aggtgaggca gctgcagcgc actgagcatc aggcggtgct ggcagagcag 1800
aaaggccacc tcctcctgga cagtgacgag cggcccagtc ccgaagagga agaaggcaag 1860
cacatccacc tgggccacct gcgcttacag aggacactgc acagcatcga tctggagatc 1920
caagagggta aactggttgg aatctgtggc agtgtgggaa gtggaaaaac ctctctcatt 1980
tcagccattt taggccagat gacgcttcta gagggcagca ttgcaatcag tggaaccttc 2040
gcttatgtgg cccagcaggc ctggatcctc aatgctactc tgagagacaa catcctgttt 2100
gggaaggaat atgatgaaga aagatacaac tctgtgctga acagctgctg cctgaggcct 2160
gacctggcca ttcttcccag cagcgacctg acggagattg gagagcgagg agccaacctg 2220
agcggtgggc agcgccagag gatcagcctt gcccgggcct tgtatagtga caggagcatc 2280
tacatcctgg acgacccccct cagtgcctta gatgcccatg tgggcaacca catcttcaat 2340
agtgctatcc ggaaacatct caagtccaag acagttctgt ttgttaccca ccagttacag 2400
tacctggttg actgtgatga agtgatcttc atgaaagagg gctgtattac ggaaagaggc 2460
acccatgagg aactgatgaa tttaaatggt gactatgcta ccatttttaa taacctgttg 2520
ctgggagaga caccgccagt tgagatcaat tcaaaaaagg aaaccagtgg ttcacagaag 2580
aagtcacaag acaagggtcc taaaacagga tcagtaaaga aggaaaaagc agtaaagcca 2640
gaggaagggc agcttgtgca gctggaagag aaagggcagg gttcagtgcc ctggtcagta 2700
tatggtgtct acatccaggc tgctgggggc cccttggcat tcctggttat tatggccctt 2760
ttcatgctga atgtaggcag caccgccttc agcacctggt ggttgagtta ctggatcaag 2820
caaggaagcg ggaacaccac tgtgactcga gggaacgaga cctcggtgag tgacagcatg 2880
```

aaggacaatc ctcatatgca gtactatgcc agcatctacg ccctctccat ggcagtcatg 2940
ctgatcctga aagccattcg aggagttgtc tttgtcaagg gcacgctgcg agcttcctcc 3000
cggctgcatg acgagctttt ccgaaggatc cttcgaagcc ctatgaagtt ttttgacacg 3060
accccccacag ggaggattct caacaggttt tccaaagaca tggatgaagt tgacgtgcgg 3120
ctgccgttcc aggccgagat gttcatccag aacgttatcc tggtgttctt ctgtgtggga 3180
atgatcgcag gagtcttccc gtggttcctt gtggcagtgg ggccccttgt catcctcttt 3240
tcagtcctgc acattgtctc cagggtcctg attcgggagc tgaagcgtct ggacaatatc 3300
acgcagtcac ctttcctctc ccacatcacg tccagcatac agggccttgc caccatccac 3360
gcctacaata aagggcagga gtttctgcac agataccagg agctgctgga tgacaaccaa 3420
gctccttttt ttttgtttac gtgtgcgatg cggtggctgg ctgtgcggct ggacctcatc 3480
agcatcgccc tcatcaccac cacggggctg atgatcgttc ttatgcacgg gcagattccc 3540
ccagcctatg cgggtctcgc catctcttat gctgtccagt taacggggct gttccagttt 3600
acggtcagac tggcatctga gacagaagct cgattcacct cggtggagag gatcaatcac 3660
tacattaaga ctctgtcctt ggaagcacct gccagaatta agaacaaggc tccctcccct 3720
gactggcccc aggagggaga ggtgaccttt gagaacgcag agatgaggta ccgagaaaac 3780
ctccctctcg tcctaaagaa agtatccttc acgatcaaac ctaaagagaa gattggcatt 3840
gtggggcgga caggatcagg gaagtcctcg ctggggatgg ccctcttccg tctggtggag 3900
ttatctggag gctgcatcaa gattgatgga gtgagaatca gtgatattgg ccttgccgac 3960
ctccgaagca aactctctat cattcctcaa gagccggtgc tgttcagtgg cactgtcaga 4020
tcaaatttgg acccctttcaa ccagtacact gaagaccaga tttgggatgc cctggagagg 4080
acacacatga aagaatgtat tgctcagcta cctctgaaac ttgaatctga agtgatggag 4140
aatggggata acttctcagt gggggaacgg cagctcttgt gcatagctag agccctgctc 4200
cgccactgta agattctgat tttagatgaa gccacagctg ccatggacac agagacagac 4260
ttattgattc aagagaccat ccgagaagca tttgcagact gtaccatgct gaccattgcc 4320
catcgcctgc acacggttct aggctccgat aggattatgg tgctggccca gggacaggtg 4380
gtggagtttg acaccccatc ggtccttctg tccaacgaca gttcccgatt ctatgccatg 4440
tttgctgctg cagagaacaa ggtcgctgtc aagggctgac tcctccctgt tgacgaagtc 4500
tcttttcttt agagcattgc cattccctgc ctggggcggg cccctcatcg cgtcctccta 4560
ccgaaacctt gcctttctcg attttatctt tcgcacagca gttccggatt ggcttgtgtg 4620
tttcactttt agggagagtc atattttgat tattgtattt attccatatt catgtaaaca 4680
aaatttagtt tttgttctta attgcactct aaaaggttca gggaaccgtt attataattg 4740
tatcagaggc ctataatgaa gctttatacg tgtagctata tctatatata attctgtaca 4800
tagcctatat ttacagtgaa aatgtaagct gtttatttta tattaaaata agcactgtgc 4860
taataacagt gcatattcct ttctatcatt tttgtacagt ttgctgtact agagatctgg 4920
ttttgctatt agactgtagg aagagtagca tttcattctt ctctagctgg tggtttcacg 4980
gtgccaggtt ttctgggtgt ccaaaggaag acgtgtggca atagtgggcc ctccgacagc 5040
cccctctgcc gcctccccac ggccgctcca ggggtggctg gagacgggtg ggcggctgga 5100
gaccatgcag agcgccgtga gttctcaggg ctcctgcctt ctgtcctggt gtcacttact 5160
gtttctgtca ggagagcagc ggggcgaagc ccaggcccct tttcactccc tccatcaaga 5220
atggggatca cagagacatt cctccgagcc ggggagtttc tttcctgcct tcttcttttt 5280
gctgttgttt ctaaacaaga atcagtctat ccacagagag tcccactgcc tcaggttcct 5340
atggctggcc actgcacaga gctctccagc tccaagacct gttggttcca agccctggag 5400
ccaactgctg ctttttgagg tggcactttt tcatttgcct attcccacac ctccacagtt 5460
cagtggcagg gctcaggatt tcgtgggtct gttttccttt ctcaccgcag tcgtcgcaca 5520
gtctctctct ctctctcccc tcaaagtctg caactttaag cagctcttgc taatcagtgt 5580
ctcacactgg cgtagaagtt tttgtactgt aaagagacct acctcaggtt gctggttgct 5640
gtgtggtttg gtgtgttccc gcaaaccccc tttgtgctgt ggggctggta gctcaggtgg 5700
gcgtggtcac tgctgtcatc aattgaatgg tcagcgttgc atgtcgtgac caactagaca 5760
ttctgtcgcc ttagcatgtt tgctgaacac cttgtggaag caaaaatctg aaaatgtgaa 5820
taaaattatt ttggattttg taaaaaaaaa a 5851

<210> 212
<211> 3832
<212> DNA
<213> Homo sapiens

<400> 212

```
cacctctaac ccaggctcag agtagctgct gtttctgaga gaacgattcg taggacagcc 60
cctgacgcca ttccctttg cccttctttc tgcgggcctt tccggtactt gagcggtgtc 120
cagagcgtgg ccagttctcg tctatctggc tgcctttagg gagcggtgcc tagcgttggc 180
caatagttgg ctgtcgaaag tgccggcccc cgcgccggcg cctgcagcag ccgggtggga 240
aggctcaaga tggcgtgctt gttggagacc ccaatccgca tgagcgtcct ttcggaccag 300
gaatgaaagt tactgggaga taacttcgaa ggattttgca acaaattcga gctgtccgac 360
tctgagaatg gaagtaacag ctagcagtcg ccactatgtt gacaggctat ttgaccctga 420
tccccagaaa gttctacaag gtgtcataga catgaaaaat gctgtaattg gaaacaacaa 480
```

gcagaaagcc aatctcattg ttttaggagc tgttccaaga ttgttgtact tgcttcagca 540
agaaacctca agcacagagc tgaaaactga atgtgcagtg gtgttgggaa gtcttgctat 600
gggtactgaa aacaatgtca agtctctact ggactgccat attatccctg ccttattgca 660
aggactactg tccccagacc tgaagtttat tgaagcttgc ctccgatgcc tgcgtaccat 720
cttcaccagt cctgtcactc cagaggagct actgtataca gatgccacag tgataccaca 780
cctcatggca ctgcttagca ggtcccgcta tacccaggag tacatctgtc agatcttctc 840
acactgctgt aaagggccag atcatcaaac aattttattt aaccacggtg cagttcagaa 900
tattgctcac ctactaacct cactgtccta caaagttcga atgcaagcac tgaaatgttt 960
ctcagtttta gcttttgaaa acccccaggt atcgatgacc ctggtaaatg ttttggttga 1020
tggagaattg ttaccacaga tttttgtgaa gatgttacag agggataagc ctattgagat 1080
gcagctcaca tcagcaaaat gtttaactta catgtgtaga gctggagcaa ttcggacaga 1140
tgataactgt attgtattaa agacattacc ttgtttggtt cgaatgtgca gtaaggagag 1200
attactagag gagagagttg aaggagctga gacacttgcc tatctgattg aaccagatgt 1260
tgagctacag agaatcgcta gcataactga tcacctcatt gccatgcttg ctgattattt 1320
caagtatccc agctcagtga gtgccatcac tgatattaaa aggcttgatc atgatttaaa 1380
acatgctcac gaactccgcc aggctgcatt caagctctat gcctctcttg gagcaaatga 1440
tgaagacatc cggaagaaga tcattgagac tgaaaatatg atggaccgaa ttgtgactgg 1500
cttgtctgag tctagtgtca aggtgcggtt agctgccgtc agatgtttgc acagtttatc 1560
cagatctgtg cagcagcttc gaaccagttt ccaggatcat gctgtttgga aacctttaat 1620
gaaggtttta caaaatgcac cagatgaaat cctagtggta gcatcttcca tgctgtgtaa 1680
tcttcttctt gaattttctc caagcaaaga gccaattttg gaatcaggag ccgtagagct 1740
actttgtgga ttaactcaga gtgaaaatcc tgctttacga gtgaatggaa tttgggcttt 1800
aatgaatatg gcatttcagg ctgaacaaaa aataaaagca gatattttac gaagcttgag 1860
tactgaacag ctattccggt tattatcaga ttcagatttg aatgtgctga tgaagacatt 1920
gggacttctt agaaatctcc tctccactcg tcctcatata gataaaataa tgagtactca 1980
tggaaagcaa attatgcaag ccgtcactct tattctagaa ggggaacata acattgaggt 2040
caaagagcag acactgtgca tcttagccaa catagcggat gggacaacag caaaagatct 2100
tattatgacc aatgatgata tcctacagaa aatcaagtat tacatgggcc attcacatgt 2160
taaactgcag cttgcagcca tgttttgtat atcaaacctc atatggaatg aagaggaagg 2220
ttcacaagaa cgccaggata aattacgaga catgggcatc gtagatattc tacacaaact 2280
gagtcagtca ccagattcaa acctttgtga caaggcaaag atggcactgc agcagtacct 2340
ggcatgatgg gagtgcccct gggcacctgc gagcatccca cctccttgtt taaggaagta 2400
caggaaccag cctcatttga ttccttctat ttgcacaagt caccttggac tgcagggagc 2460
tgttttgcaa aagcagttta gtaggcttag atctcaaatt catcttgaga acattttttt 2520
gaggtagtaa tttcctcaga agactcttgt gttttgtttt ggtttttttt tctgagctac 2580
tcggactctt tattagaaca atcaatcatt ttcctttgga cctacaattt ttgcctatgc 2640
tgcagccact ttgtgagtga gaatgaatat gtctgtgtga acacacaggc atgcgtgtgt 2700
atgtgcacgt acatgggcca gaatgaatgg atgtgtgtgt gtgagggata cctcagattt 2760
ttcttttctt attttgtgtg aaaatctctt ttctacagat tttccagggt ttaagcattg 2820
cttgctgtat aaaaaacttt actgaattat atacagtttg aatgaaatgt tattttaaaa 2880
acaaaacaat tgttaagtgt tccataaagg ttatgttgaa ttttggtcag atgaatattt 2940
gtaagtaaaa aatatatgca tttctgaacc tcaacttaca tagattttct ttatataaaa 3000
aaaaagaaaa aaaaagaaaa agttggctat agttggcctg attaacaggc actatacatg 3060
gtgctgtgca aaatagtaag ctagcatctt actgccgtca atattagcag gtacagagcc 3120
ttttttcatc ctaattcagt aagttctcag gcttccaagt caaatggaga agtaaagtga 3180
ggcaacagat tcaccatagg ctttttgaag catcaaaggg aaatataact actgatagtg 3240
aaagcccagc catgacccag atgggcttac ccttgacagg aactggggac caagagcctc 3300
ccaattgccc taactcctgt cattggtatt agcaatatct ggccagattt agaaccagtc 3360
atggaaactt gtgctcaaac taaaagtagg tcatcacttc ccagaaacag aataccttgt 3420
gcttcctgaa acagcatata tcactgtgaa actaaagaat attctacaga caccccctgta 3480
gaaagaaaca aagaacagga tagtttagaa agctttctgt tagctgtatt cagcaaattt 3540
catactagtt gctcaggact gctagttggt atgcttttgt aaaacgagat tgaaatttaa 3600
taaaagatgc aactaaaatc tttcctgcat gaagctaaaa gcactcaagt tgagagaggg 3660
gcaagggaat ctccccagga tgactgaaga tgacttcttt ttacagcaag tactgttagc 3720
ctttctgcac cccttagctt ggcccctgct ccaacttctg ggtcagcagc aaggtgaact 3780
caacagaagt tgctcttgac tgtttgaagg tagaagcagc ctaccttttc tc 3832

<210> 213
<211> 4140
<212> DNA
<213> Homo sapiens

<400> 213

```
ggtgccggag cgcgggcgcg gcgcactcgg agcgggatgg agcgggcgcg cgggccgcgg 60
gccgaggccg acgcgcctga ggaggaggag gaggcggggg cggccatggc tgctgtggtg 120
```

gtggcggctg cgggtggcgc gggaccggcg gtcctgcagg tggccggtct ctaccggggc 180
ctgtgcgcgg tgcgcagccg cgccctgggc ctggggctcg tgtcacccgc gcagctgcgc 240
gtcttcccag tgcgccccgg ctcgggccgg cccgaggggg gcgccgacag cagcggggtc 300
ggggccgagg ccgagctcca ggccaaccct ttctacgacc gctaccgcga caagatccag 360
ctgctgcgca ggtcagaccc agctgctttt gagtcccgcc tggagaaacg cagtgaattt 420
cggaagcagc cagtggggca ttccaggcaa ggtgatttta tcaaatgtgt ggaacagaag 480
acagatgcct tggggaaaca gtctgtgaac agaggattca ctaaggacaa gactctcagt 540
tcaatcttta acattgagat ggtaaaagaa aaaactgcag aagaaataaa acagatttgg 600
cagcaatatt ttgcagcaaa agatacagtc tacgcagtta ttcctgcaga aaagtttgat 660
ttgatctgga accgggctca gtcctgtcca acatttctat gtgctctgcc aagaagggaa 720
ggttatgagt tttttgtagg acaatggaca ggtactgaac tccacttcac tgcacttata 780
aatattcaga cccgagggga agctgcagcc agccagctga ttttatatca ctatcctgaa 840
cttaaggaag aaaagggcat agtgctgatg actgcagaaa tggattccac atttctgaat 900
gttgctgagg cacagtgcat cgccaaccaa gttcagctct tctacgctac tgatcggaaa 960
gagacctacg ggttagtgga gacctttaac ctcagaccaa atgagttcaa atatatgtct 1020
gtcatcgctg aattggagca aagcggactt ggagcagaac tgaaatgtgc ccagaaccaa 1080
aataagactt agaactgtac aggttggccc ttcacctagt tgactcagcc ctcgatagtc 1140
tagagcccac cccctcctca ggaactcaag agctcagcat ttataatgag cagttggtaa 1200
tgagttgccc tatgtgcttg tcgcaagcag tcacagagat gagccctatt acttgatatt 1260
caggaacaaa ggtacctgaa cattctgata attatctcag catacttgag gtttcctttt 1320
ttaagtgttc gaggttataa caagagacag ccaaggacct acaagacagt tgacttgatt 1380
ttgcacagtg taacagcgca gttgcattct ggccactttg accttatagc tcccaaatga 1440
tgagtttgtc atctttatga actcatgaca ggataataag cttgaagacc tgctgtagtt 1500
agatatgggc tttaatcctt cccaggcacc agtcagctga acaaaagcat aagccaaaca 1560
tcctgtttaa actgtagaat aaccagatat tcccatcagg ttaaagactt catctagatg 1620
atgcccccca gagatgcctt tagtgtaagt agctggcttg ggtatcagc aaatttcagg 1680
tatagttaga taaacaggta cagggcctgc atactattaa accatagttt gtggcacccg 1740
cttttctaac tccacctgtt agaagctatg tgtttgaagg aatgaatcag tgcagtataa 1800
ataaaattct tttgtaagga gaagattaat cctggtttgc atgatttttt taaaaacaac 1860
tctaaacatg atacgaaaaa gtggatgaaa gcaaatgttc ccagattgga tgtggggaaa 1920
atatagcaat aatttttttt ttaagtctgg cttacaatgt ttgttataca aaataatgaa 1980
atctgagtta tgtactgtcc attgtgtcag ggctatgggc tgattttatc aaaactcatc 2040
ttgggactga aaaattgttt ggaatgccag aaataagaaa gttgttctcc agagctggaa 2100
acccatcttt cgtttgtagt gtcactgttg tggctccaag ctcagtgata ggaaaggacg 2160
gtggttacac accagccttc tgaacccaag gcccccagta ttgttgtcag ctgcctttac 2220
catggcattt ctttctcttt ctttttttcc tgagatgaag tcttgctctg tcttgcccag 2280
gctggagtac agtagcgtga tctcagctcg ctgcaacctc tacctccctg gttcaagtga 2340
ttctgctgcg tcagcctcct gaggagctag gattacaggc gcatgccacc atacctggct 2400
tatttttgta ttgttgtaga gacagggttt cactttgttg gccaggctgg ctggtctcga 2460
actcctggcc tcaagtgatc caccaccttg gcctcccaaa gtgctgggat tacaggtgtg 2520
agccaccgtg cctggcctga catttcttta ttgatctaac atgctccact ctgctgctcc 2580
tgcctaagat ctggttatat gacactgaat gtggtgagtg ggaatttaag cagtattcgc 2640
agtttgtgtg tgtgtgtttt cttccttcca gaagaatttt tataggttgg gcctgtccct 2700
aagctcttta aatagggtgg acatccact attctctgag ccgtgtctat tttgttgcac 2760
ctttgagtct atgtattgag agagacagat agtatttttt taaactgggg aagctgctat 2820
cctttcacta tttctctaaa ggttgagctg ttaactaatg taaattctgg acctgcttct 2880
ggtcctggca gtttatcttt tgagaaactt gagtcttatc tgccctgcca ttttcattaa 2940
atgccttctg accttctgaa tgttttgggt cccaagaatt tttgacatca gatggggttg 3000
tttttattgg tatccagtta tgtttgcttg tctttccaga tgggcccagt tattagccat 3060
acatagtaca ttgatacacc tccaccagcg ggtgaggaaa tgatggaaaa aggagtaaga 3120
agtggccatt cgttttaatc attcctcctg gatttgtcct cagtccccaa ctgccaagta 3180
ggatgtgtcc atggataaat gtgtggggca tgactaaagt accacgtagc tgttctttat 3240
atttatttac ctagaaagat ctggcaaaga actcaaagaa aattgtacca tttaatcagt 3300
aaatttgtcc cctggtgcta gcatggtgtt atagaaagtg gacaggcttt agagttaagt 3360
gaatctgggt tcatatgtta gtgttgctat tcattagctc tatactgttg aacaaattgc 3420
ttaaactatc taattttggg gtttttttc catctaaaat agggataata atatctacct 3480
cataggatta ttgtgagaat taaattaact tcactatagt agaaaatatc aactaccatc 3540
cttttctcta cttcccttgc ccctcattaa agactaatac aagttagcat ttcagatgtg 3600
tagatcattc tttattccag ttaaaagaac aaactttatc tcatcagttc tgaaacttta 3660
agatgcagta gcatcaccta aagtgctttt aaaatgcaga ttctcaggcc tcaaccgtac 3720
accaccccc cacacacgta ctaaatcaag aatatgtgca gaaggtactg ggaatctact 3780
tgttaatatg tgctccaaat gattctgatg taggtaatta gccagccaca ctttgagaac 3840
cactgcctta tctattcttt acaaaaatgt acattgccag gtctttcttt cctgtggatg 3900
ctaactatag gatatttagg ttcctctgtt ctttgtctcc catagtggcc ccctttgcaa 3960
actccaaata cattatattt atttattctt gtgtcttttt tcccccacta gactgtgagc 4020

tccttgaggg ccaggactta tctctgttcg cagtgccaag gacatggcct ggaccataga 4080
agatactcag ttttttgttg aataaatagg taatatggat ttcaaccaaa aaaaaaaaaa 4140

<210> 214
<211> 5972
<212> DNA
<213> Homo sapiens

<400> 214

```
ctgtgtgttc agccattact ttgctcggcg ctgctcccag gcatctccga ccctcggtgc 60
tgtggggagc cccacacttg ggctcctcgc ctctcgccct cgctccccgt ccctcctccc 120
ctctctccgc cccttccccc ttttctttct cctctctttc ttcccctctc tcccttcttt 180
cggccgccgt ctcccccgcg ccctcctcgg ggcggaggga agccgtgaag gggggaggag 240
ggctcggtgt caattttttt ttgtgtggct gcggccgtag cctgtggcgg gcaagcgggg 300
agaccccggc gcagcagaac catggatggc ccgacgcggg gccatggact ccgcaaaaag 360
cggcggtcgc ggtcgcagcg agaccgggag aggcgctccc ggggcgggct gggggccggc 420
gcggccggcg gcggcggggc tggccggacc cgggcgctct cactcgcctc gtcgtcgggc 480
tccgacaagg aagacaatgg gaagcccccg tcctccgccc cgtcccggcc cagacccccg 540
cggaggaagc ggagagagtc cacctcggca gaagaggaca tcattgatgg atttgccatg 600
accagctttg tcacttttga agcgctggag aaagatgtag cacttaagcc tcaggaacgt 660
gtggagaaac gccagacgcc cctgaccaag aagaaacgag aagcacttac caatggcttg 720
tcctttcatt caaagaagag cagactcagc cacccacacc actacagctc agatcgagaa 780
aatgaccgca atctctgcca gcaccttggg aagagaaaga aaatgccgaa ggcactcaga 840
cagctcaagc caggacagaa cagctgcagg gacagtgaca gtgaaagtgc cagtggagaa 900
tccaagggct tccaccggag cagctctcgg gaaaggctca gtgatagttc agctccttcc 960
agcttgggaa caggctactt ctgtgacagt gacagtgacc aggaagagaa ggcatcagat 1020
gccagctctg aaaaactctt caacactgtt attgtaaaca aagatccgga gttaggtgtt 1080
ggcacgctac cagaacatga cagccaggat gcagggccga ttgtccccaa gatatcgggt 1140
ctagagagaa gccaggagaa gagccaggac tgttgcaaag agccaatctt tgagcctgtg 1200
gtgcttaaag acccctgccc tcaggtcgca cagccaatac cccagccgca gacggagccc 1260
caactccgag ctccttctcc ggaccctgac ttggtgcagc gcacagaggc cccacctcaa 1320
cccccacctc tgagtacaca gccaccacag ggccctcctg aggcccagct ccagcctgcc 1380
ccgcagcctc aggtgcagag gccacccagg ccacagtccc ccacccagct gctccatcag 1440
aacctcccac ctgtgcaggc ccacccctct gctcagagcc tctcccagcc attgtcagcc 1500
tacaacagca gtagcttaag cctcaacagt ttaagcagca gcagaagcag cactccagcg 1560
aagactcagc ccgcccccac tcacatctcc caccacccct ctgcctcccc gttcccccctc 1620
tccctgccca accacagccc cctgcacagc ttcacaccca ccctccagcc ccccgcacac 1680
tcacatcacc ccaatatgtt tgcccctccc actgctctgc ctcctccacc accactgaca 1740
tcaggaagtc tgcaggtggc cggacacccg gccgggagca cttactcaga gcaagacatc 1800
ttgcgacagg aactgaacac tcgtttttttg gcctctcaga gtgctgaccg cggggcttcc 1860
ctgggccctc cgccctacct gcggaccgag ttccatcagc accagcacca gcaccagcac 1920
acccaccagc acacgcacca gcacaccttc acgccgttcc cccacgccat cccacccacc 1980
gccatcatgc cgacgccagc acctcccatg tttgacaaat accctacaaa agttgaccca 2040
ttctaccggc acagtctctt ccattcctat cctcctgcag tgtcgggcat ccccccctatg 2100
atcccaccca ctggcccttt tggttcacta caaggagcat ttcagccgaa gacatccaac 2160
cctatcgatg tcgctgctcg gcctgggaca gtcccacaca ctttactcca aaaggacccg 2220
aggttgacag atcctttcag acctatgtta aggaaaccag ggaagtggtg tgctatgcat 2280
gttcacatcg cctggcagat ttaccaccac caacagaaag tcaagaaaca gatgcagtca 2340
gacccacata agctggactt tggactgaaa cctgagttcc tgagccgccc tccaggcccc 2400
agtctttttg gagccatcca ccaccccccat gacctggcac ggccttcaac tttgttctct 2460
gccgctggtg ctgcacaccc aactgggacc ccttttgggc cacctcctca tcacagcaac 2520
ttcctcaacc ctgctgccca cctagagcct tttaatcggc cgtctacatt cacaggccta 2580
gcagcagttg gtggcaatgc cttcgggggga cttggaaatc cttccgttac acccaactca 2640
atgttcggcc acaaggatgg ccccagtgtg cagaacttta gcaaccctca cgaaccctgg 2700
aaccggctgc accgaacgcc tccgtcgttc ccgacccctc cgccctggct gaagccaggg 2760
gagctggagc gcagcgcgtc cgctgcagct catgacagag atagagatgt agataaacga 2820
gactcatctg ttagtaaaga tgacaaagaa agggaaagcg tcgagaagag acactccagc 2880
caccccttcac cagcacctgt cctcccggtg aatgccctgg gacatacccg cagctccact 2940
gaacagatcc gggctcatct gaacactgag gctcgggaga aggacaaacc caaagagagg 3000
gagagagacc actcggaatc ccgcaaggac ctggccgccg acgagcacaa ggcgaaagag 3060
ggccacctgc ccgagaagga cgggcacggc cacgagggggc gcgccgcggg cgaagaggcc 3120
aagcagctgg cccgggtgcc gtctccctac gtgcggaccc cggtggtgga gagtgccagg 3180
cccaacagca cctcgagccg ggaggccgag ccgcgcaagg gtgagccggc ctacgagaac 3240
cccaagaaga gctccgaggt caaggtgaag gaggagcgga aggaagacca tgacctgcct 3300
```

ccagaggccc cgcagaccca ccgggcctcg gagccgccgc ctcccaactc ctcgtccagc 3360
gtgcacccgg ggcccctggc ctcgatgccc atgacggtgg gggtgacggg cattcacccc 3420
atgaacagca tcagcagcct ggacaggact cgcatgatga ccccccttcat gggcatcagc 3480
cccctcccgg gcggagagcg cttcccgtac ccttctttcc actgggaccc catccgggac 3540
cccttgaggg atccttaccg agaacttgac attcaccgga gagacccgct gggcagggac 3600
ttcctgctaa ggaacgaccc gctccaccgg ctctcgactc cccggctgta cgaagccgac 3660
cgctccttca gggaccggga gcctcacgac tacagccacc accaccacca ccaccaccac 3720
ccgctgtctg tggaccctcg gcgggagcac gagcggggag gccacctgga cgagcgggag 3780
cgcttgcaca tgctcagaga agactacgag cacacgcggc tccactccgt gcaccccgcc 3840
tccctcgacg gacacctccc ccaccccagc ctcatcaccc cgggactccc cagcatgcac 3900
tatccccgca tcagccccac cgcgggcaac cagaacggac tcctcaacaa gacccctccg 3960
acagcagcgc tgagcgcacc tccccgctc atctccacgc tgggggggccg cccggtctct 4020
cccagaagga cgactcctct gtccgcagag ataagggaga ggccccccttc ccacacgctg 4080
aaggatatcg aggcccgata agccgagaac aggagcaaga acgaggaaga agaaacccta 4140
ggcagacacc aggccaggct tgagagacag aactcctgca tggctcacac agactggggg 4200
ggaaagcccc accccttccc cttgtaaaaa atgtatagac tcagtgcaca ttttgaaatg 4260
ttttgtatat tatatgttga gatttttcag atcttttagc ccagtcatat gttctcacgt 4320
ctcctacttt ttgtttctcg tataaaactt tttgatttga accaaaacag tgaagatgac 4380
aacacacacc aattggatga taattgtagc gggggcggtg gggggggagaa gtccacgcca 4440
tccatcatgc aaaattcttt cagatgaggt gggaaggccg tgtacatagt tatgtaaaaa 4500
gagattgctt catgagctaa tggttcatat atgcaaaagg gtaagatgaa agctttactt 4560
tgtacaaatg taaatagata aagtaacata atacattaat acttcttaaa atgtgctatt 4620
tgcaaactta cttaatatca gtgaacacag tcggctaaag ctgtgttccc atatattgtt 4680
atagacagct aaacccttca actatgcaat gaatgttcgg gcttttcaca aaagcccgcc 4740
taactcaaag gagccttttc aaatccattt acagcatact taaggtcata ttttccctga 4800
acaagcgctt acgtgatatg actctgtttt ccttgcttgt ttttttcaa acggagaaac 4860
atcctgtttt gcaaattgga ccccaggctg gaacttagca tctgaagttg ccgcttgtgg 4920
gctctggggg aaagtgtagc cccggagagg taactgagga catgagcaac cagtgccagg 4980
gagggtggga tttgccagat gccaaaatca ggggacgggt ggtggtgtct gtcagacaca 5040
cacaggtcgc cagtgacttc acacacacct catgtgagaa ccatgccttt tttagtgtgt 5100
cctatttcat acctgtacac acttcctcgt tttgtaatga gatttactta cacccaaaca 5160
gatcctgaaa gaaagcttca agttttctca gatgatggat atgttttcac tgtattcaat 5220
aactgacgga tgtaaggtgc acgtttcctg atgtgacgca ctgtattcca gctggtgatc 5280
aagtctggga acagccgtaa caggtcaacc ttgtggagcc atcgcgagtt agagggtgaa 5340
agatggcaga aaaaaaagtc ttgtgtgtga gtgtgttttt tgagtttgca tcaatcttaa 5400
tgtctcttca taatactttt ataatacatt aagcctcttg tctacatatt tggagagaat 5460
atgactttac tagcagagaa atacaatata tcttgtctac tggactgtaa aatatatgta 5520
tgaaataaaa ttagttccat ttggtcttct agtatattaa agtgctatct gacgttgtta 5580
tcctgttttt gcaaaaaaaa aaaaaaaaaa aagttaacta cagaccattg tttctaataa 5640
gcagagagat ctattttagt agtaaactga aggtttagtt gtgagcttca gattttgtga 5700
actccagatg ttgtgcggtg tttttttttt tttttaagac aacaactaaa aaaatgcaag 5760
gaatatgtac actggaactg tagtggtagc tttcagtatt gtaaagagat tgttctatac 5820
ggaccttttt gctgtttatc ctgtatgtaa taaagtcctt tctagatcct atgtgaaaag 5880
aaaagtgaag caactgaatc ttcagcatgt tctcatcggc ggagccttct tgtgtaatgt 5940
aaactgtgcc atgttattaa aaaatgtgaa ct 5972

<210> 215
<211> 5174
<212> DNA
<213> Homo sapiens

<400> 215

```
gagtgcgctt tagctgctgc gccacgcgcg cctcgggcct gggcgcagag gatcggccgg 60
gcgcggcggg aaggaggctg cgggcagcga ggagcggctg gcgctcgagg cggcggacgg 120
caccatgtcc ccggggagcg gggtgaagag cgagtacatg aagcgctacc aggagccgcg 180
ctgggaggag tacgggccgt gctacggcga gctgctgcac taccgcctag gccgccggct 240
gctggagcag gcgcacgcgc cctggctctg ggacgactgg ggcccggccg gctcctcgga 300
ggactcggcg tcgtcagagt cgtcgggcgc cgggggcccc gcaccccggt gcgccccgcc 360
ctcgcccccg ccgcccgtag agccggcgac ccaggaggag gcggaacggc gggcgcgcgg 420
ggccccggag gagcaggacg cggaggccgg ggacgcggag gccgaggacg cggaggacgc 480
ggctctgcca gcactgccag tgaaagatgt agaagataaa cctgaacaac aaaccagaac 540
aagagagact gacaaatcac ccaccagtac tgagcctcga cagcaaccaa gtgccttatt 600
tgctagagga aacaggaaag cggtcaaaag tccccaaaga tcatcgagta aaataaaaga 660
aaacaagcat ccatttgctc tttatggctg gggagaaaaa cagaccgata caggaagcca 720
gaagactcac aacgtctgtg cgtccgctcc tgtgcatgag attcatgaat cagcattacg 780
```

agccaagaac agaagacagg tggaaaaaag gaaactggtt gctcaaaggc agcgagctca 840
ctctgtggat gtggagaaga acagaaagat gaaggcttcc tcctcagaga acccgtggat 900
gacagaatac atgaggtgct attcggcaag agcttaaaga aacacttgcg tggacagcct 960
cttttaaaaa gtgtaaatga ctgaaaggaa aaacaaaaca aaaaccatca aaagaaacgg 1020
acacaggttt aagaaaccaa ctgattatgc aaggtttttt ttagggaatt tgtaaaagat 1080
tgttttattt tgatgaatat tggtcaccta cctcggcagt agggcagaca gttgaagcca 1140
tagacatttg gttatttatg aagataattc cctaaatctt tgatattctt ataaggtttt 1200
tgttttaaag catcttaatc ttttaagata ctgacaccaa atgcctttaa atggcaacag 1260
atgcttacag ttcagtattc ttttcataag cttaggtaga gcctattatc atcttgttct 1320
aaataacttt ccagattcca tagctataag atcattccat cctacagcat aagactcgtt 1380
ttccttatat gccgttttgt ttgtgaaaga atatcaagtc aaaaatgagt gtcagcacta 1440
ctactgattc catgtataat gaaagtagaa ctttgctagt tcctgaaaat ttttaactta 1500
tttgtatttc agttcagcag catctttatg tagattgtga tatttaagaa ttatctgggc 1560
tgggcgaggg tctctttttc tctttcagtg atcatctagg cagttattat ttaatagttt 1620
aatagctcaa gtacattcca atagacgaag tgcacacaac acaatatgtg tgtacagtag 1680
tagtaaagtt tcttttgagt agtcaaagac tcactttttt tattgccttt tttttttttt 1740
ttaaagaata catactgtgg attcagtccc ttgtcacact tgaccttttg gcatacaccc 1800
actgtggact tttgcctctt ctgtaatggc tggcaatgac atttcaaact tacaatctgg 1860
aattgcactt ggtacattgg cattgcttgt tccactggga tggggaccag tgtgaagatg 1920
cctgttagat agactgccca cccctacttt ctctttttct ttatagcact taacaataac 1980
aaagtcttga tgatgtacag tattcaaact ttaggttgaa atacgttact ctttgattcc 2040
tagccagtag atcttatcta cactttaatg ggagagaatg gtggtgtgtg agtaggcaca 2100
aatttatgta aatagtgctc cttctcttta gtatgtttgc tttgggggta gaaaaatggt 2160
tttaacaaac actggtttcc atcaaatgaa tgatgtcttc tccatcctgt ggagacaaga 2220
atctgctaga aggatatgtg ctaagttcct tataagagat aatggtgctc tgcctatgcc 2280
agcttggcac ccgaagatgt gtgagtggac gtgaggctga gtattacctt agtatttttc 2340
tctgggtctt tgggaaacca tagtcaattt ttagaacata ttgctttcat tccccataaa 2400
ctcttcacac atgataactg tttaagcttt gaaaacacat actgaagtat tgtgagctta 2460
aaaaaacttt ttaaatattt gcatagtttt gaggtgaatt tgtttcctta cagatctctc 2520
ctaatcattg agatgtatat ttcaaaagag gaaattttac atgttgtcca aaacagcctt 2580
gctagtaact ggtgaatttt ggtattaact attattaaag tctttaaacg acacaggtac 2640
ctaaagatca ccttaatgtg gcaatttgtg atggtgtagc tagctgattg tgaaaactgt 2700
tcctttaaag tcgcttcttg catgttcggt gttagtcatc cagctcaggc ttgtgttgca 2760
gctgacaatc taggaaagac ggccttagag agtggtgcag gccccacact gacggactgc 2820
cttagaaacc cgacttcctc tagactttga accgccagac ttttctcttg tttagaaaac 2880
aaacttatat ttaatgtact tactacttaa aactccagac agagatataa tgtagaaggc 2940
aaatattggc caatttttc ctctttttaa gtggaagaca aatgaacggg atttttaaag 3000
tgcttttaaa gtgcatgaat ggttaataaa tcagtatgaa ttgtaagcct tcatcttaca 3060
tccaagtcct tagttggtta gggtttcttt tctttctttt ttaaagagtg tcaattacct 3120
tttgaacctg tgaaaatttg atagttgtta acagtctgat ggtcctaatt ctttcttttc 3180
attctagaaa tgaatgtggt tgtaatcatg ttcctaattc ttgggacaac ctgcaagaca 3240
gtgagacagt ttaaaaatta cctttcatgt tgaaaaagtc tgaaacagag aacccaatga 3300
tatttaaaat aaatgctaca taaaactctt tttaaaattt tgattttaac ttaattaaaa 3360
caatgtcata aatatgcttt ttgattttgt tactgctttt aatattaaag taatagaata 3420
ttgaagcaat attgtctagc actctgctgg acattaagtc cgcgggagga gaagtgaaca 3480
ggaatcgatt ctttgtcttt taactgccct tagttaggag atgttaaaat acttggcacc 3540
tctggttata tgtatgttat gtgtgttctc ccccctaaaa tttctaagca catttattca 3600
cttttaaaat gaatctttaa aagattatag ttagtagtta tagttaatat tctatttact 3660
tggaaaaatg tgaataaatg gatcttcaaa agattcattt taaaaatgaa taaatgtata 3720
ataggctata ggtgatctta cttgcgtatt aggtaggagg cacatattta taccatttca 3780
tatgtaatat ctttgtcatt gtgtttcatc gaagatcaat tgctagcaac ttgaagggta 3840
tttatacttg ggtcacttga actcagctga ctaaattgta agaacgagag caagcaagat 3900
ggctgttatt ggaagccata acttccagaa gataattctg cacaattcgt aagttaaaaa 3960
aaatctgtag ggtcttccac tatccttttt caggttgata atgctgttct gggcacacac 4020
tttgtaaatg gaatgttatg gtacagtcgc ctctcagtat ccatggggca ttggttccag 4080
gcctccctta ggatgccaaa ctccatggat actcaaaccc cttctataaa atggtgtagt 4140
atttgcatat atcctagaca catcctcctg tatgctttaa atcatctcta gattacttaa 4200
aatacctaat acaatgtaaa tgctctgtaa atagttgtta tactatattg tttagggaat 4260
aatgacaagg aaaaaaaagc ctgtacatgt tcagcacaag tgaaaccatc ctttttgccc 4320
ccaaatattt tcaatttgta tttggttgaa tccatggatg cagaactcac ggatacagag 4380
ggccgactgt actttcttta aagtgttcaa aagtattact agcaaagagg aggaggagca 4440
aagcatatat cagaagtaaa acaatttttc ttgttgactg ctttggtaaa aaacagtttg 4500
atggatagtt ttacatttca ctggactaga taaaaaatgg tgctaatatt tatgtagctt 4560
gatgctatag ttgctttggt atcaaactta atacctaacc catataagat ccttattata 4620
taattttgtg atcagtaaaa tgatatttta aagagtgatc ttaaaaatat gacctggtca 4680

ttgcacaacg tttgcatttg aaatgaattt ttgtactata gggtggatat ggagttattc 4740
agtgcaagtg tgtgcttaat atcaaaccct atgcaaggag ctatgtctag atttttggtc 4800
caaatttgcc tccttcaagc ctactagtgt gagatggaaa aaaatcgatt gctcttttaa 4860
tattatttcc attttgaaat tctcgacact tgaatgaagg cagtagaggc ctctttttgg 4920
atttctcttc taataacaaa actttattta gggaaggttt ccctgtgcta tcgtaagttt 4980
gttttgagca ctgcattcac tttaaaattc tggaggaaca aaggctgggc acataatcac 5040
aaagcccagg ccacacaata attcggggtt gtattttcta agaactatgt attgttttta 5100
gcttatagtt aatattgtat ttacttggaa aaatgtgaat aaagttaatt aacattacaa 5160
aaaaaaaaaa aaaa 5174

<210> 216
<211> 2530
<212> DNA
<213> Homo sapiens

<400> 216

aaaatggatc ctcacttctt cacttggata taataatata gactgccgac attctgggaa 60
ctgatcagtc ttccagcttc cagtgtggtg ccctccctca attgtgctga gtgtccccca 120
atccagagat attctgtaat ttcttcctca gaaagaaaca aaacaaaatt ctgatcttcc 180
cagatgaaga taatggattc tgaattaatg catagtatag taggaagcta tcataaacct 240
ccagaaagag tatttgttcc ctcattcacc cagaatgaac catctcagaa ttgccatcct 300
gcaaacttag aagttacctc tcctaagata cttcatagcc caaatagcca agctcttatt 360
ttagccttaa aaactcttca ggaaaaaatt catcgtttag agctggagag aacacaagct 420
gaagataacc tgaacattct ttccagagaa gcagcacagt ataagaaggc cttagagaat 480
gaaacaaatg agagaaatct ggcacaccag gagctgataa agcagaaaaa agatataagt 540
atacagttaa gctcagccca gtctcgttgt actcttctag agaagcaact agaatataca 600
aagagaatgg ttctcaacgt agagcgagaa aagaacatga tcctagaaca acaggcccag 660
cttcagaggg aaaaagaaca agatcagatg aagctgtatg caaaacttga aaagcttgat 720
gtcttagaaa aagagtgttt cagacttaca acaactcaga aaactgctga ggacaagatt 780
aaacatttag aagaaaaact taaggaagaa gaacatcagc gtaagctatt tcaagacaaa 840
gcttctgagc ttcaaactgg acttgaaatc agtaaaatta taatgtcttc agtttcaaat 900
ctaaagcact ccaaggaaaa gaagaaatct tcaaagaaaa ctaaatgtat aaagagacga 960
ccaccttggc aaatttgttc aaagtttgga gcactgcctt ttgtggctga aaagatgagg 1020
caacatcgtg acccacatat ccttcagaaa ccttttaacg tgactgagac tagatgtctc 1080
cccaagcctt ctagaacaac ttcctggtgt aaagctattc ctcctgactc agaaaagtcc 1140
atttccattt gtgacaattt atctgaactt ttgatggcaa tgcaagatga gctggaccaa 1200
atgagcatgg agcaccaaga actactgaaa caaatgaagg aaactgaaag tcattcagtc 1260
tgtgacgaca tagaatgtga actagagtgt ttactcaaga aaatggaaat taaaggagaa 1320
caaatctcca aactgaagaa gcatcaagac agtgtctgta aactgcagca aaaagttcaa 1380
aactcaaaga tgagtgaagc ttcaggtatt cagcaagaag acagctaccc taaaggatca 1440
aagaacataa aaaatagccc cagaaaatgt ttgactgaca ctaacctttt tcagaaaaac 1500
agcagctttc atccaatacg agttcataat cttcaaatga aattgagaag agatgatatc 1560
atgtgggaac agtaacaaaa cagcaaaact gtcaccttaa tgaactttgt cagtgagacc 1620
ttgaattgtc taaagtggtt ttaatttaat ataactttgt gacattttga atcatgtttc 1680
tagtttctat aaaacatgaa gttgcagtat ttaaaaatta atgcctaatg acctggtggg 1740
ttccaaataa taaattaatt gctttttat ttttcttatt gattgaagcc cgtaacctca 1800
tcttgtctta gaaacattgt tggctttgca gatatcttaa gctaaattta agaaattgta 1860
ctagattgac aatattcaaa attgagttaa atccaagcta caagtaccat tcattccact 1920
tttcatttag taggtttgga accttaaaaa ccaatgttca atgaacagag ttcagaaaga 1980
tcatttagct ttcctggtgc cttctttcta ttttgtttta gagctgtgaa atgtttttct 2040
cttttggaaa aggaaaaact taatagttac acagttttgt gaagaagcct ttaatccaag 2100
ttttatgaga cagtaggaac ctatagctac ttaattttta ggagacagta attcagttgc 2160
agaagctttc ctctgctatt cccattctct tttacaaaac tagttttttt taaaaaatca 2220
atgatcaatt ttatcttact actttataac ttttgctgac ttttattctt tgcattgtat 2280
gtaatgtcca tcagtataaa ttgagactgt gaatttctag gacccacaaa agtggtattt 2340
tttttttgta caagaaagta tagaggaaga ggaagctggg cattaaatta cctcatccag 2400
cagaaattca cggtagtatg gtatattttt atttttata ttttaaaatc aataatgtca 2460
aatgtcattg tgtatgcatg ctttataata aatttacact tgatcatttt tctggaaaaa 2520
aaaaaaaaaa 2530

<210> 217
<211> 10540
<212> DNA
<213> Homo sapiens

<400> 217

```
aggagccgga ggaggagccg ccgccgccgt tgacccggcc gccggccggg agctgggaga 60
gatgcggagc ccggccaccg gcgtccccct cccaacgccg ccgccgccgc tgctgctgct 120
gttgctgctg ctgctgccgc cgccactatt gggagaccaa gtggggccct gtcgttcctt 180
ggggtccagg ggacgaggct cttcgggggc ctgcgccccc atgggctggc tctgtccatc 240
ctcagcgtcg aacctctggc tctacaccag ccgctgcagg gatgcgggca ctgagctgac 300
tggccacctg gtaccccacc acgatggcct gagggtttgg tgtccagaat ccgaggccca 360
tattccccta ccaccagctc ctgaaggctg cccctggagc tgtcgcctcc tgggcattgg 420
aggccacctt tccccacagg gcaagctcac actgcccgag gagcacccgt gcttaaaggc 480
tccacggctc agatgccagt cctgcaagct ggcacaggcc cccgggctca gggcagggga 540
aaggtcacca gaagagtccc tgggtgggcg tcggaaaagg aatgtaaata cagcccccca 600
gttccagccc cccagctacc aggccacagt gccggagaac cagccagcag gcacccctgt 660
tgcatccctg agggccatcg acccggacga gggtgaggca ggtcgactgg agtacaccat 720
ggatgccctc tttgatagcc gctccaacca gttcttctcc ctggacccag tcactggtgc 780
agtaaccaca gccgaggagc tggatcgtga gaccaagagc acccacgtct tcagggtcac 840
ggcgcaggac cacggcatgc cccgacgaag tgccctggct acactcacca tcttggttac 900
tgacaccaat gaccatgacc ctgtgttcga gcagcaggag tacaaggaga gcctcaggga 960
gaacctggag gttggctatg aggtgctcac tgtcagggcc acggatggtg atgcccctcc 1020
caatgccaat attctgtacc gcctgctgga ggggtctggg ggcagcccct ctgaagtctt 1080
tgagatcgac cctcgctctg ggtgatccg aacccgtggc cctgtggatc gggaagaggt 1140
ggaatcctac cagctgacgg tagaggcaag tgaccagggt cgggacccgg gtcctcggag 1200
taccacagcc gctgttttcc tttctgtgga ggatgacaat gataatgccc cccagtttag 1260
tgagaagcgc tatgtggtcc aggtgaggga ggatgtgact ccaggggccc cagtactccg 1320
agtcacagcc tcggatcgag acaaggggag caatgccgtg gtgcactata gcatcatgag 1380
tggcaatgct cggggacagt tttatctgga tgcccagact ggagctctgg atgtggtgag 1440
ccctcttgac tatgagacga ccaaggagta caccctacgg gtgcgagcac aggatggtgg 1500
ccgtccccca ctctctaatg tctctggctt ggtgacagta caggtcctgg atatcaacga 1560
caatgccccc atcttcgtca gcacccccttt ccaggctact gtcctggaga gtgtcccctt 1620
aggctacctg gttctccatg tccaggctat cgacgctgat gctggtgaca atgcccgcct 1680
ggaataccgc cttgctgggg tgggacatga cttccccttc accatcaaca atggcacagg 1740
ctggatctct gtggctgctg aactggaccg ggaggaagtt gatttctaca gctttggggt 1800
agaagctcga gaccatggca ctccagcact cactgcctcg gccagtgtca gcgtgactgt 1860
cctggatgtc aacgacaaca atccaacctt tacccaacca gagtacacag tgcggctcaa 1920
tgaggatgca gctgtgggca ccagcgtggt gacggtgtca gctgtggacc gtgatgctca 1980
tagtgtcatc acctaccaga tcaccagtgg caatactcga aaccgcttct ccatcaccag 2040
ccaaagtggt ggtgggctgg tatcccttgc cctgccactg gactacaaac ttgagcggca 2100
gtatgtgttg gctgttaccg cctccgatgg cactcggcag gacacggcac agattgtggt 2160
gaatgtcacc gacgccaaca cccatcgtcc tgtctttcag agctcccact atacagtgaa 2220
tgttaatgag gaccggccgg caggcaccac ggtggtgctg atcagcgcca cggatgagga 2280
cacaggtgag aatgcccgca tcacctactt catggaggac agcatccccc agttccgcat 2340
cgatgcagac acgggggctg tcaccaccca ggctgagctg gactatgaag accaagtgtc 2400
ttacaccctg gccattactg ctcgggacaa tggcattccc cagaagtccg acaccaccta 2460
cctggagatc ctggtgaacg acgtgaatga caatgcccct cagttcctgc gagactccta 2520
ccagggcagt gtctatgagg atgtgccacc cttcactagc gtcctgcaga tctcagccac 2580
tgatcgtgat tctggactta atggcagggt cttctacacc ttccaaggag gcgacgatgg 2640
agacggtgac tttattgttg agtccacgtc aggcatcgtg cgaacgctac ggaggctgga 2700
tcgagagaac gtggcccagt atgtcttgcg ggcatatgca gtggacaagg ggatgccccc 2760
agcccgcaca cctatggaag tgacagtcac tgtgttggat gtgaatgaca atcccccctgt 2820
ctttgagcag gatgagtttg atgtgtttgt ggaagagaac agccccattg ggctagccgt 2880
ggcccgggtc acagccactg accccgatga aggcaccaat gcccagatta tgtaccagat 2940
tgtggagggc aacatccctg aggtctttca gctggacatc ttctccgggg agctgacagc 3000
cctggtagac ttagactacg aggaccggcc tgagtacgtc ctggtcatcc aggccacgtc 3060
agctcctctg gtgagccggg ctacagtcca cgtccgcctc cttgaccgca atgacaaccc 3120
accagtgctg ggcaactttg agatcctttt caacaactat gtcaccaatc gctcaagcag 3180
cttccctggg ggtgccattg gccgagtacc tgcccatgac cctgatatct cagatagtct 3240
gacttacagc tttgagcggg gaaatgaact cagcctggtc ctgctcaatg cctccacggg 3300
tgagctgaag ctaagccgcg cactggacaa caaccggcct ctggaggcca tcatgagcgt 3360
gctggtgtca gacggcgtac acagcgtgac cgcccagtgc gcgctgcgtg tgaccatcat 3420
caccgatgag atgctcaccc acagcatcac gctgcgcctg gaggacatgt cacccgagcg 3480
cttcctgtca ccactgctag gcctcttcat ccaggcggtg gccgccacgc tggccacgcc 3540
accggaccac gtggtggtct tcaacgtaca gcgggacacc gacgcccccg ggggccacat 3600
cctcaacgtg agcctgtcgg tgggccagcc gccagggccc gggggcgggc cgcccttcct 3660
gccctctgag gacctgcagg agcgcctata cctcaaccgc agcctgctga cggccatctc 3720
ggcacagcgc gtgctgccct tcgacgacaa catctgcctg cgggagccct gcgagaacta 3780
catgcgctgc gtgtcggtgc tgcgcttcga ctcctccgcg cccttcatcg cctcctcctc 3840
```

cgtgctcttc cggcccatcc accccgtcgg agggctgcgc tgccgctgcc cgcccggctt 3900
cacgggtgac tactgcgaga ccgaggtgga cctctgctac tcgcggccct gtggccccca 3960
cgggcgctgc cgcagccgcg agggcggcta cacctgcctc tgtcgtgatg gctacacggg 4020
tgagcactgt gaggtgagtg ctcgctcagg ccgttgcacc ccgggtgtct gcaagaatgg 4080
gggcacctgt gtcaacctgc tggtgggcgg tttcaagtgc gattgcccat ctggagactt 4140
cgagaagccc tactgccagg tgaccacgcg cagcttcccc gcccactcct tcatcacctt 4200
tcgcggcctg cgccagcgtt tccacttcac cctggccctc tcgtttgcca caaaggagcg 4260
cgacgggttg ctgttgtaca atgggcgttt caatgagaag catgactttg tggccctcga 4320
ggtgatccag gagcaggtcc agctcacctt ctctgcaggg gagtcaacca ccacggtgtc 4380
cccattcgtg cccggaggag tcagtgatgg ccagtggcat acggtgcagc tgaaatacta 4440
caataagcca ctgttgggtc agacagggct cccacagggc ccatcagagc agaaggtggc 4500
tgtggtgacc gtggatggct gtgacacagg agtggccttg cgcttcggat ctgtcctggg 4560
caactactcc tgtgctgccc agggcaccca gggtggcagc aagaagtctc tggatctgac 4620
ggggcccctg ctactaggcg gggtgcctga cctgcccgag agcttcccag tccgaatgcg 4680
gcagttcgtg ggctgcatgc ggaacctgca ggtggacagc cggcacatag acatggctga 4740
cttcattgcc aacaatggca ccgtgcctgg ctgccctgcc aagaagaacg tgtgtgacag 4800
caacacttgc cacaatgggg gcacttgcgt gaaccagtgg gacgcgttca gctgcgagtg 4860
ccccctgggc tttgggggca agagctgcgc ccaggaaatg gccaatccac agcacttcct 4920
gggcagcagc ctggtggcct ggcatggcct ctcgctgccc atctcccaac cctggtacct 4980
cagcctcatg ttccgcacgc gccaggccga cggtgtcctg ctgcaggcca tcaccagggg 5040
gcgcagcacc atcaccctac agctacgaga gggccacgtg atgctgagcg tggagggcac 5100
agggcttcag gcctcctctc tccgtctgga gccaggccgg gccaatgacg gtgactggca 5160
ccatgcacag ctggcactgg gagccagcgg ggggcccggc catgccattc tgtccttcga 5220
ttatgggcag cagagagcag agggcaacct gggcccccgg ctgcatggtc tgcacctgag 5280
caacataaca gtgggcggaa tacctgggcc agccggcggt gtggcccgtg gctttcgggg 5340
ctgtttgcag ggtgtgcggg tgagcgatac gccggagggg gttaacagcc tggatcccag 5400
ccatggggag agcatcaacg tggagcaagg ctgtagcctg cctgaccctt gtgactcaaa 5460
cccgtgtcct gctaacagct attgcagcaa cgactgggac agctattcct gcagctgtga 5520
tccaggttac tatggtgaca actgtactaa tgtgtgtgac ctgaacccgt gtgagcacca 5580
gtctgtgtgt acccgcaagc ccagtgcccc ccatggctat acctgcgagt gtcccccaaa 5640
ttaccttggg ccatactgtg agaccaggat tgaccagcct tgtccccgtg gctggtgggg 5700
acatcccaca tgtggcccat gcaactgtga tgtcagcaaa ggctttgacc cagactgcaa 5760
caagacaagc ggcgagtgcc actgcaagga gaaccactac cggcccccag gcagccccac 5820
ctgcctcttg tgtgactgct accccacagg ctccttgtcc agagtctgtg accctgagga 5880
tggccagtgt ccatgcaagc caggtgtcat cgggcgtcag tgtgaccgct gtgacaaccc 5940
ttttgctgag gtcaccacca atggctgtga agtgaattat gacagctgcc cacgagcgat 6000
tgaggctggg atctggtggc cccgtacccg cttcgggctg cctgctgctg ctccctgtcc 6060
caaaggctcc tttgggactg ctgtgcgcca ctgtgatgag cacagggggt ggctcccccc 6120
aaacctcttc aactgcacgt ccatcacctt ctcagaactg aagggcttcg ctgagcggct 6180
acagcggaat gagtcaggcc tagactcagg gcgctcccag cagctagccc tgctcctgcg 6240
caacgccacg cagcacacag ctggctactt cggcagcgac gtcaaggtgg cctaccagct 6300
ggccacgcgg ctgctggccc acgagagcac ccagcggggc tttgggctgt ctgccacaca 6360
ggacgtgcac ttcactgaga atctgctgcg ggtgggcagc gccctcctgg acacagccaa 6420
caagcggcac tgggagctga tccagcagac agagggtggc accgcctggc tgctccagca 6480
ctatgaggcc tacgccagtg ccctggccca gaacatgcgg cacacctacc taagcccctt 6540
caccatcgtc acgcccaaca ttgtcatctc cgtagtgcgc ttggacaaag ggaactttgc 6600
tggggccaag ctgccccgct acgaggccct gcgtggggag cagcccccgg accttgagac 6660
aacagtcatt ctgcctgagt ctgtcttcag agagacgccc cccgtggtca ggcccgcagg 6720
ccccggagag gcccaggagc cagaggagct ggcacggcga cagcgacggc acccggagct 6780
gagccagggt gaggctgtgg ccagcgtcat catctaccgc accctggccg ggctactgcc 6840
tcataactat gaccctgaca agcgcagctt gagagtcccc aaacgcccga tcatcaacac 6900
acccgtggtg agcatcagcg tccatgatga tgaggagctt ctgccccggg ccctggacaa 6960
acccgtcacg gtgcagttcc gcctgctgga gacagaggag cggaccaagc ccatctgtgt 7020
cttctggaac cattcaatcc tggtcagtgg cacaggtggc tggtcggcca gaggctgtga 7080
agtcgtcttc cgcaatgaga gccacgtcag ctgccagtgc aaccacatga cgagcttcgc 7140
tgtgctcatg gacgtttctc ggcgggagaa tggggagatc ctgccactga agacactgac 7200
atacgtggct ctaggtgtca ccttggctgc ccttctgctc accttcttct tcctcactct 7260
cttgcgtatc ctgcgctcca accaacacgg catccgacgt aacctgacag ctgccctggg 7320
cctggctcag ctggtcttcc tcctgggaat caaccaggct gacctccctt ttgcctgcac 7380
agtcattgcc atcctgctgc acttcctgta cctctgcacc ttttcctggg ctctgctgga 7440
ggccttgcac ctgtaccggg cactcactga ggtgcgcgat gtcaacaccg gccccatgcg 7500
cttctactac atgctgggct ggggcgtgcc tgccttcatc acagggctag ccgtgggcct 7560
ggaccccgag ggctacggga accctgactt ctgctggctc tccatctatg acacgctcat 7620
ctggagtttt gctggcccgg tggcctttgc cgtctcgatg agtgtcttcc tgtacatcct 7680
ggcggcccgg gcctcctgtg ctgcccagcg gcagggcttt gagaagaaag gtcctgtctc 7740

```
gggcctgcag ccctccttcg ccgtcctcct gctgctgagc gccacgtggc tgctggcact 7800
gctctctgtc aacagcgaca ccctcctctt ccactacctc tttgctacct gcaattgcat 7860
ccagggcccc ttcatcttcc tctcctatgt ggtgcttagc aaggaggtcc ggaaagcact 7920
caagcttgcc tgcagccgca agcccagccc tgaccctgct ctgaccacca agtccaccct 7980
gacctcgtcc tacaactgcc ccagccccta cgcagatggg cggctgtacc agccctacgg 8040
agactcggcc ggctctctgc acagcaccag tcgctcgggc aagagtcagc ccagctacat 8100
cccccttcttg ctgagggagg agtccgcact gaaccctggc caagggcccc ctggcctggg 8160
ggatccaggc agcctgttcc tggaaggtca agaccagcag catgatcctg acacggactc 8220
cgacagtgac ctgtccttag aagacgacca gagtggctcc tatgcctcta cccactcatc 8280
agacagtgag gaggaagaag aggaggagga agaggaggcc gccttccctg gagagcaggg 8340
ctgggatagc ctgctggggc ctggagcaga gagactgccc ctgcacagta ctcccaagga 8400
tggggggccca gggcctggca aggcccccctg gccaggagac tttgggacca cagcaaaaga 8460
gagtagtggc aacggggccc ctgaggagcg gctgcgggag aatggagatg ccctgtctcg 8520
agaggggtcc ctaggccccc ttccaggctc ttctgcccag cctcacaaag gcatccttaa 8580
gaagaagtgt ctgcccacca tcagcgagaa gagcagcctc ctgcggctcc ccctggagca 8640
atgcacaggg tcttcccggg gctcctccgc tagtgagggc agccggggag gcccccctcc 8700
ccgcccaccg ccccggcaga gcctccagga gcagctgaac ggggtcatgc ccatcgccat 8760
gagcatcaag gcaggcacgg tggatgagga ctcgtcaggc tccgaatttc tcttctttaa 8820
cttcctgcat taaccctggg ccgtggttcc tacgcccgag gctcccttcc cttccccagc 8880
cgcactcatg ccctgctcct gtcttgtgct ttatcctgcc ccgctcccca tcgcctgccc 8940
gcagcagcga cgaaacgtcc atctgaggag cctgggcctt gccgggaggg gtactcaccc 9000
cacctaaggc catctagtgc caactccccc cccaccattc ccctcactgc actttggacc 9060
cctgggggcca acatctccaa gacaaagttt ttcagaaaag aggaaaaaaa gaatttaaaa 9120
aaggatctcc actcttcatg acttcaggga ttcatttttt ttatacgctg gaaattgact 9180
cccctttccc ttcccaaaga ggataggacc tcccaggatg cttcccagcc tctcctcagt 9240
ttcccatctg ctgtgcctct gggaggagag ggactcctgg ggggcctgcc cctcatacgc 9300
catcaccaaa aggaaaggac aaagccacac gcagccaggg cttcacaccc ttcaggctgc 9360
acccgggcag gcctcagaac ggtgaggggc cagggcaaag ggtgtgcctc gtcctgcccg 9420
cactgcctct cccaggaact ggaaaagccc tgtccggtga gggggcagaa ggactcagcg 9480
cccctggacc cccaaatgct gcatgaacac attttcaggg gagcctgtgc cccccaggcgg 9540
gggtcgggca gccccagccc ctctcctttt cctggactct ggccgtgcgc ggcagcccag 9600
gtgtttgctc agttgctgac ccaaaagtgc ttcattttc gtgcccgccc cgcgccccgg 9660
gcaggccagt catgtgttaa gttgcgcttc tttgctgtga tgtgggtggg ggaggaagag 9720
taaacacagt gctggctcgg ctgccctgag ggtgctcaat caagcacagg tttcaagtct 9780
gggttctggt gtccactcac ccaccccacc ccccaaaatc agacaaatgc tactttgtct 9840
aacctgctgt ggcctctgag acatgttcta tttttaaccc cttcttggaa ttggctctct 9900
tcttcaaagg accaggtcct gttcctcttt ctccccgact ccaccccagc tccctgtgaa 9960
gagagagtta atatatttgt tttatttatt tgctttttgt gttgggatgg gttcgtgtcc 10020
agtcccgggg gtctgatatg gccatcacag gctgggtgtt cccagcagcc ctggcttggg 10080
ggcttgacgc ccttcccctt gccccaggcc atcatctccc cacctctcct cccctctcct 10140
cagttttgcc gactgctttt catctgagtc accatttact ccaagcatgt attccagact 10200
tgtcactgac tttccttctg gagcaggtgg ctagaaaaag aggctgtggg caggaaagaa 10260
aggctcctgt ttctcatttg tgaggccagc ctctggcttt tctgccgtgg attctccccc 10320
gtcttctccc ctcagcaatt cctgcaaagg gttaaaaatt taactggttt ttactactga 10380
tgacttgatt taaaaaaaat acaaagatgc tggatgctaa cttggtacta accatcagat 10440
tgtacagttt ggttgttgct gtaaataggg tagcgttttg ttgttgttgt tttttcatgc 10500
cccatactac tgaataaact agttctgtgc gggtacagca                       10540
```

<210> 218
<211> 7102
<212> DNA
<213> Homo sapiens

<400> 218

```
ggcctgacta gtcgtcgccg tcttcgcggc tgctatgacc agcgggcgag gcgcccttcg 60
cagctccgcg caacgctagt cgcggccgtg cgcccgccgt agccgtctgc gtcccagcgc 120
ggccgctccc gcggccccct cggctttgcc agcgccgcct gcgaggcgga ggcaggatga 180
agatgactgt ggatttcgag gagtgtctga aggactcgcc ccgcttcagg gcagctttgg 240
aagaagtaga aggtgatgtg gcagaattgg aactaaaact tgataagctt gtgaaacttt 300
gtattgcaat gattgatact ggaaaagcct tttgtgttgc aaataaacag ttcatgaatg 360
ggattcgaga cctggctcag tattctagta atgatgctgt cgttgagaca agtttgacca 420
agttttctga cagtcttcaa gaaatgataa attttcacac aatcctgttt gaccaaactc 480
agagatcaat taaggcacag cttcagaact ttgttaaaga agatcttaga aaattcaaag 540
atgccaagaa gcaattcgaa aaagtcagtg aagaaaaaga aaatgcgtta gtaaaaaatg 600
cccaagtaca aagaaacaaa caacatgaag ttgaagaagc caccaacatt ctgacagcaa 660
```

```
caagaaaatg tttccgacac atagccctcg attatgtgct tcagattaat gttcttcaat 720
caaaaaggag atcagaaatc ctaaaatcaa tgttgtcatt tatgtatgcc catttggcct 780
tctttcatca aggatatgat ctgtttagtg aacttggacc ctacatgaag gatcttggtg 840
cacagttgga tcgactggtt gtggatgcag caaaggagaa aagagaaatg gagcaaaaac 900
attccaccat tcaacaaaag gatttctcca gtgatgattc taagttagaa tataacgtag 960
atgctgcaaa tggcatagtt atggaaggat atctgttcaa acgagccagc aatgccttca 1020
aaacttggaa caggcgctgg ttttcaatac agaataatca gttggtttac cagaaaaaat 1080
ttaaggataa tccgactgtg gtagttgaag acctcaggct ttgcacagtg aaacattgtg 1140
aagacataga gcgacgattc tgctttgagg tggtctcgcc aacaaaaagt tgcatgctcc 1200
aggcagattc cgaaaagctg cgccaggcat ggattaaggc tgttcagacc agtattgcta 1260
ctgcttatag agagaagggt gatgaatcag agaagctgga taagaaatca tctccatcca 1320
caggaagcct agattctgga aatgagtcca aagagaaatt attgaaagga gaaagtgcgc 1380
ttcagcgggt ccagtgtatc cctggcaatg ccagctgttg tgactgtggc ctggcagatc 1440
cacggtgggc cagcatcaac ctgggcatca ccttgtgtat cgagtgctcc ggaattcacc 1500
ggagccttgg ggttcatttt tcaaaagtac gatctttaac tttagacacc tgggagccag 1560
aacttttaaa gcttatgtgt gagttgggga atgatgttat aaatcgagtt tatgaagcta 1620
atgtggaaaa aatgggaata aagaaacccc aaccaggaca aagacaggag aaggaggcat 1680
atatcagagc aaaatatgtg gagaggaaat ttgtggataa atattctata tcattatcac 1740
ctcctgagca gcaaaaaaag tttgtctcta aaagttctga agaaaagagg ctgagcattt 1800
ctaaatttgg gccaggggac caagtcagag catctgccca aagttcagtc agaagtaatg 1860
acagtgaaat tcagcagagc tctgatgatg gaagagaatc tttaccctcc acggtgtcag 1920
ccaatagttt atatgagcct gaaggagaaa ggcaagattc ttctatgttt cttgactcga 1980
aacatcttaa tccaggactt cagctttata gggcgtcata tgaaaaaaac cttcctaaaa 2040
tggctgaggc tttggctcat ggtgcagacg tgaactgggc caattcagag gaaaacaaag 2100
cgacgccact tattcaggct gtattagggg gctctttggt gacgtgtgag ttcctcctac 2160
agaatggtgc taatgtcaac caaagagatg tccaagggcg gggaccattg caccatgcca 2220
ccgtcttagg gcacacaggg caggtatgtt tattcctaaa acgaggtgcc aatcaacatg 2280
ccactgatga agaagggaaa gacccttga gcatagctgt ggaagcagcc aatgctgata 2340
tagtcacctt gttacgttta gcaagaatga atgaagagat gcgggaatca gaaggacttt 2400
atggacagcc aggtgatgaa acttatcagg acatatttcg tgatttttcc caaatggcat 2460
ccaataatcc agagaaacta aatcgtttcc agcaagattc acagaaattc tgaatttttt 2520
taaaaatggg aaaatatgaa atctggtgac cccctaatgt atacagctaa aaattcacaa 2580
tttttttact gctttaattc tacttaattt tggtagatat tgaatggctt ggaaaagagg 2640
tacccattca tatatttata aattaaaaca tagttatcta gtagtaggag aggaacctag 2700
tttaggacct cttttattaa aaaagcctat ttaaagggct atttctgtag acagaagttc 2760
agtctagatt taggccatct ctaggttaca agatgatagg aggttagaca aaagtgattc 2820
ttttgccaat gggtggtggc attgattttt ttcctccatg catcttgccc aggatcactc 2880
agtcaagcat gtcttcatca agtggaagcc aactgtcctg cctgcctcaa gaatatgtgt 2940
tttctttagc ttccctgagg atttttggtg gttcagaaat aggtgtgtgt gttgatgtgt 3000
ttttggtaac ctagctttca tacacataat atatgattta ttagaactag agaatgtgtt 3060
ttccccgttt gttcataaat gcggtcggtt atacttctta ggtgacaggt tattgtccag 3120
tactttaatg tatgtatgta taatggctgt acaggtagac cataattcag tcctttagat 3180
ttattttatg cagcaaaaag aagctctttt ttttttaat cacaaaaaaa aaccccagtt 3240
caaaatgtgt agttcttcat tgttgggttg attccccaaa accttgacag atgtatcgat 3300
ggaataaaag tatgttttaa aggttacttg aatgtcacaa aattatgcct tcatcaaaac 3360
tttaaaggaa aaatcaattt ataaaatcat gtaggccatt tttatatttg aaatttgtcc 3420
ttttaaagat agttttgctg ttgctcctag tgaaagaaaa atctctgtac aaaggagaaa 3480
aagaacccca cttattctta gttaatacta aaatatagca ataaattgct gtttattttt 3540
tctacatcta ttttgtgtac ttgtacacag tgtacattct tttaaattat atcgtgtata 3600
tagctttagt attcggttac atgattcaca gacccctgaa ttccaatatt atggataaac 3660
attaccattt atatgctaca aattagtctg attccatctt tgaaaatgtc ttaaagtata 3720
aaataattaa tttgcttttt tttttttttt tttcccgaga tggcgtcttg ctgtgtcacc 3780
caggctggag tgcagtggca cgatctcagc tcactgcagc ctcctcctct tgggttcaag 3840
caattctcct gcctcagcct cccgagtagc tgggactaca ggcacccgcc accattccga 3900
gctaatttct atatttttag taaagacggg tttcaccatt ttggccaggt tggtctcaaa 3960
ctcctgacct caggtgatca gcctgcttta gcctcccaga gtgctgggat tacaggcgtg 4020
agccaccgca cccagcctaa tttgcctttt gattccttcc cattgttgtc aaaaattaag 4080
tctattcttc ttttagaaat tatatgtaaa tgatattatt gatactcttt ctaaataaga 4140
ggttttaaaa tatataaaaa ctttatgatc caattataga taaattttct aacatttccc 4200
agtcactagg gttttataag ttgattaaac atcaaatcag aatctagcca agcaggaatt 4260
atattgaaat aagttcacat tacagaaaag gttccataat ttggccaaac tgcatgcatg 4320
taatgcttat ttagtaatgg tcactgtatg ttagataagg ataaaaatat atttgtgaat 4380
tacttctgag gaaatgctaa cttccacagc attgtattta attataacaa taacaggaac 4440
attaaatctt tatctttgct gtttcacctc aaagtcatag atcatctggc tttttaggca 4500
ccttcactaa aggcaaaaat ctgatttgta gtttttatta gcatgtacct tgaaattatt 4560
```

```
tgaccaagaa gctcaaagaa aagaagtacg ttaaagaaaa agaaaaaacc tcacttttcc 4620
tttatagtgt cccttcctgt tttaattcct aaagatcctg acttttatct gaggaagtct 4680
aggtagtttt ccttttggat ctggccattg gtgcgtgttt cattcagatc acctctggaa 4740
tctctgtggt tgtgaaggca attccttatg agactcttgc ctcctgtggc ttacgtgatg 4800
aattgttaat atggcagtaa ttaattagta tctacttaaa tttagtggtc agaaatgtat 4860
ttgaagtgcc ttaaagatga tcatggttca gaatatcttc agagaaagca aaatctttaa 4920
tattgcaggg agcctagcaa tacaaataca catttctgtg gcttgatttc ttgaatatgc 4980
tgagacccaa tttggactac atttggcaga aatcatggag taaacagcaa ggctttaaat 5040
taaattaata gttacaaggt aggttggctc ttaggtgaat ttagccatgt caactaatag 5100
cttttatatg gtatgtaaca aatctgaatt atgttatgct gtgaattatt cagtgcatta 5160
taagaaatct tagttaatca tgcttttggg cttcattagt caatgtgatt ggccttttat 5220
tttctttcca taacttatct gctattatta gtttcttatt ccttattatt aatctgttag 5280
ctcattatca ttagcttact aacttgaaag ttagattaat atcaaaataa gaatagcagc 5340
actatgaaga gttaataaaa tgcaatttca tgttagagct agaataaaag taattgagca 5400
gtcatttgca aatgcttcat tataacattt atgtgcttaa gatttctaag gttttaaaaa 5460
gttaccttta atacaatcct tagaaactca gaaatttcct gatattcatt agattatggt 5520
gtttgcctaa tttataccgg ttttcactgt atattttaat gaactttgat tggttacttt 5580
tccagacata cagatttaat ttggaatatg aactttggtt cttcatgagt gcattttgaa 5640
aaagaaaaaa agtacacatt cagaatctgg ccaatttaag tttgtatttt aaaaatattt 5700
tatgcatgtt aagtatggtc gcagtaaatg aaattgtgtt taagactaaa tttactaccg 5760
ttttgaacag acattcccag atcattctta tattgtcaga taacacttgt gtataatatc 5820
actttggacc atggacatga gctggaaaaa tgatgtattt ttgtagattg tatgtccaga 5880
atgactttag aaccgcatga tacggtggaa ggagcactgg actagtcgtc actacccttt 5940
ttggattcag ggctcctcac aattaaaatg agtgtaatga aacaaggtga aaatatagaa 6000
gcatcccttt gtatactgtt ttgctactta cagtgtactt ggcattgctt tatctcactg 6060
gattctcatg gtaggatttc tgagatctta atctaagctc caaagttgtc tactttttttg 6120
atcctagggt gctccttttg ttttacagag cagggtcact tgatttgcta gctggtggca 6180
gaattggcac cattacccag gtctgactga ccaccagtca gaggcacttt atttgtatca 6240
tgaaatgatt tgaaatcatt gtaaagcagc gaagtctgat aatgaatgcc agctttcctt 6300
gtgctttgat aacaaagact ccaaatattc tggagaacct ggataaaagt ttgaagggct 6360
agattgggat ttgaagacaa aattgtagga aatcttacat ttttgcaata acaaacatta 6420
atgaaagcaa aacattataa aagtaatttt aattcaccac atacttatca atttcttgat 6480
gcttccaaat gacatctaca gatatggttt tgtggacatc tttttctgtt tacataaatt 6540
tatccactta aaatgtgata atgtggagac aaagcaagat ttgattacat aaacttttct 6600
ctgcattggt cttttcacta tcttatttgc taacttttct tatttttctt caacctcccc 6660
accctccatt ttttggatat taaacaggga aagcttgcta cattataaag attgttggca 6720
ccatttattt tacacaaagg ctaaaggtta acttttggaa atgatgagct acttttatat 6780
atgtgtttac tcatgctttg tgatatttct ggatcattcc agtcataaag acttattaca 6840
tgtactattt cttgctacct gtgaaaaggt atattttaaa gaatgtataa ccaacagtcc 6900
atttactttt tattagtatg tagaggattt gtatctaatt catgaatgta gttttacagt 6960
agtttgtcat tgtaaatgga gcaaagtaca ttatcttcta aaatgtacat tatgtaagaa 7020
ttgtaaatat acttaagtaa tttgtatgcc aaaaaattac aataagtcaa taaagatctc 7080
acctctgaaa aaaaaaaaaa aa                                          7102
```

<210> 219
<211> 2591
<212> DNA
<213> Homo sapiens

<400> 219

gccaagggca ctattggcca gttccgttca acgaagtggt tgcttttttt agttccggca 60
atgagttgcg ccggggcggc ggcggctccc cgcctttggc ggctgcgccc gggggcccgg 120
cggtccctct cagcttatgg aagaagaacc agtgtcagat ttcgcagttc aggaatgact 180
ttagacaata tcagtcgggc agctgtggat cgaataatcc gggtggatca tgcaggcgaa 240
tatggagcaa accgcatcta tgccgggcag atggctgtcc tgggtcggac cagcgtcggg 300
ccagtcattc agaaaatgtg ggatcaagaa aaggaccatt tgaaaaagtt caatgagttg 360
atggttacgt tcagggtccg gccaacagtt ctgatgccct tgtggaacgt gctggggttt 420
gcactggggg cggggaccgc cttgctcggg aaggaaggtg ccatggcctg caccgtggcg 480
gtggaagaga gcatagcaca tcactacaac aaccagatca ggacgctgat ggaggaggac 540
cctgaaaaat acgaggaact tcttcagctg ataaagaaat ttcgggatga agagcttgag 600
caccatgaca taggcctcga ccatgatgca gaattggctc cagcctatgc cgtcctgaag 660
agcattatcc aggccggatg cagagtggcg atatatttat cagaaagatt ataaagtgtg 720
tccagttttg cctgtctata aaagatgata gtaatttacc aagtgacatt tgcagagaaa 780
caggtgtaca gttatcgttg tacttttgta caatgtgaat tttgttaata aattataagg 840
tttgtttttt ttttttaaa ctctgcagtg ttgatttttc tctgggttgt tttttctgcc 900

atgagaccaa caggtcacca gccttgttca agttacagca aacgaagctg ggccttgttt 960
ggtctcatac ttaattttct tttatataca tgtttttctt ttacatgcat atatatatat 1020
tttattttat tttatgtttt ttggagacag ggcctcgctc ttttgtccag gccgggtcac 1080
aactcactgc agcctggacc tcctagcctc aagcaatcca cccacctcag ccttccaagt 1140
agctgggact acaggtgtgc accaccacag ctggctaatt ctattttttt atagaggcga 1200
agtctcacta tgtcgccagg ctggtctcta actcctgggc tcagtgatcc tcccgtttcg 1260
acttcccaaa gtgctgggat tacaggtgtg agccacttca ccaggcccat tttctcctaa 1320
aacttcaagg acaaatcatt aataatgtaa caggaatctt taggagaaaa aacaatttgg 1380
tttactgata acaaaagata attggaaaca tgagagtatt tgagattggc caagcagaac 1440
tatgaagtcc atcaagtaag tcaaagatca tcgtttctgt tttgaattgt gggtgataat 1500
gggtgggaga gtgctacagt ctgtatgtct gtgtctccct agaattcata cgatgaaatc 1560
ttcactctca agttgataga aggtggggcc cttgggaagt gtgaggtcat gagagtggag 1620
ccctcatgaa tgggatcagt gccttatgaa aggccctaga gagatacctc atcctctcca 1680
cagtgtgaga cttcaagggg aagtatgaga cttctctgag gaagcagacc cttcacaagc 1740
aaaatcagcc agcactttga tcacggactt cccagcctct aggactgtga gcaataaatg 1800
tttgatgttt ataagccacc cagactgtgg tattttgtta tagcagcctg aacagactaa 1860
gacgggggtg ttgcttccat caaaggatgt actaagttgt ggattatttg tgaaattgaa 1920
ttacaacctt ttccttaagg tcttttacca cctccccccc aaaaaaatcc cccaaaactg 1980
attcagattt tcatacttta atgaaatatt ttataatttg caaattttta agtaatttat 2040
gaaaaaccta gatcagtgga tctcctctct ggctgcccat tagaatgtcc tgtggagatt 2100
aaactttttt ttttcagttt atggaccaag agttttgatt tatttagggt ggagttcagg 2160
atcagaatgg tttcagaagc tcccaggtga ttccggagtg agttggagct gcaagcccct 2220
gagctagatt ataagatgct tctgggaaag aaccacattt taggaatttg cttcccaccc 2280
agtgccctgc atttaatcag cacctgatga cttggcagga cttgccccac cagggtctgg 2340
ctttgaaggg tagtggacac caggatcctt tggattaatc ctctgccacc tctctctttt 2400
cctcaaccga gagtgaattt atgtaattga gtgaaagtct acgaatcata attgtaataa 2460
attaaggctg ggcatttgtt tgaaattaga taggataaag ccaaaggttt gaacaagttg 2520
tggatggttt gtaaaaatta atcttacaaa ataaatgctg tgtgtgaaca cgttgattaa 2580
attcaaaaaa a 2591

<210> 220
<211> 1360
<212> DNA
<213> Homo sapiens

<400> 220

```
atggggaacc gcagcaccgc ggacgcggac gggctgctgg ctgggcgcgg gccggccgcg 60
ggggcatctg cgggggcatc tgcggggctg gctgggcagg gcgcggcggc gctggtgggg 120
ggcgtgctgc tcatcggcgc ggtgctcgcg gggaactcgc tcgtgtgcgt gagcgtggcc 180
accgagcgcg ccctgcagac gcccaccaac tccttcatcg tgagcctggc ggccgccgac 240
ctcctcctcg ctctcctggt gctgccgctc ttcgtctact ccgaggtcca gggtggcgcg 300
tggctgctga gcccccgcct gtgcgacgcc ctcatggcca tggacgtcat gctgtgcacc 360
gcctccatct tcaacctgtg cgccatcagc gtggacaggt tcgtggccgt ggccgtgccg 420
ctgcgctaca accggcaggg tgggagccgc cggcagctgc tgctcatcgg cgccacgtgg 480
ctgctgtccg cggcggtggc ggcgcccgta ctgtgcggcc tcaacgacgt gcgcggccgc 540
gaccccgccg tgtgccgcct ggaggaccgc gactacgtgg tctactcgtc cgtgtgctcc 600
ttcttcctac cctgcccgct catgctgctg ctctactggg ccacgttccg cggcctgcag 660
cgctgggagg tggcacgtcg cgccaagctg cacggccgcg cgccccgccg acccagcggc 720
cctggcccgc cttcccccac gccacccgcg ccccgcctcc cccaggaccc ctgcggcccc 780
gactgtgcgc cccccgcgcc cggccttccc cggggtccct gcggccccga ctgtgcgccc 840
gccgcgccca gcctccccca ggacccctgt ggccccgact gtgcgccccc cgcgcccggc 900
ctcccccccgg acccctgcgg ctccaactgt gctcccccccg acgccgtcag agccgccgcg 960
ctcccacccc agactccacc gcagacccgc aggaggcggc gtgccaagat caccggccgg 1020
gagcgcaagg ccatgagggt cctgccggtg gtggtcgggg ccttcctgct gtgctggacg 1080
cccttcttcg tggtgcacat cacgcaggcg ctgtgtcctg cctgctccgt gcccccgcgg 1140
ctggtcagcg ccgtcacctg gctgggctac gtcaacagcg ccctcaaccc cgtcatctac 1200
actgtcttca acgccgagtt ccgcaacgtc ttccgcaagg ccctgcgtgc ctgctgctga 1260
gccgggcacc cccggacgcc ccccggcctg atggccaggc ctcagggacc aaggagatgg 1320
ggagggcgct tttgtacgtt aattaaacaa attccttccc                       1360
```

<210> 221
<211> 13109
<212> DNA
<213> Homo sapiens

<400> 221

gggttcctcg cggcgatccc gactctcctc ccgcggctgc agcagctgcc ggttgcacac 60
ggcctcggcg gcggcgcggc gagcgcgggc ctgtggagcg gcggccgggc gcgcggcccc 120
ggcgggcacc cctcagaggg cggccgagga agctccggga ggaggagcag gaccacgagg 180
aggtgggagg cggcggccgc ctggggacca gctccgcgcc tcggcctctc cgccccctcc 240
ccagcctttc tctcgccctc ttctcccaca ctcccggccg gcgcctcggc tttgtgcgag 300
gagatggtgt agccccctgg ccgccgaagg aggagccgga cacttgtctc ccgtctccga 360
gctgctcccc acccctggag gagagacccc cccctcggct cggcgccttc tgcgtctccc 420
ggctggtggg gaagcctctg cgccgccggc accatgagtg aacagagtat ctgtcaggca 480
agagctgctg tgatggttta tgatgatgcc aataagaagt gggtgccagc tggtggctca 540
actggattca gcagagttca tatctatcac catacaggca acaacacatt cagagtggtg 600
ggcaggaaga ttcaggacca tcaggtcgtg ataaactgtg ccattcctaa agggttgaag 660
tacaatcaag ctacacagac cttccaccag tggcgagatg ctagacaggt gtatggtctc 720
aactttggca gcaaagagga tgccaatgtc ttcgcaagtg ccatgatgca tgccttagaa 780
gtgttaaatt cacaggaaac agggccaaca ttgcctagac aaaactcaca actacctgct 840
caagttcaaa atggcccatc ccaagaagaa ttggaaattc aaagaagaca actacaagaa 900
cagcaacggc aaaaggagct ggagcgggaa aggctggagc gagaaagaat ggaaagagaa 960
aggttggaga gagagaggtt agaaagggaa aggctggaga gggagcgact ggaacaagaa 1020
cagctggaga gagagagaca agaacgggaa cggcaggaac gcctggagcg gcaggaacgc 1080
ctggagcggc aggaacgcct ggagcggcag gaacgcctgg atcgggagag gcaagaaaga 1140
caagaacgag agaggctgga gagactggaa cgggagaggc aagaaaggga gcgacaagag 1200
cagttagaaa gggaacagct ggaatgggag agagagcgca gaatatcaag tgctgctgcc 1260
cctgcctctg ttgagactcc tctaaactct gtgctgggag actcttctgc ttctgagcca 1320
ggcttgcagg cagcctctca gccggccgag actccatccc aacagggcat tgtcttggga 1380
ccacttgcac ctccacctcc tccaccactc ccaccagggc ctgcacaggc ttcagtagcc 1440
ctccctcctc ccccagggcc ccctccacct cctccactcc catccaccgg gcctccaccg 1500
ccccctcctc cccctcctct ccctaatcaa gtaccccctc ctcctccacc acctcctgcc 1560
ccacccctcc ctgcatctgg attctttttg gcatccatgt cagaagacaa tcgcccttta 1620
actggacttg cagctgcaat tgccggagca aaacttagga aagtgtcacg gatggaggat 1680
acctctttcc caagtggagg gaatgctatt ggtgtgaact ccgcctcatc taaaacagat 1740
acaggccgtg gaaatggacc ccttccttta ggggtagtg gtttaatgga agaaatgagt 1800
gccctgctgg ccaggaggag aagaattgct gaaaagggat caacaataga aacagaacaa 1860
aaagaggaca aaggtgaaga ttcagagcct gtaacttcta aggcctcttc aacaagtaca 1920
cctgaaccaa caagaaaacc ttgggaaaga acaaatacaa tgaatggcag caagtcacct 1980
gttatctcca gaccaaaatc cacaccctta tcacagccca gtgccaatgg agtccagacg 2040
gaaggacttg actatgacag gctgaagcag gacattttag atgaaatgag aaaagaatta 2100
acaaagctaa aagaagagct cattgatgca atcaggcagg aactgagcaa gtcaaatact 2160
gcatagagga acagactaag gagagatagg actttaatct ggaggaaaaa tatcctacaa 2220
acaacaactg ttcacaacag caaacccta catttatgag ctgtaagaag aaaatggaga 2280
caaacagaag gagggaaaaa ccaacctact ctgaaagcct tcagacatta tgactctggt 2340
gataagctct ttccctctcc gtttgctgct tttttctggc ctttacaaca gaatggaaga 2400
gaatcattta agagttcctg taacagttat gcagaaaata ctaaaaccca tcaggcaaga 2460
tcaccacgca ttgaaatatt ttcatatcaa gataaagtcg cacattttcc acaatacatt 2520
gctaaaataa agaggagaaa ggcttaggaa gttttttttgc agagagtgct ggtaaagaat 2580
tgagcaagtt tgctattgta ttgtaatgtt tctctcaggt ttgttcttcc tatcatgttt 2640
gatattccat gaataattga gatcagccct atgtaagtta agatcataat atgtggaaca 2700
aatggaattg taagtgcttt caaagggtaa tatttataag aaagtgtccg aaaaatgttt 2760
cttcagcttg agaaatttta gaatgatagg aagtttctcg agttagcctt catgcaattt 2820
tgtagattaa aacataaaat ttgtccagaa cttaaagatt tagatgcctt cctaaattgt 2880
tacaatgctt taccaaatct atgacttcta cataacacaa accagtggtc aaatgtaaac 2940
actatattgt agatttactg taggttttca accttttttta gatttatgca tgtggacatt 3000
tttataatgt aattacaatc accacaaggt tagctttttt aattgcagac agtaatgcat 3060
gtcacactaa tatgtagtgg ccttttcaag gcctagtccc agggaaaaca ttttgtagag 3120
tatagggggag tgggaggaag gggaggaata attttttatt taaagttgat ttctgcacta 3180
tctttttctc agttacctgc atgaataaat aatgagaaat attttgtgac tttaattggt 3240
aaatatgtta caaaaccaag tacttaatct tttacatcat gtcttcagct atttgtattt 3300
taaccagtaa tttcaatggt ctgaaacatg attctgagct tcacataata tcttaactgt 3360
ggaactcaaa agtttgatca ctgaatttgg cagttattat tacctaggta cccccgctgt 3420
tacacaggtg tttagatacg tgttcctgaa tgaagctgct tttgaatttt gttatgttga 3480
aatgcaagaa ataacaatga tggcagcaat taaggtcaca gaaatcatta ggtaaaggaa 3540
aaccaatgag gagttctgca gttttctttt aataagtaaa gtgagacttg ggtggtggga 3600
agaaggaagg tgggaagaag gaattagaca ctctgcctgc cactctgcgt gtgtgtgctc 3660
tcgcgcacgt gctgtctata tggaagccac tccctttctct ttcctttgaa actggtaagg 3720
ttaaaatagg ggagaaatcc tacatgttgg aatgatagct ttttggaaaa tttaagaaac 3780
tctccaggct ctccatcttg atttatgctt gagttgttat gtgccatatt tgctttgaac 3840

tctgattatc agaagtttta ctaaaacttt gaaataattc actttcatct gctttctaga 3900
ttttgtacat ctcagtccat aaagcaaagc ttgttgatag tgtagttttc taaacgctgc 3960
aaatttgcag cctttaccac tacaaagaag tttggatgag ggattttttt tttctttgtc 4020
aaaatagttc ctgtttctgt agaaatttca tttttagatt aaactgtgat ggatgagcta 4080
tcataattca agtatacatt tcttttttct atcagatatt cattgtcatg cagtagtagt 4140
aaaaacatca aagatgcagc aagcttatta agtattattt tctaaaagaa ataggaggca 4200
ttttcatctt tattattgta cttttggtta tgcaaacact ttgataatat aaacagttat 4260
gtcccctata aatctggtca gcaacctctt ttgattttgt tgggtaagtt aaatagtctg 4320
tagtaggtag agtactgggt acaagtggtc caaactaaga taagagacta aaataaaatg 4380
ctaaatctta aaagaaactg ggtttatgca ctaaacgttt tgtgccttgg tctaatatta 4440
acatgatgta tgtgtaaact gacaaaaaaa aaaaaaaaaa aaaaccagtg ttgaatcatg 4500
ctatatttcc catcatccca gattgctaaa tgttctttcc aagaagtctg aatgaattat 4560
tatatacatt tgctatcata catgacagtt gtgtcattat tagagatttc agtaattaaa 4620
atgggaaaca gaagcctaag ttatttacct tatcaaaacc acgttccttg gaaccacatt 4680
attatgatgg tttttgtgct cttgtacgtt ggatgtctta agctgagtac agtttagggg 4740
atcctttcta atgacaggaa ggcactgctt tcctcaacac tgtgatctga cctgtgacaa 4800
gtctgtacta tccactatgt aaatggctca caagtcaatt gcaaacaaac tgaatgtaca 4860
aatcttagtg ctggtgctga aatctcttca gaatagtcat catgatttaa aagtttgttt 4920
aaaaatttgc aagcatcaag tgtcaatcaa aactgaagat gaaacactaa tgaatgttga 4980
attctcatgc ttttaggtgt acttcctttt tagaattttc agttttctgc tatttttacc 5040
gtaactattt cgtttaaatt tgtttcattt aagtttccta cacgctaact tcgtttgtgc 5100
gattgaaata aaattgcagt atatatatgt tgcttgtttg tgacacttag ataatcttct 5160
gaaataattt gttttaaagt aagttatatt ggatgtgaaa aacacctttt aaagtccaag 5220
actttactac ttaaaagttt acatgtcacg tcactattct ttacagattt catagaagtc 5280
gtgaatacta agtaatgtat acaaaagaaa tatgtgatga tgtgtctact ttttgtttga 5340
aataactgtt gggtgttccc tcttcttatt ttctctctgc ccaccttgac acagtaactt 5400
cctcttatag tcaagatgat gattaagtgt ttaattttta ttccattttg ttctgcaaca 5460
cgttcaggta cttaaaactc attcatgcag ttagaattat cctttatgta gtttgcatgg 5520
taaaggacat tgtgtggtaa agggcattat tgagtaatga attttaagta tagcaaaata 5580
tgtatagtgt ttatacatga tgctgatttc ttaaaaccta catataagca gtagtacttt 5640
ttctctatta taaagtatta attttgagtt ccattttata attctgatgt cttcattaga 5700
aatacaaaaa tacaatcctt gtatttttc tttgaccaga cccaaatttt ccccttcccc 5760
cctccattct cctctccact tctcgtggac cgcgtgttcc caatattcat taacatttgc 5820
agccattaca gagttacagt tgtaaaattt tcagctgttt tttttttttt tttttgctc 5880
tagcctgtct catgttcttg cttctcttca tatgtaagtc agtatttttg tctacaaatt 5940
aaaagtcact tgtcattcaa aaatgagtta agtaacatga taatctttta aatttaccta 6000
gctacaactt ttatgtctct tgtaaattta tatatacttt cagtgccatt gtgaagaatt 6060
ggccttttaa ttctaagtag tattacccta tgaagcacag tgtgcaaagg tatgctacaa 6120
ggtctcttta attcctgtgt ggccattata attttataac accattaccc tttaaattct 6180
accgattata agcagcgtaa aagtaactat ataaagcaaa catcgcaaag gaactctgca 6240
ggagctctta attcctttat gtagctatca taaaattcac tttcctgaag acatttactc 6300
tcattcactt ccaaactcca aacctttttc tggtagcacc acttttgttt ttaatagaaa 6360
gatgagttca tatctgtaca tctctccaaa gctctaagga atgagaaaag gatcctagta 6420
tattgaaatt actgatgttt aatacctctg ccttttcact aaaagccatt taatattttt 6480
aaagtcaaaa cttgacatac aggtatttat aaggaatctc catgactctg aaggaatgaa 6540
attgatgtag gtagctttgg ctatgtaaag acatagtaga ggacaattac ttaaagaaga 6600
gttttctttt gaggatttgt agatttgact aagcagctga agtcagtaac ccctcaagaa 6660
agtccgttta tagaagccaa gaactgttgg aggtctgtga tgcaggtttt tctgttccca 6720
acatcaaaag ctgtggatta tttggtgtgt tcccctttat gttatctctc gataattttt 6780
gagacatcaa gattttttaa aatatcaata ctggaaagat gaaaccttca aattaaaacc 6840
taaaattgat tagaaagaaa agtaaaatat taatgcagta aatttaggat ggttacagat 6900
actcaccatc aacataaata aatggagttc taggataaat tatttgtgga atactaatgg 6960
tcaagtcaca taagagtaaa atcttttggt gaggagacac atttgacaac tgctaaagaa 7020
ttaactaaat ttgtctttct aaatagtttt ctttataaag taacagaaac attgtaaaag 7080
gataaatgac tgtcataatg acttaggatg ttgtaagaat gtctgccaca tttattggtt 7140
atttctgttg aaatctgaag gaatcgatac atctgaaaat tgagaaaggg aaataggact 7200
gtccccgtta cagatactct gtgatgcaca caaccttcta aacggaagtg ttagtatcat 7260
ttaaagcaca tagtaggcct tgagtatttg cagattaaca tgcagaaaaa atcaaagatg 7320
aagccccaat taagtgtttg aaccaaagaa tcacagtcac tgagctgcac ccccgttctg 7380
tgaagcatgt taaatactca taaagagcag agagcagggc ccgtagacgc ttctggtgta 7440
ggacacagga attctaggca gagggagaaa attaggggcc ccagttggtg gctgagagta 7500
aaaagaatat accatttgag tagtgctgtt ctttttcctc agtttttaaa gacatataat 7560
tcctagtagt taggacatct aaatagctcg gtaaactggt cagaggcggg aggattgctt 7620
gaggccagaa gttttgagat cagcctgggt aacagggaga ctcatttcta caaaaaaatt 7680
taaaaattta ggccaggcat ggtggtgcac acctgtaatc ccagctactc aggagactga 7740

agcgggagga tcacctgagc ccagggagga cgcggctgca gtgagccgtt atcatgccac 7800
tgtactccag cctgggtgac agagcgagaa cctgtctcta aaaaaaaaaa aaaaaaattc 7860
taagagaata tagcttttca tagaaattat tagaaaagct tacatatttc attagagagc 7920
ccagtttgtt agactacatt tattccgtaa aagttctata tacaaatgac aacccatagt 7980
ttatcatgta aatctgattt aactgttccc ttcaattcca agaaagattt cttcagcagt 8040
agatgacaat tctaaaagca taaagaaata actaataaag gagatggatg atgttatagg 8100
ttggtttggt tgggttttag taaatagtta ttcttctaag aaggcttaga aaatcgagaa 8160
ttgaatagtt ggtactttga attccatagg aaaaagcact cagaagaaaa agtctacatt 8220
ggctagtgtt tagattgaga gttaattttc ccatcaagtc caaaagtttc catccaagtc 8280
tatagaaact gagagaaaag gttcttggct tacacttaaa atacacattg ttgcaaccat 8340
ataagagaga tacattacag aacgctttta aaaatttggt ctcttaagcg cctaacagtc 8400
catgctttag tcacatctgt gaaacctctt ttcttttttt ctttttcttt tattttttttt 8460
tttaaagatt aaaggcattt taaaaataaa tgtttcatgg ccgggcatgg tggctcacgc 8520
ctgtaatccc agcactttgg gaggctgagg tgggcagatc acctgaggtc aggagtttga 8580
gaccagcctg gccaacatga tgaaacccca tctctactaa aaatacaaaa aaattggctg 8640
ggcatagtgg tgggcacctg taatcccagc tcttcgggag gctgagacag aagagtcgct 8700
tgaatccagg aggtggaggt tgcagtgagc taagaccgcg ccactgtact ccagcctggg 8760
cgacagagcg agattccatc tctaaataaa taaatgtttt tcaacgtgtt ttatgtgaga 8820
aataccagct tttcattatt actaccagtt gatctcaggt gtcacttggt tggttctgct 8880
gaccgaggca ctgggcattt taaatatgga gtttccctcc acccattgat caagtctgag 8940
gtttgtccat ccttggtggc aggctagctt aaccatggat tcacaaaccc catttgattg 9000
tcgtaagtca taaggccacc ccagtgccag tggttgggca tcacagtgag tctgaggtga 9060
tgaatttgag ttagctgtag gctagttccg caactcagca atagcccatg gatggttggt 9120
aaaagacgtt gactttgcca cttaaatttc caggagaaga atagcttata gtttatctga 9180
ggaaagtaat tgggaaggct tagatattga gaagaaagcc ctgttaaaat ctaggtagca 9240
tttcctttct tctttattac tagagggatg ggaggttcag gcctgagtca ggactttaca 9300
atttggtgtc cttcccagag gtactttggg cttgatcatt ttaagtttca attgcaaaaa 9360
aaaatttttg acggagtgtt gctctgtcac tcaggttgga gtgcagtggt gcgatctcgg 9420
ctcactgcaa cttccccccc ctgggttcaa agggattttc ctgcctcagc ctcccaagta 9480
gctgggacta caggcgcgtg ccaccatgcc cgactaattt ttgtattttt agtagagatg 9540
gggtttcacc atgttgccca ggctggtctt gaactcctga gctcaggtga tctgcccgcc 9600
tcggccttca attgcagaag tttttgaagg gagaatattt gatcctcaca taaaatgtcc 9660
ttgtttctga aagaaaacat ggtaaaaatt gttattgagt ccatgtcttt gccaccttgt 9720
tggacttgat ttccctttct ctgtgttcct cccgccctca gtctctctca gtctgtcctt 9780
ttttgtgtgt ggagttgtgg gcaggtcatt ggactgggac tgactttacc agttttcagg 9840
ctgtggggct agtcagtcat tctctctgag ccttctacct ctaaagttga gttctttttt 9900
taaattcttt ttgttttgtt tttatttttt ccttgccact atacaagcaa ggatagataa 9960
agctttttat tattattgtt attattatta tttcgagaca gtctcgctct gtcatccagg 10020
ctggagtgca gtggtgtgat ttcagctcac tgcaacctct gcctcccggg ttcaagcgat 10080
tctcctacct cagcctcctt agtagctggg actacaggca tgcgccacca cacccggcta 10140
atttttgtat ttttagtaga gacagggttt caccgtattg gccaggctgg tctcgaactc 10200
ctgacctcag gtgatccacc caccttggcc tcccaaagtg ttgggattac aggcataagc 10260
gaccgtgccc atccccgata aagttgagtt ctgaatttcc tctttcactg tattctcggc 10320
agctactggt tggtttattc ttttttttta atcttttttg gtttattctt cttttgatct 10380
ttgaagagaa ggagaaaaaa gatctgagtt tggctctccc atcttaagtg cagcttttag 10440
ttgttttata gtcatagacg ctattgcact ggacagcatg catcctggat gtaaacaatg 10500
cggaaaaatc aatggagtga ccatcttggg gtcagtggtg ttgcataaag caccagtggt 10560
gtgctgttct gtgtcagttt cctctgcgcc acgtgctttc acagtttgaa ttaccagcat 10620
ctctgtggtg gcttcagtac attctccatt gtgcttcaac ctggaagcac attctgccgt 10680
tcagaaagac tgattccaat catgtaatgg gaccacacga gatccatgct ccaattatat 10740
gaatagcaga aggtccattt gactcttgta gttcaagagc tgtttctaag taaacagact 10800
ttccagagct cgatgaagtg aagaagccct tctttcaatt cctgggcaga attcagtggt 10860
tttcatatca tctgaacctg ctgtgctgct gtttattcta tcactgtcag ccttgaaagg 10920
aaaaaaggaa taatcttacc ctcctgccct tcttatttta tatcttccat cttatccatc 10980
tatatccatg tagtcattcc ttccgagttc cctggtttta atccaaaaga ctctgtttaa 11040
cagcaatgtc ttggcagacc aggtgccgct tccgtgccat ttgagggctc tgagtctttg 11100
cttcgtctta ggaagataaa gataatttgg cagaaagtca gagatggaaa tagcagtttg 11160
ggatgataaa agatgggtcc tgttttgctg acattctttt actttggttc ttggtgcgtg 11220
tcctttggga ttcatttctt gctagcatga ataggacatg cttttcgtta ggtgcttttc 11280
ttttttggag aacatgaagt gtttctaaca aaactgaatg agctagaaag aattctattt 11340
tgagacatct gtagttgatg caaagtattt ctgtaatcct caatgacagt acttacatat 11400
ttgcatagaa tttttttttt aatttttgag gaaacctctc tggagtctgt atttatagga 11460
gcatcctgaa agtccctatt atgtagctgg tgggagcgag agagggacag ccacactcgt 11520
gctgtgagca gagcacaact tcaggcagag tgaatgcctt ctgagttctc attggaagct 11580
ttctgttgct cagcctctgg cagatttcag aaccttctac ttaagccagg tgtggtggtg 11640

```
cgtgctccta ggcacagcga ctcgggggc tgaggcagaa ggatcacttg agcccaaagt 11700
tggaggccag cctgggcaac atagcgaggc ctgtctcctt aaaaaataaa taaataaata 11760
ataaataaat aaataaataa aaaccttgtc cttgactatt gtgctgtctg ctgcccctt 11820
gtagggatgg ggttggggtc agaataagca tgtgcatgaa tggcggaaag ctttggtggt 11880
gtttgtttgt ttgtttgttt tttgagaggt gggggagtca gcagtgaacc tttctggtta 11940
aagaacaacg tacataattt ctattatgag atttagaagg ttaaatactt ttttttttca 12000
ggcctaccaa gttgatagac caaaagtata tagtagaaca gcacagtaga aaaaatttaa 12060
aagtgaattt cttatggtcc aaaaggatgg atagtcacag tccataacct ttcataatat 12120
atgctggaat tggaatctct tgtcatcttg ctttgctctg aattttagaa ctagaagtgt 12180
gcatgtatgt caaaagatta tgcagagcta ttttctttgc taacaatgta aacatttgca 12240
ccccatgaat gggactttat aatctggaag tgagcctaag gttgcgtata ctgaagtcat 12300
gttttaaaag ttaaggttga cttaaacata tacaaggctg tattttaatt gtaactagaa 12360
atcatttact atgcatacag aacctcatgt atcttatcat ggaatttgtg aattaatggg 12420
ccttgccgaa ttcaagggta agggaaactg gtaacaactt gttttaaatc aacatactat 12480
aagctcctgc ttgcttgact caagttgttt gaaaatctga attaaacagg atctcattgt 12540
caggaaacat cttctaagct tgacatctac gtttaagaag ggaacgtgga agagaaggtg 12600
agacatgaaa ctaaaacagc tgggagggta ggatcagact gcacctgatt tttggaccaa 12660
agtttcatat aggaataaat aaaaaggagg accacttgtg agccaaccta ggaaggactt 12720
gccagaaagt agcagctcct gctgcaaaat acagtgcctt caaaatgatt ctctgtgtga 12780
attgcatgca atttcatgta accataaagt gtaacctgta tgtgcatgac atgtgtctag 12840
aatgtgtaaa gttaggattc agcaggaagg ggcctggccc tgtcactgtg gtggatcagt 12900
ggacctgctg aagtcactgg actcatgtga cttaggtttg cagcatcaga cccctgtgtt 12960
tgcattaagt ggccacatcg ttgaaatcgg atcaatctct ccctcagctt tctttcctac 13020
tttgcagctt tgctggtttt aactgcttca ttcttctgct tcttggtatc ctttttctt 13080
ttgaaataaa aacatgaaat acttaattc                                  13109
```

<210> 222
<211> 436
<212> DNA
<213> Homo sapiens

<400> 222

```
gcagctactg aactggaatt gagaacaaaa gagcagttat tgaaaaagta atattacaat 60
cttttaaaat agggaatata cttgtgtgct aatgggagaa gtcattagta aatatttaac 120
tagcccaaat agctcactta atataattcc acaaaaaaat gaattacttt attgtagaag 180
cctgtaaggc taaattatag gtagtgttac aagtttccgt tagctagtca aaaactggca 240
ttaattttac atttcctaag aatgagaact ttcaagtcat aaaaatacga aactcctaga 300
gcctgtaaaa taatccactt aatacaactg atgacgtgct aaatattaaa ctactaggtt 360
ctgaatgtaa accattatct tcctgctttt aaaaaaacta acccaagttt tgctgcactt 420
aagcattaag acttct                                                436
```

<210> 223
<211> 2776
<212> DNA
<213> Homo sapiens

<400> 223

```
cagccgccga gcggccgcga tttcccgggg actgctgggg cgcagcgggg aggcgggccg 60
gggggcggcg gggcgcgagc agagcgcggt tgacctccct ttctctgctc agctccagcg 120
tcatttcggc ctcttagttc ttctgaaccc tgctcctgag ctaggtagga aacatgagcg 180
gcaccaactt ggatgggaac gatgagtttg atgagcagtt gcgaatgcaa gaattgtacg 240
gagacggcaa ggatggtgac acccagaccg atgccggcgg agaacccgat tctctcgggc 300
agcagccgac ggacactccc tacgagtggg acctggacaa aaaggcttgg ttccccaaga 360
ttactgaaga tttcattgct acatatcagg ccaattatgg cttctctaac gatggcgcat 420
ctagttctac cgcaaatgtt gaagatgtcc atgctaggac tgcagaggaa cctccacaag 480
aaaaagcccc ggaacccact gatgccagaa agaagggaga aaaaagaaag gctgagtcag 540
gatggtttca tgttgaagaa gacagaaata caaatgtata cgtgtctggt ttgcctccag 600
atattacagt ggatgaattt atacaactta tgtccaagtt tggcattatt atgagagatc 660
ctcagacaga agaatttaag gtcaaacttt acaaagataa tcaaggaaat cttaaaggag 720
acggtctttg ctgttatttg aaaagagaat ctgtggaact tgcattaaaa cttttggatg 780
aagatgaaat tagaggctac aaattacatg ttgaggtggc aaagtttcaa ctgaagggag 840
aatatgatgc ctcaaagaag aagaagaagt gcaaagacta taagaagaag ctgtctatgc 900
aacaaaagca gttggattgg agacctgaga ggcgagccgg accatcccgg atgcgccatg 960
agcgagttgt catcatcaag aatatgtttc atcctatgga ttttgaggat gatccgttgg 1020
tgctgaatga gatcagagaa gaccttcgag tagagtgttc gaagtttgga caaattagga 1080

aactccttct ctttgatagg cacccagatg gtgtggcctc tgtgtccttt cgggatccag 1140
aggaagctga ttattgtatt cagactctcg atggaagatg gtttggtggc cgtcaaatca 1200
ctgcccaggc atgggatggg actacagatt atcaggtgga ggaaacctca agagaaaggg 1260
aggaaaggct gagaggatgg gaggctttcc tcaatgctcc tgaggccaac agaggcctta 1320
ggcgttcaga ttctgtctct gcttccgaaa gggcagggcc ttctagagca aggcattttt 1380
cagagcaccc cagcacatct aaaatgaatg ctcaagaaac tgcaactgga atggcgtttg 1440
aagaacctat agatgagaag aagtttgaaa agacagaaga tgggggagaa tttgaagaag 1500
gtgcttctga aaacaatgct aaggaaagta gccccgaaaa agaggctgaa gaaggctgcc 1560
ctgaaaaaga atctgaagag ggctgcccca aaagagggtt tgaaggcagc tgctcccaaa 1620
aagagtctga agaaggcaat cccgtaagag gatctgaaga ggatagtcct aaaaaagagt 1680
ctaaaaagaa gacactcaaa aatgattgtg aagagaatgg ccttgcaaag gaatctgaag 1740
atgacctcaa caaggagtct gaagaggagg ttggccccac aaaagagtcc gaagaagatg 1800
actcagagaa agagtctgat gaagactgct ctgaaaaaca gtctgaagat ggctccgaaa 1860
gagaatttga agaaaatggt ctcgagaaag atttggacga ggaaggttct gaaaaggagc 1920
ttcatgaaaa tgttcttgac aaagagttag aagaaaatga ctctgaaaac tccgaatttg 1980
aagatgacgg ctctgaaaaa gtgttagatg aggaaggctc tgagagagag tttgacgaag 2040
attcagatga aaaggaagaa gaggaggata catatgaaaa agtatttgat gatgagtctg 2100
atgagaaaga ggatgaagaa tatgcagatg aaaaggggct tgaagctgct gataaaaagg 2160
cggaagaagg tgatgcagat gaaaagctgt ttgaagagtc agatgacaag gaagatgaag 2220
atgcagatgg aaaggaagtt gaagatgctg acgaaaagtt gttcgaagat gatgattcca 2280
atgagaagtt gtttgatgag gaggaagatt ccagtgagaa gttgtttgac gattctgatg 2340
agaggggggac tttgggtggt tttgggagtg ttgaagaagg gcccctatcc actggcagca 2400
gctttattct cagtagcgat gatgatgacg atgatattta atcccttaaa cttgcttttt 2460
agggagagtc ctccatctac atttgcctgt gcttcagggt aattactagt agtgttacat 2520
gaacatgtgc atagtggtag gatgccatca gattaaagca ttgaagtgtt tcattgttac 2580
ctgtacctaa tggttttaaa tatatgttaa ttgattgttt agttaaaatg tcatagttac 2640
aatgcaagta aactggatac ttgttctttt gtcagatttg ttaaatgcat gcagaataat 2700
atttttaaga gtattgattg aagtttgtga tattcatcaa taaaaatgag ttgataataa 2760
aaaaaaaaaa aaaaaa                                                 2776
```

<210> 224
<211> 2389
<212> DNA
<213> Homo sapiens

<400> 224

```
aggcgcggtt gtgagtagta ccgggagtgg ggtgatcccg ggctagggga gcgcggcggc 60
cgcgatcggg cttagtcgga gctccgaagg gagtgactag gacacccggg tgggctactt 120
ttcttccggt gcttttgctt tttttttcct ttgggctcgg gctgagtgtc gcccactgag 180
caaagattcc ctcgtaaaac ccagagcgac cctcccgtca attgttgggc tcgggagtgt 240
cgcggtgccc cgagcgcgcc gggcgcggag gcaaagggag cggagccggc cgcggacggg 300
gcccggagct tgcctgcctc cctcgctcgc cccagcgggt tcgctcgcgt agagcgcagg 360
gcgcgcgcga tgaaggcggt gagcccggtg cgcccctcgg gccgcaaggc gccgtcgggc 420
tgcggcggcg gggagctggc gctgcgctgc ctggccgagc acggccacag cctgggtggc 480
tccgcagccg cggcggcggc ggcggcggca gcgcgctgta aggcggccga ggcggcggcc 540
gacgagccgg cgctgtgcct gcagtgcgat atgaacgact gctatagccg cctgcggagg 600
ctggtgccca ccatcccgcc caacaagaaa gtcagcaaag tggagatcct gcagcacgtt 660
atcgactaca tcctggacct gcagctggcg ctggagacgc acccggccct gctgaggcag 720
ccaccaccgc ccgcgccgcc acaccacccg gccgggacct gtccagccgc gccgccgcgg 780
accccgctca ctgcgctcaa caccgacccg gccggcgcgg tgaacaagca gggcgacagc 840
attctgtgcc gctgagccgc gctgtccagg tgtgcggccg cctgagcccg agccaggagc 900
actagagagg gagggggaag agcagaagtt agagaaaaaa agccaccgga ggaaaggaaa 960
aaacatcggc caacctagaa acgttttcat tcgtcattcc aagagagaga gaggaaagaa 1020
aaatacaact ttcattcttt ctttgcacgt tcataaacat tctacatacg tattctcttt 1080
tgtctcttca tttataactg ctgtgaattg tacatttctg tgtttttttgg aggtgcagtt 1140
aaacttttaa gcttaagtgt gacaggactg ataaatagaa gatcaagagt agatccgact 1200
ttagaagcct actttgtgac caaggagctc aatttttgtt ttgaagcttt actaatctac 1260
cagagcattg tagatatttt ttttttacat ctattgttta aaatagatga ttataacggg 1320
gcagagaact ttcttttctc tgcaagaatg ttacatattg tatagataaa tgagtgacat 1380
ttcataccat gtatatatag agatgttcta taagtgtgag aaagtatatg ctttaataga 1440
tactgtaatt ataagatatt tttaattaaa tattttttttg taaatattat gtgtgtgttt 1500
tttttttaatc tatgggaata tttcttttgg aaaatcattt ttcagctcaa ttacagagct 1560
cttgatatct tgaatgtctt ttctgtttgg cctggctctt aatttgcttt tgttttgccc 1620
agtatagact cggaagtaac agttatagct agtggtcttg catgattgca tgagatgttt 1680
aatcacaaat taaacttgtt ctgagtccat tcaaatgtgt tttttttaaat gtagattgaa 1740

atctttgtat ttgaagcata catgttgaaa atacacctta tcagttttta agtacagggt 1800
tttatagtgt aatatataca gagtaagtgt ttgtttttgt ttttcaactg aggtcaaaat 1860
ggattctgaa tgattttgca tatgggatga ggaaatgctt ggatccttaa ggagtttacg 1920
aaatctgctg ttttatcaaa gtgaaaaaaa attgcttatt actcttcatt ttacactaaa 1980
gcttaatgtc actaagtttc atgtctgtac agattattta aatcatggaa atgaaaaaaa 2040
tgttctctgc ttgctaccaa aggacaaact cttggaaatg aacactttct gctttccttc 2100
ctccaaagaa ttaataggca acagtgggag aaaaaaaagg cataatggca aatccttcaa 2160
gcagggataa aagtcgatct tcaaacatta acttaagcag accaaaaatt ctgatgaccg 2220
catctagatt atttttttat aaaaatgatt ttcactatag ctatgttacg ctaagctact 2280
gtcccatctc ttgtgatgtg taacttttac atgtgaatat taaagtagat ttctctgtct 2340
tgtaaaaaaa aaaaaaaaaa aaaaaaaaaa aaaaaaaaaa aaaaaaaaa            2389
```

<210> 225
<211> 1402
<212> DNA
<213> Homo sapiens

<400> 225

cgacagagag agcgagcctg tgtccaaaaa gtaagtaagt aaataaatac ataaaaataa 60
atacgggttt ctgtcacctc gcttcagaat cagacagcta gaggtggggc ctggaattgg 120
tgttgttaat gcattctcca ggaatttctc ctgcataggg aagttggaaa ccactggctt 180
ccttctctct gagcatctcc tgagactagc tcctgaagtg gatagaaagt ttttcaagta 240
gcagattaag ttttatccct taggctgcta ataaaaaaca attgactgtg attgcgtaat 300
tttacctgtc gtacaccttt cagaaggcag aggctttggt tcacccctct acctccaggg 360
cctaaaacag tgcctggagg aactcaatta aacatttgtt gagtgaatac gtgaattaca 420
ttgcagcgag gccaatcttt taaaagttac ttgtcaaatt aatctatcag ttagtggcat 480
attaaagccc agcatattac aaaaaaaatt tttttagtag tcactgattt tataaattag 540
gagccacaag cactataccc cagtgacttc tgtaagtttg gttagtttag aaaggagaga 600
gcctttccca gggcgtagtg tgctcaaggt gattagccag aaagaagatt cagaacaaag 660
catttgctta gagagtcaaa tgtaattagt cacagtgtag tggtaaatca tatatcaaac 720
ttatatttgg tatattgtct tatatcaaaa tatcatttta taaaccataa gtccaacata 780
ctgctataaa aattggtttc tagcaaatag agaaaattgt tatgttttcc aatttcactt 840
ttcatactaa taaaatttga cctttacacc ttctttttca agagttcacc acaggggcac 900
catagatatt atgtagttaa gagctcaaac tctggagaat attgtgtttg accacacata 960
gtcagggagt ttgatctaac ctctcaatgc ctcatttctc agctgaacat ggaaatagta 1020
ataataaagt tcacatactt ctaagtgctt acctaagttt ttgagacaca tccacttaat 1080
aaatggtagt tacagtgatg ataaagaatt ggggtgtata aaaataccta aaactcaaaa 1140
gctaacccat ttaattattg tactactgga cttttatgat agtaagtaga aatctgttgc 1200
ttaaaaaaga agtttctttg gtggtgtgcg tctatagtcc cagctacttc ggaggctgag 1260
gtgggagaat cacttgaacc caggagatct tggctgcagt gagctatgat tgtgccactg 1320
cactctagcc tgggtgacag agcaaggccc tgtctcaaaa aaaaaaaaaa aaaaaaaaaa 1380
aaaaaaaaaa aaaaaaaaaa aa 1402

<210> 226
<211> 432
<212> DNA
<213> Homo sapiens

<400> 226

gctacatatg gataggcaga gagaggggtc tgcttcttgt ccagctgtag ctctgtgcta 60
gtggaagcat gtcctggagt tcacgatgtg gccaagagaa cagatgtagt taggcaatgg 120
aggtgggaca gagagctgca aagtgctgca cttgcccctc ttactggacc caaaaggctc 180
tcaagtgtaa cacctttctg tagtgctgta gatcattaat ctgggtgtgt gatgaccatc 240
tgatctagca catccagtgg cattgtgcat attgggatag gaatttgatt ttgtgaactc 300
aagctattca gcagggccca tgaaaaatca ccagaagggt aggtgggtct ttgccactgt 360
ctgatgtttc aaagatttct tgtattaaat gctgtgtcca aaattataga atactctcca 420
gtttgtattt at 432

<210> 227
<211> 4949
<212> DNA
<213> Homo sapiens

<400> 227

cagccgccgg cggcgctagc acctcggagt cgggcggccg gagcagcgga gcgcagcgcg 60

```
gcgcagggaa cggcactggg ggtggggaga ccagcccggc cgagccgagg gccacccccct 120
ggtcctctgc cctcgcgccc cgctagggcc aggtgcacaa aaagctttaa acaagttaaa 180
atctaggcaa cagactccct gaagtgttga ctgcccaact taatcatgac tgtgattcaa 240
aaatgttaat aaataaacca aggtgtagat catggggaat tcatatgctg ggcaactgaa 300
atctgctcga tttgaggaag ctctccacaa ctccatagaa gcctccctca gatgtagtag 360
tgtggtacca cggccaattt tttcccagct atacctggac cctgaccagc atcctttctc 420
atctgcagat gtcaaaccca aggtggagga tctggacaaa gatttggtaa accgctacac 480
tcaaaatgga agtctggatt tttctaacaa tctaacagtt aatgaaatgg aagatgatga 540
agacgatgaa gaaatgtctg attcaaacag cccaccaatt ccctattcac aaaaacctgc 600
cccagaagga tcttgcacta cagatggttt ttgtcaagca ggaaaggatt tgcgtttggt 660
atcactgtgt atggaacaaa ttgacatccc agcaggattc ctcctggtgg gggccaagtc 720
tcccaatctg cctgaacaca tcctagtttg tgctgtggac aagcgatttc taccagatga 780
tcatggaaaa aatgcacttt tagggttttc tggaaattgt atcggctgtg gagaaagagg 840
atttcgatat ttcacggaat tttccaacca cattaacttg aagctcacca ctcagccaaa 900
gaagcagaag cacttaaagt actacctagt cagaagctcc cagggtgtac tgtctaaagg 960
acctcttatc tgctggaaag aatgtagaag ccgacaatcc tctgcttctt gccactctat 1020
taagccaagc tcttcagtgt cgtcaactgt gaccccagaa aatgggacaa ctaatggata 1080
caaatcagga ttcactcaga cagatgctgc taatggaaac agtagccatg gagggaaggg 1140
cagtgcatcc agctccactc cagcccacac agggaattac tctttgtcac cacgacctag 1200
ctatgcatca ggagatcaag ctaccatgtt catttctggg ccaccaaaga aacgacaccg 1260
gggatggtat cctgggtcac ctctccccca acctggctta gttgtacctg tccctacagt 1320
tcgccctctt tcaagaacgg agccccttt atctgcccca gttccacaga ccccactaac 1380
tggaatttta caacccaggc ccattcctgc aggggaaact gtaattgttc ctgaaaacct 1440
gctgagtaac tcaggagtta gaccagtaat tctgataggc tatggcactt taccctattt 1500
ctatggaaat gttggtgaca ttgttgtgag tcctctgctg gtgaattgct acaagattcc 1560
acagttggaa aacaaagatt tggaaaaatt agggttgacc ggcagccaat ttctgagcgt 1620
ggaaaacatg attcttctga cgatacagta tcttgtgcgg ctgggtcctg atcaggtacc 1680
tctcagggaa gaatttgagc aaattatgct gaaagctatg caagaattta ctctgagaga 1740
aagagccctg cagataggtg ctcagtgtgt ccctgtgtca ccaggacaac tcccctggct 1800
tgctagatta attgccagcg tatctcaaga cttggttcat gtggttgtga cccagaactc 1860
tttggcggag ggcatttcag agaccttaag gactctcagt gaaatgagac actatcaaag 1920
gctgccagat tatgtggtgg taatttgtgc atcgaaaatc agaggaaatg aattttgtgt 1980
ggtagtgtta ggtcagcacc agtcccgagc tctggcagag agcatgctca ccacaagtga 2040
gtttttgaaa gaaattagtt atgagcttat cacaggaaag gtcagtttcc tggcatcaca 2100
tttcaaaacc acatcattag gcgatgacct agacaagctg ctggaaaaaa tgcaacaaag 2160
aagaggagac agtgtggtta cccctttcga tggagacctt aatgaatgtg tgtcacccca 2220
ggaggctgct gctatgattc ccacacaaaa cctggattta gataatgaaa ccttccacat 2280
ttatcaaccg cagctaacag ttgccagaaa actcttatcc caggtgtgtg ctatagcgga 2340
cagtggcagc cagagcctgg acctcggtca cttcagcaaa gtagacttca tcatcattgt 2400
tcccagatcg gaggtgttgg ttcagcaaac tcttcagcgg attcgacaat cagtgtcatt 2460
tcaggctctt tgtcacatag cgaacccagt catgggctag ctgatagagt cattaattgc 2520
agagaagttc tggaagcttt caacctcctg gtgctccagg tcagctcctt cccatacact 2580
ctgcagaccc aacagtcccg cattagctct agcaatgagg ttcactggat acagctggat 2640
actggggagg acgtgggctg cgaggagaag ctgtactttg gcttgagtga gtacagcaag 2700
tctctgcagt gggggatcac gagcccactt ctgagatgtg acgagacttt tgaaaaaatg 2760
gtgaacacac tcttggagag gtaccccagg ctgcacagca tggtcgtccg ctgctatctt 2820
ctcatccagc agtactctga ggccctgatg gctctcacca ccatggcgtc actccgcgac 2880
cacagcacac cagaaacact cagcattatg gatgacctca tcagctcccc aggcaaaaac 2940
aaaagtggga ggggacacat gctcattatc agggtgccct cggtgcagct ggcgatgtta 3000
gccaaggagc ggctacagga ggtgcgcgac aaactgggtc tgcagtatcg gtttgagatc 3060
atccttggga acccggccac cgaactcagt gttgcaactc actttgtggc gcgattaaag 3120
agttggagag gaaatgaacc agaagagtgg atccctcgga cataccaaga tttagacggt 3180
ctaccttgta ttgtgatatt aactggcaaa gatcctcttg gagaaacctt tcccaggtct 3240
ttgaagtact gtgacctccg gttaattgac tcaagctatt taactcgcac ggccttggag 3300
caggaggtgg gtctggcatg ctgctatgtc tcaaaagagg tcatccgggg acccactgtt 3360
gccctggacc tcagcgggaa ggagcaggag agagctgctg tcagtgagaa tgactccgat 3420
gagctgctca tcgacctgga gcggccccag agcaacagca gcgctgtcac agggacctcg 3480
ggttccatca tggagaatgg agtgagctct tccagcacag ctgacaagtc ccagaagcag 3540
tccctgaccc ccagctttca gagcccagcc accagcttgg ggctggatga aggggtctcc 3600
gccagctcag ctggagccgg agccggggag actctgaagc aggagtgtga ctccctgggc 3660
ccccagatgg cgagcagcac cacctccaag ccgtcatcat catcctcagg acccaggacc 3720
ctcccatggc cgggacagcc catcagaggc tgccggggcc cacaggcagc cctgccacca 3780
gtggtgatcc tatccaaagc ggcctacagt ctcctgggct cccagaagag tggcaagctg 3840
ccatcctcct cctccctgct gccccacgcc gacgtggcct gggtgagctc cctgcggcca 3900
ctcctgaaca aggacatgag cagtgaggag cagtccctct actacaggca gtggaccttg 3960
```

```
gcccggcagc accacgctga ctatagcaac cagctggacc cggcctctgg cacccgaaac 4020
ttccaccccc gacggctcct gctgacaggg cccccacagg tgggaaagac gggctcctat 4080
ttacagttcc tcaggatcct cttccgcatg ctcatcaggc tcctggaggt ggacgtgtat 4140
gatgaggagg agatcaacac cgatcacaat gaaagcagtg aagtgagcca gtcagaggga 4200
gagccctggc ctgacatcga gagcttcagt aaaatgcctt ttgatgtcag tgtgcatgac 4260
cccaagtaca gtttgatgag cctggtgtat actgagaagc tggcaggggt caaacaagaa 4320
gtgataaagg aatccaaagt tgaagagccc aggaaacggg aaactgtatc cataatgctg 4380
accaaatatg cagcctataa cacctttcac cactgtgaac agtgccgcca gtacatggac 4440
ttcacctctg cctcccagat gtctgactcc acccttcatg ccttcacatt ctcttcttct 4500
atgctgggag aagaggttca gctctatttc atcattccca aatccaaaga gagtcatttt 4560
gtcttcagca agcagggcaa gcacctggag agcatgcggc tgcccctggt ttcagacaag 4620
aatttgaatg cagtcaagag ccctattttc actccttcca gtggtcgaca tgagcacgga 4680
ctcctaaacc ttttccacgc catggagggc atcagccacc ttcacctcct ggtggtcaaa 4740
gagtatgaga tgcctctgta ccgcaagtac tggcccaacc acatcatgct ggtgcttccc 4800
ggcatgttca ataatgcagg cgtgggtaag ggcccccct gggatgggga gaagggcttc 4860
gaagataaac tgagctccat tcccctggca tgaaactttc agattcccct gcctctagaa 4920
attgtcctgc cctcctcacc actcttcgg                                  4949
```

<210> 228
<211> 9308
<212> DNA
<213> Homo sapiens

<400> 228

atgagtaact cccggaataa ccgggtgatg gtggaagggg ttggcgctcg ggtagtgcgc 60
ggcccggact ggaagtgggg gaagcaggac ggcggcgagg gccatgtggg caccgtccgg 120
agcttcgaga gccccgagga ggtggtggta gtgtgggaca acggcacagc tgccaactac 180
cgctgctccg ggcttacgaa cctccgcatc ctggacagcg cgcccaccgg catcaagcat 240
gatggaacca tgtgtgatac ctgccgccag caaccaatca ttggcattcg atggaagtgt 300
gcagagtgta caaattatga tttgtgcaca gtgtgttatc atggagataa acatcattta 360
agacatcgct tttaccgaat tactacaccg ggaagtgaga gggttctgtt agagtctcgt 420
aggaaatcta agaagattac agccagagga atctttgcag gtgccagagt ggtgcgagga 480
gtggactggc agtgggaaga tcaagatgga ggaaatggac gtaggggaaa ggtaacagaa 540
atccaggact ggagtgcatc aagcccacat agcgcagcat atgtcctctg ggataatggt 600
gctaagaacc tttacagagt tggctttgag ggcatgtctg atctgaaatg tgtccaggat 660
gccaagggag gttctttcta cagagatcac tgccctgtgc taggtgagca gaatggcaac 720
aggaatcctg gtggattgca gattggtgac ctggtaaata tagatctcga cctcgaaatt 780
gtacagtctt tgcagcatgg tcatggagga tggactgatg gaatgtttga gactttaact 840
acaactggaa ctgtttgtgg cattgatgaa gatcatgaca ttgtagtaca gtatccaagt 900
ggcaataggt ggaccttcaa tcctgctgtt ctcactaaag cgaacattgt ccgaagtgga 960
gatgctgctc agggtgcaga aggaggcacc tcgcagtttc aagtgggtga tcttgtacaa 1020
gtttgttatg acctggaacg aattaaactt ctacaaagag gacatggaga atgggctgaa 1080
gcgatgcttc caactttagg taaagttggc cgagtacaac agatttattc agacagtgat 1140
ttaaaggtgg aagtttgtgg aacatcttgg acatacaatc cagcagcagt ttccaaggtg 1200
gcatctgcag gatcagccat tagcaatgca tctggtgaaa gactctcaca actcctgaag 1260
aaattatttg aaacccaaga atctggtgac ctcaatgaag aattagttaa ggctgctgcc 1320
aatggagatg ttgctaaagt ggaagatttg cttaaaagac cagatgtgga tgtaaatggg 1380
caatgtgctg gccacacagc tatgcaagct gctagtcaga atggacatgt tgacattttg 1440
aagttacttt tgaagcaaaa cgtggatgtc gaagcagagg ataaagatgg tgatagagca 1500
gttcaccatg cagcttttgg agatgaaggc gctgttatag aagtactaca tcgaggtagt 1560
gctgatttga atgctcgaaa caagcgccga cagacaccac ttcatattgc tgtcaataaa 1620
ggtcatcttc aagttgtgaa gactttattg gactttggct gtcatcccag tctccaggat 1680
tctgaaggtg ataccccctct tcatgatgca ataagtaaga aacgtgatga tatcctagca 1740
gttcttttgg aagctggagc agatgttacc atcacaaaca ataatggatt taatgctctg 1800
catcatgctg cactaagggg aaatcccagt gcaatgcgtg ttttactatc taaattacca 1860
agaccatgga ttgtggatga gaagaaagat gatggttata ctgccttaca tctggctgcc 1920
cttaataatc acgtagaagt ggctgaactg ttggtacatc agggtaatgc aaacctggat 1980
atccagaatg tgaaccaaca aactgcccta caccttgctg ttgaacgaca gcatacccag 2040
attgttaggc ttttggtccg tgcaggtgcc aagcttgata ttcaggataa ggatggggat 2100
actcctttgc atgaagctct aaggcatcac actttgtctc agctacgtca gctccaagat 2160
atgcaagatg tggggaaggt ggatgctgcc tgggagccat ccaaaaacac gttaataatg 2220
ggacttggta cccagggggc agagaagaag agtgcagcat ctattgcctg tttcttggca 2280
gccaatggtg ctgacctgag cattcgaaat aagaagggtc aatcgccact tgatctctgt 2340
cctgatccga atctctgcaa agcactggca aagtgtcata aggaaaaagt cagtggtcaa 2400
gtgggttctc ggagtccttc tatgattagt aatgattctg aaaccttaga agagtgtatg 2460

```
gtgtgctcag atatgaagag agatactctt tttggtccat gtggacatat tgctacctgt 2520
tctttatgtt ctccacgtgt caagaaatgc ctcatctgta aagaacaggt tcaatccagg 2580
acaaagattg aagaatgtgt ggtatgctct gacaagaaag cagctgttct tttcaaccc 2640
tgtggccaca tgtgtgcttg tgagaactgt gctaacctga tgaaaaagtg tgtgcagtgt 2700
cgagcagtag ttgaacgaag agtgcctttc attatgtgct gtggagggaa aagttcagaa 2760
gatgccactg atgatatctc aagtgggaat attccagtat tacaaaagga caaggataat 2820
accaatgtca atgcagatgt gcaaaagttg cagcaacagt tacaagacat taaagagcag 2880
acaatgtgcc ctgtgtgtct agatcgtctg aagaatatga ttttcctttg tggtcacgga 2940
acctgtcaac tctgtggaga ccgcatgagt gaatgtccta tctgtcgcaa ggctattgaa 3000
cgaaggattc ttttgtatta actaagacac atggtgtatt ttgttagcta atgtatctag 3060
tcatgagatc ttaataggct tttgatctag ttggaagttc tgatgagtta atttctaata 3120
tcatagtttc tttactagag tataattggg ctgtaaatgt accagaacaa aaaccctac 3180
aaaatggtgt tggaaattgt gttttttgtt tttgttttaa atttgaaaca tcaaattcat 3240
gtaactcata ggataattta cctttggctt ctaagaggaa agtcctttaa ggatatcctt 3300
ttttaaaaaa ttgcattttt ctcttataat ttgtaaattt gttggatctc aaaagacata 3360
attctttgtg atcagttatc cttcatttca tcgtggtttt acacagtgag ttgataacag 3420
gttctctgag aagtcatgca tcaaataaaa gaggcaggtc aaacaattat gtcacatggt 3480
aaattataaa atgacagtac aagttccaga tagttaaggg aataccgaag ggatgattct 3540
tttttaaga taacaggaag ttacccacat gtttgtttct gaattcttag agtaaatgga 3600
agcatagaat gagggaataa tgactttgca tttctcttgt tttctagatt caaaaggaac 3660
attgtttaac ttgaatcaga ttaccagttt caaggtgact gatagacaag aaaaggaaaa 3720
ataagcaata atagtgggca actgaagaga aaaaaaaaac gagtatctat taactggcca 3780
ctaacagttg cctttcttac attaatttat acactatttt gttcagccag tgtttttaaa 3840
aaaaatctat gaaaagtgta cttccggttt tctgtgatta cttatctggg cttgatctga 3900
ccagtgaaat gacattgccc tatttggacc tctgaggttc tatttagctt tgcagatgta 3960
catagtatcc cagtgatctg caaaattaat gcctttcca agaaaaaatc ttttcttctc 4020
tgtatcagtt aattctgaca gtgttagtga ttctgtcttc attataggcc ttatttccat 4080
tatctctttc tttatagtat tttttgttat aaagaaaaca gtctttctgt gtatacctac 4140
ggatgagggt attatttaaa ctgccaacaa tatccaagac atggtcaata acctaattat 4200
aaatacttta gaaagagtga ccaggacatg tatagaaatg tctgcttacc tgtagacttt 4260
aaaaacaaac aaaatttttg gagcatttaa tcattttttt tctcctttta tctcctttgt 4320
aatcttattg tctcctgagt aaatatacac ataaatgttt ggggattcat tgctgctaga 4380
ttatatcagg tgtttacata gtgtctacta tatgctgttg ataagctttt tcctaaaaat 4440
agttatcctc ttttgtagtt tttttccctc ctttaaatct ggctagcttt tcttacttca 4500
gtttttatag aacaccaact aaatttgaaa gtatgaagtc tctagccctc ttacactgtt 4560
tctggctgtt taatccttgc tgcctttgca agtcctggtg aaagcaatgg ggagctttgc 4620
tggctggttt tgtagtctca acaattccat gtctgtgttt agctgagtaa ctgctatcat 4680
ttagaacagt aaaattttta tagatttcaa atttgaaaat tcatgtacac tcagttatct 4740
aataaggggc caccgacgct gtttttaaaa ctattttgaa gttataaaaa ggtagaacat 4800
ctgactgatg atgttcacat aaagagacag acagactatc atgttgcaga catgaaaaca 4860
gcatgtcagg tttttccttt ccttccatgc catgggctac ccatctcttc ccactagatc 4920
acctggtgag gagggagcac ctggctgact catgcagagt ttggccaatc agagaattgt 4980
gtggtctcca gctctacttt ccttaaacaa atcctttctg ttttctgagc tgcagtgcta 5040
aaacagatcc ctaaaaggta atagcatctt ccagactagt ttaattattt cctttataag 5100
aatatgacat aaatttttat tatagaaata atgtcattat aattttagtg ctagtacttg 5160
tgggttttct gtatttgtat caccttgaaa tttgttccat gaatagaaaa tacttcttta 5220
ttcatgtttt caatcactta gaattatttt aaatttattt tgggtttgtg agagataata 5280
agattaagaa cttcatcatt tcagtgactt tcctccaact ctctgaatct gctacctctt 5340
attatatgtc ccttagcagt acttttccag ttaggttcgt taccctgctc attagctaaa 5400
cctctttatc ttgtgcctca aaagagcttt tacttttctt agaaacattt ataaaaaaga 5460
aaaaaactga cacgcattca ttcaaaaaag tatttattgg gctttaggga ttagatacac 5520
aacagtggaa aaatggcatc ttagcacact cagaatttat tgtgcagcag ttgtgtattt 5580
taaggaatct gtagttaaaa ggaagagatc caaaggtcct gataacattt cctaacgttt 5640
cagaagagtt gtcattgagt agtgtttgga tttcctctct gtatccctca ccctaccatt 5700
ttctccttta ttcttctaag attgttagcg ttgttactca aaacatacac atacaacata 5760
ccagatggtg ctgttctaat ggtatttgtt cacgtaattt aacgttctta ctgcttatac 5820
tatatacata tttaattttg ttgggagcaa gaaggttttc aagaaaagat aattttgtat 5880
tagtgtagga atatattaga caaatgttta gttggtcaaa ttatgcataa gatatttgta 5940
actttaaaca ttgcaaattg accagctagc tatggaagca taaaattaat tgataattaa 6000
tgttggaaat acatttctaa aatttatatt tttatggtgt tagcctgtaa gagcatttta 6060
gcatattgaa atgcatgctg cttaagatgt gtctggttca aatatcaaaa cttgctgggt 6120
catttttaca tagtagaaac atactgtcta tgtgttgccc aattgctgtt tgtccactct 6180
gaattctgga cccttttggg actaagaatt agttaaattc atgaactaag agaattagag 6240
atggggtaga gtaggatcag gacagtgggg ttacaacaga taggaaagga aagtcagcat 6300
ttgagtgtag agaaaaaaag aggatcatac agatgtcatc aaattatagg ctttaataca 6360
```

ggttgctttt tttaatccaa aaggaaaagc acttatccta tcagaatgtc taccatgttt 6420
tatagtaata atttgtgtaa aaaattgaat ttttgaatag ttttttgtat tttttgtgat 6480
gaaaaactca taacataaaa tttaccatat taactgtttc taaagaatac agttcagcca 6540
tgtgaagtac atttacactg ttgtgaagca gatctccaga actttttcat cttgcaaaac 6600
tgaaactcca tacccattaa acaactcccc tttttgtttt ctcctcagtg gaatagaatt 6660
ttgactccat ataaatcaag aaacacctaa attctttagt tgtttgtgtt tgcaagatct 6720
aaggtcatgg taaacattaa gttcttaaaa tttttgggag ggacagtgca cctctcctct 6780
gaattgttag caattaaatt gagtaggttt taaatgtcta ttcattggaa gggtttgtta 6840
tttcattttg agccagaggg gagaggcaca ttttaaatat cagaattaga ttagctttga 6900
gtttgtacaa ttgggaacat aatagatttt cataaattat gtgtgccttg ttggaagtgt 6960
caactgtctt tatgtctgct tgtaaaagtt tcaaaatatg ttttccctca aaaaggcaac 7020
gttacttcat ttgcttgaat attatgatag gaatgcttac tgatattact tgatagtcat 7080
atatagccta ggaaatttaa catatatata actatagcag tattaataat gatagttgta 7140
cttctttaaa acattaaatt tgaggaaact ttaatgctgt ctcgtgtaca ttgctttact 7200
acagtgaggg ggaatatcct ttagattgag cctcaattta ctggttagta gtatgtgaaa 7260
ctctggtata aaaacgtaaa ctagacagta gagccgatga attaaaattg taaattgcta 7320
cattggcatt ttctacctcc ttttctgtca gagtattact ttttccagca tttattctta 7380
tttgtgagta aagaggaaat gggaacctga ggttaaaatt gacatttttg tttcattgag 7440
aatttaagca gtaggtacag gagaagtgac ttgtcacatt aatttggtgc ctaaatctgt 7500
aactacaagt tgtgatcgac atgtacaaaa tgtctaagaa aggtcatatg ctgaatattt 7560
tacttttcct gtatagtctg catgatttgt ttcataaacc cagcttattt cctccaaaaa 7620
gcaaaatggt cctgtaattt ttaaagtaaa ataaacgtgc cattttgtct gcaatctata 7680
atttcaggaa gttattgaaa gttctgactc agggcttttt aacagttcaa gcaattgtca 7740
gttatatttt ggaaactcca tctgtgtaat tctccagtgc cttgaaagaa ttattaactt 7800
ggcaacacta ttaaaacttt ataaaagatg gtctttagtg cacgtgtatc attatataca 7860
cgttttaaag tcatattgct tagcttgtta ataatgattc tgcatgtgtg ctgggtttgg 7920
gtaattcttt aaaggaagtt ttctagattt gcacttgatg tttgtttttt aaaaactgat 7980
tatttatggc cgtgacactg ttaccagaaa agtaattcta attaagttat tatgcaaagt 8040
catctataag tagcatctgg gaagaggaga tcgaggccac agtttgctat tttagtatga 8100
aaggaggatc tgtttgggaa acatagattg tcttcccctc aaatgagggg aaaaaaaaaa 8160
gacccctttgt tcaaatggat tctgttgtaa aaaattattt ttaaaggaaa tcacaaattg 8220
tatgtcattc ttaatgctag tcttatagaa taaatccata aaattgtttt tatgttcagt 8280
atgtttatgt cattctaaat gcagcaaatt caatgatagc agttcaattg actcatagca 8340
gtgttttgta ttttttctaa ttctttagct ttcaatattg gattaaagtc ttgtttgtga 8400
atatagtttc cgtatggcaa atgatttctt gcttattagc ttttgttaaa gaatgcttag 8460
taagagctaa gcttttaaaa gtaatgcaaa catttatcgt taataaaacc tatggtgtaa 8520
tatcatataa tgcttttctt tgatctttgg agaattattc ttttatagta gtatacatga 8580
attttgattt ttaaagcatt taaaaacaaa tctcaataca ttaaaaaacc tgttattgtt 8640
aaaaaggaaa ttaccatgcc tttaagaaac aaggatgtac atcttcaatt cagcataagt 8700
gtccacatct agaaggctct cattgcagtt gtttacagtt aaggtacctc tatctaaagg 8760
gccaaagaag catttcatat tttaacacct cacattcttt caggattaag acatatgaaa 8820
atagtctgaa taggataaat ttgataggaa gtaacttaac cagtctggaa gattcagctt 8880
tttctataaa aagcttattc ctcttcacaa ctcagatgta gatttcattt tcaagaggta 8940
gatattttaa agcaatgatc tatttgggtt ttagttttat gagtaaatca ttaattgtat 9000
ggtttggggg ttattttatt ttccatgtta atataagcac ttatttctga agttattgag 9060
aggtcatcta gctccagttc aaaagattat gtatatctga tgtttaagag gaaaaacatc 9120
tgaaaaaata tcttttgttt atttgaaaaa agcatatata ttccaacttt aaaattgtga 9180
atttatttg agtaacctct aattaactgt atttttttgt ttctgttgct gtatagttag 9240
aaatttcatg gcaatatttt ttactaaatt gaaactatag gttttaaaaa taaagacatt 9300
ttagaaaa 9308

<210> 229
<211> 1081
<212> DNA
<213> Homo sapiens

<400> 229

```
agtttgtagc ggacaacatg gcggccttca tgctgggctc gctgctgcgg acgttcaagc 60
agatggttcc ttcatcagct tcaggccaag ttcgaagtca ctatgtagac tggagaatgt 120
ggcgcgatgt gaagagacga aaaatggcct atgaatacgc agatgagagg ctacgtatta 180
attcactcag gaagaatacc attttgccaa aaattcttca ggatgtggct gatgaagaaa 240
ttgctgccct cccccgggat agctgtcctg ttagaatcag aaatcggtgt gttatgacgt 300
cccgtccgcg tggtgtgaag cggcgctgga ggcttagtcg tatagtcttc cgtcacttag 360
ctgaccatgg gcaactttct gggatccagc gagcgacatg gtaaatgagc tccagaacct 420
attgagcttg cagggaagcc aagcttgcag ttccagcaag caaagatttt ttttaataga 480

ccaaacccta atctctacag gggcccagta cagttgtttg gcctacctga tgctatctct 540
aaactacttt taaaatgaag acatttgggt gtttcatgtc agtgaattat cttttctctt 600
ggacttaaca aacatactgt tcccatacat agtacaaaga gtagcataat aaatattttc 660
tgtgaatgtt ttttataccc tccttttaag tttaatcttt tagtaacata taggggtttt 720
tgtattaaga gcattatttc ataaaatgct atcttataca taatccaact aattgacatg 780
tagctcatgt atggcagttt gtaatcctgg actagctttg ctgacctgca gagctcccct 840
taactattat tgttagataa acagattgga tagcaaggct gctgttctgg attatctaat 900
gctccacttc ttcttgtgat ggctgaacaa aagaaccact aagtgttttc ttgtccatcc 960
ttaagggact ttaaaacaaa ttatgcctct ttaagtcctt gaaagtctcc atctaaagag 1020
gaaaagtttg ttggaacagt atatattccc tatttttatg tttatttaat ccataacaga 1080
a                                                                 1081
```

<210> 230
<211> 2950
<212> DNA
<213> Homo sapiens

<400> 230

gcgccgagcg ccgccgccgc cgccgccgcc gccgctccgc tgcccgcgcc gcccgcggct 60
cccgatggag actgacgcgc cccagcccgg cctcgcctcc ccggactcgc cgcacgaccc 120
ctgcaagatg ttcatcgggg gactcagttg gcagactacg caggaagggc tgcgcgaata 180
cttcggccag ttcggggagg tgaaggagtg tctggtgatg cgggaccccc tgaccaagag 240
atccaggggt ttcggcttcg tcactttcat ggaccaggcg gggtggata aagtgctggc 300
gcaatcgcgg cacgagctcg actccaaaac aattgaccct aaggtggcct tccctcggcg 360
agcacagccc aagatggtga ctcgaacgaa gaagatcttt gtgggggggc tgtcggtgaa 420
caccacggtg gaggacgtga agcaatattt tgagcagttt gggaaggtgg acgacgccat 480
gctgatgttt gacaaaacca ccaaccggca ccgagggttc gggtttgtca cgtttgagag 540
tgaggacatc gtggagaaag tgtgtgaaat tcattttcat gaaatcaaca acaaaatggt 600
ggaatgtaag aaagctcagc caaaggaggt gatgtcgcca acgggctcag cccgggggag 660
gtctcgagtc atgccctacg gaatggacgc cttcatgctg ggcatcggca tgctgggtta 720
cccaggtttc caagccacaa cctacgccag ccggagttat acaggcctcg cccctggcta 780
cacctaccag ttccccgaat tccgtgtaga gcggacccct ctcccgagcg ccccagtcct 840
ccccgagctt acagccattc ctctcactgc ctacggacca atggcggcgg cagcggcggc 900
agcggctgtg gttcgaggga caggctctca cccctggacg atggctcccc ctccaggttc 960
gactcccagc cgcacagggg gcttcctggg gaccaccagc cccggcccca tggccgagct 1020
ctacggggcg gccaaccagg actcgggggt cagcagttac atcagcgccg ccagccctgc 1080
ccccagcacc ggcttcggcc acagtcttgg gggccctttg attgccacag ccttcaccaa 1140
tgggtaccac tgaagcaggg gacggtggca ggagcgcccc agcctgcagc tgactgagga 1200
ccacgagtga gccagcgagg gggcgggaga cctcagccgc agccgccgcc ccctcccctg 1260
cagcgactcg gacccgctac tgcctgcccc caactccccg ggcccggccc ctgccctgct 1320
gcccccaaca gcgtctggct cccctactaa cgtccccctc ttcgcccttg cccccatccc 1380
cacccgcccc tctcccggcc ctgcttttat ttattttgga ttagccggtt gcccacccca 1440
gccctctggt ccatccctcc ctccgtgccg cgccccccta ggaccgcccc ctccccaaaa 1500
ggcttttgga tttgtgcata gctggagtga aggcggaggg agcctgctac aggccgcagc 1560
ccaacccctg tttttattc agatttcccc tcctttaccc tttccctttt tttttttttt 1620
ttttttttta aagaaacctt ttttaaacta tttctaggtt tgtgaatgtg aagccccagg 1680
ccgcaggggg caagggggcca ggtgcccccc accagctgag aacaaagtgt ctatctgggt 1740
gtgggcccct ggccgcctcc ctccagccct ggagaggagg gcagggctgc ggggaggcca 1800
ggccgagccc ctggaaccat cccgtcctgt atcatatgta aatactgtga ggtgatgtgc 1860
ccacccctct ctaagacccc tcgggggtga ggggctcccc cctcccctgt ttctgtcccc 1920
tcagacaccg ttactgtaag cttgcaggcc tcagctgtgg ccacggcagg cccgctctct 1980
caggccctca gggtcaaggc cttggttgga cctgcccact ccaaaaaccc agtgtggggg 2040
caaagggcgt gggaagagca gggcttgccc agcgacactg ctggacagga attaactctc 2100
caaaggtctc ccctgctccc tacctaggtt ggggcttcat ggtttctgct cagtctgtcc 2160
cccttccccc tcgacccccg caatgagtgg gcaccagggg acgctctggc gagggcagac 2220
cccaggggaa agcaaagggc gtctcaggga acccccacat tctctcactg aagtttccca 2280
ccaggatgac ccccacagcca gagtcccttg gcagcccctc accccagacc ccccttctaa 2340
ggaaaaagag aggttcagag cgttggacct tcatgaacag tggcctggct ggcgtggcag 2400
ggccaaggcc cacccactgc catccccctt ctgtgtgtcc ctctccccca gttatgaggc 2460
ccagggcctg agctctcttc cccagcattg cccccacccg gaaaccccac ctttggagag 2520
ttaattgtct gtgtgaggtg cttaacctat cagccctgag aacacaaagc aataatcttt 2580
gttactgaga tgcgcggctg ttcgtgtttt gggttttttt tttttttaat gtttcctaat 2640
aaaagagaag ctgcatttta ttggttttta tttttaattt tctacacgtt tgagctgagt 2700
cctgagacac ttagcttccc tctccctcat tcccggaccc ttccacccca ctgggcccaa 2760
ccatgggctc aggaccctgg aattccgttt tctgatttgc tttgggattt tttttttttt 2820

taagatgtta catggtgttt cgaagccagc aagttaccat cctccggtgt ctcttctctc 2880
cacatctgta acttcttttt ccaggtttta ttttcagttt taaattccta ataaattatt 2940
tgaaaacgtt 2950

<210> 231
<211> 1012
<212> DNA
<213> Homo sapiens

<400> 231

```
tttttttttt tttttcttga atgtgcttcg caagccaggc tatcttccaa ggaaggcaga 60
cagtgggaga gcagagggac tgactgcagg caagcacatt gaagaaagac actggcgggt 120
ttccccaccc tcaccccaaa gcagaaaact agcagacgtc agctcagccc cgtcctgggc 180
acagacacta cacaaggaga cgctggaagt taagcaatac tttaatactg taatatgttt 240
gttttctttt tctttctttt ttttctacca aaaaaaagta agtaaactaa aacacaaaaa 300
catataaata aaatccatcc ctcttgtcgg ggacctgcag gggggcaacc ttaatccaaa 360
cacctggcta tcaaataatc agaatgtatt gtctcagaca ggatttcagt tccgggaggc 420
aggggcatga tgggggaggg ggccggaggc tgagagacaa aagttccaga gcctccctcg 480
aaggttctct actactgtat tctgtacata atgtaccatc ccatgtggaa tctgtgagtg 540
tcctcttaag tagcgtgggc tagccaatct gccgttcatg gtgtattgta aactccgaat 600
tccatatgta ataggatgca agtctaagcg tttcatgtgg acataaatgt atctaaataa 660
aactttccct agcactgtgg ctgacctcac ccttactttt atactttagt atgaaactga 720
tgagaacttt ggtagtgagt attttttta tatatataca tatatatgta tctatctata 780
tatatatctc aagcatcttt caggtctttg tgtgtggctt tcttaaagcc ctgttgtaaa 840
aaattactat gtggatggca gtctctcaca tcacagatgt ggaaagtata attttatatt 900
tgtattttca aataaataag tttgtgaaag gtttccatcc tctactgtgg tccagaaatc 960
aatgtgtttg tctgacaaaa aaaaaaaata aaataaaata aactgttttg aa         1012
```

<210> 232
<211> 4170
<212> DNA
<213> Homo sapiens

<400> 232

```
ctcttccctt aggtgtttaa gttccgcgcg caggccaggc tgcaacctga cggccagatc 60
cctcgctgtc ctagtcgctg ctccttggag tcatgttccc agccgcccct tctccgcgga 120
ccccgggtac cgggtcccga aggggcccgc tggccggact cgggcccggc tccacgcccc 180
ggacggctag caggaagggt ctgcccctgg ggtctgcagt cagctcccca gtgctcttct 240
cgccggtcgg ccggcgtagc tcgctaagct cgcggggaac accaacacga atgttcccac 300
accactccat aactgagtct gtgaactatg atgtgaaaac gtttggatct tctcttcctg 360
ttaaagtcat ggaagcccta acattggctg aagtcgatga ccagctgacc attaacatag 420
atgaaggtgg atgggcttgt ctggtgtgca aagagaagct cattatttgg aagattgctc 480
tgtcacctat tactaagtta tccgtttgca aagaacttca gctgccacct agtgatttcc 540
actggagtgc cgacttagtg gctctttctt actcttctcc ctcaggtgaa gcacattcta 600
ctcaggctgt tgctgtcatg gttgccacca gagaaggatc tatccgctat tggccaagcc 660
ttgctggtga agatacctac acagaggctt ttgtagattc gggaggtgat aagacttaca 720
gtttcctaac agcagtgcag ggaggaagtt ttattttgtc ttcatcagga agccaactaa 780
ttcggttgat acctgagagc tcaggaaaga ttcatcagca tatcctgcct caggggcaag 840
gcatgctttc aggaattggt cgaaaagttt cttctctttt tggaatttta tctcctagta 900
gtgatctcac actttcaagt gttctctggg atagagagag atcaagcttt tatagcctga 960
cgagttcaaa catcagtaaa tgggaattag atgattcttc agaaaagcat gcatacagtt 1020
gggatataaa tagagccctg aaggaaaaca ttaccgatgc tatttgggga tctgaaagta 1080
actatgaagc tattaaagaa ggagtcaaca ttcgatattt ggacttgaag caaaactgtg 1140
atgggctggt gattttggca gcagcatggc actcagcaga caatccatgt ctcatctatt 1200
actctctgat aacaatagaa gataatggtt gccaaatgtc agatgcagtt actgtagaag 1260
tcactcaata taatccacct tttcagtctg aagacctgat tttgtgtcag ttgacggtcc 1320
caaacttttc aaaccagact gcctatctgt ataacgaaag tgctgtctat gtgtgctcca 1380
caggaactgg gaaattttct cttccccagg agaaaattgt ctttaatgca caaggagata 1440
gtgttttagg tgctggtgcc tgtggtggtg ttcctatcat tttttctaga aacagtggac 1500
tggtgtctat tacttcaagg gaaaatgtgt ctatattggc agaagacttg gaagggtctt 1560
tagcatcttc agttgctgga ccaaacagtg agagtatgat ttttgagacc actacaaaga 1620
atgaaactat agcccaggaa gataaaatca agttgctgaa agctgccttt ctgcaatact 1680
gcagaaaaga tttaggtcat gctcaaatgg tggttgatga gctcttttcc tctcactctg 1740
atttggattc tgattctgaa ctagacaggg cagttaccca aatcagtgta gacctgatgg 1800
atgactaccc agcatctgac ccacggtggg ctgagtctgt ccctgaggaa gcacctgggt 1860
```

```
tcagcaatac gtcactgatt atccttcacc agctagaaga caagatgaaa gctcactctt 1920
ttcttatgga ctttattcat caagttggct tatttggacg tctaggcagt tttccagtta 1980
gagggacacc gatggccact cgactgttgc tctgtgagca tgccgaaaag ctgtcagccg 2040
ccattgttct caagaaccac cactcccggc tttctgacct tgtcaacaca gccatattga 2100
ttgctttgaa caagagggag tatgaaatcc catccaacct gactcctgca gatgtctttt 2160
tcagggaggt atcccaagta gataccatct gtgagtgctt actggagcat gaggagcaag 2220
tcttgaggga tgcacctatg gattccattg aatgggctga agtggtgatc aatgtgaaca 2280
atattctcaa ggatatgctg caggctgcta gtcattatcg ccaaaataga aactctttgt 2340
atagaagaga agaatcacta gaaaaagaac ctgaatatgt tccatggacg gcaacaagtg 2400
gtcctggtgg catccgaacg gtaataatac gccagcatga gattgtcctg aaggtggctt 2460
atccacaggc agacagcaac ctccgaaaca tcgtgaccga gcagctggta gccctgatcg 2520
attgcttcct ggatggttat gtttctcagc ttaagtctgt ggataaatcc agtaatcggg 2580
aaagatatga caatctggag atggaatacc tacagaaaag atcagatctc ttatctcctc 2640
ttctttcact aggccagtac ctgtgggctg cttctctagc agagaaatac tgtgactttg 2700
atatattggt acaaatgtgt gagcagactg acaaccagag ccgactccag cgctacatga 2760
cccagtttgc tgatcagaat tttcagact ttctcttccg ttggtatctg gagaaaggaa 2820
agcgaggcaa attattatct cagcccattt ctcagcatgg acagttggca aatttttgc 2880
aagctcatga acatctcagc tggttacatg aaattaatag ccaagaatta gaaaaggctc 2940
atgcaacact tctgggtttg gcaaatatgg aaactcgtta ctttgcaaag aagaaaaccc 3000
ttcttggctt gagtaaattg gctgcattag cttcagactt ttcagaggat atgctacaag 3060
aaaaaattga agaaatggct gagcaggagc gctttctact gcatcaggag accctacctg 3120
aacagctgct ggcggagaaa cagctaaatc tcagtgcgat gccagtattg actgcaccac 3180
aactcattgg tctatatatc tgtgaagaaa atagaagagc taatgaatat gatttcaaga 3240
aagctttgga cttgttggaa tatattgatg aggaagaaga tataaatata aatgatctaa 3300
aactggaaat cctttgcaaa gctcttcaga gagataactg gtccagttct gatggcaaag 3360
atgatccaat tgaagtatct aaagacagta tatttgtgaa gatcttacag aaacttttaa 3420
aagatggcat tcagctcagt gagtacttac cggaggtgaa agacctgcta caagcggatc 3480
agcttggaag cttaaagtcc aatccttact tcgagtttgt tttgaaagca aattatgaat 3540
attatgttca gggacaaata taactttttc taaaaatggc cattgtttat gaaatctgta 3600
taagtgtgtc cttatacaaa ttttaggcca taaacaagtg taagtttgta caatttcata 3660
acatgtatag ctgagttttt atactttata tgtaggaagc taatataaaa tagttatgta 3720
actgtgattt tggttttcag ttatgtgact tgtttttttcc acctgaaatg tgtcagttgt 3780
tgttcctgta ctcggtgccc tttcttttta ctctcacgtg gtcccaggtt ctggagttct 3840
tgtcctggtt ctagctgctc acatgtacaa atcacttcta ggcctcagtt tctgcgacta 3900
tgaaaattac tagattgcac tagcttgtct ctaaaattgc tgtgactcca gatactttgc 3960
actgaagaga atctagggtg tttgatatct gtttcagtta gggctaatgg gaaatgtcta 4020
gtaagataaa tgtcaacttt tgctgactta ttatgagatg aaaaaccaaa ggagagtggg 4080
cctaactcat gtgagcttga taactgatga actcattggg agcattttaa acttttctac 4140
ataaataata aatgagcact aatgaaagta                                  4170
```

<210> 233
<211> 2705
<212> DNA
<213> Homo sapiens

<400> 233

cacatggaaa ctgttcttaa agctgctggg ccctgaaatt ttactcagca gtttgaaatc 60
aagacatagc ttttctcatt caccctccca cttggggcta atgcacagac atgaacatct 120
attgaggaaa accacaaaaa acttcaaaac agctacaacg actctgcagt ctacacttct 180
cctgttactg cttgtgcctc tgataaagcc agcaccacca acccagcagg actcacgcat 240
tatctatgat tatggaacag ataattttga agaatccata tttagccaag attatgagga 300
taaatacctg gatggaaaaa atattaagga aaaagaaact gtgataatac ccaatgagaa 360
aagtcttcaa ttacaaaaag atgaggcaat aacaccatta cctcccaaga aagaaaatga 420
tgaaatgccc acgtgtctgc tgtgtgtttg tttaagtggc tctgtatact gtgaagaagt 480
tgacattgat gctgtaccac ccttaccaaa ggaatcagcc tatctttacg cacgattcaa 540
caaaattaaa aagctgactg ccaaagattt tgcagacata cctaacttaa gaagactcga 600
ttttacagga aatttgatag aagatataga agatggtact ttttcaaaac tttctctgtt 660
agaagaactt tcacttgctg aaaatcaact actaaaactt ccagttcttc ctcccaagct 720
cactttattt aatgcaaaat acaacaaaat caagagtagg ggaatcaaag caaatgcatt 780
caaaaaactg aataacctca ccttcctcta cttggaccat aatgccctgg aatccgtgcc 840
tcttaattta ccagaaagtc tacgtgtaat tcatcttcag ttcaacaaca tagcttcaat 900
tacagatgac acattctgca aggctaatga caccagttac atccgggacc gcattgaaga 960
gatacgcctg gagggcaatc caatcgtcct gggaaagcat ccaaacagtt ttatttgctt 1020
aaaaagatta ccgatagggt catactttta acctctattg gtacaacata taaatgaaag 1080
tacacctaca ctaatagtct gtctcaacaa tgagtaaagg aacttaagta ttggtttaat 1140

attaaccttg tatctcattt tgaaggaatt taatatttta agcaaggatg ttcaaaatct 1200
tacatataat aagtaaaaag taagactgaa tgtctacgtt cgaaacaaag taatatgaaa 1260
atatttaaac agcattacaa aatcctagtt tatactagac taccatttaa aaatcatgtt 1320
tttatataaa tgcccaaatt tgagatgcat tattcctatt actaatgatg taagtacgag 1380
gataaatcca agaaactttc aactctttgc ctttcctggc ctttactgga tcccaaaagc 1440
atttaaggta catgttccaa aaactttgaa aagctaaatg tttcccatga tcgctcattc 1500
ttcttttatg attcatacgt tattccttat aaagtaagaa ctttgttttc ctcctatcaa 1560
ggcagctatt ttattaaatt tttcacttag tctgagaaat agcagatagt ctcatattta 1620
ggaaaacttt ccaaataaaa taaatgttat tctctgataa agagctaata cagaaatgtt 1680
caagttattt tactttctgg taatgtcttc agtaaaatat tttctttatc taaatattaa 1740
cattctaagt ctaccaaaaa aagttttaaa ctcaagcagg ccaaaaccaa tatgcttata 1800
agaaataatg aaaagttcat ccatttctga taaagttctc tatggcaaag tctttcaaat 1860
acgagataac tgcaaaatat tttcctttta tactacagaa atgagaatct catcaataaa 1920
ttagttcaag cataagatga aaacagaata ttctgtggtg ccagtgcaca ctaccttccc 1980
acccatacac atccatgttc actgtaacaa actgaatatt cacaataaag cttctgagta 2040
acactttctg attactcatg ataaactgac atggctaact gcaagaatta aatcttctat 2100
ctgagagtaa taatttatga tgactcagtg gtgccagagt aaagtttcta aaataacatt 2160
cctctcactt gtaccccact aaaagtatta gactacacat tacattgaag ttaaacacaa 2220
aattatcagt gttttagaaa catgagtccg gactgtgtaa gtaaaagtac aaacattatt 2280
tccaccataa agtatgtatt gaaatcaagt tgtctctgtg tacagaatac atacttattc 2340
ccatttttaa gcatttgctt ctgttttccc tacctagaat gtcagatgtt tttcagttat 2400
ctccccattt gtcaaagttg acctcaagat aacatttttc attaaagcat ctgagatcta 2460
agaacacaat tattattcta acaatgatta ttagctcatt cacttatttt gataactaat 2520
gatcacagct attatactac tttctcgtta ttttgtgtgc atgcctcatt tccctgactt 2580
aaacctcact gagagcgcaa aatgcagctt tatacttttt actttcaatt gcctagcaca 2640
atagtgagta catttgaatt gaatatataa taaatattgc aaaataaaat ccatctaaat 2700
agaaa 2705

<210> 234
<211> 4001
<212> DNA
<213> Homo sapiens

<400> 234

aggcatcagc aatctatcag ggaacggcgg tggccggtgc ggcgtgttcg gtggcggctc 60
tggccgctca ggcgcctgcg gctgggtgag cgcacgcgag gcggcgaggc ggcagcgtgt 120
ttctaggtcg tggcgtcggg cttccggagc tttggcggca gctaggggag gatggcggag 180
tcttcggata agctctatcg agtcgagtac gccaagagcg ggcgcgcctc ttgcaagaaa 240
tgcagcgaga gcatccccaa ggactcgctc cggatggcca tcatggtgca gtcgcccatg 300
tttgatggaa aagtcccaca ctggtaccac ttctcctgct tctggaaggt gggccactcc 360
atccggcacc ctgacgttga ggtggatggg ttctctgagc ttcggtggga tgaccagcag 420
aaagtcaaga agacagcgga agctggagga gtgacaggca aaggccagga tggaattggt 480
agcaaggcag agaagactct gggtgacttt gcagcagagt atgccaagtc caacagaagt 540
acgtgcaagg ggtgtatgga gaagatagaa aagggccagg tgcgcctgtc caagaagatg 600
gtggacccgg agaagccaca gctaggcatg attgaccgct ggtaccatcc aggctgcttt 660
gtcaagaaca gggaggagct gggtttccgg cccgagtaca gtgcgagtca gctcaagggc 720
ttcagcctcc ttgctacaga ggataaagaa gccctgaaga agcagctccc aggagtcaag 780
agtgaaggaa agagaaaagg cgatgaggtg gatggagtgg atgaagtggc gaagaagaaa 840
tctaaaaaag aaaaagacaa ggatagtaag cttgaaaaag ccctaaaggc tcagaacgac 900
ctgatctgga acatcaagga cgagctaaag aaagtgtgtt caactaatga cctgaaggag 960
ctactcatct tcaacaagca gcaagtgcct tctggggagt cggcgatctt ggaccgagta 1020
gctgatggca tggtgttcgg tgccctcctt ccctgcgagg aatgctcggg tcagctggtc 1080
ttcaagagcg atgcctatta ctgcactggg gacgtcactg cctggaccaa gtgtatggtc 1140
aagacacaga cacccaaccg gaaggagtgg gtaaccccaa aggaattccg agaaatctct 1200
tacctcaaga aattgaaggt taaaaaacag gaccgtatat tcccccaga aaccagcgcc 1260
tccgtggcgg ccacgcctcc gccctccaca gcctcggctc ctgctgctgt gaactcctct 1320
gcttcagcag ataagccatt atccaacatg aagatcctga ctctcgggaa gctgtcccgg 1380
aacaaggatg aagtgaaggc catgattgag aaactcgggg ggaagttgac ggggacggcc 1440
aacaaggctt ccctgtgcat cagcaccaaa aaggaggtgg aaaagatgaa taagaagatg 1500
gaggaagtaa aggaagccaa catccgagtt gtgtctgagg acttcctcca ggacgtctcc 1560
gcctccacca agagccttca ggagttgttc ttagcgcaca tcttgtcccc ttgggggca 1620
gaggtgaagg cagagcctgt tgaagttgtg gccccaagag ggaagtcagg ggctgcgctc 1680
tccaaaaaaa gcaagggcca ggtcaaggag gaaggtatca acaaatctga aaagagaatg 1740
aaattaactc ttaaaggagg agcagctgtg gatcctgatt ctggactgga acactctgcg 1800
catgtcctgg agaaaggtgg gaaggtcttc agtgccaccc ttggcctggt ggacatcgtt 1860

```
aaaggaacca actcctacta caagctgcag cttctggagg acgacaagga aaacaggtat 1920
tggatattca ggtcctgggg ccgtgtgggt acggtgatcg gtagcaacaa actggaacag 1980
atgccgtcca aggaggatgc cattgagcac ttcatgaaat tatatgaaga aaaaaccggg 2040
aacgcttggc actccaaaaa tttcacgaag tatcccaaaa agttctaccc cctggagatt 2100
gactatggcc aggatgaaga ggcagtgaag aagctgacag taaatcctgg caccaagtcc 2160
aagctcccca agccagttca ggacctcatc aagatgatct ttgatgtgga aagtatgaag 2220
aaagccatgg tggagtatga gatcgacctt cagaagatgc ccttggggaa gctgagcaaa 2280
aggcagatcc aggccgcata ctccatcctc agtgaggtcc agcaggcggt gtctcagggc 2340
agcagcgact ctcagatcct ggatctctca aatcgctttt acaccctgat cccccacgac 2400
tttgggatga agaagcctcc gctcctgaac aatgcagaca gtgtgcaggc caaggtggaa 2460
atgcttgaca acctgctgga catcgaggtg gcctacagtc tgctcagggg agggtctgat 2520
gatagcagca aggatcccat cgatgtcaac tatgagaagc tcaaaactga cattaaggtg 2580
gttgacagag attctgaaga agccgagatc atcaggaagt atgttaagaa cactcatgca 2640
accacacaca atgcgtatga cttggaagtc atcgatatct ttaagataga gcgtgaaggc 2700
gaatgccagc gttacaagcc ctttaagcag cttcataacc gaagattgct gtggcacggg 2760
tccaggacca ccaactttgc tgggatcctg tcccagggtc ttcggatagc cccgcctgaa 2820
gcgcccgtga caggctacat gtttggtaaa gggatctatt tcgctgacat ggtctccaag 2880
agtgccaact actgccatac gtctcaggga gacccaatag gcttaatcct gttgggagaa 2940
gttgcccttg gaaacatgta tgaactgaag cacgcttcac atatcagcaa gttacccaag 3000
ggcaagcaca gtgtcaaagg tttgggcaaa actacccctg atccttcagc taacattagt 3060
ctggatggtg tagacgttcc tcttgggacc gggatttcat ctggtgtgaa tgacacctct 3120
ctactatata acgagtacat tgtctatgat attgctcagg taaatctgaa gtatctgctg 3180
aaactgaaat tcaattttaa gacctccctg tggtaattgg gagaggtagc cgagtcacac 3240
ccggtggctc tggtatgaat tcacccgaag cgcttctgca ccaactcacc tggccgctaa 3300
gttgctgatg ggtagtacct gtactaaacc acctcagaaa ggattttaca gaaacgtgtt 3360
aaaggttttc tctaacttct caagtccctt gttttgtgtt gtgtctgtgg ggaggggttg 3420
ttttggggtt gtttttgttt tttcttgcca ggtagataaa actgacatag agaaaaggct 3480
ggagagagat tctgttgcat agactagtcc tatggaaaaa accaagcttc gttagaatgt 3540
ctgccttact ggtttcccca gggaaggaaa aatacacttc caccctttttt tctaagtgtt 3600
cgtctttagt tttgattttg gaaagatgtt aagcatttat ttttagttaa aaataaaaac 3660
taatttcata ctatttagat tttctttttt atcttgcact tattgtcccc tttttagttt 3720
tttttgtttg cctcttgtgg tgaggggtgt gggaagacca aaggaaggaa cgctaacaat 3780
ttctcatact tagaaacaaa aagagctttc cttctccagg aatactgaac atgggagctc 3840
ttgaaatatg tagtattaaa agttgcattt gaaattcttg actttcttat gggcactttt 3900
gtcttccaaa ttaaaactct accacaaata tacttaccca agggctaata gtaatactcg 3960
attaaaaatg cagatgcctt ctctaaaaaa aaaaaaaaaa a                     4001
```

<210> 235
<211> 2309
<212> DNA
<213> Homo sapiens

<400> 235

```
gcgggggcgg gccggggcgg ggccaggccg gctagagggg cgggtctagc ggcggccccc 60
ggcgaagttc actgcgcttg cgctgacaga cgcaagatgg cggacagtgc ggaactaaag 120
caaatggtta tgagccttag agtttctgaa ctccaagtac tgttgggcta cgccgggaga 180
aacaagcacg gacgcaaaca cgaacttctc acaaaagccc tgcatttgct aaaggctggc 240
tgtagtcctg ctgtgcaaat gaaaattaag gaactctata ggcggcggtt cccacagaaa 300
atcatgacgc ctgcagactt gtccatcccc aacgtacatt caagtcctat gccagcaact 360
ttgtctccat ctaccattcc acaactcact tacgatggtc accctgcatc atcgccatta 420
ctccctgttt ctcttctggg acctaaacat gaactggaac tcccacatct tacatcagct 480
cttcacccag tccatccgga tataaaactt caaaaattac cattttatga tttactggat 540
gaactgataa aacccaccag tctagcatca gacaacagtc agcgctttcg agaaacctgt 600
tttgcatttg ccttgacacc acaacaagtg cagcaaatca gtagttccat ggatatttct 660
gggaccaaat gtgacttcac agtacaggtc cagttaaggt tttgtttatc agaaaccagt 720
tgtccacaag aagatcactt cccacccaat ctttgtgtga aagtgaatac aaaaccttgc 780
agccttccag gttaccttcc acctacaaaa aatggcgtgg aaccaaagcg acccagccga 840
ccaattaata tcacctcact tgtccgactg tccacaacag taccaaacac gattgttgtt 900
tcttggactg cagaaattgg aagaaactat tccatggcag tatatcttgt aaaacagttg 960
tcctcaacag ttcttcttca gaggttacga gcaaagggaa taaggaatcc ggatcattct 1020
agagctttaa ttaaagagaa gttgactgcg gatccagaca gtgaaatagc tacaaccagc 1080
ctaagggttt ctctactatg tccacttggt aaaatgcggc tgacaattcc gtgtcgggcc 1140
cttacatgtt ctcatctaca atgttttgac gcaactcttt acattcagat gaatgagaaa 1200
aaaccaacct gggtttgtcc tgtctgtgat aagaaggctc catatgaaca ccttattatt 1260
gatggcttgt ttatggaaat cctaaagtac tgtacagact gtgatgaaat acaatttaag 1320

gaggatggca cttgggcacc gatgagatca aaaaaggaag tacaggaagt ttctgcctct 1380
tacaatggag tcgatggatg cttgagctcc acattggagc atcaggtagc gtctcaccac 1440
cagtcctcaa ataaaaacaa gaaagtagaa gtgattgacc taaccataga cagttcatct 1500
gatgaagagg aagaagagcc atctgccaag aggacctgtc cttccctatc tcccacatca 1560
ccactaaata ataaaggcat tttaagtctt ccacatcaag catctccagt atcccgcacc 1620
ccaagccttc ctgctgtaga cacaagctac attaatacct ccctcatcca agactatagg 1680
catcctttcc acatgacacc catgccttac gacttacaag gattagattt ctttcctttc 1740
ttatcaggag acaatcagca ttacaacacc tccttgcttg ccgctgcagc agcagcagtt 1800
tcagatgatc aagacctcct acactcgtct cggtttttcc cgtatacctc ctcacagatg 1860
tttcttgatc agttaagtgc aggaggcagt acttctctgc caaccaccaa tggaagcagt 1920
agtggcagta acagcagcct ggtttcttcc aacagcctaa gggaaagcca tagccacacc 1980
gtcacaaaca ggagcagcac ggacacggca tccatctttg gcatcatacc agacattatt 2040
tcattggact gattcccagg ccctgctgct cccatcccca ccccagatcg aatgaacttg 2100
gcagaaagaa gagaactttg tgctctgttt taccttactc tgtttagaaa agtatacaag 2160
cgtgtttttt ttcctttttt tagggaaaaa attaaaagaa atgtacagag aacaaaacta 2220
tattttcagt tttacttttg tatataaatc taagactgcc tgtgtgataa aacacttgtt 2280
taaaaaaaaa aaaaaaaaaa aaaaaaaaa                                    2309
```

<210> 236
<211> 3157
<212> DNA
<213> Homo sapiens

<400> 236

cccgccccga ctgggcaggc gggggagccc ctacttctct ccccccgggc gggggagccg 60
gggggcagcg ccggagcccg gggggagctc agccccgccg accggccggc cagggcaggg 120
ggcagctagg acggccccgg tccaggtgga ggccgcagag ggcccagggc aagcagaggc 180
agcaatggtt ggtcctgacg gtggctgagc cccagcccc tggaatatgc agcccggggg 240
agccccagac agcggcaagg acgaggtggc ggagtggggc gggaggcatg gtctccacct 300
accgggtggc cgtgctgggg gcgcgaggtg tgggcaagag tgccatcgtg cgccagttct 360
tgtacaacga gttcagcgag gtctgcgtcc ccaccaccgc ccgccgcctt tacctgcctg 420
ctgtcgtcat gaacggccac gtgcacgacc tccagatcct cgactttcca cccatcagcg 480
ccttccctgt caatacgctc caggagtggg cagacacctg ctgcagggga ctccggagtg 540
tccacgccta catcctggtc tacgacatct gctgctttga cagctttgag tacgtcaaga 600
ccatccgcca gcagatcctg gagacgaggg tgatcggaac ctcagagacg cccatcatca 660
tcgtgggcaa caagcgggac ctgcagcgcg gacgcgtgat cccgcgctgg aacgtgtcgc 720
acctggtacg caagacctgg aagtgcggct acgtggaatg ctcggccaag tacaactggc 780
acatcctgct gctcttcagc gagctgctca agagcgtcgg ctgcgcccgt tgcaagcacg 840
tgcacgctgc cctgcgcttc cagggcgcgc tgcgccgcaa ccgctgcgcc atcatgtgac 900
gcctgcgcgc ccctcgggct gcaccggcac tggccgagcg gagggcgggg ccgtactgcg 960
gggctggggc ggggagcggg cgggaaatgg aactgtgacg gtcccggcct gaggcccctg 1020
cagccacgca cctcccggtg agaagcagag cgcgagaggg agccctccgt aactgcccag 1080
ccctgcccct tgcccccgtg gcttcctggg acagccgcct tcagtgctgt atttagtgca 1140
gtgcccggcc cgacccgcgg gggtgccaca gccttttggg atgggggtga gcgtgcaatg 1200
gaggctgggg gtggcgaggt gccgccttgg ccgggccccc acgtgtcttc tccagaatgt 1260
gtctgtcttt gcctggtgtc ttcctttccc gtgtccgccc accccagcgt ctgttggtac 1320
ttacctgtct cacctaccct ccagtcccct cccagctccg ctcacagggc tctcatttcg 1380
tccatcccct tgtcgcagat cctggcagct tctttgtgag gccaggcctt ctgactgtca 1440
gcaccaccgg cacagggcag agatgcgggt ggcccaagga ccacgatcaa ggggtccggg 1500
ggaccgaggt cccagatcag tgaggggaga aggttgagct ctccggcttc cagggagacc 1560
tccccgccca gcagccccca gagacacaac aacctacctt ccagccttaa ctcgatggtc 1620
cgtccctgcc aggtgcccct cactcttcct gaccccaaag ccagatcacc ccctgggtta 1680
aaactttttt tctttttttt tttggacaga gtgtggaaag ggagcccccc aaaggatagc 1740
ttctttttca tgatgccagg ctccagtcct ttattccctt ctgcatactg caatctgatc 1800
tgtcagactg gggaatgttg ggttctgggg tctggtcgtg ggcaggatgg tgcccagaag 1860
ggggttaggt tgtcccagtg aaaattctgt tgccccgtct caaccccatc tgactacccc 1920
agactctgcc tgcctcagat ctcagactat cctgattaat ctggggaaga acagagccag 1980
ggaaagaatg gtggggaccc ctgtacttgg gggagacaca cctgcatctt cctcctgcca 2040
cagatggagg ccctcaggat ctgacaccct cttgtcccaa caccagtcag ccctataccc 2100
taactcactc caccccattt tctccggctg cctggccggg tttctacctc tcgtcaccgg 2160
agctgatcac tgtcagtttt gtaccaattt agaaataaca ataataatga agattctagg 2220
aatggcatga gggattgatg ggggacttgg agggagggac aagtggtgcc ctgtcccctg 2280
ctcccctggc caaagaaagc tgtccttgag gctgagccct cagccctggc ctggtggggg 2340
gacagcaagg tcccttgtta taagaggggc agagaggaca actccgcttt ggccaaccta 2400
gccaaggctg cagcatatag accaggaaat caggtagccc agactggtga tggagcagag 2460

tctgggggaa gggtcgtggg tggggaattt atcaccaaca tccattgtag gggggaatcta 2520
tgattctgct tccccagcgg attcccactc tgtccaccaa gtggggggta gcacagcctc 2580
acagcaaccg ccctgacctt gggcagtcta gtgttcctgc attctagtcc ctgctgtgct 2640
gcaggacttt gggcaagtga cctgccctct gtgagcctcc ctctgacaca gaggaggtgg 2700
ctccccttcc ccacacctta gagtggctgg gagggtaaca aagagggcct gccccctttag 2760
tctcctgcac ccctgccccc tggttcacca gagggagcgg atgaaggatg gcagcatctc 2820
acatgcccca tcaccaactc tgaggcacct ggggtggggg ggcggagccc aggcctctgg 2880
ctgctcccct gtgggagcca ttggaatgta tcccctgaca ggccccccttc cgcctccacc 2940
tcaacccagg tcttggattt caggtccctc cacccccatt ctgagtctct gtccttctcc 3000
ttccacccgc tcccagggtt tcccaccaca gggtctggaa gtgtgtgtga cgcccattga 3060
gctgttaccc gaagtcagat taaaaatcag ggagtgtttt ccctcgtttc tgtaaaaaaa 3120
aaaaaaaaaa aaaaaaaaaa aaaaaaaaaa aaaaaaa 3157

<210> 237
<211> 1115
<212> DNA
<213> Homo sapiens

<400> 237

```
caaagttcat agcaggttga acatgcttgc tgtcattttc acagattctg gtctttctta 60
tggggaacct gagttcctgc attaaagttc ccccagaaga tcaatggaag gtggattcca 120
aagagtgggc aagtgaggac cccttctctg ttaccaaggt gaccccaagg aacacagtaa 180
atgtggcggc ttatttggcc tccccaggac ggactggagc atcagtagtg cctgagttca 240
tcaaaggaca gatgctcaag acaccctgat caccattagg tggaattgaa ggagccaaaa 300
atgggcgcag tggctcctca gcagagacgc tcctgaatgt atagacatgg gaaccacttt 360
cagcatcaaa aaaggaaacg ttctgcatgc ccatatccag aaaaatcccc actcgctgta 420
acttgcggtc tactaagagg aaagtcagcg gcaccgtgct ggcagagagg cggcttccat 480
ccctcaaact cacagtccag aatccaagct ctgtggtcag ctggatcttc cctttgcagt 540
gaacagattc tctgcagact cccaggtccc attctgtgct tgttcccacg tccacctccc 600
agtagtggcg gccacaggta aagcgagggg agcccaggat gcaaacggac acgtcaaatc 660
tctcggcaag gtcttgccga ttctgtgtga tgagcccact tcggacgctc ctgaggtcgt 720
cagaaatgag gaggaagttg ttggctgtgt cggcatccaa ggtcatatcc actgtgaaaa 780
ggaaaacaag ttgctcagca agtggacaga agccaacccc atgcctctct tctacttcaa 840
aggcccaaat atctcctgac tttagtttct ttaattctgt ttcttcctcc aaaatcaaaa 900
cttttcatga ggaaacttcc ctatttcaaa ttttcatagg tatgctttgt ggcaatacgt 960
gactcttatt cacagtagca attcttattt tactttacgt cacgtgtttg cttcactgaa 1020
cttttcttca tttagagtgg agggtctctg aaggcagaca ccatgccccc ctttctacca 1080
ccttgatgtg tgaacaaata aatgataatt aagac                            1115
```

<210> 238
<211> 2583
<212> DNA
<213> Homo sapiens

<400> 238

```
gcatgcgcgc tcgcgctccc gccctctagc tgcgctcggc tgagtcagtc agtctgtcgg 60
agtctgtcct cggagcaggc ggagtaaagg gacttgagcg agccagttgc cggattattc 120
tatttcccct ccctctctcc cgccccgtat ctcttttcac ccttctccca ccctcgctcg 180
cgtagccatg gcggagccgt cggcggccac tcagtcccat tccatctcct cgtcgtcctt 240
cggagccgag ccgtccgcgc ccggcggcgg cgggagccca ggagcctgcc ccgccctggg 300
gacgaagagc tgcagctcct cctgtgcggt gcacgatctg attttctgga gagatgtgaa 360
gaagactggg tttgtctttg gcaccacgct gatcatgctg ctttccctgg cagctttcag 420
tgtcatcagt gtggtttctt acctcatcct ggctcttctc tctgtcacca tcagcttcag 480
gatctacaag tccgtcatcc aagctgtaca gaagtcagaa gaaggccatc cattcaaagc 540
ctacctggac gtagacatta ctctgtcctc agaagctttc cataattaca tgaatgctgc 600
catggtgcac atcaacaggg ccctgaaact cattattcgt ctctttctgg tagaagatct 660
ggttgactcc ttgaagctgg ctgtcttcat gtggctgatg acctatgttg gtgctgtttt 720
taacggaatc acccttctaa ttcttgctga actgctcatt ttcagtgtcc cgattgtcta 780
tgagaagtac aagacccaga ttgatcacta tgttggcatc gcccgagatc agaccaagtc 840
aattgttgaa aagatccaag caaaactccc tggaatcgcc aaaaaaaagg cagaataagt 900
acatggaaac cagaaatgca acagttacta aaacaccatt taatagttat aacgtcgtta 960
cttgtactat gaaggaaaat actcagtgtc agcttgagcc tgcattccaa gctttttttt 1020
taatttggtg ttttctccca tcctttccct ttaaccctca gtatcaagca caaaaattga 1080
tggactgata aaagaactat cttagaactc agaagaagaa agaatcaaat tcataggata 1140
agtcaatacc ttaatggtgg tagagccttt acctgtagct tgaaagggga aagattggag 1200
```

```
gtaagagaga aaatgaaaga acacctctgg gtccttctgt ccagttttca gcactagtct 1260
tactcagcta tccattatag ttttgccctt aagaagtcat gattaactta tgaaaaaatt 1320
atttggggac aggagtgtga taccttcctt ggtttttttt tgcagccctc aaatcctatc 1380
ttcctgcccc acaatgtgag cagctacccc tgatactcct tttctttaat gatttaacta 1440
tcaacttgat aaataactta taggtgatag tgataattcc tgattccaag aatgccatct 1500
gataaaaaag aatagaaatg gaaagtggga ctgagaggga gtcagcaggc atgctgcggt 1560
ggcggtcact ccctctgcca ctatccccag ggaaggaaag gctccgccat ttgggaaagt 1620
ggtttctacg tcactggaca ccggttctga gcattagttt gagaactcgt tcccgaatgt 1680
gctttcctcc ctctcccctg cccacctcaa gtttaataaa taaggttgta cttttcttac 1740
tataaaataa atgtctgtaa ctgctgtgca ctgctgtaaa cttgttagag aaaaaaataa 1800
cctgcatgtg ggctcctcag ttattgagtt tttgtgatcc tatctcagtc tggggggaa 1860
cattctcaag aggtgaaata cagaaagcct ttttttcttg atcttttccc gagattcaaa 1920
tctccgattc ccatttgggg gcaagttttt ttcttcacct tcaatatgag aattcagcga 1980
acttgaaaga aaaatcatct gtgagttcct tcaggttctc actcatagtc atgatccttc 2040
agagggaata tgcactggcg agtttaaagt aagggctatg atatttgatg gtcccaaagt 2100
acggcagctg caaaaagtag tggaaggaaa ttgtctacgt gtcttggaaa aattagttag 2160
gaatttggat gggtaaaagg taccccttgcc ttactccatc ttatttcttt agccccctttt 2220
gagtgtttta actggtttca tgtcctagta ggaagtgcat tctccatcct catcctctgc 2280
cctcccagga agtcagtgat tgtctttttg ggcttcccct ccaaaggacc ttctgcagtg 2340
gaagtgccac atccagttct tttcttttgt tgctgctgtg tttagataat tgaagagatc 2400
tttgtgccac acaggatttt tttttttttt taagaaaaac ctatagatga aaaattacta 2460
atgaaactgt gtgtacgtgt ctgtgcgtgc aacataaaaa tacagtagca cctaaggagc 2520
ttgaatcttg gttcctgtaa aatttcaaat tgatgtggta ttaataaaaa aaaaaaaaaa 2580
aca                                                               2583
```

<210> 239
<211> 3850
<212> DNA
<213> Homo sapiens

<400> 239

cggcggcggc agcggcagag gtggcggcgg cgtgtccgtg agagtggcgt gggggcgggg 60
aggagaggcg gcggcggcag tgtccaagct acgccactcg ggccggggcg ttgggagcgg 120
gagtgcagag cgtggtcgtg gcggcggcgg tgagaagagc gaggcggagg aggggggtgcc 180
atggccgggc agcagttcca gtacgatgac agtgggaaca ccttcttcta cttcctcacc 240
tccttcgtgg ggctcatcgt gatcccggcg acatactacc tctggccccg agatcagaat 300
gccgagcaaa ttcgattaaa gaatatcaga aaagtatatg gaaggtgtat gtggtatcgt 360
ttacggttat taaaacccca gccaaatatt attcctacag taaagaaaat agttctgctt 420
gcaggatggg cattgttctt attccttgca tataaagttt ccaaaacaga ccgagaatac 480
caagaataca atccttatga agtattaaat ttggatcctg gagccacagt agcagaaatt 540
aaaaaacaat atcgtttgct gtcacttaaa tatcatccag ataaaggagg tgatgaggtt 600
atgttcatga ggatagcaaa agcttatgct gctttaacgg atgaagagtc ccggaaaaat 660
tgggaagaat ttggaaatcc agatgggcct caagccacaa gctttggaat tgccctgcca 720
gcttggatag ttgaccagaa aaactcaatt ctggttttac ttgtatatgg attggcattt 780
atggttatcc ttccagttgt tgtgggctct tggtggtatc gctcaatacg ctatagtgga 840
gaccagattc taatacgcac aacacagatt tatacatact ttgtttataa aacccgaaat 900
atggatatga aacgtcttat catggttttg gctggagctt ctgaatttga tcctcagtat 960
aataaagatg ccacaagcag accaacggat aatattctaa taccacagct aatcagagaa 1020
attggcagca ttaatttaaa gaagaatgag cctccactta cctgcccata tagcctgaag 1080
gccagagttc ttttactgtc tcatcttgct agaatgaaaa ttcctgagac ccttgaagaa 1140
gatcagcaat tcatgctaaa aaagtgtcct gccctacttc aagaaatggt taatgtaatc 1200
tgccaactaa tagtaatggc ccggaaccgt gaagaaaggg agtttcgtgc tccaactttg 1260
gcatccctag aaaactgcat gaagctttct cagatggccg ttcagggact tcagcaattt 1320
aagtctcccc ttctgcagct ccctcatatt gaagaggaca atcttagacg ggtttctaat 1380
cataagaagt ataaaattaa aactatccag gatttggtga gtttaaaaga atcagatcgt 1440
cacactctac tgcacttcct tgaagatgaa aaatatgaag aggttatggc tgtccttggg 1500
agttttccat atgtgaccat ggatataaaa tcacaggtgt tagatgatga agatagcaac 1560
aacatcacag taggatcctt agttacagtg ttggttaagt tgacaaggca aacaatggct 1620
gaagtatttg aaaaggagca gtccatctgt gctgcagagg aacagccagc agaagatggg 1680
cagggtgaaa ctaacaagaa caggacaaaa ggaggatggc aacagaagag taaaggaccc 1740
aagaaaactg ctaaatcaaa aaaaaagaaa cctttaaaaa aaaaacctac acctgtgcta 1800
ttaccacagt caaagcaaca gaaacaaaag caggcaaatg gagtcgttgg gaatgaagct 1860
gcagtaaagg aagatgaaga agaagtttca gataagggca gtgattctga agaagaagaa 1920
accaatagag attcccaaag tgagaaagat gatggtagtg acagagactc tgatagagag 1980
caagatgaaa aacaaaacaa agatgatgaa gcagagtggc aagaattaca acaaagcata 2040

```
cagcgaaaag agagagctct attggaaacc aaatcaaaaa taacacatcc tgtgtatagc 2100
ctttactttc ctgaggaaaa acaagaatgg tggtggcttt acattgcaga taggaaggag 2160
cagacattaa tatccatgcc atatcatgtg tgtacgctga aagatacaga ggaggtagag 2220
ctgaagtttc ctgcaccagg caagcctgga aattatcagt atactgtgtt tctgagatca 2280
gactcctata tgggtttgga tcagattaaa ccattgaagt tggaagttca tgaggctaag 2340
cctgtgccag aaaatcaccc acagtgggat acagcaatag aggggatga agaccaggag 2400
gacagtgagg gctttgaaga tagctttgag gaagaagagg aggaagaaga agatgatgac 2460
taagcagtac tctgaatgga ccacagtgtt tgcacatatt tgcaattttt tgctgttttg 2520
gaagtgtatc ataaaccaga aacagtacag aactgatgtt gagggaggtg tagtttttttt 2580
actcttgaaa tgggtgcata atataactag gcagtggcgg tgccttggta caacctgaaa 2640
aatgttaagg cttattgaaa cctttcaagt aggggatggt acatttattt catctgcaaa 2700
tgataataaa tcctttgtta ttataactgt ccagaagtgt gggctatgta ttatctgatc 2760
agtctatggt cccagtaaaa gtaaagatgc aggaaacaca gtctgtaaat gagcgacttt 2820
tctttgttca gctttagttt tagcaaacac cacaaatatg ttttaagtaa catcgctcaa 2880
gtttaagtaa catcgctcaa gttgataatc tcttgataag ctctgttgtt gacattttgc 2940
agtgatacaa cagctccact catagattta aacttttatt tttacttatc ttggtcataa 3000
gttggcattc tctcacattc cacatgatat agagggctac gttttggaat tttccttttc 3060
ttaattgccc agagttatca gacagattat aaaaatggct tttaatggct taaaccattt 3120
ctaaacctct atcttagcag atcaatgcag gatctaattc ttttgataag ttctagctct 3180
aaaagtgata gtgggactgt atgttttctg atactggtgg cttatgttat taaacctttt 3240
ttaaaaaagg ttcactctaa aagctgaact acatccttag ttttcagtct acttgactct 3300
atcaggagct ttttaaggaa agtaagtata acatgcaaag gaagcttttt ttgtattcat 3360
tttggactcc tgtcaataaa aatagaagtt tgttgactcg ttttatgttt caatggtgtg 3420
tgtcttttta ctatcaggac ataaataggg caatccactt ctttattttt caactaaaga 3480
ttgaatagta ttgtacatta ctgctaaagt gactgctatt tctgtatact gtagaaaaac 3540
ccaggagtga gagggatttc ccctcatagt acaactggaa ggatagtgct tgtaaagagt 3600
agagatgtgt acatgatgaa tcattgaggg agggtggata tttttattcc tagatatgga 3660
ggaaacataa gtctgtagta ttataaaact gattgtaata attctttcct atcaaaatct 3720
ccataggtca aaatattgtt ggaatactaa aatttgcaac cttgtttact ttaaaaggtt 3780
gccactttca gtgcagaata ctaccggcat cttgttactg caatagttgg aaataaaatg 3840
tgaaaattaa                                                        3850
```

<210> 240
<211> 2673
<212> DNA
<213> Homo sapiens

<400> 240

```
attgtgggat tggatgagtc tcaagatgga caaccgggat gttgcaggaa aggctaaccg 60
gtggtttggg gttgctcccc ctaaatctgg aaaaatgaac atgaacatcc ttcaccagga 120
agagctcatc gctcagaaga aacgggaaat tgaagccaaa atggaacaga aagccaagca 180
gaatcaggtg gccagccctc agcccccaca tcctggcgaa cgccccgacc agtgcgccca 240
gcgcccctcc cagcacaccc acccccagcg ctgggaagag gtccctgctc atcagcaggc 300
ggacaggcct ggggctggcc agcctgccgg gccctgtgaa gagctactcc cacgccaagc 360
agctgcccgt ggcgcaccgc ccgagtgtct tccagtcccc tgacgaggac gaggaggagg 420
actatgagca gtggctggag atcaaagtgt gtctcaccaa gacagtgaga ccgactcccc 480
cctcgtcatg gtgttttgtg cctggactcg ggacgcccga cagcctcctg catctcacac 540
tcttctcccc ccacccccctc tgtcgttgtg gtccatagag agagtgtgcc tattgactgt 600
ggggtgtgtg agttgaaccc cagtactgac agcctcctta aagtttcacc cccagaggga 660
gccgagactc ggaaagtgat agagaaattg gcccgctttg tggcagaagg aggccccgag 720
ttagaaaaag tagctatgga ggactacaag gataacccag catttgcatt tttgcacgat 780
aagaatagca gggaattcct ctactacagg aagaaggtgg ctgagataag aaaggaagca 840
cagaagtcgc aggcagcctc tcagaaagtt tcacccccag aggacgaaga ggtcaagaac 900
cttgcagaaa agttggccag gttcatagcg gacgggggtc ccgaggtgga aaccattgcc 960
ctccagaaca accgtgagaa ccaggcattc agctttctgt atgagcccaa tagccaaggg 1020
tacaagtact accgacagaa gctggaggag ttccggaaag ccaaggccag ctccacaggc 1080
agcttcacag cacctgatcc cggcctgaag cgcaagtccc ctcctgaggc cctgtcaggg 1140
tccttacccc cagccaccac ctgccccgcc tcgtccacgc ctgcgcccac tatcatccct 1200
gctccagctg cccccgggaa gccagcctcc gcagccaccg tgaagaggaa gcggaagagc 1260
cggtgggggc ctgaagagga taaggtagag ctcccacctg ctgaactggt gcagagggac 1320
gtggatgcct ctccctcgcc tctgtcagtt caggacctca aggggctcgg ctatgagaag 1380
gggaagcctg tgggtctagt gggcgtcaca gagctttcag acgcccagaa gaagcagctg 1440
aaggagcagc aggagatgca gcagatgtac gacatgatca tgcagcacaa gcgggccatg 1500
caggacatgc agctgctgtg ggagaaggca gtccaacagc accagcacgg ctatgacagt 1560
gatgaggagg tggacagcga gctgggcacc tgggagcacc agctgcggcg catggagatg 1620

gataagacca gggaatgggc cgagcagctg acaaagatgg gccgggggcaa gcacttcatc 1680
ggagacttcc tgcctccaga cgagctggaa aagtttatgg agaccttcaa ggccctgaag 1740
gagggccgtg agcctgacta ctcagagtac aaggagttca agctgactgt ggagaacatc 1800
ggctaccaga tgctgatgaa gatgggctgg aaggagggcg aggggctggg ctcagagggc 1860
cagggcatca agaacccagt gaacaagggc accaccacag tggacggcgc tggcttcggc 1920
attgaccggc cggcggagct ctccaaggag gacgacgagt atgaggcgtt ccgcaagagg 1980
atgatgctgg cctaccgctt ccggcccaac cccctgaaca atcccagacg gccttactac 2040
tgagtgttct ggaaatacat actttctgaa tgaccaaccg tccctcgact gtggaatgtt 2100
ccggcctgca tttctgccca ccccttccgt tgtcacgagt gccgtgccgt gtaataaagt 2160
cccagtgctc atccaccaca gatgcctggt cctgttgcag gaagcccctc ctgttagcag 2220
cgggatgcaa gatgctgtct cggggctctc cttattggcc tgtggagtct ccatccccgc 2280
tcagcctggg ttgcactgga ggggaggaag aaagttgttt tcagcatagt aaagctggaa 2340
ggccaggtgc agtgtctcac ccctgtcatc ccagcacttt gagaggccaa ggcgggcgga 2400
tcactggagg tcaggagttt aagaccagcc tggccaacat ggtgaaatcc tgtctctact 2460
aaaaatataa aattagctgg gcatggtggt gcacacctgt cgtctcagct actcgggagg 2520
cagaggcagg agaatggctt gaacctggga agcggttgca gtgagccaag attgcgcctt 2580
tgcactccag cctgggcaag agagcaagac tctgtctcaa ataataataa taataataaa 2640
gctggagact tggccacacc tgggctaaag gaa                              2673
```

<210> 241
<211> 2540
<212> DNA
<213> Homo sapiens

<400> 241

cgccgcctcc ctcccgcaca gcagccgcca gcgcggcctc ctgcaccatg tcggtggccg 60
gcctcaagaa gcagttccat aaagccactc agaaagtgag tgagaaggtt ggaggagctg 120
aaggaaccaa gctagatgat gacttcaaag agatggaaag gaaagtggat gtcaccagca 180
gggctgtgat ggaaataatg actaaaacaa ttgaatacct tcaacccaat ccagcttcca 240
gagctaagct cagcatgatc aacaccatgt caaaaatccg tggccaggag aaggggccag 300
gctatcctca ggcagaggcg ctgctggcag aggccatgct caaatttgga agagagcttg 360
gagatgattg caactttggc ccagcacttg gtgaggtcgg ggaggccatg cgggaactgt 420
cggaggtcaa agactctttg gacatagaag tgaagcagaa cttcattgac cctcttcaga 480
atcttcatga caaagatctt agggaaattc aacatcatct aaagaagttg gagggtcgac 540
gcctggattt tgattataag aagaaacgac aaggcaagat tccggatgaa gagcttcgtc 600
aagctctaga gaaatttgat gagtctaagg aaattgctga gtcaagcatg ttcaatctct 660
tggagatgga tattgaacaa gtgagccagc tctctgcact tgtgcaagct cagctggagt 720
accacaagca ggcagtccag atcctgcagc aagtcacggt cagactggaa gaaagaataa 780
gacaggcttc atctcagcct agaagggaat atcaacctaa accacgaatg agcctggagt 840
ttccaactgg agacagtact cagcccaatg gggtctctcc ccacacaggc actcccaaac 900
cttcaggtgt ccaaatggat cagccctgct gccgagctct gtacgacttt gaacctgaaa 960
atgaagggga gttgggattt aaagagggcg atatcatcac actcactaac caaattgatg 1020
agaactggta tgaggggatg ctgcatggcc attcaggctt cttccccatc aattatgtgg 1080
aaattctggt tgccctgccc cattaggatg ttatgctggc tggctcgcct cctcttgacc 1140
cagatagtta cggttaacca ctgctttggc aatgctgctt ataacacatc ccaagtgcag 1200
gccgcagtgg tccacgtcat ccagccccac caagtgactt tggttgactt gtgggctccc 1260
acaggagtca tggtgatgga tgatatcctc ttagcctggt gggcgtggca tgtgcttttt 1320
aaaacatcat ctgagaccag ccagtagtca cagaactgct gtttacacag ttctcaggag 1380
gctgtggttt attagaatat gaccatgagc catttcacag aaaaaccatc ccaccgaaga 1440
tattgtctat caccccaggg gccatctgaa ggtctctttg catttctcca tgcaaagagg 1500
agaaagcttt tgctttcaca ctgtcccttc ccaaatatgt gagtcatgga attgtcaaag 1560
taagccttcc ctcaccagca aattgtctcc tgatctgaat gaatttgtct cttaatgcat 1620
ccatagaaaa gtgttaattg tgggttcaaa gcattctctg caaataggca tctcagctcc 1680
tcacacttat ggctatttct gacgtatagc cagttttctt ccctccttgc tattaaagcc 1740
agagtcgtaa ttccaaatta tttttcagta agacagttaa tcagcattat tgtgagaggg 1800
actgaaaaga aattctccat tatgaggaat tgggaagaaa tctggtatcc aagcttaaat 1860
ttcttgctat acagaactat gtatgtattt aggctattct gaagggcaca gggaagggga 1920
acaaatatct tcacttcagt tttatttgtg aattacatgt ttcatgaatc catttggcac 1980
agagacacaa ggaagaaaac actagtaacc aactttccac tagttcatat actgagaaac 2040
agtaaatacc tttcctttcc acttttaccc tgtgttcttt gaacatcatt tgtgcagatt 2100
ctgccctcaa tgaggaccaa ataaagatga tttttgtgct tagcagttta aggtatatgg 2160
ctggcatatg caaaactctt tcccaattca gtcgctactt ttacttctgc cctttctatc 2220
catcgtcttc attttgtgtg tacagtgctg tgtgtaagct tatcagtgtg ttttttttatt 2280
tgtatcagtc atgaaagtcc tgttaggtat ccagagttct atttatctag ctgtacagac 2340
tctttcagag gtttaacgtg ctgcttccga tgtgccacct gcagtagtgg atcatgtgga 2400

gtgaaaggca aatcttactg cttaatgtat aaactctcac cacaggaagc atcgctgttt 2460
ccaataaata ttgctgaaga cagaaaaaaa aaaaaaaaaa aaaaaaaaaa aaaaaaaaaa 2520
aaaaaaaaaa aaaaaaaaaa 2540

<210> 242
<211> 1942
<212> DNA
<213> Homo sapiens

<400> 242

```
gcaacttgaa ttggtcccag ctgctcccag aagaacgggc gggttggtcc ctatgccacc 60
cctggagagc tactcgccgc ccactttgcc gtgaagggct gtgcggttcc cgtgcgcgcc 120
ggagcctgct gtggcctctt atgcactcca ccacccccat cagctccctc ttctccttca 180
ccagccccgc agtgaagaga ctgctaggct ggaagcaagg agatgaagag gaaaagtggg 240
cagagaaggc agtggactct ctagtgaaga agttaaagaa gaagaaggga gccatggacg 300
agctggagag ggctctcagc tgcccggggc agcccagcaa atgcgtcacg attccccgct 360
ccctggacgg gcggctgcag gtgtcccacc gcaagggcct gccccatgtg atttactgtc 420
gcgtgtggcg ctggccggat ctgcagtccc accacgagct gaagccgctg gagtgctgtg 480
agttcccatt tggctccaag cagaaagaag tgtgcattaa cccttaccac taccgccggg 540
tggagactcc agtactgcct cctgtgctcg tgccaagaca cagtgaatat aacccccagc 600
tcagcctcct ggccaagttc cgcagcgcct ccctgcacag tgagccactc atgccacaca 660
acgccaccta tcctgactct ttccagcagc ctccgtgctc tgcactccct ccctcaccca 720
gccacgcgtt ctcccagtcc ccgtgcacgg ccagctaccc tcactcccca ggaagtcctt 780
ctgagccaga gagtccctat caacactcag actttcgacc agtttgttac gaggagcccc 840
agcactggtg ctcggtcgcc tactatgaac tgaacaaccg agttggggag acattccagg 900
cttcctcccg aagtgtgctc atagatgggt tcaccgaccc ttcaaataac aggaacagat 960
tctgtcttgg acttctttct aatgtaaaca gaaactcaac gatagaaaat accaggagac 1020
atataggaaa gggtgtgcac ttgtactacg tcgggggaga ggtgtatgcc gagtgcgtga 1080
gtgacagcag catctttgtg cagagccgga actgcaacta tcaacacggc ttccacccag 1140
ctaccgtctg caagatcccc agcggctgca gcctcaaggt cttcaacaac cagctcttcg 1200
ctcagctcct ggcccagtca gttcaccacg gctttgaagt cgtgtatgaa ctgaccaaga 1260
tgtgtactat ccggatgagt tttgttaagg gttggggtgc tgagtatcat cgccaggatg 1320
tcaccagcac cccctgctgg attgagattc atcttcatgg gccactgcag tggctggaca 1380
aagttctgac tcagatgggc tctccacata accccatttc ttcagtgtct taacagtcat 1440
gtcttaagct gcatttccat aggatagagg ctattgcagg gagtggcttg tatcatttca 1500
gatttgcaac tgaagtttct aaaaacatgt gtaaatacat agaatgtata ctgttcttat 1560
ttttttaat caccgtttgt tttgtgcttt ctagttaacc tgatgccagt acagtgcaat 1620
tggaaaagca ggactttggt gcctgtgcta taagcagcag attttgtggg aggaaacact 1680
tgagaggcga tattgtcaac agtatttgaa gggtgttagc agaataaaag acagctttag 1740
tcagccgtgt cattataaag catgttgtgt ggcctcacag aaacattgaa actgtttata 1800
cagcaaaagt caggtattag cagcactaaa gcaaatatca ctcagatgaa acaaagcagt 1860
gaaaccctac agtttaaatg atgtcacttt tagtgctgtt ggcaagaaaa aaaaaaaaaa 1920
aaaccaaaaa aaaaaaaaaa aa                                          1942
```

<210> 243
<211> 3394
<212> DNA
<213> Homo sapiens

<400> 243

```
ccgcgcgtgt ggcagtcgcg gaaggcgcgg gagcttgcgt gctgctgggc ctgagctgtc 60
tgtctcgttt ctgtccgcgc gccctgcatc ccggccccgg gcgcccgctg gaggtcgccg 120
aggagccaca gggctgactg gtctgctgcc cgggcccagg agtgcctggt gtagcagtcg 180
cggagccatc ccggcgtctg ctgccatgac cgactctccc ctcagaggag actcttcctc 240
agcggtggct gcagagacag atgagcggcg gctcctggcc gcgggaccgt gagacgggtt 300
cgtggccggc catttagggg gacgctgcga ccaccgcctg cgcccctccg gactggttcc 360
ttgggccccg gaagctcgcg gcgggccctg cgggaggcgg catgctcccg cggaggctgc 420
tggccgcctg gctggcgggg acgcggggcg ggggcctgct ggcgcttctg gccaatcagt 480
gccgcttcgt cacgggcctg cgcgtgcggc gcgcgcagca gatcgcgcag ctctacggcc 540
gcctctactc cgagagctca cgccgcgttc tcctcggccg cctctggcgc cggctgcacg 600
gccgtcctgg ccatgcctct gccttgatgg cggcgttagc cggcgtcttc gtttgggacg 660
aggagaggat ccaggaggag gagttgcaga gatctattaa tgagatgaag cggttggaag 720
aaatgtcaaa tatgtttcag agctctggag tccagcacca ccctccagaa ccaaaagccc 780
aaacagaagg gaatgaagat tcagagggca aagagcaacg ttgggaaatg gtgatggata 840
agaaacactt taagctgtgg cggcgcccaa ttacaggcac ccacctttac cagtaccgag 900
```

```
tttttggaac ctacacagat gtgacacctc ggcagttctt caatgttcag ctggacacag 960
agtatagaaa aaaatgggat gccctggtaa tcaagctgga ggtgattgag agggatgtgg 1020
ttagtggttc cgaggttctt cactgggtaa cccattttcc ttatccaatg tactcacggg 1080
attatgttta tgttcggcgg tatagtgtgg atcaggaaaa caacatgatg gtgttggtgt 1140
cgcgtgctgt ggagcatccg agtgtgccag agtctccaga attcgtcagg gtcagatcat 1200
atgaatccca aatggttatc cgtccccaca agtcatttga tgagaatggc tttgactact 1260
tactaacata cagtgacaat ccccaaacgg tgtttcctcg ctactgtgtt agttggatgg 1320
tttccagtgg catgccagat ttcctggaga agctgcacat ggccactctg aaagccaaga 1380
atatggagat taaagtaaag gactacatct cagctaagcc tctggaaatg agtagtgaag 1440
ccaaggccac cagccagtcc tctgagcgaa agaacgaggg cagctgtggc cctgctcgga 1500
ttgagtatgc ttgacaggct ttgggataag aagggacaag gtgcttctag ccctgtctca 1560
gtccgttatc actctgctgt agaaggggga catgccacat gtattagaag gcatctgctg 1620
taacttccag tgcaagataa ttcaataact gatgtcccat ttcattcaga gcccttattg 1680
ctcttatcaa aacagaagaa ggctacattt gtgggagtgt tgtcatattc tcaggccaac 1740
tgttttgaaa ttcggtatct cactgagcta atctggaaca aacctctcac ctcaggccag 1800
aaggggatga cctccatttg cttctctgag tagtttcctc tgctgacatt ccaaatccca 1860
ccatcgattg tgcagcgctt tggatttcct tcagttctcc aggtccacct ggaaagtata 1920
gttggccagt tgagtctctc aaatgagggg ctactgggag tgctcttggt aacaatcatg 1980
atgtgaatgg gtgtgaacga tacttggcta tgttaagtgc cttgtccgca ccttgctttt 2040
atctctagag acatgaagtt attattaatt tttttttttt ttaagtagag atggagtttc 2100
actctgtttc ccaggctggt cttgaactcc tgggccatgc ctggccaggg acatgaattt 2160
gtacaaagaa atttccctcc ctgcctgcac aatatcaccc attgactcac cttatccaaa 2220
gcaagtttcc tgtgaatcgg ccagttcttc tatattcatt ggatcattgc ctccttccta 2280
accttcccca tttaccaaga acactgggag actaatcctt ttagatagta gctttttgat 2340
gctcaaaaca tcacatttaa atttagttta aaaatttttt aacttttgtg tcaaatagga 2400
gttgaggaat tgagcaggat tctaccctag tccgattgta tagaaaacac cattttgatt 2460
caggtattat ttttcatatt tcaggtttga cttgttcttt tcagaaggct aaagtcagag 2520
gaatgggggc tgggccactc ccttggagct ctcagatcta cagacaagct gtgtgaatgc 2580
atagatgtaa tcttgtctca aatactaata cagtggagat ttggtttatg ttaccattaa 2640
gttcctctaa aaagtttttc ttcctctctt cagagccaaa ataaaagtga actacactgt 2700
tcagataagg tcacaatctg atgctgtcag tttgaccgag ctggttttgc ttatggtcat 2760
gctgcaattt gttagaataa tagggatcaa gttttaaatc ctcctccttc cctttttttct 2820
ggagtcttga gggccagagt ttttgttttt gtttttgttt ttgttttcct gcttgctact 2880
gttttgtggt gttgaaaagt ggtttaaacc tgagactaac ttaaacactt ccttgacctt 2940
cttgttgcct gttcattttt gtgccaagga agtagctgcc ccagtgtatg tcttgccttc 3000
tccgcgtcat tgttggaaga ggagagatgc atcgagcagt cccagctgct tttcatttat 3060
tacttcttct ttccaggacc tgacagaagt cagggaagag tccctgggtt atgtccaaac 3120
ttagcacctg caattgttgg gatgtggatg gatgtgtgca taagagagag agagaatatg 3180
tgtgtgtgtg tgtgcgtctg cgagcgcaca cacatgcaca agtgcgaagg agttgcggtt 3240
gctccatgtt ctgacttagg gcaatttgat tctgcacttg gggtctgtct gtacagttac 3300
tcatgtcatt gtaatgattt cactcctaac tgtgacattt ttatcaaatg tgtgaataaa 3360
tacataaaga ttggtacaaa aaaaaaaaaa aaaa                              3394
```

<210> 244
<211> 4344
<212> DNA
<213> Homo sapiens

<400> 244

```
gctgaggaga aagcggcgcg cggagggtgt gagatggcag acaggtttgc aggaaaccct 60
cagaaagggg gctggaggat ttagccactc tgtcctcccc ttccggcagt ccagggcctc 120
ctccccgagca cagcggcgct atggactctc caggatacaa ctgcttcgtg gacaaagaca 180
agatggacgc tgccatccag gacctggggc ccaaggagct gagctgcact gaactgcagg 240
aactgaagca gctggcgcgc cagggctact gggcccaaag ccacgccctg cggggaaagg 300
tgtaccagcg cctgatccgg gacattccct gccgcacggt cacgcctgac gccagcgtgt 360
acagcgacat cgtgggcaag atcgtgggca agcacagcag cagctgcctg ccgctgcccg 420
agttcgtgga caacacgcag gtgcccagct actgcctgaa tgcacgcggc gaggggccg 480
tgcgcaagat cctcctgtgc ctggccaacc agttccccga catctccttc tgccccgccc 540
tgccggccgt ggtggccctg ctgctgcact acagcatcga cgaggccgag tgcttcgaga 600
aggcctgccg catcctggcc tgcaatgacc ccggcaggag gctgatcgac cagagcttcc 660
tggcctttga gtcgtcctgc atgacgtttg gggacctggt gaacaagtac tgccaggcgg 720
cccacaagct gatggtggcc gtgtcggagg atgtcctgca ggtctatgcg gactggcagc 780
gctggctgtt tggggagctg cccctctgct acttcgcccg ggtctttgac gtcttcctgg 840
tggagggcta caaggtgctg taccgcgtgg cgctggccat cctcaagttc ttccacaagg 900
tgagggccgg gcagccgctg gagtcggaca gcgtgaagca ggacatccgc acgttcgtca 960
```

gagacatcgc gaagacggtg tcccctgaga agctgctgga gaaagcgttc gccatccgcc 1020
tcttctcccg caaggagatc cagctcctgc agatggccaa tgagaaagcc ctgaagcaga 1080
agggcatcac cgtgaagcag aagaggcagt ttgtacactt ggccgtccat gcagagaact 1140
tccgctcgga gatcgtcagc gtgagggaga tgagagacat ctggtcctgg gtccccgagc 1200
gctttgccct gtgccagccc cttctgctgt tctcctccct gcagcacggg tacagcctgg 1260
ccaggttcta cttccagtgt gaaggacatg agcctaccct cttgctcatc aagaccacgc 1320
agaaggaggt gtgtggtgct tacctgtcca cagactggag tgagagaaat aagtttggag 1380
gcaaactggg cttctttggg accggagaat gctttgtgtt taggctgcag cctgaggtgc 1440
agcgctacga gtgggtggtg atcaagcacc ccgagctgac caagcccccca cccttgatgg 1500
ctgccgagcc caccgcccca ctcagccact ccgcctcctc agaccccgct gaccgcctct 1560
cgcccttcct ggccgctcgc cacttcaacc tgccctccaa gaccgagtcc atgttcatgg 1620
cgggggggcag cgactgcctc atcgtcgggg gaggaggcgg ccaggcgctc tacatcgatg 1680
gggacctgaa ccggggccgc acaagccact gcgacacctt caacaaccag cccctctgct 1740
ccgagaactt cctcattgct gccgtggagg cctggggctt ccaggaccct gacacccagt 1800
gacggcctgt gccacggtga ctgagccgtg gtggggcggt gggccgaggc tgggctgccg 1860
cctcgggcag cagagagcag atgaaacccc catgtggtag gcagggttgg gggacggcag 1920
gaccccatgg ccaagcctgg cgttgcctgg acctgctgct gcctctacct ggggtttggg 1980
ctgggcttcc ccagtccacc tgcatctggg tcagagctgg agggcctgct gtgccccccag 2040
ccccacccag agctggcata ggaagtacct tcctcctccg tggaggcttc catagcccaa 2100
ggccttggga gtgtctgggt cttgctgccc ctgagcctct ctaggcagcc tgagcccctg 2160
gggtgggagt acaacggcag tgggaacgtg cagctaaggg tgggcctgtg gtgcccccctg 2220
ctggtgccgc atagcatcgc gccctcctga gcagggccct gcagcctggc tggctggcga 2280
ctgaatccca gcgtagatcc cgagaacaga ctggcaccct tgggcctgcc ccctggcgga 2340
ccccaccccc tgtcctgggc gtttgcccca gttccctccc caggcctcct gggccacctc 2400
tcacattaac ccttcttggc tgcctctgag gctcccctgg gctgcgtttg ggagctgctg 2460
ggtgggagga ggagggtggg atttgtgctg ggctccaccc ccagggaggg tgggagctgg 2520
agggggatgt cacatgtcct tgtgtttctg cagagcctga agcttttgct gggcctccct 2580
gcgtgttccc atctgaagag gtagccccgg gaaaggccct cagggaccga cccagaccag 2640
caagctccat ttccaggggt ctgcccagag aagcctggac ccaggggggct tctcagctcc 2700
cctcccttcc agcccaggcc tccttgagct cctgtagcct ctgcagctgc ccatttcacc 2760
tctttgtcat tgcaatgacc tggggcccag ggtcacgccc aagcccctct ttgtggcatg 2820
ctcatctcag tgcagcccca actgcttctc tgcaccaggc cccgtgccct cccctggccc 2880
tggccacaca gcacctcctt ggccaccttg catggatgca tgtcctccct ctgtccctct 2940
tgtggtgctg tggctggcca cgtgtcaggg ttttctgtga gcctctcacc ttacagcctt 3000
tgggcccctg ggcccctgcc ctgcctgagc cctgcgctgg ccagacgcag gtaggtgcca 3060
ggggaggcct tgcctctggg cactataggg ggcagccccc tgccttgtga cctccctcca 3120
tgccccatga cctccttcaa ctcgtcttga gggtctggtc accacggagg ggagaaggga 3180
gacacagagg cccaagtggc tgttctggga ccatggaagc ctgggtcaca gaggcaggta 3240
ccagcagtcc tgcccccacc cccgtccctg ctggcctcca gccagtgcca cttggagtcc 3300
cctgcaccgt gccactttcc atccccacac cattgcctgc tcctcccatg ggctttagc 3360
ctcccctgac catctgctca tgtagcctct gactgggcgc acagtggtgc aggaggaagg 3420
accgggaacc ctgtgtggct ttgggcaagc tgacaaaccc gtctggaact cagtttcccc 3480
agctgtgaaa tggggccagt ccccatgccc tgctgtcctc ctcacaggac tgttgagagg 3540
ctcgcagggg gatggggtga atccctctca cggggagagg tatgggaggg aagtcgccac 3600
tcatctcagc ccaccgcagc cctccaggcc cccgcctgag cccctgaggc cctcatctgt 3660
accgccagct ttggtgttca ttaaacatgg tcttgctgca tcaactccag ggctccaagc 3720
tgagtctgat ctcttcagcc agaggggcct ccgggggctt cctgggccta cagccaagca 3780
ggtgtgggag gctgcccgct gtagtgctca cccacattct atgactggaa tgctgcaagc 3840
ctcttccaga gggagccctc caggagcagg gaagtcatat ccgtggacca tttgtaggtt 3900
tccctctgca aacttcctga actgggtggt gatttttctg gcgagttaag tccagcaggt 3960
tctgcgtggc tgcccccagg gggccccacc cggccttcc agtgatctgg accaggaaga 4020
cggattgctg ctgggtcatg atttccacat catgagcgaa gttctttgtt ttttgtgtgc 4080
atgcaaaata ttgtttcttt aaatttaata tcttttccta acttttctac taacctagaa 4140
ggtatgtgtg attattatct tttttattct tatcactgta cttgttagta tctgatatta 4200
ggtattccca aatagaacat aatttatttt gttaatgttc tctatttctt cttgattatt 4260
tattagcttt ggatgttaaa aattagccaa aagtggctgc tctgcctatg aagtagccat 4320
tctttgttta aaaaaaaaaa aaaa 4344

<210> 245
<211> 2299
<212> DNA

<213> Homo sapiens

<400> 245

agcgggcggc tgctaggagg caccgaggca gcggcggggc tctgggcgcg cggctggatg 60

```
cccccggcct gcggctccct gcgcttcccg ccgtccaggg gcaccagtca tgggcgccgc 120
agccgctgag gcgccgctcc ggctgcctgc cgcgcctccg ctcgccttct gctgctacac 180
gtcggtgctt ctgctcttcg ccttctctct gccagggagc cgcgcgtcca accagccccc 240
gggtggtggc ggcggcagcg gcggggactg tcccggcggc aaaggcaaga gcatcaactg 300
ctcagaatta aatgtgaggg agtctgacgt aagagtttgt gatgagtcat catgtaaata 360
tggaggagtc tgtaaagaag atggagatgg tttgaaatgt gcatgccaat ttcagtgcca 420
tacaaattat attcctgtct gtggatcaaa tggggacact tatcaaaatg aatgctttct 480
cagaagggct gcttgtaagc accagaaaga gataacagta atagcaagag gaccatgcta 540
ctctgataat ggatctggat ctggagaagg agaagaggaa gggtcagggg cagaagttca 600
cagaaaacac tccaagtgtg gaccctgcaa atataaagct gagtgtgatg aagatgcaga 660
aaatgttggg tgtgtatgta atatagattg cagtggatac agttttaatc ctgtgtgtgc 720
ttctgatggg agttcctata acaatccctg ttttgttcga gaagcatctt gtataaagca 780
agaacaaatt gatataaggc atcttggtca ttgcacagat acagatgaca ctagtttgtt 840
gggaaagaaa gatgatggac tacaatatcg accagatgtg aaagatgcta gtgatcaaag 900
agaagatgtt tatattggaa accacatgcc ttgccctgaa aacctcaatg gttactgcat 960
ccatggaaaa tgtgaattca tctattctac tcagaaggct tcttgtagat gtgaatctgg 1020
ctacactgga cagcactgtg aaaagacaga ctttagtatt ctctatgtag tgccaagtag 1080
gcaaaagctc actcatgttc ttattgcagc aattattgga gctgtacaga ttgccatcat 1140
agtagcaatt gtaatgtgca taacaagaaa atgccccaaa aacaatagag gacgtcgaca 1200
gaagcaaaac ctaggtcatt ttacttcaga tacgtcatcc agaatggttt aaactgatga 1260
cttttatatg tacactgacc atgtgatgta catttattat gtcttttttt aaagaatgga 1320
aatatttatt tcagaggcct tatttttgga catttttagt gtagtactgt tggctcgtat 1380
ttagaatatt cagctacgac agttttggac tgtttagtag tctttgtttt atgtttttaa 1440
atacagaaat tgctttcaca aatttgtacc acatggtaat tctaagactt gttctttacc 1500
catggaatgt aatatttttg caaagatgga ctacttcaca aatggttata aagtcatatc 1560
cacttcttcc acaatgacca cagcaaatga ccaagcatga actaaaggta aagatgttta 1620
cagattactt ttcttacaaa aaaatctaga agacactgtg tttaaataga tatttaaatg 1680
tttttgagat ttagtaactg atttttttaga cactgcctat cgcatgaact gtaaagctgt 1740
gtgtattagg tgtaaaatat ttataagata tatggactgg ggaatttgat tattcctccc 1800
tttgaaaaaa tagtcctaat aatttgaaca aatatgttag taatgatgga acagatcaat 1860
gaaaagtaga tatagatatt gtgaaaatag gctgtttaac aaacagattg gaataaagcc 1920
tattctacca gttaaactac tttaatacac attcattttt aaagaaaatg tttgttttaa 1980
cataaataaa caaatcgtat cagtgtttgt gaataaaata caaaaatgat tgttaatgat 2040
tggtgctctt aaagtgagct taaaatttat ccaagacgta tatccaaatt tgtcctgtag 2100
taatagatta atattcatag attgttggtg tttaaagatc tgaagtgtga gtagaatgta 2160
ttcagctgtt taacatgtag tttagatatt caaaagtatg catgtagaat ttaaagaata 2220
tgttaaaaat tattaatctt aatattttgt ttggaaaagc atgttataat ataatgtttt 2280
cacaaaaaaa aaaaaaaaa                                              2299
```

<210> 246
<211> 5100
<212> DNA
<213> Homo sapiens

<400> 246

gttagctgag caactgggcc gaagagaaga tgaggcaaaa attcgccatg gccttggcct 60
ctccctttgg gctagtggaa acttggagga ggcccaacac cagctttata gggcatcagc 120
cttgtttgaa acaatccgac atgaggcaca gctgagcacg gactacaaac tctccctctt 180
tgacctgcag acatcatcct accaggcctt gcagcgggtg ctcgtcagcc taggccatca 240
tgatgaagcc ctggctgtgg cagaaagggg acggacaagg gcatttgctg atcttctggt 300
ggaacgacaa acaggacaac aagactccga cccctactcc ccagtcacta ttgatcagat 360
cttagagatg gtaaatggcc agaggggact agtgctttac tattccctgg ctgcaggcta 420
tctgtatagc tggctgctgg ctcctggggc aggaattgtg aagtttcatg aacactacct 480
gggtgagaac acagtggaaa actcaagtga cttccaggcc agcagcagtg taacccttcc 540
aacagcaacc ggctcagccc tggagcagca cattgccagt gtccgggagg ccctgggggt 600
ggagtctcac tactcaaggg cctgtgccag cagtgagaca gagagtgaag cgggagacat 660
catggaccag caatttgaag agatgaacaa caaactcaac tcggtcactg accccactgg 720
ctttctgcgg atggttcgcc gcaataacct gtttaacagg agctgccaga gcatgacgag 780
cctgttcagt aacactgtgt caccgaccca ggacgggacc tcctctcttc ccaggaggca 840
gagctcgttt gccaagcccc cgctccgtgc cctgtatgac ctgctcatcg cgcccatgga 900
aggggggcctg atgcactcca gcggccccgt gggccggcac cggcagctca tcctggttct 960
ggaggggggag ctctacctca ttcctttcgc cctcctgaag ggaagctcct ccaatgagta 1020
cctctacgag cgcttcggcc tccttgctgt cccttccatc cgctccctca gcgtgcagtc 1080
caagtctcac ttacggaaga acccgcccac atactccagc tccacatcca tggcggctgt 1140
catcggcaac cccaagctac catcggccgt gatggacagg tggctgtggg ggcccatgcc 1200

atcggccgag gaagaggcct acatggtgtc cgagctgctg ggctgccagc ccctagtggg 1260
cagtgtggcc accaaggaga gggtcatgag tgccctgacc caggctgaat gcgtccactt 1320
tgccacccac atctcctgga agctgtcggc cttggtcctc acgcccagca tggacggcaa 1380
ccctgccagc agcaagagct ccttcggcca cccctacacg atccctgagt ccttgcgggt 1440
gcaggacgat gccagtgatg gggagagcat ctcggactgc ccgcccctgc aggagctgct 1500
gcttactgcc gccgacgtcc tggacctgca gctgcctgtg aagctggtgg tgcttggctc 1560
ctcccaggag tccaacagca aagtcacagc cgacggggtc atcgcgctga caagggcctt 1620
cctggctgcc ggcgctcagt gtgtcctcgt gtctctgtgg cctgtgccag tggctgcttc 1680
taagatgttc atccatgcct tctactcatc cctgctgaac ggcctgaaag ccagcgccgc 1740
cctgggggag gccatgaagg tggtgcagag cagcaaggcc ttctcgcacc cctccaactg 1800
ggcagggttc atgctcatcg ggagtgacgt taagctgaac agcccctcat cactcatcgg 1860
ccaggccctc acagagatcc tgcagcaccc ggagcgtgcg cgggacgccc tgcgagtgct 1920
gctgcacctg gtggagaaat ccctgcagcg catccagaat gggcagcgca atgccatgta 1980
cacatcccag cagagtgtgg agaacaaagt gggcggcatc cctggctggc aggccctcct 2040
caccgctgtg ggcttccggc tggacccccc aaccagtggc ctgccagcgg ctgtcttctt 2100
cccaacctcc gacccgggcg accggctcca gcagtgcagc agcacactcc agtccctgct 2160
gggtctgccc aatcctgccc tccaagccct ttgcaaactc atcactgcct ccgagacggg 2220
cgagcagctc atcagccggg ctgttaaaaa tatggttgga atgctccacc aggtgctggt 2280
tcagctccag gctggcgaga aggagcagga cttggcatca gctcccattc aggtctccat 2340
cagcgtccag ctgtggcggc tcccgggatg ccacgagttc ctggcagctc taggttttga 2400
tctctgtgaa gttggtcagg aggaagtaat cctgaaaacc gggaagcaag ctaatcgacg 2460
gactgtgcac ttcgcgctcc agtccctgct gtctctgttt gattctactg agctacccaa 2520
gcgcctcagc cttgacagct cctcctccct cgagtctctt gcttctgctc agtctgtttc 2580
caacgccctg cccttgggtt accagcaacc cccttctct cccaccggtg cggacagcat 2640
cgcctcagat gccatctctg tgtacagtct gagctccatt gcctcctcaa tgagctttgt 2700
ctccaaaccc gagggtggat cagagggtgg aggccccgga ggacggcagg accatgaccg 2760
gtccaagaac gcttacctgc agagatccac cctgcctagg agccagctgc ctccccagac 2820
ccgccctgca ggcaacaaag atgaagaaga atatgaaggg ttttctatca tcagtaacga 2880
gcccttggcg acctaccaag aaaaccgaaa cacatgcttc tcaccagacc acaaacaacc 2940
ccaacctggg acagccggag gcatgagagt ctcggtgagc tccaaaggga gcatcagcac 3000
tccaaattct ccagtgaaaa tgactctgat tcccagcccc aactcaccct tccaaaaggt 3060
gggaaaacta gcaagctcag atacaggaga atcagaccag tctagcacag aaacggacag 3120
taccgtgaaa tcccaagaag aaagcaaccc aaaactggat ccacaagagt tagcccagaa 3180
aattctggag gagacacaga gtcatctcat tgcggtggag cgtcttcaga ggagcggcgg 3240
ccaggtgagc aagagtaata accctgaaga cggcgttcag gcgcccagca gcactgctgt 3300
cttcagagcg tcagaaacca gtgcgttcag caggcctgtt ctctcccatc agaagagtca 3360
gccatcacca gtcactgtta aaccaaagcc cccagccagg agctcctccc tgcccaaggt 3420
gagttccgga tatagcagcc ccaccacctc agagatgtcc atcaaagaca gcccgagcca 3480
gcacagtggc cggccatcgc ccggctgcga ctcacagact tcccagctgg accagcctct 3540
ctttaaactg aagtacccca gctctcctta cagcgctcac atttccaaat caccaaggaa 3600
catgtcccca agctccggcc accagtctcc tgctggcagt gcaccctccc cagctctctc 3660
ctactcctca gctggatctg ctcgctcaag tccagcagac gctcccgaca tagacaaact 3720
gaaaatggca gccattgatg aaaaggtgca ggctgtccat aacctgaaga tgttctggca 3780
gagcacaccc cagcattcca cagggccaat gaagatcttc cggggggctc ctggcacgat 3840
gacttccaaa agggatgtcc tcagtctgtt gaatttgtca ccacggcaca ataagaagga 3900
ggagggagtg gataagcttg aactgaagga gctgtccctg cagcagcatg acggagctcc 3960
accgaaagcc cctcccaacg gacactggcg caccgagacc acctcgctgg gctcactgcc 4020
gctgcccgcc ggccctcccg ccacagcccc cgcgcgccct ttgaggcttc cttctggaaa 4080
tggctacaag ttcctgtctc caggaagatt tttcccttct tccaaatgct aaagcatctt 4140
ttatacccac tgactctgag cagcctgcag atgggggcct ggcgtttgct tcagcccttc 4200
cctgagtgca gtccccccac agccaccagc accctcatga tgctgtgctc tgcaggcgag 4260
gggcaccacc acgtccaaag gctgccacac acactggtgg cttttctggg ccacattcac 4320
caaaagccag gacttctgtg gggctggtac aggaggctca tggaatttcc tacacacttc 4380
accaaatgtt tgtttacctt tgaactccct gcttctcatc tttgatatga ttcttcacca 4440
ggattttgta caaaaatgat catggttctg ggtggggag aggaaaggga gcacatattt 4500
tgctaagagg tatatatgca gtacctttta tacacagaaa tacaaataga gctttttcta 4560
aggctttcgg ttgctggttg ggggtgggat atatgaaatt tcagttgaat tctacaggaa 4620
agtgttatat tagccaaaaa cagaaaaatg gtttttaaaa tgccaatgat ttgtaattaa 4680
attgtagcaa ttttggtctc atgatagtgt aatatctcag caacaatgca ataattcaca 4740
ttgaaacagc gcttccattc tgaactctgc tctgcaaatg tcttttgggt cccgctgtca 4800
accaaacttg aattttctta aaaaaaaaat tatgtaactc ttatccaggc attttagaac 4860
tatgcaattg tgatttaaaa tgcaactttg tgcttttaaa acattattga cctttttgt 4920
atatggataa acaacttggt tttattatca atagtacttt gcatttatag ctattatttt 4980
taaaagctca aaaagttttt taaaatgtca aattttgaat agtcatcaat aagtaattat 5040
caagtttgga aggaaaggtt gaaatgactc cgtttcctta taagcaaaaa aaaaaaaaaa 5100

<210> 247
<211> 4862
<212> DNA
<213> Homo sapiens

<400> 247

aggacccgga agtgagggtg tgagaggcct ctctggaagt tgtcccgggt gttcgccgct 60
ggagcccggg tcgagaggac gaggtgccgc tgcctggaga atcctccgct gccgtcggct 120
cccggagccc agccctttcc taacccaacc caacctagcc cagtcccagc cgccagcgcc 180
tgtccctgtc acggacccca gcgttaccat gcatcctgcc gtcttcctat ccttacccga 240
cctcagatgc tcccttctgc tcctggtaac ttgggttttt actcctgtaa caactgaaat 300
aacaagtctt gatacagaga atatagatga aattttaaac aatgctgatg ttgctttagt 360
aaatttttat gctgactggt gtcgtttcag tcagatgttg catccaattt ttgaggaagc 420
ttccgatgtc attaaggaag aatttccaaa tgaaaatcaa gtagtgtttg ccagagttga 480
ttgtgatcag cactctgaca tagcccagag atacaggata agcaaatacc caaccctcaa 540
attgtttcgt aatgggatga tgatgaagag agaatacagg ggtcagcgat cagtgaaagc 600
attggcagat tacatcaggc aacaaaaaag tgaccccatt caagaaattc gggacttagc 660
agaaatcacc actcttgatc gcagcaaaag aaatatcatt ggatattttg agcaaaagga 720
ctcggacaac tatagagttt ttgaacgagt agcgaatatt ttgcatgatg actgtgcctt 780
tctttctgca tttggggatg tttcaaaacc ggaaagatat agtggcgaca acataatcta 840
caaaccacca gggcattctg ctccggatat ggtgtacttg ggagctatga caaatttga 900
tgtgacttac aattggattc aagataaatg tgttcctctt gtccgagaaa taacatttga 960
aaatggagag gaattgacag aagaaggact gccttttctc atactctttc acatgaaaga 1020
agatacagaa agtttagaaa tattccagaa tgaagtagct cggcaattaa taagtgaaaa 1080
aggtacaata aactttttac atgccgattg tgacaaattt agacatcctc ttctgcacat 1140
acagaaaact ccagcagatt gtcctgtaat cgctattgac agctttaggc atatgtatgt 1200
gtttggagac ttcaaagatg tattaattcc tggaaaactc aagcaattcg tatttgactt 1260
acattctgga aaactgcaca gagaattcca tcatggacct gacccaactg atacagcccc 1320
aggagagcaa gcccaagatg tagcaagcag tccacctgag agctccttcc agaaactagc 1380
acccagtgaa tataggtata ctctattgag ggatcgagat gagctttaaa aacttgaaaa 1440
acagtttgta agcctttcaa cagcagcatc aacctacgtg gtggaaatag taaacctata 1500
ttttcataat tctatgtgta tttttatttt gaataaacag aaagaaattt tgggttttta 1560
atttttttct ccccgactca aaatgcattg tcatttaata tagtagcctc ttaaaaaaaa 1620
aaaacctgct aggatttaaa aataaaaatc agaggcctat ctccacttta aatctgtcct 1680
gtaaaagttt tataaatcaa atgaaaggtg acattgccag aaacttacca ttaacttgca 1740
ctactagggt agggaggact tagggatgtt tcctgtgtcg tatgtgcttt tctttctttc 1800
atatgatcaa ttctgttggt attttcagta tctcatttct caaagctaaa gagatataca 1860
ttctggatac ttgggagggg aataaattaa agttttcaca ctgtgtactg tgttttactg 1920
attggttgga tattgcttat gaaaattcca tagtggtatt tttttggatt cttaatgtgt 1980
aacttaaaca tactttgaag tggaggagag tcataagaca gaacatttgg caggaattgt 2040
ccttatgaaa caagaaaaag aaaatgaaaa gtattattaa gcttctgtgt ttgtctaaaa 2100
atgtggcata tggatggcat ttaaaacttt gaatgaatta tacctaaatc tgggacaggg 2160
aggtgacagt ggaacaggct accaatcaga actagatgac ttttaaggct cctcctatta 2220
tgagacttca atttccaaag agaagaacta gcagagaaat tgtatttcag taattttaag 2280
ctccttctgt cttgtagagt cttgttatag ttgtataaat caaaaacaca gaataaggaa 2340
catatttaac tttttttcat tataaaatgg ttagaggacc ctacccccctc tagattccct 2400
gatttcccca ggcctgcagc atacagtaag atgggtccct gtgccaggcc tcaatactgc 2460
cagggaataa aaccagaggg agaggaccct cagtgtcata tcaggaagcc cagtgccaga 2520
ggacagacag gttcaaaact ggcttttcct ctgggcctgg gttggtgcta taggccaagg 2580
gtcattttat acttgggtat aaatcaatcc cagtttggga aaagattatt tttaagctta 2640
aaaggctgac atgtgccatt atatgtagta tgtaatatat gtaacatctt ccaattcttt 2700
taaaataaaa ttaatattta taatggatat ttaatgattg ttatttttaa aaaccagctt 2760
ataattcctc gttatgcatg atttatccaa agtttccata gttttattca aaataataaa 2820
tgttaataag gtgataaggg gtatatttaa tgtattgtat caaattgtga ataagaaagt 2880
aggatggagc tttctagagg ttgggcctta gttctgttat cctcattgct tttaaccaat 2940
aagttaaatg aagttagagt tatggtcttc aggttagatt atggaccaga tctgtgaggg 3000
tcagcatgga aattcacatt caacaaggta gcacacagga ccaagagcag cacatgcaat 3060
caactggaat aatatagtaa tcctgtaact gggtttgaaa aaataatcaa caaaagatac 3120
aattcaaggg ttaggttgca gagagctggc ttgagagtag ttattatgaa aaaggcctca 3180
aggagtacgt gttcagtatg ctctaagatg ataaagtggc tgttaaaaag ggagttgatt 3240
tgaggaagta ttacttagca ttcatgcata ttgggcttag gctctagccc tgccactatc 3300
attgtcttct ctggactgtg aagtcactga ggacaaggaa actaaattta atgtctgtat 3360
cactagtgcc tagaatttct ggacacttag tagtcaccat caggcgttta tttaatgaat 3420

```
gagaagcaaa gtgaccttgg ttactttttt accctgaggg gctcagcact cattaggact 3480
tggtgcctaa ttttataaaa agtcactaag ctcaagtgct tggatgaaag gacagcgtgg 3540
ataaaaaggt ttttaaaaca tggatgttaa ggctgttttg cttggagaag acttgggact 3600
gggacagtct ttagatatta tttgaaatgc tggcactgtc tatctggatc ccagggcttg 3660
aactaggatt tgaggaagtc acagggaagc agatttcagt ctgacattta ttcagtgcaa 3720
gtttttttggt gctgtagtat atgatgaaag atgtaaagct gaataaagca ttatttctgc 3780
cctagagttg ttcacagcct agtcaggcat atggatatgt aaacaatgac tgtaacgtgt 3840
tatagatgta aagacaaaat aaaggttaaa gagggcataa aggagcactc aattgcagag 3900
atttgaggac attattttta ttttgagctt taaaaagatg aataggtgtt ctcaggaggt 3960
agggatctgg ctgagaggga ataatctgag caaaggtatg aaacagccta atgcattaga 4020
gaaaaaagtt cttttagtaa ggcatttggg gttggggaag ctagaaaaag aaatgggagc 4080
tggtcacaca gggccttgtg tgccagacta aggggtttgt agtatatatt gtaggcagaa 4140
gagatccatc aacagattgc aagcaaggaa gtatgttcac tttaaagttt gagaaagaat 4200
agtgtggaag cacgtctcaa atttagactt gttccccctc tgaaccgtga atcagaccat 4260
ttcaggtaga agtcttcccc ggtttatctg atctactcgg ggcctcaggc ttctcagctg 4320
ggaagagagg atgcaagacc agactgaaga acacggttga gtccccagaa ccaaaagggg 4380
gcctttctgc ttcttagcca gctacctctt cgagtttttc aaattgtgag gggggaccata 4440
aaaggatgga aacttttaga tgacattcta caaattattt ttttctttaa attaaaagaa 4500
cctagccaat aagatagaga atgggcatct aaggcatctc agagctctct gatgaagcca 4560
ggttgtcaaa gatcatttgc aaaagaaggg aaaactggca tgacaaaagc tacagagagg 4620
agagtgaaat atagaagtgt ttgaaatgtt caagctcaca ataagcttaa atttatagaa 4680
aatgctaagg ttgtcaagaa ggcttttttt tttttctttt ttaaacctga gggcaaaaag 4740
gaatggataa agtagtgtaa tggattgaca atcaggaaga acagaataac tcagtttttt 4800
ttctcctaca aggagatatg gctggaccaa aataaaatga catgaaattg caaaaatgaa 4860
aa                                                                4862
```

<210> 248
<211> 9950
<212> DNA
<213> Homo sapiens

<400> 248

```
acactcctag aatatgcaga gaaatggaaa acttcagaag atcctttacc tttattggag 60
gtatacacag tggctatcca aagttatgtt aaagcccgac cttatcttac ctctgaatgt 120
gaaaatgtag ccttggttct ggaacgcttg gcattaagct gtgttgaact tttactgtgt 180
ctgcctgttg agttatcaga taaacagtgg gaacaatttc agacactggt gcagtgttat 240
atttaaagcc tgataagctt gcttttaaaa atctgttgta attctttgga tgtttattta 300
atatttatag gtagctcatg aaaagctgat ggagaatggc agctgtgaat tgcatttttt 360
agctactcta gctcaagaga ctggggtgtg gaaaaacccg gtactgtgca ctattctttc 420
ccaggaacca ttggataagg ataaagcttt tgagggtccc atcttgttgg atatgagaat 480
taaacatcta atcaaaacaa atcagttaag tcaagcaact gctctagcaa agctgtgttc 540
tgaccatcca gagattggca taaaaggtag ttttaagcaa acttaccttg tctgtctttg 600
tacatcatca ccaaatggaa agttaatcga agagatttca gaagttgatt gcaaagatgc 660
actggaaatg atctgtaact tagaatctga gggtgatgaa aaaagcgctc ttgttttatg 720
tactgcgttt ttgtcacgtc agctccaaca aggagatatg tactgcgctt gggaacttac 780
tctcttttgg agtaaattac aacaaagagt agaaccatct atacaagtgt accttgagag 840
gtgtcgtcaa ctttctttgt taacgaaaac agtatatcac attttcttcc tgattaaagt 900
tattaattca gagactgaag ggctggact tgctacctgt atagaactgt gtgtaaaggc 960
tcttcgcttg gagtctacag aaaatactga agtgaaaata tctatttgca agaccatttc 1020
atgtttgttg cctgatgatc tggaagttaa acgtgcttgt caactgagtg aatttcttat 1080
tgagcctaca gtagatgcgt attatgctgt ggaaatgttg tataatcagc cagaccagaa 1140
atatgatgaa gagaatcttc caataccaaa ttctttacgc tgtgagctgt tacttgtatt 1200
gaaaactcaa tggccctttg atccagaatt ctgggattgg aaaaccttga aacgacaatg 1260
tcttgcatta atgggagaag aagcatccat tgtgtcttca atagatgaac taaatgacag 1320
tgaagtatat gaaaaagtgg tagactacca agaagagagt aaagaaactt ctatgaatgg 1380
gctttctggt ggagttggtg ctaattctgg ccttcttaaa gacattggtg atgaaaagca 1440
gaagaagaga gagataaaac agttaagaga gaggggattt atatctgctc ggtttaggaa 1500
ttggcaagcc tacatgcagt attgtgtgtt gtgtgacaaa gaattccttg gtcacagaat 1560
agtacgacat gctcagaaac attacaaaga tggaatttat agttgcccca tatgtgcaaa 1620
gaactttaat tctaaagaaa cttttgtccc tcatgtcaca ctgcatgtta aacaatctag 1680
taaagagaga ctagcagcta tgaaaccatt aagaagattg ggaaggcctc caaagatcac 1740
aactaccaat gaaaatcaga agactaatac tgtggctaaa caggagcagc gacctataaa 1800
aaagaatagt ctctattcaa cagattttat agtgtttaat gacaatgatg gttcagatga 1860
tgagaatgat gacaaagata aatcctatga gccagaagtg attccagtcc agaaaccagt 1920
acctgttaat gaatttaatt gccctgtaac tttttgtaaa aagggcttta agtactttaa 1980
```

aaatttaatt gctcatgtga aggggcataa agataatgaa gacgccaagc gctttcttga 2040
aatgcagagc aaaaaagtta tttgccagta ctgtaggcgg cattttgtga gtgttactca 2100
tctcaatgat cacttacaga tgcactgtgg cagtaaacca tatatctgta tacagatgaa 2160
atgtaaagct ggttttaata gttacgccga gcttttaacc caccgaaagg agcatcaagt 2220
ctttagagca aaatgtatgt ttcctaaatg tggaagaatt ttttcggaag cttatttact 2280
atatgatcat gaagcacaac attataatac gtacacttgt aagttcacag gttgtggtaa 2340
agtttatcgt tctcagggtg agctggaaaa gcatctggat gatcacagta ctcctcctga 2400
aaaagtgctg cctcctgaag cccaacttaa ttcatctgga gattccattc agccttctga 2460
agtgaatcag aacacagcag agaatattga gaaagaaaga tctatgcttc cttcagaaaa 2520
taacattgaa aacagcttac tagcagatag aagtgatgct tgggataaaa gcaaagcaga 2580
atcagctgtg accaaacaag accagatttc tgcctctgag ctcaggcaag ctaatggacc 2640
attgtcaaat ggtttggaaa accctgctac tactcctcta cttcaatcca gtgaagtagc 2700
tgtgtccatt aaggtgtctc tcaatcaggg gattgaggat aactttggaa agcaagaaaa 2760
ctcaactgtg gaaggcagtg gtgaagcact ggtcacagac ttacatacgc cagttgaaga 2820
tacttgtaat gatttgtgtc atccaggttt ccaggagaga aaagaacaag attgctttaa 2880
tgatgcccat gttactcaga attctttagt aaattcagaa actctcaaaa taggtgacct 2940
taccccacaa aacttagaaa gacaagtgaa caacttgatg accttttctg tgcaaaatca 3000
ggcagcattt caaaacaatt taccaacttc caaatttgaa tgtggagata atgttaaaac 3060
atcatccaat ctttataatt tacctcttaa gacattagaa agtattgcat ttgttccacc 3120
gcagtccgac ctaagtaatt cattaggaac tccatcagtg cctccaaaag ctccagttca 3180
gaaattcagc tgccaggtcg agggatgtac tcgaacctat aattcttcac agagtattgg 3240
gaaacacatg aagacagcac accctgacca atatgctgca tttaaaatgc agcgcaaaag 3300
taaaaaaggt cagaaagcta acaacttaaa tacaccaaat aatggaaagt ttgtttattt 3360
tttgccatca ccggtgaaca gctcaaatcc atttttttaca tcacagacca aagccaatgg 3420
gaatcctgct tgttcggccc agttgcagca tgtctcgcca cccatttttc cagctcattt 3480
agcaagtgtg tcaactccat tgttgtcctc aatggaaagt gtcataaatc caaatataac 3540
ttctcaggat aaaaatgaac aaggtggtat gttatgttcc caaatggaaa atttacctag 3600
tactgccttg ccagcacaaa tggaagatct aaccaaaaca gttctgcctt tgaatattga 3660
cagtggctca gatcctttcc ttcctttacc tgcagaaagt agttcaatgt ctctcttccc 3720
ttcaccagca gatagtggga ctaattctgt tttttcccaa ctggaaaata atacaaatca 3780
ttattcctca cagattgaag gaaacactaa ttcctccttt ctaaaggggg gtaatggtga 3840
aaatgcagtt tttccttcac aagtgaatgt tgcaaataac ttcagtagca ccaatgccca 3900
acagtctgca cctgaaaaag ttaaaaaaga ccgtgggcgg ggcccaaatg ggaaggaaag 3960
aaaacctaag cacaacaaaa gggctaaatg gcctgcaatt atcagagatg ggaaatttat 4020
ctgtagcagg tgttacaggg cttttactaa tcccagatca ctgggtgggc acttatccaa 4080
gcgatcttac tgtaaaccac tggatggagc cgaaattgct caagaacttc tacagagtaa 4140
tggacagcct tctcttcttg ccagcatgat tctctccaca aatgcagtaa atttgcagca 4200
gccacaacaa tctaccttca atccagaagc atgttttaaa gatccatcat ttctacagct 4260
tcttgctgaa aatcgctcgc cagcattttt accaaataca tttcctcgat ctggtgtgac 4320
taactttaat accagtgtca gtcaagaagg tagtgaaatt attaaacagg ctttggaaac 4380
tgctggcatt cccagtacat ttgagggtgc cgaaatgctt tctcatgttt caacaggttg 4440
tgtctctgat gcatcacaag taaatgcaac ggtgatgcca aatccaactg taccacccct 4500
gttgcacact gtatgccatc caaacacctt gctgaccaac cagaatagga cgtcaaactc 4560
caaaacttcc tccattgagg aatgtagcag cttgcctgtt tttccaacga atgacttact 4620
actgaagact gttgaaaatg gtttgtgctc tagttcattt cctaattctg gtgggccatc 4680
acaaaatttt accagtaaca gttctcgtgt ttctgttata agtggtcctc agaacacaag 4740
atccagtcat ttaaataaaa agggaaacag tgcttctaag agaagaaaga aagttgctcc 4800
tccactaatt gcacctaacg cttcccaaaa cttggtaaca agtgacttaa caacaatggg 4860
actcatagca aagagtgttg aaatcccaac tactaacctt cattcaaatg taattccaac 4920
ttgtgaacct cagagtttgg tggaaaatct aacacagaaa ttaaataatg ttaacaatca 4980
gttatttatg actgatgtaa aagagaattt caaaaccagt cttgagtccc atacagtgtt 5040
agccccttta acattaaaaa ctgaaaatgg tgattcccaa atgatggctt tgaattcatg 5100
cacaacttca ataaattctg atttgcagat ttctgaagac aatgttatac aaaactttga 5160
aaagactctt gaaattatta aaactgctat gaattctcaa atacttgagg taaaaagtgg 5220
atctcagggt gctggtgaaa cttcacaaaa tgctcaaata aattataaca ttcagcttcc 5280
ttcagtaaac actgtgcaaa ataacaaatt acccgattct tctccgtttt cctcctttat 5340
aagtgtcatg ccaacaaaaa gtaacattcc tcagtctgaa gtatcacata aggaggatca 5400
aatacaggaa attttagaag gcttacagaa attaaaatta gaaaatgacc tatccactcc 5460
agcatcccaa tgtgtactga taaatacatc agtgacactg actcccacgc ctgttaaatc 5520
aactgcagat atcacagtta ttcagccagt ttctgaaatg ataaacattc aatttaatga 5580
caaagttaat aaaccctttg tgtgtcaaaa ccaaggctgt aactacagtg ctatgacaaa 5640
ggatgcacta tttaagcact atggtaaaat tcatcaatac actccagaaa tgattcttga 5700
aattaagaag aatcaattga aatttgctcc ctttaaatgt gtagtaccta catgtacaaa 5760
aacatttaca agaaattcta acctccgggc acactgtcag ttggtgcatc attttacaac 5820
tgaagaaatg gtaaagttaa aaattaaaag gccttatgga agaaaatctc agagtgaaaa 5880

tgtgccggcc tcacgaagta cacaagtgaa aaaacagcta gctatgacag aggaaaataa 5940
aaaggaatct cagcctgctt tagaattgag agcagagacc caaaataccc acagtaatgt 6000
agcagtgatc ccagaaaaac aacttgtaga aaaaaaaagt cctgacaaaa cagaaagttc 6060
tttacaagtg attacagtta cttcagaaca atgtaataca aatgcactca caaacacaca 6120
aaccaaagga cggaagatta ggaggcataa aaaagaaaag gaggagaaaa aacgaaagaa 6180
gccagtttcc caatcccttg agtttccaac aagatacagt ccttacagac cttatcgatg 6240
tgttcaccag ggatgctttg ctgcctttac gatacagcaa aacttgattc tccattacca 6300
ggctgtacac aaatcagatc tacctgcatt ttcagcagag gtcgaagagg aaagtgaagc 6360
tggtaaagaa agtgaagaaa ctgaaactaa acaaactttg aaagaatttc gatgtcaggt 6420
aagtgactgt tctcgaattt tccaagcaat tactggccta atacaacact acatgaaact 6480
tcatgaaatg actcctgaag aaattgaaag tatgactgct tcagtggatg ttgggaagtt 6540
tccatgtgac cagttagagt gtaaatcttc atttactaca tatttgaact atgttgttca 6600
tctagaggca gaccacggga ttggactaag ggcaagtaaa acagaagaag atggtgtata 6660
caaatgtgat tgtgaaggct gtgaccgtat atatgcaacc cggtcgaatc tcctccgaca 6720
catttttaat aagcataatg acaaacataa ggctcatttg attcgtccaa gaagattaac 6780
accaggccag gaaaatatgt caagcaaggc aaaccaagaa aaatcaaagt ctaaacatcg 6840
ggggaccaag cacagcagat gtggaaagga aggaataaaa atgcccaaga ccaaacgaaa 6900
gaaaaaaaat aatttagaaa acaagaatgc aaagattgtg cagattgaag aaaataagcc 6960
ttattctctg aaacgtggga agcatgtata ttctataaag gctagaaatg atgccctgtc 7020
tgagtgtaca agcagatttg taacccagta tccatgtatg ataaagggat gtacttcagt 7080
tgttacaagt gaaagcaata taattagaca ttataagtgc cataaattat ctaaggcatt 7140
tacatcacaa caccgaaatc ttcttattgt attcaaacgg tgttgcaact cacaagtaaa 7200
ggaaacgtct gagcaagaag gtgctaagaa tgatgtgaaa gattctgaca cgtgtgtatc 7260
agagagcaat gataattcaa gaacaacagc tacagtttca caaaaggaag ttgaaaaaaa 7320
tgaaaaagat gaaatggatg aactaacaga attgtttatt acaaaattaa taaatgaaga 7380
tagcacaagt gtagagaccc aagctaatac ttcttcaaat gtaagtaatg attttcagga 7440
agataacctc tgccagtcag aaagacaaaa agcaagtaat ttgaagagag ttaataagga 7500
aaaaaatgtc tcacaaaata aaaaaaggaa agttgaaaaa gctgaaccag catcagcagc 7560
tgagttaagt agcgtgcgta aagaagaaga aactgctgtt gccattcaaa ccattgagga 7620
gcatcctgca tcttttgact ggagctcttt taagccaatg ggatttgaag tatcatttct 7680
gaagtttctt gaggagtctg cagtgaagca gaagaaaaat actgacaaag accatccgaa 7740
tactggaaac aaaaaaggat cccattcaaa ttcaagaaaa aatattgata agactgctgt 7800
gactagtgga aatcatgtat gtccttgtaa agaaagcgaa acgtttgtac agtttgccaa 7860
tccatcacag cttcagtgca gtgataatgt aaaaattgtt ttagacaaga atcttaaaga 7920
ttgcactgag cttgtcttaa agcaacttca ggaaatgaaa cctaccgtca gtctgaaaaa 7980
acttgaagta cattcaaatg atccagatat gtctgttatg aaagatatca gtataggtaa 8040
agccacaggc agaggtcagt actgataatt aatgtagtat aaatacatca tttaccattt 8100
tattttaaat aggaagccat caagcatgct agaattgtga aactttcatt atattttttt 8160
gttgttgaca tgaattaacc tggccaaaaa caaaaaagaa aaaaaaaaca tgacatttgt 8220
catgtaaaac ttttttttat ccctatggga cttgaggaac agaatcagta cttcagttat 8280
tgtaaatagt gagctaaacc tcaaatttct atcacccagt tgcccttttc atgaactaaa 8340
caattatctg tgtgattggt atgtttcacc aggtcactgc tcatgtataa cagtactctt 8400
tatttgtaga taccttttttt gtatatattt attattgtaa atcatgtgct gccaccagca 8460
gtctgtaagt ctaaactatt aaatgacaca tttatatttg gaattttaat ttttaactaa 8520
gcgatcaagt tttttaaatt gttcttttat tgttttaaat taagaacttt ttgaactaaa 8580
accagaaact ctgccatagt tcattgattt ttacatgaaa catttattta caagatgata 8640
tcaagattac ttttcacaaa ataggcacat tatatcaaca ctttgctctg ctttgtaaat 8700
ttgccatcca ggattttggg gtggtattca tgtgaaatta aagcagattc tctagcagtt 8760
tcctatagct acagtttttt tttttcattg cttctaattg gatatagtta ctatgagtcc 8820
atgaaataca atggaaatgt gaataaagaa tttactacat tgaagatttt aaacatttgg 8880
tattaaaaaa aaatcctttg tgaatgtgat agaaaactag tagtgtggag cagtgtaaat 8940
atttgaggtg gtgatttgtg aagtagccca gtattgcctt aaataaaatc acatttcatt 9000
tcttgtttta tacatgtgat cttataaaga tttttgtttt tgttttgttt tccattttct 9060
gagatttata gattggtgta tagctttaaa ctgtataaag ttttgtggaa agattttttt 9120
aaacattttt ataaatctta aaattgatat catcaaagtg agcccatctt gtgtttataa 9180
aatacaagag tccttaacat ttataattac atagataaaa cactttctat aaggaagttg 9240
gatgttttga ttttactgtt tatagatgtt agattgtaca gatttgtctg tatttctcac 9300
catatctaat gatacttttt tcattagatt ggtcttcaag aacagtatta gttataatta 9360
ttttggttat tcagtatata gttagctctt acagtttagc tttattcacc atatttatac 9420
tgtggattca cagcgagagg tagaggttat tccaggagag ttgatgacct tcatttaaag 9480
tccaactaaa atcagtagta gaaacataag aaaacatctt tgcaatattt acttttgttt 9540
ctgtttgccg taaatagtaa cattgttttt ttttattttg tgtttgttat aaaacagttg 9600
cattcacaat attattggcc tgagatattg atgatattgt gatggtatga aaatgtgtac 9660
attccctgtg caacatcaga tttgcaggaa aaatgaagca cttactgaaa tcgctggtac 9720
tctgaataaa taagcatggt caaggagtga actattttct tttggaaatt tctttttaat 9780

ttttattatt aaagtatttt atttttaggg ccttttgggt tttattgaat agttcatttc 9840
acctgtttaa gacttactac caataagcat aaaacctgac acgttaaaat ccctgccctt 9900
tggtgagccc actgttattt attaaaataa aagttatttt atgggtgatt 9950

<210> 249
<211> 2829
<212> DNA
<213> Homo sapiens

<400> 249

actgagagac gccctggaac gtctgtggca gcttctgtct cgctgggacc cgcactgaca 60
gcgggaggca gagggaggaa cctggacgcc ggaagccggg aaatggactc agtggcattt 120
gaggatgtgg ctataaactt cacctgtgag gagtgggctt tgctgggtcc atcacagaag 180
agtctctaca gagatgtgat gcaggaaacc atcagaaacc tggactgtat agactgaata 240
agaggagggg ttggtcttgc tgtctcaaga gtagcagaga ctgaagaaat tcatatgaat 300
gatgatggca aaatcatgat atagaagaag atcagtacaa agatctcaga agaaatctaa 360
gatgtcatat ggtagagaga gcctgtgaaa ttaaagataa tagtcaatgt ggaggaccct 420
ttacccagac tcaagacagt attgtgaacg agaaaatacc tggagtagat ccatgggaaa 480
gcagtgagtg tacagacgtc ctcatgggtc gttcatctct taattgctac attagagttg 540
acagtgaaca caaaccatat gagtatcaag aatatggaga gaagccatat acacatacac 600
aatgtgggac agctttcagt tatcagccct gctttcaaat acatgaaaga cctcagcatg 660
gaaagaaact ctatgattgt aaggaatgtg caagcttcag ttctcttgaa aaccttcaaa 720
gacacatggc agcacaccat ggagatggac ctcgtatatg taagttgtgt ggaaacgcct 780
ttatttggcc tagtttattt catatgctta gaagaactca cactgaagag aaaccatatg 840
aatatgagca gtgttctaca gcgtttcctg cttatagttc cactctaaga catgaaagaa 900
cacacagtgg agagaaaccc tatcaatgta aacaatgtgg gaaagccttc agttgttcct 960
gttacactca actatatgaa aggactcaca ctggagaaca atcctatgaa tgtaagcaat 1020
gtgggaaagc attttatcat cttggaagct ttcaaagaca catgatagtg cacactggag 1080
atgggcctca taaatgtaag atatgtggaa aaggctttct ttctcccagt tcagttcgaa 1140
gacataaaag aactcacact ggagagaaac cgtatgaatg taagtattgt gggaaagcat 1200
tctctgattg cacaggtttt cgaagacaca tgataacgca cactggagat ggacctcata 1260
aatgcaaggt atgtgggaaa gcctttgatt ctcccagttt atgtcgaagg catgaaacag 1320
ctcatactgg ggagaaaccc tataaatgtg aatgtgggaa agcctttagt gatttctatt 1380
actttcgaaa tcatgaaact actcacactg gagagaagcc atataaatgt aaacaatgtg 1440
gaaaagcctt catttgttgc acttaccttc aaatacatga aagaattcac actggggaga 1500
gaccctataa atgtaaacaa tgcggaaaag ccttcaggtc ttccaattac attcgagtac 1560
atgaaaagac tcacactgga gaaaagccct atgaatgtaa gcagtgtgga aaagcacttt 1620
ctcatctgaa aagctttcag agacacatga taatgcacac tggagatgga cctcataaat 1680
gtaagatatg tgggaaaagc tttgattctc ccagttcatt tcgaagacat gaaagaattc 1740
acactgggga gagaccctat aagtgtaaac tatgtgggaa aggcttcagg tcttccagtt 1800
acattcaact acatgaaagg actcacactg gagagaaacc ctatggttgt cagcaatgtg 1860
ggaaagcatt atctgatctc tcaagctttc gaagacacat gataacacat actggaaatg 1920
gacctcataa atgtaagata tgtgggaaag gctttgatta tcccagttca gtgcaaagac 1980
atgaaagaac tcacactgga gagaaaccct atgaatgcaa ggaatgtggt aaagccttca 2040
gtcattcaag ttacttacga atacacgaaa gagttcatac tggagagaag ccgtataaat 2100
gtaaggaatg tgggaaacca ttccattgtc ccagtgcctt tcataaacat gaaaggaccc 2160
acagtatgga gaaaccctat aagtgtaaag aatgtgggga agcatttcat tgtatcagtt 2220
cctttcataa acatgaaatg actcactaga gaaaacccct atgagtgttg aacatgtgag 2280
aaagccttaa gtactttcag cttcttacaa atacataaga gactcacact gaaaaaagtt 2340
gtatgaatat aaaaatatac taaaaccttc catttttttc agtacctttt gaaaacatga 2400
ccaaactcat acttcaaaaa tatatatata atgaatgtga ggaatatgaa aaaactttct 2460
ttctctagca aactttcaaa ggtggttatt ataacatact agcaatggac cttactactg 2520
taaggaatgt agaaatgcat ttactagtcc taactcattt cagttacatg aaagatttca 2580
aactgcagag aaccactatg aatgtaaaca atgtggtaaa gcatttagta gcttcagttt 2640
cttttgaata cagtcttctc tagtttctga ggtgaattga ttccagcacc ctcaggaata 2700
cctatattca gagatattca agtccctgat attaaaaaca gcatatttgc atataatcta 2760
ggcacatttt tctgtatatc ttaaatcatt tctagattac ttataataca caatgcattg 2820
taaattctg 2829

<210> 250
<211> 4722
<212> DNA
<213> Homo sapiens

<400> 250

ggccattgtt tctctctgct gccggagctc caggtctacc cttcactgct ctgtgtcctc 60
agcgtgtgtg gcttcgtgac ctgaagatac tgggaaatcc atagctaaga tgccaggacc 120
ccctgaaagc ctagacatgt tatatgttct cattttgctg aagacttttg cccagggcca 180
ggcattaaag attcttttca aaaagtgata ctgagagaat atgtaaaatg tggacataag 240
gatttacagt taagaaaagg atgtaaaagt atgaatgagt gtaatgtgca caaagaaggt 300
tataatgaac taaaccagta tttgacaact acccagagca aaatatttca atgtgataaa 360
tatgtgaaag tctttcataa acttttaaat tcaaatagac ataacacaaa acatactgga 420
aagaaacctt tcaaatgtaa aaaatgtggc aaatcatttt gcatgctttt acacctatgt 480
cagcataaaa gaattcatat tagagagaat tcttaccgat gtgaagaatg tggcaaagcc 540
tttatctggt tttcaaccct tactagacac aggagagttc atactggaga gaaatcctac 600
aaatatgaat gtggcaaatc ttttaaccag gactcaaacc ttactacaca taagagaatt 660
catactggac agaaacccta caaatgtgaa gaatgtggca catctttcta ccaattctca 720
taccttacta ggcataagct aattcatact agagagaaac cctataaatg tgaacaatat 780
ggcaaaactt ttaaccaatc ttcaaccctt actggacata agataattca taatggagaa 840
aaaccctata aatgtgaaga atgtggcaaa gcctttagta ttttctcaac ccctactaaa 900
cataagataa ttcacactga agagaaatcc cacagatgtg aagaatattg caaagcttat 960
aaggagtcct cacaccttac tacacataaa agaattcata ctggagagaa accctacaaa 1020
tgtgaagaat gtggcaaagc ctttagtatt ttctcaaccc ttactaaaca taagataatt 1080
cacactgaag agaaatccca cagatgtgaa gaatgtggca aagcttataa ggagtcttca 1140
caccttacta cacataaaag aattcatact ggagagaaac cctacaaatg tgaagaatgt 1200
ggcaaaacct ttagtgtatt ctcaattctt actaaacata aaataattca tacagaagag 1260
aaaccctaca aatgtgaaga atgtggcaaa gcttttaaac gatcttcaac ccttactaaa 1320
cataggataa ttcatactga agagaaaccc tacaaatgtg aagaatgtgg caaagctttt 1380
aaccaatctt caacccttag tatacataaa ataattcata ctggagaaaa accctacaaa 1440
tgtgaagaat gtggcaaagc ttttaaacga tcttcaaccc ttactataca taaaatgatt 1500
cacactggag aaaaacccta caaatgtgaa gaatgtggca aagcttttaa tcggtcctca 1560
caccttacta cacataagag aattcatact ggacacaaac cctacaaatg taaagaatgt 1620
ggcaaatcct ttagtgtatt ctcaaccctt actaaacaca agataattca tactgataag 1680
aaaccctaca aatgtgaaga atgtggcaaa gcttttaacc gatcttcaat ccttagtata 1740
cataagaaaa ttcatactgg agaaaaaccc tacaaatgtg aagaatgtgg caaagctttt 1800
aagcggtcct cacacctcgc tgggcacaag caaattcata gtgtacaaaa accctacaaa 1860
tgtgaagaat gtggcaaagc ctttagtata ttctcaaccc ttactaaaca taagataatt 1920
catactgaag agaaacccta caaatgtgaa aaatgtggca aaactttcta ccgattctca 1980
aaccttaata cgcataagat aattcatact ggagagaaac cttgcaaatg tgaagaatgt 2040
ggcaaagctt ttaaccattc ctcaaacctt attaaacata agctaattca tactggagac 2100
aaaccctaca aatgtgaagc atgtggcaaa gcttttaggc ggtcttcaca tcttagtaga 2160
cataagataa ttcatattgg aattcatact gaagagactg tacaaaagtg aagaatgtgg 2220
caaaggcctt tactgctcct attcccttac taaagaatgt ggcaaagctt ttcaccagta 2280
ctttaccctt aatacacata agataattaa tgctggagag aaaccctaca aatgtgaaga 2340
atgtggcaaa gatttctatt gattctcata ccttactaaa tataagataa ttcatattgg 2400
agagaaattc tacagatgtg aagaatgtgg caaaggcttt aattagttct catcccttac 2460
taaacataag agaattcata ccatagagaa atcctacaaa tatgaagaat gtgacaaagc 2520
ttttaaccac ttctcaaccc tgcctacacg taagataatt catactggaa ggaaacccta 2580
caaatatgag gaatgtctca aagcttttta ctgattctta taccttacta aacataaaat 2640
aattcataaa ggagataaat tatacaaatg tgaagaatgt ggcaaagctt ttaacaaatc 2700
ctcatccatt agtaaacata agataattca tactggagag aaaacctaca aatgtgagga 2760
atgtggcaaa gcctttagcc tgtccctcca atttactgca cataagataa tttatactgg 2820
agagaagccc tacaaatgtg aaaaatgtgg caaacctttt aaccaatcct caacccttac 2880
tacacattag ataattcatg ctggagagaa accctacaaa tgtgaaaaat gtggcaaagc 2940
ttttaaccaa ttttcaaacc ttactaaaca taagataact catactggag aaaaatctta 3000
caaatgtgaa gaatgtggca aagcctttat ccagtcctca actcctagta aacataatta 3060
atgatggaga gaaaccatac aactgtgaag aatgtggcaa agcttttaac cagtcctcaa 3120
actttattga acaaaataat tcatacagga gagaaaccct acaaatgtga agaatgtgac 3180
aaagccttta accagtcctc aatttttact aaacataaga aaattcatac tggagagaaa 3240
ccctatgatt gtgaaaaata tggcaaaggc tttaactagt cctcagttct taacacacat 3300
acgataattc ttactgcaga gaaactctac aaaccagtaa gatgtgacag tgcttctgac 3360
aacatctcaa actttctaa tcataaaaga aatcatattg gtgagaaatc ctagaaatgt 3420
ggagaatgta acaaagtatt taaatggttg tcacacttga ttataggtaa tattcatatt 3480
ggaaaaattt cctacaagta agaacaatgt ggcaaagttt ttaactaata caccttattg 3540
cacagaaaat catttatatt tgagaaaaat tgtagaaata tagactgtga aaaagacgtc 3600
aatatctgct cacatcttac taaacaccag agagttcatg cttaataaaa gcatgataag 3660
tgcaattact gccaaaagat ctttcagaaa atattatcct ttaaagtgaa ggagagtatt 3720
tatattaaag atgaacatta caaccataaa gagggttgaa gtacctttac ttgtatcaga 3780
tcttattgtc cacattttgt actacagaaa aactctgaag aggtcactca aactttgttc 3840
aacatcaggg aatttatatt ggagagctgt cttgcaaatg taataaattt gggaaaacaa 3900

```
attttcaaaa actacagctt agaaaacacc agagtttata cgaaaatata ttttcaaagg 3960
tgtagtaaaa ataaaaaaaa ttttaatcca aatttgtcta tgtaaatacc agaatttata 4020
gtagaaatat atgaggaagc gacacttcga atattctact aaatgagagt tctgagtata 4080
gaaaataaaa ctaaagttgg tagaaaaatt atttgtatat aatgttaaga ggagtaaaag 4140
atttttgta gaataataac tatattcgga ttatactttg tttcttgaaa aaattacaga 4200
ttttttgaaa agcaaatgat gtaactcaac tcattatttt ctgctgtttc ttcattctta 4260
ttcacttgtg aaagcttgtg atcatttgtt gctgcatcag aggtatgaga gattcttctt 4320
cattagatgg gcattattta tgatcttttc tatggatgag taagaatatt aaaatgtaag 4380
atgcatggtg aaaatctaag tggagaggtt ctttgtggtt aacttatact attgagtgat 4440
gcacaaggta ggtgttaaga gtaatattct tttgcattat gagaaaacta gtatattatt 4500
catatatttt actaattgta cttttttat tatactttaa gttttagggt acatgtgcac 4560
aatgtgcagg ttagttacat atgtatacat gtgccatgct ggtgcgctgc acccactaac 4620
tcgtcatcta gcattaggta tatctcccaa tgctatccgt ccccctccc cccaattgta 4680
cttttatata ataaaatgca gtacatttta aaaaatttta aa                   4722
```

<210> 251
<211> 1905
<212> DNA
<213> Homo sapiens

<400> 251

```
gttagtctcc gctgctagtt cttggctctg ggaggcccag gtggctctgc agcagcctct 60
gccaccctgt gacctgcgag tattgggaca tccctagctg acgccaggac acccgggaag 120
ccgaggaatg gtgagtggac tgcgcctggc ttcccgaagt ggagaagagg gctggttgaa 180
acctgctgtg gcgcgactcg ggcctccacg ccatcggctc cggaacctgc ggaccgagtc 240
gccgtggcgt agccggggct cagtcctctt ctgttcaggg ccgggccggg cggggcgggc 300
agcggaaccc ctgcatcctg tctgtacctg cgggcgccac ttccgccggc cggatccctg 360
tcgggaaccc ctcgcctccc ctatccagga ctcggtggct tttgaggatg tggctgtgaa 420
ctttacccag gaggaatggg ctttgctaga ttcttctcag aagaatctct acagagaagt 480
gatgcaggaa acctgtagga acctggcttc tgtaggaagc caatggaaag accagaatat 540
tgaagatcac ttcgaaaaac ctgggaaaga tataagaaat catatcgtac agagactgtg 600
tgaaagtaaa gaagatggtc agtatggaga agttgtcagc caaattccaa atcttgatct 660
gaacgagaac atttctactg gattaaaacc atgtgaatgc agtatttgtg gaaaagtctt 720
tgtacgtcat tccctcttta ataggcatat cctagctcac tcaggataca aaccatatgg 780
agagaagcaa tataaatgtg aacagtgtgg gaaattcttc gtttctgttc caggtgttag 840
aagacacatg ataatgcaca gtggaaatcc agcttataaa tgtacgatat gtgggaaagc 900
tttttatttt ctcaattcag ttgaaagaca tcagagaact cacacaggag aaaaaccta 960
taaatgtaaa caatgtggta aagcattcac tgtttccggt tcttgtctaa tacatgaacg 1020
aactcacact ggagagaaac cctacgaatg taaggaatgt gggaaaacat tcagattttc 1080
ttgttctttt aagacgcatg aaaggactca cactggagaa agaccctata aatgtaccaa 1140
atgtgataaa gccttcagct gttccacttc ccttcgttac catggaagca ttcatactgg 1200
agagagaccc tatgagtgta aacaatgtgg caaagccttt agtcgtttga gttccctttg 1260
taaccataga agtactcata ccggagagaa accctatgaa tgtaaacaat gtgatcaagc 1320
cttcagtcgc ctcagttccc ttcacctcca cgaaagaatt catactggag aaaaacccta 1380
tgaatgtaag aaatgcggta aagcctacac tcgttccagt caccttactc gccatgaaag 1440
aagtcatgat atagaggctg ggtgtagtga ctcagcctat aatcccagca ctttgggagg 1500
ccaaggcgtg tggattgctt gagcccagga gttcataacc agcctggatt aaaacaatgt 1560
gaaacaacct gggcaacatg gtgaaaccct gtctttacaa aaaataaaat aatgccgggc 1620
acggtggctt acgcctgtaa tcccagcact ttgggaggcc gaggcgggtg gatcacgagg 1680
tcaggagatc gagaccatcc tggccaacac ggtgaaaccc cgtctctact aaaaatacaa 1740
aaaattagct gggcgtggtg gcgggcgcct gtggtcccag ttactcgggg ggctgaggca 1800
ggagaatggc atgaacccgg gaggcggaac ttgcggtgag ccgagatcgc accactgcac 1860
ttcagcctgg gtgacagagt gagactctgt ctcaaaaaat aagat                1905
```

<210> 252
<211> 2952
<212> DNA
<213> Homo sapiens

<400> 252

agactgacca ctagccgacg cgggcgagag ggacaggagc gtgacctccc catcccgagg 60
ggccggacgc tcgggcgcct ccccgctccc cccactcgga ggccgcgcgc gccgttagcc 120
ccttcctcgc tcccccgccc cagtcccgca gtccgggagg cgggggtcgg cagccggctg 180
agtgggaacc gcgcggtgtc tgaggaggca gtcggcgacc ggtttccact tcaagcgtga 240
ccctttttgcc tgtgggatga gctccagcat ggggtgaggt acagaagaga gacttgaaga 300

gcgtgccttg ggactcaagc gccaaacctg taccctagcg agtgtcctac tccgcatccg 360
taatggaagg aaatgcacat cttactccag aggcacaaga ggaggacatc ccatgcggct 420
actcctgccc agcgtggtgg ggcagcagaa gctccagagc ccagacttgc aggctcacgg 480
tgcagggtga acctggccac agctcaccct ggaacagcca caatgtctgc cccttagaga 540
agaaccctga aatcagacca gtttttgcgg cctccccctt tcctctctgt tacagtgccc 600
tttccaggcc ttaagagaag taaaacttag ctgcagcgcc aggaggtgga ccccagagtg 660
tgagtggcac gcttccctgt gaacccgtcc tcaccatgtt tgccacatct ggggcagtgg 720
cagcggggaa gccttactcg tgcagcgaat gtggcaagag cttctgctac agctcagtgc 780
tgctgcgaca tgaacgagct cacggcggtg acggccgctt ccgttgccta gaatgcggtg 840
agcgctgtgc acgggctgct gacctccgag cgcacaggcg cacgcatgct ggccagaccc 900
tctacatctg cagtgagtgc ggacaaagct tccgccacag cggccgtctt gacctacact 960
tgggcgcaca ccggcagcga tgccgcactt gcccctgccg cacatgcggc cggcgcttcc 1020
cgcacctccc ggcgctgctg ctacaccggc gccgccagca tctgccagag cggccccgcc 1080
gctgcccgct gtgcgcccgc accttccggc agagcgcgct gctcttccac caggcgcggg 1140
cgcacccctt ggggacaacc tctgaccctg ctgccccacc ccaccgctgc gcgcagtgcc 1200
cgcgagcctt ccgaagcggc gccgggctgc ggagtcacgc gcgcatccac gtgtcccgga 1260
gccccacgcg accccgtgtc tcagacgccc accagtgtgg cgtgtgcggc aagtgctttg 1320
gcaagagctc tacgctgacg cgacacctgc agacgcactc gggggagaaa cccttcaagt 1380
gcccggagtg cggcaagggc ttcctggaga gtgccacgct ggtgcgccac cagcgcacac 1440
acacgggcga gaagccgtac gcatgtggcg actgtggacg ctgcttcagc gagagttcca 1500
cgctgctgcg ccatcggcgc agccatcagg gcgagcggcc acatgcgtgc gccacttgcg 1560
gcaagggttt cgggcagcgc tccgacctgg tggtgcacca gcgcatccac acgggcgaga 1620
agcccttcgc gtgccccgag tgcggccgcc gcttcagcga ccgctcggac ctcaccaagc 1680
accggcgcac gcacacgggc gagaagccct accgctgcga actgtgcggc aagcggttca 1740
cgtgcgtgtc caatctcaac gtgcatcggc gcaaccatgc cggccacaag ccacacaaat 1800
gccccgagtg cagcaaggcc ttcagcgtcg cctccaagct tgcactgcac cgcaagacgc 1860
acctgggcga acggccagcg gagtgcgcag agtgcggcaa gtgcttcagc cacagccgct 1920
cgctgtcaca gcatcagcgg gcccacacgc gcgcccgcac cgctgccgcc gttgccatcc 1980
agtccgcagt gggcactgcc ctcgtctttg aggggccggc tgaacaggaa aagccagggt 2040
tctctgtgtc ctagttgagg gaggcttgct gaggcttctc taaaggtggt tgggcaagca 2100
cctatatagt atcacgggga cagttgaggc aactcgtaga tggagatttg ggaaaagacg 2160
atgtggcctc ctacctttcc agtttctgtt ggcagccctt cacgtagcct cctgcctcgc 2220
ctctacacct actaccctgt cggccctttt gccatgctgt cctcgtataa ctcggattct 2280
ctcctcaggt gtaggtgcag ggagtcaggg aacccttaga ctcccctgtg tgcaagagcc 2340
caggtgttgg tgtgtccctt taatgctact gtgctctctg gtgtttctga ttttcctgcc 2400
tttattctgt cttctcttgt cctatctcat tccagcccac atcttctcct ttcctgatta 2460
cttttgttgt cctgcctctt caggtaatgg tcacagattt ggctgtaggc acgttaccag 2520
ccctgtggct tcttgactct tggttccctg ttaactctgt ttctgagaaa tgtgggtatg 2580
gaggtgggtg ggaaagctca cttccatgaa ggatgtctcc atgctaggag ctgcctgcac 2640
cctggcagag gtggccagtc acgtgaaggt gggcagggcc cttagcatgg ccacacatgt 2700
ccccagggca gatcaagggg cctctcagaa ccatgttccc cagccaggtg aggaccattt 2760
tcactgggac ccaggccaaa accatgtggg tgcacaaagc caggcactgc caagtggaac 2820
atgaggttat ttccaaatca tgggagccac cagcagggag agggcaggat ggaaaatccc 2880
ctggagccgg tcaacttttt gctcatggct agtgaaataa agttgtttga gtactaaaaa 2940
aaaaaaaaaa aa 2952

<210> 253
<211> 60
<212> DNA
<213> Homo sapiens

<400> 253

tcgccttagc atgtttgctg aacaccttgt ggaagcaaaa atctgaaaat gtgaataaaa 60

<210> 254
<211> 60
<212> DNA
<213> Homo sapiens

<400> 254
ggtgaactca acagaagttg ctcttgactg tttgaaggta gaagcagcct accttttctc 60

<210> 255
<211> 60
<212> DNA
<213> Homo sapiens

<400> 255
ctccacctgt tagaagctat gtgtttgaag gaatgaatca gtgcagtata aataaaattc 60

<210> 256
<211> 60
<212> DNA
<213> Homo sapiens

<400> 256
agcatgttct catcggcgga gccttcttgt gtaatgtaaa ctgtgccatg ttattaaaaa 60

<210> 257
<211> 60
<212> DNA
<213> Homo sapiens

<400> 257
ccaggccaca caataattcg gggttgtatt ttctaagaac tatgtattgt ttttagctta 60

<210> 258
<211> 60
<212> DNA
<213> Homo sapiens

<400> 258
agaggaagag gaagctgggc attaaattac ctcatccagc agaaattcac ggtagtatgg 60

<210> 259
<211> 60
<212> DNA
<213> Homo sapiens

<400> 259
catcagattg tacagtttgg ttgttgctgt aaatatggta gcgttttgtt gttgttgttt 60

<210> 260
<211> 60
<212> DNA
<213> Homo sapiens

<400> 260
taagtaattt gtatgccaaa aaattacaat aagtcaataa agatctcacc tctgaaaaaa 60

<210> 261
<211> 60
<212> DNA
<213> Homo sapiens

<400> 261
aaggctgggc atttgtttga aattagatag gataaagcca aaggtttgaa caagttgtgg 60

<210> 262
<211> 60
<212> DNA
<213> Homo sapiens

<400> 262
ctcagggacc aaggagatgg ggagggcgct tttgtacgtt aattaaacaa attccttccc 60

<210> 263
<211> 60
<212> DNA
<213> Homo sapiens

<400> 263
gggtttatgc actaaacgtt ttgtgccttg gtctaatatt aacatgatgt atgtgtaaac 60

<210> 264
<211> 60
<212> DNA
<213> Homo sapiens

<400> 264
tgctaaatat taaactacta ggttctgaat gtaaaccatt atcttcctgc ttttaaaaaa 60

<210> 265
<211> 60
<212> DNA
<213> Homo sapiens

<400> 265
ttaagagtat tgattgaagt ttgtgatatt catcaataaa aatgagttga taataaaaaa 60

<210> 266
<211> 60
<212> DNA
<213> Homo sapiens

<400> 266
gctaagctac tgtcccatct cttgtgatgt gtaactttta catgtgaata ttaaagtaga 60

<210> 267
<211> 60
<212> DNA
<213> Homo sapiens

<400> 267
agaaatctgt tgcttaaaaa agaagtttct ttggtggtgt gcgtctatag tcccagctac 60

<210> 268
<211> 60

<212> DNA
<213> Homo sapiens

<400> 268
cagtggcatt gtgcatattg ggataggaat ttgattttgt gaactcaagc tattcagcag 60

<210> 269
<211> 60
<212> DNA
<213> Homo sapiens

<400> 269
agggcttcga agataaactg agctccattc ccctggcatg aaactttcag attccccctgc 60

<210> 270
<211> 60
<212> DNA
<213> Homo sapiens

<400> 270
cgcatgagtg aatgtcctat ctgtcgcaag gctattgaac gaaggattct tttgtattaa 60

<210> 271
<211> 60
<212> DNA
<213> Homo sapiens

<400> 271
gtccttgaaa gtctccatct aaagaggaaa agtttgttgg aacagtatat attccctatt 60

<210> 272
<211> 60
<212> DNA
<213> Homo sapiens

<400> 272
acttcttttt ccaggtttta ttttcagttt taaattccta ataaattatt tgaaaacgtt 60

<210> 273
<211> 60
<212> DNA
<213> Homo sapiens

<400> 273
aagtttgtga aaggtttcca tcctctactg tggtccagaa atcaatgtgt ttgtctgaca 60

<210> 274
<211> 60
<212> DNA
<213> Homo sapiens

<400> 274
ctaactcatg tgagcttgat aactgatgaa ctcattggga gcattttaaa cttttctaca 60

<210> 275
<211> 60
<212> DNA
<213> Homo sapiens

<400> 275
gcgcaaaatg cagctttata ctttttactt tcaattgcct agcacaatag tgagtacatt 60

<210> 276
<211> 60
<212> DNA
<213> Homo sapiens

<400> 276
ttctccagga atactgaaca tgggagctct tgaaatatgt agtattaaaa gttgcatttg 60

<210> 277
<211> 60
<212> DNA
<213> Homo sapiens

<400> 277
aaagaagaga actttgtgct ctgttttacc ttactctgtt tagaaaagta tacaagcgtg 60

<210> 278
<211> 60
<212> DNA
<213> Homo sapiens

<400> 278
ccattgagct gttacccgaa gtcagattaa aaatcaggga gtgttttccc tcgtttctgt 60

<210> 279
<211> 60
<212> DNA
<213> Homo sapiens

<400> 279
atgctttgtg gcaatacgtg actcttattc acagtagcaa ttcttatttt actttacgtc 60

<210> 280
<211> 60
<212> DNA
<213> Homo sapiens

<400> 280
aatacagtag cacctaagga gcttgaatct tggttcctgt aaaatttcaa attgatgtgg 60

<210> 281
<211> 60
<212> DNA
<213> Homo sapiens

<400> 281
cagtgcagaa tactaccggc atcttgttac tgcaatagtt ggaaataaaa tgtgaaaatt 60

<210> 282
<211> 60
<212> DNA
<213> Homo sapiens

<400> 282
gtctcaaata ataataataa taataaagct ggagacttgg ccacacctgg gctaaaggaa 60

<210> 283
<211> 60
<212> DNA
<213> Homo sapiens

<400> 283
atgtataaac tctcaccaca ggaagcatcg ctgtttccaa taaatattgc tgaagacaga 60

<210> 284
<211> 60
<212> DNA
<213> Homo sapiens

<400> 284
agatgaaaca aagcagtgaa accctacagt ttaaatgatg tcacttttag tgctgttggc 60

<210> 285
<211> 60
<212> DNA
<213> Homo sapiens

<400> 285
gtctgtctgt acagttactc atgtcattgt aatgatttca ctcctaactg tgacattttt 60

<210> 286
<211> 60
<212> DNA
<213> Homo sapiens

<400> 286
taatgttctc tatttcttct tgattattta ttagctttgg atgttaaaaa ttagccaaaa 60

<210> 287
<211> 60
<212> DNA
<213> Homo sapiens

<400> 287
ttattaatct taatattttg tttggaaaag catgttataa tataatgttt tcacaaaaaa 60

<210> 288
<211> 60
<212> DNA
<213> Homo sapiens

<400> 288
tcaataagta attatcaagt ttggaaggaa aggttgaaat gactccgttt ccttataagc 60

<210> 289
<211> 60
<212> DNA
<213> Homo sapiens

<400> 289
ctcctacaag gagatatggc tggaccaaaa taaaatgaca tgaaattgca aaaatgaaaa 60

<210> 290
<211> 60

<212> DNA
<213> Homo sapiens

<400> 290
tcaaagtcta aacatcgggg gaccaagcac agcagatgtg gaaaggaagg aataaaaatg 60

<210> 291
<211> 60
<212> DNA
<213> Homo sapiens

<400> 291
ttctgtatat cttaaatcat ttctagatta cttataatac acaatgcatt gtaaattctg 60

<210> 292
<211> 60
<212> DNA
<213> Homo sapiens

<400> 292
taataactat attcagatta tactttgttt cttgaaaaaa ttacagattt tttgaaaagc 60

<210> 293
<211> 60
<212> DNA
<213> Homo sapiens

<400> 293
cgtctctact aaaaatacaa aaaattagct gggcgtggtg gcgggcgcct gtggtcccag 60

<210> 294
<211> 60
<212> DNA
<213> Homo sapiens

<400> 294
tggagccggt caacttttg ctcatggcta gtgaaataaa gttgtttgag tactaaaaaa 60

## Description

1. BACKGROUND OF THE INVENTION

The identification of patient subpopulations most likely to respond to therapy is a central goal of modem molecular medicine. This notion is particularly important for cancer due to the large number of approved and experimental therapies (Rothenberg et al., 2003, Nat. Rev. Cancer 3:303-309), low response rates to many current treatments, and clinical importance of using the optimal therapy in the first treatment cycle (Dracopoli, 2005, Curr. Mol. Med. 5:103-110). In addition, the narrow therapeutic index and severe toxicity profiles associated with currently marketed cytotoxics results in a pressing need for accurate response prediction. Although recent studies have identified gene expression signatures associated with response to cytotoxic chemotherapies (Folgueria et al., 2005, Clin. Cancer Res. 11:7434-7443; Ayers et al., 2004, 22:2284-2293; Chang et al., 2003, Lancet 362:362-369; Rouzier et al., 2005, Proc. Natl. Acad. Sci. USA 102: 8315-8320), these examples (and others from the literature) remain unvalidated and have not yet had a major effect on clinical practice. In addition to technical issues, such as lack of a standard technology platform and difficulties surrounding the collection of clinical samples, the myriad of cellular processes affected by cytotoxic chemotherapies may hinder the identification of practical and robust gene expression predictors of response to these agents. One exception may be the recent finding by microarray that low mRNA expression of the microtubule-associate protein Tau is predictive of improved response to paclitaxel (Rouzier et al., *supra*).

To improve on the limitations of cytotoxic chemotherapies, current approaches to drug design in oncology are aimed at modulating specific cell signaling pathways important for tumor growth and survival (Hahn and Weinberg, 2002, Nat. Rev. Cancer 2:331-341; Hanahan and Weinberg, 2000, Cell 100:57-70; Trosko et al., 2004, Ann. N.Y. Acad. Sci. 1028:192-201). In cancer cells, these pathways become deregulated resulting in aberrant signaling, inhibition of apoptosis, increased metastasis, and increased cell proliferation (reviewed in Adjei and Hildalgo, 2005, J. Clin. Oncol. 23:5386-5403). Although normal cells integrate multiple signaling pathways for controlled growth and proliferation, tumors seem to be heavily reliant on activation of one or two pathways ("oncogene activation"). In addition to the well-known dependence of chronic myelogenous leukemia on BCR-ABL, studies of the epidermal growth factor receptor and MYC pathways showed that inactivation of a single critical oncogene can induce cell death or differentiation into cells with a normal phenotype (Lynch et al,. 2004, N.Engl. J. Med. 350: 2129-2139; Paez et al., 2004, Science 304:1497-1500; Weinstein, 2002, Science 297:63-64; Jain et al., 2002, Science 297:102-104; Gorre et al., 2001, Science 293:876-880; Druker et al., 2001, N. Engl. J. Med. 344:1031-1037). The components of these aberrant signaling pathways represent attractive selective targets for new anticancer therapies. In addition, responder identification for target therapies may be more achievable than for cytotoxics, as it seems logical that patients with tumors that are "driven" by a particular pathway will respond to therapeutics targeting components of that pathway. Therefore, it is crucial that we develop methods to identify which pathways are active in which tumors and use this information to guide therapeutic decisions. One way to enable this is to identify gene expression profiles that are indicative of pathway activation status.

Current methods for assessing pathway activation in tumors involve the measurement of drug targets, known oncogenes, or known tumor suppressors. However, one pathway can be activated at multiple points, so it is not always feasible to assess pathway activation by evaluating known cancer-associated genes (Downward, 2006, Nature 439:274-275). RAS and its effectors regulate cell growth, differentiation, motility, survival, and death (Downward, 2002, Nat. Rev. Cancer 3:11-22). RAS proteins are members of a large superfamily of GTP binding proteins that serve as a molecular switch, converting signals from the cell membrane to the nucleus. Some distinct members of the RAS family include HRas, KRas, MRas, NRas, and RRas (Adjei, 2001, J. Nat. Cancer Instit. 93:1062-1073). Deregulation of RAS pathways by mutational activation or by receptor-mediated activation of RAS contribute to human malignancies (Downward, supra). Approximately one third of all human cancers, including cancers of the pancreas, colon, and lung, express a constitutively active RAS (Downward, supra). Aberrant RAS signaling in tumors may also be caused by loss of GTPase activating proteins (GAPs), such as neurofibromin, encoded by *NF1;* growth factor receptor activation, such as EGFR and ERBB2, or mutation or amplification of RAS effectors, such as BRAF mutation, PTEN loss, AKT2 amplification, or PI3K amplification (Downward, supra). This pathway can be activated by multiple growth factors through receptor tyrosine kinases and has effects on multiple processes, including cell growth and survival, metastatic competence, and therapy resistance (Downward, supra). Therefore, inhibition of RAS or its upstream activators or downstream effectors may be a promising pharmacologic strategy for cancer therapy (Cox and Der, 2002 Curr. Opin. Pharmacol. 2:388-93; Blum and Kloog, 2005, Drug Resist. Updat. 8:369-80; Downward, supra; Dancey, 2002, Curr. Pharm. Des. 8:2259-2267). RAS pathway activation is also an indicator of resistance to therapeutic agents targeting EGFR and PI3K (Massarelli et al., 2007, Clin. Cancer Res. 13:2890-2896; Raponi et al., 2008, Curr. Opin. Pharmacology 8:413-418; Ihle et al., 2009, Cancer Res. 69:143-150). Accordingly, accurate determination of RAS pathway activation will be critical for the identification of potential responders to these emerging novel therapeutics.

However, the RAS pathway can be activated by aberrations at multiple points, and assessing pathway activity may not be straightforward (Downward, supra). For example, RAS itself (K-RAS, N-RAS, H-RAS) is frequently mutated in cancers. RAS mutations are common in pancreatic, lungadenocarcinoma, and colorectal cancers (Downward, supra). The RAS pathway can also be activated by loss of GAPs, such as neurofibromin (Weiss et al., 1999, Am. J. Med. Genet. 89:14-22); growth factor receptor activation (Mendelsohn and Baselga, 2000, Oncogene 19:6550-6565); and mutation or amplification of RAS pathway effectors (Bellacosa, 1995, Intl. J. Cancer 64:280-285; Simpson and Parsons, 2001, Exp. Cell Res. 264:29-41). Although RAS pathway activation can be assessed by sequence analysis (Bos, 1989, Cancer Res. 49:4682-4689), this may not be the optimal way to measure pathway activation. Sequence analysis of RAS misses other pathway activators and is not quantitative. In addition, oncogenic pathways are complex, so important pathway mediators may be missed by testing only a few well-characterized pathway components.

Examples like this suggest that a gene expression signature-based readout of pathway activation may be more appropriate than relying on a single indicator of pathway activity, as the same signature of gene expression may be elicited by activation of multiple components of the pathway. In addition, by integrating expression data from multiple genes, a quantitative assessment of pathway activity may be possible. In addition to using gene expression signatures for tumor classification by assessing pathway activation status, gene expression signatures for pathway activation may also be used as pharmacodynamic biomarkers, i.e., monitoring pathway inhibition in patient tumors or peripheral tissues post-treatment; as response prediction biomarkers, i.e., prospectively identifying patients harboring tumors that have high levels of a particular pathway activity before treating the patients with inhibitors targeting the pathway or identifying patients harboring tumors that have high levels of a particular pathway activity and are therefore likely to be resistant to particular inhibitors; and as early efficacy biomarkers, i.e., an early readout of efficacy.

## 2. BRIEF DESCRIPTION OF THE DRAWINGS

The patent or application file contains at least one drawing executed in color. Copies of this patent or patent application publication with color drawing(s) will be provided by the Office upon request and payment of the necessary fee.

FIGURE 1 shows a summary of RAS pathway activation and gene expression signature. RAS is activated by growth factors through receptor tyrosine kinases. The autophosphorylated receptor binds to the SH2 domain of GRB2. Through its SH3 domain, GRB2 is bound to SOS, so activation of the receptor tyrosine kinase results in recruitment of SOS to the plasma membrane, where RAS is also localized as a result of farnesylation. The increased proximity of SOS to RAS results in increased nucleotide exchange on RAS, with GDP being replaced with GTP. GTP-bound RAS is able to bind and activate several families of effector enzymes (such as the RAF, PI3K, RALGDS, and PLCε pathways). This signaling cascade affects multiple cellular processes and results in a gene expression "signature" of pathway activity. Activation of this pathway has been implicated in many cancers, and this activation can occur via aberrations in multiple pathway components. Because activation of various pathway components may lead to the same gene expression profile, a signature of pathway activation is likely to provide more accurate information than the assessment of a single known oncogene or tumor suppressor.

FIGURE 2 shows that the RAS pathway signature is significantly coherent in panel of breast cancer cell lines. A) Coherency test demonstrates that the "up" and "down" arms of the RAS pathway signature significantly correlates within one arm and anticorrelate between the opposing arms. B) Heatmap showing that "up" and "down" arms of RAS signature cluster apart in breast cancer cell line panel. C) Mean of the genes in the "up" arm is plotted against the mean of the genes in the "down" arm for each breast cell line. The "up" and "down" scores significantly anticorrelate in this dataset. D) Genes remaining after refinement of the signature are shown in the heatmap.

FIGURE 3 show that a different RAS pathway signature identified by Nevin et al., (Nevin's signature) is not coherent in the panel of breast cancer cell lines. A) Coherency test results: genes in the "up" and "down" arms of the Nevins signature do not significantly correlate within one arm and do not anticorrelate between the arms. B) The "up" and "down" arms do not cluster apart in the heatmap. C) Graph showing the mean of the genes in the "up" arm plotted against the mean of the genes in the "down" arm. The "up" and "down" scores correlate, rather than anticorrelate.

FIGURE 4 shows that the inventive RAS pathway signature is consistent with other RAS signatures across four cell line panels. Pair-wise scatter plots for RAS signatures are shown in breast (A); colon (B); lung (C); and lymphoma (D). Significance of Pearson, Kendal, and Spearman correlations are shown for every plot.

FIGURE 5 shows that the RAS pathway signature is predictive of RAS and BRAF mutation status in Colon, Lung, and Breast Cancer Cell Lines. Bar graphs show the signature scores for the RAS pathway in colon (A); lung (B); and breast (C) cancer cell line sets. Each graph is split into two parts according to RAS mutational status. The sorted RAS pathway signature scores for RAS wildtype cell lines are shown on the left, and the sorted RAS pathway signature scores for the mutant cell lines are shown on the right. Prediction of high RAS pathway signature score in non-mutant cell lines may be due to other means of RAS pathway upregulation.

FIGURE 6 shows that the RAS pathway signature is predictive of RAS mutations human NSCLC tumors.

FIGURE 7 shows that the RAS pathway signature is coherent and consistent with other RAS signatures, developed by others, in formalin fixed, paraffin embedded (FFPE) samples obtained from lung, ovarian, and breast tumors.

FIGURES 7A, 7C, and 7E, show the coherency of RAS pathway signature in lung, ovarian and breast tumors, respectively. FIGURES 7B, 7D, and 7F, show pairwise correlations between the inventive RAS pathway signature ("ours") and other RAS signatures in lung, ovarian, and breast tumors, respectively.

FIGURE 8 shows the distribution of RAS pathway signature scores in subtypes of ovarian tumor samples. Our RAS pathway signature score was calculated in the Mayo Ovarian FFPE tumor dataset. The dataset was stratified by histological type of tumor. The box plot shows the distribution of the RAS pathway signatures cores among subtypes.

FIGURE 9 shows that the inventive RAS pathway signature score is high in adenocarcinomas and low in squamous non-small cell lung carcinoma (NSCLC). Our RAS pathway signature score was calculated in a dataset of fresh frozen lung tumor samples. The box plot shows the distributions of RAS scores for adenocarcinomas and squamous cell carcinomas. The difference between these two groups is significant at 0.05 level by both t-test and wilcoxon rank sum test. Virtually all squamous cell carcinomas had negative RAS pathway signature scores, whereas 70% of adenocarcinomas had positive RAS pathway signature scores.

FIGURE 10 shows a pie-chart of GFS/RAS expression in triple negative tumors. Only about half of "triple negative" breast tumors have high RAS scores. RAS signature was scored in "triple negative" and Her2+ fresh frozen breast tumors.

FIGURE 11 shows the distribution of RAS pathway signature scores across eleven tumor types.

FIGURE 12 shows that K-RAS siRNA knockdown suggests that RAS pathway signature score is more predictive of RAS dependence than K-ras mutational status.

FIGURE 13 show that a high baseline RAS signature score predicts resistance to AKT inhibitor (AKTi) MK-6673, in a breast cancer cell line. Resistant cell lines are defined as those with percent inhibition <60% and sensitive as those with percent inhibition >60% (p-value by Fisher Exact test <0.002).

FIGURE 14 shows the generation of breast cancer cell lines with acquired resistance to AKTi MK-2206. Top left panel: to generate cell liens with acquired AKTi resistance, we cultured two PTEN mutant breast cancer lines in increasing concentrations of MK-2206 for a period of ~7 months initially at a low concentration (20nM) of inhibitor. To control for the possibility that resistance could be acquired by genetic drift over multiple passages in culture, we also grew control flasks of each breast cancer cell line in the presence of DMSO vehicle for the course of the experiment. Inhibitor concentration was increased by 5-10 nM when the growth rate of the cells reached the level of vehicle controls. Top right panel: Resulting cell populations that could be grown in high concentrations of MK-2206 (>2μM) were removed from drug and then tested for resistance to MK-2206 in growth assay. Parental (triangles) and resistance (squares) ZR-75-1 cells were treated with MK-2206 at the indicated concentrations and cell viability was measured 72 hours after treatment by Alamar Blue assay. The percentage of viable cells is shown relative to untreated controls. Similar data were obtained for CAMA-1 cells. Bottom panel: Analysis of RAS pathway signature in CAMA-1R and ZR-75-1R cells. To assess whether deregulation of the RAS pathway could account for the resistance phenotype, the AKTi resistance signatures for each cell line were compared to the RAS pathway signature. The table in the bottom panel shows that the RAS pathway is significantly modified in cell lines with acquired AKT resistance.

FIGURE 15 shows that RAS signature score correlates with MEK inhibitor (MEKi) sensitivity in chronic beryllium disease (CBD) lung samples.

FIGURE 16 shows that RAS signature score correlates with MEKi sensitivity in CBD-Lung cell lines having mutant RAS.

FIGURE 17 shows that RAS signature score correlates with MEKi sensitivity in CBD-Lung cell lines having wild-type RAS.

FIGURE 18 shows that the RAS pathway signature score is down regulated by MEKi AZD6244 in vivo at 4 hours post-dose but not at 24 hours post-dose, consistent with AZD's short half-life in vivo.

FIGURE 19 shows that the blood concentration of AZD6244 in mice peaks about 2 hours post-dose and decreases rapidly thereafter.

## 3. DETAILED DESCRIPTION OF THE INVENTION

The scope of the inventions is determined by the appended claims. This description is by way of several exemplary illustrations, of varying detail and specificity. Other features and advantages of these embodiments are apparent from the additional descriptions provided herein, including the different examples. The provided examples illustrate different components and methodology useful in practicing various embodiments of the invention.

### 3.1 Introduction

Various embodiments of the disclosure relate to sets of genetic biomarkers whose expression patterns correlate with an important characteristic of cancer cells, i.e., deregulation of the RAS signaling pathway. In some embodiments, these sets of biomarkers may be split into two opposing "arms" - the "up" arm (Table 2a), which are the genes that are upregulated, and the "down" arm (Table 2b), which are the genes that are downregulated, as signaling through the RAS pathway increases. More specifically, some aspects of the disclosure provide for sets of genetic biomarkers whose expression correlates with the regulation status of the RAS signaling pathway of a tumor cell sample of a patient, and which can be used to classify tumors with deregulated RAS signaling pathway from tumors with regulated RAS signaling pathway. RAS signaling pathway regulation status is a useful indicator of the likelihood that a patient will respond to certain therapies, such as inhibitors of the RAS signaling pathway, or likelihood that a patient will be resistant to certain therapies, such as EGFR or PI3K pathway inhibitors. Such therapies include, but are not limited to: PI3K inhibitors LY249002, wortmannin, and PX-866; AKT inhibitors 17-AAG, PX316, miltefosine, and perifosin; EGFR inhibitors ZD 1839; IMC-C225; ERBB2 inhibitor Herceptin; RAS inhibitors ISIS 2503 and farnesyl transferase inhibitor R115777, L731735, SCH 66336, and BMS214662; Raf inhibitors ISIS 5132 and BAY43-9006; MEK inhibitors PD184322 and CI-1040 (reviewed in Henson and Gibson 2006, Cellular Signalling 18:2089-2097; Hennessy et al., 2005, Nat. Rev. Drug Disc. 4:988-1004; reviewed in Dancey, 2002, Curr. Pharm. Des. 8:2259-2267; Sebolt-Leopold et al., 1999, Nat. Med 5:810-816; Downward, 2003, Nat. Rev. Cancer 3:11-22). In one aspect of the disclosure, methods are provided for use of these biomarkers to distinguish between patient groups that will likely respond to inhibitors of the RAS signaling pathway (predicted responders) and patient groups that will not likely respond to inhibitors of the RAS pathway signaling pathway (predicted non-responders) and to determine general courses of treatment. In another aspect of the invention, methods are provided for use of these biomarkers to distinguish between patient groups that will not likely respond to inhibitors of the PI3K signaling pathway or EGFR inhibitors (predicted non-responders) and patient groups that will likely respond to inhibitors of the PI3K signaling pathway or EGFR inhibitors. Another aspect of the disclosure relates to biomarkers whose expression correlates with a pharmacodynamic effect of a therapeutic agent on the RAS signaling pathway in subject with cancer. In yet other aspects of the disclosure, methods are provided for use of these biomarkers to measure the pharmacodynamic effect of a therapeutic agent on the RAS signaling pathway in a subject with cancer and the use of these biomarkers to rank the efficacy of therapeutic agents to modulate the RAS signaling pathway. Microarrays comprising these biomarkers are also provided, as well as methods of contructing such microarrays. Each of the biomarkers correspond to a gene in the human genome, i.e., such biomarker is identifiable as all or a portion of a gene. Finally, because each of the above biomarkers correlate with cancer-related conditions, the biomarkers, or the proteins they encode, are likely to be targets for drugs against cancer.

3.2 Definitions

Unless defined otherwise, all technical and scientific terms used herein have the same meaning as commonly understood to one of ordinary skill in the art to which this invention belongs.

As used herein, oligonucleotide sequences that are complementary to one or more of the genes described herein, refers to oligonucleotides that are capable of hybridizing under stringent conditions to at least part of the nucleotide sequence of said genes. Such hybridizable oligonucleotides will typically exhibit at least about 75% sequence identity at the nucleotide level to said genes, preferably about 80% or 85% sequence identity or more preferably about 90% or 95% or more sequence identity to said genes.

"Bind(s) substantially" refers to complementary hybridization between a probe nucleic acid and a target nucleic acid and embraces minor mismatches that can be accommodated by reducing the stringency of the hybridization media to achieve the desired detection of the target polynucleotide sequence.

The phrase "hybridizing specifically to" refers to the binding, duplexing or hybridizing of a molecule substantially to or only to a particular nucleotide sequence or sequences under stringent conditions when that sequence is present in a complex mixture (e.g., total cellular) DNA or RNA.

"Biomarker" means any gene, protein, or an EST derived from that gene, the expression or level of which changes between certain conditions. Where the expression of the gene correlates with a certain condition, the gene is a biomarker for that condition.

"Biomarker-derived polynucleotides" means the RNA transcribed from a biomarker gene, any cDNA or cRNA produced therefrom, and any nucleic acid derived therefrom, such as synthetic nucleic acid having a sequence derived from the gene corresponding to the biomarker gene.

A gene marker is "informative" for a condition, phenotype, genotype or clinical characteristic if the expression of the gene marker is correlated or anti-correlated with the condition, phenotype, genotype or clinical characteristic to a greater degree than would be expected by chance.

As used herein, the term "gene" has its meaning as understood in the art. However, it will be appreciated by those of ordinary skill in the art that the term "gene" may include gene regulatory sequences (e.g., promoters, enhancers, etc.) and/or intron sequences. It will further be appreciated that definitions of gene include references to nucleic acids that do not encode proteins but rather encode functional RNA molecules such as tRNAs. For clarity, the term gene generally refers to a portion of a nucleic acid that encodes a protein; the term may optionally encompass regulatory sequences. This definition is not intended to exclude application of the term "gene" to non-protein coding expression units but rather to clarify that, in most cases, the term as used in this document refers to a protein coding nucleic acid. In some cases, the gene includes regulatory sequences involved in transcription, or message production or composition. In other embodiments, the gene comprises transcribed sequences that encode for a protein, polypeptide or peptide. In keeping with the terminology described herein, an "isolated gene" may comprise transcribed nucleic acid(s), regulatory sequences, coding sequences, or the like, isolated substantially away from other such sequences, such as other naturally occurring genes, regulatory sequences, polypeptide or peptide encoding sequences, etc. In this respect, the term "gene" is used for simplicity to refer to a nucleic acid comprising a nucleotide sequence that is transcribed, and the complement thereof. In particular embodiments, the transcribed nucleotide sequence comprises at least one functional protein, polypeptide and/or peptide encoding unit. As will be understood by those in the art, this functional term "gene" includes both genomic sequences, RNA or cDNA sequences, or smaller engineered nucleic acid segments, including nucleic acid segments of a non-transcribed part of a gene, including but not limited to the non-transcribed promoter or enhancer regions of a gene. Smaller engineered gene nucleic acid segments may express, or may be adapted to express using nucleic acid manipulation technology, proteins, polypeptides, domains, peptides, fusion proteins, mutants and/or such like. The sequences which are located 5' of the coding region and which are present on the mRNA are referred to as 5' untranslated sequences ("5'UTR"). The sequences which are located 3' or downstream of the coding region and which are present on the mRNA are referred to as 3' untranslated sequences, or ("3'UTR").

"Signature" refers to the differential expression pattern. It could be expressed as the number of individual unique probes whose expression is detected when a cRNA product is used in microarray analysis. A signature may be exemplified by a particular set of biomarkers.

A "similarity value" is a number that represents the degree of similarity between two things being compared. For example, a similarity value may be a number that indicates the overall similarity between a cell sample expression profile using specific phenotype-related biomarkers and a control specific to that template (for instance, the similarity to a "deregulated RAS signaling pathway" template, where the phenotype is deregulated RAS signaling pathway status). The similarity value may be expressed as a similarity metric, such as a correlation coefficient, or may simply be expressed as the expression level difference, or the aggregate of the expression level differences, between a cell sample expression profile and a baseline template.

As used herein, the terms "measuring expression levels," "obtaining expression level," and "detecting an expression level" and the like, includes methods that quantify a gene expression level of, for example, a transcript of a gene, or a protein encoded by a gene, as well as methods that determine whether a gene of interest is expressed at all. Thus, an assay which provides a "yes" or "no" result without necessarily providing quantification, of an amount of expression is an assay that "measures expression" as that term is used herein. Alternatively, a measured or obtained expression level may be expressed as any quantitative value, for example, a fold-change in expression, up or down, relative to a control gene or relative to the same gene in another sample, or a log ratio of expression, or any visual representation thereof, such as, for example, a "heatmap" where a color intensity is representative of the amount of gene expression detected. The genes identified as being differentially expressed in tumor cells having RAS signaling pathway deregulation may be used in a variety of nucleic acid or protein detection assays to detect or quantify the expression level of a gene or multiple genes in a given sample. Exemplary methods for detecting the level of expression of a gene include, but are not limited to, Northern blotting, dot or slot blots, reporter gene matrix (see for example, US 5,569,588) nuclease protection, RT-PCR, microarray profiling, differential display, 2D gel electrophoresis, SELDI-TOF, ICAT, enzyme assay, antibody assay, and the like.

A "patient" can mean either a human or non-human animal, preferably a mammal.

As used herein, "subject", as refers to an organism or to a cell sample, tissue sample or organ sample derived therefrom, including, for example, cultured cell lines, biopsy, blood sample, or fluid sample containing a cell. In many instances, the subject or sample derived therefrom, comprises a plurality of cell types. In one embodiment, the sample includes, for example, a mixture of tumor and normal cells. In one embodiment, the sample comprises at least 10%, 15%, 20%, et seq., 90%, or 95% tumor cells. The organism may be an animal, including but not limited to, an animal, such as a cow, a pig, a mouse, a rat, a chicken, a cat, a dog, etc., and is usually a mammal, such as a human.

As used herein, the term "pathway" is intended to mean a set of system components involved in two or more sequential molecular interactions that result in the production of a product or activity. A pathway can produce a variety of products or activities that can include, for example, intermolecular interactions, changes in expression of a nucleic acid or polypeptide, the formation or dissociation of a complex between two or more molecules, accumulation or destruction of a metabolic product, activation or deactivation of an enzyme or binding activity. Thus, the term "pathway" includes a variety of pathway types, such as, for example, a biochemical pathway, a gene expression pathway, and a regulatory pathway. Similarly, a pathway can include a combination of these exemplary pathway types.

"RAS signaling pathway" or "RAS pathway" is initiated by growth factors through receptor tyrosine kinases. The autophosphorylated receptor binds to the SH2 domain of GRB2. Through its SH3 domain, GRB2 is bound to SOS, so activation of the receptor tyrosine kinase results in recruitment of SOS to the plasma membrane, where RAS is also localized as a result of farnesylation. The increased proximity of SOS to RAS results in increased nucleotide exchange on RAS, with GDP being replaced with GTP. GTP-bound RAS is able to bind and activate several families of effector enzymes (such as the RAF, PI3K, RALGDS, and PLCε pathways)(reviewed in Downward, 2003, Nat. Rev. Cancer 3:11-22)(See Figure 1). This signaling cascade affects multiple cellular processes, such as cell-cycle progression, transcription, survival, cytoskeletal signals, translation, vesicle transport, and calcium signaling, and results in a gene expression "signature" of pathway activity.

Table 1: Representative RAS pathway genes

| Gene Name | Transcript ID |
|---|---|
| CDH13 | NM_001257 |
| RASGRP1 | NM_005739 |
| FAM13A1 | NM_014883 |
| G3BP1 | NM_005754 |
| RASGRP2 | NM_153819 |
| CNKSR1 | NM_006314 |
| NET1 | NM_001047160 |
| PAK4 | NM_005884 |
| DLC1 | NM_182643 |
| CDC42EP2 | NM_006779 |
| VAV3 | NM_006113 |
| ARFGEF2 | NM_006420 |
| RABAC1 | NM_006423 |
| GNA13 | NM_006572 |
| CFL1 | NM_005507 |
| GRAP | NM_006613 |
| CYSLTR1 | NM_006639 |
| FRS3 | NM_006653 |
| UTS2 | NM_021995 |
| RALBP1 | NM_006788 |
| ADAP1 | NM_006869 |
| CDC42EP1 | NM_007061 |
| RASSF1 | NM_007182 |
| NISCH | NM_007184 |
| AKAP13 | NM_006738 |
| CHRM4 | NM_000741 |
| GRIN1 | NM_052899 |
| FMNL2 | NM_052905 |
| SNX26 | NM_052948 |
| EVI5L | NM_145245 |
| RASGRP4 | NM_170604 |
| SLC26A8 | NM_052961 |
| RAB39B | NM_171998 |

(continued)

| Gene Name | Transcript ID |
|---|---|
| ARAP2 | NM_015230 |
| ARAP1 | NM_001040118 |
| AGAR2 | NM_014770 |
| AGAP1 | NM_001037131 |
| AGAP3 | NM_031946 |
| TAGAP | NM_054114 |
| FGD4 | NM_139241 |
| CCR1 | NM_001295 |
| CNN1 | NM_001299 |
| IQGAP3 | NM_178229 |
| TBC1D20 | NM_144628 |
| GAB4 | NM_001037814 |
| ABRA | NM_139166 |
| CRKL | NM_005207 |
| ADORA3 | NM_001081976 |
| MAPK14 | NM_001315 |
| SESN3 | NM_144665 |
| CSK | NM_004383 |
| RTN4RL1 | NM_178568 |
| CDC42EP5 | NM_145057 |
| DIRAS1 | NM_145173 |
| ADRA2A | NM_000681 |
| CTNND2 | NM_001332 |
| ROPN1B | NM_001012337 |
| FGD5 | NM_152536 |
| SH3D19 | NM_001009555 |
| ADRB2 | NM_000024 |
| AMOT | NM_133265 |
| ADRB3 | NM_000025 |
| RP13-102H20.1 | NM_144967 |
| DAB2 | NM_001343 |
| SPRED1 | NM_152594 |
| DENND2C | NM_198459 |
| RHOV | NM_133639 |
| DMPK | NM_004409 |
| DOCK2 | NM_004946 |
| DOK1 | NM_001381 |
| DYRK1A | NM_101395 |

(continued)

| Gene Name | Transcript ID |
|---|---|
| ECT2 | NM_018098 |
| ABCA1 | NM_005502 |
| EDN1 | NM_001955 |
| EFNB1 | NM_004429 |
| EFNB3 | NM_001406 |
| SPRED2 | NM_181784 |
| MUC20 | NM_152673 |
| ARHGAP27 | NM_199282 |
| EPHB2 | NM_004442 |
| EPHB6 | NM_004445 |
| EPO | NM_000799 |
| F2R | NM_001992 |
| F7 | NM_019616 |
| RTKN2 | NM_145307 |
| RASGEF1A | NM_145313 |
| SPATA13 | NM_153023 |
| FGD2 | NM_173558 |
| FGD1 | NM_004463 |
| FGF2 | NM_002006 |
| RRAS2 | NM_012250 |
| MRAS | NM_012219 |
| RASA3 | NM_007368 |
| RHOBTB3 | NM_014899 |
| CNKSR2 | NM_014927 |
| DAAM1 | NM_014992 |
| FOXJ1 | NM_001454 |
| RGL1 | NM_015149 |
| FLT1 | NM_002019 |
| FLT3 | NM_004119 |
| ARHGEF9 | NM_015185 |
| MCF2L | NM_001112732 |
| FBXW11 | NM_033645 |
| ARHGEF12 | NM_015313 |
| PPP1 R13B | NM_015316 |
| ARHGEF18 | NM_015318 |
| SRGAP2 | NM_015326 |
| FNTA | NM_001018677 |
| DAAM2 | NM_015345 |

(continued)

| Gene Name | Transcript ID |
|---|---|
| CDC42EP4 | NM_012121 |
| SH3BP1 | NM_018957 |
| NUP62 | NM_012346 |
| PLXNB2 | NM_012401 |
| SMPX | NM_014332 |
| ARHGAP30 | NM_181720 |
| SHC2 | NM_012435 |
| RASGRP3 | NM_170672 |
| FBXO8 | NM_012180 |
| ARFGAP3 | NM_014570 |
| GFRA1 | NM_005264 |
| LAT | NM_001014989 |
| DENND2A | NM_015689 |
| RND1 | NM_014470 |
| SGSM3 | NM_015705 |
| GNA11 | NM_002067 |
| GNA12 | NM_007353 |
| GNA15 | NM_002068 |
| GNB1 | NM_002074 |
| GPR4 | NM_005282 |
| RASSF3 | NM_178169 |
| KSR2 | NM_173598 |
| GRB2 | NM_203506 |
| GIT1 | NM_014030 |
| DBNL | NM_014063 |
| ABR | NM_021962 |
| GRPR | NM_005314 |
| GPR132 | NM_013345 |
| RHOD | NM_014578 |
| HGF | NM_000601 |
| TAX1BP3 | NM_014604 |
| HRAS | NM_176795 |
| AGFG1 | NM_004504 |
| APOA1 | NM_000039 |
| HTR2C | NM_000868 |
| C20orf95 | ENST00000243967 |
| APOC3 | NM_000040 |
| IGF1 | NM_000618 |

(continued)

| Gene Name | Transcript ID |
|---|---|
| APOE | NM_000041 |
| RTN4RL2 | NM_178570 |
| CXCL10 | NM_001565 |
| INPPL1 | NM_001567 |
| AQP9 | NM_020980 |
| KCNH2 | NM_000238 |
| KISS1 | NM_002256 |
| ARF6 | NM_001663 |
| KRAS | NM_004985 |
| RHOA | NM_001664 |
| RASL11A | NM_206827 |
| RHOB | NM_004040 |
| RND3 | NM_005168 |
| RHOG | NM_001665 |
| ARHGAP1 | NM_004308 |
| STMN1 | NM_203399 |
| ARHGAP4 | NM_001666 |
| LCK | NM_005356 |
| ARHGAP5 | NM_001173 |
| ARHGAP6 | NM_001174 |
| LGALS3 | NM_002306 |
| ARHGDIA | NM_004309 |
| LGALS8 | NM_201545 |
| ARHGDIB | NM_001175 |
| ARHGDIG | NM_001176 |
| LIMK1 | NM_002314 |
| LIMK2 | NM_005569 |
| RHOH | NM_004310 |
| SPRED3 | NM_001042522 |
| SHC4 | NM_203349 |
| LTK | NM_206961 |
| MAP1LC3C | NM_001004343 |
| MYO9B | NM_004145 |
| MYOC | NM_000261 |
| NEK3 | NM_002498 |
| NF1 | NM_000267 |
| NGF | NM_002506 |
| NOTCH2 | NM_024408 |

(continued)

| Gene Name | Transcript ID |
|---|---|
| NRAS | NM_002524 |
| NTRK1 | NM_001007792 |
| NTSR1 | NM_002531 |
| OPHN1 | NM_002547 |
| P2RX7 | NM_002562 |
| P2RY2 | NM_002564 |
| PAFAH1B1 | NM_000430 |
| PAK1 | NM_002576 |
| DEF6 | NM_022047 |
| PAK3 | NM_002578 |
| ARHGEF4 | NM_015320 |
| ARHGEF3 | NM_019555 |
| PARD6A | NM_001037281 |
| STMN3 | NM_015894 |
| ZDHHC9 | NM_016032 |
| PLCE1 | NM_016341 |
| TBC1D7 | NM_016495 |
| PTPLAD1 | NM_016395 |
| ENPP2 | NM_006209 |
| RAFGEF6 | NM_016340 |
| SERPINF1 | NM_002615 |
| PIN1 | NM_006221 |
| PITX1 | NM_002653 |
| PKD3 | NM_005813 |
| SHC3 | NM_016848 |
| PLD1 | NM_002662 |
| PLEK | NM_002664 |
| PLXNB1 | NM_002673 |
| RIN2 | NM_018993 |
| RHOF | NM_019034 |
| WDR44 | NM_019045 |
| DIRAS2 | NM_017594 |
| RASIP1 | NM_017805 |
| RALGPS2 | NM_152663 |
| ARHGAP17 | NM_001006634 |
| FAIM | NM_001033031 |
| PLEKHG6 | NM_018173 |
| SYNJ2BP | NM_018373 |

(continued)

| Gene Name | Transcript ID |
|---|---|
| ARFGAP1 | NM_018209 |
| FGD6 | NM_018351 |
| ARHGAP15 | NM_018460 |
| C3orf10 | NM_018462 |
| MAPK1 | NM_002745 |
| MAPK3 | NM_002746 |
| MAPK11 | NM_002751 |
| MAPK13 | NM_002754 |
| MAP2K1 | NM_002755 |
| MAP2K2 | NM_030662 |
| PRLR | No_000949 |
| PARD3 | NM_019619 |
| LTB4R2 | NM_019839 |
| PSD | NM_002779 |
| GRIPAP1 | NM_020137 |
| CIAPIN1 | NM_020313 |
| RAB25 | NM_020387 |
| RGL3 | N_001035223 |
| RHOJ | NM_020663 |
| SRGAP1 | N_020762 |
| PTK6 | NM_005975 |
| ARHGAP20 | NM_020809 |
| PREX1 | NM_020820 |
| ARHGAP21 | NM_020824 |
| RANBP10 | NM_020850 |
| ARHGAP23 | ENST00000300901 |
| ALS2 | NM_020919 |
| RAP2C | NM_021183 |
| PTPRK | NM_002844 |
| RHOU | NM_021205 |
| ARHGAP22 | NM_021226 |
| RGL2 | NM_004761 |
| RAC1 | NM_006908 |
| RAC3 | NM_005052 |
| RAF1 | NM_002880 |
| RALA | NM_005402 |
| RALB | NM_002881 |
| RALGDS | NM_001042368 |

(continued)

| Gene Name | Transcript ID |
|---|---|
| RAP1A | NM_001010935 |
| RAP2A | NM_021033 |
| RASA2 | NM_006506 |
| RASGRF1 | NM_002891 |
| RASGRF2 | NM_006909 |
| BCL6 | NM_001706 |
| ROCK1 | NM_005406 |
| BCR | NM_004327 |
| RRAS | NM_006270 |
| RREB1 | NM_001003699 |
| RTKN | NM_001015055 |
| RSU1 | NM_012425 |
| MAP12 | NM_002969 |
| SDCBP | NM_005625 |
| SEMA4A | NM_022367 |
| ARHGAP9 | NM_032496 |
| ARAP3 | NM_022481 |
| ITSN1 | NM_003024 |
| SH3GL1 | NM_003025 |
| SHC1 | NM_003029 |
| SMAP2 | NM_022733 |
| EPS8L2 | NM_022772 |
| PLEKHG2 | NM_022835 |
| SLC26A10 | NM_133489 |
| SOS1 | NM_005633 |
| SOS2 | NM_006939 |
| SRC | NM_005417 |
| ST5 | NM_005418 |
| TAC1 | NM_013998 |
| TACT1 | NM_001058 |
| BTK | NM_000061 |
| TIAM1 | NM_003253 |
| C3AR1 | NM_004054 |
| TRIO | NM_007118 |
| TSC1 | NM_000368 |
| TTN | NM_133432 |
| WNT7A | NM_004625 |
| FMNL1 | NM_005892 |

(continued)

| Gene Name | Transcript ID |
|---|---|
| YWHAB | NM_139323 |
| CXCR4 | NM_003467 |
| MAPKAPK3 | NM_004635 |
| ARHGAP10 | NM_024605 |
| ELMO3 | NM_024712 |
| ARHGAP28 | NM_001010000 |
| ARHGEF5 | NM_005435 |
| RIN3 | NM_024832 |
| NUP85 | NM_024844 |
| DOCK5 | NM_024940 |
| SHOC2 | NM_007373 |
| MAP1LC3B | NM_022818 |
| SPRY4 | NM_030964 |
| ARHGAP24 | NM_001025616 |
| RASSF5 | NM_182663 |
| RASSF4 | NM_032023 |
| OBSCN | NM_001098623 |
| ANKRD27 | NM_032139 |
| ULK1 | NM_003565 |
| SYDE2 | NM_032184 |
| ARFGAP2 | NM_032389 |
| RASAL1 | NM_004658 |
| MAP1LC3A | MM_181509 |
| PARD6B | NM_032521 |
| GPR65 | NM_003608 |
| SPRYD3 | NM_032840 |
| ARHGAP19 | NM_032900 |
| RERG | NM_032918 |
| SYDE1 | NM_033025 |
| DOCK7 | NM_033407 |
| SCIN | NM_001112706 |
| RFXANK | NM_003721 |
| GBF1 | NM_004193 |
| IQGAP1 | NM_003870 |
| WISP1 | NM_003882 |
| KSR1 | NM_014238 |
| ARHGAP11B | NM_001039841 |
| FGD3 | NM_001083536 |

(continued)

| Gene Name | Transcript ID |
|---|---|
| KALRN | NM_001024660 |
| F2RL3 | NM_003950 |
| DOK2 | NM_003974 |
| PRC1 | NM_199414 |
| USP6 | NM_004505 |
| FMNL3 | NM_198900 |
| MAPKAPK2 | NM_004759 |
| CYTH3 | NM_004227 |
| CYTH1 | NM_004762 |
| GPR55 | NM_005683 |
| ARHGAP18 | NM_033515 |
| GRAP2 | NM_004810 |
| ARHGAP29 | NM_004815 |
| SYTL5 | NM_138780 |
| ARHGAP12 | NM_018287 |
| BAG3 | NM_004281 |
| CD44 | NM_001001390 |
| RIN1 | NM_004292 |
| TRAF4 | NM_004295 |
| GNA14 | NM_004297 |
| RAPGEF2 | NM_014247 |
| NOS1AP | NM_014697 |
| DOCK4 | NM_014705 |
| STARD8 | NM_014725 |
| GIT2 | NM_057170 |
| ARHGAP11A | NM_014783 |
| ARHGEF11 | NM_014784 |
| ELMO1 | NM_014800 |
| FARP2 | NM_014808 |
| SRGAP3 | NM_014850 |
| G3BP2 | NM_203504 |
| MFN2 | NM_014874 |
| ARHGAP25 | NM_001007231 |
| CDC42 | NM_001039802 |

Unless otherwise indicated, "RAS pathway signature" or "RAS pathway signature score" refers to or is based on, respectively, the 147 biomarkers presented in Tables 2a and 2b, or subsets of these biomarkers.

"RAS pathway agent" refers to an agent that modulates signaling through the RAS pathway. A RAS pathway inhibitor inhibits signaling through the RAS pathway. Molecular targets of such agents include, but are not limited to:

RAS, RAF, MEK, MAPK, ELK1, and the genes listed in the Table 1. Such agents are well known in the art and include, but are not limited to: RAS inhibitors ISIS 2503 and farnesyl transferase inhibitor R115777, L731735, SCH 66336, and BMS214662; Raf inhibitors ISIS 5132 and BAY43-9006; MEK inhibitors PD184322 and CI-1040 (reviewed in Dancey, 2002, Curr. Pharm. Des. 8:2259-2267; Sebolt-Leopold et al., 1999, Nat. Med 5:810-816; Downward, 2003, Nat. Rev. Cancer 3:11-22).

"Growth factor signaling pathway" is initiated by binding of growth factors (including, but not limited to, heregulin, insulin, IGF, FGF, EGF) to receptor tyrosine kinases (including, but not limited to the ERBB family of receptors). The binding of a growth factor to its corresponding receptor leads to receptor dimerization, phosphorylation of key tyrosine residues, and recruitment of several proteins at the intracellular portion of the receptor. These proteins then initiate intracellular signaling via several pathways, such as PI3K/AKT, RAS/ERK, and JAK/STAT signaling pathways, leading to the activation of anti-apoptotic proteins and the inactivation of pro-apoptotic proteins (reviewed in Henson and Gibson, 2006, Cellular Signaling 18:2089-2097). In this application, unless otherwise specified, it will be understood that "growth factor signaling pathway" refers to signaling through PI3K/AKT signaling pathway, initiated by the binding of an external growth factor to a membrane tyrosine kinase receptor.

"PI3K signaling pathway," also known as the "PI3K/AKT signaling pathway" or "AKT signaling pathway" refers to one of the intracellular signaling pathways activated by the binding of growth factors to receptor tyrosine kinases. On activation, PI3K phosphorylates phosphatidylinositol-4,5-bisphosphate (PIP2) to phsophatidylinositol-3,4,5-triphosphate (PIP3), a process that is reversed by PTEN. PIP3 signals activate the kinase PDK1, which in turn activates the kinase AKT. See also PCT application, "Methods and Gene Expression Signature for Assessing Growth Factor Signaling Pathway Regulation Status," by James Watters et al., filed on March 19, 2009, for an illustration and description of the PI3K signaling pathway. In addition, see Hennessy et al., 2005, Nat. Rev. Drug Discov. 4:988-1004 for a review of the PI3K/AKT signaling cascade).

"Growth factor pathway agent" or "PI3K agent" refers to an agent which modulates growth factor pathway signaling through the PI3K/AKT signaling arm. A growth factor pathway or PI3K inhibitor inhibits growth factor pathway signaling through the PI3K/AKT signaling arm. Molecular targets of such inhibitors may include PI3K, AKT, mTOR, PDK1, MYC, cMET, FGFR2, growth factors (EGF, b-FGF, IGF1, Insulin, or Heregulin) and their corresponding receptors. Such agents are well known in the art and include, but are not limited to: phosphatidylinositol ether lipid analogs, alkylphospholipid analogs, allosteric AKT inhibitors, HSP90 inhibitor, alkylphospholipid perifosine, rapamycin, RAD001, FTY720, PDK1 inhibitors (BX-795, BX-912, and BX-320 (Feldman et al., 2005, J. Biol. Chem. 280:19867-19874); 7-hydroxystaurosporine (Sato et al., 2002, Oncogene, 21:1727-1738)); PI3K inhibitors, such as wortmannin (Wymann et al., 1996, Mol. Cell. Biol. 16:1722-1733); LY294002 (Vlahos et al., 1994, J. Biol. Chem. 269:5241-5248; Wetzker and Rommel, 2004, Curr. Pharm. Des. 10:1915-1922); IC87114 (Finan and Thomas, 2004, Biochem. Soc. Trans. 32:378-382; WO0181346); WO01372557; US6403588; WO0143266); AKT antibodies (Shin et al., 2005, Cancer Res. 65:2815-2824) (see also Cheng et al., Oncogene, 2005, 24:7482-7492 for review on inhibitors of AKT pathway), and IGF1R inhibitors (such as monoclonal antibody MK-0646 U.S. Patent 7,241,444). The inhibitors and agents listed in the PCT application, "Methods and Gene Expression Signature for Assessing Growth Factor Signaling Pathway Regulation Status," by James Watters et al., filed on March 19, 2009, that were used to identify and refine the growth factor signaling pathway biomarkers are also exemplary growth factor pathway agents (i.e., AKT1/2 inhibitors L-001154547 ('547; 3-phenyl-2-(4-{[4-(5-pyridin-2-yl-1H-1,2,4-triazol-3-yl)piperidin-1-yl]methyl}phenyl)-1,6-naphthyridin-5(6H)-one; disclosed in WO2006065601), L-01173931 ('931; 6-Methyl-3-phenyl-2-(4-{[4-(5-pyridin-2-yl-1H-1,2,4-triazol-3-yl)piperidin-1-yl]-methyl}phenyl)-1,6-naphthyridin-5(6H)-one; disclosed in WO2006065601; gamma secretase inhibitor 421B (US 7,138,400 and WO02/36555); cMET inhibitors L-001501404 (4-(6-Phenyl-[1,2,4]triazolo[4,3-b][1,2,4)triazin-3-ylmethyl)-phenol, see also US 7,122,548), MK-2461 (*N*-[(*2R*)-1,4-dioxan-2-ylmethyl)-*N*-methyl-*N'*-[3-(1-methyl-1*H*-pyrazol-4-yl)-5-oxo-*5H*-benzo[4,5]cyclohepta[1,2-*b*]pyridin-7-yl]sulfamide), and L-001793225 (1-[3-(1-Methyl-1*H*-pyrazol-4-yl)-5-oxo-5*H*-benzo[4,5]cyclohepta[1,2-b]pyridin-7-yl]-*N*-(pyridin-2-ylmethyl)methanesulfonamide.

The term "deregulated signaling pathway" is used herein to mean that the signaling pathway is either hyperactivated or hypoactivated. A RAS signaling pathway is hyperactivated in a sample (for example, a tumor sample) if it has at least 10%, 20%, 50%, 75%, 100%, 200%, 500%, 1000% greater activity/signaling than the RAS signaling pathway in a normal (regulated) sample. A RAS signaling pathway is hypoactivated if it has at least 10%, 20%, 50%, 75%, 100% less activity/signaling in a sample (for example, a tumor sample) than the RAS signaling pathway in a normal (regulated) sample. The normal sample with the regulated RAS signaling pathway may be from adjacent normal tissue or may be other tumor samples which do not have deregulated RAS signaling. Alternatively, comparison of samples RAS signaling pathway status may be done with identical samples which have been treated with a drug or agent vs. vehicle. The change in activation or regulation status may be due to a mutation of one or more genes in the RAS signaling pathway (such as point mutations, deletion, or amplification), changes in transcriptional regulation (such as methylation, phosphorylation, or acetylation changes), or changes in protein regulation (such as translation or post-translational control mechanisms).

The term "oncogenic pathway" is used herein to mean a pathway that when hyperactivated or hypoactivated contributes to cancer initiation or progression. In one embodiment, an oncogenic pathway is one that contains an oncogene or a tumor suppressor gene.

The term "treating" in its various grammatical forms in relation to the present invention refers to preventing (i.e., chemoprevention), curing, reversing, attenuating, alleviating, minimizing, suppressing, or halting the deleterious effects of a disease state, disease progression, disease causative agent (e.g., bacteria or viruses), or other abnormal condition. For example, treatment may involve alleviating a symptom (i.e., not necessarily all the symptoms) of a disease of attenuating the progression of a disease.

"Treatment of cancer," as used herein, refers to partially or totally inhibiting, delaying, or preventing the progression of cancer including cancer metastasis; inhibiting, delaying, or preventing the recurrence of cancer including cancer metastasis; or preventing the onset or development of cancer (chemoprevention) in a mammal, for example, a human. In addition, the methods of the present invention may be practiced for the treatment of human patients with cancer. However, it is also likely that the methods would also be effective in the treatment of cancer in other mammals.

As used herein, the term "therapeutically effective amount" is intended to qualify the amount of the treatment in a therapeutic regiment necessary to treat cancer. This includes combination therapy involving the use of multiple therapeutic agents, such as a combined amount of a first and second treatment where the combined amount will achieve the desired biological response. The desired biological response is partial or total inhibition, delay, or prevention of the progression of cancer including cancer metastasis; inhibition, delay, or prevention of the recurrence of cancer including cancer metastasis; or the prevention of the onset of development of cancer (chemoprevention) in a mammal, for example, a human.

"Displaying or outputting a classification result, prediction result, or efficacy result" means that the results of a gene expression based sample classification or prediction are communicated to a user using any medium, such as for example, orally, writing, visual display, etc., computer readable medium or computer system. It will be clear to one skilled in the art that outputting the result is not limited to outputting to a user or a linked external component(s), such as a computer system or computer memory, but may alternatively or additionally be outputting to internal components, such as any computer readable medium. Computer readable media may include, but are not limited to hard drives, floppy disks, CD-ROMs, DVDs, DATs. Computer readable media does not include carrier waves or other wave forms for data transmission. It will be clear to one skilled in the art that the various sample classification methods disclosed and claimed herein, can, but need not be, computer-implemented, and that, for example, the displacing or outputting step can be done by, for example, by communicating to a person orally or in writing (e.g., in handwriting).

## 3.3 BIOMARKERS USERFUL IN CLASSIFYING TUMORS AND PREDICTING RESPONSE TO THERAPEUTIC AGENTS

### 3.3.1 Biomarker Sets

One aspect of the invention provides a set of 147 biomarkers whose expression is correlated with RAS signaling pathway deregulation by clustering analysis. These biomarkers identified as useful for classifying tumors according to regulation status of the RAS signaling pathway, predicting response of a cancer patient to a compound that modulates the RAS signaling pathway, predicting resistance of a cancer patient to a compound that modulates the PI3K signaling pathway or EGFR, or measuring pharmacodynamic effect on the RAS signaling pathway of a therapeutic agent, are listed as SEQ ID NOs: 1-105 and 211-252 (see also Tables 2a and 2b). Another aspect of the invention provides a method of using these biomarkers to distinguish tumor types in diagnosis or to predict response to therapeutic agents. In one embodiment of the invention, the 147 biomarker set may be split into two opposing "arms" - the "up" arm (see Table 2a), which are the 105 genes that are upregulated, and the "down" arm (Table 2b), which are the 42 genes that are downregulated, as signaling through the RAS pathway increases.

In one embodiment, the invention provides a set of 147 biomarkers that can classify tumors by RAS pathway regulation status, i.e., distinguish between tumors having regulated and deregulated RAS signaling pathways. These biomarkers are listed in Tables 2a and 2b. The invention also provides subsets of at least 5, 10, 20, 30, 40, 50, 60, 70, 80, 90, 100, 110, 120, 130, and 140 biomarkers, drawn from the set of 147 (Tables 2a and 2b), wherein at least one biomarker from the subset is selected from Table 2b, that can distinguish between tumors having deregulated and regulated RAS signaling pathways. Alternatively, at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, or 42 biomarkers is selected from Table 2b for each aforementioned subset. Alternatively, a subset of at least 3, 5, 10, 15, 20, 25, 30, 35, 40, 45, 50, 55, 60, 65, 70, 75, 80, 85,90, 95, or 100 biomarkers, drawn from the "up" arm (see Table 2a) and a subset of at least 3, 5, 10, 15, 20, 25, 30, 35, or 40 biomarkers from the "down" arm (see Table 2b) that can distinguish between tumors having deregulated and regulated RAS signaling pathways are provided. The invention also provides a method of using the above biomarkers to distinguish between tumors having deregulated or regulated RAS signaling pathway.

In another embodiment, the invention provides a set of 147 genetic biomarkers that can be used to predict response of a subject to a RAS signaling pathway agent. In a more specific embodiment, the invention provides a subset of at least 5, 10, 20, 30, 40, 50, 60, 70, 80, 90, 100, 110, 120, 130, and 140 biomarkers, drawn from the set of 147 (Tables 2a and 2b), wherein at least one biomarker from the subset is selected from Table 2b, that can be used to predict the response of a subject to an agent that modulates the RAS signaling pathway. In another embodiment, the invention provides a set of 147 biomarkers that can be used to select a RAS pathway agent for treatment of a subject with cancer. In a more specific embodiment, the invention provides a subset of at least 5, 10, 20, 30, 40, 50, 60, 70, 80, 90, 100, 110, 120, 130, and 140 biomarkers, drawn from the set of 147 (Tables 2a and 2b), wherein at least one biomarker from the subset is selected from Table 2b, that can be used to select a RAS pathway agent for treatment of a subject with cancer. Alternatively, at least 2, 3,4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, or 42 biomarkers is selected from Table 2b for each aforementioned subset. Alternatively, a subset of at least 3, 5, 10, 15, 20, 2S, 30, 35, 40, 45, 50, 55, 60, 65, 70, 75, 80, 85, 90, 95, or 100 biomarkers, drawn from the "up" arm (see Table 2a) and a subset of at least 3, 5, 10, 15, 20, 25, 30, 35, or 40 biomarkers from the "down" arm (see Table 2b) can be used to predict response of a subject to a RAS signaling pathway agent or to select a RAS signaling pathway agent for treatment of a subject with cancer.

In another embodiment, the invention provides a set of 147 genetic biomarkers that can be used to predict resistance of a subject to a PI3K signaling pathway agent. In a more specific embodiment, the invention provides a subset of at least 5, 10, 20, 30, 40, 50, 60, 70, 80, 90, 100, 110, 120, 130, and 140 biomarkers, drawn from the set of 147 (Tables 2a and 2b), wherein at least one biomarker from the subset is selected from Table 2b, that can be used to predict the resistance of a subject to an agent that modulates the PI3K signaling pathway. In another embodiment, the invention provides a set of 147 biomarkers that can be used to exclude a PI3K pathway agent for treatment of a subject with cancer. In a more specific embodiment, the invention provides a subset of at least 5, 10, 20, 30, 40, 50, 60, 70, 80, 90, 100, 110, 120, 130, and 140 biomarkers, drawn from the set of 147 (Tables 2a and 2b), wherein at least one biomarker from the subset is selected from Table 2b, that can be used to select a RAS pathway agent for treatment of a subject with cancer. Alternatively, at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, or 42 biomarkers is selected from Table 2b for each aforementioned subset. Alternatively, a subset of at least 3, 5, 10, 15, 20, 25, 30, 35, 40, 45, 50, 55, 60, 65, 70, 75, 80, 85, 90, 95, or 100 biomarkers, drawn from the "up" arm (see Table 2a) and a subset of at least 3, 5, 10, 15, 20, 25, 30, 35, or 40 biomarkers from the "down" arm (see Table 2b) can be used to predict resistance of a subject to a PI3K signaling pathway agent or to exclude a PI3K signaling pathway agent for treatment of a subject with cancer.

In another embodiment, the invention provides a set of 147 genetic biomarkers that can be used to determine whether an agent has a pharmacodynamic effect on the RAS signaling pathway. The biomarkers provided may be used to monitor inhibition of the RAS signaling pathway at various time points following treatment with said agent. In a more specific embodiment, the invention provides a subset of at least 5, 10, 20, 30, 40, 50, 60, 70, 80, 90, 100, 110, 120, 130, and 140 biomarkers, drawn from the set of 147 (Tables 2a and 2b), wherein at least one biomarker from the subset is selected from Table 2b, that can be used to monitor pharmacodynamic activity of an agent on the RAS signaling pathway. Alternatively, at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 3 1, 32, 33, 34, 35, 36, 37, 38, 39, 40, 41, or 42 biomarkers is selected from Table 2b for each aforementioned subset. Alternatively, a subset of at least 3, 5, 10,15,20, 25, 30, 35,40,45, 50, 55,60, 65, 70, 75, 80, 85, 90, 95, or 100 biomarkers, drawn from the "up" arm (see Table 2a) and a subset of at least 3, 5, 10, 15, 20, 25, 30, 35, or 40 biomarkers from the "down" arm (see Table 2b) can be used to determine whether an agent has a pharmacodynamic effect on the RAS signaling pathway or monitor pharmacodynamic activity of an agent on the RAS signaling pathway.

Any of the sets of biomarkers provided above may be used alone specifically or in combination with biomarkers outside the set. For example, biomarkers that distinguish RAS signaling pathway regulation status may be used in combination with biomarkers that distinguish growth factor pathway signaling status (see PCT application, "Methods and Gene Expression Signature for Assessing Growth Factor Signaling Pathway Regulation Status" by James Watters et al., filed on March 19, 2009, incorporated herein in its entirety) or p53 functional status (see U.S. non-provisional application, "Gene Expression Signature for Assessing p53 Pathway Functional Status," by Andrey Loboda et al., filed March 19, 2009. Any of the biomarker sets provided above may also be used in combination with other biomarkers for cancer, or for any other clinical or physiological condition.

### 3.3.2 Identification of the Biomarkers

The present invention provides sets of biomarkers for the identification of conditions or indications associated with cancer. Generally, the biomarker sets were identified by determining which of ~44,000 human biomarkers had expression patterns that correlated with the conditions or indications.

In one embodiment, the method for identifying biomarker sets is as follows. After extraction and labeling of target polynucleotides, the expression of all biomarkers (genes) in a sample X is compared to the expression of all biomarkers in a standard or control. In one embodiment, the standard or control comprises target polynucleotides derived from a sample from a normal individual (i.e. an individual not having RAS pathway deregulation). Alternatively, the standard or control comprises polynucleotides derived from normal tissue adjacent to a tumor or from tumors not have RAS pathway deregulation. In a preferred embodiment, the standard or control is a pool of target polynucleotide molecules. The pool may be derived from collected samples from a number of normal individuals. In another embodiment, the pool comprises samples taken from a number of individuals with tumors not having RAS pathway deregulation. In another preferred embodiment, the pool comprises an artificially-generated population of nucleic acids designed to approximate the level of nucleic acid derived from each biomarker found in a pool of biomarker-derived nucleic acids derived from tumor samples. In yet another embodiment, the pool is derived from normal or cancer lines or cell line samples.

The comparison may be accomplished by any means known in the art. For example, expression levels of various biomarkers may be assessed by separation of target polynucleotide molecules (e.g. RNA or cDNA) derived from the biomarkers in agarose or polyacrylamide gels, followed by hybridization with biomarker-specific oligonucleotide probes. Alternatively, the comparison may be accomplished by the labeling of target polynucleotide molecules followed by separation on a sequencing gel. Polynucleotide samples are placed on the gel such that patient and control or standard polynucleotides are in adjacent lanes. Comparison of expression levels is accomplished visually or by means of densitometer. In a preferred embodiment, the expression of all biomarkers is assessed simultaneously by hybridization to a microarray. In each approach, biomarkers meeting certain criteria are identified as associated with tumors having RAS signaling pathway deregulation.

A biomarker is selected based upon significant difference of expression in a sample as compared to a standard or control condition. Selection may be made based upon either significant up- or down regulation of the biomarker in the patient sample. Selection may also be made by calculation of the statistical significance (i.e., the p-value) of the correlation between the expression of the biomarker and the condition or indication. Preferably, both selection criteria are used. Thus, in one embodiment of the invention, biomarkers associated with deregulated RAS signaling pathway in a tumor are selected where the biomarkers show both more than twofold change (increase or decrease) in expression as compared to a standard, and the p-value for the correlation between the existence of RAS signaling pathway deregulation and the change in biomarker expression is no more than 0.01 (i.e., is statistically significant).

Expression profiles comprising a plurality of different genes in a plurality of *N* cancer tumor samples can be used to identify markers that correlate with, and therefore are useful for discriminating different clinical categories. In a specific embodiment, a correlation coefficient $\rho$ between a vector $\vec{c}$ representing clinical categories or clinical parameters, e.g., a regulated or deregulated RAS signaling pathway, in the *N* tumor samples and a vector $\vec{r}$ representing the measured expression levels of a gene in the *N* tumor samples is used as a measure of the correlation between the expression level of the gene and RAS signaling pathway status. The expression levels can be a measured abundance level of a transcript of the gene, or any transformation of the measured abundance, e.g., a logarithmic or a log ratio. Specifically, the correlation coefficient may be calculated as:

$$\rho = (\vec{c} \bullet \vec{r}) / (\|\vec{c}\| \cdot \|\vec{r}\|) \qquad (1)$$

Biomarkers for which the coefficient of correlation exceeds a cutoff are identified as RAS pathway signaling status-informative biomarkers specific for a particular clinical category, *e.g.*, deregulated RAS pathway signaling status, within a given patient subset. Such a cutoff or threshold may correspond to a certain significance of the set of obtained discriminating genes. The threshold may also be selected based on the number of samples used. For example, a threshold can be calculated as $3 \times 1/\sqrt{n-3}$, where $1/\sqrt{n-3}$ is the distribution width and *n* = the number of samples. In a specific embodiment, markers are chosen if the correlation coefficient is greater than about 0.3 or less than about -0.3.

Next, the significance of the set of biomarker genes can be evaluated. The significance may be calculated by any appropriate statistical method. In a specific example, a Monte-Carlo technique is used to randomize the association between the expression profiles of the plurality of patients and the clinical categories to generate a set of randomized data. The same biomarker selection procedure as used to select the biomarker set is applied to the randomized data to obtain a control biomarker set. A plurality of such runs can be performed to generate a probability distribution of the number of genes in control biomarker sets. In a preferred embodiment, 10,000 such runs are performed. From the probability distribution, the probability of finding a biomarker set consisting of a given number of biomarkers when no correlation between the expression levels and phenotype is expected (i.e., based randomized data) can be determined. The significance of the biomarker set obtained from the real data can be evaluated based on the number of biomarkers in the biomarker set by comparing to the probability of obtaining a control biomarker set consisting of the same number of biomarkers using the randomized data. In one embodiment, if the probability of obtaining a control biomarker set consisting of the same number of biomarkers using the randomized data is below a given probability threshold, the biomarker set is said to be significant.

Once a biomarker set is identified, the biomarkers may be rank-ordered in order of correlation or significance of discrimination. One means of rank ordering is by the amplitude of correlation between the change in gene expression of the biomarker and the specific condition being discriminated. Another, preferred, means is to use a statistical metric. In a specific embodiment, the metric is a t-test-like statistic:

$$t = \left(\langle x_1\rangle - \langle x_2\rangle\right) \Big/ \sqrt{\left[\sigma_1^2(n_1-1) + \sigma_2^2(n_2-1)\right] \Big/ (n_1+n_2-1) \Big/ \left(1/n_1 + 1/n_2\right)} \qquad (2)$$

In this equation, $\langle x_1\rangle$ is the error-weighted average of the log ratio of transcript expression measurements within a first clinical group (*e.g.*, deregulated RAS pathway signaling), $\langle x_2\rangle$ is the error-weighted average of log ratio within a second, related clinical group (*e.g.*, regulated RAS pathway signaling), $\sigma_1$ is the variance of the log ratio within the first clinical group (*e.g.*, deregulated RAS pathway signaling), $n_1$ is the number of samples for which valid measurements of log ratios are available, $\sigma_2$ is the variance of log ratio within the second clinical group (*e.g.*, regulated RAS pathway signaling), and $n_2$ is the number of samples for which valid measurements of log ratios are available. The *t*-value represents the variance-compensated difference between two means. The rank-ordered biomarker set may be used to optimize the number of biomarkers in the set used for discrimination.

A set of genes for RAS pathway signaling status can also be identified using an iterative approach. This is accomplished generally in a "leave one out" method as follows. In a first run, a subset, for example five, of the biomarkers from the top of the ranked list is used to generate a template, where out of *N* samples, *N*-1 are used to generate the template, and the status of the remaining sample is predicted. This process is repeated for every sample until every one of the *N* samples is predicted once. In a second run, one or more additional biomarkers, for example five additional biomarkers, are added, so that a template is now generated from 10 biomarkers, and the outcome of the remaining sample is predicted. This process is repeated until the entire set of biomarkers is used to generate the template. For each of the runs, type 1 error (false negative) and type 2 errors (false positive) are counted. The set of top-ranked biomarkers that corresponds to lowest type 1 error rate, or type 2 error rate, or preferably the total of type 1 and type 2 error rate is selected.

For RAS pathway signaling status biomarkers, validation of the marker set may be accomplished by an additional statistic, a survival model. This statistic generates the probability of tumor distant metastases as a function of time since initial diagnosis. A number of models may be used, including Weibull, normal, log-normal, log logistic, log-exponential, or log-Rayleigh (Chapter 12 "Life Testing", S-PLUS 2000 GUIDE TO STATISTICS, Vol. 2, p. 368 (2000)). For the "normal" model, the probability of distant metastases P at time *t* is calculated as

$$P = \alpha \times \exp\left(-t^2/\tau^2\right) \qquad (3)$$

where *a* is fixed and equal to 1, and $\tau$ is a parameter to be fitted and measures the "expected lifetime".

It is preferable that the above biomarker identification process be iterated one or more times by excluding one or more samples from the biomarker selection or ranking (*i.e.*, from the calculation of correlation). Those samples being excluded are the ones that can not be predicted correctly from the previous iteration. Preferably, those samples excluded from biomarker selection in this iteration process are included in the classifier performance evaluation, to avoid overstating the performance.

Once a set of genes for RAS pathway signaling status has been identified, the biomarkers may be split into two opposing "arms" - the "up" arm (see Table 2a), which are the genes that are upregulated, and the "down" arm (see Table 2b), which are the genes that are downregulated, as signaling through the RAS pathway increases.

It will be apparent to those skilled in the art that the above methods, in particular the statistical methods, described above, are not limited to the identification of biomarkers associated with RAS signaling pathway regulation status, but may be used to identify set of biomarker genes associated with any phenotype. The phenotype can be the presence or absence of a disease such as cancer, or the presence or absence of any identifying clinical condition associated with that cancer. In the disease context, the phenotype may be prognosis such as survival time, probability of distant metastases of disease condition, or likelihood of a particular response to a therapeutic or prophylactic regimen. The phenotype need not be cancer, or a disease; the phenotype may be a nominal characteristic associated with a healthy individual.

3.3.3 Sample Collection

In the present invention, target polynucleotide molecules are typically extracted from a sample taken from an individual afflicted with cancer or tumor cell lines, and corresponding normal/control tissues or cell lines, respectively. The sample may be collected in any clinically acceptable manner, but must be collected such that biomarker-derived polynucleotides (i.e., RNA) are preserved. mRNA or nucleic acids derived therefrom (i.e., cDNA or amplified DNA) are preferably labeled distinguishably from standard or control polynucleotide molecules, and both are simultaneously or independently hybridized to a microarray comprising some or all of the biomarkers or biomarker sets or subsets described above. Alternatively, mRNA or nucleic acids derived therefrom may be labeled with the same label as the standard or control polynucleotide molecules, wherein the intensity of hybridization of each at a particular probe is compared. A sample may comprise any clinically relevant tissue sample, such as a tumor biopsy or fine needle aspirate, or a sample of bodily fluid, such as blood, plasma, serum, lymph, ascitic fluid, cystic fluid, urine. The sample may be taken from a human, or, in a veterinary context, from non-human animals such as ruminants, horses, swine or sheep, or from domestic companion animals such as felines and canines. Additionally, the samples may be from frozen or archived formalin-fixed, paraffin-embedded (FFPE) tissue samples.

Methods for preparing total and poly(A)+ RNA are well known and are described generally in Sambrook et al., MOLECULAR CLONING--A LABORATORY MANUAL (2ND ED.), Vols. 1-3, Cold Spring Harbor Laboratory, Cold Spring Harbor, N.Y. (1989)) and Ausubel et al., CURRENT PROTOCOLS IN MOLECULAR BIOLOGY, vol. 2, Current Protocols Publishing, New York (1994)).

RNA may be isolated from eukaryotic cells by procedures that involve lysis of the cells and denaturation of the proteins contained therein. Cells of interest include wild-type cells (i.e., non-cancerous), drug-exposed wild-type cells, tumor-or tumor-derived cells, modified cells, normal or tumor cell line cells, and drug-exposed modified cells.

Additional steps may be employed to remove DNA. Cell lysis may be accomplished with a nonionic detergent, followed by microcentrifugation to remove the nuclei and hence the bulk of the cellular DNA. In one embodiment, RNA is extracted from cells of the various types of interest using guanidinium thiocyanate lysis followed by CsCl centrifugation to separate the RNA from DNA (Chirgwin et al., Biochemistry 18:5294-5299 (1979)). Poly(A)+ RNA is selected by selection with oligo-dT cellulose (see Sambrook et al, MOLECULAR CLONING--A LABORATORY MANUAL (2ND ED.), Vols. 1-3, Cold Spring Harbor Laboratory, Cold Spring Harbor, N.Y. (1989). Alternatively, separation of RNA from DNA can be accomplished by organic extraction, for example, with hot phenol or phenol/chloroform/isoamyl alcohol.

If desired, RNase inhibitors may be added to the lysis buffer. Likewise, for certain cell types, it may be desirable to add a protein denaturation/digestion step to the protocol.

For many applications, it is desirable to preferentially enrich mRNA with respect to other cellular RNAs, such as transfer RNA (tRNA) and ribosomal RNA (rRNA). Most mRNAs contain a poly(A) tail at their 3' end. This allows them to be enriched by affinity chromatography, for example, using oligo(dT) or poly(U) coupled to a solid support, such as cellulose or Sephadex® (see Ausubel et al., CURRENT PROTOCOLS IN MOLECULAR BIOLOGY, vol. 2, Current Protocols Publishing, New York (1994). Once bound, poly(A)+ mRNA is eluted from the affinity column using 2 mM EDTA/0.1% SDS.

The sample of RNA can comprise a plurality of different mRNA molecules, each different mRNA molecule having a different nucleotide sequence. In a specific embodiment, the mRNA molecules in the RNA sample comprise at least 100 different nucleotide sequences. More preferably, the mRNA molecules of the RNA sample comprise mRNA molecules corresponding to each of the biomarker genes. In another specific embodiment, the RNA sample is a mammalian RNA sample.

In a specific embodiment, total RNA or RNA from cells is used in the methods of the invention. The source of the RNA can be cells of a plant or animal, human, mammal, primate, non-human animal, dog, cat, mouse, rat, bird, yeast, eukaryote, prokaryote, etc. In specific embodiments, the method of the invention is used with a sample containing total mRNA or total RNA from $1\times10^6$ cells or less. In another embodiment, proteins can be isolated from the foregoing sources, by methods known in the art, for use in expression analysis at the protein level.

Probes to the homologs of the biomarker sequences disclosed herein can be employed preferably wherein non-human nucleic acid is being assayed.

3.4 Methods of Using RAS Signaling Pathway Deregulation Biomarker Sets

3.4.1 Diagnostic/Tumor Classifcation Methods

The invention provides for methods of using the biomarker sets to analyze a sample from an individual so as to determine or classify the individual's tumor type at a molecular level, whether a tumor has a deregulated or regulated RAS signaling pathway. The individual need not actually be afflicted with cancer. Essentially, the expression of specific biomarker genes in the individual, or a sample taken therefrom, is compared to a standard or control. For example, assume two cancer-related conditions, X and Y. One can compare the level of expression of RAS signaling pathway biomarkers for condition X in an individual to the level of the biomarker-derived polynucleotide in a control, wherein the level represents the level of expression exhibited by samples having condition X. In this instance, if the expression of the markers in the individual's sample is substantially (i.e., statistically) different from that of the control, then the individual does not have condition X. Where, as here, the choice is bimodal (i.e. a sample is either X or Y), the individual can additionally be said to have condition Y. Of course, the comparison to a control representing condition Y can also be performed. Preferably, both are performed simultaneously, such that each control acts as both a positive and a negative control. The distinguishing result may thus either be a demonstrable difference from the expression levels (i.e. the amount of marker-derived RNA, or polynucleotides derived therefrom) represented by the control, or no significant difference.

Thus, in one embodiment, the method of determining a particular tumor-related status of an individual comprises the steps of (1) hybridizing labeled target polynucleotides from an individual to a microarray containing the above biomarker set or a subset of the biomarkers; (2) hybridizing standard or control polynucleotide molecules to the microarray, wherein the standard or control molecules are differentially labeled from the target molecules; and (3) determining the difference in transcript levels, or lack thereof, between the target and standard or control, wherein the difference, or lack thereof, determines the individual's tumor-related status. In a more specific embodiment, the standard or control molecules comprise biomarker-derived polynucleotides from a pool of samples from normal individuals, a pool of samples from normal adjacent tissue, or a pool of tumor samples from individuals with cancer. In a preferred embodiment, the standard or control is artificially-generated pool of biomarker-derived polynucleotides, which pool is designed to mimic the level of biomarker expression exhibited by clinical samples of normal or cancer tumor tissue having a particular clinical indication (i.e. cancerous or non-cancerous; RAS signaling pathway regulated or deregulated). In another specific embodiment, the control molecules comprise a pool derived from normal or cancer cell lines.

The present invention provides a set of biomarkers useful for distinguishing deregulated from regulated RAS signaling pathway tumor types. Thus, in one embodiment of the above method, the level of polynucleotides (i.e., mRNA or polynucleotides derived therefrom) in a sample from an individual, expressed from the biomarkers provided in Tables 2a and 2b are compared to the level of expression of the same biomarkers from a control, wherein the control comprises biomarker-related polynucleotides derived from deregulated RAS signaling pathway tumor samples, regulated RAS signaling pathway tumor samples, or both. The comparison may be to both deregulated and regulated RAS signaling pathway tumor samples, and the comparison may be to polynucleotide pools from a number of deregulated and regulated RAS signaling pathway tumor samples, respectively. Where the individual's biomarker expression most closely resembles or correlates with the deregulated control, and does not resemble or correlate with the regulated control, the individual is classified as having a deregulated RAS signaling pathway. Where the pool is not pure deregulated or regulated RAS signaling pathway type tumors samples, for example, a sporadic pool is used, a set of experiments using individuals with known RAS signaling pathway status may be hybridized against the pool in order to define the expression templates for the deregulated and regulated group. Each individual with unknown RAS signaling pathway status is hybridized against the same pool and the expression profile is compared to the template(s) to determine the individual's RAS signaling pathway status.

In another specific embodiment, the method comprises:

(i) calculating a measure of similarity between a first expression profile and a deregulated RAS signaling pathway template, or calculating a first measure of similarity between said first expression profile and said deregulated RAS signaling pathway template and a second measure of similarity between said first expression profile and a regulated RAS signaling pathway template, said first expression profile comprising the expression levels of a first plurality of genes in the tumor cell sample, said deregulated RAS signaling pathway template comprising expression levels of said first plurality of genes that are average expression levels of the respective genes in a plurality of tumor cell samples having at least one or more components of said RAS signaling pathway with abnormal activity, and said regulated RAS signaling pathway template comprising expression levels of said first plurality of genes that are average expression levels of the respective genes in a plurality of tumor cells samples not having at least one or more components of said RAS signaling pathway with abnormal activity, said first plurality of genes consisting of at least 5 of the genes for which biomarkers are listed in Tables 2a and 2b, wherein at least 1 gene of said 5 genes is selected from Table 2b;

(ii) classifying said tumor cell sample as having said deregulated RAS signaling pathway if said first expression profile has a high similarity to said deregulated RAS signaling pathway template or has a higher similarity to said deregulated RAS signaling pathway template than to said regulated RAS signaling pathway template, or classifying said tumor cell sample as having said regulated RAS signaling pathway if said first expression profile has a low similarity to said deregulated RAS signaling pathway template or has a higher similarity to said regulated RAS signaling pathway template than to said deregulated RAS signaling pathway template; wherein said first expression profile has a high similarity to said deregulated RAS signaling pathway template if the similarity to said deregulated RAS signaling pathway template is above a predetermined threshold, or has a low similarity to said deregulated RAS signaling pathway template if the similarity to said deregulated RAS signaling pathway template is below said predetermined threshold; and

(iii) displaying; or outputting to a user, user interface device, a computer readable storage medium, or a local or remote computer system; the classification produced by said classifying step (ii).

For the above embodiments, the fullest of biomarkers may be used (i.e., the complete set of biomarkers from Tables 2a and 2b). In other embodiments, subsets 10, 15, 20, 25,30,35,40,45,50,55,60,65,70,75,80,85,90,95,100,105, 110,115,120,125,130,135, or 140 of the 147 biomarkers may be used, wherein at least 1, 2, 3, 4, 5, 6, 7, 8, 9, 10, 11, 12,13, 14, 15, 16, 17, 18, 19, 20, 21, 22, 23, 24, 25, 26, 27, 28, 29, 30, 31, 32, 33, 34, 35, 36, 37, 38, 39, 40, 41, or 42 biomarkers of each subset is selected from Table 2b. Alternatively, a subset of at least 3, 5, 10, 15, 20, 25, 30, 35, 40, 45, 50, 55, 60, 65, 70, 75, 80, 85, 90, 95, or 100 biomarkers, drawn from the "up" arm (see Table 2a) and a subset of at least 3, 5, 10, 15,20,25, 30, 35, or 40 biomarkers from the "down" arm (see Table 2b) can be used.

In another embodiment, the expression profile is a differential expression profile comprising differential measurements of said plurality of genes in a sample derived from a patient versus measurements of said plurality of genes in a control sample. The differential measurements can be xdev, log(ratio), error-weighted log(ratio), or a mean subtracted log(intensity) (see, e.g., PCT publication WO00/39339, published on July 6,2000; PCT publication WO2004/065545, published August 5, 2004. The similarity between the biomarker expression profile of a sample or an individual and that of a control can be assessed a number of ways using any method known in the art. For example, Dai et al. describe a number of different ways of calculating gene expression templates and corresponding biomarker genets useful in classifying breast cancer patients (US 7,171311; WO2002/103320 WO2005/086891; WO2006015312; WO2006/084272). Similarly, Linsley et al. (US2003/0104426) and Radish et al. (US20070154931) disclose gene biomarker genesets and methods of calculating gene expression templates useful in classifying chronic myelogenous leukemia patients. In the simplest case, the profiles can be compared visually in a printout of expression difference data. Alternatively, the similarity can be calculated mathematically.

In one embodiment, the similarity measure between two patients (or samples) x and y, or patient (or sample) x and a template y, can be calculated using the following equation:

$$S = 1 - \left[ \sum_{i=1}^{N_V} \frac{\left(x_i - \bar{x}\right)}{\sigma_{x_i}} \frac{\left(y_i - \bar{y}\right)}{\sigma_{y_i}} \Bigg/ \sqrt{\sum_{i=1}^{N_V} \left( \frac{x_i - \bar{x}}{\sigma_{x_i}} \right)^2 \sum_{i=1}^{N_V} \left( \frac{y_i - \bar{y}}{\sigma_{y_i}} \right)^2} \right] \quad (4)$$

In this equation, $\chi$ and $y$ are two patients with components of log ratio $x_i$ and $y_i$, $i$ = 1, 2,..., $N$ = 4,986. Associated with every value $x_i$ is error $\sigma_{x_i}$. The smaller the value $\sigma_{x_i}$, the more reliable the measurement $x_i$. $\bar{x} = \sum_{i=1}^{N_V} \frac{x_i}{\sigma_{x_i}^2} \Big/ \sum_{i=1}^{N_V} \frac{1}{\sigma_{x_i}^2}$ is the error-weighted arithmetic mean.

In one embodiment, the similarity is represented by a correlation coefficient between the patient or sample profile and the template. In one embodiment, a correlation coefficient above a correlation threshold indicates high similarity, whereas a correlation coefficient below the threshold indicates low similarity. In some embodiments, the correlation threshold is set as 0.3, 0.4, 0.5, or 0.6. In another embodiment, similarity between a sample or patient profile and a template is represented by a distance between the sample profile and the template. In one embodiment, a distance below a given value indicates a high similarity, whereas a distance equal to or greater than the given value indicates low similarity.

In a preferred embodiment, templates are developed for sample comparison. The template may be defined as the error-weighted log ratio average of the expression difference for the group of biomarker genes able to differentiate the particular RAS signaling pathway regulation status. For example, templates are defined for deregulated RAS signaling pathway samples and for regulated RAS signaling pathway samples. Next, a classifier parameter is calculated. This parameter may be calculated using either expression level differences between the sample and template, or by calculation of a correlation coefficient. Such a coefficient, $P_i$, can be calculated using the following equation:

$$P_i = \left(\vec{c}_i \bullet \vec{y}\right) / \left(\|\vec{c}_i\| \cdot \|\vec{y}\|\right) \quad (5)$$

where $i$ = 1 and 2.

As an illustration, in one embodiment, a template for a sample classification based upon one phenotypic endpoint, for example, RAS signaling pathway deregulated status, is defined as $\vec{c}_1$ (e.g., a profile consisting of correlation values, $C_1$, associated with, for example, RAS signaling pathway regulation status) and/or a template for second phenotypic endpoint, i.e., RAS signaling pathway regulated status, is defined as $\vec{c}_2$ (e.g., a profile consisting of correlation values, $C_2$, associated with, for example, RAS signaling pathway regulation status). Either one or both of the two classifier parameters ($P_1$ and $P_2$) can then be used to measure degrees of similarities between a sample's profile and the templates: $P_1$ measures the similarity between the sample's profile $\vec{y}$ and the first expression template $\vec{c}_1$, and $P_2$ measures the similarity between $\vec{y}$ and the second expression template $\vec{c}_2$.

Thus, in one embodiment, $\vec{y}$ is classified, for example, as a deregulated RAS signaling pathway profile if $P_1$ is greater than a selected correlation threshold or if $P_2$ is equal to or less than a selected correlation threshold. In another embodiment, $\vec{y}$ is classified, for example, as a regulated RAS signaling pathway profile if $P_1$ is less than a selected correlation threshold or if $P_2$ is above a selected correlation threshold. In still another embodiment, $\vec{y}$ is classified, for example, as a deregulated RAS signaling pathway profile if $P_1$ is greater than a first selected correlation threshold and $\vec{y}$ is classified, for example, as a regulated RAS signaling pathway profile if $P_2$ is greater than a second selected correlation threshold.

Thus, in a more specific embodiment, the above method of determining a particular tumor-related status of an individual comprises the steps of (1) hybridizing labeled target polynucleotides from an individual to a microarray containing one of the above marker sets; (2) hybridizing standard or control polynucleotides molecules to the microarray, wherein the standard or control molecules are differentially labeled from the target molecules; and (3) determining the ratio (or difference) of transcript levels between two channels (individual and control), or simply the transcript levels of the individual; and (4) comparing the results from (3) to the predefined templates, wherein said determining is accomplished by any means known in the art (see Section 3.4.6 on Methods for Classification of Expression Profiles), and wherein the difference, or lack thereof, determines the individual's tumor-related status.

The method can use the fullest of biomarkers (*i.e.*, the complete set of biomarkers from Tables 2a and 2b). However, subsets of at least 10,15, 20,25, 30, 35,40,45, 50, 55,60, 65, 70, 75, 80, 85 ,90, 95, 100, 105, 110, 115, 120, 125, 130, 135, or 140 of the 147 biomarkers may be used, wherein at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39,40,41, or 42 biomarkers of each subset is selected from Table 2b. Alternatively, a subset of at least 3, 5, 10,15,20,25, 30, 35, 40, 45, 50, 55, 60, 65, 70, 75, 80, 85, 90, 95, or 100 biomarkers, drawn from the "up" arm (see Table 2a) and a subset of at least 3, 5, 10, 15, 20, 25, 30, 35, or 40 biomarkers from the "down" arm (see Table 2b) can be used.

In another embodiment, the above method of determining the RAS pathway regulation status of an individual uses the two "arms" of the 147 biomarkers. The "up" arm comprises the 105 genes whose expression goes up with RAS pathway activation (see Table 2a), and the "down" arm comprises the 42 genes whose expression goes down with RAS pathway activation (see Table 2b). When comparing an individual sample with a standard or control, the expression value of gene X in the sample is compared to the expression value of gene X in the standard or control. For each gene in the set of biomarkers, log(10) ratio is created for the expression value in the individual sample relative to the standard or control (differential expression value). A signature "score" is calculated by determining the mean log(10) ratio of the genes in the "up" and then subtracting the mean log(10) ratio of the genes in the "down" arm. To determine if this signature score is significant, an ANOVA calculation is performed (for example, a two tailed t-test, Wilcoxon rank-sum test, Kolmogorov-Smirnov test, etc.), in which the expression values of the genes in the two opposing arms are compared to one another. For example, if the two tailed t-test is used to determine whether the mean log(10) ratio of the genes in the "up" arm is significantly different than the mean log(10) ratio of the genes in the "down" arm, a p-value of $<0.05$ indicates that the signature in the individual sample is significantly different from the standard or control. If the signature score for a sample is above a pre-determined threshold, then the sample is considered to have deregulation of the RAS signaling pathway. The pre-determined threshold may be 0, or may be the mean, median, or a percentile of signature scores of a collection of samples or a pooled sample used as a standard or control. In an alternative embodiment, a subset of at least 3, 5, 10, 15, 20, 25, 30, 35, 40, 45, 50, 55, 60, 65, 70, 75, 80, 85, 90, 95, or 100 biomarkers, drawn from the "up" arm (see Table 2a) and a subset of at least 3, 5, 10, 15, 20, 25, 30, 35, or 40 biomarkers from the "down" arm (see Table 2b) can be used may be used for calculating this signature score. It will be recognized by those skilled in the art that other differential expression values, besides log(10)ratio may be used for calculating a signature score, as long as the value represents an objective measurement of transcript abundance of the biomarker gene. Examples include, but are not limited to: xdev, error-weighted log (ratio), and mean subtracted log(intensity).

The above described methods of using the biomarker sets may also be used to analyze a sample from an individual and then rank order the sample according to its RAS pathway deregulation status. A sample may be compared to a reference template to determine a ranking order. A sample may also be compared to a pre-determined threshold, such as a mean expression value of a biomarker set or subset for a reference sample, to determine a ranking order. A reference sample may be a "deregulated" or "regulated" RAS signaling pathway sample. A sample may also be compared to a pool of samples, and rank ordered by comparison with a pre-determined threshold of the pool of samples, such as the mean, median, or percentile expression value of a biomarker set or subset. A sample may also be rank ordered according to its signature score.

3.4.2 Methods of Predicting Response to Treatment and Assigning Treatment

The invention provides a set of biomarkers useful for distinguishing samples from those patients who are predicted to respond to treatment with an agent that modulates the RAS signaling pathway from patients who are not predicted to respond to treatment an agent that modulates the RAS signaling pathway. Thus, the invention further provides a method for using these biomarkers for determining whether an individual with cancer is a predicted responder to treatment with an agent that modulates the RAS signaling pathway. In one embodiment, the invention provides for a method of predicting response of a cancer patient to an agent that modulates the RAS signaling pathway comprising (1) comparing the level of expression of the biomarkers listed in Tables 2a and 2b in a sample taken from the individual to the level of expression of the same biomarkers in a standard or control, where the standard or control levels represent those found in a sample having a deregulated RAS signaling; and (2) determining whether the level of the biomarker-related polynucleotides in the sample from the individual is significantly different than that of the control, wherein if no substantial difference is found, the patient is predicted to respond to treatment with an agent that modulates the RAS signaling pathway, and if a substantial difference is found, the patient is predicted not to respond to treatment with an agent that modulates the RAS signaling pathway. Persons of skill in the art will readily see that the standard or control levels may be from a tumor sample having a regulated RAS signaling pathway. In a more specific embodiment, both controls are run. In case the pool is not pure "RAS regulated" or "RAS deregulated," a set of experiments of individuals with known responder status should be hybridized against the pool to define the expression templates for the predicted responder and predicted non-responder group. Each individual with unknown outcome is hybridized against the same pool and the resulting expression profile is compared to the templates to predict its outcome.

RAS signaling pathway deregulation status of a tumor may indicate a subject that is responsive to treatment with an agent that modulates the RAS signaling pathway and not responsive to PI3K pathway inhibitors. Therefore, the invention provides for a method of determining or assigning a course of treatment of a cancer patient, comprising determining whether the level of expression of the 147 biomarkers of Table 2a and 2b, or a subset thereof, correlates with the level of these biomarkers in a sample representing deregulated RAS signaling pathway status or regulated RAS signaling pathway status; and determining or assigning a course of treatment, wherein if the expression correlates with the deregulated RAS signaling pathway status pattern, the tumor is treated with an agent that modulates the RAS signaling pathway and not treated with a PI3K pathway agent.

As with the diagnostic biomarkers, the method can use the fullest of biomarkers (*i.e.*, the complete set of biomarkers from Tables 2a and 2b). However, subsets of at least 14, 15, 20, 25, 30, 35, 40, 45, 50, 55, 60, 65, 70, 75, 80, 85, 90, 95, 100, 105, 110, 115, 120, 125, 130, 135, or 140 of the 147 biomarkers may be used, wherein at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, or 42 biomarkers of each subset is selected from Table 2b. Alternatively, a subset of at least 3, 5, 10, 15, 20, 25, 30, 35, 40, 45, 50, 55, 60, 65, 70, 75, 80, 85, 90, 95, or 100 biomarkers, drawn from the "up" arm (see Table 2a) and a subset of at least 3, 5, 10, 15, 20, 25, 30, 35, or 40 biomarkers from the "down" arm (see Table 2b) can be used.

Classification of a sample as "predicted responder" or "predicted non-responder" is accomplished substantially as for the diagnostic biomarkers described above, wherein a template is generated to which the biomarker expression levels in the sample are compared.

In another embodiment, the above method of using RAS pathway regulation status of an individual to predict treatment response or assign treatment uses the two "arms" of the 147 biomarkers. The "up" arm comprises the 105 genes whose expression goes up with RAS pathway activation (see Table 2a), and the "down" arm comprises the 42 genes whose expression goes down with RAS pathway activation (see Table 2b). When comparing an individual sample with a standard or control, the expression value of gene X in the sample is compared to the expression value of gene X in the standard or control. For each gene in the set of biomarkers, log(10) ratio is created for the expression value in the individual sample relative to the standard or control. A signature "score" is calculated by determining the mean log(10) ratio of the genes in the "up" and then subtracting the mean log(10) ratio of the genes in the "down" arm. If the signature score is above a pre-determined threshold, then the sample is considered to have deregulation of the RAS signaling pathway. The pre-determined threshold may be 0, or may be the mean, median, or a percentile of signature scores of a collection of samples or a pooled sample used as a standard of control. To determine if this signature score is significant, an ANOVA calculation is performed (for example, a two tailed t-test, Wilcoxon rank-sum test, Kolmogorov-Smirnov test, etc.), in which the expression values of the genes in the two opposing arms are compared to one another. For example, if the two tailed t-test is used to determine whether the mean log(10) ratio of the genes in the "up" arm is significantly different than the mean log(10) ratio of the genes in the "down" arm, a p-value of $<0.05$ indicates that the signature in the individual sample is significantly different from the standard or control. In an alternative embodiment, a subset of at least 3, 5, 10, 15, 20, 25, 30, 35, 40, 45, 50, 55, 60, 65, 70, 75, 80, 85, 90, 95, or 100 biomarkers, drawn from the "up" arm (see Table 2a) and a subset of at least 3, 5, 10, 15, 20, 25, 30, 35, or 40 biomarkers from the "down" arm (see Table 2b) can be used may be used for calculating this signature score. It will be recognized by those skilled in the art that other differential expression values, besides log(10)ratio may be used for calculating a signature score, as long as the value represents an objective measurement of transcript abundance of the biomarker gene. Examples include, but are not limited to: xdev, error-weighted log (ratio), and mean subtracted log(intensity).

The use of the biomarkers is not restricted to predicting response to agents that modulate RAS signaling pathway for cancer-related conditions, and may be applied in a variety of phenotypes or conditions, clinical or experimental, in which gene expression plays a role. Where a set of biomarkers has been identified that corresponds to two or more phenotypes, the biomarker sets can be used to distinguish these phenotypes. For example, the phenotypes may be the diagnosis and/or prognosis of clinical states or phenotypes associated with cancers and other disease conditions, or other physiological conditions, prediction of response to agents that modulate pathways other than the RAS signaling pathway, wherein the expression level data is derived from a set of genes correlated with the particular physiological or disease condition.

### 3.4.3 Method of Determining Whether an Agent Modulates the RAS Signaling Pathway

The invention provides a set of biomarkers useful for and methods of using the biomarkers for identifying or evaluating an agent that is predicted to modify or modulate the RAS signaling pathway in a subject. "RAS signaling pathway" or "RAS pathway" is initiated by growth factors through receptor tyrosine kinases. The autophosphorylated receptor binds to the SH2 domain of GRB2. Through its SH3 domain, GRB2 is bound to SOS, so activation of the receptor tyrosine kinase results in recruitment of SOS to the plasma membrane, where RAS is also localized as a result of farnesylation. The increased proximity of SOS to RAS results in increased nucleotide exchange on RAS, with GDP being replaced with GTP. GTP-bound RAS is able to bind and activate several families of effector enzymes (such as the RAF, PI3K, RALGDS, and PLCε pathways)(reviewed in Downward, 2003, Nat. Rev. Cancer 3:11-22)(See Figure 1). This signaling cascade affects multiple cellular processes, such as cell-cycle progression, transcription, survival, cytoskeletal signals, translation, vesicle transport, and calcium signaling.

Agents affecting the RAS signaling pathway include small molecule compounds; proteins or peptides (including antibodies); siRNA, shRNA, or microRNA molecules; or any other agents that modulate one or more genes or proteins that function within the RAS signaling pathway or other signaling pathways that interact with the RAS signaling pathway.

"RAS pathway agent" refers to an agent that modulates signaling through the RAS pathway. A RAS pathway inhibitor inhibits signaling through the RAS pathway. Molecular targets of such agents include, but are not limited to: RAS, RAF, MEK, MAPK, ELK1, and the genes listed in the Table 1. Such agents are well known in the art and include, but are not limited to: RAS inhibitors ISIS 2503 and farnesyl transferase inhibitor R115777, L731735, SCH 66336, and BMS214662; Raf inhibitors ISIS 5132 and BAY43-9006; MEK inhibitors PD184322, CI-1040, and PD0325901 (reviewed in Dancey, 2002, Curr. Pharm. Des. 8:2259-2267; Sebolt-Leopold et al., 1999, Nat. Med 5:810-816; Downward, 2003, Nat. Rev. Cancer 3:11-22; Barrett et al., 2008, Bioorg. Med. Chem. Lett. 18:6501-4).

In one embodiment, the method for measuring the effect or determining whether an agent modulates the RAS signaling pathway comprises: (1) comparing the level of expression of the biomarkers listed in Table 2a and 2b in a sample treated with an agent to the level of expression of the same biomarkers in a standard or control, wherein the standard or control levels represent those found in a vehicle-treated sample; and (2) determining whether the level of the biomarker-related polynucleotides in the treated sample is significantly different than that of the vehicle-treated control, wherein if no substantial difference is found, the agent is predicted not to modulate the RAS signaling pathway, and if a substantial difference is found, the agent is predicted to modulate the RAS signaling pathway.

The method can use the fullest of biomarkers (i.e., the complete set of biomarkers from Tables 2a and 2b). However, subsets of at least 10, 15, 20, 25, 30, 35,40,45,50, 55, 60, 65, 70, 75, 80, 85 ,90, 95,100,105,110,115,120, 125,130,135, or 140 of the 147 biomarkers may be used, wherein at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, or 42 biomarkers of each subset is selected from Table 2b. Alternatively, a subset of at least 3, 5, 10, 15, 20, 25, 30, 35, 40, 45, 50, 55,60, 65, 70, 75, 80, 85, 90, 95, or 100 biomarkers, drawn from the "up" arm (see Table 2a) and a subset of at least 3, 5, 10, 15, 20, 25, 30, 35, or 40 biomarkers from the "down" arm (see Table 2b) can be used.

In another embodiment, the above method of measuring the effect of an agent on the RAS signaling pathway uses the two "arms" of the 147 biomarkers. The "up" arm comprises the 105 genes whose expression goes up with RAS pathway activation (see Table 2a), and the "down" arm comprises the 42 genes whose expression goes down with RAS pathway activation (see Table 2b). When comparing an individual sample with a standard or control, the expression value of gene X in the sample is compared to the expression value of gene X in the standard or control. For each gene in the set of biomarkers, a log(10)ratio is created for the expression value in the individual sample relative to the standard or control. A signature "score" is calculated by determining the mean log(10) ratio of the genes in the "up" arm and the subtracting the mean log(10)ratio of the genes in the "drown" arm. If the signature score is above a pre-determined threshold, then the sample is considered to have deregulation of the RAS signaling pathway (i.e., the agent modulates the RAS signaling pathway). The pre-determined threshold may be 0, or may be the mean, median, or a percentile of signature scores of a collection of samples or a pooled sample used as a standard or control. To determine if this signature score is significant, an ANOVA calculation is performed (for example, a two tailed t-test, Wilcoxon rank-sum test, Kolmogorov-Smirnov test, etc.), in which the expression values of the genes in the two opposing arms are compared to one another. For example, if the two tailed t-test is used to determine whether the mean log(10) ratio of the genes in the "up" arm is significantly different than the mean log(10) ratio of the genes in the "down" arm, a p-value of<0.05 indicates that the signature in the individual sample is significantly different from the standard or control. In an alternative embodiment, a subset of at least 3, 5,10,15,20,25, 30, 35,40,45, 50, 55,60, 65,70,75, 80, 85, 90, 95, or 100 biomarkers, drawn from the "up" arm (see Table 2a) and a subset of at least 3, 5, 10, 5, 20, 25, 30, 35, or 40 biomarkers from the "down" arm (see Table 2b) can be used may be used for calculating this signature score. It will be recognized by those skilled in the art that other differential expression values, besides log(10)ratio may be used for calculating a signature score, as long as the value represents an objective measurement of transcript abundance of the biomarker gene. Examples include, but are not limited to: xdev, error-weighted log (ratio), and mean subtracted log(intensity).

The above described methods of using the biomarker sets may also be used to rank order agents according to their effect on the biomarker sets or subsets. For example, agents may be ranked according to the change induced in differential expression value (for example, mean expression value of the biomarker set or subset or signature score) in the biomarker set or subsets. Candidate agents may also be ranked by comparison with agents known to modify the particular pathway in question.

3.4.4 Method of Measuring Pharmacodynamic Effect of an Agent

The invention provides a set of biomarkers useful for measuring the pharmacodynamic effect of an agent on the RAS signaling pathway. The biomarkers provided may be used to monitor modulation of the RAS signaling pathway at various time points following treatment with said agent in a patient or sample. Thus, the invention further provides a method for using these biomarkers as an early evaluation for efficacy of an agent which modulates the RAS signaling pathway. In one embodiment, the invention provides for a method of measuring pharmacodynamic effect of an agent that modulates the RAS signaling pathway in patient or sample comprising: (1) comparing the level of expression of the biomarkers listed in Table 2a and 2b in a sample treated with an agent to the level of expression of the same biomarkers in a standard or control, wherein the standard or control levels represent those found in a vehicle-treated sample; and (2) determining whether the level of the biomarker-related polynucleotides in the treated sample is significantly different than that of the vehicle-treated control, wherein if no substantial difference is found, the agent is predicted not to have an pharmacodynamic effect on the RAS signaling pathway, and if a substantial difference is found, the agent is predicted to have an pharmacodynamic effect on the RAS signaling pathway. The method can use the fullest of biomarkers (i.e., the complete set of biomarkers from Tables 2a and 2b). However, subsets of at least 10, 15, 20, 25, 30, 35, 40, 45, 50, 55, 60, 65, 70, 75, 80, 85 ,90, 95, 100, 105, 110, 115, 120, 125, 130, 135, or 140 of the 147 biomarkers may be used to monitor pharmacodynamic activity of an agent on the RAS signaling pathway, wherein at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, or 42 biomarkers of each subset is selected from Table 2b. Alternatively, a subset of at least 3, 5, 10, 15, 20, 25, 30, 35, 40, 45, 50, 55, 60, 65, 70, 75, 80, 85, 90, 95, or 100 biomarkers, drawn from the "up" arm (see Table 2a) and a subset of at least 3, 5, 10, 15, 20, 25, 30, 35, or 40 biomarkers from the "down" arm (see Table 2b) can be used to monitor pharmacodynamic activity of an agent on the RAS signaling pathway.

In another embodiment, the above method of measuring pharmacodynamic activity of an agent on the growth factor signaling pathway uses the two "arms" of the 147 biomarkers. The "up" arm comprises the 105 genes whose expression goes up with RAS pathway activation (see Table 2a), and the "down" arm comprises the 42 genes whose expression goes down with RAS pathway activation (see Table 2b). When comparing an individual sample with a standard or control, the expression value of gene X in the sample is compared to the expression value of gene X in the standard or control. For each gene in the set of biomarkers, a log(10)ratio is created for the expression value in the individual sample relative to the standard or control. A signature "score" is calculated by determining the mean log(10) ratio of the genes in the "up" arm and the subtracting the mean log(10)ratio of the genes in the "down" arm. If the signature score is above a pre-determined threshold, then the sample is considered to have deregulation of the growth factor signaling pathway. The pre-determined threshold may be 0, or may be the mean, median, or a percentile of signature scores of a collection of samples or a pooled sample used as a standard or control. To determine if this signature score is significant, an ANOVA calculation is performed (for example, a two tailed t-test, Wilcoxon rank-sum test, Kolmogorov-Smirnov test, etc.), in which the expression values of the genes in the two opposing arms are compared to one another. For example, if the two tailed t-test is used to determine whether the mean log(10) ratio of the genes in the "up" arm is significantly different than the mean log(10) ratio of the genes in the "down" arm, a p-value of <0.05 indicates that the signature in the individual sample is significantly different from the standard or control. Alternatively, a subset of at least 3, 5, 10, 15, 20, 25, 30, 35, 40,45, 50, 55, 60, 65, 70, 75, 80, 85, 90, 95, or 100 biomarkers, drawn from the "up" arm (see Table 2a) and a subset of at least 3, 5, 10, 15, 20, 25, 30, 35, or 40 biomarkers from the "down" arm (see Table 2b) can be used may be used for calculating this signature score. It will be recognized by those skilled in the art that other differential expression values, besides log(10)ratio may be used for calculating a signature score, as long as the value represents an objective measurement of transcript abundance of the biomarker gene. Examples include, but are not limited to: xdev, error-weighted log (ratio), and mean subtracted log(intensity).

The use of the biomarkers is not restricted to measure the pharmacodynamic effect of an agent on the RAS signaling pathway for cancer-related conditions, and may be applied in a variety of phenotypes or conditions, clinical or experimental, in which gene expression plays a role. Where a set of biomarkers has been identified that corresponds to two or more phenotypes, the biomarker sets can be used to distinguish these phenotypes. For example, the phenotypes may be the diagnosis and/or prognosis of clinical states or phenotypes associated with cancers and other disease conditions, or other physiological conditions, prediction of response to agents that modulate pathways other than the RAS signaling pathway, wherein the expression level data is derived from a set of genes correlated with the particular physiological or disease condition.

3.4.5 Improving Sensitivity to Expression Level Differences

In using the biomarkers disclosed herein, and, indeed, using any sets of biomarkers to differentiate an individual or subject having one phenotype from another individual or subject having a second phenotype, one can compare the absolute expression of each of the biomarkers in a sample to a control; for example, the control can be the average level of expression of each of the biomarkers, respectively, in a pool of individuals or subjects. To increase the sensitivity of the comparison, however, the expression level values are preferably transformed in a number of ways.

For example, the expression level of each of the biomarkers can be normalized by the average expression level of all markers the expression level of which is determined, or by the average expression level of a set of control genes. Thus, in one embodiment, the biomarkers are represented by probes on a microarray, and the expression level of each of the biomarkers is normalized by the mean or median expression level across all of the genes represented on the microarray, including any non-biomarker genes. In a specific embodiment, the normalization is carried out by dividing the median or mean level of expression of all of the genes on the microarray. In another embodiment, the expression levels of the biomarkers are normalized by the mean or median level of expression of a set of control biomarkers. In a specific embodiment, the control biomarkers comprise a set of housekeeping genes. In another specific embodiment, the normalization is accomplished by dividing by the median or mean expression level of the control genes.

The sensitivity of a biomarker-based assay will also be increased if the expression levels of individual biomarkers are compared to the expression of the same biomarkers in a pool of samples. Preferably, the comparison is to the mean or median expression level of each the biomarker genes in the pool of samples. Such a comparison may be accomplished, for example, by dividing by the mean or median expression level of the pool for each of the biomarkers from the expression level each of the biomarkers in the sample. This has the effect of accentuating the relative differences in expression between biomarkers in the sample and markers in the pool as a whole, making comparisons more sensitive and more likely to produce meaningful results that the use of absolute expression levels alone. The expression level data may be transformed in any convenient way; preferably, the expression level data for all is log transformed before means or medians are taken.

In performing comparisons to a pool, two approaches may be used. First, the expression levels of the markers in the sample may be compared to the expression level of those markers in the pool, where nucleic acid derived from the sample and nucleic acid derived from the pool are hybridized during the course of a single experiment. Such an approach requires that new pool nucleic acid be generated for each comparison or limited numbers of comparisons, and is therefore limited by the amount of nucleic acid available. Alternatively, and preferably, the expression levels in a pool, whether normalized and/or transformed or not, are stored on a computer, or on computer-readable media, to be used in comparisons to the individual expression level data from the sample (i.e., single-channel data).

Thus, the current invention provides the following method of classifying a first cell or organism as having one of at least two different phenotypes, where the different phenotypes comprise a first phenotype and a second phenotype. The level of expression of each of a plurality of genes in a first sample from the first cell or organism is compared to the level of expression of each of said genes, respectively, in a pooled sample from a plurality of cells or organisms, the plurality of cells or organisms comprising different cells or organisms exhibiting said at least two different phenotypes, respectively, to produce a first compared value. The first compared value is then compared to a second compared value, wherein said second compared value is the product of a method comprising comparing the level of expression of each of said genes in a sample from a cell or organism characterized as having said first phenotype to the level of expression of each of said genes, respectively, in the pooled sample. The first compared value is then compared to a third compared value, wherein said third compared value is the product of a method comprising comparing the level of expression of each of the genes in a sample from a cell or organism characterized as having the second phenotype to the level of expression of each of the genes, respectively, in the pooled sample. Optionally, the first compared value can be compared to additional compared values, respectively, where each additional compared value is the product of a method comprising comparing the level of expression of each of said genes in a sample from a cell or organism characterized as having a phenotype different from said first and second phenotypes but included among the at least two different phenotypes, to the level of expression of each of said genes, respectively, in said pooled sample. Finally, a determination is made as to which of said second, third, and, if present, one or more additional compared values, said first compared value is most similar, wherein the first cell or organism is determined to have the phenotype of the cell or organism used to produce said compared value most similar to said first compared value.

In a specific embodiment of this method, the compared values are each ratios of the levels of expression of each of said genes. In another specific embodiment, each of the levels of expression of each of the genes in the pooled sample is normalized prior to any of the comparing steps. In a more specific embodiment, the normalization of the levels of expression is carried out by dividing by the median or mean level of the expression of each of the genes or dividing by the mean or median level of expression of one or more housekeeping genes in the pooled sample from said cell or organism. In another specific embodiment, the normalized levels of expression are subjected to a log transform, and the comparing steps comprise subtracting the log transform from the log of the levels of expression of each of the genes in the sample. In another specific embodiment, the two or more different phenotypes are different regulation status of the RAS signaling pathway. In still another specific embodiment, the two or more different phenotypes are different predicted responses to treatment with an agent that modulates the RAS signaling pathway. In yet another specific embodiment, the levels of expression of each of the genes, respectively, in the pooled sample or said levels of expression of each of said genes in a sample from the cell or organism characterized as having the first phenotype, second phenotype, or said phenotype different from said first and second phenotypes, respectively, are stored on a computer or on a computer-readable medium.

In another specific embodiment, the two phenotypes are deregulated or regulated RAS signaling pathway status. In another specific embodiment, the two phenotypes are predicted RAS signaling pathway-agent responder status. In yet another specific embodiment, the two phenotypes are pharmacodynamic effect and no pharmcodynamic effect of an agent on the RAS signaling pathway.

In another specific embodiment, the comparison is made between the expression of each of the genes in the sample and the expression of the same genes in a pool representing only one of two or more phenotypes. In the context of RAS signaling pathway status-correlated genes, for example, one can compare the expression levels of RAS signaling pathway regulation status-related genes in a sample to the average level of the expression of the same genes in a "deregulated" pool of samples (as opposed to a pool of samples that include samples from patients having regulated and deregulated RAS signaling pathway status). Thus, in this method, a sample is classified as having a deregulated RAS signaling pathway status if the level of expression of prognosis-correlated genes exceeds a chosen coefficient of correlation to the average "deregulated RAS signaling pathway" expression profile (i.e., the level of expression of RAS signaling pathway status-correlated genes in a pool of samples from patients having a "deregulated RAS signaling pathway status." Patients or subjects whose expression levels correlate more poorly with the "deregulated RAS signaling pathway" expression profile (i.e., whose correlation coefficient fails to exceed the chosen coefficient) are classified as having a regulated RAS signaling pathway status.

Of course, single-channel data may also be used without specific comparison to a mathematical sample pool. For example, a sample may be classified as having a first or a second phenotype, wherein the first and second phenotypes are related, by calculating the similarity between the expression of at least 5 markers in the sample, where the markers are correlated with the first or second phenotype, to the expression of the same markers in a first phenotype template and a second phenotype template, by (a) labeling nucleic acids derived from a sample with a fluorophore to obtain a pool of fluorophore-labeled nucleic acids; (b) contacting said fluorophore-labeled nucleic acid with a microarray under conditions such that hybridization can occur, detecting at each of a plurality of discrete loci on the microarray a flourescent emission signal from said fluorophore-labeled nucleic acid that is bound to said microarray under said conditions; and (c) determining the similarity of marker gene expression in the individual sample to the first and second templates, wherein if said expression is more similar to the first template, the sample is classified as having the first phenotype, and if said expression is more similar to the second template, the sample is classified as having the second phenotype.

3.4.6 Methods for Classification of Expression Profiles

In preferred embodiments, the methods of the invention use a classifier for predicting RAS signaling pathway regulation status of a sample, predicting response to agents that modulate the RAS signaling pathway, assigning treatment to a subject, and/or measuring pharmacodynamic effect of an agent. The classifier can be based on any appropriate pattern recognition method that receives an input comprising a biomarker profile and provides an output comprising data indicating which patient subset the patient belongs. The classifier can be trained with training data from a training population of subjects. Typically, the training data comprise for each of the subjects in the training population a training marker profile comprising measurements of respective gene products of a plurality of genes in a suitable sample taken from the patient and outcome information, i.e., deregulated or regulated RAS signaling pathway status.

In preferred embodiments, the classifier can be based on a classification (pattern recognition) method described below, e.g., profile similarity; artificial neural network); support vector machine (SVM); logic regression, linear or quadratic discriminant analysis, decision trees, clustering, principal component analysis, nearest neighbor classifier analysis (described *infra*). Such classifiers can be trained with the training population using methods described in the relevant sections, *infra*.

The biomarker profile can be obtained by measuring the plurality of gene products in a cell sample from the subject using a method known in the art, e.g., a method described *infra*.

Various known statistical pattern recognition methods can be used in conjunction with the present invention. A classifier based on any of such methods can be constructed using the biomarker profiles and RAS pathway signalling status data of training patients. Such a classifier can then be used to evaluate the RAS pathway signalling status of a patient based on the patient's biomarker profile. The methods can also be used to identify biomarkers that discriminate between different RAS signalling pathway regulation status using a biomarker profile and RAS signalling pathway regulation data of training patients.

A. Profile Matching

A subject can be classified by comparing a biomarker profile obtained in a suitable sample from the subject with a biomarker profile that is representative of a particular phenotypic state. Such a marker profile is also termed a "template profile" or a "template." The degree of similarity to such a template profile provides an evaluation of the subject' s phenotype. If the degree of similarity of the subject marker profile and a template profile is above a predetermined threshold, the subject is assigned the classification represented by the template. For example, a subject's outcome prediction can be evaluated by comparing a biomarker profile of the subject to a predetermined template profile corresponding to a given phenotype or outcome, e.g., a RAS signalling pathway template comprising measurements of the plurality of biomarkers which are representative of levels of the biomarkers in a plurality of subjects that have tumors with deregulated RAS signalling pathway status.

In one embodiment, the similarity is represented by a correlation coefficient between the subject's profile and the template. In one embodiment, a correlation coefficient above a correlation threshold indicates a high similarity, whereas a correlation coefficient below the threshold indicates a low similarity.

In a specific embodiment, $P_i$ measures the similarity between the subject's profile $\vec{y}$ and a template profile comprising measurements of marker gene products representative of measurements of marker gene products in subjects having a particular outcome or phenotype, e.g., deregulated RAS signalling pathway status $\vec{z}_1$ or a regulated RAS signalling pathway status $\vec{z}_2$. Such a coefficient, $P_i$, can be calculated using the following equation:

$$P_i = (\vec{z}_i \bullet \vec{y})/(\|\vec{z}_i\| \cdot \|\vec{y}\|)$$

where $i$ designates the $i$th template. Thus, in one embodiment, $\vec{y}$ is classified as a deregulated RAS signalling pathway profile if $P_1$ is greater than a selected correlation threshold. In another embodiment, $\vec{y}$ is classified as a regulated RAS signalling pathway profile if $P_2$ is greater than a selected correlation threshold. In preferred embodiments, the correlation threshold is set as 0.3, 0.4, 0.5 or 0.6. In another embodiment, $\vec{y}$ is classified as a deregulated RAS signalling pathway profile if $P_1$ is greater than $P_2$, whereas $\vec{y}$ is classified as a regulated RAS signalling pathway profile if $P_1$ is less than $P_2$.

In another embodiment, the correlation coefficient is a weighted dot product of the patient's profile $\vec{y}$ and a template profile, in which measurements of each different marker is assigned a weight.

In another embodiment, similarity between a patient's profile and a template is represented by a distance between the patient's profile and the template. In one embodiment, a distance below a given value indicates high similarity, whereas a distance equal to or greater than the given value indicates low similarity.

In one embodiment, the Euclidian distance according to the formula

$$D_i = \|\vec{y} - \vec{z}_i\|$$

is used, where $D_i$ measures the distance between the subject's profile $\vec{y}$ and a template profile comprising measurements of marker gene products representative of measurements of marker gene products in subjects having a particular RAS signaling pathway regulation status, e.g., the deregulated RAS signaling pathway $\vec{z}_1$, or the regulated RAS signaling pathway template $\vec{z}_2$. In other embodiments, the Euclidian distance is squared to place progressively greater weight on cellular constituents that are further apart. In alternative embodiments, the distance measure $D_i$ is the Manhattan distance provide by

$$D_i = \sum_n |y(n) - z_i(n)|$$

where $y(n)$ and $z_i(n)$ are respectively measurements of the $n$th marker gene product in the subject's profile $\vec{y}$ and a template profile.

In another embodiment, the distance is defined as $D_i = 1 - P_i$, where $P_i$ is the correlation coefficient or normalized dot product as described above.

In still other embodiments, the distance measure may be the Chebychev distance, the power distance, and percent disagreement, all of which are well known in the art.

B. Artificial Neural Network

In some embodiments, a neural network is used. A neural network can be constructed for a selected set of molecular markers of the invention. A neural network is a two-stage regression or classification model. A neural network has a layered structure that includes a layer of input units (and the bias) connected by a layer of weights to a layer of output units. For regression, the layer of output units typically includes just one output unit. However, neural networks can handle multiple quantitative responses in a seamless fashion.

In multilayer neural networks, there are input units (input layer), hidden units (hidden layer), and output units (output layer). There is, furthermore, a single bias unit that is connected to each unit other than the input units. Neural networks are described in Duda et al., 2001, Pattern Classification, Second Edition, John Wiley & Sons, Inc., New York; and Hastie et al., 2001, The Elements of Statistical Learning, Springer-Verlag, New York.

The basic approach to the use of neural networks is to start with an untrained network, present a training pattern, e.g., biomarker profiles from training patients, to the input layer, and to pass signals through the net and determine the output, e.g., the RAS signaling pathway regulation status in the training patients, at the output layer. These outputs are then compared to the target values; any difference corresponds to an error. This error or criterion function is some scalar function of the weights and is minimized when the network outputs match the desired outputs. Thus, the weights are adjusted to reduce this measure of error. For regression, this error can be sum-of-squared errors. For classification, this error can be either squared error or cross-entropy (deviation). See, *e.g.*, Hastie et al., 2001, The Elements of Statistical Learning, Springer-Verlag, New York.

Three commonly used training protocols are stochastic, batch, and on-line. In stochastic training, patterns are chosen randomly from the training set and the network weights are updated for each pattern presentation. Multilayer nonlinear networks trained by gradient descent methods such as stochastic back-propagation perform a maximum-likelihood estimation of the weight values in the model defined by the network topology. In batch training, all patterns are presented to the network before learning takes place. Typically, in batch training, several passes are made through the training data. In online training, each pattern is presented once and only once to the net.

In some embodiments, consideration is given to starting values for weights. If the weights are near zero, then the operative part of the sigmoid commonly used in the hidden layer of a neural network (see, *e.g.*, Hastie et al., 2001, The Elements of Statistical Learning, Springer-Verlag, New York) is roughly linear, and hence the neural network collapses into an approximately linear model. In some embodiments, starting values for weights are chosen to be random values near zero. Hence the model starts out nearly linear, and becomes nonlinear as the weights increase. Individual units localize to directions and introduce nonlinearities where needed. Use of exact zero weights leads to zero derivatives and perfect symmetry, and the algorithm never moves. Alternatively, starting with large weights often leads to poor solutions.

Since the scaling of inputs determines the effective scaling of weights in the bottom layer, it can have a large effect on the quality of the final solution. Thus, in some embodiments, at the outset all expression values are standardized to have mean zero and a standard deviation of one. This ensures all inputs are treated equally in the regularization process, and allows one to choose a meaningful range for the random starting weights. With standardization inputs, it is typical to take random uniform weights over the range [-0.7, +0.7].

A recurrent problem in the use of networks having a hidden layer is the optimal number of hidden units to use in the network. The number of inputs and outputs of a network are determined by the problem to be solved. In the present invention, the number of inputs for a given neural network can be the number of molecular markers in the selected set of molecular markers of the invention. The number of output for the neural network will typically be just one. However, in some embodiment more than one output is used so that more than just two states can be defined by the network. If too many hidden units are used in a neural network, the network will have too many degrees of freedom and is trained too long, there is a danger that the network will overfit the data. If there are too few hidden units, the training set cannot be learned. Generally speaking, however, it is better to have too many hidden units than too few. With too few hidden units, the model might not have enough flexibility to capture the nonlinearities in the data; with too many hidden units, the extra weight can be shrunk towards zero if appropriate regularization or pruning, as described below, is used. In typical embodiments, the number of hidden units is somewhere in the range of 5 to 100, with the number increasing with the number of inputs and number of training cases.

One general approach to determining the number of hidden units to use is to apply a regularization approach. In the regularization approach, a new criterion function is constructed that depends not only on the classical training error, but also on classifier complexity. Specifically, the new criterion function penalizes highly complex models; searching for the minimum in this criterion is to balance error on the training set with error on the training set plus a regularization term, which expresses constraints or desirable properties of solutions:

$$J = J_{pat} + \lambda J_{reg}.$$

The parameter $\lambda$ is adjusted to impose the regularization more or less strongly. In other words, larger values for $\lambda$ will tend to shrink weights towards zero: typically cross-validation with a validation set is used to estimate $\lambda$. This validation set can be obtained by setting aside a random subset of the training population. Other forms of penalty can also be used, for example the weight elimination penalty (see, *e.g.*, Hastie et al., 2001, The Elements of Statistical Learning, Springer-Verlag, New York).

Another approach to determine the number of hidden units to use is to eliminate - prune - weights that are least needed. In one approach, the weights with the smallest magnitude are eliminated (set to zero). Such magnitude-based pruning can work, but is nonoptimal; sometimes weights with small magnitudes are important for learning and training data. In some embodiments, rather than using a magnitude-based pruning approach, Wald statistics are computed. The fundamental idea in Wald Statistics is that they can be used to estimate the importance of a hidden unit (weight) in a model. Then, hidden units having the least importance are eliminated (by setting their input and output weights to zero). Two algorithms in this regard are the *Optimal Brain Damage* (OBD) and the *Optimal Brain Surgeon* (OBS) algorithms that use second-order approximation to predict how the training error depends upon a weight, and eliminate the weight that leads to the smallest increase in training error.

Optimal Brain Damage and Optimal Brain Surgeon share the same basic approach of training a network to local minimum error at weight **w**, and then pruning a weight that leads to the smallest increase in the training error. The predicted functional increase in the error for a change in full weight vector $\delta$**w** is:

$$\delta J = \left(\frac{\partial J}{\partial w}\right)^t \cdot \delta w + \frac{1}{2}\delta w^t \cdot \frac{\partial^2 J}{\partial w^2} \cdot \delta w + O\left(\|\delta w\|^3\right)$$

where $\frac{\partial^2 J}{\partial w^2}$ is the Hessian matrix. The first term vanishes because we are at a local minimum in error; third and higher order terms are ignored. The general solution for minimizing this function given the constraint of deleting one weight is:

$$\delta w = -\frac{w_q}{[\mathbf{H}^{-1}]_{qq}}\mathbf{H}^{-1} \cdot u_q \text{ and } L_q = \frac{1}{2} - \frac{w_q^2}{[\mathbf{H}^{-1}]_{qq}}$$

Here, $u_q$ is the unit vector along the qth direction in weight space and $L_q$ is approximation to the saliency of the weight *q* - the increase in training error if weight *q* is pruned and the other weights updated $\delta$w. These equations require the inverse of **H**. One method to calculate this inverse matrix is to start with a small value, $H_0^{-1} = \alpha^{-1}\mathbf{I},$ where $\alpha$ is a small parameter - effectively a weight constant. Next the matrix is updated with each pattern according to

$$\mathbf{H}_{m+1}^{-1} = \mathbf{H}_{m}^{-1} - \frac{\mathbf{H}_{m}^{-1}\mathbf{X}_{m+1}\mathbf{X}_{m+1}^{T}\mathbf{H}_{m}^{-1}}{\frac{n}{a_m} + \mathbf{X}_{m+1}^{T}\mathbf{H}_{m}^{-1}\mathbf{X}_{m+1}}$$

where the subscripts correspond to the pattern being presented and $a_m$ decreases with $m$. After the full training set has been presented, the inverse Hessian matrix is given by $\mathbf{H}^{-1} = H_n^{-1}$. In algorithmic form, the Optimal Brain Surgeon method is:

begin initialize $n_H$, **w**, θ

train a reasonably large network to minimum error

do compute $\mathbf{H}^{-1}$ by Eqn. 1

$$q^* \leftarrow \arg\min_q \; w_q^2/(2[H^{-1}]_{qq}) \quad \text{(saliency } L_q\text{)}$$

$$\mathbf{w} \leftarrow \mathrm{w}- \frac{w_{q^*}}{[H^{-1}]_{q^*q^*}} H^{-1}e_{q^*} \quad \text{(saliency } L_q\text{)}$$

until $J(\mathbf{w}) > \theta$

return **w**

end

The Optimal Brain Damage method is computationally simpler because the calculation of the inverse Hessian matrix in line 3 is particularly simple for a diagonal matrix. The above algorithm terminates when the error is greater than a criterion initialized to be θ. Another approach is to change line 6 to terminate when the change in J(**w**) due to elimination of a weight is greater than some criterion value.

In some embodiments, a back-propagation neural network (see, for example Abdi, 1994, "A neural network primer", J. Biol System. 2, 247-283) containing a single hidden layer of ten neurons (ten hidden units) found in EasyNN-Plus version 4.0g software package (Neural Planner Software Inc.) is used. In a specific example, parameter values within the EasyNN-Plus program are set as follows: a learning rate of 0.05, and a momentum of 0.2. In some embodiments in which the EasyNN-Plus version 4.0g software package is used, "outlier" samples are identified by performing twenty independently-seeded trials involving 20,000 learning cycles each.

C. Support Vector Machine

In some embodiments of the present invention, support vector machines (SVMs) are used to classify subjects using expression profiles of marker genes described in the present invention. General description of SVM can be found in, for example, Cristianini and Shawe-Taylor, 2000, An Introduction to Support Vector Machines, Cambridge University Press, Cambridge, Boser et al., 1992, "A training algorithm for optimal margin classifiers, in Proceedings of the 5th Annual ACM Workshop on Computational Learning Theory, ACM Press, Pittsburgh, PA, pp. 142-152; Vapnik, 1998, Statistical Learning Theory, Wiley, New York; Duda, Pattern Classification, Second Edition, 2001, John Wiley & Sons, Inc.; Hastie, 2001, The Elements of Statistical Learning, Springer, New York; and Furey et al., 2000, Bioinformatics 16, 906-914. Applications of SVM in biological applications are described in Jaakkola et al., Proceedings of the 7th International Conference on Intelligent Systems for Molecular Biology, AAAI Press, Menlo Park, CA (1999); Brown et al., Proc. Natl. Acad. Sci. 97(1):262-67 (2000); Zien et al., Bioinformatics, 16(9):799-807 (2000); Furey et al., Bioinformatics, 16(10):906-914 (2000)

In one approach, when a SVM is used, the gene expression data is standardized to have mean zero and unit variance and the members of a training population are randomly divided into a training set and a test set. For example, in one embodiment, two thirds of the members of the training population are placed in the training set and one third of the members of the training population are placed in the test set. The expression values for a selected set of genes of the present invention is used to train the SVM. Then the ability for the trained SVM to correctly classify members in the test set is determined. In some embodiments, this computation is performed several times for a given selected set of molecular markers. In each iteration of the computation, the members of the training population are randomly assigned to the training set and the test set. Then, the quality of the combination of molecular markers is taken as the average of each such iteration of the SVM computation.

Support vector machines map a given set of binary labeled training data to a high-dimensional feature space and separate the two classes of data with a maximum margin hyperplane. In general, this hyperplane corresponds to a nonlinear decision boundary in the input space. Let $X \in R_0 \subseteq \Re^n$ be the input vectors, $y \in \{-1,+1\}$ be the labels, and $\phi$: $R_0 \rightarrow F$ be the mapping from input space to feature space. Then the SVM learning algorithm finds a hyperplane ($w,b$) such that the quantity

$$\gamma = \min_i y_i\{\langle w, \phi(\mathbf{X}_i)\rangle - b\}$$

is maximized, where the vector $w$ has the same dimensionality as $F$, $b$ is a real number, and $\gamma$ is called the *margin*. The corresponding decision function is then

$$f(\mathbf{X}) = sign(\langle w, \phi(\mathbf{X})\rangle - b)$$

This minimum occurs when

$$w = \sum_i \alpha_i y_i \phi(X_i)$$

where $\{\alpha_i\}$ are positive real numbers that maximize

$$\sum_i \alpha_i - \sum_{ij} \alpha_i \alpha_j y_i y_j \langle \phi(X_i), \phi(X_j)\rangle$$

subject to

$$\sum_i \alpha_i y_i = 0, \alpha_i > 0$$

The decision function can equivalently be expressed as

$$f(\mathbf{X}) = sign(\sum_i \alpha_i y_i \langle \phi(X_i, \phi(\mathbf{X})\rangle - b)$$

From this equation it can be seen that the $\alpha_i$ associated with the training point $\mathbf{X_i}$ expresses the strength with which that point is embedded in the final decision function. A remarkable property of this alternative representation is that only a subset of the points will be associated with a non-zero $\alpha_i$. These points are called *support vectors* and are the points that lie closest to the separating hyperplane. The sparseness of the $\alpha$ vector has several computational and learning theoretic consequences. It is important to note that neither the learning algorithm nor the decision function needs to represent explicitly the image of points in the feature space, $\phi(\mathbf{X}_i)$, since both use only the dot products between such images, $\langle \phi(X_i), \phi(X_j)\rangle$. Hence, if one were given a function $K(\mathbf{X},\mathbf{Y}) = \langle \phi(\mathbf{X}), \phi(\mathbf{X})\rangle$, one could learn and use the maximum margin hyperplane in the feature space without ever explicitly performing the mapping. For each continuous positive definite function $K(\mathbf{X},\mathbf{Y})$ there exists a mapping $\phi$ such that $K(\mathbf{X},\mathbf{Y}) = \langle \phi(\mathbf{X}), \phi(\mathbf{X})\rangle$ for all $\mathbf{X},\mathbf{Y} \in R_0$ (Mercer's Theorem). The function $K(\mathbf{X},\mathbf{Y})$ is called the *kernel function*. The use of a kernel function allows the support vector machine to operate efficiently in a nonlinear high-dimensional feature spaces without being adversely affected by the dimensionality of that space. Indeed, it is possible to work with feature spaces of infinite dimension. Moreover, Mercer's theorem makes it possible to learn in the feature space without even knowing $\phi$ and $F$. The matrix $K_{ij} = \langle \phi(\mathbf{X}_i), \phi(\mathbf{X}_j)\rangle$ is called the *kernel*

*matrix*. Finally, note that the learning algorithm is a quadratic optimization problem that has only a global optimum. The absence of local minima is a significant difference from standard pattern recognition techniques such as neural networks. For moderate sample sizes, the optimization problem can be solved with simple gradient descent techniques. In the presence of noise, the standard maximum margin algorithm described above can be subject to overfitting, and more sophisticated techniques should be used. This problem arises because the maximum margin algorithm always finds a perfectly consistent hypothesis and does not tolerate training error. Sometimes, however, it is necessary to trade some training accuracy for better predictive power. The need for tolerating training error has led to the development the soft-margin and the margin-distribution classifiers. One of these techniques replaces the kernel matrix in the training phase as follows:

$$K \leftarrow K + \lambda I$$

while still using the standard kernel function in the decision phase. By tuning λ, one can control the training error, and it is possible to prove that the risk of misclassifying unseen points can be decreased with a suitable choice of λ.

If instead of controlling the overall training error one wants to control the trade-off between false positives and false negatives, it is possible to modify *K* as follows:

$$K \leftarrow K + \lambda D$$

where *D* is a diagonal matrix whose entries are either $d^+$ or $d^-$, in locations corresponding to positive and negative examples. It is possible to prove that this technique is equivalent to controlling the size of the $\alpha_i$ in a way that depends on the size of the class, introducing a bias for larger $\alpha_i$ in the class with smaller *d*. This in turn corresponds to an asymmetric margin; i.e., the class with smaller *d* will be kept further away from the decision boundary. In some cases, the extreme imbalance of the two classes, along with the presence of noise, creates a situation in which points from the minority class can be easily mistaken for mislabelled points. Enforcing a strong bias against training errors in the minority class provides protection agaist such errors and forces the SVM to make the positive examples support vectors. Thus, choosing $d^+ = \frac{1}{n^+}$ and $d^- = \frac{1}{n^-}$ provides a heuristic way to automatically adjust the relative importance of the two classes, based on their respective cardinalities. This technique effectively controls the trade-off between sensitivity and specificity.

In the present invention, a linear kernel can be used. The similarity between two marker profiles X and Y can be the dot product X·Y. In one embodiment, the kernel is

$$K(X, Y) = X \cdot Y + 1$$

In another embodiment, a kernel of degree *d* is used

$$K(X, Y) = (X \cdot Y + 1)^d ,$$

where *d* can be either 2, 3, ...

In still another embodiment, a Gaussian kernel is used

$$K(\mathbf{X}, \mathbf{Y}) = \exp\left(\frac{-|X - Y|^2}{2\sigma^2}\right)$$

where σ is the width of the Gaussian.

D. Logistic Regression

In some embodiments, the classifier is based on a regression model, preferably a logistic regression model. Such a regression model includes a coefficient for each of the molecular markers in a selected set of molecular biomarkers of the invention. In such embodiments, the coefficients for the regression model are computed using, for example, a maximum likelihood approach. In particular embodiments, molecular biomarker data from two different classification or phenotype groups, e.g., deregulated or regulated RAS signaling pathway, response or non-response to treatment to an agent that modulates the RAS signaling pathway, is used and the dependent variable is the phenotypic status of the patient for which molecular marker characteristic data are from.

Some embodiments of the present invention provide generalizations of the logistic regression model that handle multicategory (polychotomous) responses. Such embodiments can be used to discriminate an organism into one or three or more classification groups, e.g., good, intermediate, and poor therapeutic response to treatment with RAS signaling pathway agents. Such regression models use multicategory logit models that simultaneously refer to all pairs of categories, and describe the odds of response in one category instead of another. Once the model specifies logits for a certain (J-1) pairs of categories, the rest are redundant. See, for example, Agresti, An Introduction to Categorical Data Analysis, John Wiley & Sons, Inc., 1996, New York, Chapter 8.

E. Discriminant Analysis

Linear discriminant analysis (LDA) attempts to classify a subject into one of two categories based on certain object properties. In other words, LDA tests whether object attributes measured in an experiment predict categorization of the objects. LDA typically requires continuous independent variables and a dichotomous categorical dependent variable. In the present invention, the expression values for the selected set of molecular markers of the invention across a subset of the training population serve as the requisite continuous independent variables. The clinical group classification of each of the members of the training population serves as the dichotomous categorical dependent variable.

LDA seeks the linear combination of variables that maximizes the ratio of between-group variance and within-group variance by using the grouping information. Implicitly, the linear weights used by LDA depend on how the expression of a molecular biomarker across the training set separates in the two groups (*e.g.*, a group that has deregulated RAS signaling pathway and a group that have regulated RAS signaling pathway status) and how this gene expression correlates with the expression of other genes. In some embodiments, LDA is applied to the data matrix of the N members in the training sample by K genes in a combination of genes described in the present invention. Then, the linear discriminant of each member of the training population is plotted. Ideally, those members of the training population representing a first subgroup (*e.g.* those subjects that have deregulated RAS signaling pathway status) will cluster into one range of linear discriminant values (*e.g.*, negative) and those member of the training population representing a second subgroup (*e.g.* those subjects that have regulated RAS signaling pathway status) will cluster into a second range of linear discriminant values (*e.g.*, positive). The LDA is considered more successful when the separation between the clusters of discriminant values is larger. For more information on linear discriminant analysis, see Duda, Pattern Classification, Second Edition, 2001, John Wiley & Sons, Inc; and Hastie, 2001, The Elements of Statistical Learning, Springer, New York; Venables & Ripley, 1997, Modern Applied Statistics with s-plus, Springer, New York.

Quadratic discriminant analysis (QDA) takes the same input parameters and returns the same results as LDA. QDA uses quadratic equations, rather than linear equations, to produce results. LDA and QDA are interchangeable, and which to use is a matter of preference and/or availability of software to support the analysis. Logistic regression takes the same input parameters and returns the same results as LDA and QDA.

F. Decision Trees

In some embodiments of the present invention, decision trees are used to classify subjects using expression data for a selected set of molecular biomarkers of the invention. Decision tree algorithms belong to the class of supervised learning algorithms. The aim of a decision tree is to induce a classifier (a tree) from real-world example data. This tree can be used to classify unseen examples which have not been used to derive the decision tree.

A decision tree is derived from training data. An example contains values for the different attributes and what class the example belongs. In one embodiment, the training data is expression data for a combination of genes described in the present invention across the training population.

The following algorithm describes a decision tree derivation:

```
Tree (Examples, Class, Attributes)
              Create a root node
              If all Examples have the same Class value, give the root this label
              Else if Attributes is empty label the root according to the most common value
              Else begin
                      Calculate the information gain for each attribute
                      Select the attribute A with highest information gain and make
```

```
this the root attribute
                       For each possible value, v, of this attribute
                              Add a new branch below the root, corresponding to A = v
                              Let Examples(v) be those examples with A = v
                              If Examples(v) is empty, make the new branch a leaf node labeled
 with the most common value among Examples
                              Else let the new branch be the tree created by
                                 Tree(Examples(v),Class,Attributes - {A})
               end
```

A more detailed description of the calculation of information gain is shown in the following. If the possible classes $v_i$ of the examples have probabilities $P(v_i)$ then the information content I of the actual answer is given by:

$$I(P(v_1),...,P(v_n)) = \sum_{i=1}^{n} - P(v_i)\log_2 P(v_i)$$

The I- value shows how much information we need in order to be able to describe the outcome of a classification for the specific dataset used. Supposing that the dataset contains p positive and n negative (examples (*e.g.* individuals), the information contained in a correct answer is:

$$I(\frac{p}{p+n}, \frac{n}{p+n}) = -\frac{p}{p+n}\log_2\frac{p}{p+n} - \frac{n}{p+n}\log_2\frac{n}{p+n}$$

where $\log_2$ is the logarithm using base two. By testing single attributes the amount of information needed to make a correct classification can be reduced. The remainder for a specific attribute A (*e.g.* a gene biomarker) shows how much the information that is needed can be reduced.

$$\mathrm{Re}\,mainder(A) = \sum_{i=1}^{v} \frac{p_i + n_i}{p+n} I(\frac{p_i}{p_i + n_i}, \frac{n_i}{p_i + n_i})$$

"v" is the number of unique attribute values for attribute A in a certain dataset, "i" is a certain attribute value, "$p_i$" is the number of examples for attribute A where the classifcation is positive, "$n_i$" is the number of examples for attribute A where the classification is negative.

The information gain of a specific attribute A is calculated as the difference between the information content for the classes and the remainder of attribute A:

$$Gain(A) = I(\frac{p}{p+n}, \frac{n}{p+n}) - \mathrm{Re}\,mainder(A)$$

The information gain is used to evaluate how important the different attributes are for the classification (how well they split up the examples), and the attribute with the highest information.

In general there are a number of different decision tree algorithms, many of which are described in Duda, Pattern Classification, Second Edition, 2001, John Wiley & Sons, Inc. Decision tree algorithms often require consideration of feature processing, impurity measure, stopping criterion, and pruning. Specific decision tree algorithms include, cut are not limited to classification and regression trees (CART), multivariate decision trees, ID3, and C4.5.

In one approach, when an exemplary embodiment of a decision tree is used, the gene expression data for a selected set of molecular markers of the invention across a training population is standardized to have mean zero and unit variance. The members of the training population are randomly divided into a training set and a test set. For example, in one embodiment, two thirds of the members of the training population are placed in the training set and one third of the members of the training population are placed in the test set. The expression values for a select combination of genes described in the present invention is used to construct the decision tree. Then, the ability for the decision tree to correctly classify members in the test set is determined. In some embodiments, this computation is performed several times for a given combination of molecular markers. In each iteration of the computation, the members of the training population are randomly assigned to the training set and the test set. Then, the quality of the combination of molecular markers is taken as the average of each such iteration of the decision tree computation.

G. Clustering

In some embodiments, the expression values for a selected set of molecular markers of the invention are used to cluster a training set. For example, consider the case in which ten gene biomarkers described in one of the genesets of the present invention are used. Each member **m** of the training population will have expression values for each of the ten biomarkers. Such values from a member **m** in the training population define the vector:

| | | | | | | | | | |
|---|---|---|---|---|---|---|---|---|---|
| $X_{1m}$ | $X_{2m}$ | $X_{3m}$ | $X_{4m}$ | $X_{5m}$ | $X_{6m}$ | $X_{7m}$ | $X_{8m}$ | $X_{9m}$ | $X_{10m}$ |

where $X_{im}$ is the expression level of the $i^{th}$ gene in organism **m**. If there are **m** organisms in the training set, selection of **i** genes will define **m** vectors. Note that the methods of the present invention do not require that each the expression value of every single gene used in the vectors be represented in every single vector **m**. In other words, data from a subject in which one of the $\mathbf{i}^{th}$ genes is not found can still be used for clustering. In such instances, the missing expression value is assigned either a "zero" or some other normalized value. In some embodiments, prior to clustering, the gene expression values are normalized to have a mean value of zero and unit variance.

Those members of the training population that exhibit similar expression patterns across the training group will tend to cluster together. A particular combination of genes of the present invention is considered to be a good classifer in this aspect of the invention when the vectors cluster into the trait groups found in the training population. For instance, if the training population includes patients with good or poor prognosis, a clustering classifier will cluster the population into two groups, with each group uniquely representing either a deregulated RAS signalling pathway status or a regulated RAS signalling pathway status.

Clustering is described on pages 211-256 of Duda and Hart, Pattern Classification and Scene Analysis, 1973, John Wiley & Sons, Inc., New York. As described in Section 6.7 of Duda, the clustering problem is described as one of finding natural groupings in a dataset. To identify natural groupings, two issues are addressed. First, a way to measure similarity (or dissimilarity) between two samples is determined. This metric (similarity measure) is used to ensure that the samples in one cluster are more like one another than they are to samples in other clusters. Second, a mechanism for partitioning the data into clusters using the similarity measure is determined.

Similarity measures are discussed in Section 6.7 of Duda, where it is stated that one way to begin a clustering investigation is to define a distance function and to compute the matrix of distances between all pairs of samples in a dataset. If distance is a good measure of similarity, then the distance between samples in the same cluster will be significantly less than the distance between samples in different clusters. However, as stated on page 215 of Duda, clustering does not require the use of a distance metric. For example, a nonmetric similarity function s(x, x') can be used to compare two vectors x and x'. Conventionally, s(x, x') is a symmetric function whose value is large when x and x' are somehow "similar". An example of a nonmetric similarity function s(x, x') is provided on page 216 of Duda.

Once a method for measuring "similarity" or "dissimilarity" between points in a dataset has been selected, clustering requires a criterion function that measures the clustering quality of any partition of the data. Partitions of the data set that extremize the criterion function are used to cluster the data. See page 217 of Duda. Criterion functions are discussed in Section 6.8 of Duda.

More recently, Duda et al., Pattern Classification, 2nd edition, John Wiley & Sons, Inc. New York, has been published. Pages 537-563 describe clustering in detail. More information on clustering techniques can be found in Kaufman and Rousseeuw, 1990, Finding Groups in Data: An Introduction to Cluster Analysis, Wiley, New York, NY; Everitt, 1993, Cluster analysis (3d ed.), Wiley, New York, NY; and Backer, 1995, Computer-Assisted Reasoning in Cluster Analysis, Prentice Hall, Upper Saddle River, New Jersey. Particular exemplary clustering techniques that can be used in the present invention include, but are not limited to, hierarchical clustering (agglomerative clustering using nearest-neighbor algorithm, farthest-neighbor algorithm, the average linkage algorithm, the centroid algorithm, or the sum-of-squares algorithm), k-means clustering, fuzzy k-means clustering algorithm, and Jarvis-Patrick clustering.

H. Principal Component Analysis

Principal component analysis (PCA) has been proposed to analyze gene expression data. Principal component analysis is a classical technique to reduce the dimensionality of a data set by transforming the data to a new set of variable (principal components) that summarize the features of the data. See, for example, Jolliffe, 1986, Principal Component Analysis, Springer, New York. Principal components (PCs) are uncorrelate and are ordered such that the

$k^{th}$ PC has the $k$th largest variance among PCs. The $k^{th}$ PC can be interpreted as the direction that maximizes the variation of the projections of the data points such that it is orthogonal to the first $k$ - 1 PCs. The first few PCs capture most of the variation in the data set. In contrast, the last few PCs are often assumed to capture only the residual 'noise' in the data.

PCA can also be used to create a classifier in accordance with the present invention. In such an approach, vectors for a selected set of molecular biomarkers of the invention can be constructed in the same manner described for clustering above. In fact, the set of vectors, where each vector represents the expression values for the select genes from a particular member of the training population, can be considered a matrix. In some embodiments, this matrix is represented in a Free-Wilson method of qualitative binary description of monomers (Kubinyi, 1990, 3D QSAR in drug design theory methods and applications, Pergamon Press, Oxford, pp 589-638), and distributed in a maximally compressed space using PCA so that the first principal component (PC) captures the largest amount of variance information possible, the second principal component (PC) captures the second largest amount of all variance information, and so forth until all variance information in the matrix has been accounted for.

Then, each of the vectors (where each vector represents a member of the training population) is plotted. Many different types of plots are possible. In some embodiments, a one-dimensional plot is made. In this one-dimensional plot, the value for the first principal component from each of the members of the training population is plotted. In this form of plot, the expectation is that members of a first group will cluster in one range of first principal component values and members of a second group will cluster in a second range of first principal component values.

In one example, the training population comprises two classification groups. The first principal component is computed using the molecular biomarker expression values for the select genes of the present invention across the entire training population data set where the classification outcomes are known. Then, each member of the training set is plotted as a function of the value for the first principal component. In this example, those members of the training population in which the first principal component is positive represent one classification outcome and those members of the training population in which the first principal component is negative represent the other classification outcome.

In some embodiments, the members of the training population are plotted against more than one principal component. For example, in some embodiments, the members of the training population are plotted on a two-dimensional plot in which the first dimension is the first principal component and the second dimension is the second principal component. In such a two-dimensional plot, the expectation is that members of each subgroup represented in the training population will cluster into discrete groups. For example, a first cluster of members in the two-dimensional plot will represent subjects in the first classification group, a second cluster of members in the two-dimensional plot will represent subjects in the second classification group, and so forth.

In some embodiments, the members of the training population are plotted against more than two principal components and a determination is made as to whether the members of the training population are clustering into groups that each uniquely represents a subgroup found in the training population. In some embodiments, principal component analysis is performed by using *the R mva* package (Anderson, 1973, Cluster Analysis for applications, Academic Press, New York 1973; Gordon, Classification, Second Edition, Chapman and Hall, CRC, 1999.). Principal component analysis is further described in Duda, Pattern Classification, Second Edition, 2001, John Wiley & Sons, Inc.

I. Nearest Neighbor Classifier Analysis

Nearest neighbor classifiers are memory-based and require no model to be fit. Given a query point $x_0$, the $k$ training points $x_{(r)}$, $r$, ... , $k$ closest in distance to $x_0$ are identified and then the point $x_0$ is classified using the $k$ nearest neighbors. Ties can be broken at random. In some embodiments, Euclidean distance in feature space is used to determine distance as:

$$d_{(i)} = \left\| x_{(i)} - x_0 \right\|.$$

Typically, when the nearest neighbor algorithm is used, the expression data used to compute the linear discriminant is standardized to have mean zero and variance 1. In the present invention, the members of the training population are randomly divided into a training set and a test set. For example, in one embodiment, two thirds of the members of the training population are placed in the training set and one third of the members of the training population are placed in the test set. Profiles of a selected set of molecular biomarkers of the invention represents the feature space into which members of the test set are plotted. Next, the ability of the training set to correctly characterize the members of the test set is computed. In some embodiments, nearest neighbor computation is performed several times for a given combination of genes of the present invention. In each iteration of the computation, the members of the training population are randomly assigned to the training set and the test set. Then, the quality of the combination of genes is taken as the average of each such iteration of the nearest neighbor computation.

The nearest neighbor rule can be refined to deal with issues of unequal class priors, differential misclassification costs, and feature selection. Many of these refinements involve some form of weighted voting for the neighbors. For more information on nearest neighbor analysis, see Duda, Pattern Classification, Second Edition, 2001, John Wiley & Sons, Inc; and Hastie, 2001, The Elements of Statistical Learning, Springer, New York.

J. Evolutionary Methods

Inspired by the process of biological evolution, evolutionary methods of classifier design employ a stochastic search for an optimal classifier. In broad overview, such methods create several classifiers - a population - from measurements of gene products of the present invention. Each classifier varies somewhat from the other. Next, the classifiers are scored on expression data across the training population. In keeping with the analogy with biological evolution, the resulting (scalar) score is sometimes called the fitness. The classifiers are ranked according to their score and the best classifiers are retained (some portion of the total population of classifiers). Again, in keeping with biological terminology, this is called survival of the fittest. The classifiers are stochastically altered in the next generation - the children or offspring. Some offspring classifiers will have higher scores than their parent in the previous generation, some will have lower scores. The overall process is then repeated for the subsequent generation: The classifiers are scored and the best ones are retained, randomly altered to give yet another generation, and so on. In part, because of the ranking, each generation has, on average, a slightly higher score than the previous one. The process is halted when the single best classifier in a generation has a score that exceeds a desired criterion value. More information on evolutionary methods is found in, for example, Duda, Pattern Classification, Second Edition, 2001, John Wiley & Sons, Inc.

K. Bagging, Boosting and the Random Subspace Method

Bagging, boosting and the random subspace method are combining techniques that can be used to improve weak classifiers. These techniques are designed for, and usually applied to, decision trees. In addition, Skurichina and Duin provide evidence to suggest that such techniques can also be useful in linear discriminant analysis.

In bagging, one samples the training set, generating random independent bootstrap replicates, constructs the classifier on each of these, and aggregates them by a simple majority vote in the final decision rule. See, for example, Breiman, 1996, Machine Learning 24, 123-140; and Efron & Tibshirani, An Introduction to Bootstrap, Chapman & Hall, New York, 1993.

In boosting, classifiers are constructed on weighted versions of the training set, which are dependent on previous classification results. Initially, all objects have equal weights, and the first classifier is constructed on this data set. Then, weights are changed according to the performance of the classifier. Erroneously classified objects (molecular biomarkers in the data set) get larger weights, and the next classifier is boosted on the reweighted training set. In this way, a sequence of training sets and classifiers is obtained, which is then combined by simple majority voting or by weighted majority voting in the final decision. See, for example, Freund & Schapire, "experiments with a new boosting algorithm," Proceedings 13th International Conference on Machine Learning, 1996, 148-156.

To illustrate boosting, consider the case where there are two phenotypic groups exhibited by the population under study, phenotype 1, and phenotype 2. Given a vector of molecular markers *X,* a classifier *G(X)* produces a prediction taking one of the type values in the two value sent:{ phenotype 1, phenotype 2}. The error rate on the training sample is

$$\overline{\text{err}} = \frac{1}{N}\sum_{i=1}^{N} I(y_i \neq G(x_i))$$

where N is the number of subjects in the training set (the sum total of the subjects that have either phenotype 1 or phenotype 2).

A weak classifier is one whose error rate is only slightly better than random guessing. In the boosting algorithm, the weak classification algorithm is repeatedly applied to modified versions of the data, thereby producing a sequence of weak classifiers $G_m(x)$, *m,* = 1,2, ..., *M*. The predictions from all of the classifiers in this sequence are then combined through a weighted majority vote to produce the final prediction:

$$G(x) = sign\left(\sum_{m=1}^{M} \alpha_m G_m(x)\right)$$

Here $\alpha_1$, $\alpha_2$, ..., $\alpha_M$ are computed by the boosting algorithm and their purpose is to weigh the contribution of each respective $G_m(x)$. Their effect is to give higher influence to the more accurate classifiers in the sequence.

The data modifications at each boosting step consist of applying weights $w_1$, $w_2$, ..., $w_n$ to each of the training observations ($x_i$, $y_i$), $i$ = 1, 2, ..., N. Initially all the weights are set to $w_i$ = 1/N, so that the first step simply trains the classifier on the data in the usual manner. For each successive iteration $m$ = 2, 3, ..., $M$ the observation weights are individually modified and the classification algorithm is reapplied to the weighted observations. At stem $m$, those observations that were misclassified by the classifier $G_{m-1}(x)$ induced at the previous step have their weights increased, whereas the weights are decreased for those that were classified correctly. Thus as iterations proceed, observations that are difficult to correctly classify receive ever-increasing influence. Each successive classifier is thereby forced to concentrate on those training observations that are missed by previous ones in the sequence.

The exemplary boosting algorithm is summarized as follows:

1. Initialize the observation weights $w_i$ = 1/N, $i$ = 1, 2, ..., $N$.

2. For $m$ = 1 to $M$:

   (a) Fit a classifier $G_m(x)$ to the training set using weights $w_i$.
   (b) Compute

$$\text{err}_m = \frac{\sum_{i=1}^{N} w_i I(y_i \neq G_m(x_i))}{\sum_{i=1}^{N} w_i}$$

   (c) Compute $\alpha_m$=log((1-$\text{err}_m$)/$\text{err}_m$).
   (d) *Set* $w_i \leftarrow w_i \cdot \exp[\alpha_m \cdot I(y_i \neq G_m(x_i))]$, $i$ = 1, 2, ..., $N$.

3. Output $G(x) = \text{sign}\left[\sum_{m=1}^{M} \alpha_m G_m(x)\right]$

In the algorithm, the current classifier $G_m(x)$ is induced on the weighted observations at line 2a. The resulting weighted error rate is computed at line 2b. Line 2c calculates the weight $\alpha_m$ given to G$m$($x$) in producing the final classifier G($x$) (line 3). The individual weights of each of the observations are updated for the next iteration at line 2d. Observations misclassified by G$m$($x$) have their weights scaled by a factor exp($\alpha_m$), increasing their relative influence for inducing the next classifier $G_{m+1}(x)$ in the sequence. In some embodiments, modifications of the Freund and Schapire, 1997, Journal of Computer and System Sciences 55, pp. 119-139, boosting method are used. See, for example, Hasti et al., The Elements of Statistical Learning, 2001, Springer, New York, Chapter 10. In some embodiments, boosting or adaptive boosting methods are used.

In some embodiments, modifications of Freund and Schapire, 1997, Journal of Computer and System Sciences 55, pp. 119-139, are used. For example, in some embodiments, feature preselection is performed using a technique such as the nonparametric scoring methods of Park et al., 2002, Pac. Symp. Biocomput. 6, 52-63. Feature preselection is a form of dimensionality reduction in which the genes that discriminate between classifications the best are selected for use in the classifier. Then, the LogitBoost procedure introduced by Friedman et al., 2000, Ann Stat 28, 337-407 is used rather than the boosting procedure of Freund and Schapire. In some embodiments, the boosting and other classification methods of Ben-Dor et al., 2000, Journal of Computational Biology 7, 559-583 are used in the present invention. In some embodiments, the boosting and other classification methods of Freund and Schapire, 1997, Journal of Computer and System Sciences 55, 119-139, are used.

In the random subspace method, classifiers are constructed in random subspaces of the data feature space. These classifiers are usually combined by simple majority voting in the final decision rule. See, for example, Ho, "The Random subspace method for constructing decision forests," IEEE Trans Pattern Analysis and Machine Intelligence, 1998; 20(8): 832-844.

L. Other Algorithms

The pattern classification and statistical techniques described above are merely examples of the types of models that can be used to construct a model for classifcation. Moreover, combinations of the techniques described above can be used. Some combinations, such as the use of the combination of decision trees and boosting, have been described. However, many other combinations are possible. In addition, in other techniques in the art such as Projection Pursuit and Weighted Voting can be used to construct a classifier.

3.5 Determination of Biomarker Gene Expression Levels

3.5.1 *Methods*

The expression levels of the biomarker genes in a sample may be determined by any means known in the art. The expression level may be determined by isolating and determining the level (i.e., amount) of nucleic acid transcribed from each biomarker gene. Alternatively, or additionally, the level of specific proteins translated from mRNA transcribed from a biomarker gene may be determined.

The level of expression of specific biomarker genes can be accomplished by determining the amount of mRNA, or polynucleotides derived therefrom, present in a sample. Any method for determining RNA levels can be used. For example, RNA is isolated from a sample and separated on an agarose gel. The separated RNA is then transferred to a solid support, such as a filter. Nucleic acid probes representing one or more biomarkers are then hybridized to the filter by northern hybridization, and the amount of biomarker-derived RNA is determined. Such determination can be visual, or machine-aided, for example, by use of a densitometer. Another method of determining RNA levels is by use of a dot-blot or a slot-blot. In this method, RNA, or nucleic acid derived therefrom, from a sample is labeled. The RNA or nucleic acid derived therefrom is then hybridized to a filter containing oligonucleotides derived from one or more biomarker genes, wherein the oligonucleotides are placed upon the filter at discrete, easily-identifiable locations. Hybridization, or lack thereof, of the labeled RNA to the filter-bound oligonucleotides is determined visually or by densitometer. Polynuelcotides can be labeled using a radiolabel or a fluorescent (i.e., visible) label.

These examples are not intended to be limiting. Other methods of determining RNA abundance are known in the art, including, but not limited to quantitative PCR methods, such as TAQMAN®, and Nanostring's NCOUNTER™ Digital Gene Expression System (Seattle, WA) (See also WO2007076128; WO2007076129).

The level of expression of particular biomarker genes may also be assessed by determining the level of the specif protein expressed from the biomarker genes. This can be accomplished, for example, by separation of proteins from a sample on a polyacrylamide gel, followed by identification of specific biomarker-derived proteins using antibodies in a western blot. Alternatively, proteins can be separated by two-dimensional gel electrophoresis systems. Two-dimensional gel electrophoresis is well-known in the art and typically involves isoelectric focusing along a first dimension followed by SDS-PAGE electrophoresis along a second dimension. See, e.g., Hames et al, 1990, GEL ELECTROPHORESIS OF PROTEINS: A PRACTICAL APPROACH, IRL Press, New York; Shevchenko et al., Proc. Nat'l Acad. Sci. USA 93:1440-1445 (1996); Sagliocco et al., Yeast 12:1519-1533 (1996); Lander, Science 274:536-539 (1996). The resulting electropherograms can be analyzed by numerous techniques, including mass spectrometric techniques, western blotting and immunoblot analysis using polyclonal and monoclonal antibodies.

Alternatively, biomarker-derived protein levels can be determined by constructing an antibody microarray in which binding sites comprise immobilized, preferably monoclonal, antibodies specific to a plurality of protein species encoded by the cell genome. Preferably, antibodies are present for a substantial fraction of the biomarker-derived proteins of interest. Methods for making monoclonal antibodies are well known (see, e. g., Harlow and Lane, 1988, ANTIBODIES: A LABORATORY MANUAL, Cold Spring Harbor, ). In one embodiment, monoclonal antibodies are raised against synthetic peptide fragments designed based on genomic sequence of the cell. With such an antibody array, proteins from the cell are contacted to the array, and their binding is assayed with assays known in the art. Generally, the expression, and the level of expression, of proteins of diagnostic or prognostic interest can be detected through immunohistochemical staining of tissue slices or sections.

Finally, expression of biomarker genes in a number of tissue specimens may be characterized using a "tissue array" (Kononen et al., Nat. Med 4(7):844-7 (1998)). In a tissue array, multiple tissue samples are assessed on the same microarray. The arrays allow in situ detection of RNA and protein levels; consecutive sections allow the analysis of multiple samples simultaneously.

3.5.2 *Microarrays*

In preferred embodiments, polynucleotide microarrays are used to measure expression so that the expression status of each of the biomarkers above is assessed simultaneously. In a specific embodiment, the invention provides for oligonucleotide or cDNA arrays comprising probes hybridizable to the genes corresponding to each of the biomarker sets described above (i.e., biomarkers to determine the molecular type or subtype of a tumor; biomarkers to classify the RAS pathway signaling status of a tumor; biomarkers to predict response of a subject to a compound that modulates the RAS signaling pathway; biomarkers to measure pharmacodynamic effect of a therapeutic agent on the RAS signaling pathway).

The microarrays provided by the present invention may comprise probes hybridizable to the genes corresponding to biomarkers able to distinguish the status of one, two, or all three of the clinical conditions noted above. In particular, the invention provides polynucleotide arrays comprising probes to a subset or subsets of at least 5, 10, 20, 30, 40, 50, 60, 70, 80, 90, 100, 110, 120, 130, 140 genetic biomarkers, up to the full set of 147 biomarkers of Tables 2a and 2b, which distinguish RAS signaling pathway deregulated and regulated patients or tumors.

For example, in a specific embodiment, the microarray is a screening or scanning array as described in Altschuler et al., International Publication WO 02/18646, published Mar. 7, 2002 and Scherer et al., International Publication WO 02/16650, published Feb. 28, 2002. The scanning and screening arrays comprise regularly-spaced, positionally-addressable probes derived from genomic nucleic acid sequence, both expressed and unexpressed. Such arrays may comprise probes corresponding to a subset of, or all of, the biomarkers listed in Tables 2a and 2b, or a subset thereof as described above, and can be used to monitor biomarker expression in the same way as a microarray containing only biomarkers listed in Table 2a and 2b.

In yet another specific embodiment, the microarray is a commercially-available cDNA microarray that comprises at least five of the biomarkers listed in Tables 2a and 2b, wherein at least 1 biomarker is selected from Table 2b. Preferably, a commercially-available cDNA microarray comprises all of the biomarkers listed in Tables 2a and 2b. However, such a microarray may comprise 5, 10, 15, 25, 50, 75, 100, 125, 140 or more of the biomarkers in any of Tables 2a and 2b, up to the maximum number of biomarkers in Tables 2a and 2b, and may comprise all of the biomarkers in any one of Table 2a and 2b and a subset of another of Table 2a and 2b, or subsets of each as described above. In a specific embodiment of the microarrays used in the methods disclosed herein, the biomarkers that are all or a portion of Tables 2a and 2b make up at least 50%, 60%, 70%, 80%, 90%, 95% or 98% of the probes on the microarray.

General methods pertaining to the construction of microarrays comprising the biomarker sets and/or subsets above are described in the following sections.

3.5.2.1 *Construction of microarrays*

Microarrays are prepared by selecting probes which comprise a polynucleotide sequence, and then immobilizing such probes to a solid support or surface. For example, the probes may comprise DNA sequences, RNA sequences, or copolymer sequences of DNA and RNA. The polynucleotide sequences of the probes may also comprise DNA and/or RNA analogues, or combinations thereof. For example, the polynucleotide sequences of the probes may be full or partial fragments of genomic DNA. The polynucleotide sequences of the probes may also be synthesized nucleotide sequences, such as synthetic oligonucleotide sequences. The probe sequences can be synthesized either enzymatically in vivo, enzymatically in vitro (e.g., by PCR), or non-enzymatically in vitro.

The probe or probes used in the methods of the invention are preferably immobilized to a solid support which may be either porous or non-porous. For example, the probes of the invention may be polynucleotide sequences which are attached to a nitrocellulose or nylon membrane or filter covalently at either the 3' or the 5' end of the polynucleotide. Such hybridization probes are well known in the art (see, e.g., Sambrook et al., MOLECULAR CLONING--A LABORATORY MANUAL (2ND ED.), Vols. 1-3, Cold Spring Harbor Laboratory, Cold Spring Harbor, N.Y. (1989). Alternatively, the solid support or surface may be a glass or plastic surface. In a particularly preferred embodiment, hybridization levels are measured to microarrays of probes consisting of a solid phase on the surface of which are immobilized a population of polynucleotides, such as a population of DNA or DNA mimics, or, alternatively, a population of RNA or RNA mimics. The solid phase may be a nonporous or, optionally, a porous material such as a gel.

In preferred embodiments, a microarray comprises a support or surface with an ordered array of binding (e.g., hybridization) sites or "probes" each representing one of the biomarkers described herein. Preferably the microarrays are addressable arrays, and more preferably positionally addressable arrays. More specifically, each probe of the array is preferably located at a known, predetermined position on the solid support such that the identity (i.e., the sequence) of each probe can be determined from its position in the array (i.e., on the support or surface). In preferred embodiments, each probe is covalently attached to the solid support at a single site.

Microarrays can be made in a number of ways, of which several are described below. However produced, microarrays share certain characteristics. The arrays are reproducible, allowing multiple copies of a given array to be produced and easily compared with each other. Preferably, microarrays are made from materials that are stable under binding (e.g., nucleic acid hybridization) conditions. The microarrays are preferably small, e.g., between 1 $cm^2$ and 25 $cm^2$, between 12 $cm^2$ and 13 $cm^2$, or 3 $cm^2$. However, larger arrays are also contemplated and may be preferable, e.g., for use in screening arrays. Preferably, a given binding site or unique set of binding sites in the microarray will specifically bind (e.g., hybridize) to the product of a single gene in a cell (e.g., to a specific mRNA, or to a specific cDNA derived therefrom). However, in general, other related or similar sequences will cross hybridize to a given binding site.

The microarrays of the present invention include one or more test probes, each of which has a polynucleotide sequence that is complementary to a subsequence of RNA or DNA to be detected. Preferably, the position of each probe on the solid surface is known. Indeed, the microarrays are preferably positionally addressable arrays. Specifically, each probe of the array is preferably located at a known, predetermined position on the solid support such that the identity (i.e., the sequence) of each probe can be determined from its position on the array (i.e., on the support or surface).

According to the invention, the microarray is an array (i.e., a matrix) in which each position represents one of the biomarkers described herein. For example, each position can contain a DNA or DNA analogue based on genomic DNA to which a particular RNA or cDNA transcribed from that genetic biomarker can specifically hybridize. The DNA or DNA analogue can be, e.g., a synthetic oligomer or a gene fragment. In one embodiment, probes representing each of the biomarkers is present on the array.

3.5.2.2 Preparing Probes for Microarrays

As noted above, the "probe" to which a particular polynucleotide molecule specifcally hybridizes according to the invention contains a complementary genomic polynucleotide sequence. The probes of the microarray preferably consist of nucleotide sequences of no more than 1,000 nucleotides. In some embodiments, the probes of the array consist of nucleotide sequences of 10 to 1,000 nucleotides. In a preferred embodiment, the nucleotide sequences of the probes are in the range of 10-200 nucleotides in length and are genomic sequences of a species of organism, such that a plurality of different probes is present, with sequences complementary and thus capable of hybridizing to the genome of such a species of organism, sequentially tiled across all or a portion of such genome. In other specific embodiments, the probes are in the range of 10-30 nucleotides in length, in the range of 10-40 nucleotides in length, in the range of 20-50 nucleotides in length, in the range of 40-80 nucleotides in length, in the range of 50-150 nucleotides in length, in the range of 80-120 nucleotides in length, and most preferably are 60 nucleotides in length.

The probes may comprise DNA or DNA "mimics." (e.g., derivatives and analogues) corresponding to a portion of an organism's genome. In another embodiment, the probes of the microarray are complementary RNA or RNA mimics. DNA mimics are polymers composed of subunits capable of specific, Watson-Crick-like hybridization with DNA, or of specific hybridization with RNA. The nucleic acids can be modified at the base moiety, at the sugar moiety, or at the phosphate backbone. Exemplary DNA mimics include, e.g., phosphorothioates.

DNA can be obtained, e.g., by polymerase chain reaction (PCR) amplification of genomic DNA or cloned sequences. PCR primers are preferably chosen based on a known sequence of the genome that will result in amplification of specific fragments of genomic DNA. Computer programs that are well known in the art are useful in the design of primers with the required specificity and optimal amplification properties, such as Oligo version 5.0 (National Biosciences). Typically each probe on the microarray will be between 10 bases and 50,000 bases, usually between 300 bases and 1, 000 bases in length. PCR methods are well known in the art, and are described, for example, in Innis et al., eds., PCR PROTOCOLS: A GUIDE TO METHODS AND APPLICATIONS, Academic Press Inc., San Diego, Calif. (1990). It will be apparent to one skilled in the art that controlled robotic systems are useful for isolating and amplifying nucleic acids.

An alternative, preferred means for generating the polynucleotide probes of the microarray is by synthesis of synthetic polynucleotides or oligonucleotides, e.g., using N-phosphonate or phosphoramidite chemistries (Froehler et al., Nucleic Acid Res. 14:5399-5407 (1986); McBride et al., Tetrahedron Lett. 24:246-248 (1983)). Synthetic sequences are typically between about 10 and about 500 bases in length, more typically between about 20 and about 100 bases, and most preferably between about 40 and about 70 bases in length. In some embodiments, synthetic nucleic acids include non-natural bases, such as, but by no means limited to, inosine. As noted above, nucleic acid analogues may be used as binding sites for hybridization. An example of a suitable nucleic acid analogue is peptide nucleic acid (see, e.g., Egholm et al., Nature 363:566-568 (1993); U.S. Pat. No. 5,539,083). Probes are preferably selected using an algorithm that takes into account binding energies, base composition, sequence complexity, cross-hybridization binding energies, and secondary structure (see Friend et al., International Patent Publication WO 01/05935, published Jan. 25, 2001; Hughes et al., Nat. Biotech. 19:342-7 (2001)).

A skilled artisan will also appreciate that positive control probes, e.g., probes known to be complementary and hybridizable to sequences in the target polynucleotide molecules, and negative control probes, e.g., probes known to not be complementary and hybridizable to sequences in the target polynucleotide molecules, should be included on the array. In one embodiment, positive controls are synthesized along the perimeter of the array. In another embodiment, positive controls are synthesized in diagonal stripes across the array. In still another embodiment, the reverse complement for each probe is synthesized next to the position of the probe to serve as a negative control. In yet another embodiment, sequences from other species of organism are used as negative controls or as "spike-in" controls.

3.5.2.3 Attaching Probes to the Solid Surface

The probes are attached to a solid support or surface, which may be made, e.g., from glass, plastic (e.g., polypropylene, nylon), polyacrylamide, nitrocellulose, gel, or other porous or nonporous material. A preferred method for attaching the nucleic acids to a surface is by printing on glass plates, as is described generally by Schena et al, Science 270:467-470 (1995). This method is especially useful for preparing microarrays of cDNA (See also, DeRisi et al, Nature Genetics 14:457-460 (1996); Shalon et al., Genome Res. 6:639-645 (1996); and Schena et al., Proc. Natl.

Acad. Sci. U.S.A. 93:10539-11286 (1995)).

A second preferred method for making microarrays is by making high-density oligonucleotide arrays. Techniques are known for producing arrays containing thousands of oligonucleotides complementary to defined sequences, at defined locations on a surface using photolithographic techniques for synthesis in situ (see, Fodor et al., 1991 , Science 251:767-773; Pease et al, 1994, Proc. Natl. Acad. Sci. U.S.A. 91:5022-5026; Lockhart et al., 1996, Nature Biotechnology 14:1675; U.S. Pat. Nos. 5,578,832; 5, 556,752; and 5,510,270) or other methods for rapid synthesis and deposition of defined oligonucleotides (Blanchard et al., Biosensors & Bioelectronics 11:687-690). When these methods are used, oligonucleotides (e.g., 60-mers) of known sequence are synthesized directly on a surface such as a derivatized glass slide. Usually, the array produced is redundant, with several oligonucleotide molecules per RNA.

Other methods for making microarrays, e.g., by masking (Maskos and Southern, 1992, Nuc. Acids. Res. 20:1679-1684), may also be used. In principle, and as noted *supra*, any type of array, for example, dot blots on a nylon hybridization membrane (see Sambrook et al., MOLECULAR CLONING--A LABORATORY MANUAL (2ND ED.), Vols. 1-3, Cold Spring Harbor Laboratory, Cold Spring Harbor, N.Y. (1989)) could be used. However, as will be recognized by those skilled in the art, very small arrays will frequently be preferred because hybridization volumes will be smaller.

In one embodiment, the arrays of the present invention are prepared by synthesizing polynucleotide probes on a support. In such an embodiment, polynucleotide probes are attached to the support covalently at either the 3' or the 5' end of the polynucleotide.

In a particularly preferred embodiment, microarrays of the invention are manufactured by means of an ink jet printing device for oligonucleotide synthesis, e.g., using the methods and systems described by Blanchard in U.S. Pat. No. 6,028,189; Blanchard et al., 1996, Biosensors and Bioelectronics 11:687-690; Blanchard, 1998, in SYNTHETIC DNA ARRAYS IN GENETIC ENGINEERING, Vol. 20, J. K. Setlow, Ed., Plenum Press, New York at pages 111-123. Specifically, the oligonucleotide probes in such microarrays are preferably synthesized in arrays, e.g., on a glass slide, by serially depositing individual nucleotide bases in "microdroplets" of a high surface tension solvent such as propylene carbonate. The microdroplets have small volumes (e.g., 100 pL or less, more preferably 50 pL or less) and are separated from each other on the microarray (e.g., by hydrophobic domains) to form circular surface tension wells which define the locations of the array elements (i.e., the different probes). Microarrays manufactured by this ink-jet method are typically of high density, preferably having a density of at least about 2,500 different probes per 1 $cm^2$. The polynucleotide probes are attached to the support covalently at either the 3' or the 5' end of the polynucleotide.

3.5.2.4 Target Polynucleotide Molecules

The polynucleotide molecules which may be analyzed by the present invention (the "target polynucleotide molecules") may be from any clinically relevant source, but are expressed RNA or a nucleic acid derived therefrom (e.g., cDNA or amplified RNA derived from cDNA that incorporates an RNA polymerase promoter), including naturally occurring nucleic acid molecules, as well as synthetic nucleic acid molecules. In one embodiment, the target polynucleotide molecules comprise RNA, including, but by no means limited to, total cellular RNA, poly(A)+ messenger RNA (mRNA) or fraction thereof, cytoplasmic RNA, or RNA transcribed from cDNA (i.e., cRNA; see, e.g., Linsley & Schelter, U.S. patent application Ser. No. 09/411,074, filed Oct. 4, 1999, or U.S. Pat. Nos. 5,545,522, 5,891,636, or 5,716,785). Methods for preparing total and poly(A)+ RNA are well known in the art, and are described generally, e.g., in Sambrook et al., MOLECULAR CLONING--A LABORATORY MANUAL (2ND ED.), Vols. 1-3, Cold Spring Harbor Laboratory, Cold Spring Harbor, N.Y. (1989). In one embodiment, RNA is extracted from cells of the various types of interest in this invention using guanidinium thiocyanate lysis followed by CsCl centrifugation (Chirgwin et al., 1979, Biochemistry 18:5294-5299). In another embodiment, total RNA is extracted using a silica gel-based column, commercially available examples of which include RNeasy (Qiagen, Valencia, Calif.) and StrataPrep (Stratagene, La Jolla, Calif.). In an alternative embodiment, which is preferred for S. *cerevisiae,* RNA is extracted from cells using phenol and chloroform, as described in Ausubel et al., eds., 1989, CURRENT PROTOCOLS IN MOLECULAR BIOLOGY, Vol. III, Green Publishing Associates, Inc., John Wiley & Sons, Inc., New York, at pp. 13.12.1-13.12.5). Poly(A)+ RNA can be selected, e.g., by selection with oligo-dT cellulose or, alternatively, by oligo-dT primed reverse transcription of total cellular RNA. In one embodiment, RNA can be fragmented by methods known in the art, e.g., by incubation with $ZnCl_2$, to generate fragments of RNA. In another embodiment, the polynucleotide molecules analyzed by the invention comprise cDNA, or PCR products of amplified RNA or cDNA.

In one embodiment, total RNA, mRNA, or nucleic acids derived therefrom, is isolated from a sample taken from a person afflicted with breast cancer. Target polynucleotide molecules that are poorly expressed in particular cells may be enriched using normalization techniques (Bonaldo et al., 1996, Genome Res. 6:791-806).

As described above, the target polynucleotides are detectably labeled at one or more nucleotides. Any method known in the art may be used to detectably label the target polynucleotides. Preferably, this labeling incorporates the label uniformly along the length of the RNA, and more preferably, the labeling is carried out at a high degree of efficiency. One embodiment for this labeling uses oligo-dT primed reverse transcription to incorporate the label; however, conventional methods of this method are biased toward generating 3' end fragments. Thus, in a preferred embodiment, random primers (e.g., 9-meres) are used in reverse transcription to uniformly incorporate labeled nucleotides over the fill length of the target polynucleotides. Alternatively, random primers may be used in conjunction with PCR methods or T7 promoter-based in vitro transcription methods in order to amplify the target polynucleotides.

In a preferred embodiment, the detectable label is a luminescent label. For example, fluorescent labels, bioluminescent labels, chemi-luminescent labels, and colorimetric labels may be used in the present invention. In a highly preferred embodiment, the label is a fluorescent label, such as a fluorescein, a phosphor, a rhodamine, or a polymethine dye derivative. Examples of commercially available fluorescent labels include, for example, fluorescent phosphoramidites such as FluorePrime (Amersham Pharmacia, Piscataway, N.J.), Fluoredite (Millipore, Bedford, Mass.), FAM (ABI, Foster City, Calif.), and Cy3 or Cy5 (Amersham Pharmacia, Piscataway, N.J.). In another embodiment, the detectable label is a radiolabeled nucleotide.

In a further preferred embodiment, target polynucleotide molecules from a patient sample are labeled differentially from target polynucleotide molecules of a standard. The standard can comprise target polynucleotide molecules from normal individuals (i.e., those not afflicted with cancer). In a highly preferred embodiment, the standard comprises target polynucleotide molecules pooled from samples from normal individuals or tumor samples from individuals having cancer. In another embodiment, the target polynucleotide molecules are derived from the same individual, but are taken at different time points, and thus indicate the efficacy of a treatment by a change in expression of the biomarkers, or lack thereof during and after the course of treatment (i.e., RAS pathway therapeutic agent), wherein a change in the expression of the biomarkers from a RAS pathway deregulation pattern to a RAS pathway regulation pattern indicates that the treatment is efficacious. In this embodiment, different timepoints are differentially labeled.

3.5.2.5 Hybridization to Microarrays

Nucleic acid hybridization and wash conditions are chosen so that the target polynucleotide molecules specifically bind or specifically hybridize to the complementary polynucleotide sequences of the array, preferably to a specific array site, wherein its complementary DNA is located.

Arrays containing double-stranded probe DNA situated thereon are preferably subjected to denaturing conditions to render the DNA single-stranded prior to contacting with the target polynucleotide molecules. Arrays containing single-stranded probe DNA (e.g., synthetic oligodeoxyribonucleic acids) may need to be denatured prior to contacting with the target polynucleotide molecules, e.g., to remove hairpins or dimers which form due to self complementary sequences.

Optimal hybridization conditions will depend on the length (e.g., oligomer versus polynucleotide greater than 200 bases) and type (e.g., RNA, or DNA) of probe and target nucleic acids. One of skill in the art will appreciate that as the oligonucleotides become shorter, it may become necessary to adjust their length to achieve a relatively uniform melting temperature for satisfactory hybridization results. General parameters for specific (i.e., stringent) hybridization conditions for nucleic acids are described in Sambrook et al., MOLECULAR CLONING--A LABORATORY MANUAL (2ND ED.), Vols. 1-3, Cold Spring Harbor Laboratory, Cold Spring Harbor, N.Y. (1989), and in Ausubel et al., CURRENT PROTOCOLS IN MOLECULAR BIOLOGY, vol. 2, Current Protocols Publishing, New York (1994). Typical hybridization conditions for the cDNA microarrays of Schena et al. are hybridization in 5×SSC plus 0.2% SDS at 65°C. for four hours, followed by washes at 25°C. in low stringency wash buffer (1×SSC plus 0.2% SDS), followed by 10 minutes at 25°C in higher stringency wash buffer (0.1×SSC plus 0.2% SDS) (Schena et al., Proc. Natl. Acad. Sci. U.S.A. 93:10614 (1993)). Useful hybridization conditions are also provided in, e.g., Tijessen, 1993, HYBRIDIZATION WITH NUCLEIC ACID PROBES, Elsevier Science Publishers B. V.; and Kricka, 1992, NONISOTOPIC DNA PROBE TECHNIQUES, Academic Press, San Diego, Calif.

Particularly preferred hybridization conditions include hybridization at a temperature at or near the mean melting temperature of the probes (e. g., within 5°C., more preferably within 2°C.) in 1 M NaCl, 50 mM MES buffer (pH 6.5), 0.5% sodium sarcosine and 30% formamide.

3.5.2.6 Signal Detection and Data Analysis

When fluorescently labeled probes are used, the fluorescence emissions at each site of a microarray may be, preferably, detected by scanning confocal laser microscopy. In one embodiment, a separate scan, using the appropriate excitation line, is carried out for each of the two fluorophores used. Alternatively, a laser may be used that allows simultaneous specimen illumination at wavelengths specific to the two fluorophores and emissions from the two fluorophores can be analyzed simultaneously (see Shalon et al., 1996, "A DNA microarray system for analyzing complex DNA samples using two-color fluorescent probe hybridization," Genome Research 6:639-645, ). In a preferred embodiment, the arrays are scanned with a laser fluorescent scanner with a computer controlled X-Y stage and a microscope objective. Sequential excitation of the two fluorophores is achieved with a multi-line, mixed gas laser and the emitted light is split by wavelength and detected with two photomultiplier tubes. Fluorescence laser scanning devices are described in Schena et al., Genome Res. 6:639-645 (1996), and in other references cited herein. Alternatively, the fiber-optic bundle described by Ferguson et al., Nature Biotech. 14:1681-1684 (1996), may be used to monitor mRNA abundance levels at a large number of sites simultaneously.

Signals are recorded and, in a preferred embodiment, analyzed by computer, e.g., using a 12 or 16 bit analog to digital board. In one embodiment the scanned image is despeckled using a graphics program (e.g., Hijaak Graphics Suite) and then analyzed using an image gridding program that creates a spreadsheet of the average hybridization at each wavelength at each site. If necessary, an experimentally determined correction for "cross talk" (or overlap) between the channels for the two fluors may be made. For any particular hybridization site on the transcript array, a ratio of the emission of the two fluorophores can be calculated. The ratio is independent of the absolute expression level of the cognate gene, but is useful for genes whose expression is signifcantly modulated in association with the different breast cancer-related condition.

3.6 Computer-Facilitated Analysis

The present disclosure further provides for kits comprising the biomarker sets above. In a preferred embodiment, the kit contains a microarray ready for hybridization to target polynucleotide molecules, plus software for the data analyses described above.

The analytic methods described in the previous sections can be implemented by use of the following computer systems and according to the following programs and methods. A Computer system comprises internal components linked to external components. The internal components of a typical computer system include a processor element interconnected with a main memory. For example, the computer system can be an Intel 8086-, 80386-, 80486-, Pentium®, or Pentium®-based processor with preferably 32 MB or more of main memory.

The external components may include mass storage. This mass storage can be one or more hard disks (which are typically packaged together with the processor and memory). Such hard disks are preferably of 1 GB or greater storage capacity. Other external components include a user interface device, which can be a monitor, together with an inputting device, which can be a "mouse", or other graphic input devices, and/or a keyboard. A printing device can also be attached to the computer.

Typically, a computer system is also linked to network link, which can be part of an Ethernet link to other local computer systems, remote computer systems, or wide area communication networks, such as the Internet. This network link allows the computer system to share data and processing tasks with other computer systems.

Loaded into memory during operation of this system are several software components, which are both standard in the art and special to the instant invention. These software components collectively cause the computer system to function according to the methods of this invention. These software components are typically stored on the mass storage device. A software component comprises the operating system, which is responsible for managing computer system and its network interconnections. This operating system can be, for example, of the Microsoft Windows® family, such as Windows 3.1, Windows 95, Windows 98, Windows 2000, or Windows NT. The software component represents common languages and functions conveniently present on this system to assist programs implementing the methods specific to this invention. Many high or low level computer languages can be used to program the analytic methods of this invention. Instructions can be interpreted during run-time or compiled. Preferred languages include C/C++, FORTRAN and JAVA. Most preferably, the methods of this invention are programmed in mathematical software packages that allow symbolic entry of equations and high-level specification of processing, including some or all of the algorithms to be used, thereby freeing a user of the need to procedurally program individual equations or algorithms. Such packages include Mathlab from Mathworks (Natick, Mass.), Mathematica® from Wolfram Research (Champaign, Ill.), or S-Plus®D from Math Soft (Cambridge, Mass.). Specifically, the software component includes the analytic methods of the invention as programmed in a procedural language or symbolic package.

The software to be included with the kit comprises the data analysis methods of the invention as disclosed herein. In particular, the software may include mathematical routines for biomarker discovery, including the calculation of correlation coefficients between clinical categories (i.e., RAS signaling pathway regulation status) and biomarker expression. The software may also include mathematical routines for calculating the correlation between sample biomarker expression and control biomarker expression, using array-generated fluorescence data, to determine the clinical classification of a sample.

## EXAMPLES

Example 1: Identification of gene-expression based RAS pathway activity biomarkers

Genome wide gene expression profiling provides a new paradigm for detecting and understanding oncogene deregulation by measuring coherent changes in multiple genes downstream from oncogene signaling. A recent study by Bild et al. (2006, Nature 439:353-357) set the stage for developing oncogene signatures for activation of the RAS, Myc, E2F3, Src, and beta-catenin pathways. These signatures were derived from primary human mammary epithelial cells stably transfected with each of these five oncogenes. The linear combination of genes in the signatures was shown to be predictive of sensitivity to therapeutic agents targeting specific pathways. Although this study provided an important proof of concept for developing oncogene signatures, it left open for interpretation the exact methods for using the genes in the signatures for measuring oncogene deregulation in tumor samples. Specifically, the expectation from this study was that the genes from the RAS signature that were perturbed in opposite directions by RAS overexpression would be anti-correlated when assessed in large sets of tumor samples that have variable RAS activity. However, these genes showed a positive correlation in such tumor samples. Thus, a linear combination of genes with the same signs as observed in the training set would fail to detect upregulation of RAS signaling in test sets from human tumors.

An alternative method for developing a RAS signature was proposed by Sweet-Cordero et al. (2005, Nat. Genet. 37:48-55). This group used cross-species gene expression analysis to derive a signature of oncogenic KRAS2. They obtained gene expression profiles from the tumors of mice genetically engineered to express activated KRAS2 in lung tissues and compared these profiles to human lung cancers. A common gene expression signature was found between mice and humans. A third approach to derive a RAS signature was used by Blum et al. (2007, Cancer Res. 67:3320-3328), who derived a RAS signature by blocking RAS activity with Salirasib (S-Farnesylthiosalicylic Acid). A RAS signature was derived from gene expression changes in 5 human tumor cell lines at 24-72 hr post treatment. While these three signatures report on a similar biological state (RAS activity), the signatures contain different genes, and did not show a coherent pattern of expression in cell line panels or tumors based on our internal gene expression profiling data.

We wished to identify a gene expression signature of RAS pathway activity that was coherent across various cell lines and tumor datasets and could be used in pre-clinical models. We started with four RAS pathway signatures identified in three publications: 1) Bild et al., 2006, Nature 439:353-357 (referred to hereinafter as "Nevins"); 2) Blum et al., 2007, Cancer Res. 67:3320-3328 (referred to hereinafter as "Blum"; and 3) Sweet-Carder et al., 2005, Nat. Genet. 37:48-55 (original signature referred to hereinafter as "Jacks" and refined signature referred to hereinafter as "Jacks123"). All of these RAS signatures were split into two opposing "arms" - the "up" arm, comprising the set of genes that are upregulated as signaling through the RAS pathway increases, and the "down" arm, comprising the set of genes that are downregulated as signaling through the RAS pathway increases.

We derived our own RAS signature using supervised analysis of the Nevins, Blum, Jacks, and Jacks 123 signatures and their consensus prediction of RAS mutation status generated in lung cell lines. Specifically, we used the consensus prediction of KRAS mutation status generated in lung cell lines (RAS mutation status identified from the Welcome Trust Sanger Institute (http://www.sanger.ae.uk/genctics/CGP/CellLines/). We derived a coherent "up" core set of 105 genes from the above published signatures (See Table 2a). Using this core set of "up" genes as a seed set, we then identified genes that were anti-correleated with the "up" genes and were upregulated in RAS wild-type cell lines compared to mutant. These 42 anti-correlated genes represent the "down" arm of our RAS signature (See Table 2b).

**Table 2a: "Up" arm of the RAS pathway signature gene set**

| Gene Symbol | Transcript ID | SEQ ID NO: | Probe SEQ ID NO: |
|---|---|---|---|
| ADAM8 | NM_001109 | 1 | 106 |
| ADRB2 | NM_000024 | 2 | 107 |
| ANGPTL4 | NM_139314 | 3 | 108 |
| ARNTL2 | NM_020183 | 4 | 109 |
| C19orf10 | NM_019107 | 5 | 110 |
| C20orf42 | NM_017671 | 6 | 111 |
| CALM2 | NM_001743 | 7 | 112 |
| CALU | NM_001219 | 8 | 113 |
| CAPZA1 | N_006135 | 9 | 114 |
| CCL20 | NM_004591 | 10 | 115 |
| CD274 | N_014143 | 11 | 116 |
| CDCP1 | NM_022842 | 12 | 117 |
| CLCF1 | NM_013246 | 13 | 118 |

(continued)

| Gene Symbol | Transcript ID | SEQ ID NO: | Probe SEQ ID NO: |
|---|---|---|---|
| CSNK1 D | NM_139062 | 14 | 119 |
| CXCL1 | NM_001511 | 15 | 120 |
| CXCL2 | NM_002089 | 16 | 121 |
| CXCL3 | NM_002090 | 17 | 122 |
| CXCL5 | NM_002994 | 18 | 123 |
| DENND2C | NM_198459 | 19 | 124 |
| DUSP1 | NM_004417 | 20 | 125 |
| DUSP4 | NM_001394 | 21 | 126 |
| DUSP5 | NM_004419 | 22 | 127 |
| DUSP6 | NM_022652 | 23 | 128 |
| EFNB1 | NM_004429 | 24 | 129 |
| EGR1 | NM_001964 | 25 | 130 |
| EHD1 | NM_006795 | 26 | 131 |
| ELK3 | NM_005230 | 27 | 132 |
| EREG | NM_001432 | 28 | 133 |
| FOS | NM_005252 | 29 | 134 |
| FOXQ1 | NM_033260 | 30 | 135 |
| G0S2 | NM_015714 | 31 | 136 |
| GDF15 | NM_004864 | 32 | 137 |
| GLTP | NM_016433 | 33 | 138 |
| HBEGF | NM_001945 | 34 | 139 |
| IER3 | NM_003897 | 35 | 140 |
| IL13RA2 | NM_000640 | 36 | 141 |
| IL1A | NM_000575 | 37 | 142 |
| IL1B | NM_000576 | 38 | 143 |
| IL8 | NM_000584 | 39 | 144 |
| ITGA2 | NM_002203 | 40 | 145 |
| ITPR3 | NM_002224 | 41 | 146 |
| KCNK1 | NM_002245 | 42 | 147 |
| KCNN4 | NM_002250 | 43 | 148 |
| KLF5 | NM_001730 | 44 | 149 |
| KLF6 | NM_001300 | 45 | 150 |
| LAMA3 | NM_198129 | 46 | 151 |
| LDLR | NM_000527 | 47 | 152 |
| LHFPL2 | NM_005779 | 48 | 153 |
| LIF | NM_002309 | 49 | 154 |
| MALL | NM_005434 | 50 | 155 |
| MAP1LC3B | hCT1640758.2 | 51 | 156 |

(continued)

| Gene Symbol | Transcript ID | SEQ ID NO: | Probe SEQ ID NO: |
|---|---|---|---|
| MAST4 | BX101442 | 52 | 157 |
| MMP14 | NM_004995 | 53 | 158 |
| MXD1 | N_002357 | 54 | 159 |
| NAV3 | NM_014903 | 55 | 160 |
| NDRG1 | NM_006096 | 56 | 161 |
| NFKBIZ | N_031419 | 57 | 162 |
| NPAL1 | NM_207330 | 58 | 163 |
| NT5E | NM_002526 | 59 | 164 |
| OXSR1 | NM_005109 | 60 | 165 |
| PBEF1 | NM_005746 | 61 | 166 |
| PHLDA1 | AK074510 | 62 | 167 |
| PHLDA2 | NM_003311 | 63 | 168 |
| PI3 | NM_002638 | 64 | 169 |
| PIK3CD | N_005026 | 65 | 170 |
| PIM1 | NM_002648 | 66 | 171 |
| PLAUR | NM_001005376 | 67 | 172 |
| PNMA2 | ENST00000305426 | 68 | 173 |
| PPP1R15A | NM_014330 | 69 | 174 |
| PRNP | NM_183079 | 70 | 175 |
| PTGS2 | NM_000963 | 71 | 176 |
| PTHLH | NM_198965 | 72 | 177 |
| PTPRE | NM_006504 | 73 | 178 |
| PTX3 | NM_002852 | 74 | 179 |
| PVR | NM_006505 | 75 | 180 |
| RPRC1 | NM_018067 | 76 | 181 |
| S100A6 | NM_014624 | 77 | 182 |
| SDC1 | NM_002997 | 78 | 183 |
| SDC4 | NM_002999 | 79 | 184 |
| SEMA4B | HSS00219047 | 80 | 185 |
| SERPINB1 | NM_030666 | 81 | 186 |
| SERPINB2 | NM_002575 | 82 | 187 |
| SERPINB5 | NM_002639 | 83 | 188 |
| SESN2 | NM_031459 | 84 | 189 |
| SFN | NM_006142 | 85 | 190 |
| SLC16A3 | NM_004207 | 86 | 191 |
| SLC2A14 | MM_153449 | 87 | 192 |
| SLC2A3 | NM_006931 | 88 | 193 |
| SLC9A1 | NM_003047 | 89 | 194 |

(continued)

| Gene Symbol | Transcript ID | SEQ ID NO: | Probe SEQ ID NO: |
|---|---|---|---|
| SPRY4 | NM_030964 | 90 | 195 |
| TFPI2 | NM_006528 | 91 | 196 |
| TGFA | NM_003236 | 92 | 197 |
| TIMP1 | NM_003254 | 93 | 198 |
| TMEM45B | NM_138788 | 94 | 199 |
| TNFRSF10A | NM_003844 | 95 | 200 |
| TNFRSF10B | NM_003842 | 96 | 201 |
| TNFRSF12A | NM_016639 | 97 | 202 |
| TNS4 | NM_032865 | 98 | 203 |
| TOR9AIP1 | NM_015602 | 99 | 204 |
| TSC22D1 | NM_006022 | 100 | 205 |
| TUBA1 | NM_006000 | 101 | 206 |
| UAP1 | NM_003115 | 102 | 207 |
| UPP1 | NM_181597 | 103 | 208 |
| VEGF | NM_003376 | 104 | 209 |
| ZFP36 | NM_003407 | 105 | 210 |

**Table 2b: "Down" arm of the RAS pathway signature gene set**

| Gene Symbol | Transcript ID | SEQ ID NO: | Probe SEQ ID NO: |
|---|---|---|---|
| ABCC5 | NM_005688 | 211 | 253 |
| ARMC8 | N_015396 | 212 | 254 |
| ATPAF1 | NM_022745 | 213 | 255 |
| AUTS2 | NM_015570 | 214 | 256 |
| C1orf96 | NM_145257 | 215 | 257 |
| C6orf182 | NM_173830 | 216 | 258 |
| CELSR2 | NM_001408 | 217 | 259 |
| CENTB2 | NM_012287 | 218 | 260 |
| COQ7 | NM_016138 | 219 | 261 |
| DRD4 | NM_000797 | 220 | 262 |
| ENAH | NM_018212 | 221 | 263 |
| HNRPU | Contig24903_RC | 222 | 264 |
| HTATSF1 | NM_014500 | 223 | 265 |
| ID4 | NM_001546 | 224 | 266 |
| ITSN1 | AF003738 | 225 | 267 |
| JMJD2C | Contig25062_RC | 226 | 268 |
| KIAA1772 | NM_024935 | 227 | 269 |
| MIB1 | NM_020774 | 228 | 270 |

(continued)

| Gene Symbol | Transcript ID | SEQ ID NO: | Probe SEQ ID NO: |
|---|---|---|---|
| MRPS14 | NM_022100 | 229 | 271 |
| MSI1 | NM_002442 | 230 | 272 |
| MSI2 | Contig57081_RC | 231 | 273 |
| NUP133 | NM_018230 | 232 | 274 |
| OGN | NM_024416 | 233 | 275 |
| PARP1 | NM_001618 | 234 | 276 |
| PIAS1 | NM_016166 | 235 | 277 |
| RASP10B | NM_033315 | 236 | 278 |
| RFPL3S | AJ010233 | 237 | 279 |
| RTN3 | NM_006054 | 238 | 280 |
| SEC63 | NM_007214 | 239 | 281 |
| SF4 | NM_982812 | 240 | 282 |
| SH3GL2 | NM_003026 | 241 | 283 |
| SMAD9 | NM_005905 | 242 | 284 |
| STARD7 | NM_020151 | 243 | 285 |
| TBG1D24 | NM_020705 | 244 | 286 |
| TMEFF1 | NM_003692 | 245 | 287 |
| TTC28 | NM_015281 | 246 | 288 |
| TXNDC4 | NM_015051 | 247 | 289 |
| ZNF292 | ENST00000339907 | 248 | 290 |
| ZNF441 | NM_152355 | 249 | 291 |
| ZNF493 | NM_175910 | 250 | 292 |
| ZNF669 | NM_024804 | 251 | 293 |
| ZNF672 | NM_024836 | 252 | 294 |

Example 2: Coherency of RAS pathway signature in cell line panels

As a first step in the analysis of the RAS signatures, we assessed the coherency of the signatures across four cell line panels from lung, colon, breast, and lymphoid malignancies. The purpose of coherence analysis is to show the statistical significance of the difference between the "Up" and "Down" arms of the signature in a new dataset. Two correlation coefficients were calculated for all of the genes in both the Up and Down arms. First, the correlation between each gene in the Up arm and the average of all genes in the Up arm is calculated. Second, the anticorrelation between each gene in the Up arm and the average of all genes in the Down arm is calculated. This is repeated for genes in the Down arm. If the signature is coherent, most of the genes from the Up arm should correlate with the average of all Up genes and anticorrelate with the average of all genes in the Down arm. A Fisher exact test is calculated for correlation within and between arms of the signature to assess the significance of signature coherence in a new dataset. Signatures are refined by filtering out the genes that do not show the correct correlation-anticorrelation behavior. This filtering process enables the identification of the subset of signature genes that retains information regarding signaling activity and elimination of genes that are not robustly co-regulated in a new dataset. RAS pathway activity (or regulation) is summarized into a RAS signature score, which is calculated as: (mean expression of Up genes (Table 2a)) - (mean expression of Down genes (Table 2b)). A sample with a RAS pathway signature score >0 is classified as having a deregulated RAS pathway, while a RAS pathway signature score <0 is classified as having a regulated RAS pathway.

Initial signature coherence analysis and pairwise comparison of cell lines was performed on cell lines (CMTI portion of the Cell Line Atlas (breast, colon, lung, lymphoma)). Prediction of RAS mutation status was also performed on cell lines from the cell lines atlas for which RAS mutation data was available. Further check of the coherence of signatures was performed on the Netherlands Cancer Institute (NKI) colon and breast datasets, fresh tumors (Tumor Atlas for breast, colon, lung), and formalin-fixed paraffin embedded (FFPE) samples (the Mayo FFPE datasets for lung, ovarian and breast).

Total RNA was isolated from cell lines and converted to fluorescently labeled cRNA that was hybridized to DNA oligonucleotide microarrays as described previously (Hughes et al., 2001, Nat. Biotechnol. 19:342-347; Marton et al., 1998, Nat. Med. 4:1293-1301). Briefly, 4 μg of total RNA from each sample was used to synthesized dsDNA through reverse transcription. cRNA was produced by *in vitro* transcription and labeled post-synthetically with Cy3 or Cy5. Probe sequences were chosen to maximize gene specificity and minimize the 3'-replication bias inherent in reverse transcription of mRNA. In addition, all microarrays contained approximately 2,000 control probes for quality control purposes. All probes on the microarrays were synthesized *in situ* with inkjet technology (Agilent Technologies, Palo Alto, CA; Hughes et al, 2001, Nat. Biotechnol. 19:342-347). After hybridization, arrays were scanned and fluorescence intensities for each probe were recorded. Ratios of transcript abundance (experimental to control) were obtained following normalization and correction of the array intensity data. Gene expression data was analyzed using Rosetta Resolver gene expression analysis software (version 7.0, Rosetta Biosoftware, Seattle, WA) and MATLAB (The MathWorks, Natick, MA).

Table 1 summarizes the results of this coherency test for all of the signatures in the four cell line panels. The analysis shows that Nevins (activated RAS expression in HMEC) and Jacks (activated RAS expression in mouse lung) signatures, the two signatures that are based on constitutive deregulation of RAS signaling, are not coherent with p-value >0.05 based on a Fisher exact test, while our RAS signature and the Blum signature (cell line treatment with RAS inhibitor) are coherent across all the datasets.

Figure 2A shows that the "up" and "down" arms of the 147 gene RAS pathway signature is highly coherent in the breast cell line panel, with a p-value of less than $10^{-9}$ by a Fisher exact test. A heatmap based on all the genes (Figure 2B) and a heatmap after filtering the genes (Figure 2D) show that the "UP" and "DOWN" arms of our signature cluster apart in this dataset. Finally, Figure 2C shows a scatter plot of the "UP" and "DOWN" arms for the signature before filtering. The p-value of the anticorrelation is significant based on the Kendall, Spearman, or Pearson correlation tests. As an example of a signature that is not coherent, Figures 3A, B, and C show a similar analysis done for the Nevins signature in breast cell lines. Figure 3A demonstrates that this signature is not coherent by our coherence test with p-value >0.05. The same can be seen in Figure 3B, in which genes from "UP" and "DROWN" arms cluster together in the heatmap. As shown in Figure 3C, The "UP" and "DOWN" arms of this signature correlate rather than anticorrelate. This lack of coherence for the Nevins signature presents a problem for scoring their RAS signature unless we leverage an independent line of evidence to confirm the scores.

Table 3: Coherency test for RAS signatures across four cell line panels

| | Our RAS | Nevins | Jacks | Blum |
|---|---|---|---|---|
| Lymphoma | <e-12 | >0.05 | >0.05 | <e-9 |
| Lung | <e-12 | >0.05 | >0.05 | <e-9 |
| Breast | <e-12 | >0.05 | >0.05 | <e-9 |
| Colon | <e-12 | >0.05 | >0.05 | <e-9 |

Example 3: Consensus of different signatures in cell lines

In this analysis we wished to assess if the different RAS pathway signatures significantly correlate and thus make similar predictions about RAS pathway deregulation in the four cell line panels. Figures 4A-D show the pair-wise scatter plots for our RAS signature, the Nevins UP signature, the Blum signature, and the Jack original and refined signatures. In breast cell lines (Figure 4A), we see significant pairwise correlations between our signature and Nevins, Blum and Jack's refined signatures but not with the original Jacks signature. The negative sign of correlation between our RAS and Blum's RAS signatures is due to sign selection for Blum's results. We assigned the genes that are upregulated by RAS inhibitors into the "Down" arm and those that are downregulated by RAS inhibitors into the "Up" arm. One possible explanation for this observation is that the acute inhibition of RAS leads to changes in expression that are mimicking upregulated RAS signaling rather than reversing it. Nevertheless, the significance of pairwise correlations is very high with p-values based on Kendal, Pearson, or Spearman correlations lower than $10^{-9}$ for all but Jack's original signature. Our RAS pathway signature also showed a wide dynamic range relative to the other signatures, with scores ranging from -0.5 to 0.5.

Similar to the breast cell line panel, the colon (Figure 4B), lung (Figure 4C) and lymphoma (Figure 4D) panels showed significant pairwise correlation between our RAS signature and other RAS signatures. As was the case with the breast panel, we saw a negative sign of correlation between our RAS and the Blum RAS signature. The Jacks original RAS signature shows significant correlation with our RAS signature in lung and lymphoma panels, but not in the colon panel. Interestingly, the dynamic range of our RAS signature in colon panel was rather narrow for all but two cell lines, which have very negative scores (discussed below). The dynamic range in lymphoma and lung was similar to what was observed in breast.

Example 4: Prediction of RAS mutations in cell lines and tumors by RAS pathway signature

We then assessed the ability of our RAS pathway signature to predict RAS mutations in cell lines (Figures 5A, B, C). Cell lines were grown in 10% fetal calf serum in tissue culture plates. RNA was extracted using RNEasy kits according to manufacturer instructions. Cell lines were profiled at baseline on Agilent gene expression microarrays. KRas mutation status was obtained from publicly available data sources (Sanger Center database). RAS pathway signature score was calculated and samples were classified as previously described.

All but one lung cell line with RAS mutations had positive signature scores (Figure 5B), while 63% of RAS wt cell lines had negative scores. Thus, our signature has a high sensitivity but low specificity of prediction of RAS mutation status. Low specificity can be attributed to mutations in other members of the RAS pathway in RAS wt cell lines (for example, BRAF), which would contribute to high RAS signature scores in RAS wildtype cell lines. Qualitatively, we can see that BRAF contributes to RAS score in colon and breast cell lines.

In colon cell lines the situation is more complicated. Our RAS signature has a low dynamic range for all but one cell line (for the other cell line with low signature score shown in Figure 5A, mutation status is not known). Distributions of signature scores are similar for RAS mutant and RAS wt colon cell line groups, with slightly higher scores for RAS mutant cell lines compared to RAS wt. This observation can be attributed to BRAF mutant status of RAS wt cell lines. Indeed, RAS wt cell lines with BRAF mutations have higher scores than those without BRAF mutations. Four out of five BRAF wt RAS wt cell lines have negative scores, including the cell line with the lowest signature score.

For breast cell lines (Figure 5C), there are only two cell lines with RAS mutations and both of them show highly positive RAS scores. Among RAS wt breast cell lines 30 % have high RAS signature scores. Again, it is possible that this can be attributed to other mutations in the pathway among RAS wt, BRAF wt cell lines.

Figure 6 shows that the RAS pathway signature is able to accurately predict RAS mutations in NSCLC tumor samples (11/12 correct predictions). Tumors were extracted from patients, flash frozen, macrodissected, and then RNA was extracted using RNEasy kits according to manufacturer instructions. Tumors were profiled at baseline on Affymetrix gene expression microarrays. KRas mutation status was obtained by targeted genotyping of the KRas gene. RAS pathway signature score was calculated as previously described.

Example 5: RAS pathway signature geneset is coherent in human tumors and can be used to rank tumors based on score

The coherence of our RAS signature was assessed in fresh frozen tumor samples from lung, breast, colon, and gastric tumors and in FFPE samples from lung, breast and ovarian tumors. RAS pathway signatures score was calculated and samples were classified as previously described. In fresh tumors the signature was significantly coherent across all tumor types (data not shown). The significance of coherence in breast tumors was highest when scored in triple negative tumors only. In FFPE samples, our RAS pathway signature coherence was high in all available tumor types: lung (Figure 7A), ovarian (Figure 7C) and breast (Figure 7E). Each of these datasets showed coherency of the "Up" and "Down" arms of our RAS signature with p-value less than $10^{-10}$. We also observed signifcant correlation between our RAS signature and published RAS signatures in FFPE samples in lung (Figure 7B), ovarian (Figure 7D), and breast (Figure 7F).

Example 6: RAS pathway signature predicts the prevalence of RAS deregulation in tumor subtypes

We then assessed the expression of our RAS pathway signature in tumor datasets with available histology information to predict the prevalence of RAS pathway activation in tumor subtypes. In ovarian tumors, we observed a high prevalence of RAS pathway deregulation in the Carcinoma, Clear Cell Adenocarcinoma, and Andometroid Cystadenoma subtypes, while we observed a low prevalence of RAS pathway activation in Papilary Serous Adenocarcinoma, Benign Serous Adenoma, and Adenoma (Figure 8).

In non-small cell lung tumors, our RAS pathway signature was differentially expressed between squamous cell carcinoma and adenocarcinoma subtypes (Figure 9). Expression data suggest very low incidence of RAS pathway deregulation in squamous cell carcinoma and approximately 70-75% rate of RAS pathway deregulation in adenocarcinoma. Our cell line data suggest that RAS pathway deregulation is very low in small cell lung cancer as well.

In breast tumors, RAS signature levels were variable across tumor subtypes. In triple negative tumors (HER2-, ER-, PR-), RAS deregulation was observed in about half of the cases. Combined with the growth factor signature, which is high in most of the triple negative tumors, RAS pathway signature low/Growth Factor Signaling Pathway high tumors comprise 48% triple negative tumors (Figure 10B). See PCT application, "Methods and Gene Expression Signature for Assessing Growth Factor Signaling Pathway Regulation Status" by James Watters et al., filed on March 19, 2009, for description and methods of using Growth Factor Signaling Pathway biomarkers.

The RAS pathway signature score was also calculated in a dataset of fresh frozen tumor specimens from various tissues of origin. The distribution of the scores was plotted across tumor types (Figure 11), showing that the RAS pathway signature score can be used to rank tumors according to RAS pathway deregulation.

Example 7: K-RAS siRNA knockdown suggests that RAS pathway signature is more redictive of RAS dependence than K-RAS mutational status

K-RAS mutant lung cancer cell lines with high or low RAS pathway signature scores were treated with siRNAs targeting *K-RAS* gene, and the effects on cell viability was assessed using the ATP vialight assay (Lonza Rockland, Inc., Rockland, ME) (Figure 12).

Example 8: Upregulation of RAS pathway signature is associated with acquired resistance to AKT inhibitor in breast cancer cell lines

High baseline levels of our RAS pathway signature predict resistance to a small molecule inhibitor of AKT (MK-6673; WO2006/135627), a central mediator of PI3K pathway signaling, in a panel of breast cancer cell lines, none of which harbor a KRas mutation (Figure 13). Cells were profiled at baseline on Agilent gene expression microarrays. Sensitivity to and AKTi was determined by incubating cells with MK-6673 for 72 hours and assessing viability using the ATPlite assay (Perkin Elmer, Waltham, MA). RAS pathway signature score was calculated as previously described. Defining resistant cell lines as those with % inhibition <60% and sensitive as those with % inhibition>60%, our RAS signature achieved 78% classifcation accuracy (p-value by Fisher Exact test<0.002).

To further investigate mechanisms by which tumor cells may acquire resistance to AKT inhibition, we generated resistant versions of two normally drug sensitive breast cancer cell lines by long-term culture in the presence of increasing doses of another AKT compound (MK-2206; WO2008/070016). Gene expression profiling of surviving cells demonstrated that resistance was achieved by up-regulation of the RAS pathway signature in both cell lines (Figure 14).

Example 9: RAS pathway signature predicts response to MEK inhibitor

The RAS pathway signature predicts sensitivity to inhibition of MEK, a key component of RAS pathway signaling. In a panel of approximately 100 lung cancer cell lines, high baseline RAS pathway signature scores predicts sensitivity to MEK inhibition (MEKi385, also known as PD 0325901 (*N*-(2,3-dihydroxy-propoxy)-3,4-difluoro-2-(2-fluoro-4-iodo-phenylamino)-benzamide), Barrett et al., 2008, Bioorg. Med. Chem. Lett. 18:6501-4) (Figure 15). Cells were profiled at baseline on Affymetrix gene expression microarrays as previously described. RAS pathway signature scores were calculate dna samples were classified as previously described. Sensitivity to MEKi was determined by incubating cells with drug for 72 hours and assessing viability using the ATPlite assay (Perkin Elmer, Waltham, MA). Importantly, this relationship between the RAS pathway signature and MEK inhibition was observed in both KRas mutant (Figure 16) and KRas wildtype cell lines (Figure 17).

Example 10: RAS pathway signature can be used to measure pharmacodynamic effect of an agent *in vivo* on the RAS pathway

The RAS pathway signature has been shown to predict RAS pathway deregulation in cell lines and tumor samples and predict response of cell lines to RAS pathway inhibitors. Next, we wished to investigate whether RAS pathway signature scores would decrease *in vivo* following treatment with a RAS pathway inhibitor in a RAS driven animal model.

Mice carrying p53 loss of function and KRas mutant lung tumors were selected as the cancer model (7450 KP-model, whose genetics is $KRas^{LSL-G12D}$; $P53^{LSL-R270HFL}$). In this mouse model, oncogenic KRas initiates and drives tumor development; mutant p53 increases tumor aggressiveness. KP mice develop lung adenocarcinomas with an average survival time of ~12 weeks after being given 2-5 x $10^7$ pfu AdenoCre intranasally. The 7450 KP-model closely mimics human lung cancer progression.

The 7450 KP mice were treated with a small molecule MEK inhibitor, AZD6244 (Ohren et al., 2004, Nat. Struct. Mol. Biol. 11:119201197). AZD6244 is a potent and selective noncompetitive inhibitor of MEK1 and MEK2, with an *in vitro* IC50 of 10 to 14 nmol/L against purified enzyme. AZD6244 significantly halted tumor progression compared vehicle after 4 weeks of treatment (data not shown). AZD6244 is cleared quickly post-dost in CD-1 nude mice; blood concentration of AZD6244 peaked before 2 hours and then decreased rapidly (data not shown). 7450 KP-model mice were treated with AZD6244 (150 mpk; n=12 per time point) or vehicle with three doses timed 10 hours apart. Tumors were extracted at 0, 4, and 24 hours post last dose and subjected to gene expression profiling as previously described. RAS signature pathway signatures scores were calculated as previously described for each time point and plotted (Figure 18). As shown in Figures 18 and 19, RAS pathway signature is down-regulated by MEK inhibitor AZD6244 *in vivo* at 4 hours but not at 24 hours, consistent with the compounds short half-life *in vivo.* This data suggests that the RAS pathway signature could be used as an early readout of compound efficacy.

## Claims

1. A method for predicting response of a human subject with cancer to an agent that modulates the RAS signaling pathway, said method comprising:

   (a) classifying said human subject as having a deregulated or regulated RAS signaling pathway, wherein said classifying comprises:

      (i) calculating a measure of similarity between a first expression profile and a regulated RAS signaling pathway template, said first expression profile comprising the expression levels of a first plurality of genes in a cancer cell sample derived from said human subject, said regulated RAS signaling pathway template comprising expression levels of said first plurality of genes that are average expression levels of the respective genes in a plurality of human control cell samples not having at least one or more effectors and mediators of said RAS signaling pathway with abnormal activity, said first plurality of genes consisting of at least 5 of the genes for which biomarkers are listed in Tables 2a and 2b, wherein at least 1 gene of said 5 genes is selected from Table 2b;
      (ii) classifying said cell sample as having said regulated RAS signaling pathway if said first expression profile has a high similarity to said regulated RAS signaling pathway template, or classifying said cell sample as having said deregulated RAS signaling pathway if said first expression profile has a low similarity to said regulated RAS signaling pathway template; wherein said first expression profile has a high similarity to said regulated RAS signaling pathway template if the similarity to said regulated RAS signaling pathway template is above a predetermined threshold, or has a low similarity to said regulated RAS signaling pathway template if the similarity to said regulated RAS signaling pathway template is below said predetermined threshold; wherein the pre-determined threshold may be O, or may be the mean, median or a percentile of signature scores of a collection of samples or a pooled sample used as a standard or control and

wherein said human subject is predicted to respond to said agent if said cell sample is classified as having a deregulated RAS signaling pathway.

**2.** A method for predicting response of a human subject with cancer to an agent that modulates the RAS signaling pathway and/or PI3K signaling pathway, said method comprising:

(a) classifying said human subject as having a deregulated or regulated RAS signaling pathway, wherein said classifying comprises:

(i) calculating a signature score by a method comprising: a) calculating a differential expression value of a first expression level of each of a first plurality of genes and each of a second plurality of genes in a cancer cell sample derived from said human subject relative to a second expression level of each of said first plurality of genes and each of said second plurality of genes in an human control cell sample, said first plurality of genes consisting of at least 3 or more of the genes for which biomarkers are listed in Table 2a and said second plurality of genes consisting of at least 3 or more of the genes for which biomarkers are listed in Table 2b; b) calculating the mean differential expression values of the expression levels of said first plurality of genes and said second plurality of genes; and c) subtracting said mean differential expression value of said second plurality of genes from said mean differential expression value of said first plurality of genes to obtain said signature score;
(ii) classifying said cell sample as having a deregulated RAS signaling pathway: a) if said obtained signature score is above a predetermined threshold wherein the pre-determinded threshold may be O, or may be the mean, median or a percentile of signature scores of a collection of samples or a pooled sample used as a standard or control and b) if said signature score is statistically significant;

wherein said human subject is predicted to respond to said agent if said cell sample is classified as having a deregulated RAS signaling pathway.

**3.** A method of assigning treatment to a human subject with cancer, said method comprising:

(a) classifying said human subject as having a deregulated or regulated RAS signaling pathway, wherein said classifying comprises:

(i) calculating a signature score by a method comprising: a) calculating a differential expression value of a first expression level of each of a first plurality of genes and each of a second plurality of genes in a cancer cell sample derived from said human subject relative to a second expression level of each of said first plurality of genes and each of said second plurality of genes in an human control cell sample, said first plurality of genes consisting of at least 3 or more of the genes for which biomarkers are listed in Table 2a and said second plurality of genes consisting of at least 3 or more of the genes for which biomarkers are listed in Table 2b; b) calculating the mean differential expression values of the expression levels of said first plurality of genes and said second plurality of genes; and c) subtracting said mean differential expression value of said second plurality of genes from said mean differential expression value of said first plurality of genes to obtain said signature score;
(ii) classifying said cell sample as having a deregulated RAS signaling pathway a) if said obtained signature score is above a predetermined threshold wherein the pre-determinded threshold may be O, or may be the mean, median or a percentile of signature scores of a collection of samples or a pooled sample used as a standard or control and b) if said signature score is statistically significant;

(b) assigning said human subject for treatment with an agent that modulates the RAS signaling pathway, if said cell sample is classified as having deregulated RAS signaling pathway.

**4.** A method of assigning treatment to a human subject with cancer, said method comprising:

(a) classifying said human subject as having a deregulated or regulated RAS signaling pathway, wherein said classifying comprises:

(i) calculating a signature score by a method comprising: a) calculating a differential expression value of a first expression level of each of a first plurality of genes and each of a second plurality of genes in a cancer cell sample derived from said human subject relative to a second expression level of each of said first plurality of genes and each of said second plurality of genes in an human control cell sample, said first plurality of genes consisting of at least 3 or more of the genes for which biomarkers are listed in Table 2a and said second plurality of genes consisting of at least 3 or more of the genes for which biomarkers are listed in Table 2b; b) calculating the mean differential expression values of the expression levels of said first plurality of genes and said second plurality of genes; and c) subtracting said mean differential expression value of said second plurality of genes from said mean differential expression value of said first plurality of genes to obtain said signature score;
(ii) classifying said cell sample as having a deregulated RAS signaling pathway a) if said obtained signature score is above a predetermined threshold wherein the pre-determinded threshold may be O, or may be the mean, median or a percentile of signature scores of a collection of samples or a pooled sample used as a standard or control and b) if said signature score is statistically significant;

(b) not assigning said human subject for treatment with an agent that modulates the PI3K signaling pathway, if said cell sample is classified as having deregulated RAS signaling pathway.

**5.** The method of any one of claims 2-4, wherein said first plurality of genes consists of at least 5 or more of the genes for which biomarkers are listed in Table 2a and said second plurality of genes consists of at least 5 or more genes for which biomarkers are listed in Table 2b.

**6.** The method of any one of claims 2-4, wherein said first plurality of genes consists of at least 10 or more of the genes for which biomarkers are listed in Table 2a and said second plurality of genes consists of at least 10 or more genes for which biomarkers are listed in Table 2b.

**7.** The method of any one of claims 2-4, wherein said first plurality of genes consists of at least 20 or more of the genes for which biomarkers are listed in Table 2a and said second plurality of genes consists of at least 20 or more genes for which biomarkers are listed in Table 2b.

**8.** The method of any one of claims 2-4, wherein said first plurality of genes consists of all of the genes listed in Table 2a and said second plurality of genes consists of all of the genes for which biomarkers are listed in Table 2b.

**9.** The method of any one of claims 2-4, wherein said differential expression value is log(10) ratio.

**10.** The method of any one of claims 2-4, wherein said threshold is 0.

**11.** The method of any one of claims 2-4, wherein said signature scores is statistically significant if it has a p-value less than 0.05.

**12.** The method of any previous claim, wherein said agent is a PI3K inhibitor.

**13.** The method of any one of claims 1-11, wherein said agent is an AKT inhibitor.

**14.** The method of any one of claims 1-11, wherein said agent is a MEK inhibitor.

## Patentansprüche

**1.** Ein Verfahren zur Vorhersage der Reaktion eines menschlichen Subjekts mit Krebs auf ein Mittel, das den RAS-Signalweg moduliert, wobei das Verfahren umfasst:

(a) Klassifizieren des menschlichen Subjekts als einen deregulierten oder regulierten RAS-Signalweg besitzend, wobei diese Klassifizierung umfasst:

(i) Berechnen eines Ähnlichkeitsgrads zwischen einem ersten Expressionsprofil und einem Template des regulierten RAS-Signalwegs, wobei das erste Expressionsprofil die Expressionsgrade einer ersten Mehrzahl von Genen in einer Krebszellenprobe, die von dem menschlichen Subjekt stammt, umfasst, das Template des regulierten RAS-Signalwegs Expressionsgrade der ersten Mehrzahl von Genen umfasst, die mittlere Expressionsgrade der entsprechenden Gene in einer Mehrzahl von menschlichen Kontrollzellenproben ohne wenigstens einen oder mehrere Effektoren und Mediatoren des RAS-Signalwegs mit abnormaler Aktivität sind, die erste Mehrzahl von Genen aus wenigstens 5 der Gene, für die Biomarker in den Tabellen 2a und 2b aufgeführt sind, besteht, wobei wenigstens 1 Gen dieser 5 Gene ausgewählt ist aus Tabelle 2b,
(ii) Klassifizieren der Zellprobe als den regulierten RAS-Signalweg besitzend, wenn das erste Expressionsprofil eine hohe Ähnlichkeit mit dem Template des regulierten RAS-Signalwegs besitzt, oder Klassifizieren der Zellprobe als den deregulierten RAS-Signalweg besitzend, wenn das erste Expressionsprofil eine geringe Ähnlichkeit mit dem Template des regulierten RAS-Signalwegs besitzt, wobei das erste Expressionsprofil eine hohe Ähnlichkeit mit dem Template des regulierten RAS-Signalwegs besitzt, wenn die Ähnlichkeit mit dem Template des regulierten RAS-Signalwegs über einem vorbestimmten Schwellenwert liegt, oder eine geringe Ähnlichkeit mit dem Template des regulierten RAS-Signalwegs besitzt, wenn die Ähnlichkeit mit dem Template des regulierten RAS-Signalwegs unter dem vorbestimmten Schwellenwert liegt, wobei der vorbestimmte Schwellenwert 0 sein kann oder der Mittelwert, der Medianwert oder ein Perzentilwert von Signature-Scores einer Probenkollektion oder einer Mischprobe, die als Referenz oder Kontrolle verwendet wird, sein kann, und

wobei das menschliche Subjekt als auf das Mittel reagierend prognostiziert wird, wenn die Zellprobe als einen deregulierten RAS-Signalweg besitzend klassifiziert wird.

**2.** Ein Verfahren zur Vorhersage der Reaktion eines menschlichen Subjekts mit Krebs auf ein Mittel, das den RAS-Signalweg und/oder PI3K-Signalweg moduliert, wobei das Verfahren umfasst:

(a) Klassifizieren des menschlichen Subjekts als einen deregulierten oder regulierten RAS-Signalweg besitzend, wobei diese Klassifizierung umfasst:

(i) Berechnen eines Signature-Scores durch ein Verfahren, das umfasst: a) Berechnen eines Expressions-Differenzwerts eines ersten Expressionsgrades von jedem einer ersten Mehrzahl von Genen und jedem einer zweiten Mehrzahl von Genen in einer Krebszellenprobe, die von dem menschlichen Subjekt stammt, relativ zu einem zweiten Expressionsgrad von jedem der ersten Mehrzahl von Genen und jedem der zweiten Mehrzahl von Genen in einer menschlichen Kontrollzellenprobe, wobei die erste Mehrzahl von Genen aus wenigstens 3 oder mehr der Gene, für die Biomarker in Tabelle 2a aufgeführt sind, besteht und die zweite Mehrzahl von Genen aus wenigstens 3 oder mehr der Gene, für die Biomarker in Tabelle 2b aufgeführt sind, besteht, b) Berechnen der mittleren Expressions-Differenzwerte für die Expressionsgrade der ersten Mehrzahl von Genen und der zweiten Mehrzahl von Genen, und c) Subtrahieren des mittleren Expressions-Differenzwerts der zweiten Mehrzahl von Genen von dem mittleren Expressions-Differenzwert der ersten Mehrzahl von Genen, um den Signature-Score zu erhalten,
(ii) Klassifizieren der Zellprobe als einen deregulierten RAS-Signalweg besitzend, a) wenn der erhaltene Signature-Score über einem vorbestimmten Schwellenwert liegt, wobei der vorbestimmte Schwellenwert 0 sein kann oder der Mittelwert, der Medianwert oder ein Perzentilwert von Signature-Scores einer Probenkollektion oder einer Mischprobe, die als Referenz oder Kontrolle verwendet wird, sein kann, und b) wenn der Signature-Score statistisch signifikant ist,

wobei das menschliche Subjekt als auf das Mittel reagierend prognostiziert wird, wenn die Zellprobe als einen deregulierten RAS-Signalweg besitzend klassifiziert wird.

**3.** Ein Verfahren zum Zuweisen einer Behandlung einem menschlichen Subjekt mit Krebs, wobei das Verfahren umfasst:

(a) Klassifizieren des menschlichen Subjekts als einen deregulierten oder regulierten RAS-Signalweg besitzend, wobei diese Klassifizierung umfasst:

(i) Berechnen eines Signature-Scores durch ein Verfahren, das umfasst: a) Berechnen eines Expressions-Differenzwerts eines ersten Expressionsgrades von jedem einer ersten Mehrzahl von Genen und jedem einer zweiten Mehrzahl von Genen in einer Krebszellenprobe, die von dem menschlichen Subjekt stammt, relativ zu einem zweiten Expressionsgrad von jedem der ersten Mehrzahl von Genen und jedem der zweiten Mehrzahl von Genen in einer menschlichen Kontrollzellenprobe, wobei die erste Mehrzahl von Genen aus wenigstens 3 oder mehr der Gene, für die Biomarker in Tabelle 2a aufgeführt sind, besteht und die zweite Mehrzahl von Genen aus wenigstens 3 oder mehr der Gene, für die Biomarker in Tabelle 2b aufgeführt sind, besteht, b) Berechnen der mittleren Expressions-Differenzwerte für die Expressionsgrade der ersten Mehrzahl von Genen und der zweiten Mehrzahl von Genen, und c) Subtrahieren des mittleren Expressions-Differenzwerts der zweiten Mehrzahl von Genen von dem mittleren Expressions-Differenzwert der ersten Mehrzahl von Genen, um den Signature-Score zu erhalten,
(ii) Klassifizieren der Zellprobe als einen deregulierten RAS-Signalweg besitzend, a) wenn der erhaltene Signature-Score über einem vorbestimmten Schwellenwert liegt, wobei der vorbestimmte Schwellenwert 0 sein kann oder der Mittelwert, der Medianwert oder ein Perzentilwert von Signature-Scores einer Probenkollektion oder einer Mischprobe, die als Referenz oder Kontrolle verwendet wird, sein kann, und b) wenn der Signature-Score statistisch signifikant ist,

(b) dem menschlichen Subjekt eine Behandlung mit einem Mittel, das den RAS-Signalweg moduliert, zuweisen, wenn die Zellprobe als einen deregulierten RAS-Signalweg besitzend klassifiziert wird.

**4.** Ein Verfahren zum Zuweisen einer Behandlung einem menschlichen Subjekt mit Krebs, wobei das Verfahren umfasst:

(a) Klassifizieren des menschlichen Subjekts als einen deregulierten oder regulierten RAS-Signalweg besitzend, wobei diese Klassifizierung umfasst:

(i) Berechnen eines Signature-Scores durch ein Verfahren, das umfasst: a) Berechnen eines Expressions-Differenzwerts eines ersten Expressionsgrades von jedem einer ersten Mehrzahl von Genen und jedem einer zweiten Mehrzahl von Genen in einer Krebszellenprobe, die von dem menschlichen Subjekt stammt, relativ zu einem zweiten Expressionsgrad von jedem der ersten Mehrzahl von Genen und jedem der zweiten Mehrzahl von Genen in einer menschlichen Kontrollzellenprobe, wobei die erste Mehrzahl von Genen aus wenigstens 3 oder mehr der Gene, für die Biomarker in Tabelle 2a aufgeführt sind, besteht und die zweite Mehrzahl von Genen aus wenigstens 3 oder mehr der Gene, für die Biomarker in Tabelle 2b aufgeführt sind, besteht, b) Berechnen der mittleren Expressions-Differenzwerte für die Expressionsgrade der ersten Mehrzahl von Genen und der zweiten Mehrzahl von Genen, und c) Subtrahieren des mittleren Expressions-Differenzwerts der zweiten Mehrzahl von Genen von dem mittleren Expressions-Differenzwert der ersten Mehrzahl von Genen, um den Signature-Score zu erhalten,
(ii) Klassifizieren der Zellprobe als einen deregulierten RAS-Signalweg besitzend, a) wenn der erhaltene Signature-Score über einem vorbestimmten Schwellenwert liegt, wobei der vorbestimmte Schwellenwert 0 sein kann oder der Mittelwert, der Medianwert oder ein Perzentilwert von Signature-Scores einer Probenkollektion oder einer Mischprobe, die als Referenz oder Kontrolle verwendet wird, sein kann, und b) wenn der Signature-Score statistisch signifikant ist,

(b) dem menschlichen Subjekt keine Behandlung mit einem Mittel, das den PI3K-Singalweg moduliert, zuweisen, wenn die Zellprobe als einen deregulierten RAS-Signalweg besitzend klassifiziert wird.

**5.** Das Verfahren nach einem der Ansprüche 2-4, wobei die erste Mehrzahl von Genen aus wenigstens 5 oder mehr der Gene, für die Biomarker in Tabelle 2a aufgeführt sind, besteht und die zweite Mehrzahl von Genen aus wenigstens 5 oder mehr Genen, für die Biomarker in Tabelle 2b aufgeführt sind, besteht.

**6.** Das Verfahren nach einem der Ansprüche 2-4, wobei die erste Mehrzahl von Genen aus wenigstens 10 oder mehr der Gene, für die Biomarker in Tabelle 2a aufgeführt sind, besteht und die zweite Mehrzahl von Genen aus wenigstens 10 oder mehr Genen, für die Biomarker in Tabelle 2b aufgeführt sind, besteht.

**7.** Das Verfahren nach einem der Ansprüche 2-4, wobei die erste Mehrzahl von Genen aus wenigstens 20 oder mehr der Gene, für die Biomarker in Tabelle 2a aufgeführt sind, besteht und die zweite Mehrzahl von Genen aus wenigstens 20 oder mehr Genen, für die Biomarker in Tabelle 2b aufgeführt sind, besteht.

**8.** Das Verfahren nach einem der Ansprüche 2-4, wobei die erste Mehrzahl von Genen aus allen der in Tabelle 2a aufgeführten Genen besteht und die zweite Mehrzahl von Genen aus allen der Gene, für die Biomarker in Tabelle 2b aufgeführt sind, besteht.

**9.** Das Verfahren nach einem der Ansprüche 2-4, wobei der Expressions-Differenzwert ein log(10)-Verhältnis ist.

**10.** Das Verfahren nach einem der Ansprüche 2-4, wobei der Schwellenwert 0 ist.

**11.** Das Verfahren nach einem der Ansprüche 2-4, wobei der Signature-Score statistisch signifikant ist, wenn er einen p-Wert von weniger als 0,05 besitzt.

**12.** Das Verfahren nach einem vorhergehenden Anspruch, wobei das Mittel ein PI3K-inhibitor ist.

**13.** Das Verfahren nach einem der Ansprüche 1-11, wobei das Mittel ein AKT-Inhibitor ist.

**14.** Das Verfahren nach einem der Ansprüche 1-11, wobei das Mittel ein MEK-Inhibitor ist.

**Revendications**

**1.** Procédé de prédiction de la réponse d'un sujet humain atteint d'un cancer à un agent qui module la voie de signalisation RAS, ledit procédé comprenant :

(a) le classement dudit sujet humain comme présentant une voie de signalisation RAS dérégulée ou régulée, ledit classement comprenant :

(i) le calcul d'une mesure de similitude entre un premier profil d'expression et une matrice de voie de signalisation RAS régulée, ledit premier profil d'expression comprenant les niveaux d'expression d'une première pluralité de gènes dans un échantillon de cellules cancéreuses obtenu auprès dudit sujet humain, ladite matrice de voie de signalisation RAS régulée comprenant des niveaux d'expression de ladite première pluralité de gènes qui sont des niveaux d'expression moyens des gènes respectifs dans une pluralité d' échantillons de cellules témoins humaines ne présentant pas au moins un ou plusieurs effecteurs et médiateurs de ladite voie de signalisation RAS avec une activité anormale, ladite première pluralité de gènes étant constituée par au moins 5 des gènes pour lesquels des biomarqueurs sont répertoriés dans les tableaux 2a et 2b, au moins 1 gène desdits 5 gènes étant sélectionné dans le tableau 2b ;
(ii) le classement dudit échantillon de cellules comme présentant ladite voie de signalisation RAS régulée si ledit premier profil d'expression présente une forte similitude avec ladite matrice de voie de signalisation RAS régulée, ou le classement dudit échantillon de cellules comme présentant ladite voie de signalisation RAS dérégulée si ledit premier profil d'expression présente une faible similitude avec ladite matrice de voie de signalisation RAS régulée ; ledit premier profil d'expression présentant une forte similitude avec ladite matrice de voie de signalisation RAS régulée si la similitude avec ladite matrice de voie de signalisation RAS régulée est supérieure à un seuil prédéterminé, ou présentant une faible similitude avec ladite matrice de voie de signalisation RAS régulée si la similitude avec ladite matrice de voie de signalisation RAS régulée est inférieure audit seuil prédéterminé ; le seuil prédéterminé pouvant être 0, ou pouvant être la moyenne, la médiane ou un percentile de scores de signature d'un recueil d'échantillons ou d'un échantillon global utilisé en tant qu'étalon ou témoin et

ledit sujet humain étant prévu pour répondre audit agent si ledit échantillon de cellules est classé comme présentant une voie de signalisation RAS dérégulée.

**2.** Procédé de prédiction d'une réponse d'un sujet humain atteint d'un cancer à un agent qui module la voie de signalisation RAS et/ou la voie de signalisation PI3K, ledit procédé comprenant :

(a) le classement dudit sujet humain comme présentant une voie de signalisation RAS dérégulée ou régulée, ledit classement comprenant :

(i) le calcul d'un score de signature par un procédé comprenant : a) le calcul d'une valeur d'expression différentielle d'un premier niveau d'expression de chacun d'une première pluralité de gènes et de chacun d'une deuxième pluralité de gènes dans un échantillon de cellules cancéreuses obtenu auprès d'un sujet humain par rapport à un deuxième niveau d'expression de chacun de ladite première pluralité de gènes et de chacun de ladite deuxième pluralité de gènes dans un échantillon de cellules témoins humaines, ladite première pluralité de gènes étant constituée par au moins 3 ou plus des gènes pour lesquels des biomarqueurs sont répertoriés dans le tableau 2a et ladite deuxième pluralité de gènes étant constituée par au moins 3 ou plus des gènes pour lesquels des biomarqueurs sont répertoriés dans le tableau 2b ; b) le calcul des valeurs d'expression différentielle moyennes des niveaux d'expression de ladite première pluralité de gènes et de ladite deuxième pluralité de gènes ; et c) la soustraction de ladite valeur d'expression différentielle moyenne de ladite deuxième pluralité de gènes à ladite valeur d'expression différentielle moyenne de ladite première pluralité de gènes pour obtenir ledit score de signature ;
(ii) le classement dudit échantillon de cellules comme présentant une voie de signalisation RAS dérégulée : a) si ledit score de signature obtenu est supérieur à un seuil prédéterminé, ledit score prédéterminé pouvant être 0, ou pouvant être la moyenne, la médiane ou un percentile de scores de signature d'un recueil d' échantillons ou d'un échantillon global utilisé en tant qu'étalon ou témoin et b) si ledit score de signature est significatif sur le plan statistique ;

ledit sujet humain étant prévu pour répondre audit agent si ledit échantillon de cellules est classé comme présentant une voie de signalisation RAS dérégulée.

**3.** Procédé d'attribution d'un traitement à un sujet humain atteint d'un cancer, ledit procédé comprenant :

(a) le classement dudit sujet humain comme présentant une voie de signalisation RAS dérégulée ou régulée, ledit classement comprenant :

(i) le calcul d'un score de signature par un procédé comprenant : a) le calcul d'une valeur d'expression différentielle d'un premier niveau d'expression de chacun d'une première pluralité de gènes et de chacun d'une deuxième pluralité de gènes dans un échantillon de cellules cancéreuses obtenu auprès d'un sujet humain par rapport à un deuxième niveau d'expression de chacun de ladite première pluralité de gènes et de chacun de ladite deuxième pluralité de gènes dans un échantillon de cellules témoins humaines, ladite première pluralité de gènes étant constituée par au moins 3 ou plus des gènes pour lesquels des biomarqueurs sont répertoriés dans le tableau 2a et ladite deuxième pluralité de gènes étant constituée par au moins 3 ou plus des gènes pour lesquels des biomarqueurs sont répertoriés dans le tableau 2b ; b) le calcul des valeurs d'expression différentielle moyennes des niveaux d'expression de ladite première pluralité de gènes et de ladite deuxième pluralité de gènes ; et c) la soustraction de ladite valeur d'expression différentielle moyenne de ladite deuxième pluralité de gènes à ladite valeur d'expression différentielle moyenne de ladite première pluralité de gènes pour obtenir ledit score de signature ;
(ii) le classement dudit échantillon de cellules comme présentant une voie de signalisation RAS dérégulée a) si ledit score de signature obtenu est supérieur à un seuil prédéterminé, le seuil prédéterminé pouvant être 0, ou pouvant être la moyenne, la médiane ou un percentile de scores de signature d'un recueil d' échantillons ou d'un échantillon global utilisé en tant qu'étalon ou témoin et b) si ledit score de signature est significatif sur le plan statistique ;

(b) l'attribution audit sujet humain d'un traitement avec un agent qui module la voie de signalisation RAS, si ledit échantillon de cellules est classé comme présentant une voie de signalisation RAS dérégulée.

**4.** Procédé d'attribution d'un traitement à un sujet humain atteint d'un cancer, ledit procédé comprenant :

(a) le classement dudit sujet humain comme présentant une voie de signalisation RAS dérégulée ou régulée, ledit classement comprenant :

(i) le calcul d'un score de signature par un procédé comprenant : a) le calcul d'une valeur d'expression différentielle d'un premier niveau d'expression de chacun d'une première pluralité de gènes et de chacun d'une deuxième pluralité de gènes dans un échantillon de cellules cancéreuses obtenu auprès dudit sujet humain par rapport à un deuxième niveau d'expression de chacun de ladite première pluralité de gènes et de chacun de ladite deuxième pluralité de gènes dans un échantillon de cellules témoins humaines, ladite première pluralité de gènes étant constituée par au moins 3 ou plus des gènes pour lesquels des biomarqueurs sont répertoriés dans le tableau 2a et ladite deuxième pluralité de gènes étant constituée par au moins 3 ou plus des gènes pour lesquels des biomarqueurs sont répertoriés dans le tableau 2b ; b) le calcul des valeurs d'expression différentielle moyennes des niveaux d'expression de ladite première pluralité de gènes et de ladite deuxième pluralité de gènes ; et c) la soustraction de ladite valeur d'expression différentielle moyenne de ladite deuxième pluralité de gènes à ladite valeur d'expression différentielle moyenne de ladite première pluralité de gènes pour obtenir ledit score de signature ;
(ii) le classement dudit échantillon de cellules comme présentant une voie de signalisation RAS dérégulée a) si ledit score de signature obtenu est supérieur à un seuil prédéterminé, le seuil prédéterminé pouvant être 0, ou pouvant être la moyenne, la médiane ou un percentile de scores de signature d'un recueil d' échantillons ou d'un échantillon global utilisé en tant qu'étalon ou témoin et b) si ledit score de signature est significatif sur le plan statistique ;

(b) la non-attribution audit sujet humain d'un traitement avec un agent qui module la voie de signalisation PI3K, si ledit échantillon de cellules est classé comme présentant une voie de signalisation RAS dérégulée.

**5.** Procédé selon l'une quelconque des revendications 2-4, ladite première pluralité de gènes étant constituée par au moins 5 ou plus des gènes pour lesquels des biomarqueurs sont répertoriés dans le tableau 2a et ladite deuxième pluralité de gènes étant constituée par au moins 5 ou plus des gènes pour lesquels des biomarqueurs sont répertoriés dans le tableau 2b.

**6.** Procédé selon l'une quelconque des revendications 2-4, ladite première pluralité de gènes étant constituée par au moins 10 ou plus des gènes pour lesquels des biomarqueurs sont répertoriés dans le tableau 2a et ladite deuxième pluralité de gènes étant constituée par au moins 10 ou plus des gènes pour lesquels des biomarqueurs sont répertoriés dans le tableau 2b.

**7.** Procédé selon l'une quelconque des revendications 2-4, ladite première pluralité de gènes étant constituée par au moins 20 ou plus des gènes pour lesquels des biomarqueurs sont répertoriés dans le tableau 2a et ladite deuxième pluralité de gènes étant constituée par au moins 20 ou plus des gènes pour lesquels des biomarqueurs sont répertoriés dans le tableau 2b.

**8.** Procédé selon l'une quelconque des revendications 2-4, ladite première pluralité de gènes étant constituée par la totalité des gènes répertoriés dans le tableau 2a et ladite deuxième pluralité de gènes étant constituée par la totalité des gènes pour lesquels des biomarqueurs sont répertoriés dans le tableau 2b.

**9.** Procédé selon l'une quelconque des revendications 2-4, ladite valeur d'expression différentielle étant un rapport log(10).

**10.** Procédé selon l'une quelconque des revendications 2-4, ledit seuil étant 0.

**11.** Procédé selon l'une quelconque des revendications 2-4, lesdits scores de signature étant significatifs sur le plan statistique s'ils ont une valeur p inférieure à 0,05.

**12.** Procédé selon l'une quelconque des revendications précédentes, ledit agent étant un inhibiteur de PI3K.

**13.** Procédé selon l'une quelconque des revendications 1-11, ledit agent étant un inhibiteur de AKT.

**14.** Procédé selon l'une quelconque des revendications 1-11, ledit agent étant un inhibiteur de MEK.

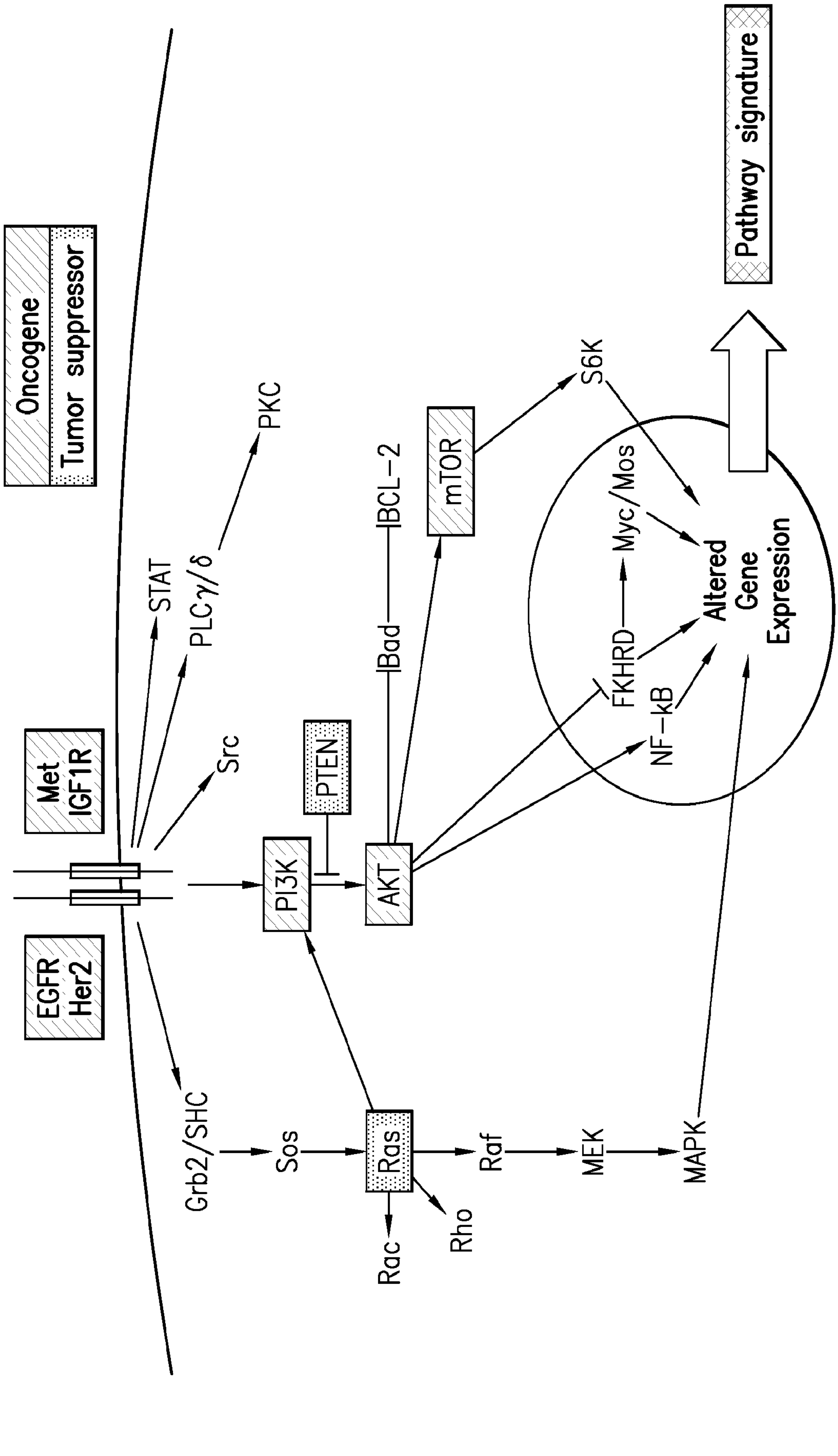
Oncogene
Tumor suppressor
Pathway signature
EGFR
Her2
Met
IGF1R
Grb2/SHC
Sos
Ras
Rac
Rho
Raf
MEK
MAPK
PI3K
PTEN
AKT
Src
STAT
PLCγ/δ
PKC
Bad
BCL-2
mTOR
S6K
FKHRD
NF-kB
Myc/Mos
Altered
Gene
Expression

FIG. 1

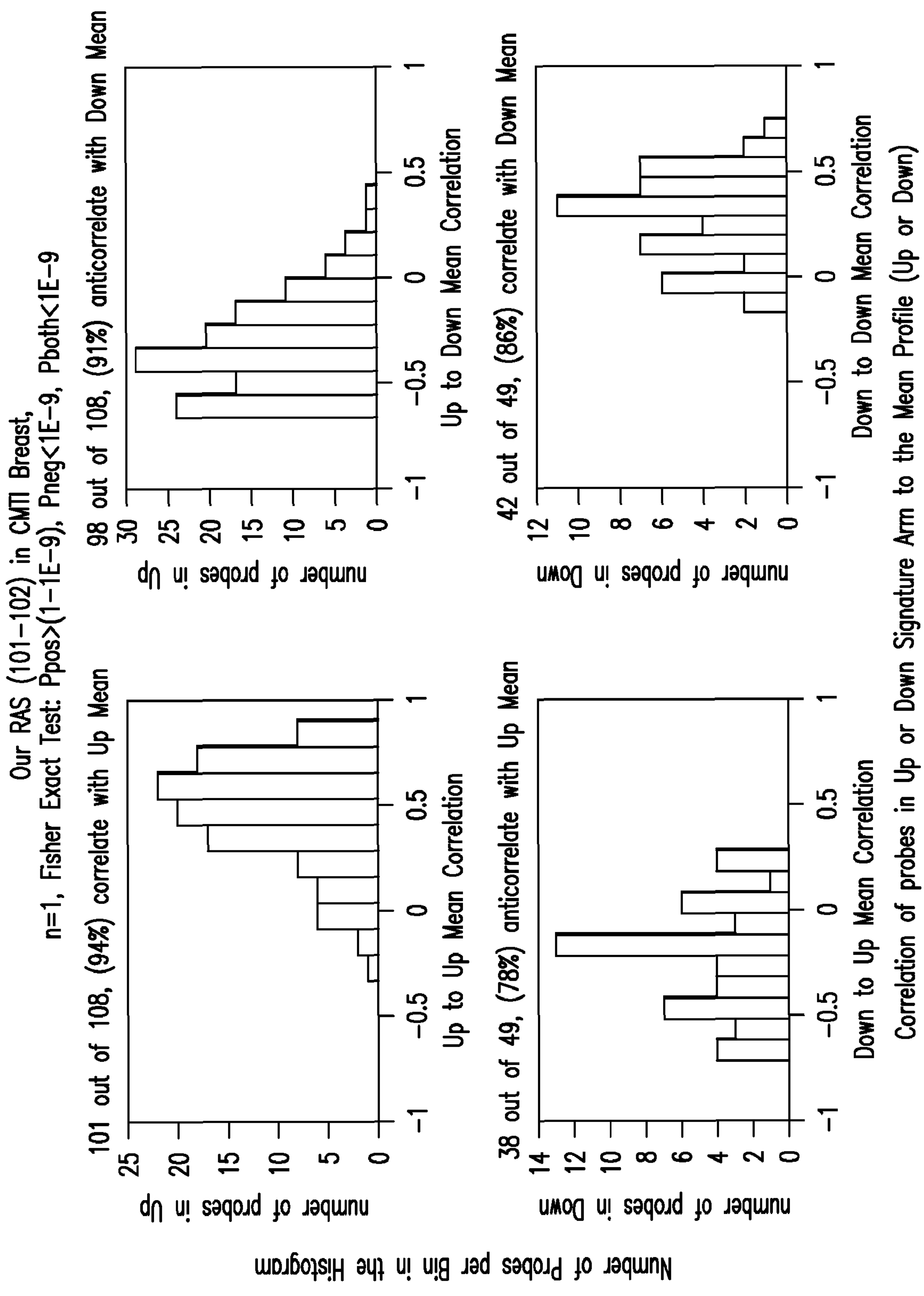
Our RAS (101-102) in CMTI Breast,
n=1, Fisher Exact Test: Ppos>(1-1E-9), Pneg<1E-9, Pboth<1E-9
101 out of 108, (94%) correlate with Up Mean
98 out of 108, (91%) anticorrelate with Down Mean
38 out of 49, (78%) anticorrelate with Up Mean
42 out of 49, (86%) correlate with Down Mean
number of probes in Up
number of probes in Down
Up to Up Mean Correlation
Up to Down Mean Correlation
Down to Up Mean Correlation
Down to Down Mean Correlation
Number of Probes per Bin in the Histogram
Correlation of probes in Up or Down Signature Arm to the Mean Profile (Up or Down)

FIG.2A

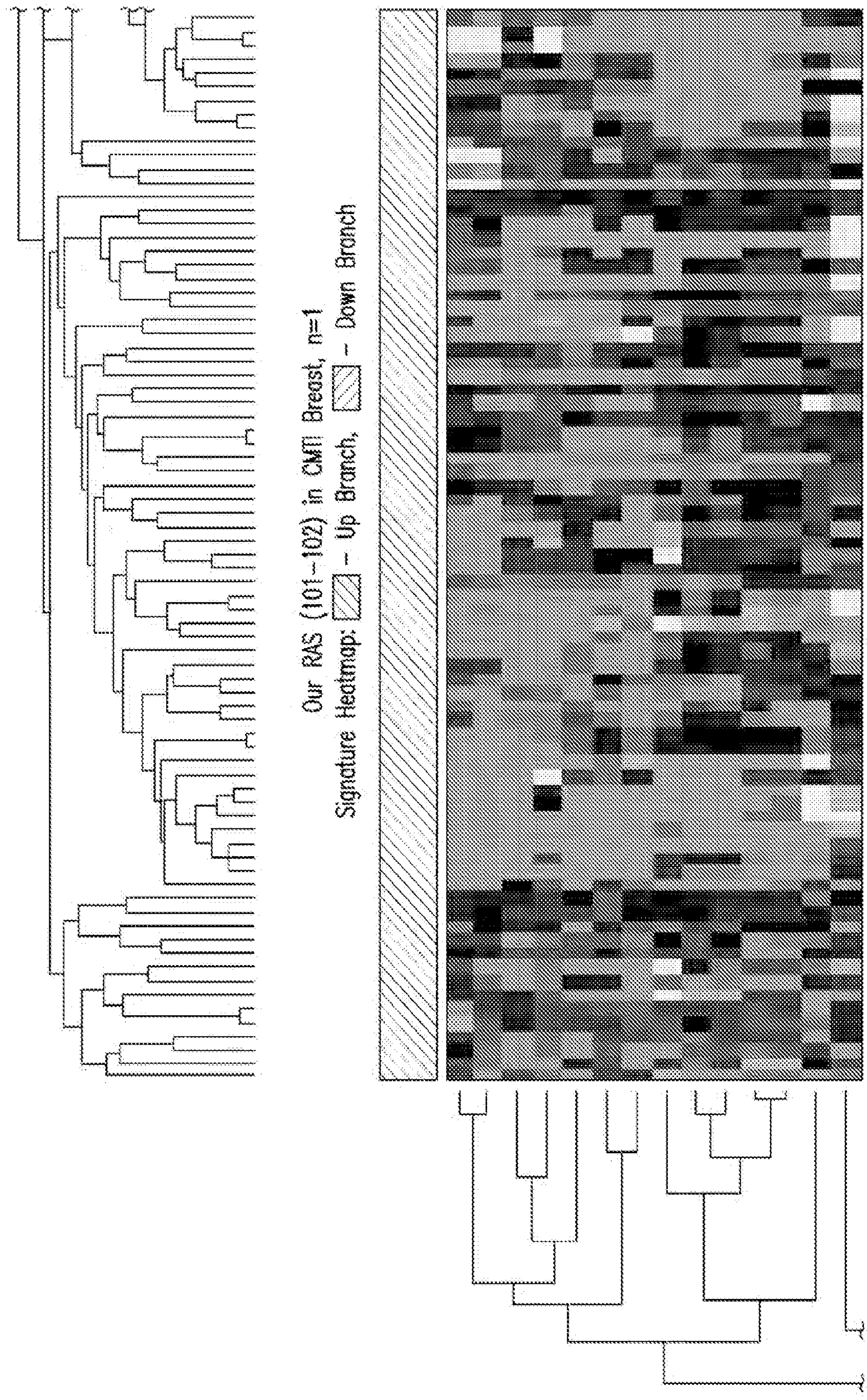
Our RAS (101-102) in CMT Breast, n=1
Signature Heatmap: - Up Branch, - Down Branch

FIG.2B-1

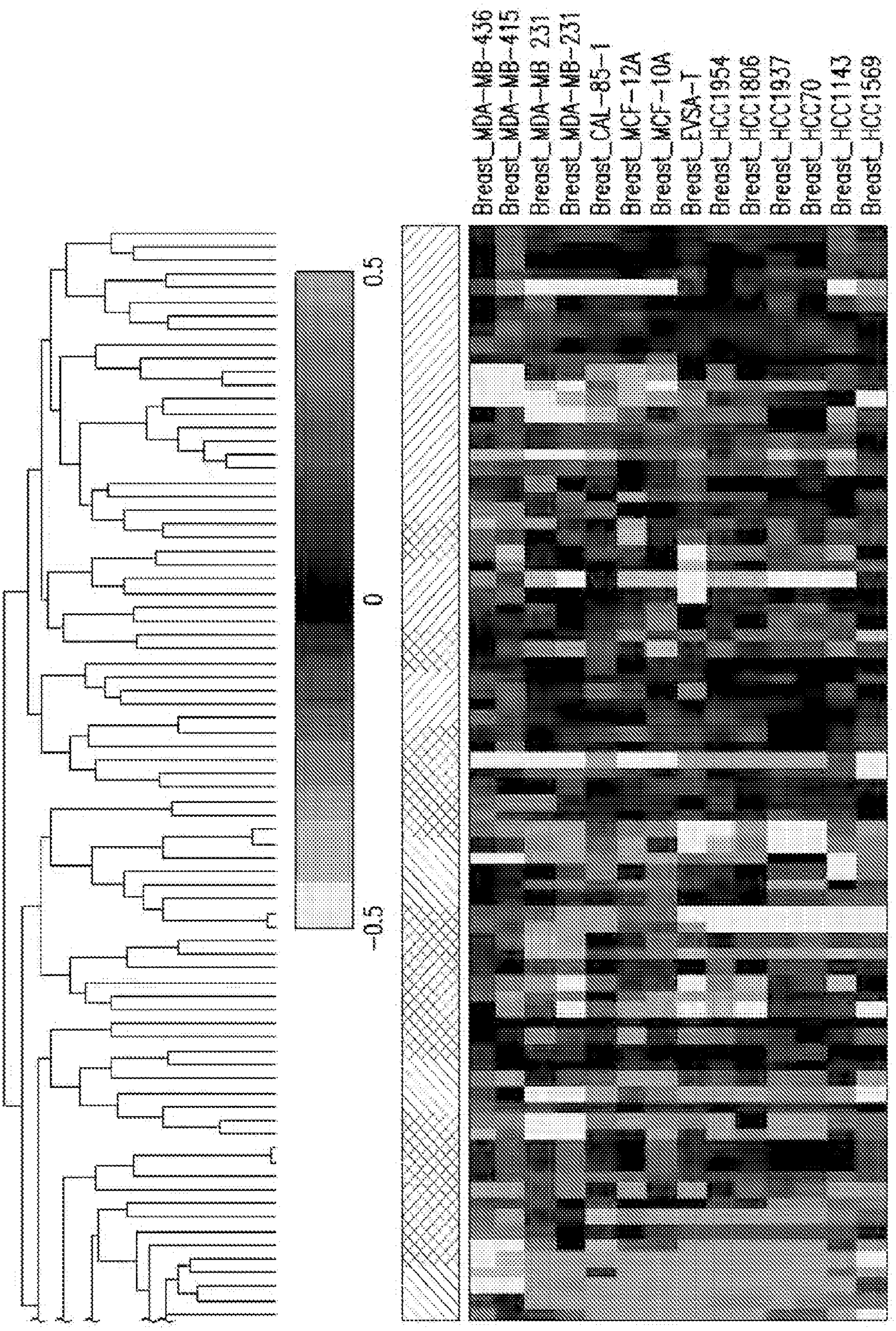
0.5
0
-0.5
Breast_MDA-MB-436
Breast_MDA-MB-415
Breast_MDA-MB 231
Breast_MDA-MB-231
Breast_CAL-85-1
Breast_MCF-12A
Breast_MCF-10A
Breast_EVSA-T
Breast_HCC1954
Breast_HCC1806
Breast_HCC1937
Breast_HCC70
Breast_HCC1143
Breast_HCC1569

FIG.2B-2

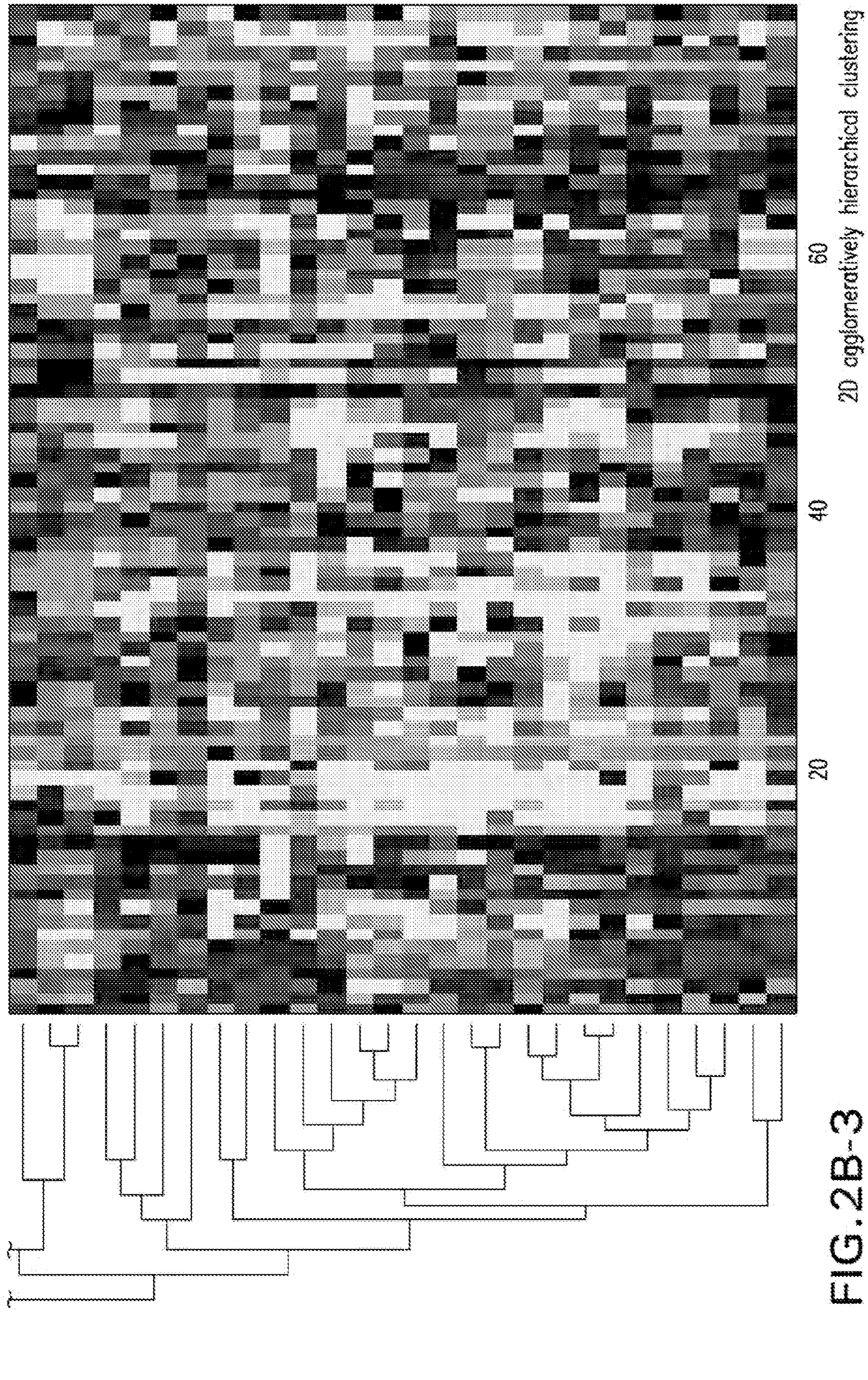


FIG. 2B-3

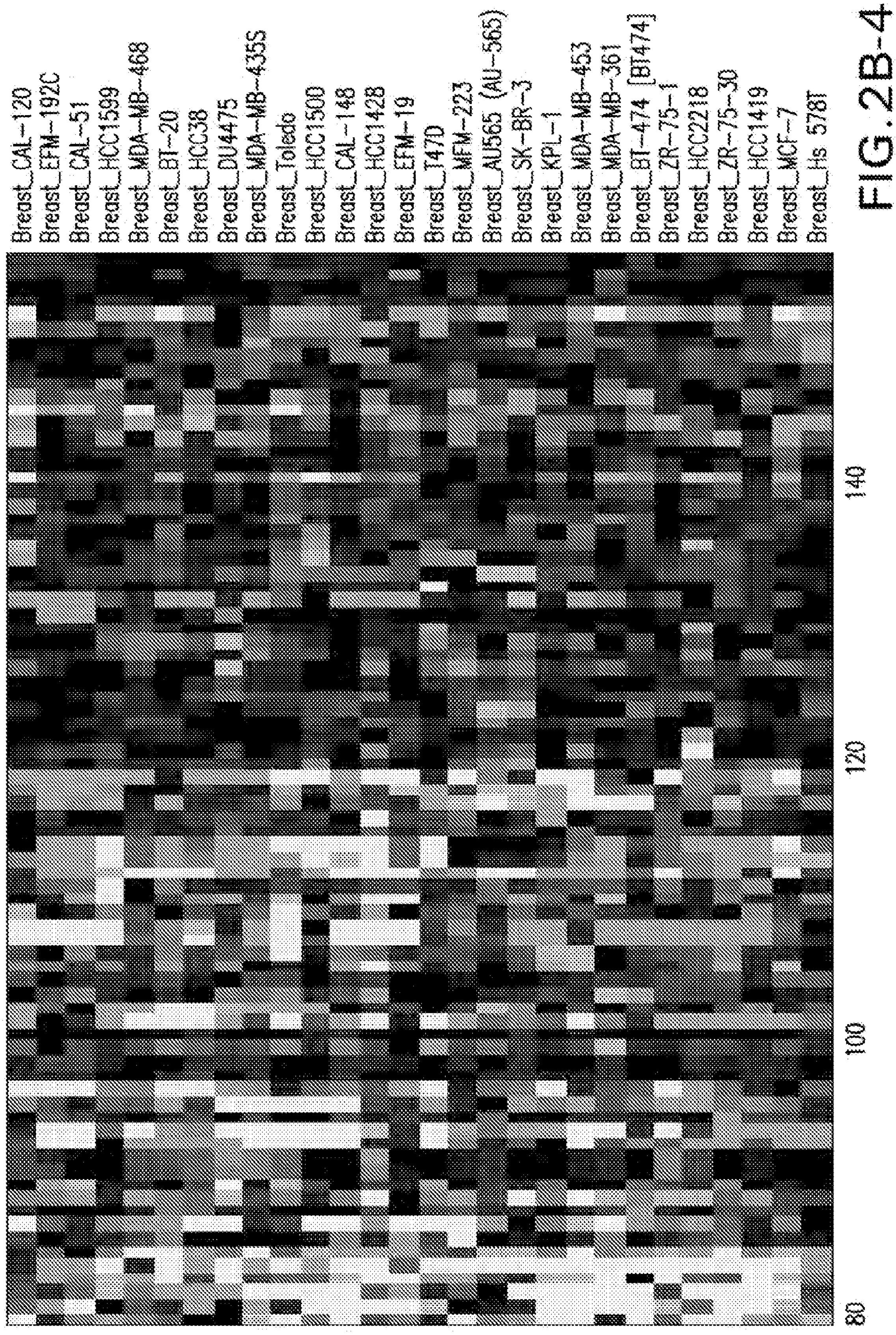
Breast_CAL-120
Breast_EFM-192C
Breast_CAL-51
Breast_HCC1599
Breast_MDA-MB-468
Breast_BT-20
Breast_HCC38
Breast_DU4475
Breast_MDA-MB-435S
Breast_Toledo
Breast_HCC1500
Breast_CAL-148
Breast_HCC1428
Breast_EFM-19
Breast_T47D
Breast_MFM-223
Breast_AU565 (AU-565)
Breast_SK-BR-3
Breast_KPL-1
Breast_MDA-MB-453
Breast_MDA-MB-361
Breast_BT-474 [BT474]
Breast_ZR-75-1
Breast_HCC2218
Breast_ZR-75-30
Breast_HCC1419
Breast_MCF-7
Breast_Hs 578T
80
100
120
140

FIG.2B-4

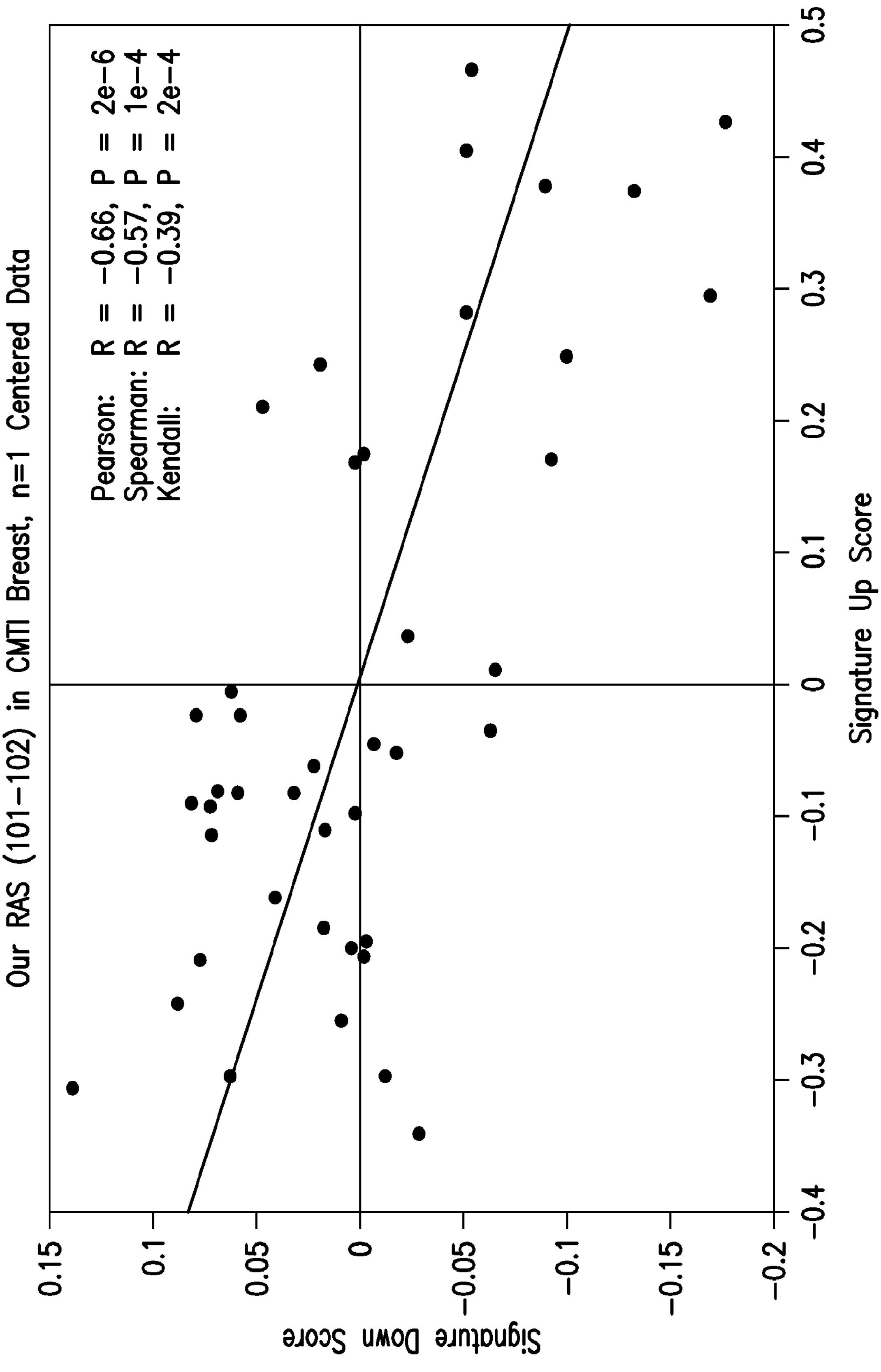
Our RAS (101–102) in CMTI Breast, n=1 Centered Data
Pearson: R = –0.66, P = 2e–6
Spearman: R = –0.57, P = 1e–4
Kendall: R = –0.39, P = 2e–4
Signature Up Score
Signature Down Score
–0.4
–0.3
–0.2
–0.1
0
0.1
0.2
0.3
0.4
0.5
0.15
0.1
0.05
0
–0.05
–0.1
–0.15
–0.2

FIG.2C

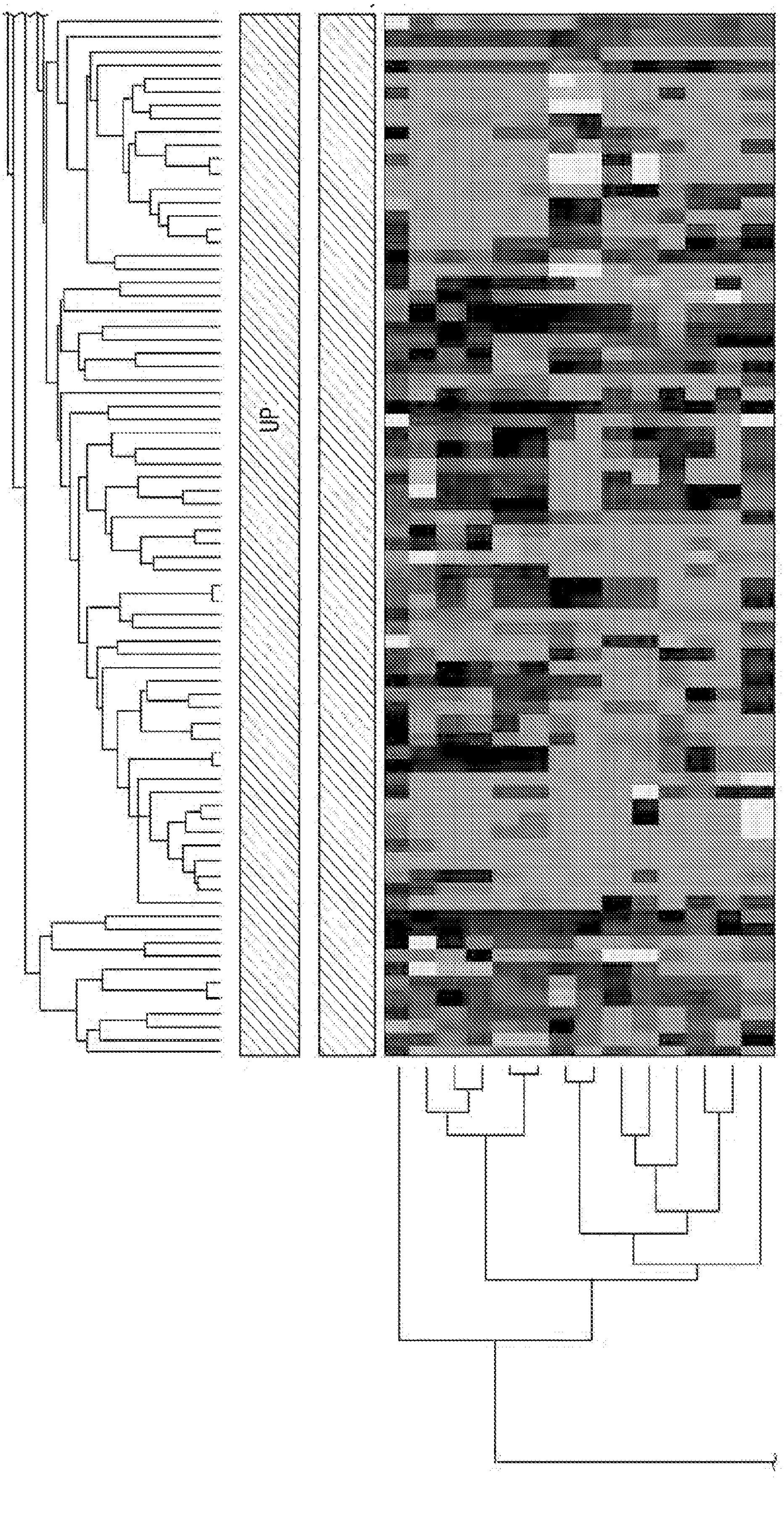


FIG. 2D-1

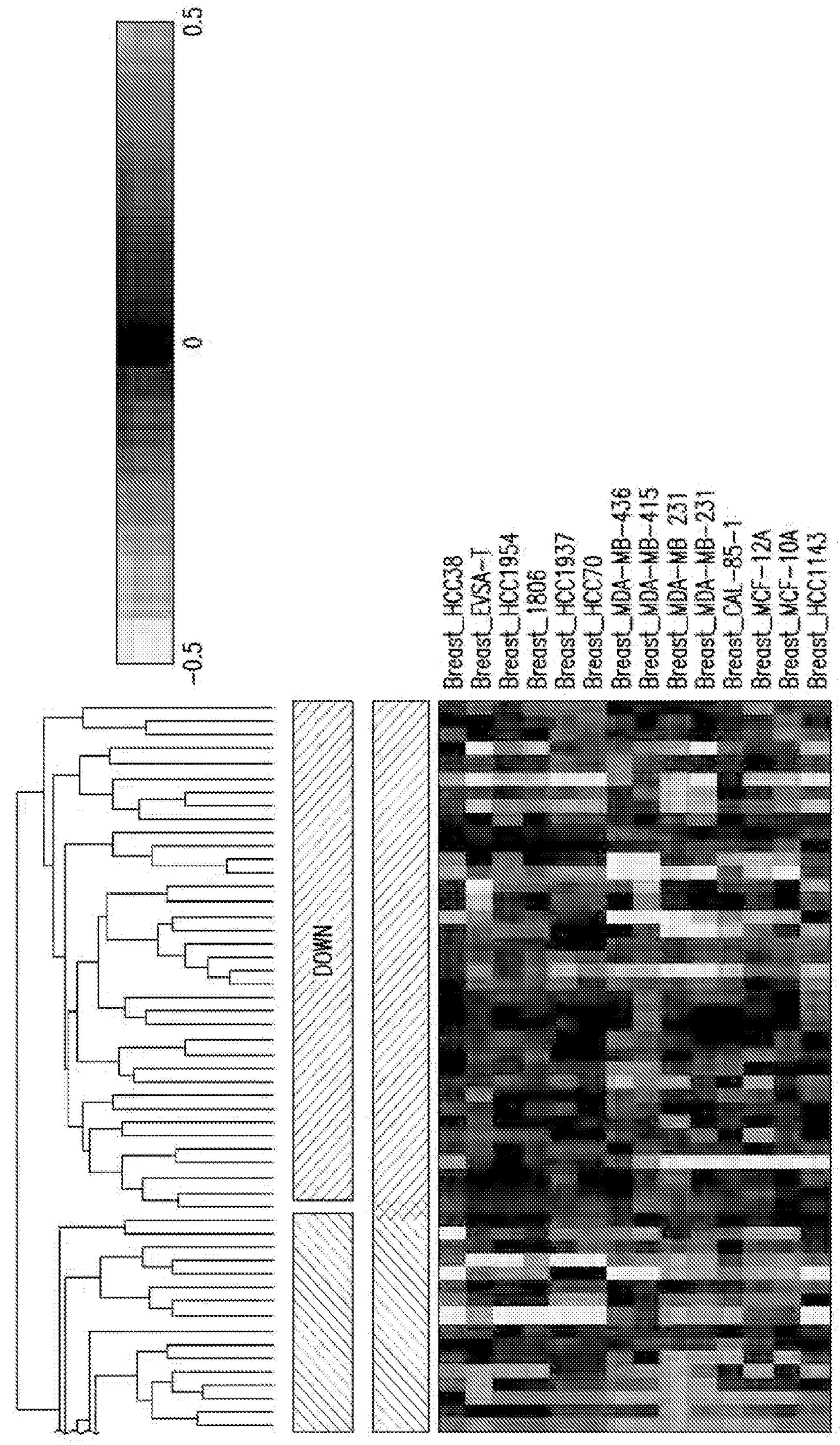
-0.5
0
0.5
DOWN
Breast_HCC38
Breast_EVSA-T
Breast_HCC1954
Breast_1806
Breast_HCC1937
Breast_HCC70
Breast_MDA-MB-436
Breast_MDA-MB-415
Breast_MDA-MB 231
Breast_MDA-MB-231
Breast_CAL-85-1
Breast_MCF-12A
Breast_MCF-10A
Breast_HCC1143

FIG.2D-2

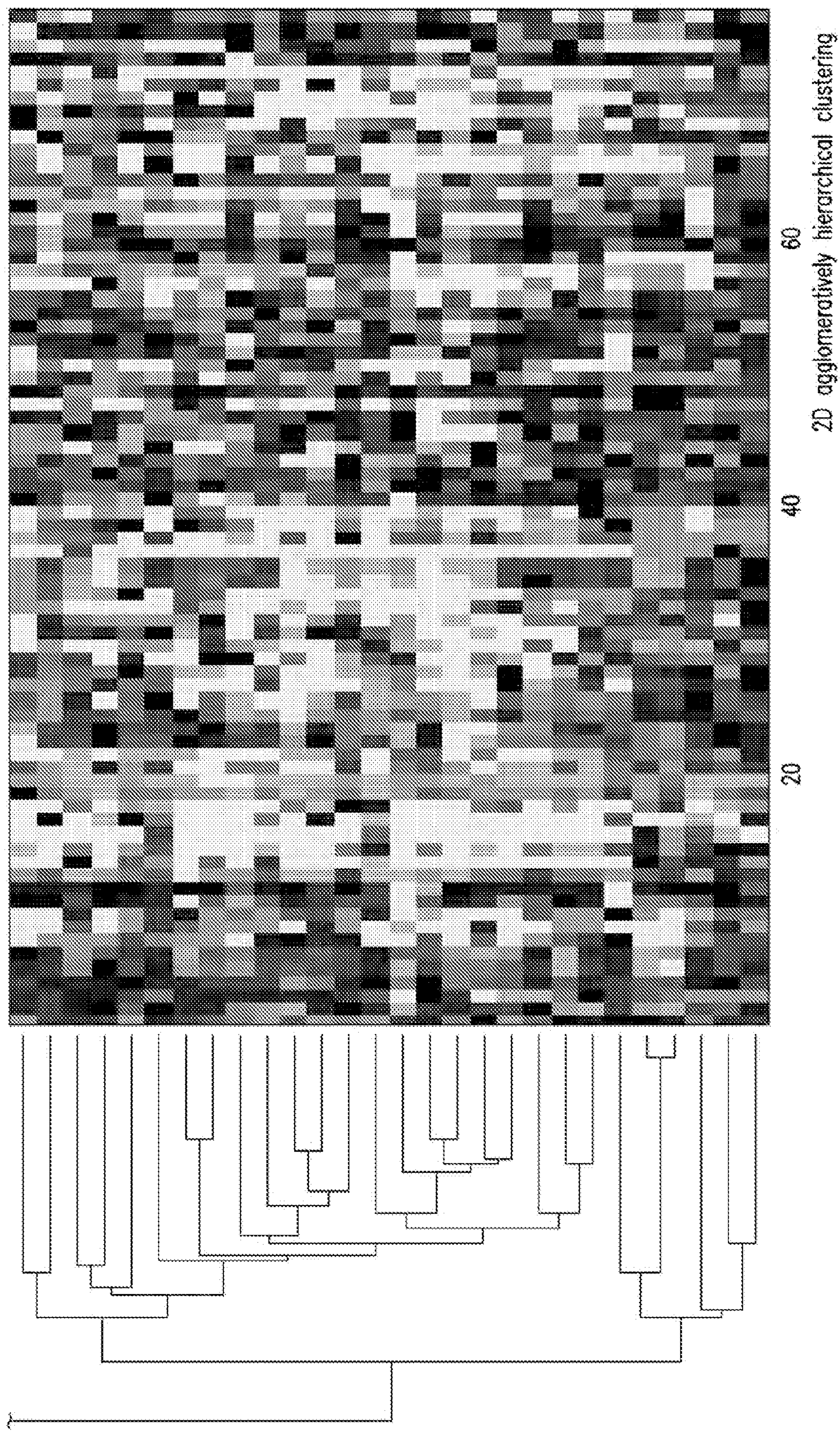


FIG. 2D-3

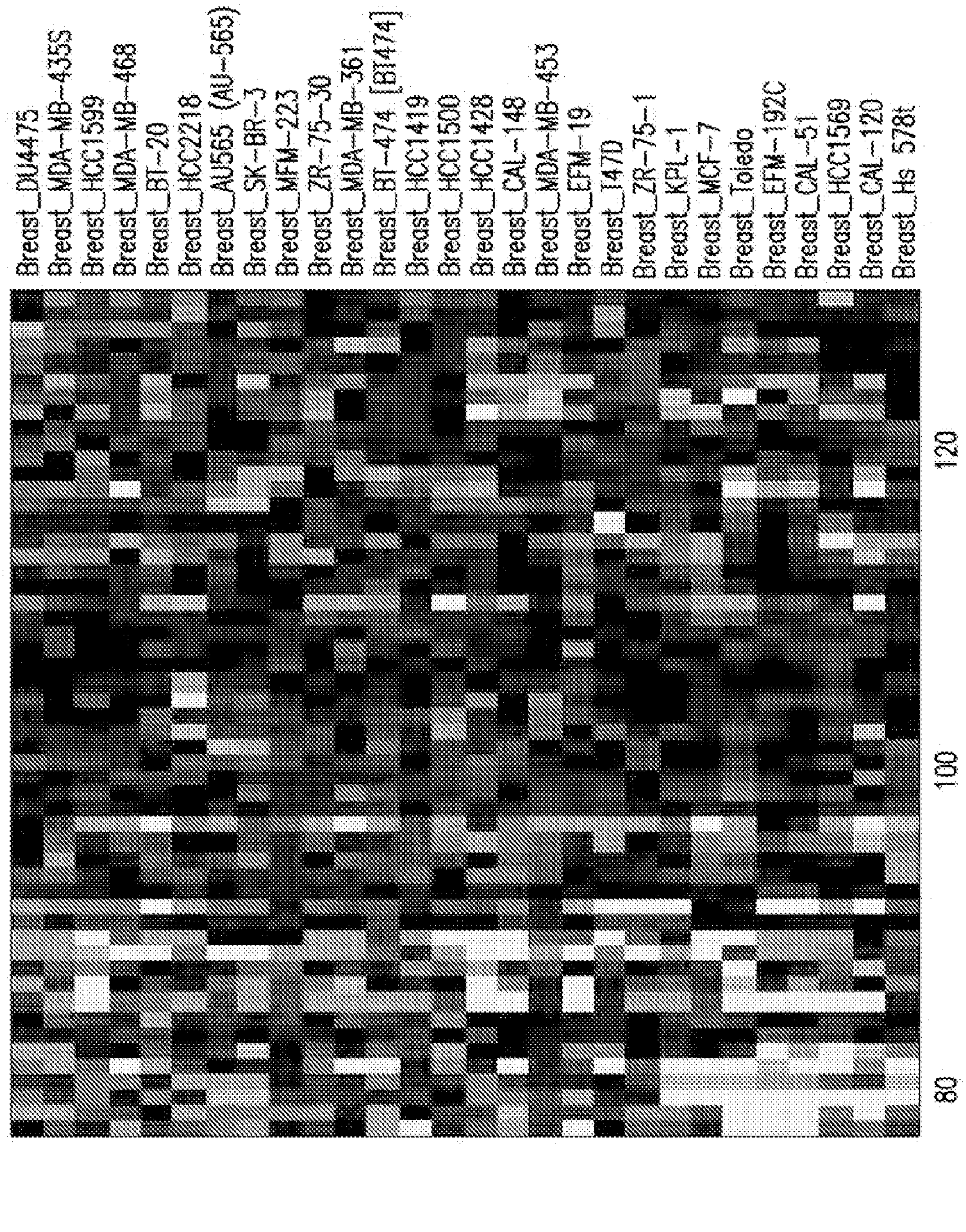
Breast_DU4475
Breast_MDA-MB-435S
Breast_HCC1599
Breast_MDA-MB-468
Breast_BT-20
Breast_HCC2218
Breast_AU565 (AU-565)
Breast_SK-BR-3
Breast_MFM-223
Breast_ZR-75-30
Breast_MDA-MB-361
Breast_BT-474 [BT474]
Breast_HCC1419
Breast_HCC1500
Breast_HCC1428
Breast_CAL-148
Breast_MDA-MB-453
Breast_EFM-19
Breast_T47D
Breast_ZR-75-1
Breast_KPL-1
Breast_MCF-7
Breast_Toledo
Breast_EFM-192C
Breast_CAL-51
Breast_HCC1569
Breast_CAL-120
Breast_Hs 578t
80
100
120

FIG. 2D-4

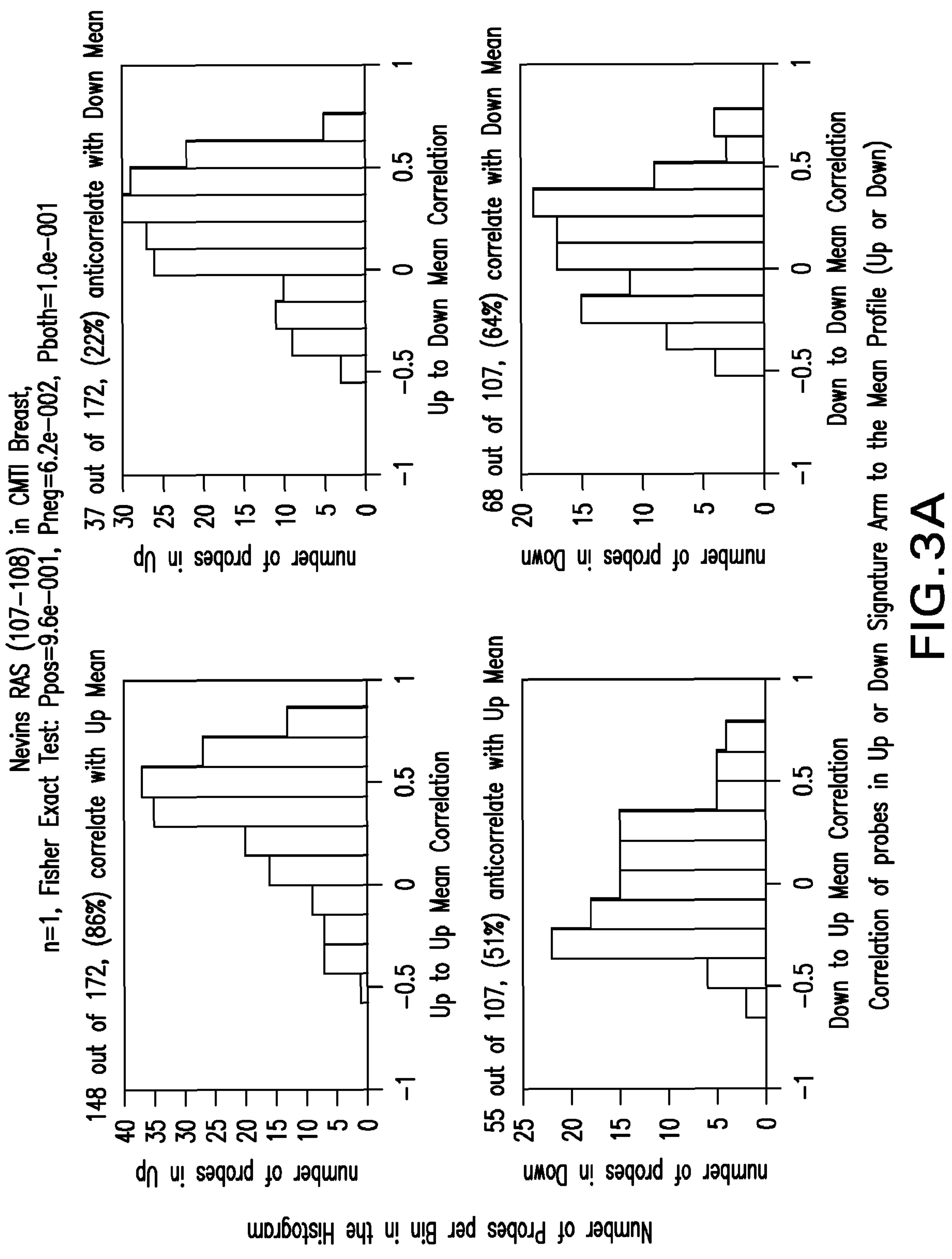
Nevins RAS (107-108) in CMTI Breast,
n=1, Fisher Exact Test: Ppos=9.6e-001, Pneg=6.2e-002, Pboth=1.0e-001
148 out of 172, (86%) correlate with Up Mean
37 out of 172, (22%) anticorrelate with Down Mean
55 out of 107, (51%) anticorrelate with Up Mean
68 out of 107, (64%) correlate with Down Mean
number of probes in Up
number of probes in Down
Up to Up Mean Correlation
Up to Down Mean Correlation
Down to Up Mean Correlation
Down to Down Mean Correlation
Number of Probes per Bin in the Histogram
Correlation of probes in Up or Down Signature Arm to the Mean Profile (Up or Down)

FIG.3A

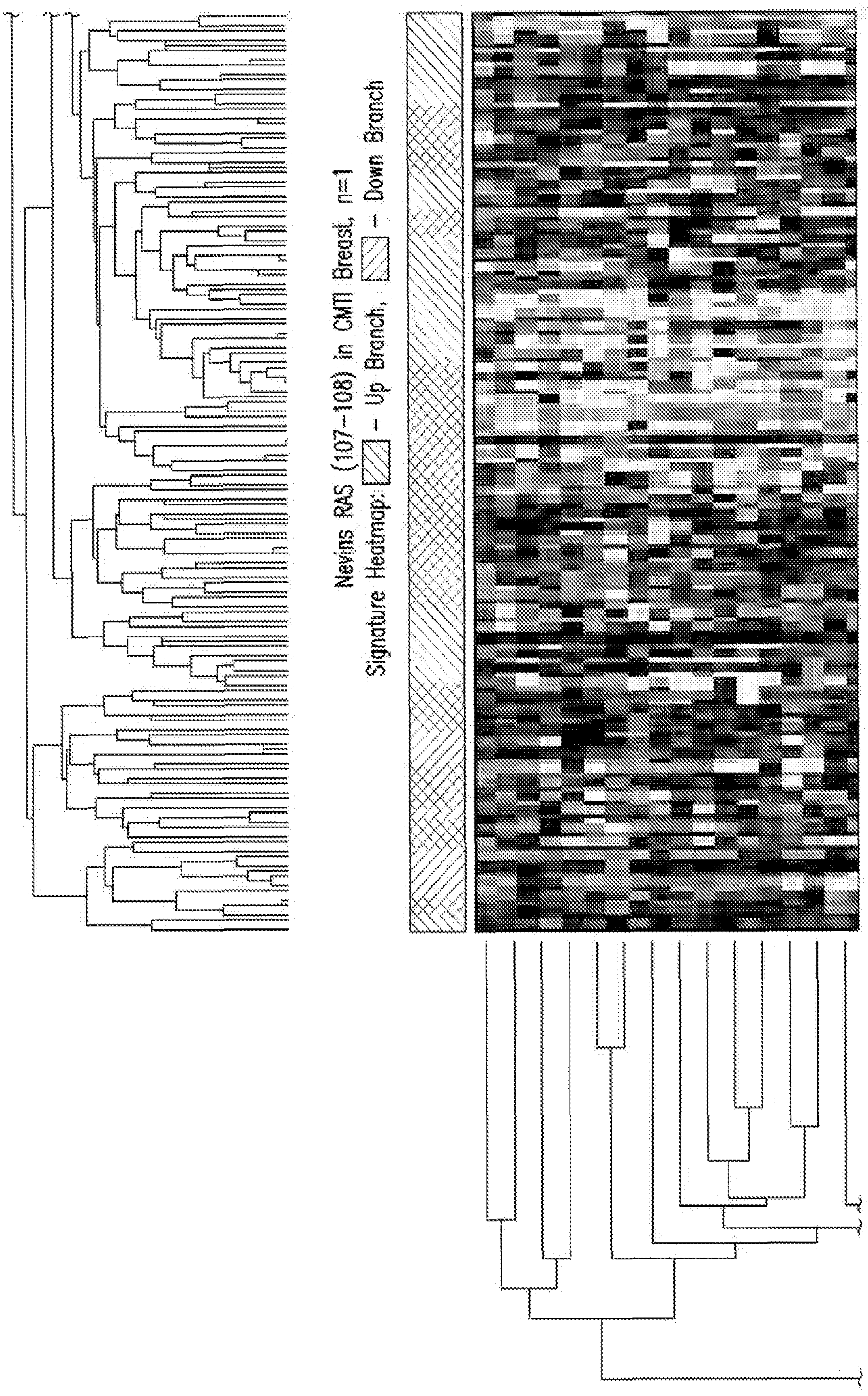

Nevins RAS (107-108) in CMTI Breast, n=1
Signature Heatmap: - Up Branch, - Down Branch


FIG. 3B-1

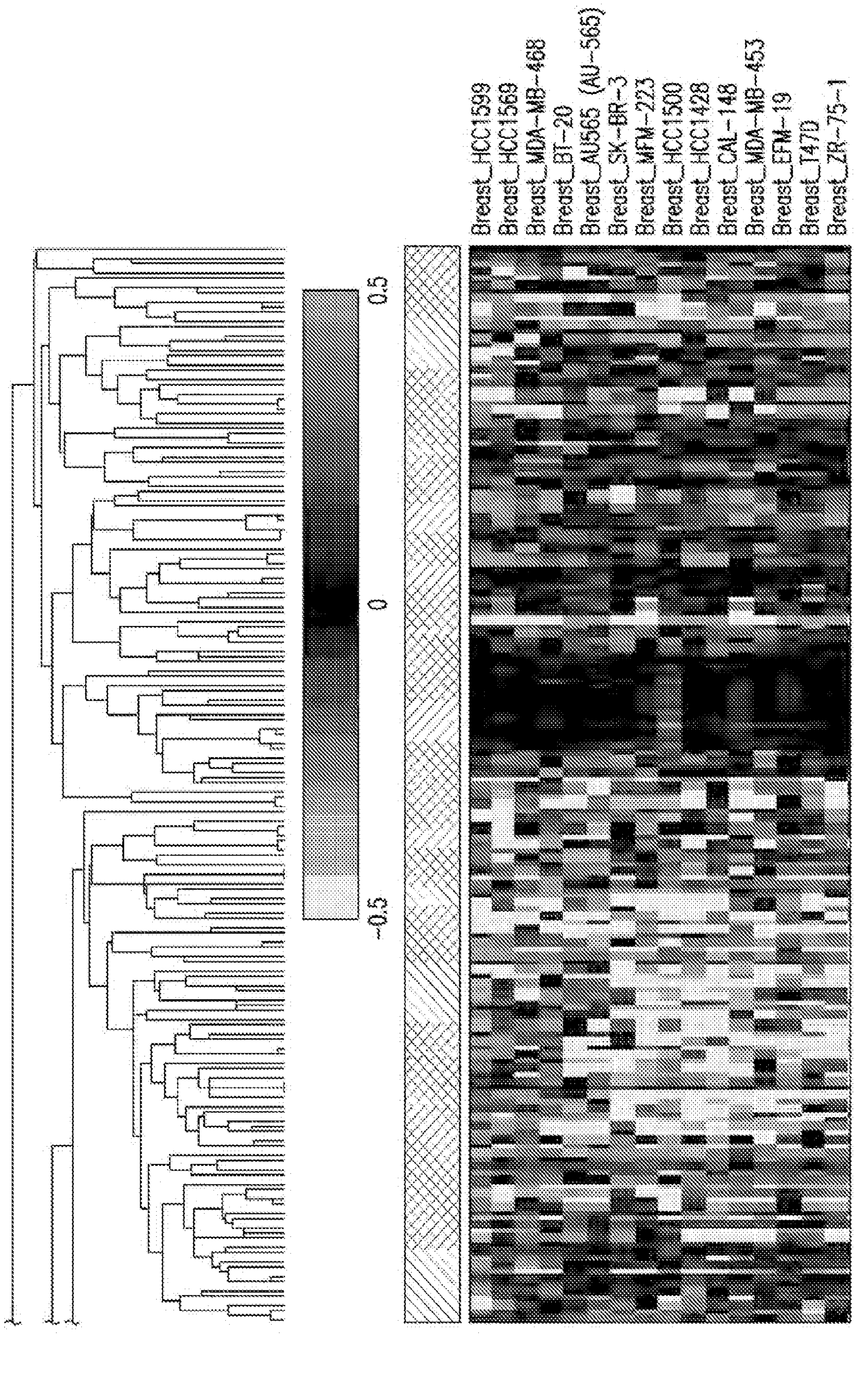
Breast_HCC1599
Breast_HCC1569
Breast_MDA-MB-468
Breast_BT-20
Breast_AU565 (AU-565)
Breast_SK-BR-3
Breast_MFM-223
Breast_HCC1500
Breast_HCC1428
Breast_CAL-148
Breast_MDA-MB-453
Breast_EFM-19
Breast_T47D
Breast_ZR-75-1
0.5
0
-0.5

FIG.3B-2

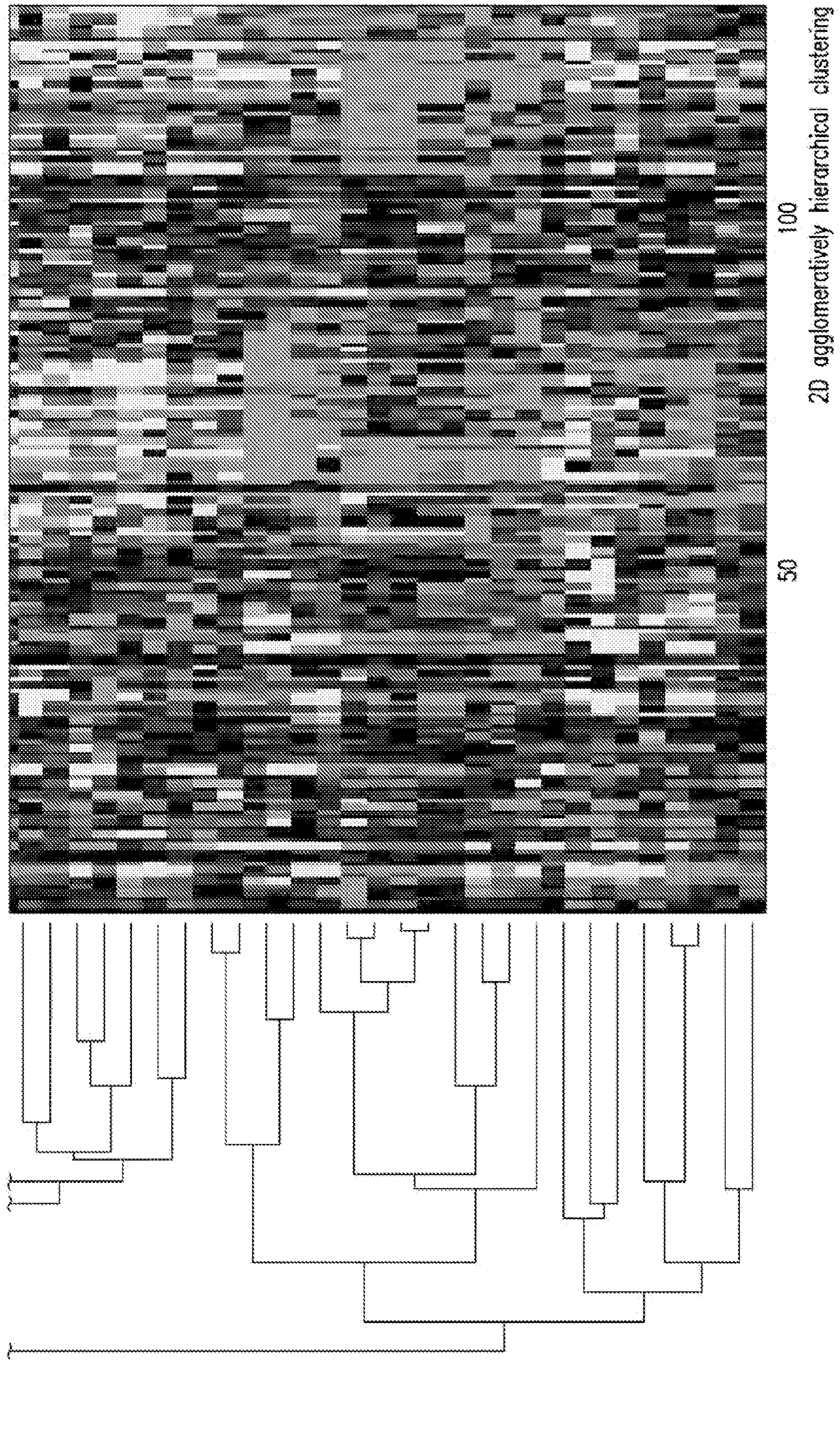


FIG.3B-3

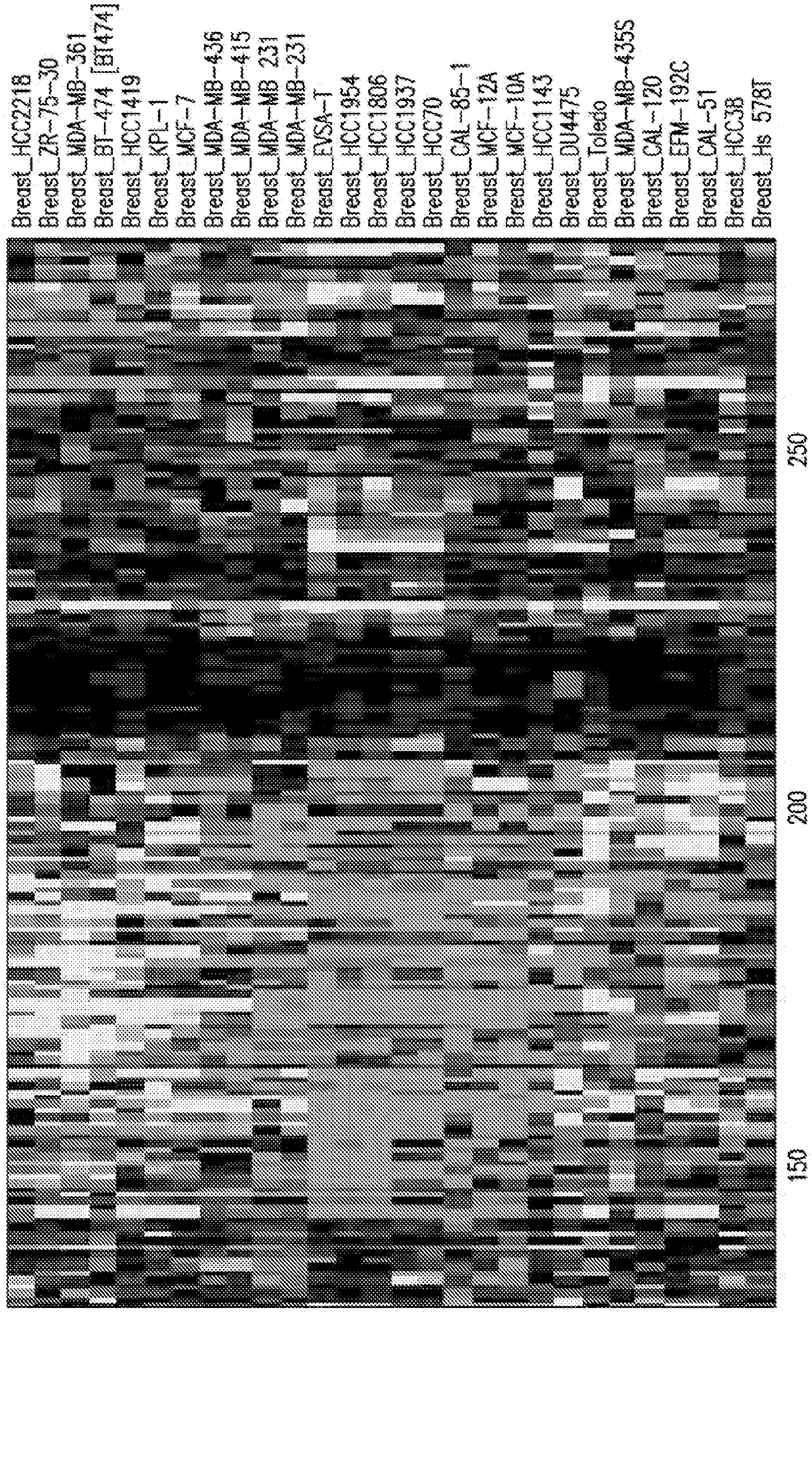
Breast_HCC2218
Breast_ZR-75-30
Breast_MDA-MB-361
Breast_BT-474 [BT474]
Breast_HCC1419
Breast_KPL-1
Breast_MCF-7
Breast_MDA-MB-436
Breast_MDA-MB-415
Breast_MDA-MB 231
Breast_MDA-MB-231
Breast_EVSA-T
Breast_HCC1954
Breast_HCC1806
Breast_HCC1937
Breast_HCC70
Breast_CAL-85-1
Breast_MCF-12A
Breast_MCF-10A
Breast_HCC1143
Breast_DU4475
Breast_Toledo
Breast_MDA-MB-435S
Breast_CAL-120
Breast_EFM-192C
Breast_CAL-51
Breast_HCC38
Breast_Hs 578T
150
200
250

FIG.3B-4

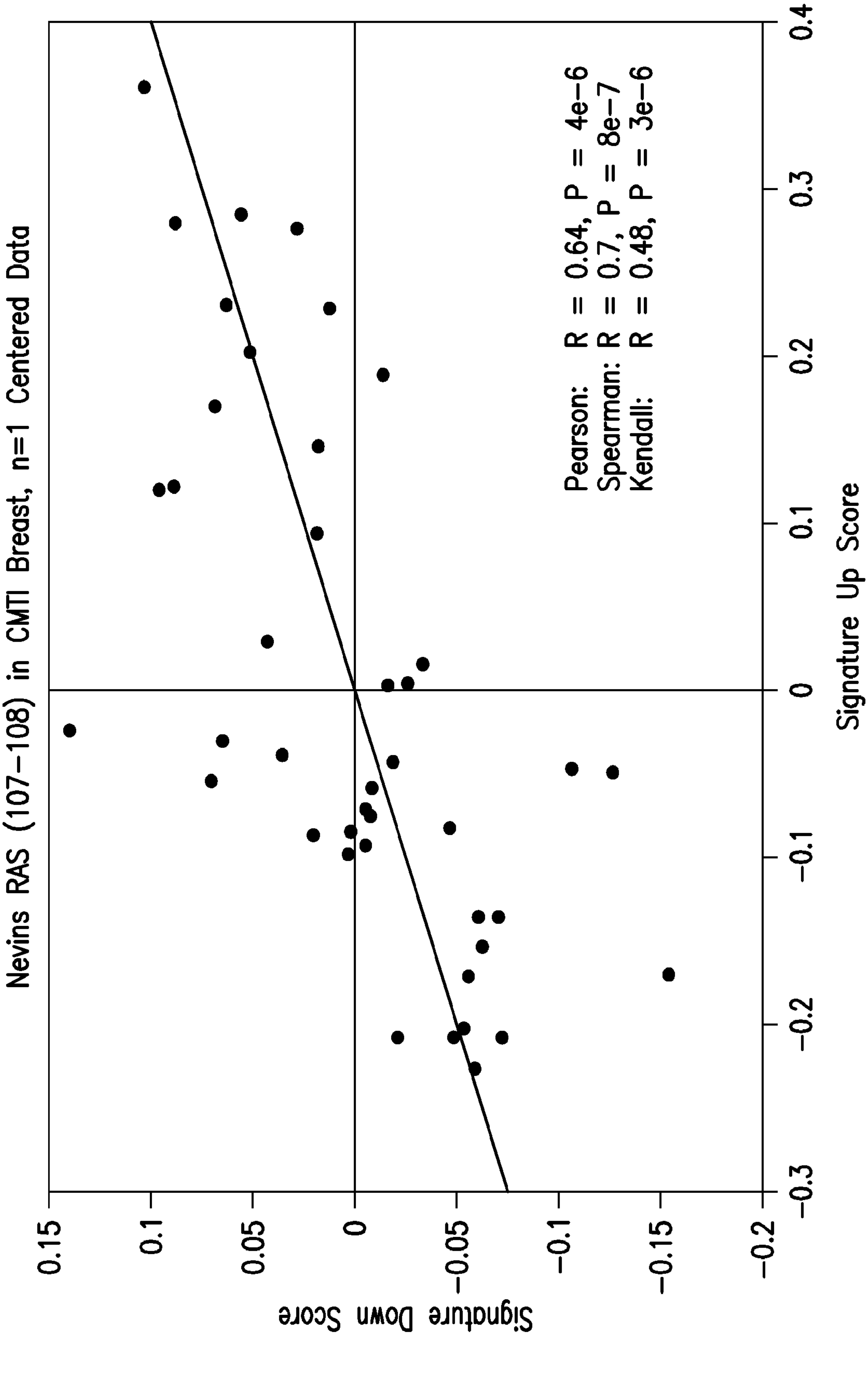
Nevins RAS (107−108) in CMTI Breast, n=1 Centered Data
Pearson: R = 0.64, P = 4e−6
Spearman: R = 0.7, P = 8e−7
Kendall: R = 0.48, P = 3e−6
Signature Up Score
Signature Down Score
−0.3
−0.2
−0.1
0
0.1
0.2
0.3
0.4
0.15
0.1
0.05
0
−0.05
−0.1
−0.15
−0.2

FIG.3C

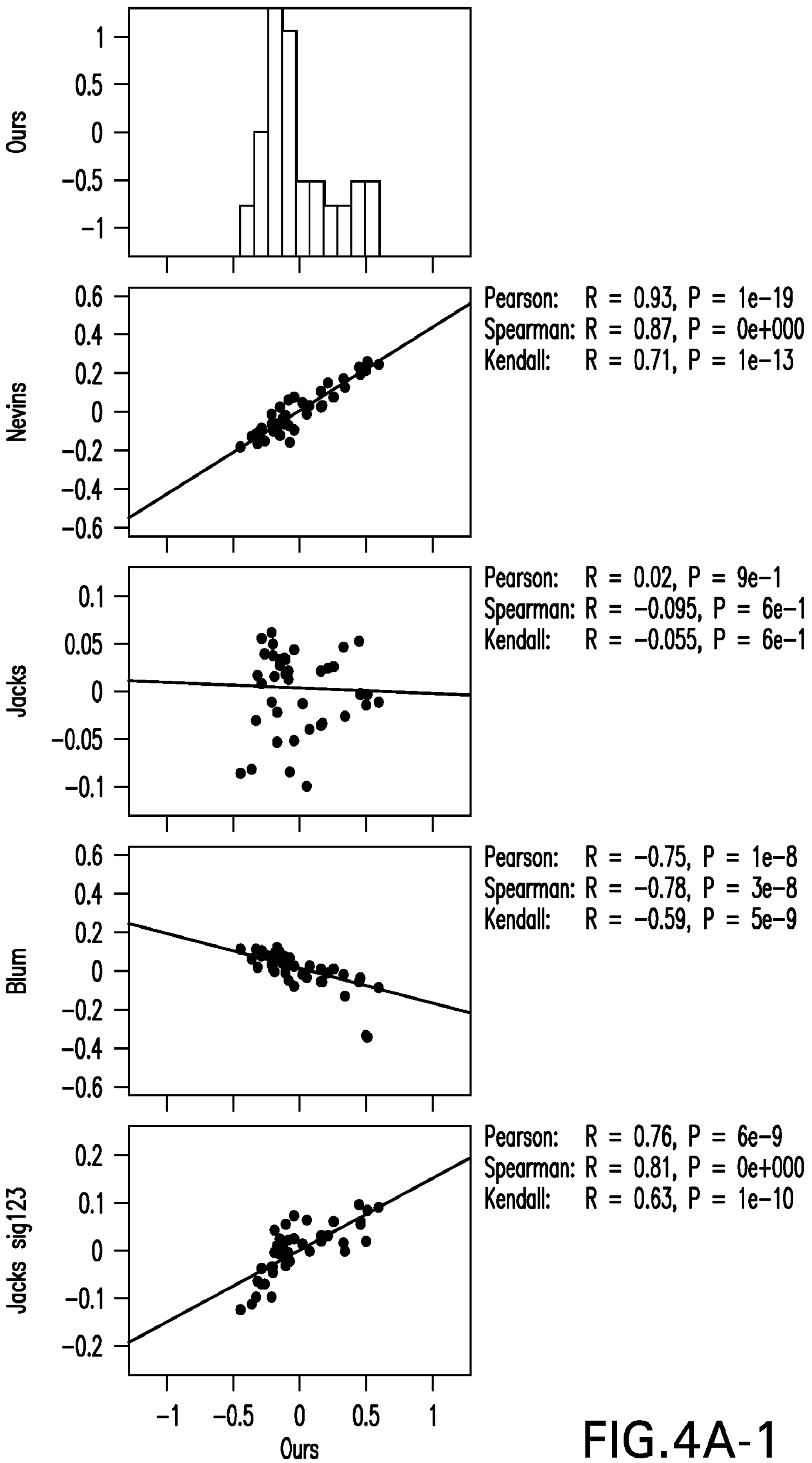
Ours
1
0.5
0
-0.5
-1
Nevins
0.6
0.4
0.2
0
-0.2
-0.4
-0.6
Pearson: R = 0.93, P = 1e-19
Spearman: R = 0.87, P = 0e+000
Kendall: R = 0.71, P = 1e-13
Jacks
0.1
0.05
0
-0.05
-0.1
Pearson: R = 0.02, P = 9e-1
Spearman: R = -0.095, P = 6e-1
Kendall: R = -0.055, P = 6e-1
Blum
Pearson: R = -0.75, P = 1e-8
Spearman: R = -0.78, P = 3e-8
Kendall: R = -0.59, P = 5e-9
Jacks sig123
0.2
0.1
0
-0.1
-0.2
Pearson: R = 0.76, P = 6e-9
Spearman: R = 0.81, P = 0e+000
Kendall: R = 0.63, P = 1e-10
-1
-0.5
0
0.5
1
Ours

FIG.4A-1

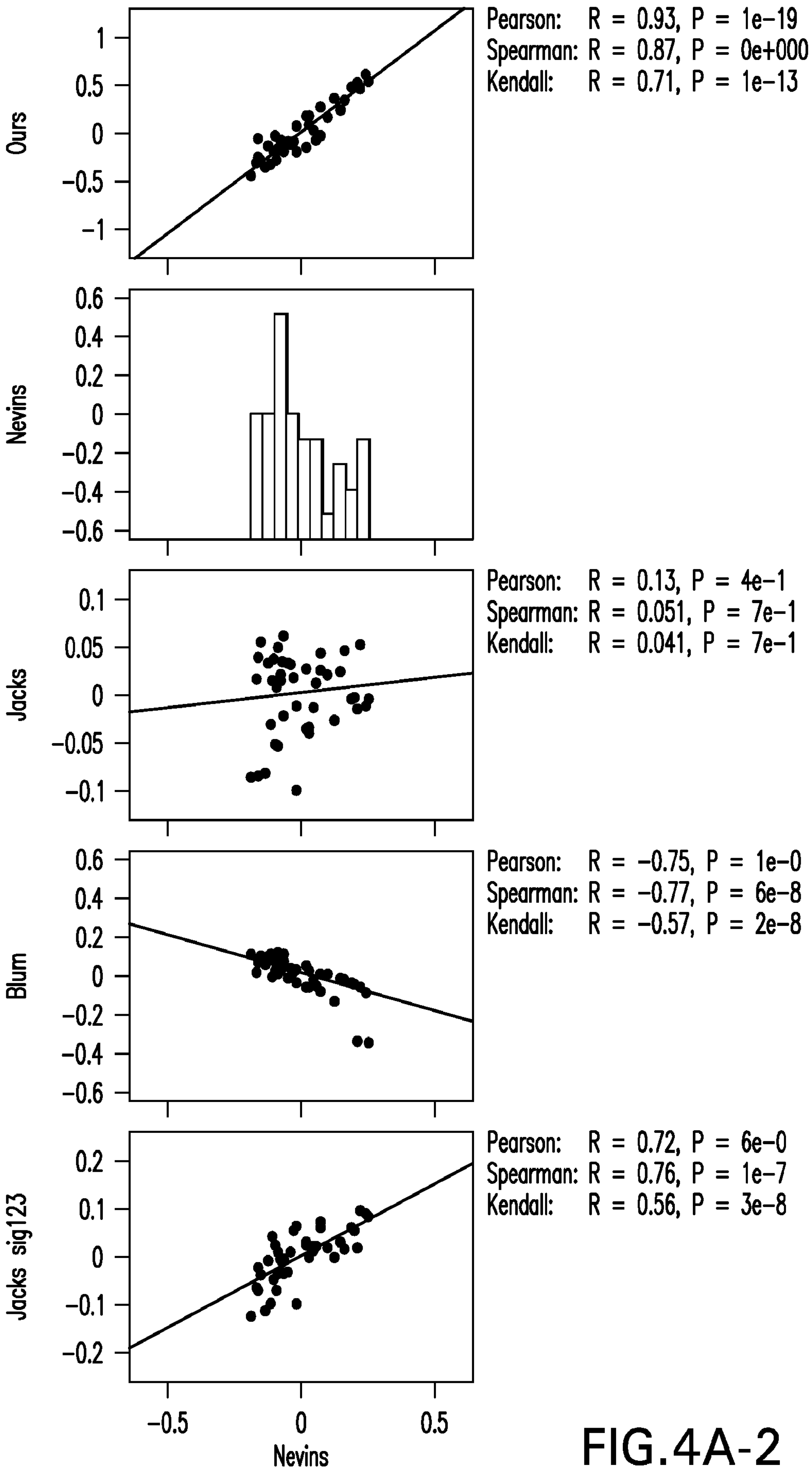
Pearson: R = 0.93, P = 1e−19
Spearman: R = 0.87, P = 0e+000
Kendall: R = 0.71, P = 1e−13
Pearson: R = 0.13, P = 4e−1
Spearman: R = 0.051, P = 7e−1
Kendall: R = 0.041, P = 7e−1
Pearson: R = −0.75, P = 1e−0
Spearman: R = −0.77, P = 6e−8
Kendall: R = −0.57, P = 2e−8
Pearson: R = 0.72, P = 6e−0
Spearman: R = 0.76, P = 1e−7
Kendall: R = 0.56, P = 3e−8
Ours
Nevins
Jacks
Blum
Jacks sig123
−0.5
0
0.5
Nevins

FIG.4A-2

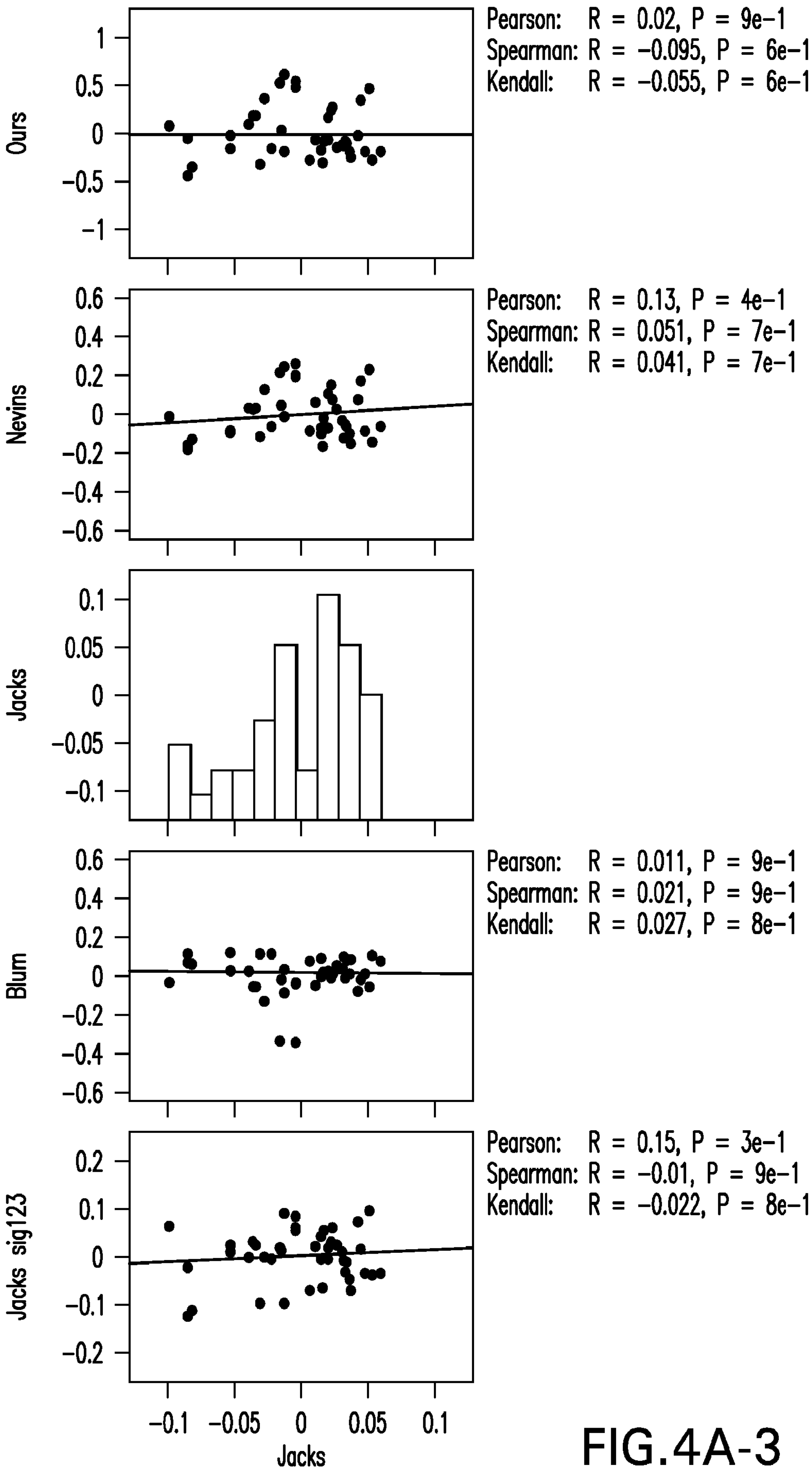
Pearson: R = 0.02, P = 9e−1
Spearman: R = −0.095, P = 6e−1
Kendall: R = −0.055, P = 6e−1
Ours
Pearson: R = 0.13, P = 4e−1
Spearman: R = 0.051, P = 7e−1
Kendall: R = 0.041, P = 7e−1
Nevins
Jacks
Pearson: R = 0.011, P = 9e−1
Spearman: R = 0.021, P = 9e−1
Kendall: R = 0.027, P = 8e−1
Blum
Pearson: R = 0.15, P = 3e−1
Spearman: R = −0.01, P = 9e−1
Kendall: R = −0.022, P = 8e−1
Jacks sig123
−0.1 −0.05 0 0.05 0.1
Jacks

FIG.4A-3

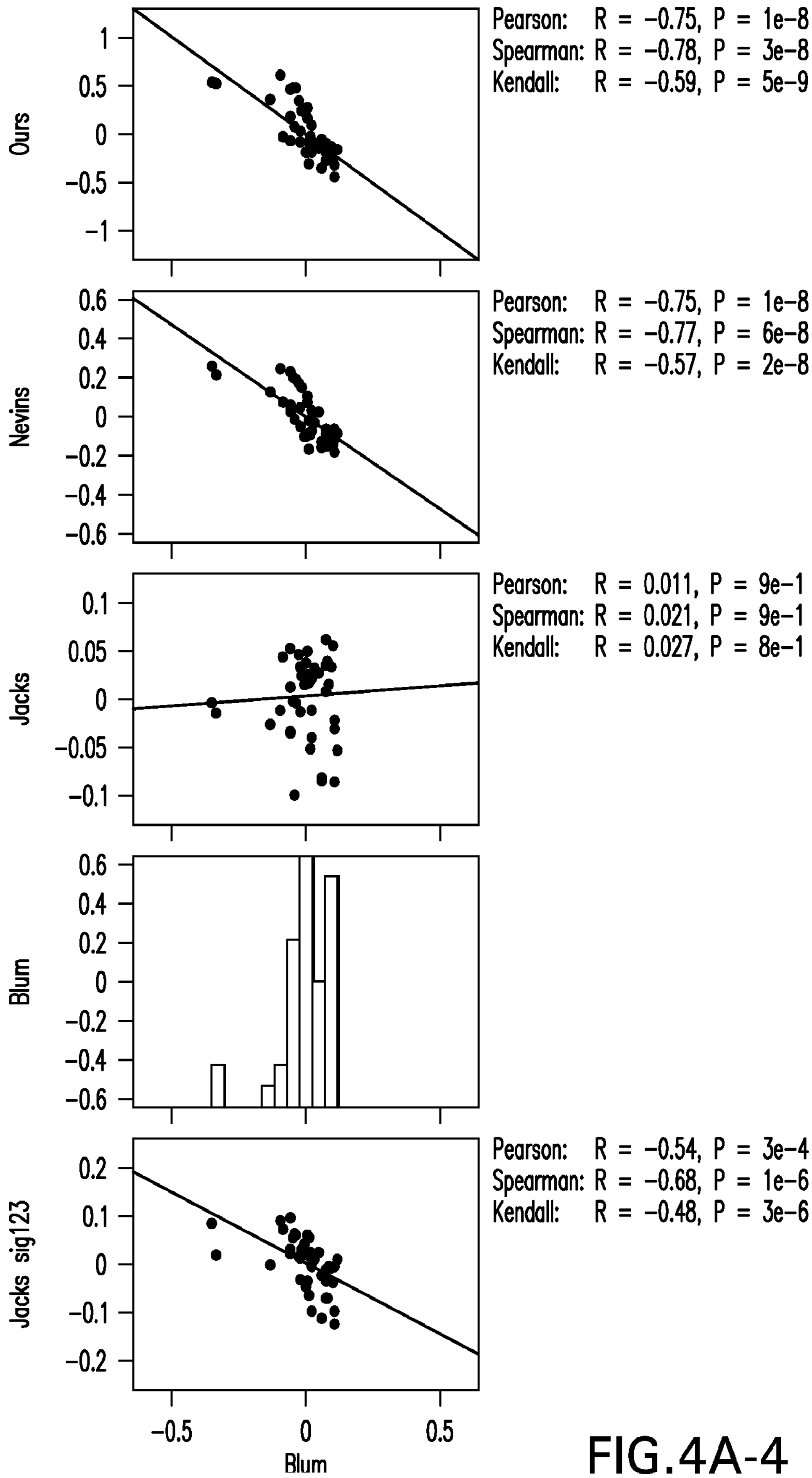
Pearson: R = −0.75, P = 1e−8
Spearman: R = −0.78, P = 3e−8
Kendall: R = −0.59, P = 5e−9
Ours
Pearson: R = −0.75, P = 1e−8
Spearman: R = −0.77, P = 6e−8
Kendall: R = −0.57, P = 2e−8
Nevins
Pearson: R = 0.011, P = 9e−1
Spearman: R = 0.021, P = 9e−1
Kendall: R = 0.027, P = 8e−1
Jacks
Blum
Pearson: R = −0.54, P = 3e−4
Spearman: R = −0.68, P = 1e−6
Kendall: R = −0.48, P = 3e−6
Jacks sig123
−0.5
0
0.5
Blum

FIG.4A-4

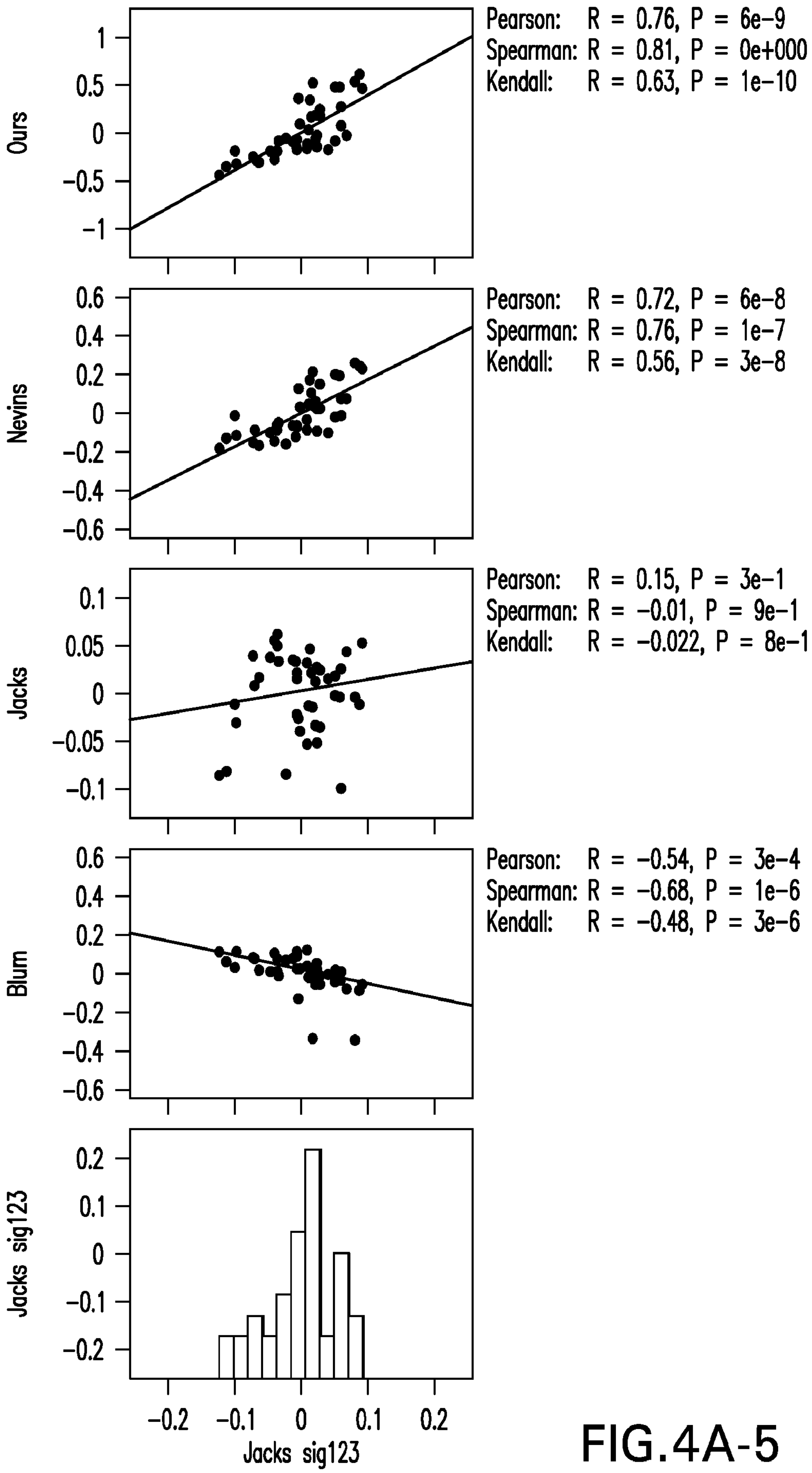
Pearson: R = 0.76, P = 6e−9
Spearman: R = 0.81, P = 0e+000
Kendall: R = 0.63, P = 1e−10
Pearson: R = 0.72, P = 6e−8
Spearman: R = 0.76, P = 1e−7
Kendall: R = 0.56, P = 3e−8
Pearson: R = 0.15, P = 3e−1
Spearman: R = −0.01, P = 9e−1
Kendall: R = −0.022, P = 8e−1
Pearson: R = −0.54, P = 3e−4
Spearman: R = −0.68, P = 1e−6
Kendall: R = −0.48, P = 3e−6
Ours
Nevins
Jacks
Blum
Jacks sig123
Jacks sig123

FIG.4A-5

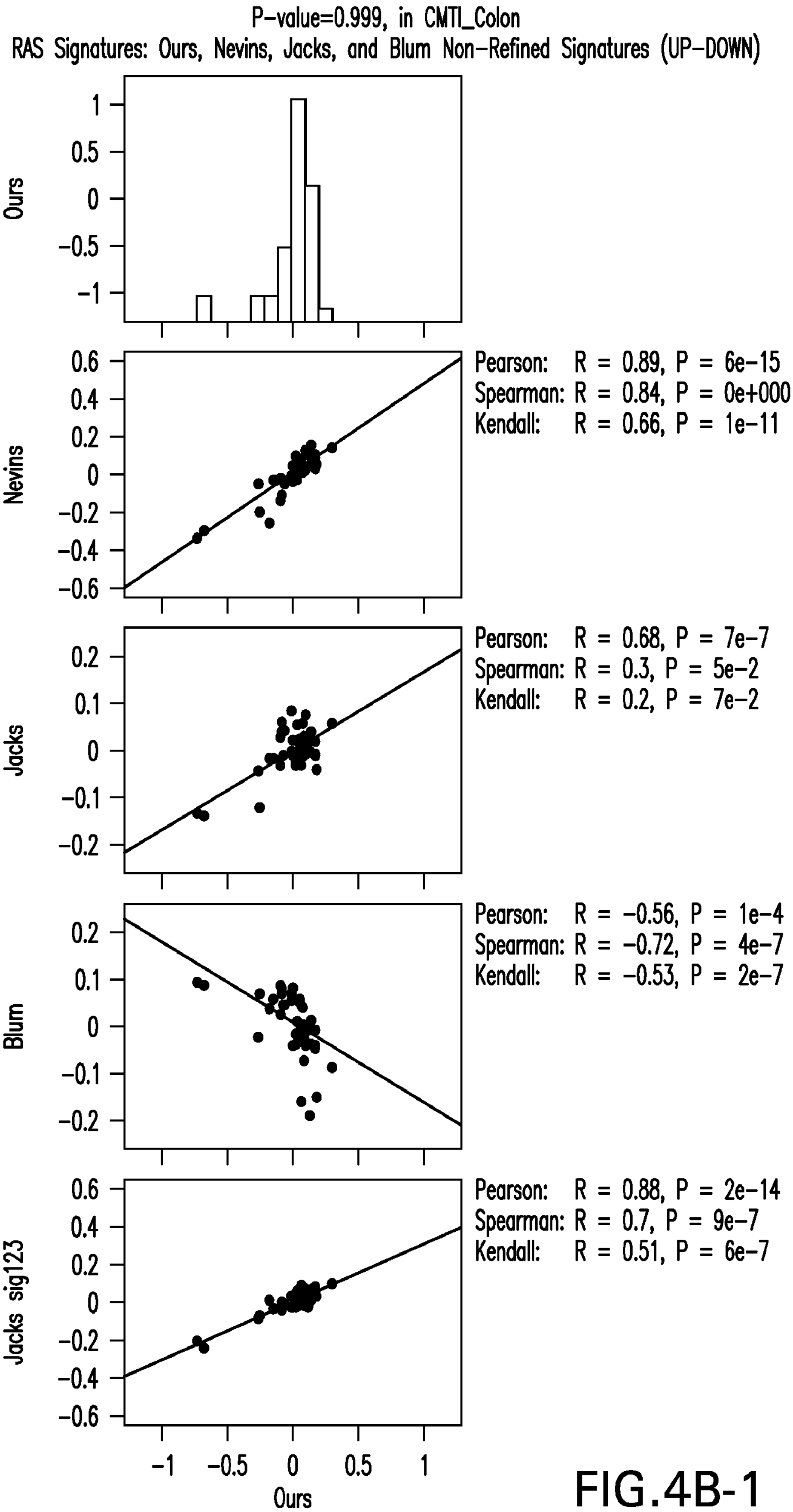
P-value=0.999, in CMTI_Colon
RAS Signatures: Ours, Nevins, Jacks, and Blum Non-Refined Signatures (UP-DOWN)
Ours
Nevins
Jacks
Blum
Jacks sig123
Ours
Pearson: R = 0.89, P = 6e-15
Spearman: R = 0.84, P = 0e+000
Kendall: R = 0.66, P = 1e-11
Pearson: R = 0.68, P = 7e-7
Spearman: R = 0.3, P = 5e-2
Kendall: R = 0.2, P = 7e-2
Pearson: R = -0.56, P = 1e-4
Spearman: R = -0.72, P = 4e-7
Kendall: R = -0.53, P = 2e-7
Pearson: R = 0.88, P = 2e-14
Spearman: R = 0.7, P = 9e-7
Kendall: R = 0.51, P = 6e-7

FIG.4B-1

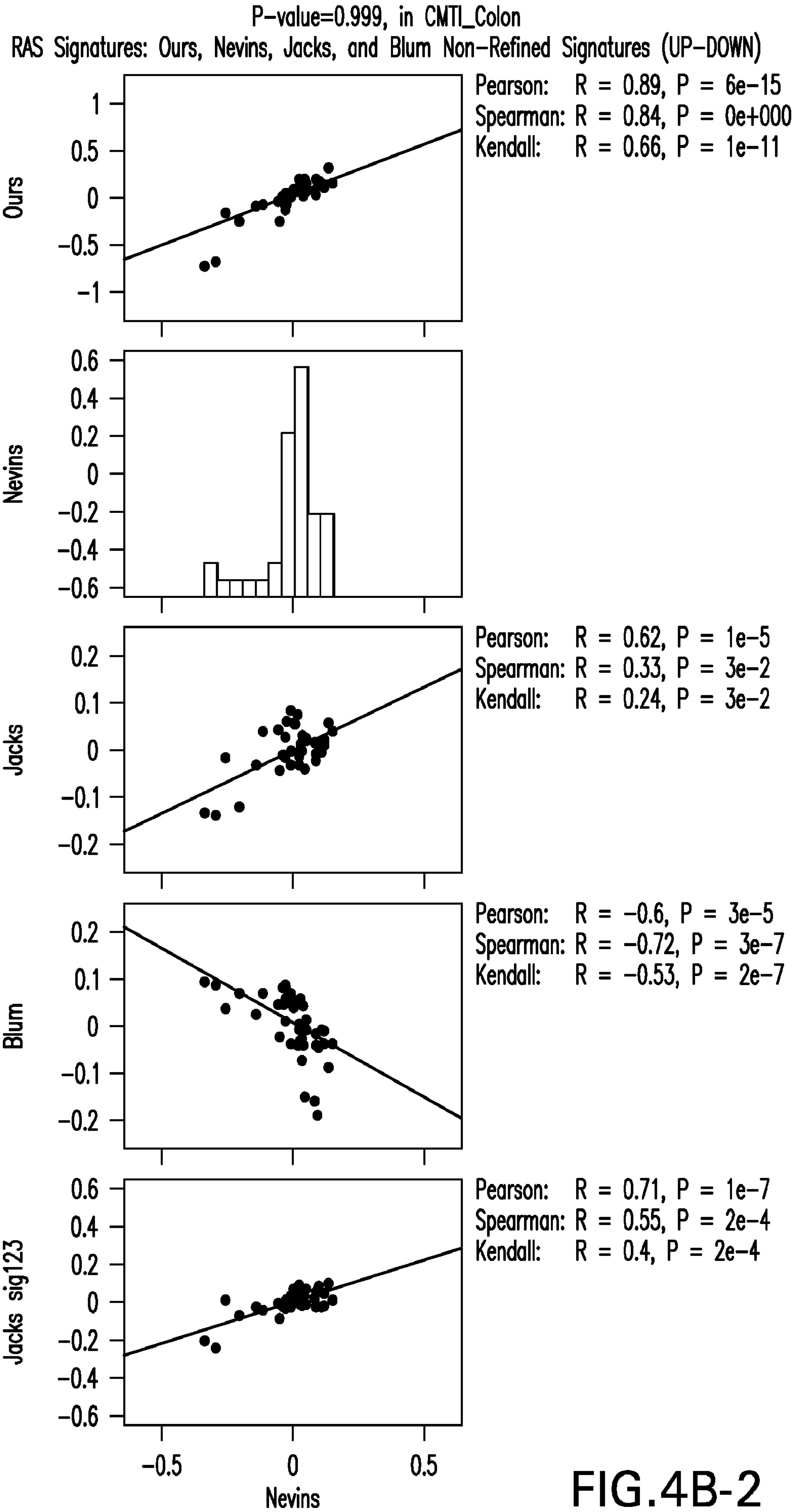
P-value=0.999, in CMTI_Colon
RAS Signatures: Ours, Nevins, Jacks, and Blum Non-Refined Signatures (UP-DOWN)
Pearson: R = 0.89, P = 6e-15
Spearman: R = 0.84, P = 0e+000
Kendall: R = 0.66, P = 1e-11
Ours
Nevins
Pearson: R = 0.62, P = 1e-5
Spearman: R = 0.33, P = 3e-2
Kendall: R = 0.24, P = 3e-2
Jacks
Pearson: R = -0.6, P = 3e-5
Spearman: R = -0.72, P = 3e-7
Kendall: R = -0.53, P = 2e-7
Blum
Pearson: R = 0.71, P = 1e-7
Spearman: R = 0.55, P = 2e-4
Kendall: R = 0.4, P = 2e-4
Jacks sig123
-0.5
0
0.5
Nevins

FIG.4B-2

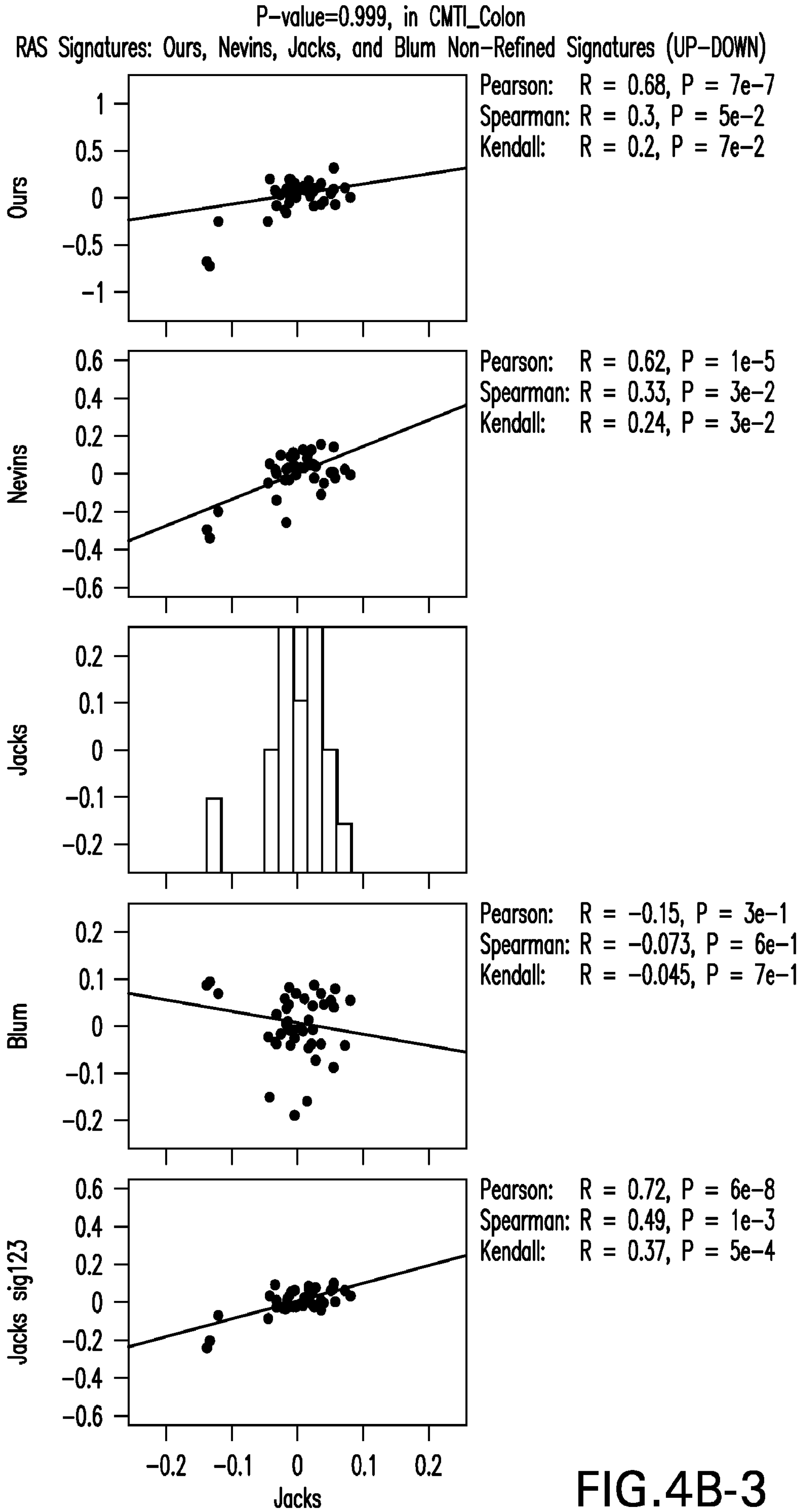
P-value=0.999, in CMTI_Colon
RAS Signatures: Ours, Nevins, Jacks, and Blum Non-Refined Signatures (UP-DOWN)
Pearson: R = 0.68, P = 7e-7
Spearman: R = 0.3, P = 5e-2
Kendall: R = 0.2, P = 7e-2
Ours
1
0.5
0
-0.5
-1
Pearson: R = 0.62, P = 1e-5
Spearman: R = 0.33, P = 3e-2
Kendall: R = 0.24, P = 3e-2
Nevins
0.6
0.4
0.2
0
-0.2
-0.4
-0.6
Jacks
0.2
0.1
0
-0.1
-0.2
Pearson: R = -0.15, P = 3e-1
Spearman: R = -0.073, P = 6e-1
Kendall: R = -0.045, P = 7e-1
Blum
0.2
0.1
0
-0.1
-0.2
Pearson: R = 0.72, P = 6e-8
Spearman: R = 0.49, P = 1e-3
Kendall: R = 0.37, P = 5e-4
Jacks sig123
0.6
0.4
0.2
0
-0.2
-0.4
-0.6
-0.2
-0.1
0
0.1
0.2
Jacks

FIG.4B-3

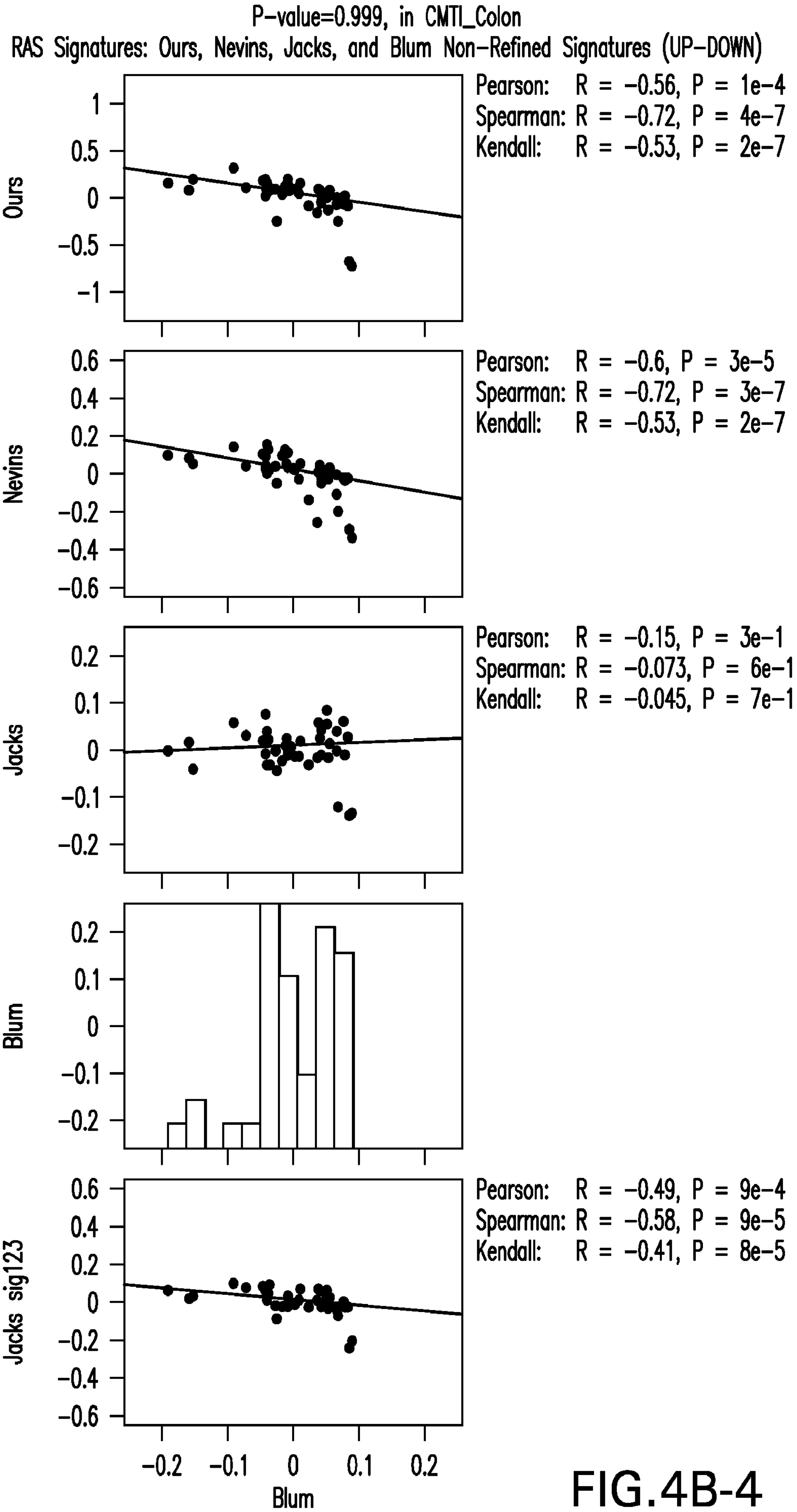
P-value=0.999, in CMTI_Colon
RAS Signatures: Ours, Nevins, Jacks, and Blum Non-Refined Signatures (UP-DOWN)
Pearson: R = -0.56, P = 1e-4
Spearman: R = -0.72, P = 4e-7
Kendall: R = -0.53, P = 2e-7
Ours
Pearson: R = -0.6, P = 3e-5
Spearman: R = -0.72, P = 3e-7
Kendall: R = -0.53, P = 2e-7
Nevins
Pearson: R = -0.15, P = 3e-1
Spearman: R = -0.073, P = 6e-1
Kendall: R = -0.045, P = 7e-1
Jacks
Blum
Pearson: R = -0.49, P = 9e-4
Spearman: R = -0.58, P = 9e-5
Kendall: R = -0.41, P = 8e-5
Jacks sig123
-0.2 -0.1 0 0.1 0.2
Blum

FIG.4B-4

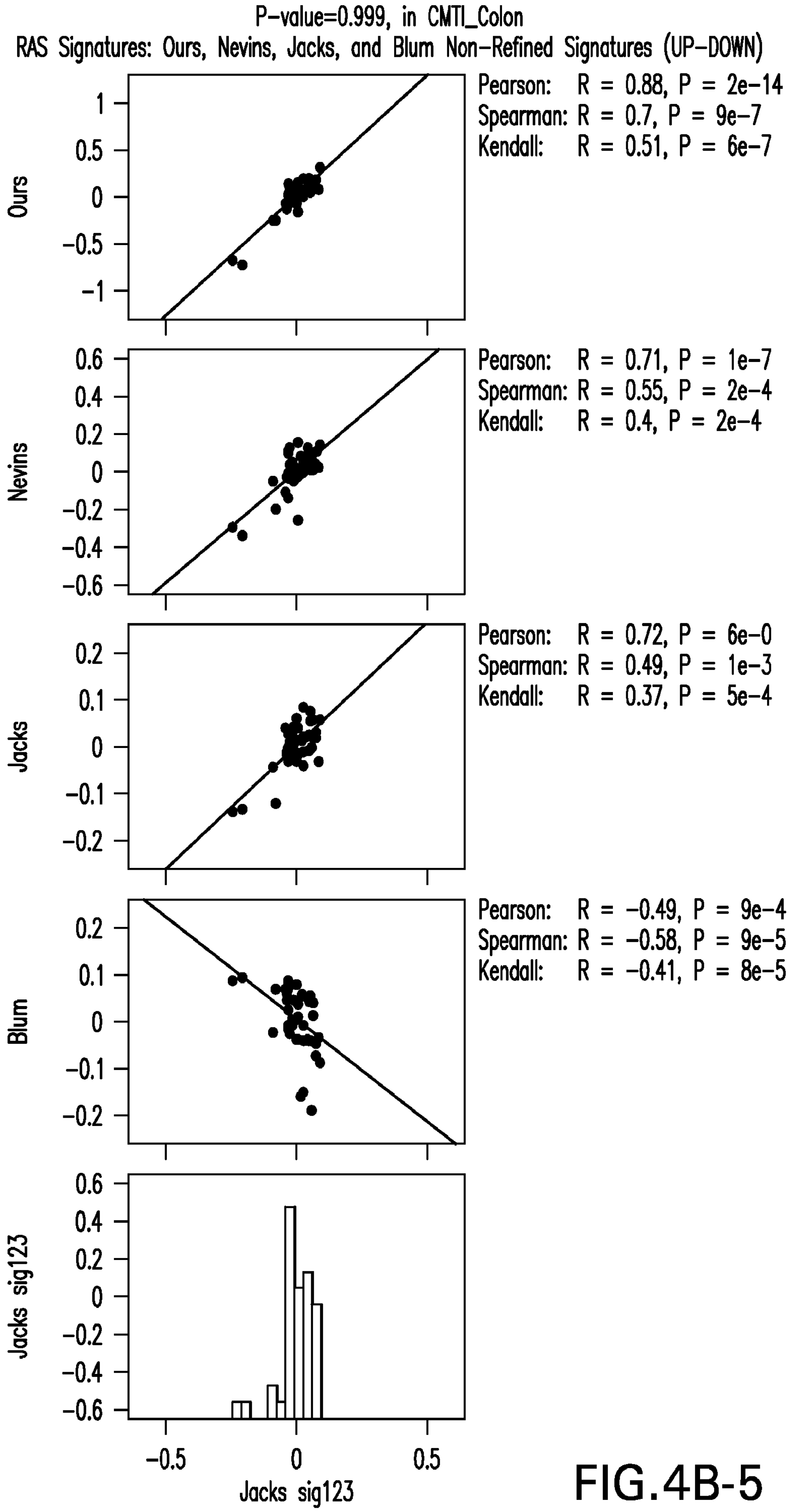
P-value=0.999, in CMTI_Colon
RAS Signatures: Ours, Nevins, Jacks, and Blum Non-Refined Signatures (UP-DOWN)
Pearson: R = 0.88, P = 2e-14
Spearman: R = 0.7, P = 9e-7
Kendall: R = 0.51, P = 6e-7
Pearson: R = 0.71, P = 1e-7
Spearman: R = 0.55, P = 2e-4
Kendall: R = 0.4, P = 2e-4
Pearson: R = 0.72, P = 6e-0
Spearman: R = 0.49, P = 1e-3
Kendall: R = 0.37, P = 5e-4
Pearson: R = -0.49, P = 9e-4
Spearman: R = -0.58, P = 9e-5
Kendall: R = -0.41, P = 8e-5
Ours
Nevins
Jacks
Blum
Jacks sig123
Jacks sig123

FIG.4B-5

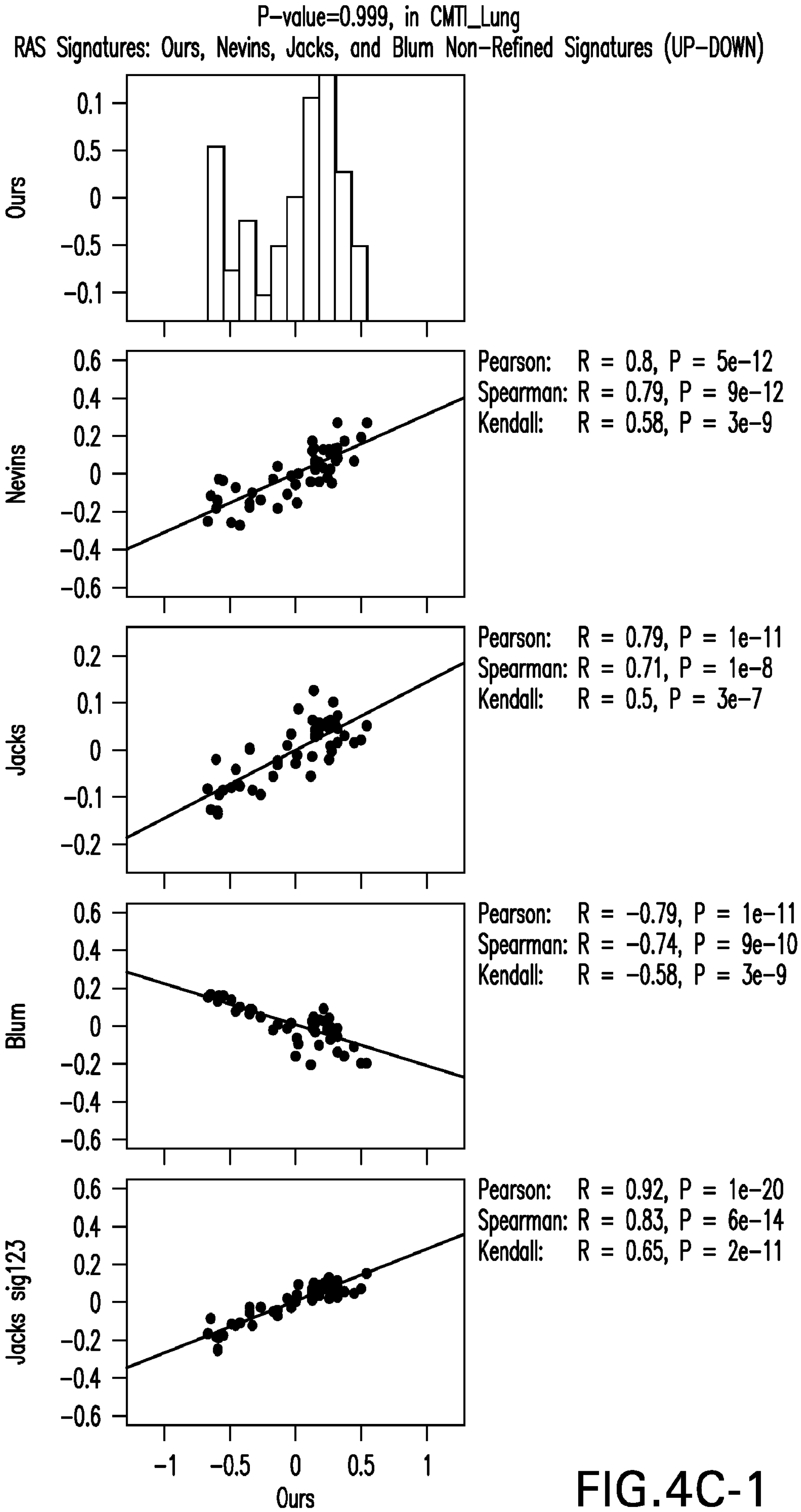
P−value=0.999, in CMTI_Lung
RAS Signatures: Ours, Nevins, Jacks, and Blum Non−Refined Signatures (UP−DOWN)
Ours
0.1
0.5
0
−0.5
−0.1
Nevins
Pearson: R = 0.8, P = 5e−12
Spearman: R = 0.79, P = 9e−12
Kendall: R = 0.58, P = 3e−9
Jacks
Pearson: R = 0.79, P = 1e−11
Spearman: R = 0.71, P = 1e−8
Kendall: R = 0.5, P = 3e−7
Blum
Pearson: R = −0.79, P = 1e−11
Spearman: R = −0.74, P = 9e−10
Kendall: R = −0.58, P = 3e−9
Jacks sig123
Pearson: R = 0.92, P = 1e−20
Spearman: R = 0.83, P = 6e−14
Kendall: R = 0.65, P = 2e−11
−1
−0.5
0
0.5
1
Ours

FIG.4C-1

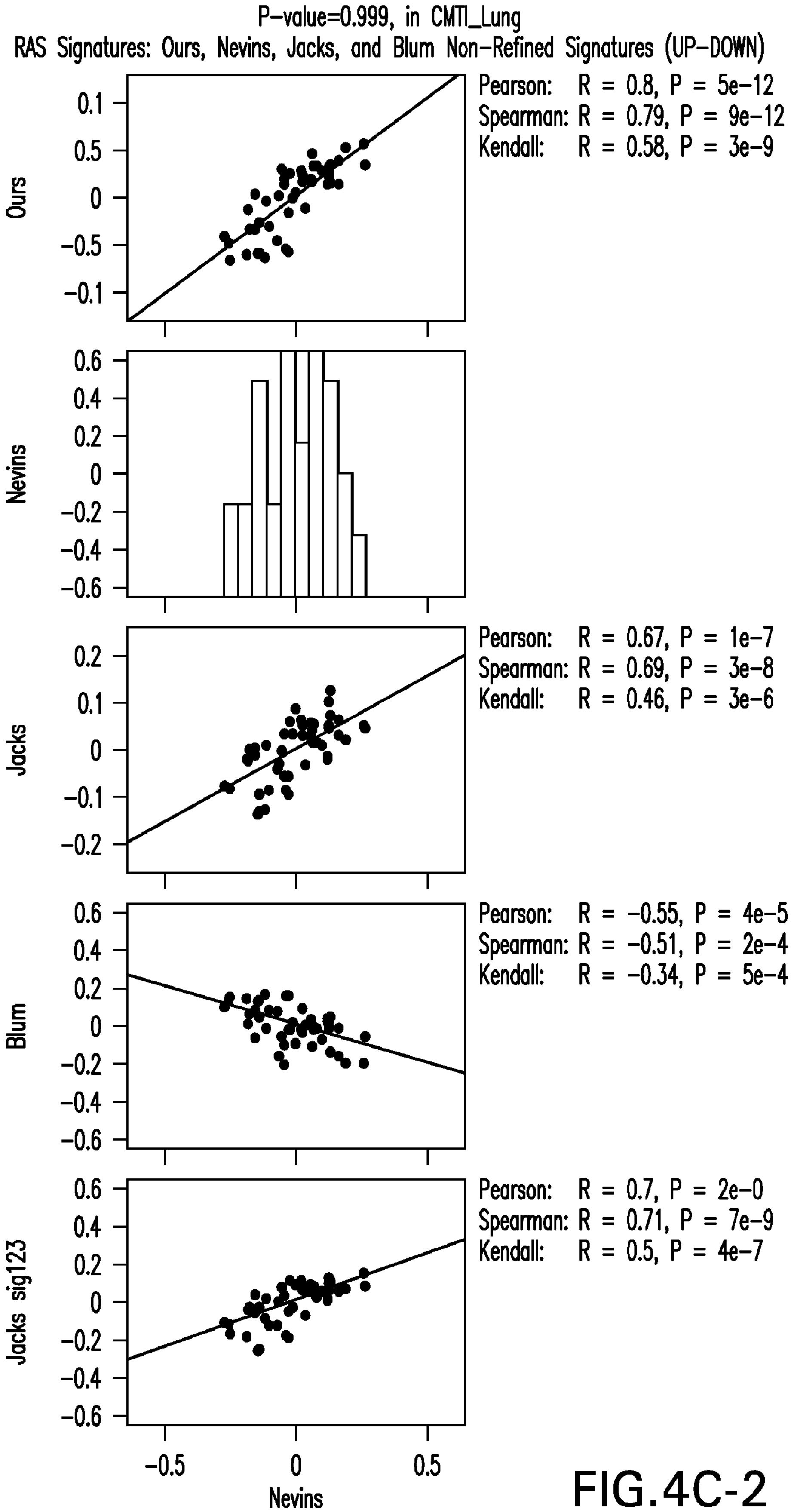
P–value=0.999, in CMTI_Lung
RAS Signatures: Ours, Nevins, Jacks, and Blum Non–Refined Signatures (UP–DOWN)
Pearson: R = 0.8, P = 5e–12
Spearman: R = 0.79, P = 9e–12
Kendall: R = 0.58, P = 3e–9
Pearson: R = 0.67, P = 1e–7
Spearman: R = 0.69, P = 3e–8
Kendall: R = 0.46, P = 3e–6
Pearson: R = –0.55, P = 4e–5
Spearman: R = –0.51, P = 2e–4
Kendall: R = –0.34, P = 5e–4
Pearson: R = 0.7, P = 2e–0
Spearman: R = 0.71, P = 7e–9
Kendall: R = 0.5, P = 4e–7
Ours
Nevins
Jacks
Blum
Jacks sig123
Nevins

FIG.4C-2

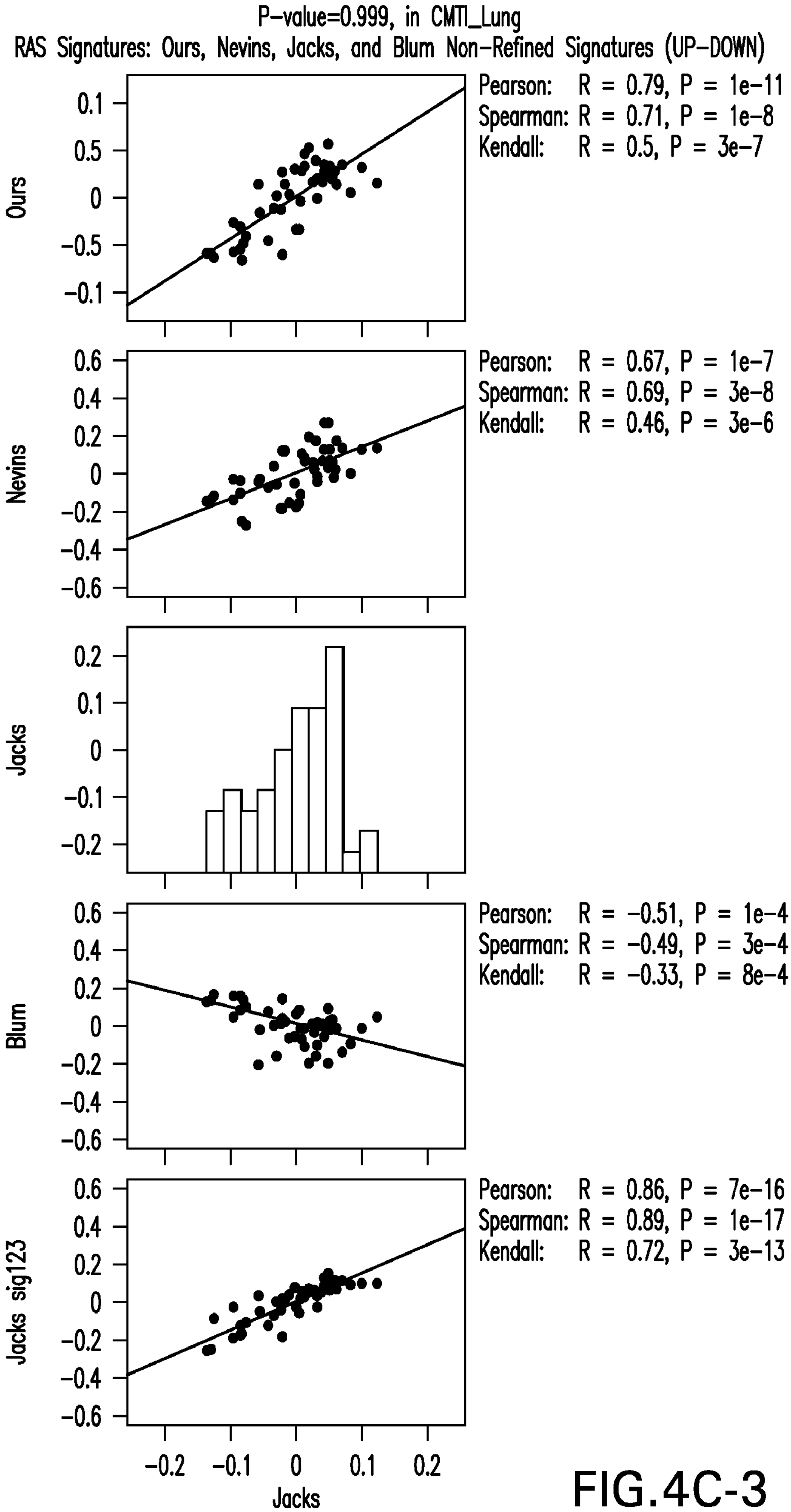
P–value=0.999, in CMTI_Lung
RAS Signatures: Ours, Nevins, Jacks, and Blum Non–Refined Signatures (UP–DOWN)
Pearson: R = 0.79, P = 1e–11
Spearman: R = 0.71, P = 1e–8
Kendall: R = 0.5, P = 3e–7
Pearson: R = 0.67, P = 1e–7
Spearman: R = 0.69, P = 3e–8
Kendall: R = 0.46, P = 3e–6
Pearson: R = –0.51, P = 1e–4
Spearman: R = –0.49, P = 3e–4
Kendall: R = –0.33, P = 8e–4
Pearson: R = 0.86, P = 7e–16
Spearman: R = 0.89, P = 1e–17
Kendall: R = 0.72, P = 3e–13
Ours
Nevins
Jacks
Blum
Jacks sig123
Jacks

FIG.4C-3

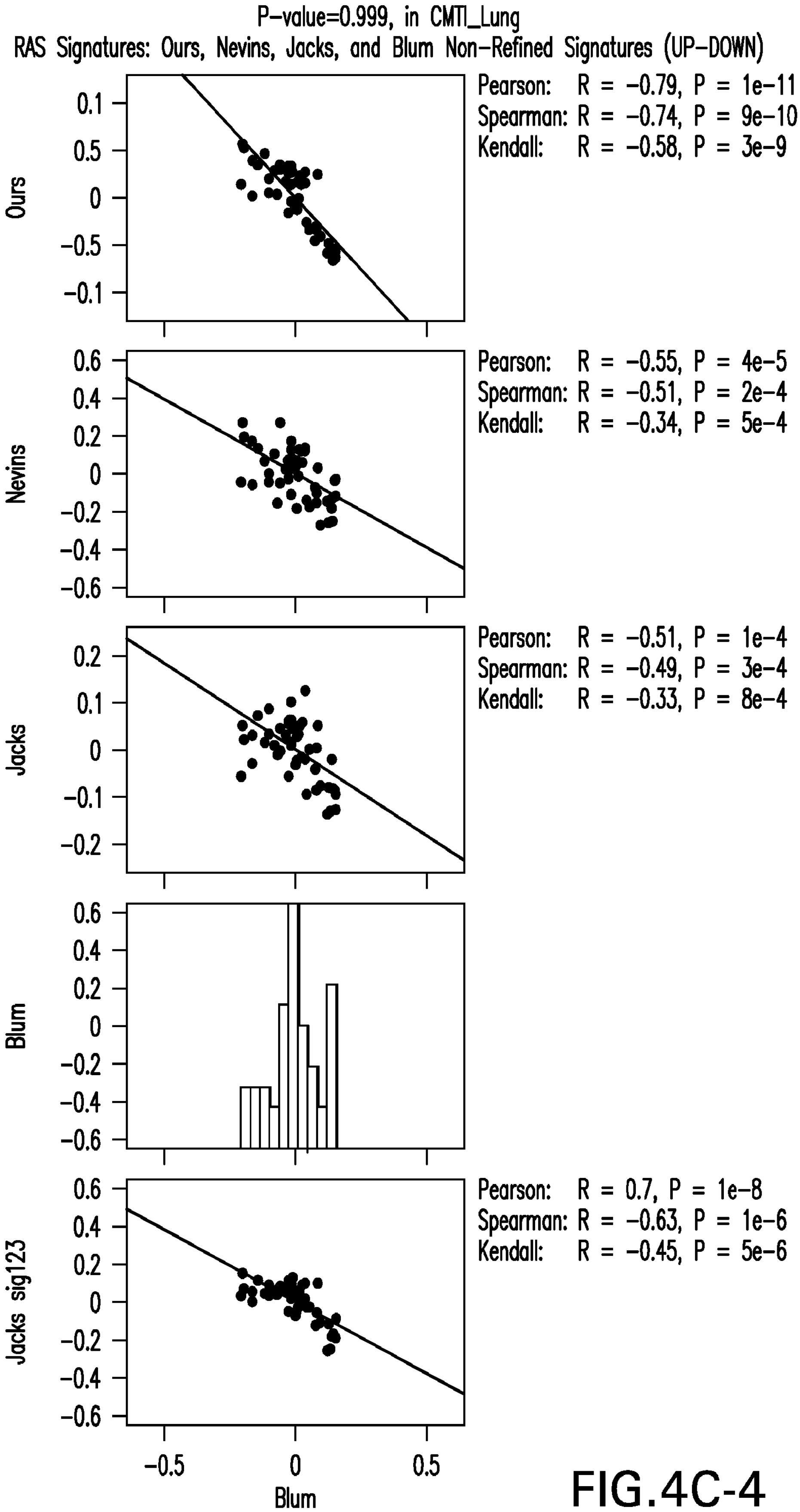
P−value=0.999, in CMTI_Lung
RAS Signatures: Ours, Nevins, Jacks, and Blum Non−Refined Signatures (UP−DOWN)
Pearson: R = −0.79, P = 1e−11
Spearman: R = −0.74, P = 9e−10
Kendall: R = −0.58, P = 3e−9
Ours
Pearson: R = −0.55, P = 4e−5
Spearman: R = −0.51, P = 2e−4
Kendall: R = −0.34, P = 5e−4
Nevins
Pearson: R = −0.51, P = 1e−4
Spearman: R = −0.49, P = 3e−4
Kendall: R = −0.33, P = 8e−4
Jacks
Blum
Pearson: R = 0.7, P = 1e−8
Spearman: R = −0.63, P = 1e−6
Kendall: R = −0.45, P = 5e−6
Jacks sig123
−0.5
0
0.5
Blum

FIG.4C-4

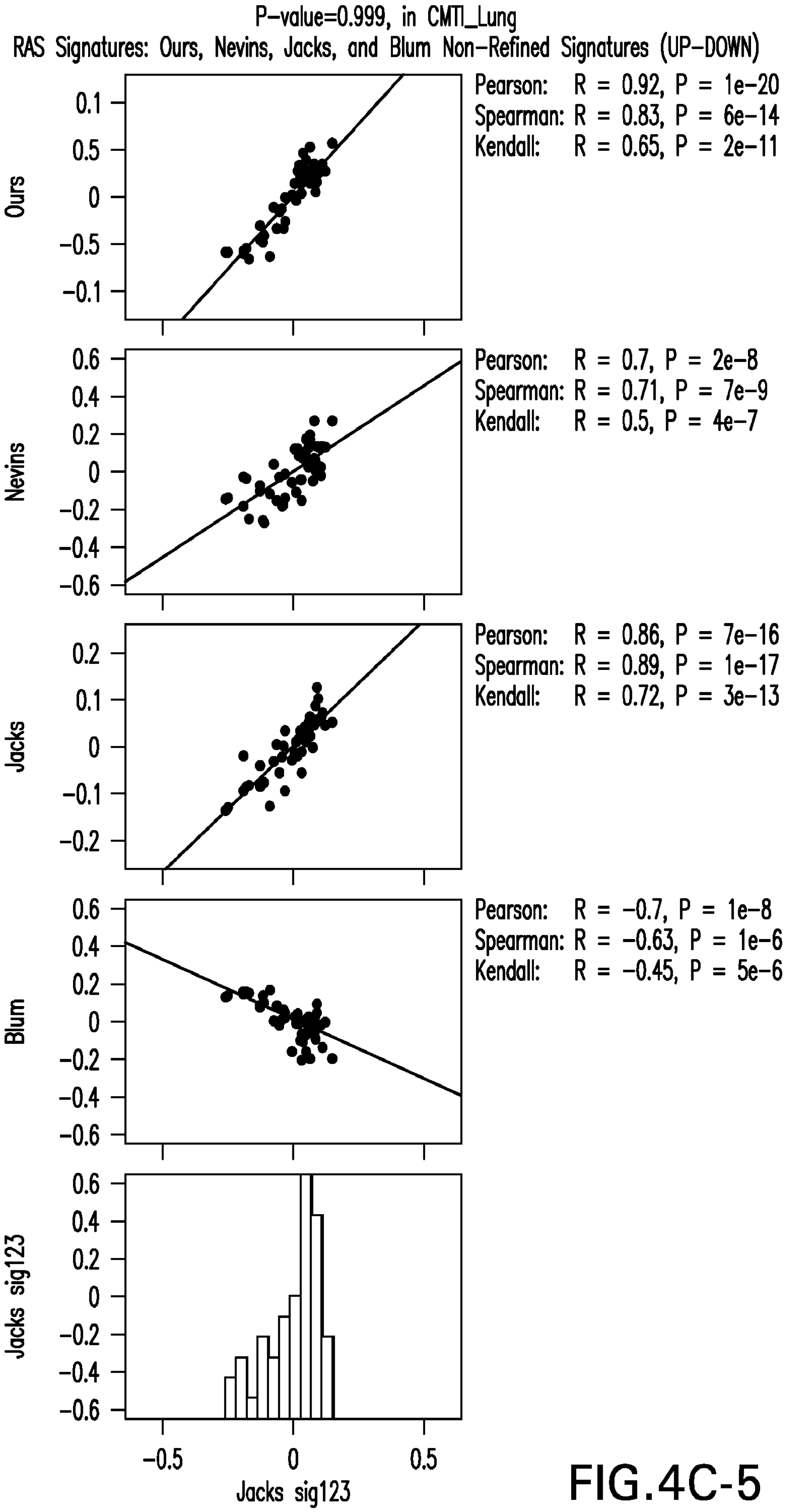
P–value=0.999, in CMTI_Lung
RAS Signatures: Ours, Nevins, Jacks, and Blum Non–Refined Signatures (UP–DOWN)
Pearson: R = 0.92, P = 1e–20
Spearman: R = 0.83, P = 6e–14
Kendall: R = 0.65, P = 2e–11
Pearson: R = 0.7, P = 2e–8
Spearman: R = 0.71, P = 7e–9
Kendall: R = 0.5, P = 4e–7
Pearson: R = 0.86, P = 7e–16
Spearman: R = 0.89, P = 1e–17
Kendall: R = 0.72, P = 3e–13
Pearson: R = –0.7, P = 1e–8
Spearman: R = –0.63, P = 1e–6
Kendall: R = –0.45, P = 5e–6
Ours
Nevins
Jacks
Blum
Jacks sig123
Jacks sig123

FIG.4C-5

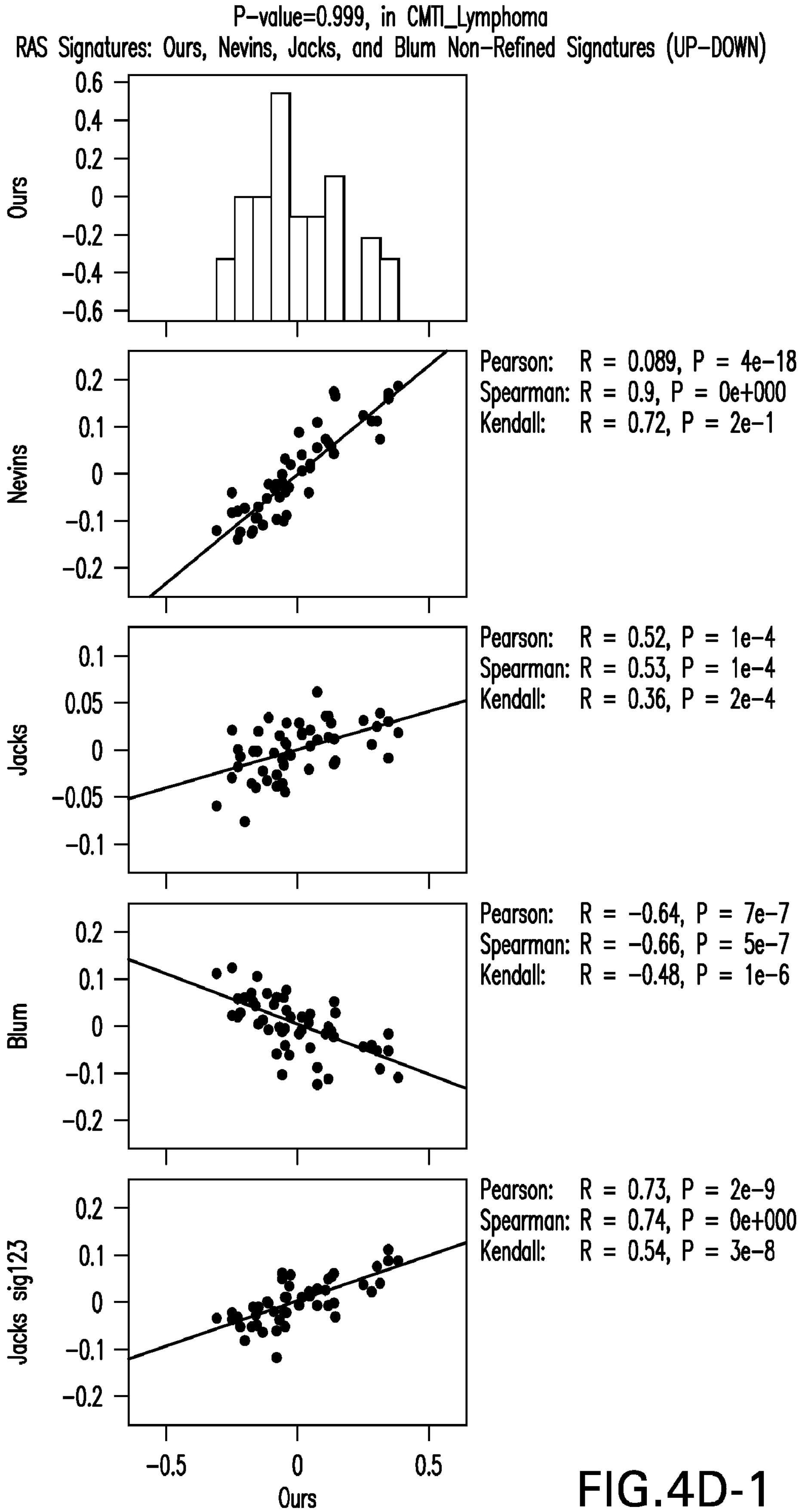
P−value=0.999, in CMTI_Lymphoma
RAS Signatures: Ours, Nevins, Jacks, and Blum Non−Refined Signatures (UP−DOWN)
Ours
Nevins
Jacks
Blum
Jacks sig123
Pearson: R = 0.089, P = 4e−18
Spearman: R = 0.9, P = 0e+000
Kendall: R = 0.72, P = 2e−1
Pearson: R = 0.52, P = 1e−4
Spearman: R = 0.53, P = 1e−4
Kendall: R = 0.36, P = 2e−4
Pearson: R = −0.64, P = 7e−7
Spearman: R = −0.66, P = 5e−7
Kendall: R = −0.48, P = 1e−6
Pearson: R = 0.73, P = 2e−9
Spearman: R = 0.74, P = 0e+000
Kendall: R = 0.54, P = 3e−8
−0.5
0
0.5
Ours

FIG.4D-1

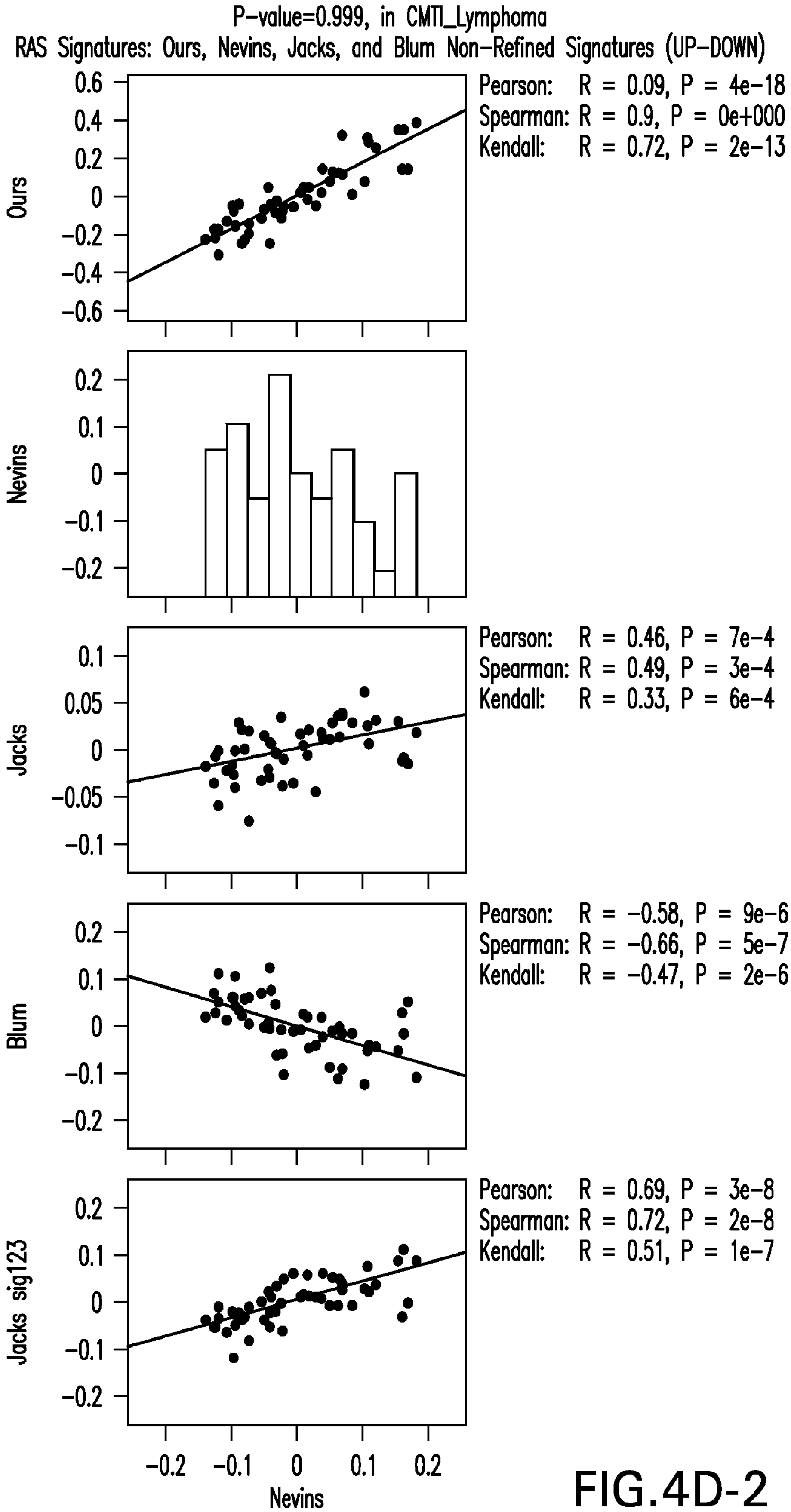
P-value=0.999, in CMTI_Lymphoma
RAS Signatures: Ours, Nevins, Jacks, and Blum Non-Refined Signatures (UP-DOWN)
Pearson: R = 0.09, P = 4e-18
Spearman: R = 0.9, P = 0e+000
Kendall: R = 0.72, P = 2e-13
Ours
Nevins
Pearson: R = 0.46, P = 7e-4
Spearman: R = 0.49, P = 3e-4
Kendall: R = 0.33, P = 6e-4
Jacks
Pearson: R = -0.58, P = 9e-6
Spearman: R = -0.66, P = 5e-7
Kendall: R = -0.47, P = 2e-6
Blum
Pearson: R = 0.69, P = 3e-8
Spearman: R = 0.72, P = 2e-8
Kendall: R = 0.51, P = 1e-7
Jacks sig123
-0.2 -0.1 0 0.1 0.2
Nevins

FIG.4D-2

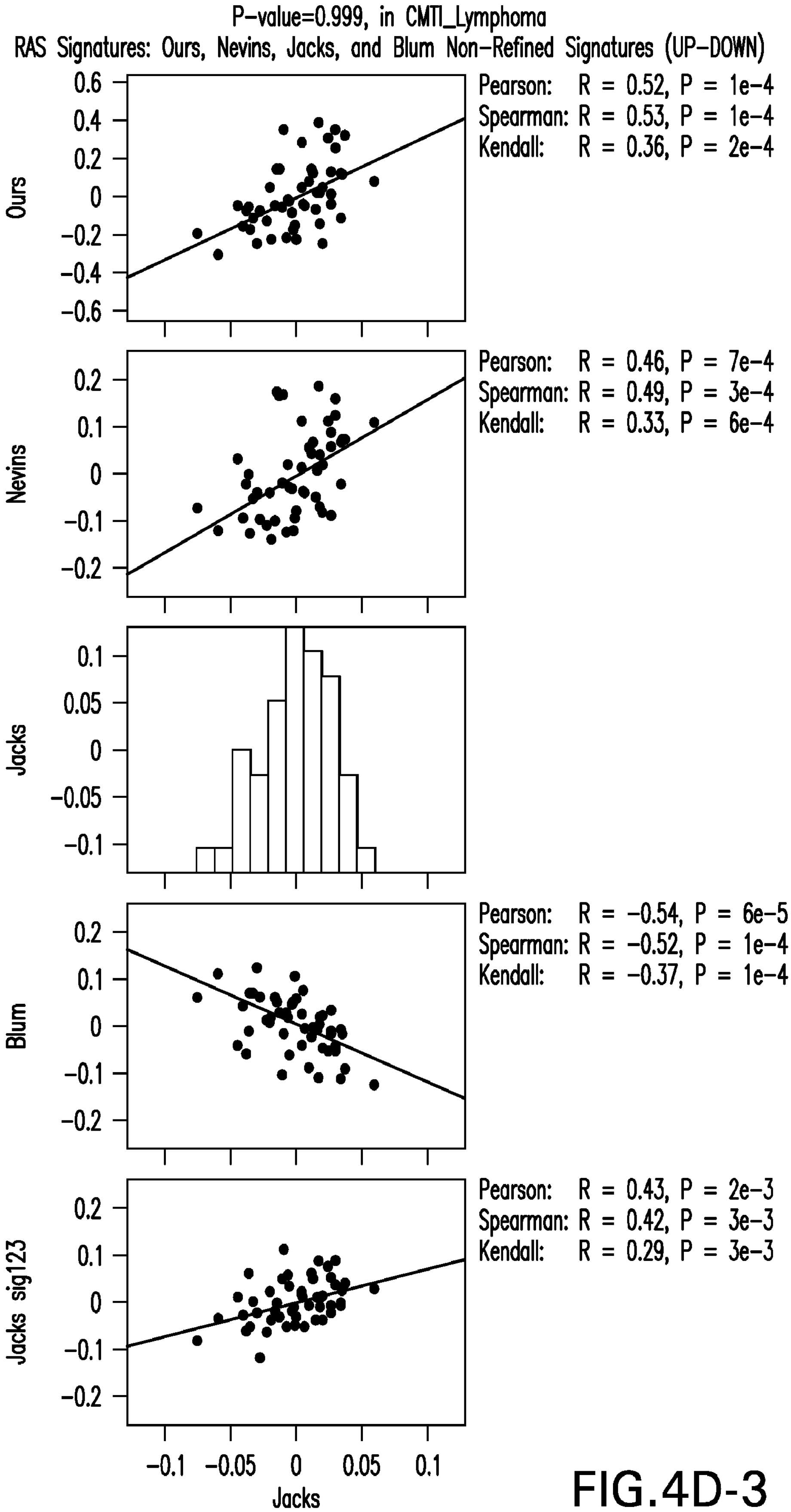
P-value=0.999, in CMTI_Lymphoma
RAS Signatures: Ours, Nevins, Jacks, and Blum Non-Refined Signatures (UP-DOWN)
Pearson: R = 0.52, P = 1e-4
Spearman: R = 0.53, P = 1e-4
Kendall: R = 0.36, P = 2e-4
Ours
Pearson: R = 0.46, P = 7e-4
Spearman: R = 0.49, P = 3e-4
Kendall: R = 0.33, P = 6e-4
Nevins
Jacks
Pearson: R = -0.54, P = 6e-5
Spearman: R = -0.52, P = 1e-4
Kendall: R = -0.37, P = 1e-4
Blum
Pearson: R = 0.43, P = 2e-3
Spearman: R = 0.42, P = 3e-3
Kendall: R = 0.29, P = 3e-3
Jacks sig123
-0.1 -0.05 0 0.05 0.1
Jacks

FIG.4D-3

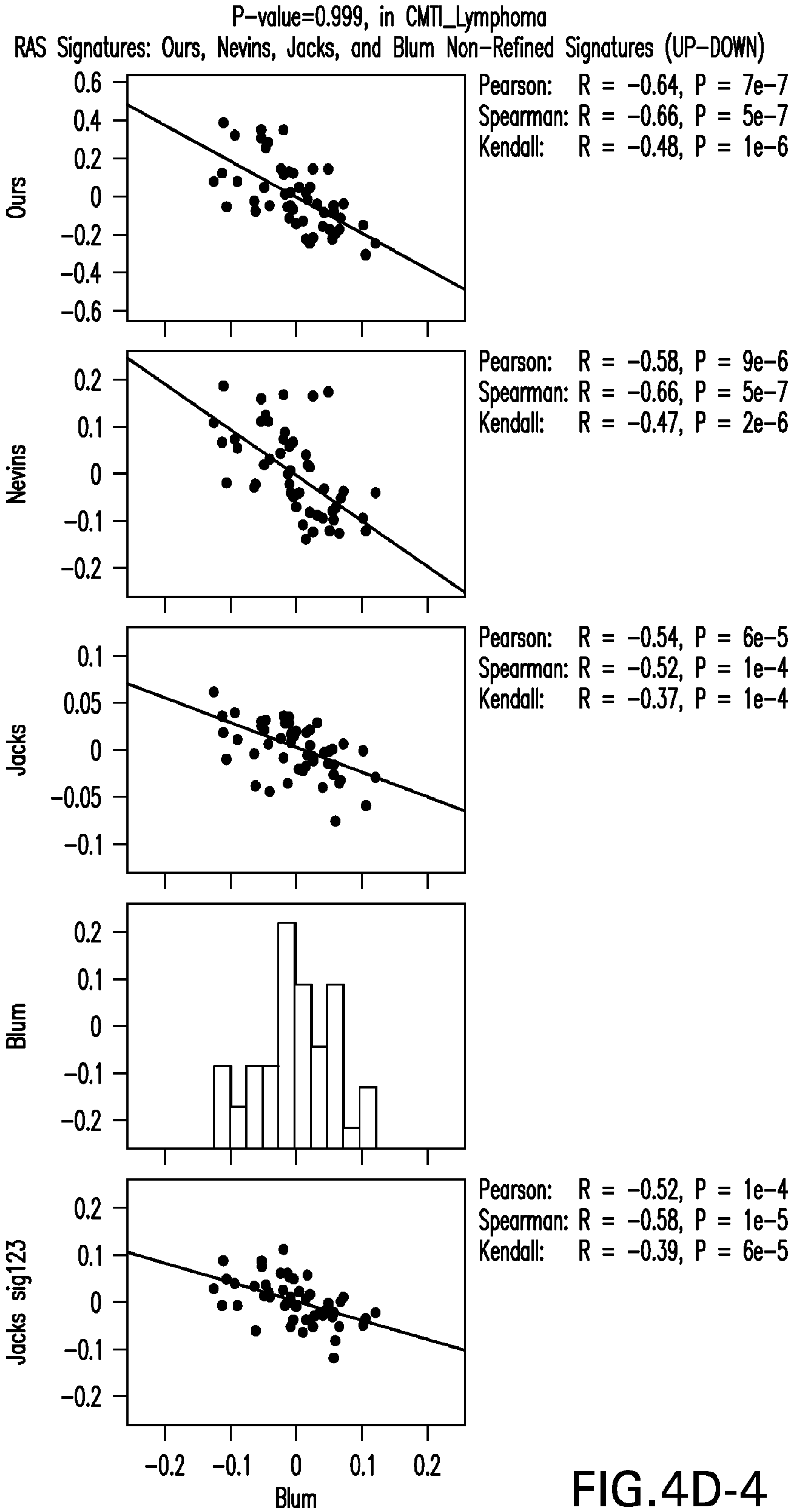
P−value=0.999, in CMTI_Lymphoma
RAS Signatures: Ours, Nevins, Jacks, and Blum Non−Refined Signatures (UP−DOWN)
Pearson: R = −0.64, P = 7e−7
Spearman: R = −0.66, P = 5e−7
Kendall: R = −0.48, P = 1e−6
Ours
Pearson: R = −0.58, P = 9e−6
Spearman: R = −0.66, P = 5e−7
Kendall: R = −0.47, P = 2e−6
Nevins
Pearson: R = −0.54, P = 6e−5
Spearman: R = −0.52, P = 1e−4
Kendall: R = −0.37, P = 1e−4
Jacks
Blum
Pearson: R = −0.52, P = 1e−4
Spearman: R = −0.58, P = 1e−5
Kendall: R = −0.39, P = 6e−5
Jacks sig123
Blum

FIG.4D-4

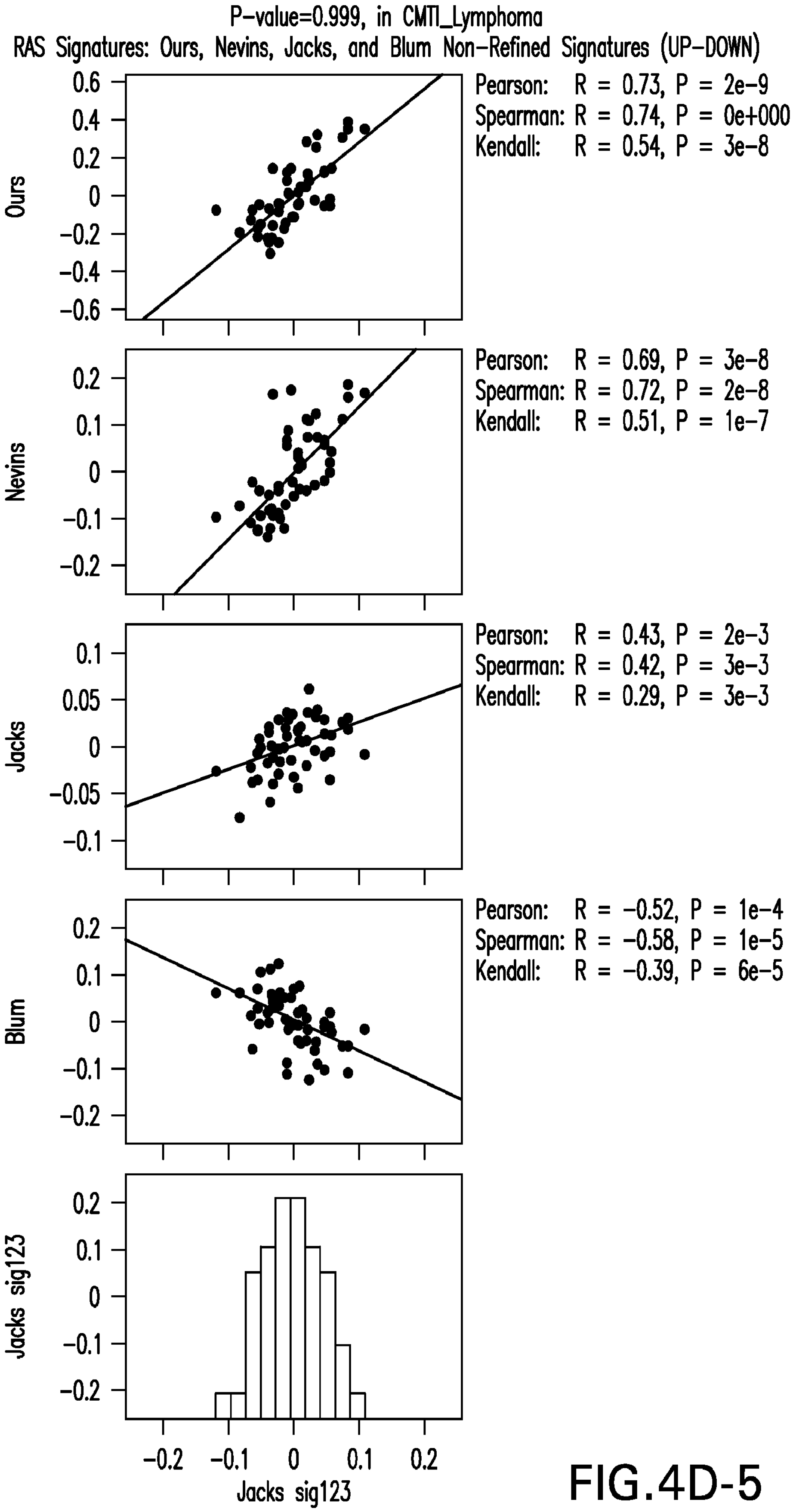
P-value=0.999, in CMTI_Lymphoma
RAS Signatures: Ours, Nevins, Jacks, and Blum Non-Refined Signatures (UP-DOWN)
Pearson: R = 0.73, P = 2e-9
Spearman: R = 0.74, P = 0e+000
Kendall: R = 0.54, P = 3e-8
Ours
Pearson: R = 0.69, P = 3e-8
Spearman: R = 0.72, P = 2e-8
Kendall: R = 0.51, P = 1e-7
Nevins
Pearson: R = 0.43, P = 2e-3
Spearman: R = 0.42, P = 3e-3
Kendall: R = 0.29, P = 3e-3
Jacks
Pearson: R = -0.52, P = 1e-4
Spearman: R = -0.58, P = 1e-5
Kendall: R = -0.39, P = 6e-5
Blum
Jacks sig123
Jacks sig123

FIG.4D-5

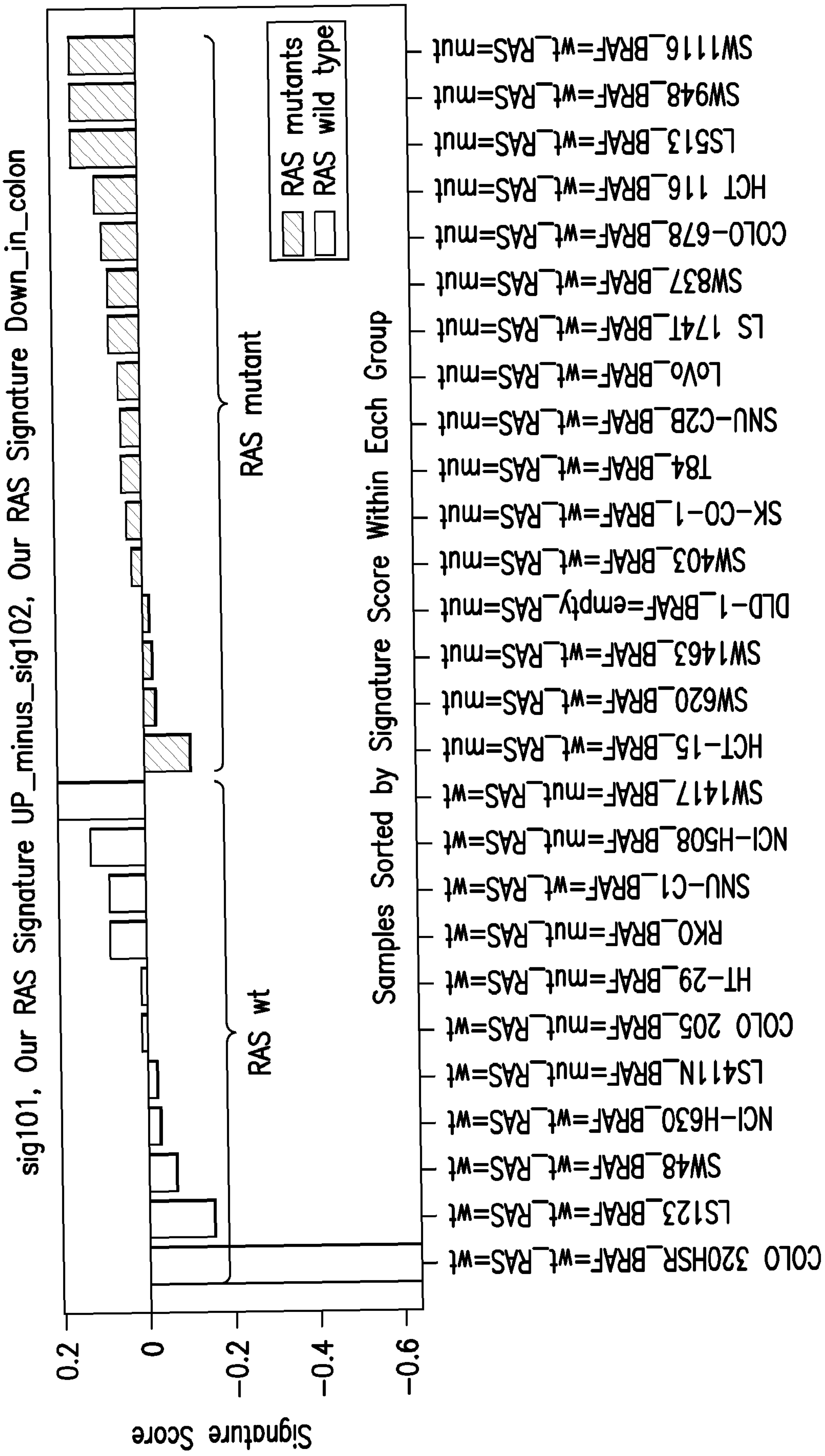
sig101, Our RAS Signature UP_minus_sig102, Our RAS Signature Down_in_colon
Signature Score
0.2
0
-0.2
-0.4
-0.6
RAS mutants
RAS wild type
RAS mutant
RAS wt
Samples Sorted by Signature Score Within Each Group
SW1116_BRAF=wt_RAS=mut
SW948_BRAF=wt_RAS=mut
LS513_BRAF=wt_RAS=mut
HCT 116_BRAF=wt_RAS=mut
COLO-678_BRAF=wt_RAS=mut
SW837_BRAF=wt_RAS=mut
LS 174T_BRAF=wt_RAS=mut
LoVo_BRAF=wt_RAS=mut
SNU-C2B_BRAF=wt_RAS=mut
T84_BRAF=wt_RAS=mut
SK-CO-1_BRAF=wt_RAS=mut
SW403_BRAF=wt_RAS=mut
DLD-1_BRAF=empty_RAS=mut
SW1463_BRAF=wt_RAS=mut
SW620_BRAF=wt_RAS=mut
HCT-15_BRAF=wt_RAS=mut
SW1417_BRAF=mut_RAS=wt
NCI-H508_BRAF=mut_RAS=wt
SNU-C1_BRAF=wt_RAS=wt
RKO_BRAF=mut_RAS=wt
HT-29_BRAF=mut_RAS=wt
COLO 205_BRAF=mut_RAS=wt
LS411N_BRAF=mut_RAS=wt
NCI-H630_BRAF=wt_RAS=wt
SW48_BRAF=wt_RAS=wt
LS123_BRAF=wt_RAS=wt
COLO 320HSR_BRAF=wt_RAS=wt

FIG.5A

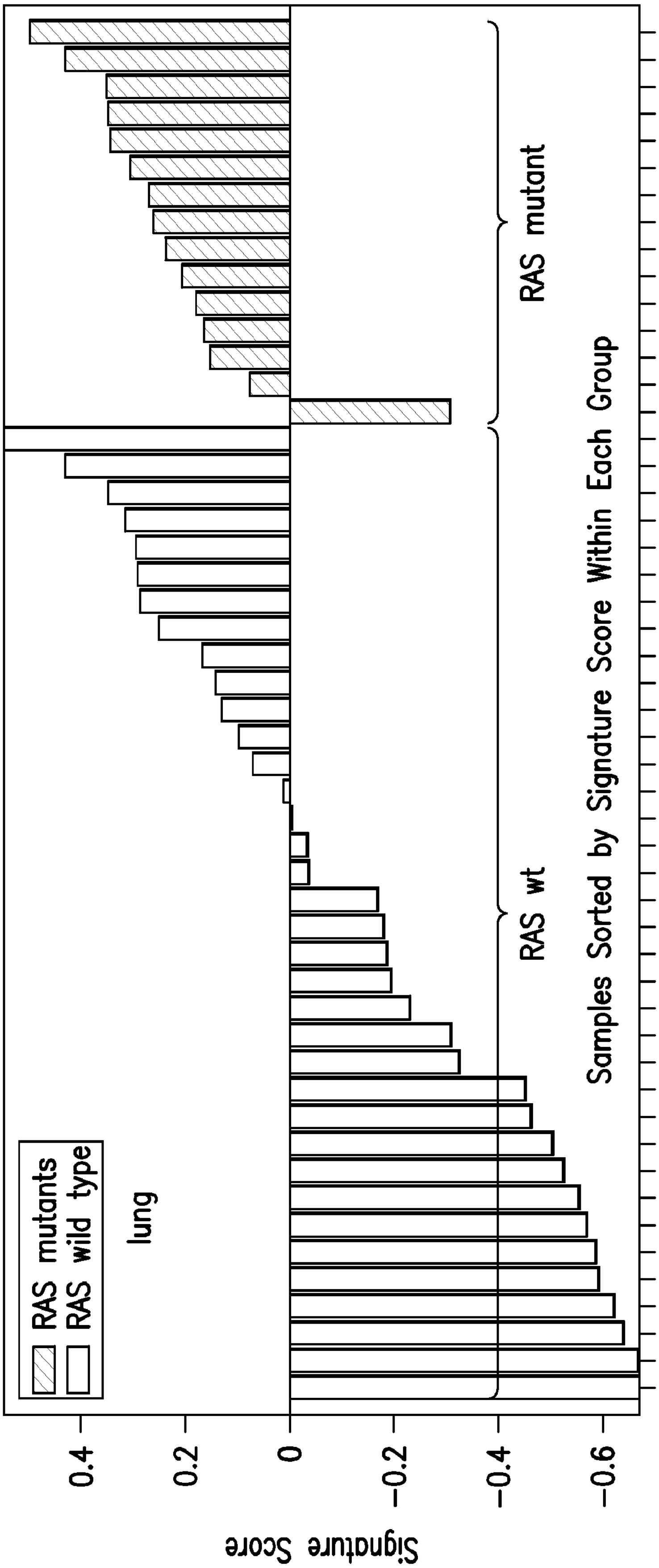
RAS mutants
RAS wild type
lung
RAS mutant
RAS wt
Samples Sorted by Signature Score Within Each Group
Signature Score
0.4
0.2
0
−0.2
−0.4
−0.6

FIG.5B

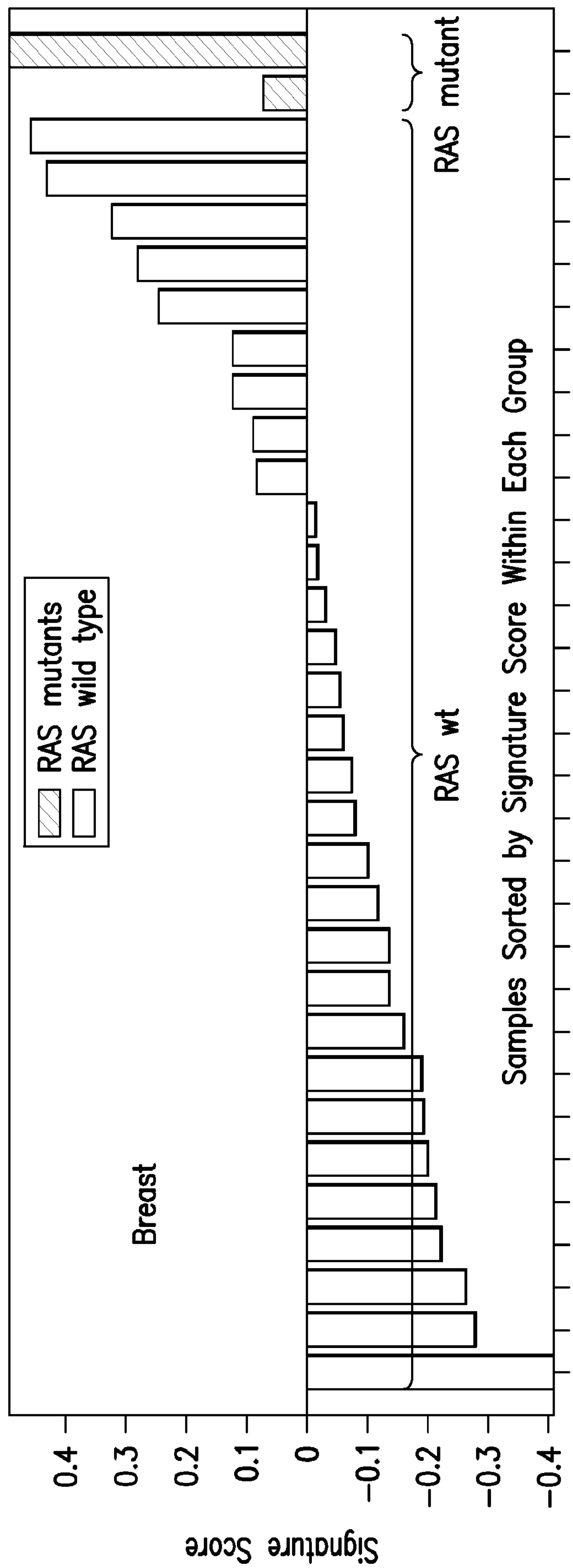
Breast
RAS mutants
RAS wild type
RAS wt
RAS mutant
Samples Sorted by Signature Score Within Each Group
Signature Score
0.4
0.3
0.2
0.1
0
−0.1
−0.2
−0.3
−0.4

FIG.5C

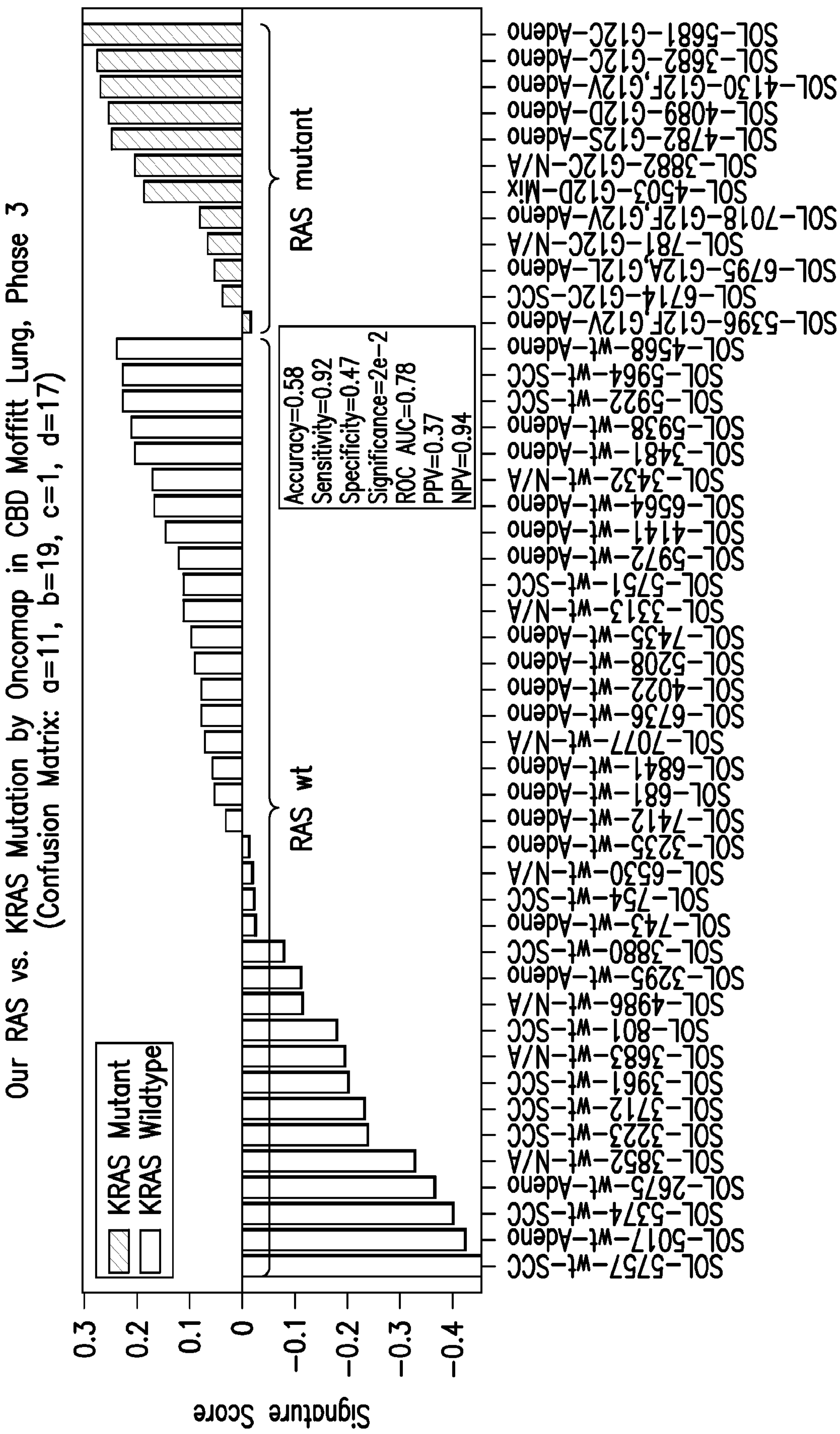
Our RAS vs. KRAS Mutation by Oncomap in CBD Moffitt Lung, Phase 3
(Confusion Matrix: a=11, b=19, c=1, d=17)
KRAS Mutant
KRAS Wildtype
RAS mutant
RAS wt
Accuracy=0.58
Sensitivity=0.92
Specificity=0.47
Significance=2e-2
ROC AUC=0.78
PPV=0.37
NPV=0.94
Signature Score
0.3
0.2
0.1
0
-0.1
-0.2
-0.3
-0.4
SOL-5681-G12C-Adeno
SOL-3682-G12C-Adeno
SOL-4130-G12F,G12V-Adeno
SOL-4089-G12D-Adeno
SOL-4782-G12S-Adeno
SOL-3882-G12C-N/A
SOL-4503-G12D-Mix
SOL-7018-G12F,G12V-Adeno
SOL-781-G12C-N/A
SOL-6795-G12A,G12L-Adeno
SOL-6714-G12C-SCC
SOL-5396-G12F,G12V-Adeno
SOL-4568-wt-Adeno
SOL-5964-wt-SCC
SOL-5922-wt-SCC
SOL-5938-wt-Adeno
SOL-3481-wt-Adeno
SOL-3432-wt-N/A
SOL-6564-wt-Adeno
SOL-4141-wt-Adeno
SOL-5972-wt-Adeno
SOL-5751-wt-SCC
SOL-3313-wt-N/A
SOL-7435-wt-Adeno
SOL-5208-wt-Adeno
SOL-4022-wt-Adeno
SOL-6736-wt-Adeno
SOL-7077-wt-N/A
SOL-6841-wt-Adeno
SOL-681-wt-Adeno
SOL-7412-wt-Adeno
SOL-3235-wt-Adeno
SOL-6530-wt-N/A
SOL-754-wt-SCC
SOL-743-wt-Adeno
SOL-3880-wt-SCC
SOL-3295-wt-Adeno
SOL-4986-wt-N/A
SOL-801-wt-SCC
SOL-3683-wt-N/A
SOL-3961-wt-SCC
SOL-3712-wt-SCC
SOL-3223-wt-SCC
SOL-3852-wt-N/A
SOL-2675-wt-Adeno
SOL-5374-wt-SCC
SOL-5017-wt-Adeno
SOL-5757-wt-SCC

FIG.6

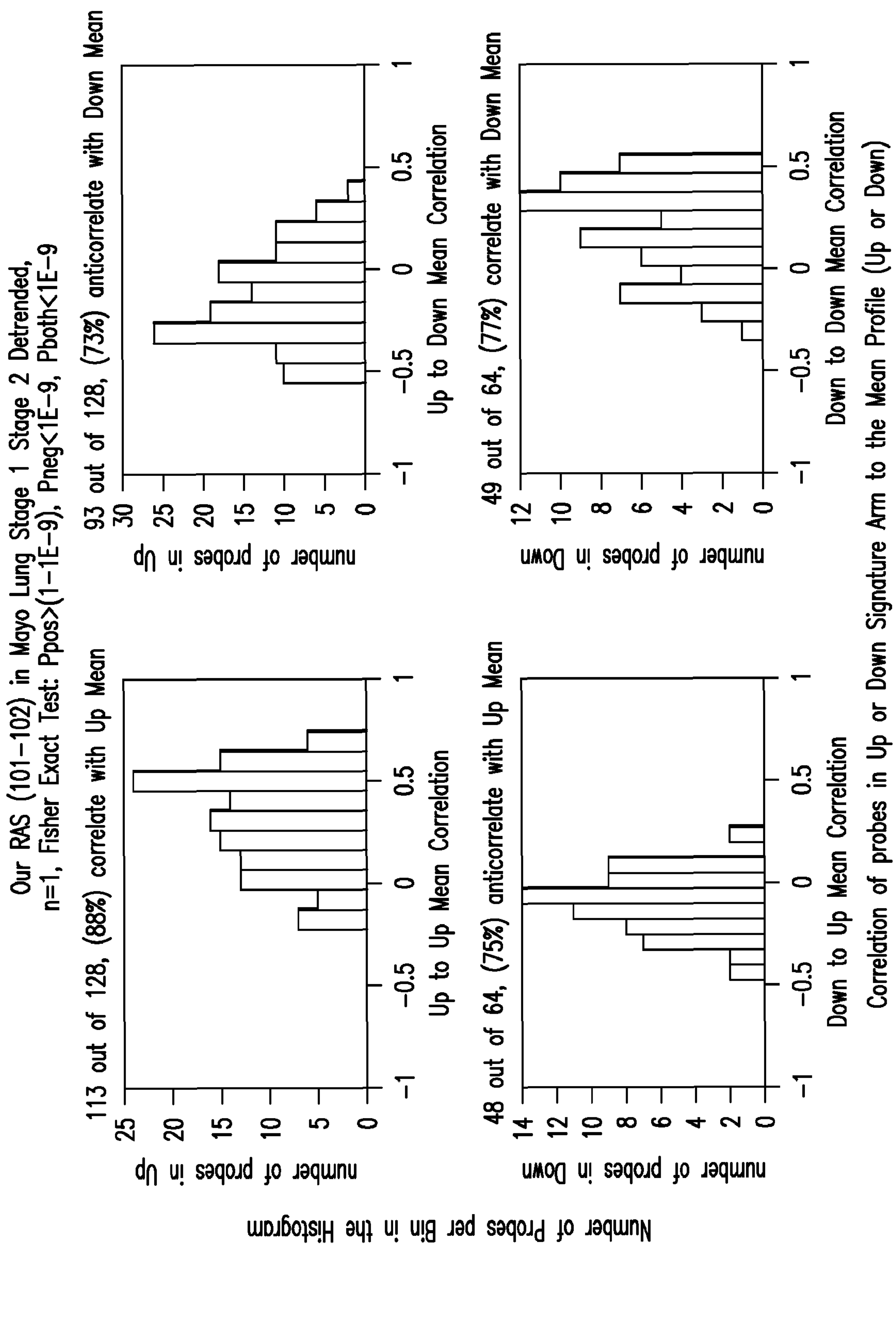
Our RAS (101-102) in Mayo Lung Stage 1 Stage 2 Detrended,
n=1, Fisher Exact Test: Ppos>(1-1E-9), Pneg<1E-9, Pboth<1E-9
113 out of 128, (88%) correlate with Up Mean
93 out of 128, (73%) anticorrelate with Down Mean
48 out of 64, (75%) anticorrelate with Up Mean
49 out of 64, (77%) correlate with Down Mean
number of probes in Up
number of probes in Down
Number of Probes per Bin in the Histogram
Up to Up Mean Correlation
Up to Down Mean Correlation
Down to Up Mean Correlation
Down to Down Mean Correlation
Correlation of probes in Up or Down Signature Arm to the Mean Profile (Up or Down)
0
5
10
15
20
25
30
2
4
6
8
10
12
14
-1
-0.5
0
0.5
1

FIG.7A

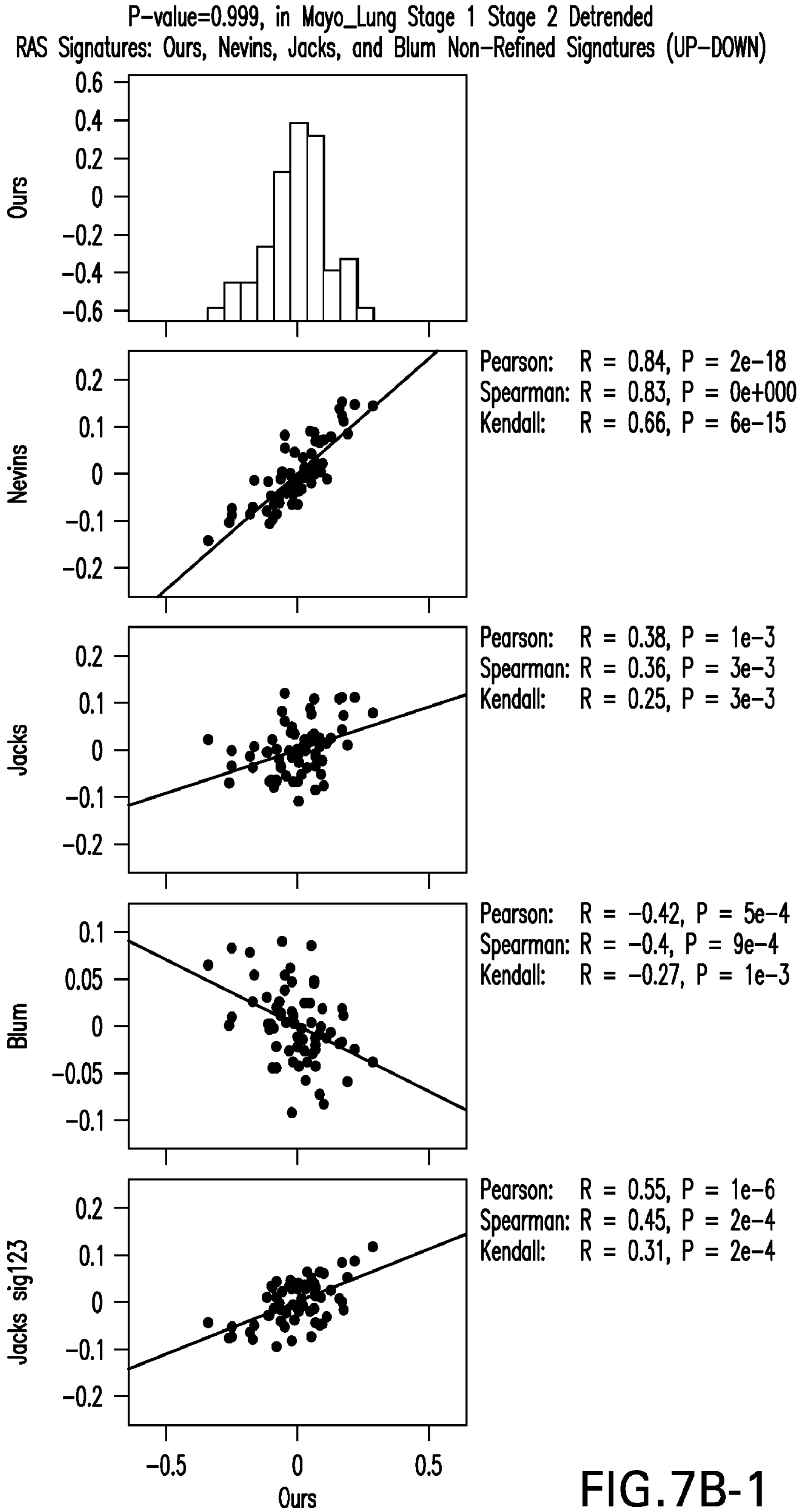

P−value=0.999, in Mayo_Lung Stage 1 Stage 2 Detrended
RAS Signatures: Ours, Nevins, Jacks, and Blum Non−Refined Signatures (UP−DOWN)
Ours
Nevins
Jacks
Blum
Jacks sig123
Ours
Pearson: R = 0.84, P = 2e−18
Spearman: R = 0.83, P = 0e+000
Kendall: R = 0.66, P = 6e−15
Pearson: R = 0.38, P = 1e−3
Spearman: R = 0.36, P = 3e−3
Kendall: R = 0.25, P = 3e−3
Pearson: R = −0.42, P = 5e−4
Spearman: R = −0.4, P = 9e−4
Kendall: R = −0.27, P = 1e−3
Pearson: R = 0.55, P = 1e−6
Spearman: R = 0.45, P = 2e−4
Kendall: R = 0.31, P = 2e−4


FIG.7B-1

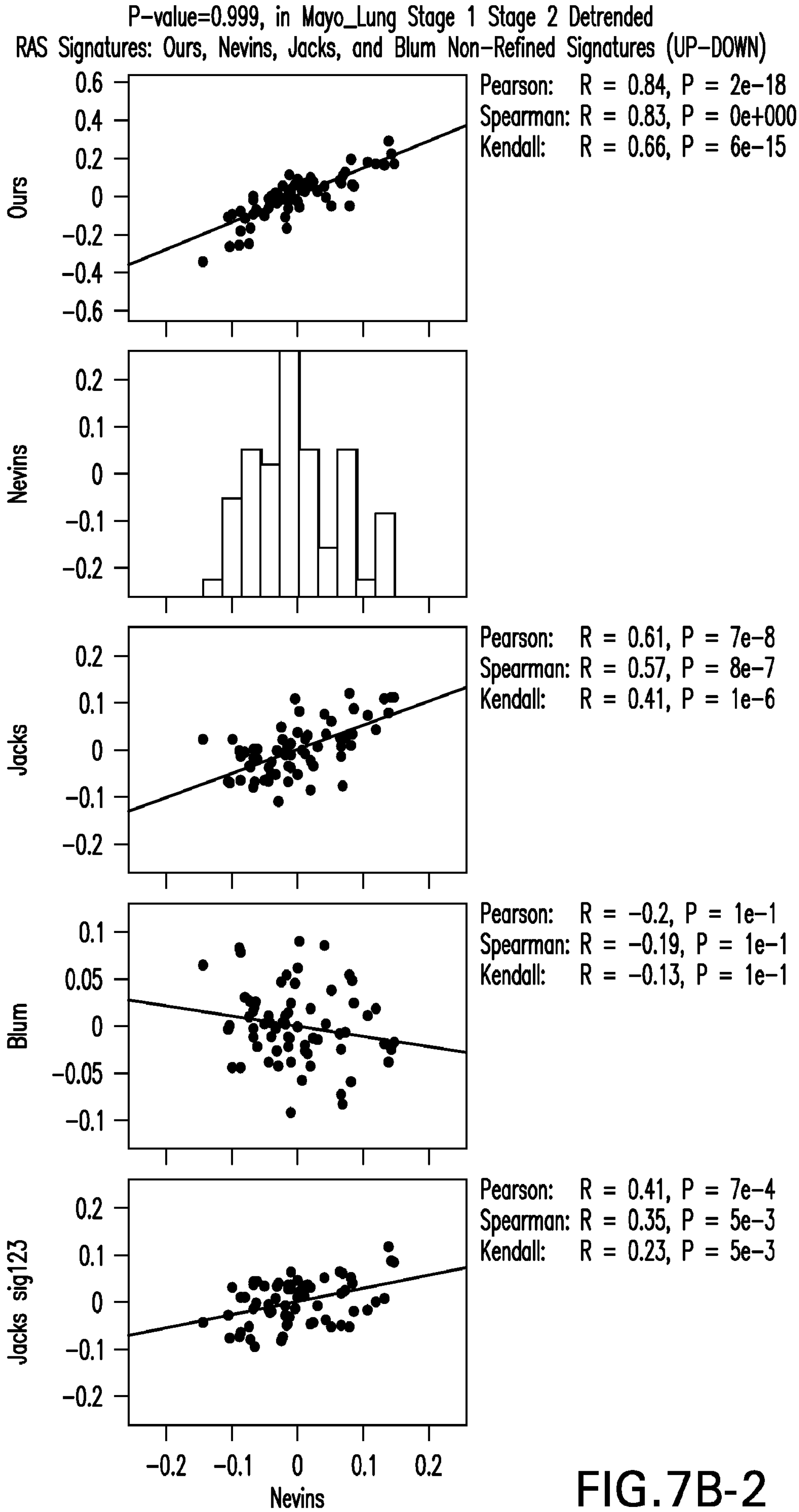
P–value=0.999, in Mayo_Lung Stage 1 Stage 2 Detrended
RAS Signatures: Ours, Nevins, Jacks, and Blum Non–Refined Signatures (UP–DOWN)
Ours
Pearson: R = 0.84, P = 2e–18
Spearman: R = 0.83, P = 0e+000
Kendall: R = 0.66, P = 6e–15
Nevins
Jacks
Pearson: R = 0.61, P = 7e–8
Spearman: R = 0.57, P = 8e–7
Kendall: R = 0.41, P = 1e–6
Blum
Pearson: R = –0.2, P = 1e–1
Spearman: R = –0.19, P = 1e–1
Kendall: R = –0.13, P = 1e–1
Jacks sig123
Pearson: R = 0.41, P = 7e–4
Spearman: R = 0.35, P = 5e–3
Kendall: R = 0.23, P = 5e–3
Nevins

FIG.7B-2

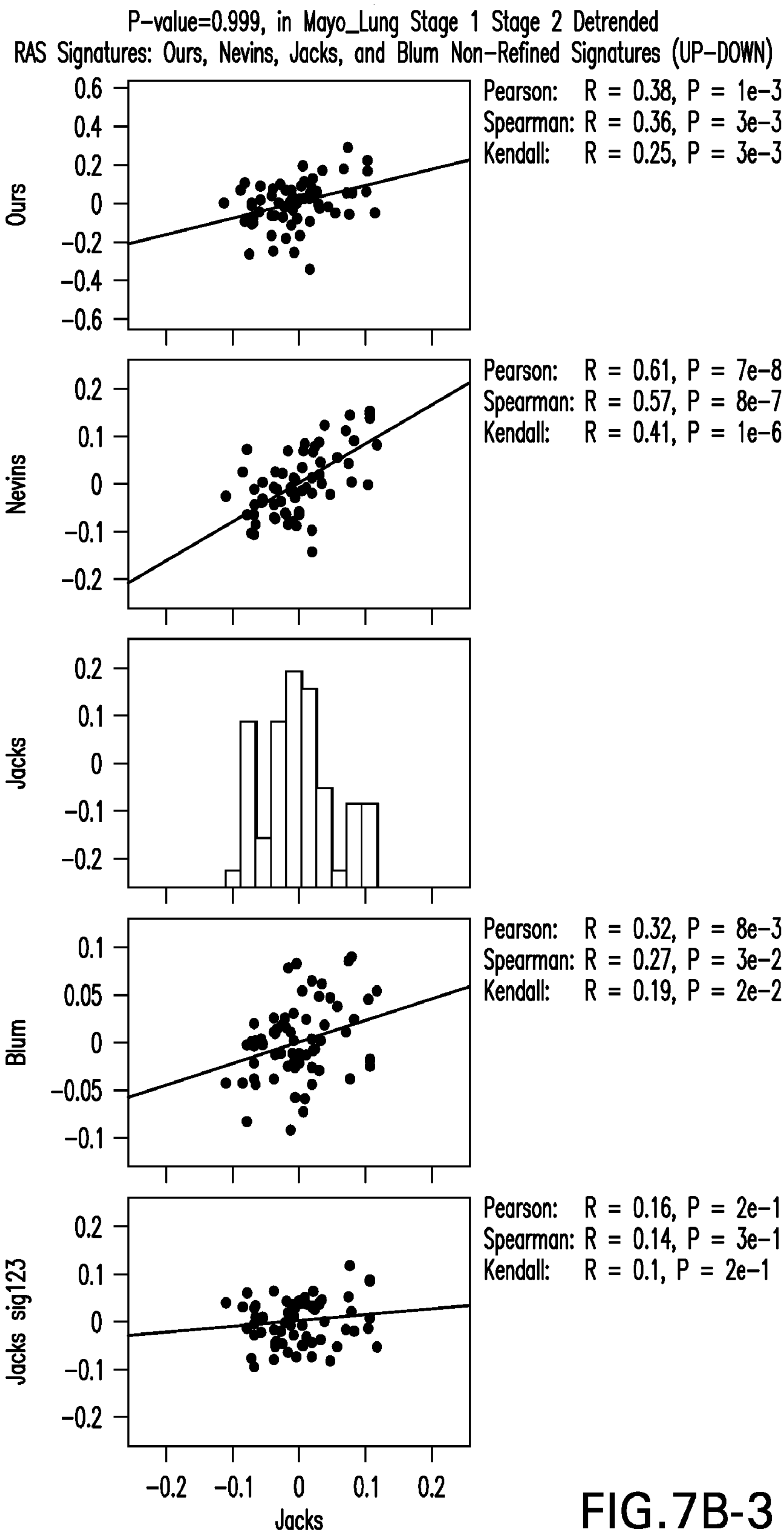
P-value=0.999, in Mayo_Lung Stage 1 Stage 2 Detrended
RAS Signatures: Ours, Nevins, Jacks, and Blum Non-Refined Signatures (UP-DOWN)
Pearson: R = 0.38, P = 1e-3
Spearman: R = 0.36, P = 3e-3
Kendall: R = 0.25, P = 3e-3
Ours
Pearson: R = 0.61, P = 7e-8
Spearman: R = 0.57, P = 8e-7
Kendall: R = 0.41, P = 1e-6
Nevins
Jacks
Pearson: R = 0.32, P = 8e-3
Spearman: R = 0.27, P = 3e-2
Kendall: R = 0.19, P = 2e-2
Blum
Pearson: R = 0.16, P = 2e-1
Spearman: R = 0.14, P = 3e-1
Kendall: R = 0.1, P = 2e-1
Jacks sig123
Jacks

FIG.7B-3

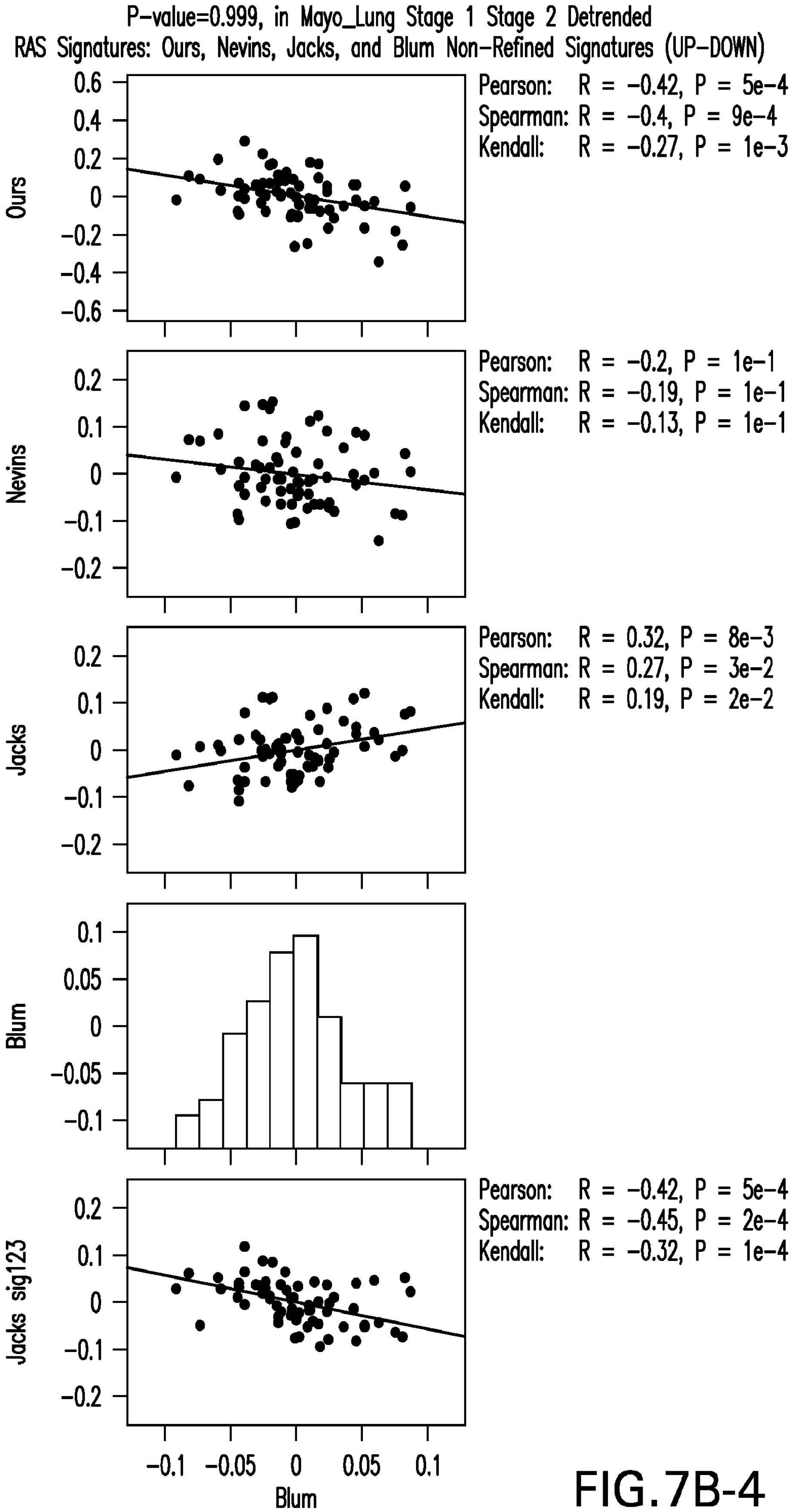
P-value=0.999, in Mayo_Lung Stage 1 Stage 2 Detrended
RAS Signatures: Ours, Nevins, Jacks, and Blum Non-Refined Signatures (UP-DOWN)
Pearson: R = -0.42, P = 5e-4
Spearman: R = -0.4, P = 9e-4
Kendall: R = -0.27, P = 1e-3
Ours
Pearson: R = -0.2, P = 1e-1
Spearman: R = -0.19, P = 1e-1
Kendall: R = -0.13, P = 1e-1
Nevins
Pearson: R = 0.32, P = 8e-3
Spearman: R = 0.27, P = 3e-2
Kendall: R = 0.19, P = 2e-2
Jacks
Blum
Pearson: R = -0.42, P = 5e-4
Spearman: R = -0.45, P = 2e-4
Kendall: R = -0.32, P = 1e-4
Jacks sig123
-0.1 -0.05 0 0.05 0.1
Blum

FIG.7B-4

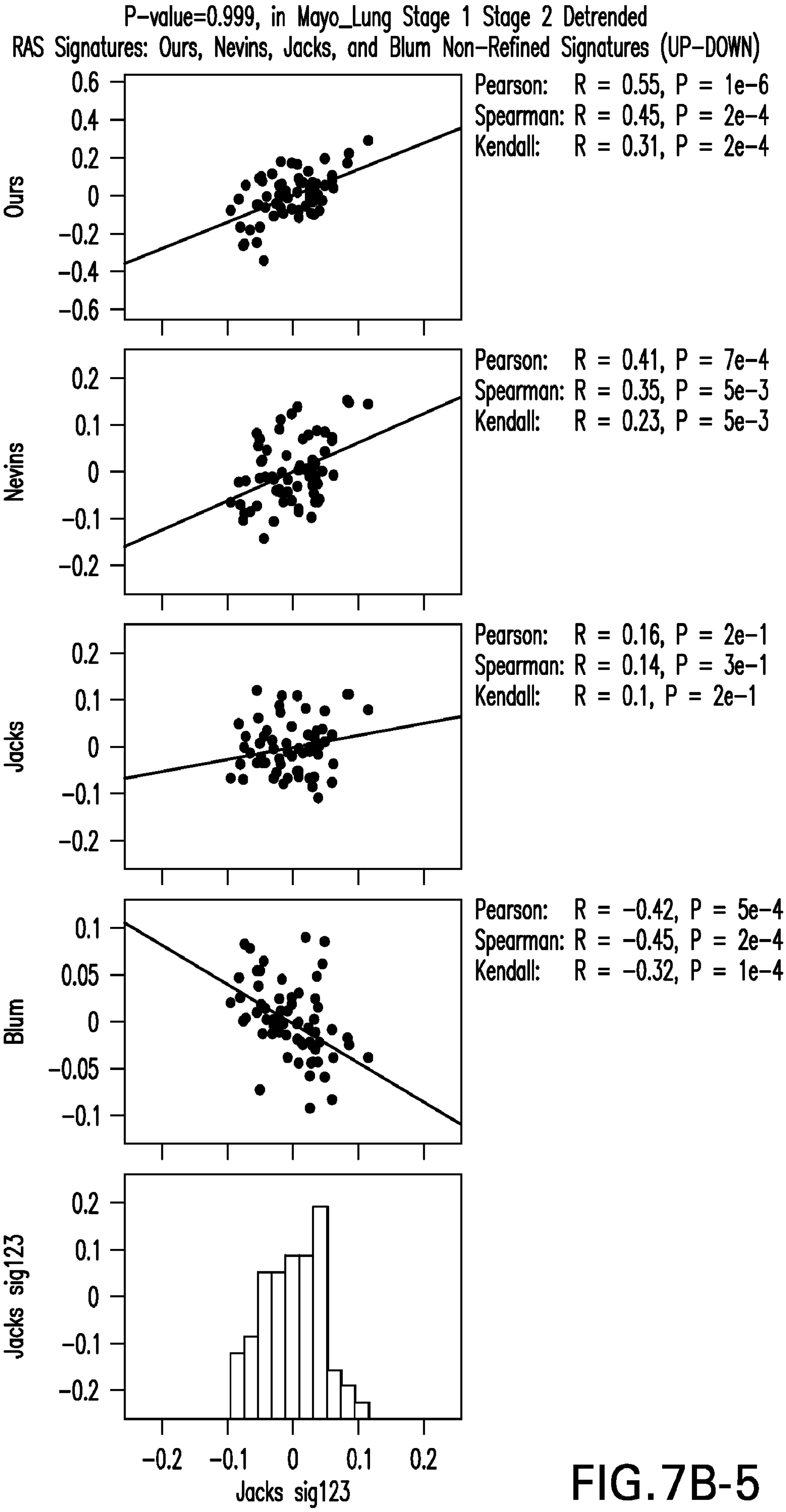
P-value=0.999, in Mayo_Lung Stage 1 Stage 2 Detrended
RAS Signatures: Ours, Nevins, Jacks, and Blum Non-Refined Signatures (UP-DOWN)
Pearson: R = 0.55, P = 1e-6
Spearman: R = 0.45, P = 2e-4
Kendall: R = 0.31, P = 2e-4
Ours
Pearson: R = 0.41, P = 7e-4
Spearman: R = 0.35, P = 5e-3
Kendall: R = 0.23, P = 5e-3
Nevins
Pearson: R = 0.16, P = 2e-1
Spearman: R = 0.14, P = 3e-1
Kendall: R = 0.1, P = 2e-1
Jacks
Pearson: R = -0.42, P = 5e-4
Spearman: R = -0.45, P = 2e-4
Kendall: R = -0.32, P = 1e-4
Blum
Jacks sig123
Jacks sig123

FIG.7B-5

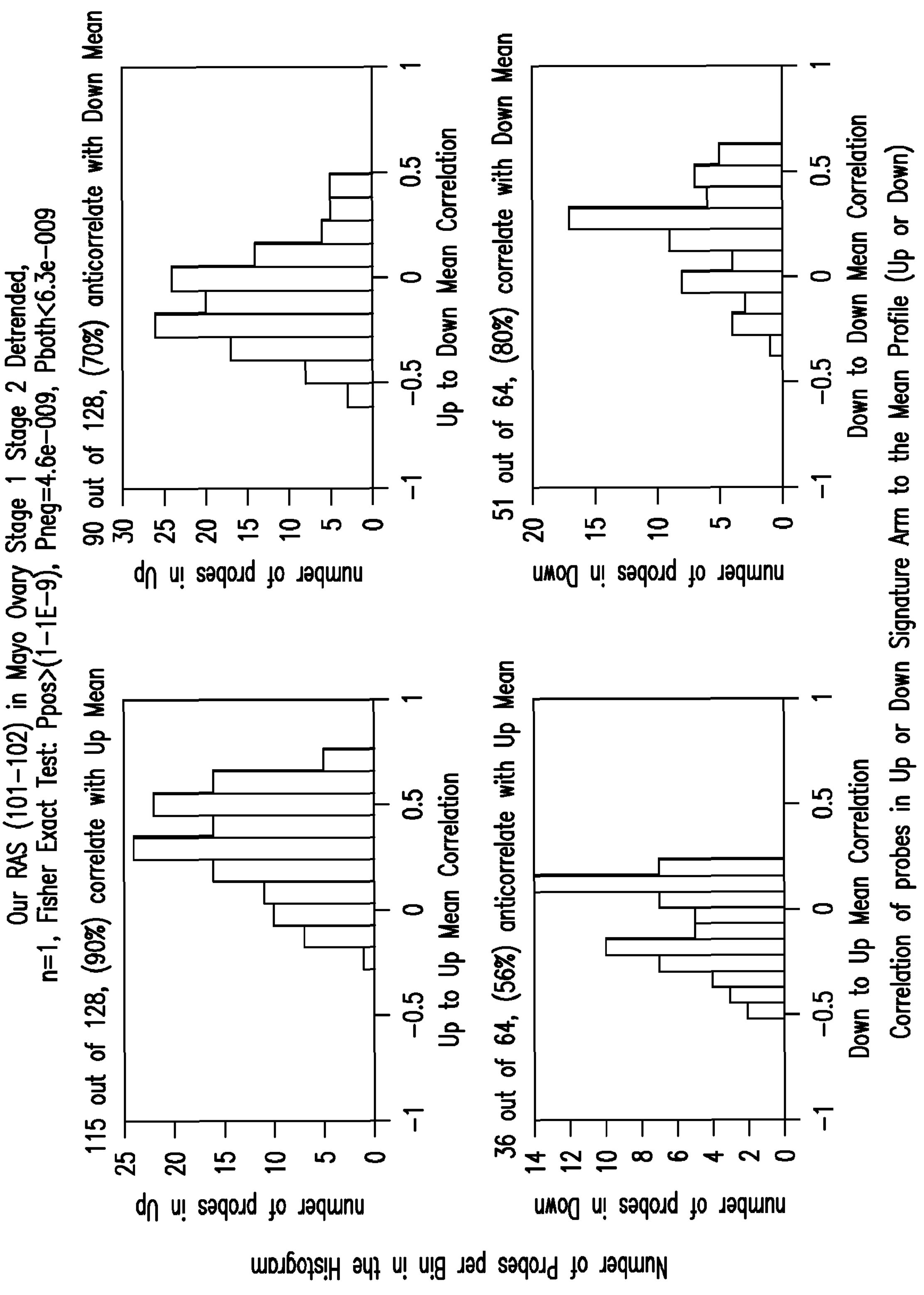
Our RAS (101-102) in Mayo Ovary Stage 1 Stage 2 Detrended,
n=1, Fisher Exact Test: Ppos>(1-1E-9), Pneg=4.6e-009, Pboth<6.3e-009
115 out of 128, (90%) correlate with Up Mean
90 out of 128, (70%) anticorrelate with Down Mean
36 out of 64, (56%) anticorrelate with Up Mean
51 out of 64, (80%) correlate with Down Mean
number of probes in Up
number of probes in Down
Up to Up Mean Correlation
Up to Down Mean Correlation
Down to Up Mean Correlation
Down to Down Mean Correlation
Number of Probes per Bin in the Histogram
Correlation of probes in Up or Down Signature Arm to the Mean Profile (Up or Down)

FIG.7C

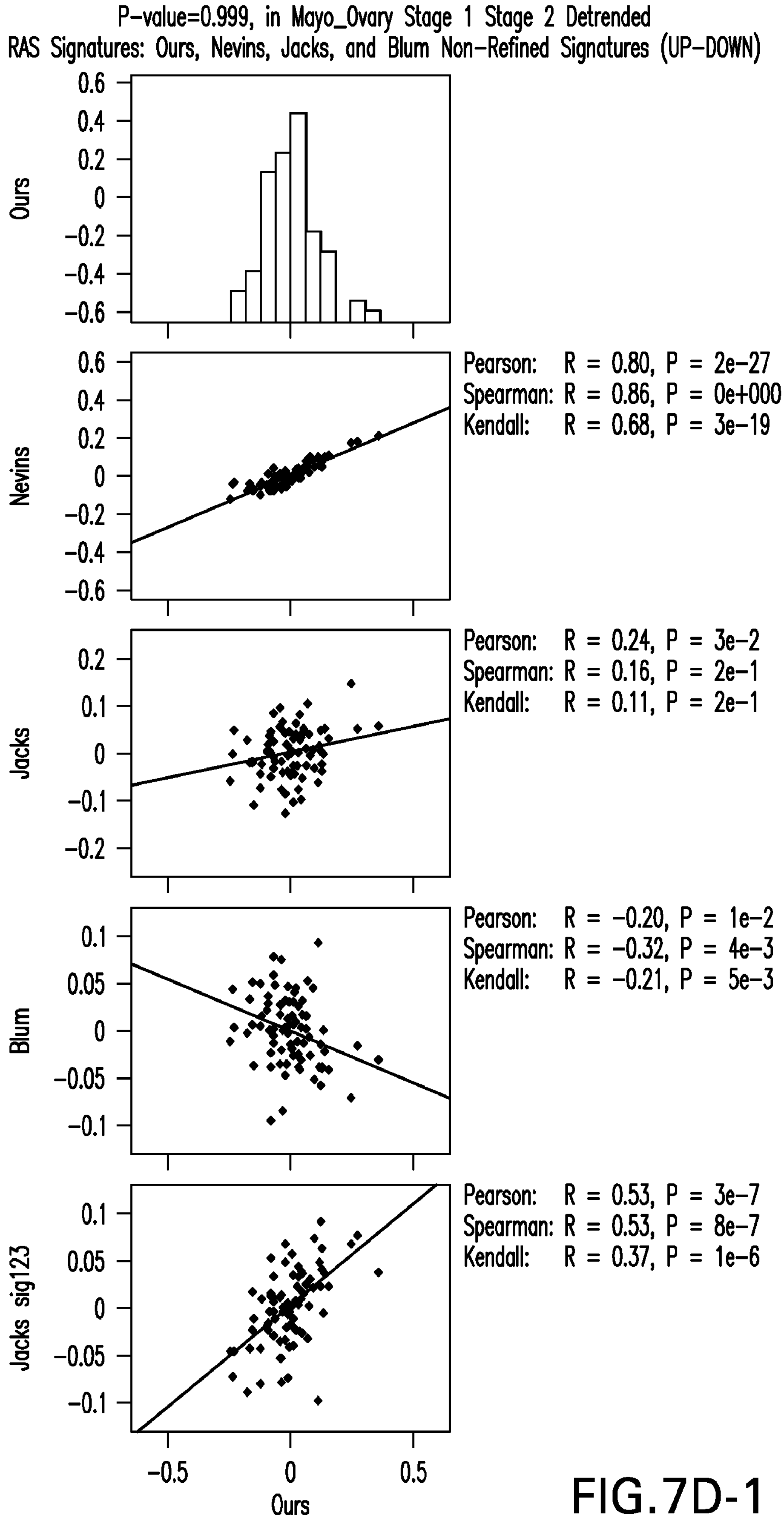
P-value=0.999, in Mayo_Ovary Stage 1 Stage 2 Detrended
RAS Signatures: Ours, Nevins, Jacks, and Blum Non-Refined Signatures (UP-DOWN)
Ours
0.6
0.4
0.2
0
-0.2
-0.4
-0.6
Nevins
Pearson: R = 0.80, P = 2e-27
Spearman: R = 0.86, P = 0e+000
Kendall: R = 0.68, P = 3e-19
Jacks
0.2
0.1
0
-0.1
-0.2
Pearson: R = 0.24, P = 3e-2
Spearman: R = 0.16, P = 2e-1
Kendall: R = 0.11, P = 2e-1
Blum
0.1
0.05
0
-0.05
-0.1
Pearson: R = -0.20, P = 1e-2
Spearman: R = -0.32, P = 4e-3
Kendall: R = -0.21, P = 5e-3
Jacks sig123
Pearson: R = 0.53, P = 3e-7
Spearman: R = 0.53, P = 8e-7
Kendall: R = 0.37, P = 1e-6
-0.5
0
0.5
Ours

FIG.7D-1

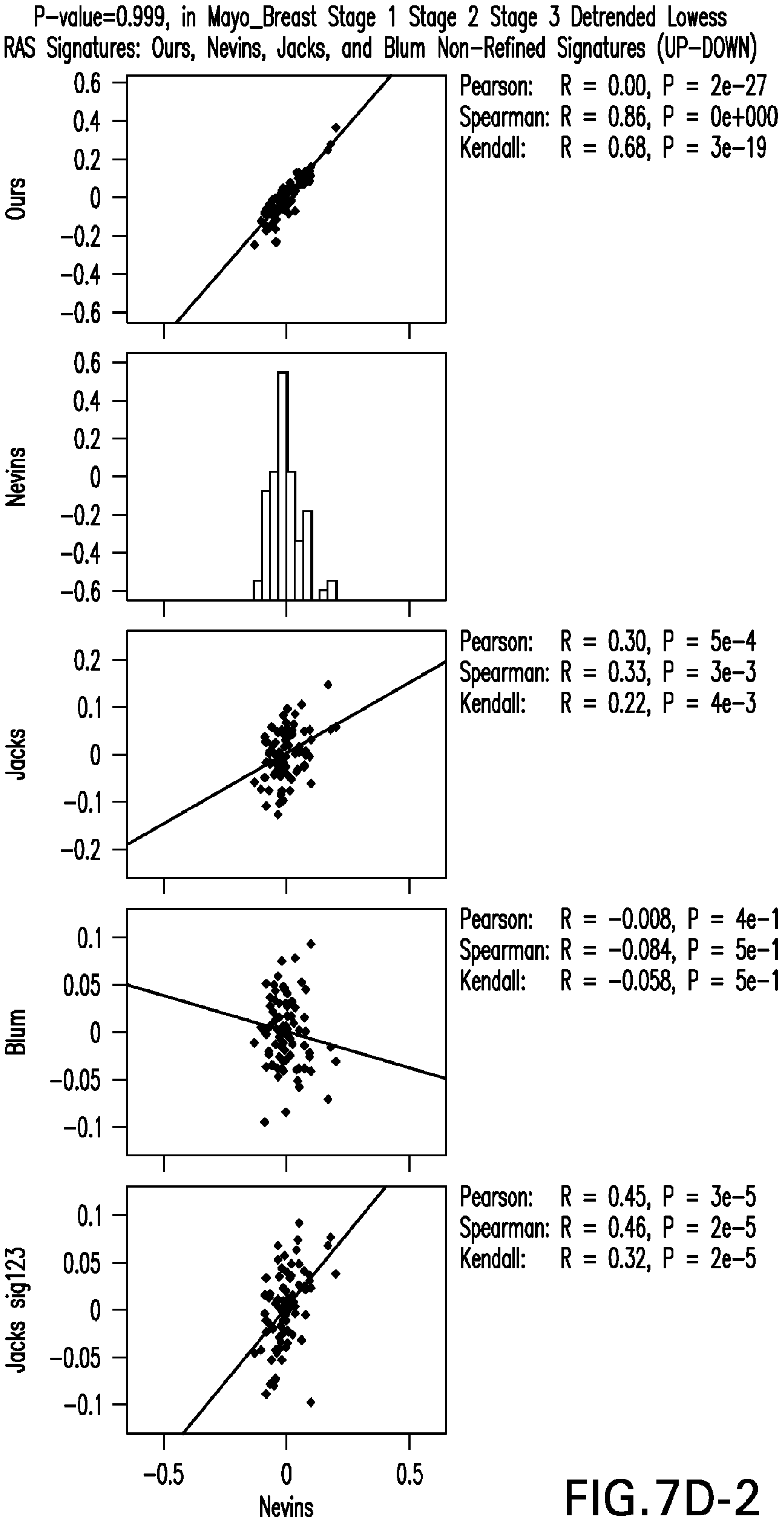
P−value=0.999, in Mayo_Breast Stage 1 Stage 2 Stage 3 Detrended Lowess
RAS Signatures: Ours, Nevins, Jacks, and Blum Non−Refined Signatures (UP−DOWN)
Ours
Pearson: R = 0.00, P = 2e−27
Spearman: R = 0.86, P = 0e+000
Kendall: R = 0.68, P = 3e−19
Nevins
Jacks
Pearson: R = 0.30, P = 5e−4
Spearman: R = 0.33, P = 3e−3
Kendall: R = 0.22, P = 4e−3
Blum
Pearson: R = −0.008, P = 4e−1
Spearman: R = −0.084, P = 5e−1
Kendall: R = −0.058, P = 5e−1
Jacks sig123
Pearson: R = 0.45, P = 3e−5
Spearman: R = 0.46, P = 2e−5
Kendall: R = 0.32, P = 2e−5
Nevins

FIG.7D-2

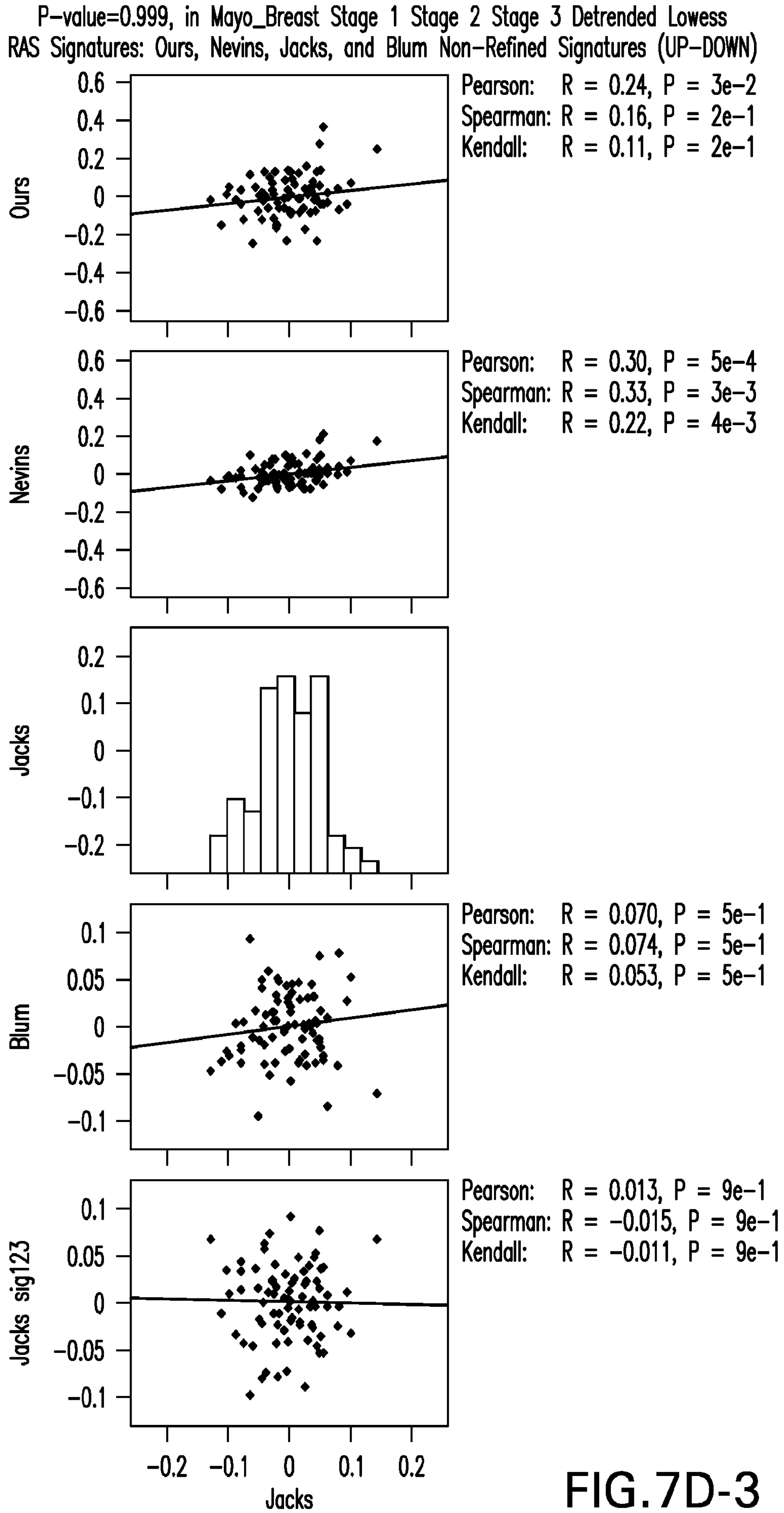
P−value=0.999, in Mayo_Breast Stage 1 Stage 2 Stage 3 Detrended Lowess
RAS Signatures: Ours, Nevins, Jacks, and Blum Non−Refined Signatures (UP−DOWN)
Pearson: R = 0.24, P = 3e−2
Spearman: R = 0.16, P = 2e−1
Kendall: R = 0.11, P = 2e−1
Ours
Pearson: R = 0.30, P = 5e−4
Spearman: R = 0.33, P = 3e−3
Kendall: R = 0.22, P = 4e−3
Nevins
Jacks
Pearson: R = 0.070, P = 5e−1
Spearman: R = 0.074, P = 5e−1
Kendall: R = 0.053, P = 5e−1
Blum
Pearson: R = 0.013, P = 9e−1
Spearman: R = −0.015, P = 9e−1
Kendall: R = −0.011, P = 9e−1
Jacks sig123
−0.2 −0.1 0 0.1 0.2
Jacks

FIG.7D-3

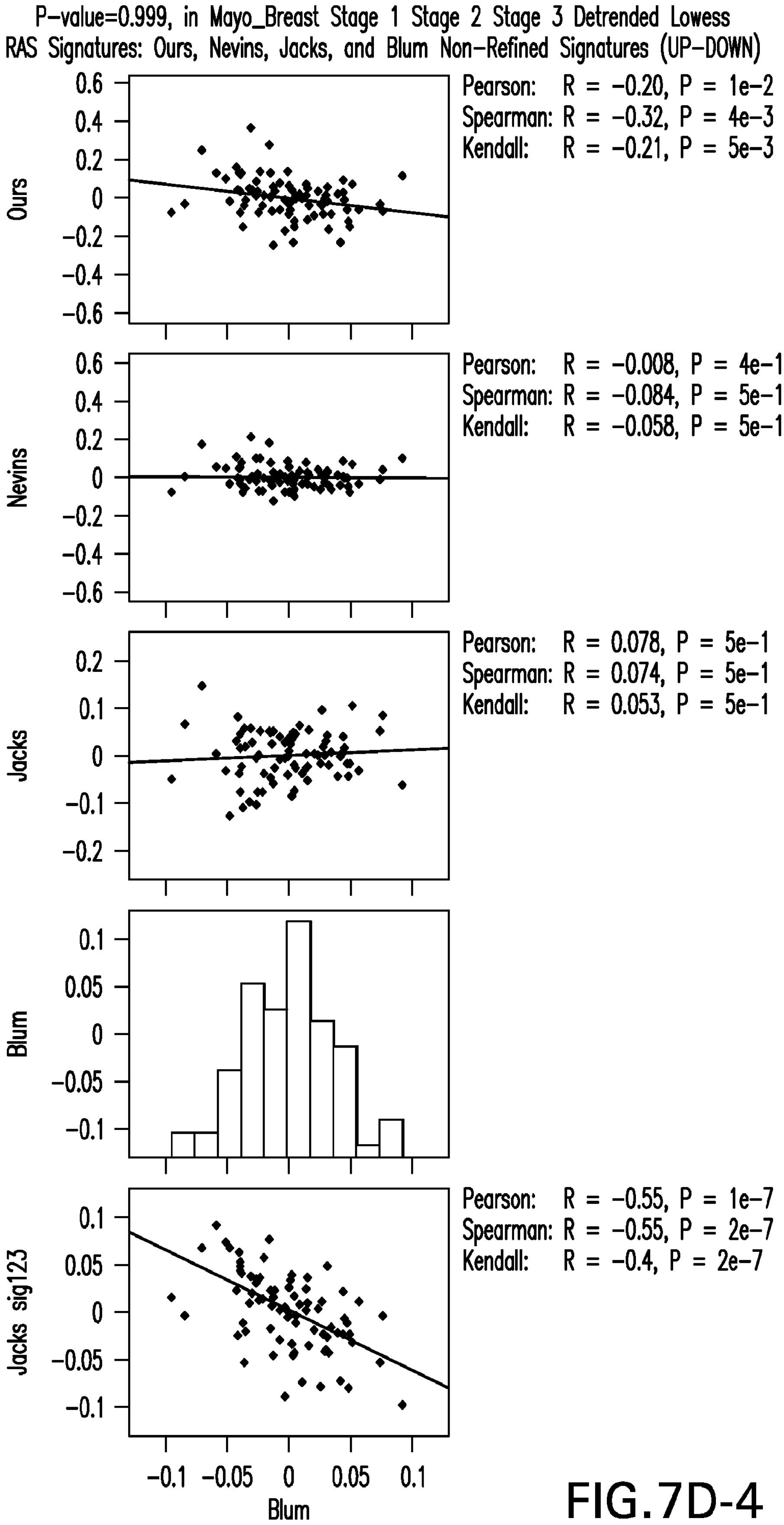
P–value=0.999, in Mayo_Breast Stage 1 Stage 2 Stage 3 Detrended Lowess
RAS Signatures: Ours, Nevins, Jacks, and Blum Non–Refined Signatures (UP–DOWN)
Pearson: R = –0.20, P = 1e–2
Spearman: R = –0.32, P = 4e–3
Kendall: R = –0.21, P = 5e–3
Ours
0.6
0.4
0.2
0
–0.2
–0.4
–0.6
Pearson: R = –0.008, P = 4e–1
Spearman: R = –0.084, P = 5e–1
Kendall: R = –0.058, P = 5e–1
Nevins
Pearson: R = 0.078, P = 5e–1
Spearman: R = 0.074, P = 5e–1
Kendall: R = 0.053, P = 5e–1
Jacks
0.1
–0.1
–0.2
Blum
0.05
–0.05
Pearson: R = –0.55, P = 1e–7
Spearman: R = –0.55, P = 2e–7
Kendall: R = –0.4, P = 2e–7
Jacks sig123
–0.1 –0.05 0 0.05 0.1
Blum

FIG.7D-4

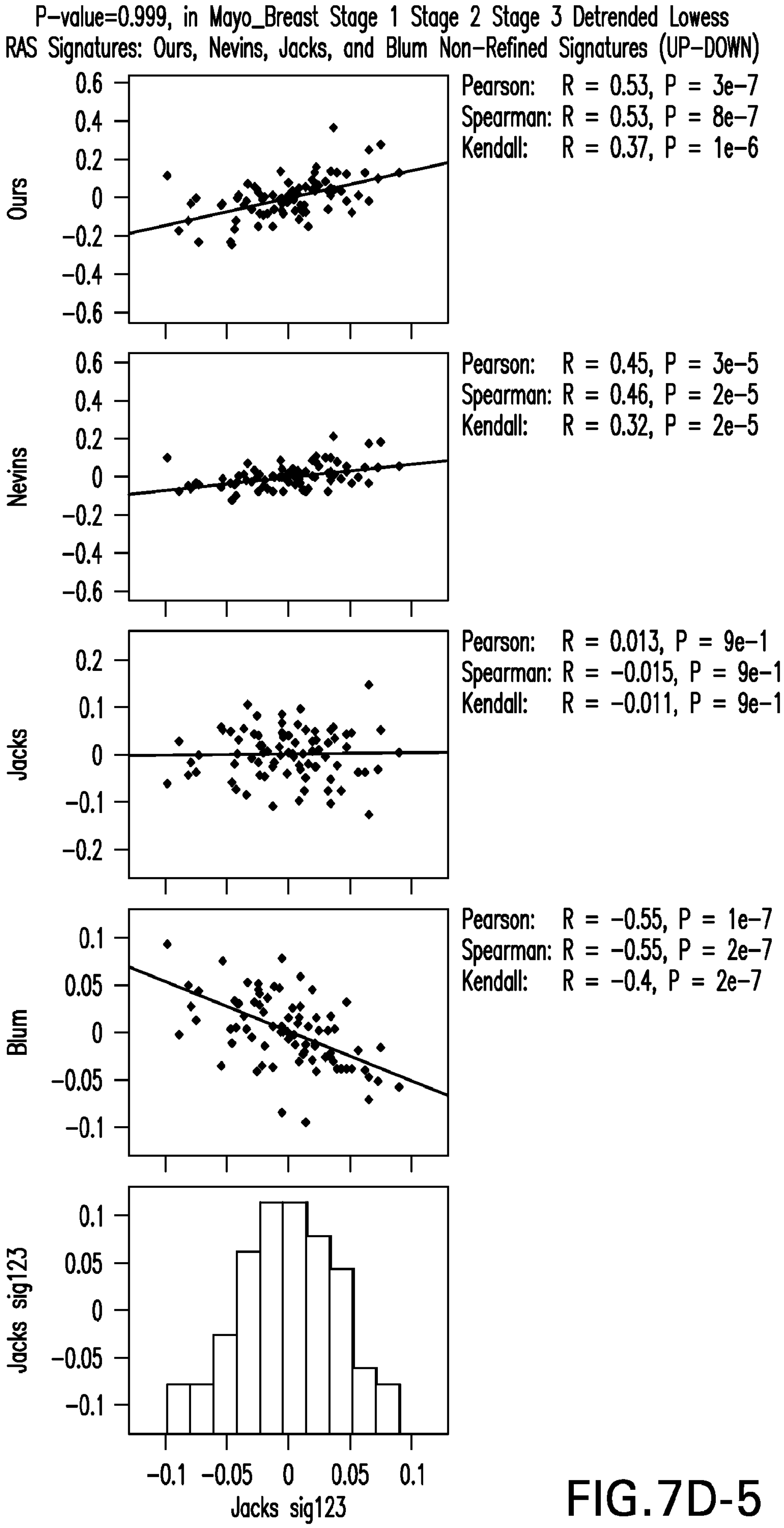
P-value=0.999, in Mayo_Breast Stage 1 Stage 2 Stage 3 Detrended Lowess
RAS Signatures: Ours, Nevins, Jacks, and Blum Non-Refined Signatures (UP-DOWN)
Pearson: R = 0.53, P = 3e-7
Spearman: R = 0.53, P = 8e-7
Kendall: R = 0.37, P = 1e-6
Pearson: R = 0.45, P = 3e-5
Spearman: R = 0.46, P = 2e-5
Kendall: R = 0.32, P = 2e-5
Pearson: R = 0.013, P = 9e-1
Spearman: R = -0.015, P = 9e-1
Kendall: R = -0.011, P = 9e-1
Pearson: R = -0.55, P = 1e-7
Spearman: R = -0.55, P = 2e-7
Kendall: R = -0.4, P = 2e-7
Ours
Nevins
Jacks
Blum
Jacks sig123
0.6
0.4
0.2
0
-0.2
-0.4
-0.6
0.1
0.05
-0.05
-0.1
-0.1 -0.05 0 0.05 0.1
Jacks sig123

FIG.7D-5

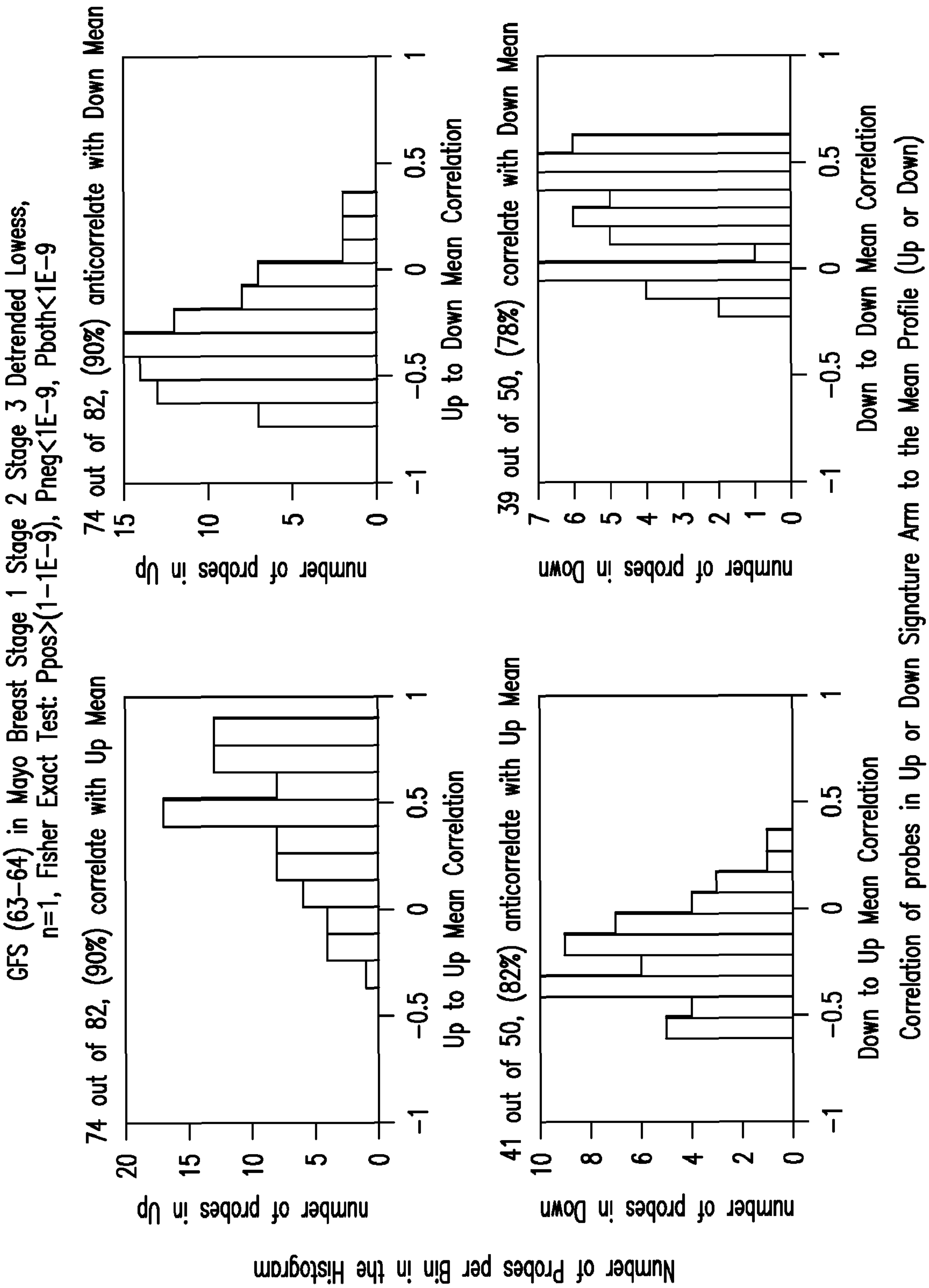
GFS (63–64) in Mayo Breast Stage 1 Stage 2 Stage 3 Detrended Lowess,
n=1, Fisher Exact Test: Ppos>(1−1E−9), Pneg<1E−9, Pboth<1E−9
74 out of 82, (90%) correlate with Up Mean
number of probes in Up
Up to Up Mean Correlation
74 out of 82, (90%) anticorrelate with Down Mean
number of probes in Up
Up to Down Mean Correlation
41 out of 50, (82%) anticorrelate with Up Mean
number of probes in Down
Down to Up Mean Correlation
39 out of 50, (78%) correlate with Down Mean
number of probes in Down
Down to Down Mean Correlation
Number of Probes per Bin in the Histogram
Correlation of probes in Up or Down Signature Arm to the Mean Profile (Up or Down)

FIG.7E

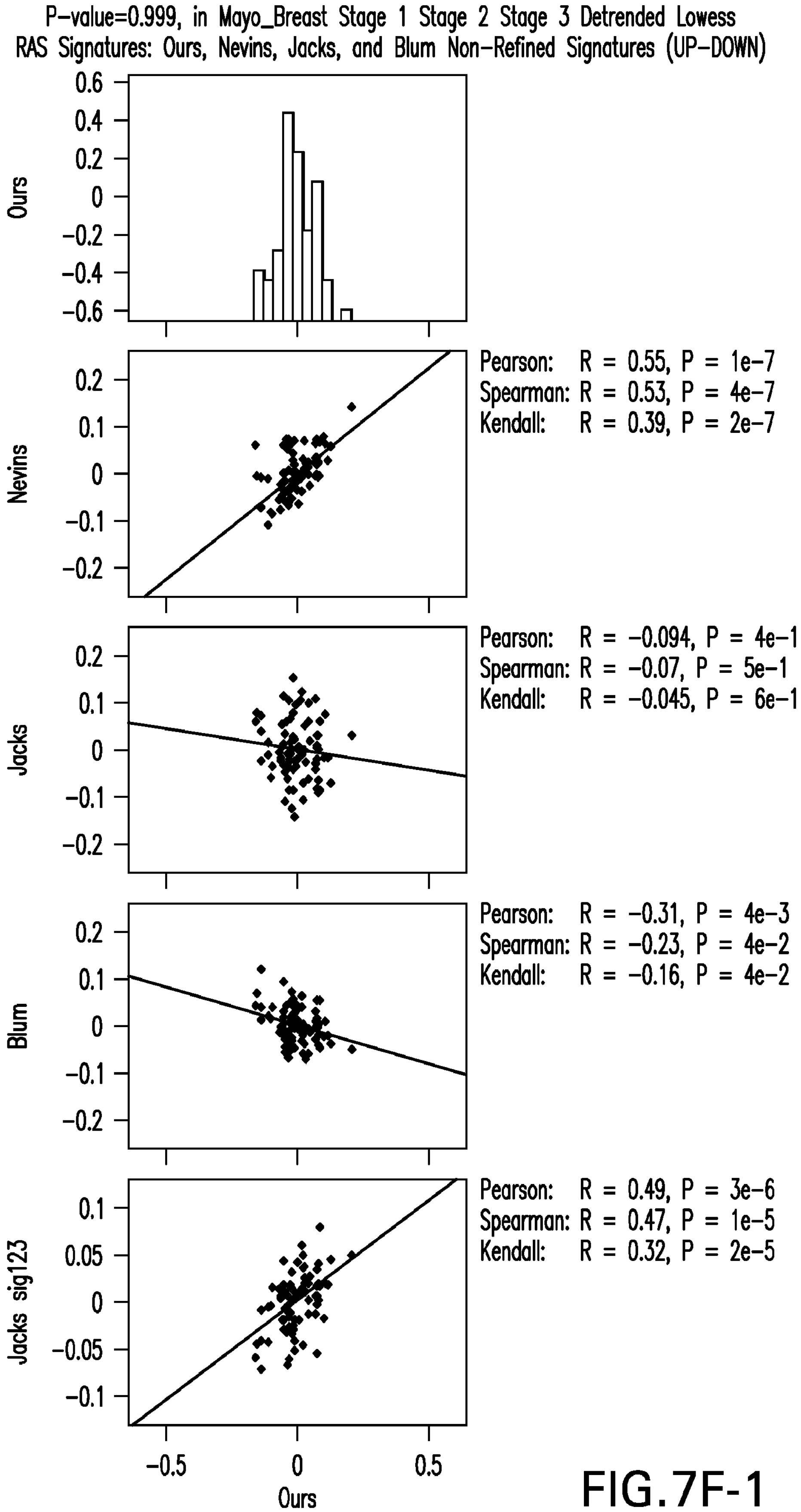
P−value=0.999, in Mayo_Breast Stage 1 Stage 2 Stage 3 Detrended Lowess
RAS Signatures: Ours, Nevins, Jacks, and Blum Non−Refined Signatures (UP−DOWN)
Ours
Nevins
Pearson: R = 0.55, P = 1e−7
Spearman: R = 0.53, P = 4e−7
Kendall: R = 0.39, P = 2e−7
Jacks
Pearson: R = −0.094, P = 4e−1
Spearman: R = −0.07, P = 5e−1
Kendall: R = −0.045, P = 6e−1
Blum
Pearson: R = −0.31, P = 4e−3
Spearman: R = −0.23, P = 4e−2
Kendall: R = −0.16, P = 4e−2
Jacks sig123
Pearson: R = 0.49, P = 3e−6
Spearman: R = 0.47, P = 1e−5
Kendall: R = 0.32, P = 2e−5
−0.5
0
0.5
Ours

FIG.7F-1

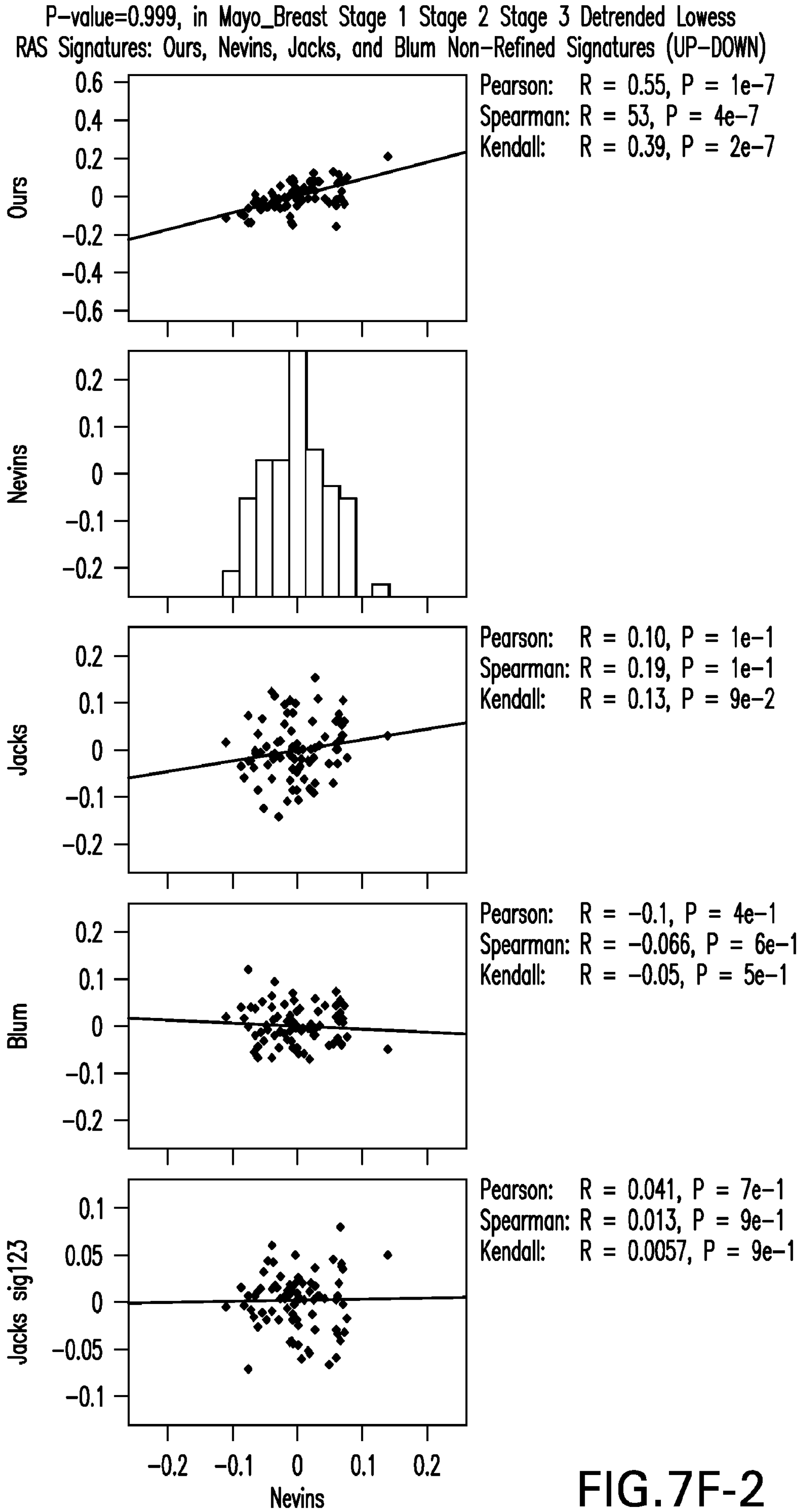

P-value=0.999, in Mayo_Breast Stage 1 Stage 2 Stage 3 Detrended Lowess
RAS Signatures: Ours, Nevins, Jacks, and Blum Non-Refined Signatures (UP-DOWN)
Pearson: R = 0.55, P = 1e-7
Spearman: R = 53, P = 4e-7
Kendall: R = 0.39, P = 2e-7
Ours
0.6
0.4
0.2
0
-0.2
-0.4
-0.6
Nevins
0.2
0.1
0
-0.1
-0.2
Pearson: R = 0.10, P = 1e-1
Spearman: R = 0.19, P = 1e-1
Kendall: R = 0.13, P = 9e-2
Jacks
Pearson: R = -0.1, P = 4e-1
Spearman: R = -0.066, P = 6e-1
Kendall: R = -0.05, P = 5e-1
Blum
Pearson: R = 0.041, P = 7e-1
Spearman: R = 0.013, P = 9e-1
Kendall: R = 0.0057, P = 9e-1
Jacks sig123
0.1
0.05
0
-0.05
-0.1
-0.2
-0.1
0
0.1
0.2
Nevins


FIG.7F-2

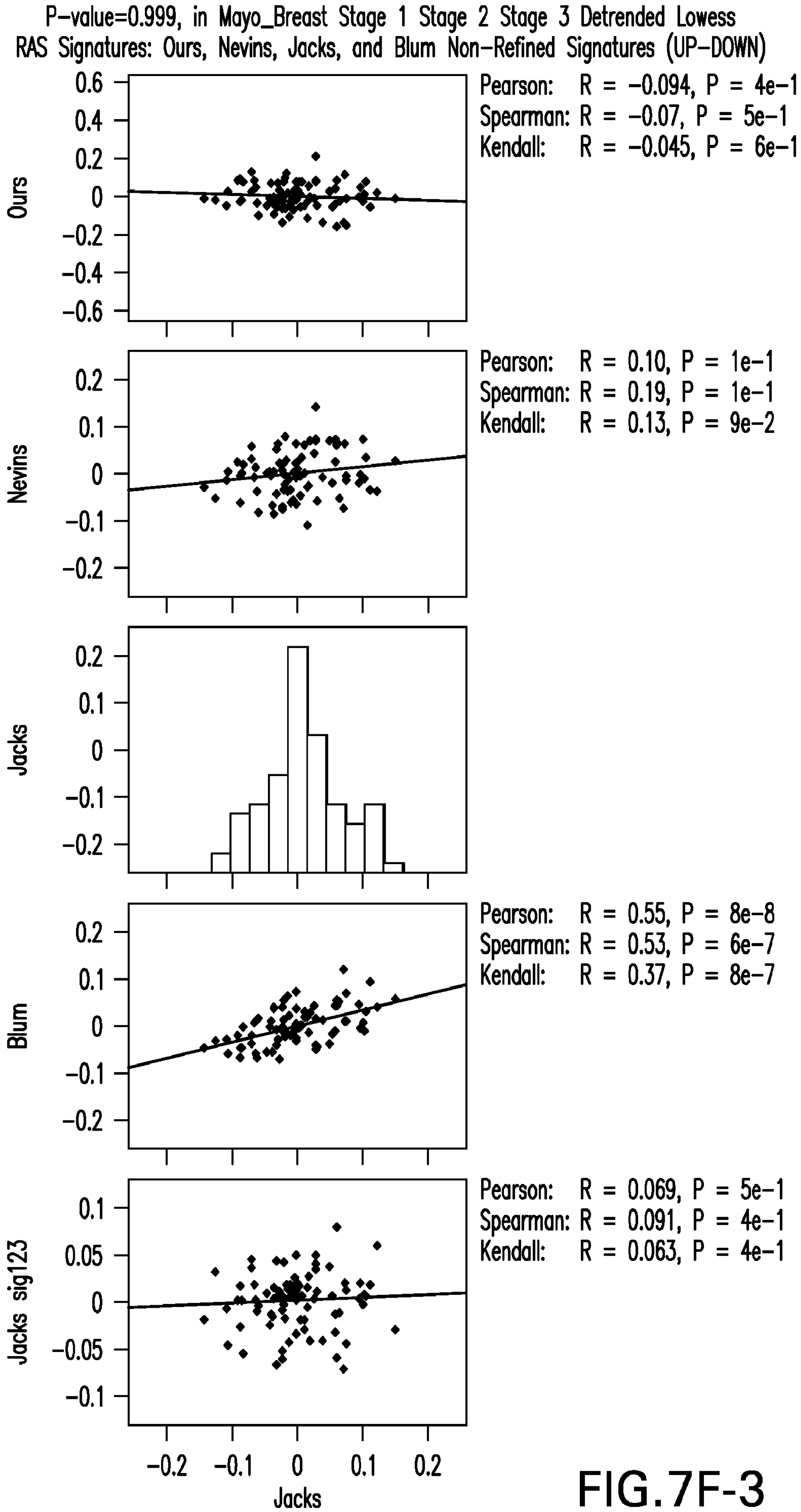
P–value=0.999, in Mayo_Breast Stage 1 Stage 2 Stage 3 Detrended Lowess
RAS Signatures: Ours, Nevins, Jacks, and Blum Non–Refined Signatures (UP–DOWN)
Pearson: R = –0.094, P = 4e–1
Spearman: R = –0.07, P = 5e–1
Kendall: R = –0.045, P = 6e–1
Pearson: R = 0.10, P = 1e–1
Spearman: R = 0.19, P = 1e–1
Kendall: R = 0.13, P = 9e–2
Pearson: R = 0.55, P = 8e–8
Spearman: R = 0.53, P = 6e–7
Kendall: R = 0.37, P = 8e–7
Pearson: R = 0.069, P = 5e–1
Spearman: R = 0.091, P = 4e–1
Kendall: R = 0.063, P = 4e–1
Ours
Nevins
Jacks
Blum
Jacks sig123
Jacks

FIG.7F-3

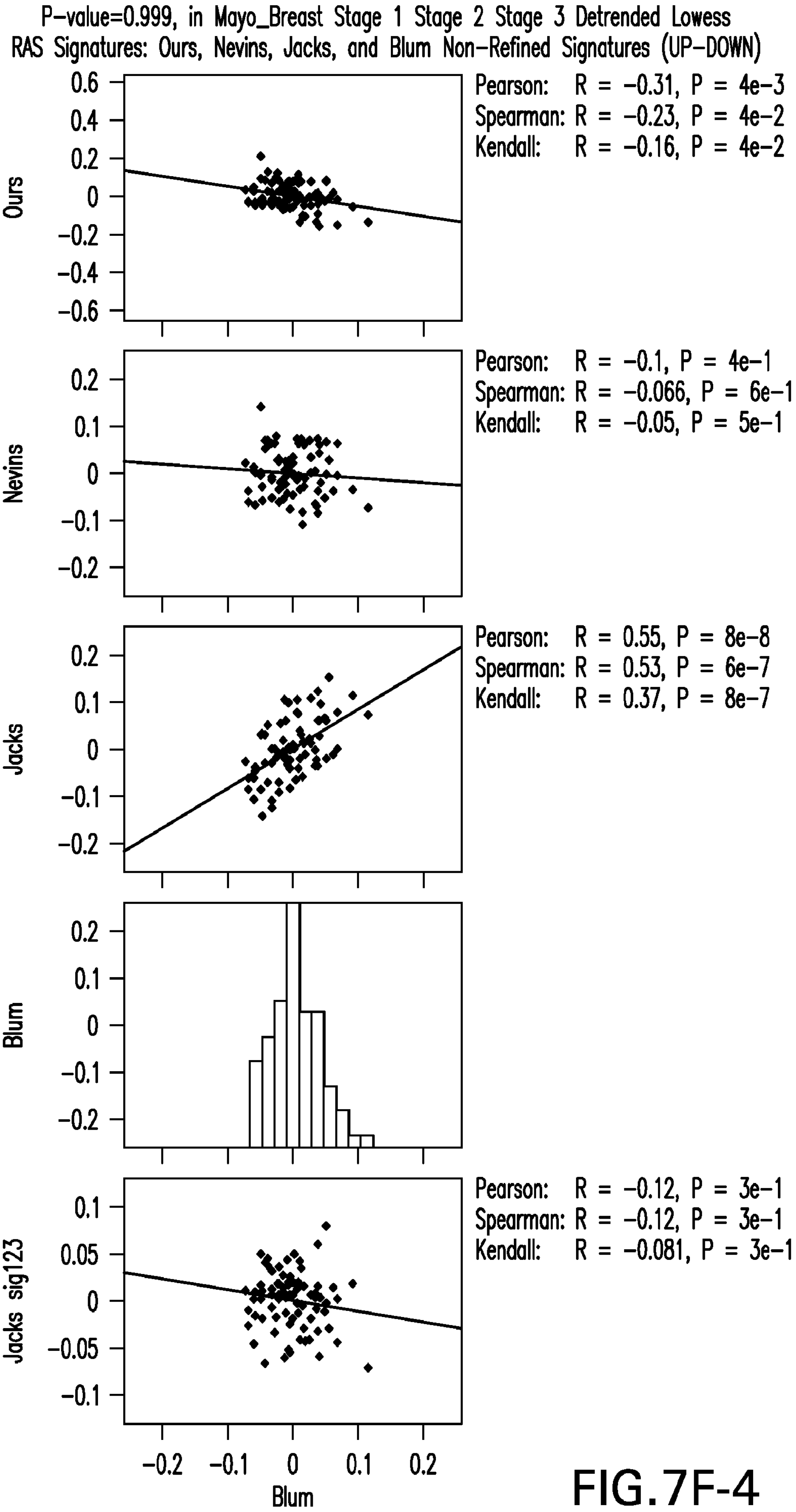
P-value=0.999, in Mayo_Breast Stage 1 Stage 2 Stage 3 Detrended Lowess
RAS Signatures: Ours, Nevins, Jacks, and Blum Non-Refined Signatures (UP-DOWN)
Pearson: R = -0.31, P = 4e-3
Spearman: R = -0.23, P = 4e-2
Kendall: R = -0.16, P = 4e-2
Ours
0.6
0.4
0.2
0
-0.2
-0.4
-0.6
Pearson: R = -0.1, P = 4e-1
Spearman: R = -0.066, P = 6e-1
Kendall: R = -0.05, P = 5e-1
Nevins
0.2
0.1
0
-0.1
-0.2
Pearson: R = 0.55, P = 8e-8
Spearman: R = 0.53, P = 6e-7
Kendall: R = 0.37, P = 8e-7
Jacks
Blum
Pearson: R = -0.12, P = 3e-1
Spearman: R = -0.12, P = 3e-1
Kendall: R = -0.081, P = 3e-1
Jacks sig123
0.1
0.05
0
-0.05
-0.1
-0.2
-0.1
0
0.1
0.2
Blum

FIG.7F-4

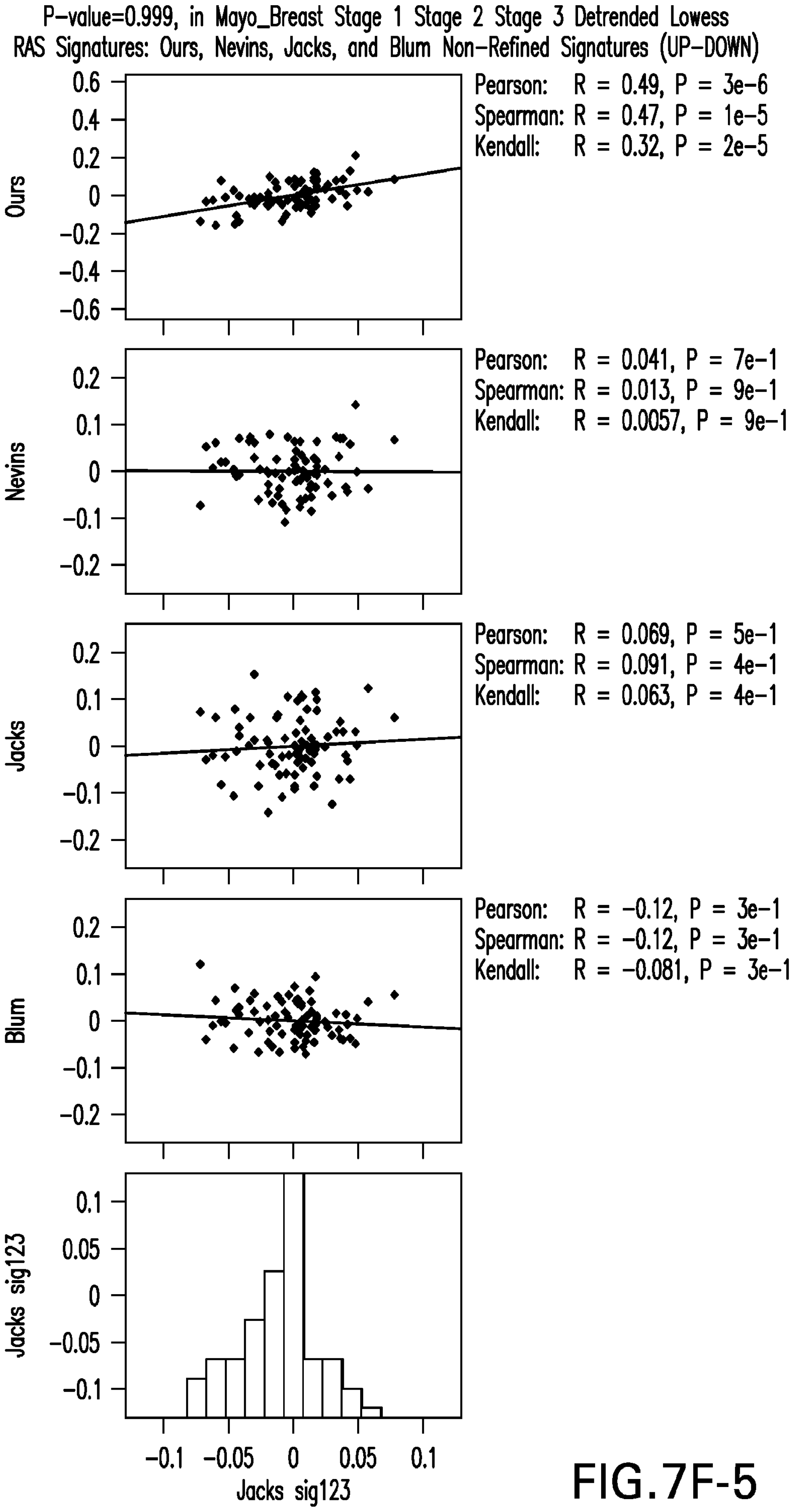
P-value=0.999, in Mayo_Breast Stage 1 Stage 2 Stage 3 Detrended Lowess
RAS Signatures: Ours, Nevins, Jacks, and Blum Non-Refined Signatures (UP-DOWN)
Ours
Pearson: R = 0.49, P = 3e-6
Spearman: R = 0.47, P = 1e-5
Kendall: R = 0.32, P = 2e-5
Nevins
Pearson: R = 0.041, P = 7e-1
Spearman: R = 0.013, P = 9e-1
Kendall: R = 0.0057, P = 9e-1
Jacks
Pearson: R = 0.069, P = 5e-1
Spearman: R = 0.091, P = 4e-1
Kendall: R = 0.063, P = 4e-1
Blum
Pearson: R = -0.12, P = 3e-1
Spearman: R = -0.12, P = 3e-1
Kendall: R = -0.081, P = 3e-1
Jacks sig123
Jacks sig123

FIG.7F-5

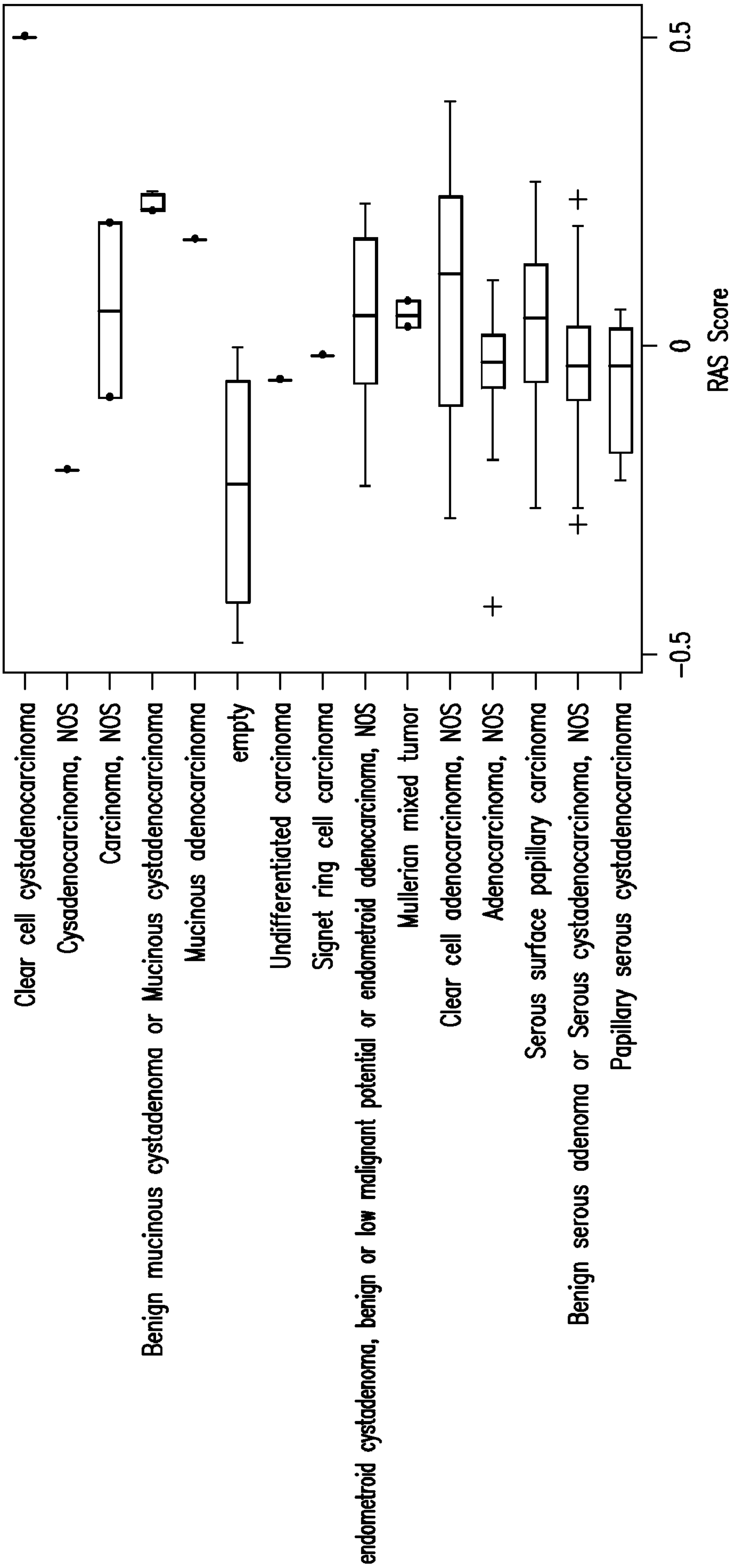
Clear cell cystadenocarcinoma
Cysadenocarcinoma, NOS
Carcinoma, NOS
Benign mucinous cystadenoma or Mucinous cystadenocarcinoma
Mucinous adenocarcinoma
empty
Undifferentiated carcinoma
Signet ring cell carcinoma
endometroid cystadenoma, benign or low malignant potential or endometroid adenocarcinoma, NOS
Mullerian mixed tumor
Clear cell adenocarcinoma, NOS
Adenocarcinoma, NOS
Serous surface papillary carcinoma
Benign serous adenoma or Serous cystadenocarcinoma, NOS
Papillary serous cystadenocarcinoma
−0.5
0
0.5
RAS Score

FIG.8

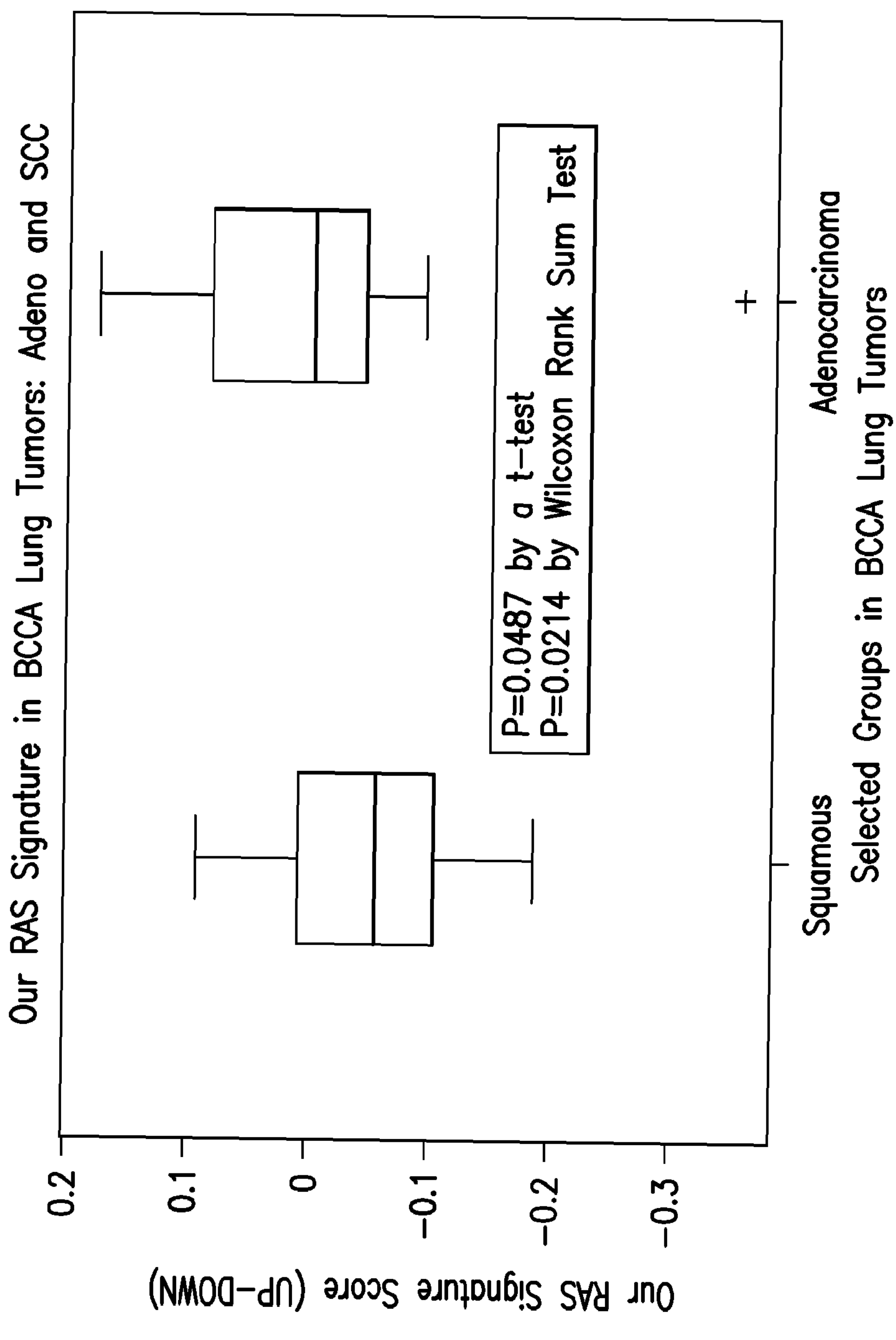
Our RAS Signature in BCCA Lung Tumors: Adeno and SCC
P=0.0487 by a t–test
P=0.0214 by Wilcoxon Rank Sum Test
Our RAS Signature Score (UP–DOWN)
0.2
0.1
0
–0.1
–0.2
–0.3
Squamous
Adenocarcinoma
Selected Groups in BCCA Lung Tumors

FIG.9

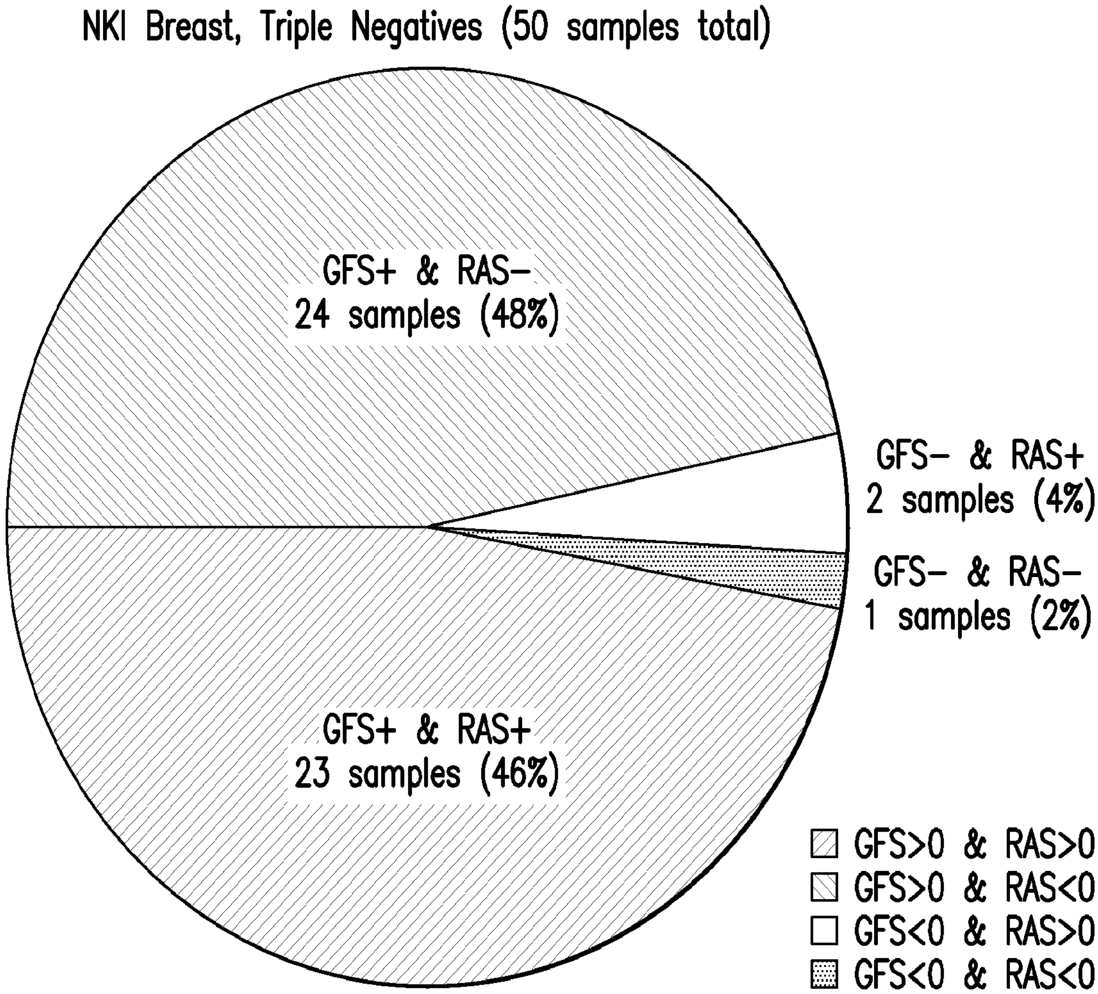
NKI Breast, Triple Negatives (50 samples total)
GFS+ & RAS-
24 samples (48%)
GFS- & RAS+
2 samples (4%)
GFS- & RAS-
1 samples (2%)
GFS+ & RAS+
23 samples (46%)
GFS>0 & RAS>0
GFS>0 & RAS<0
GFS<0 & RAS>0
GFS<0 & RAS<0

FIG.10

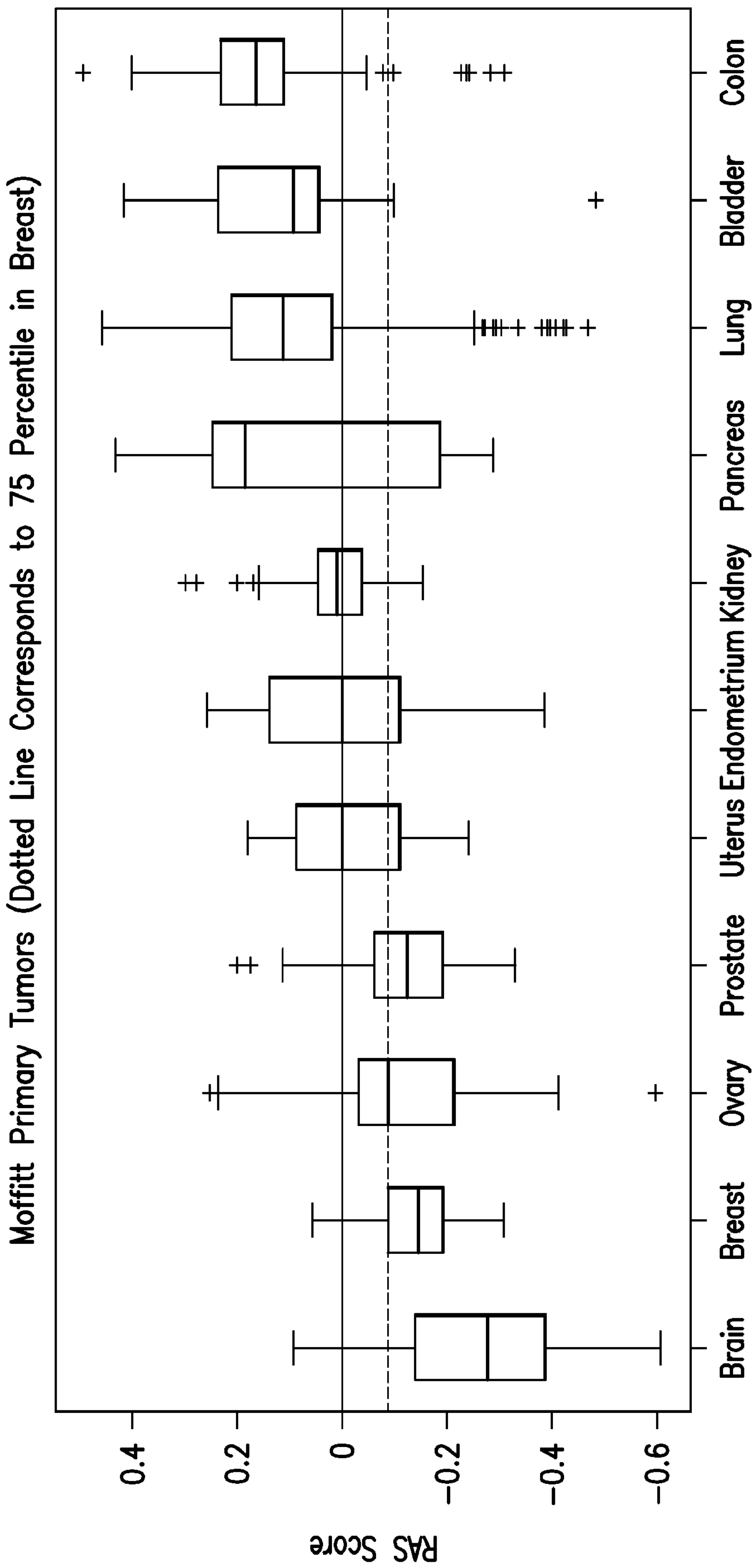
Moffitt Primary Tumors (Dotted Line Corresponds to 75 Percentile in Breast)
RAS Score
0.4
0.2
0
−0.2
−0.4
−0.6
Brain
Breast
Ovary
Prostate
Uterus
Endometrium
Kidney
Pancreas
Lung
Bladder
Colon

FIG.11

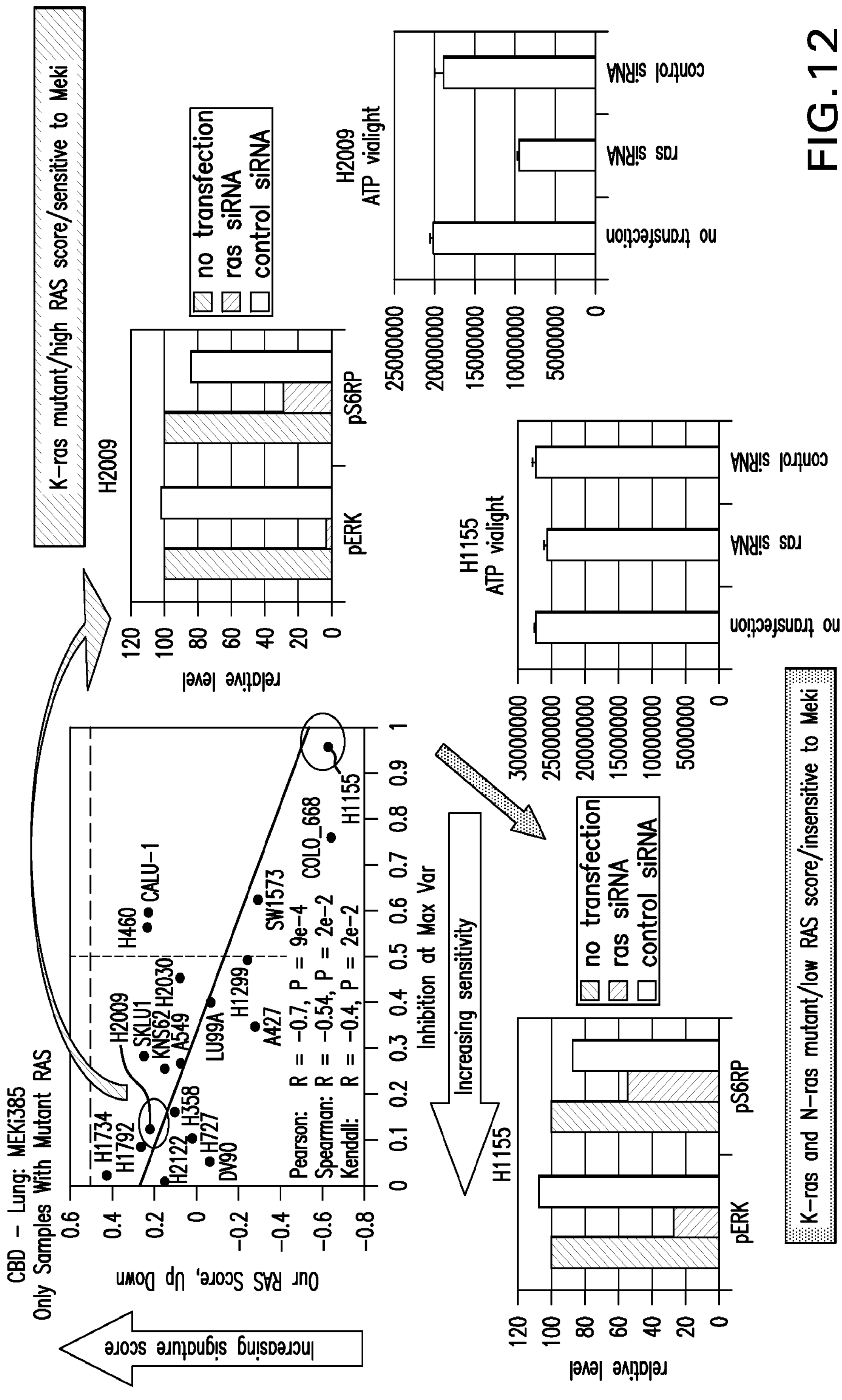

K-ras mutant/high RAS score/sensitive to Meki
CBD - Lung: MEKi385
Only Samples With Mutant RAS
Increasing signature score
Our RAS Score, Up Down
Inhibition at Max Var
Increasing sensitivity
Pearson: R = -0.7, P = 9e-4
Spearman: R = -0.54, P = 2e-2
Kendall: R = -0.4, P = 2e-2
H1734
H1792
H2009
SKLU1
KNS62
H2030
A549
H358
H727
H2122
DV90
LU99A
H1299
A427
H460
CALU-1
SW1573
COLO_668
H1155
H2009
relative level
pERK
pS6RP
no transfection
ras siRNA
control siRNA
H2009
ATP vialight
H1155
ATP vialight
H1155
K-ras and N-ras mutant/low RAS score/insensitive to Meki


FIG.12

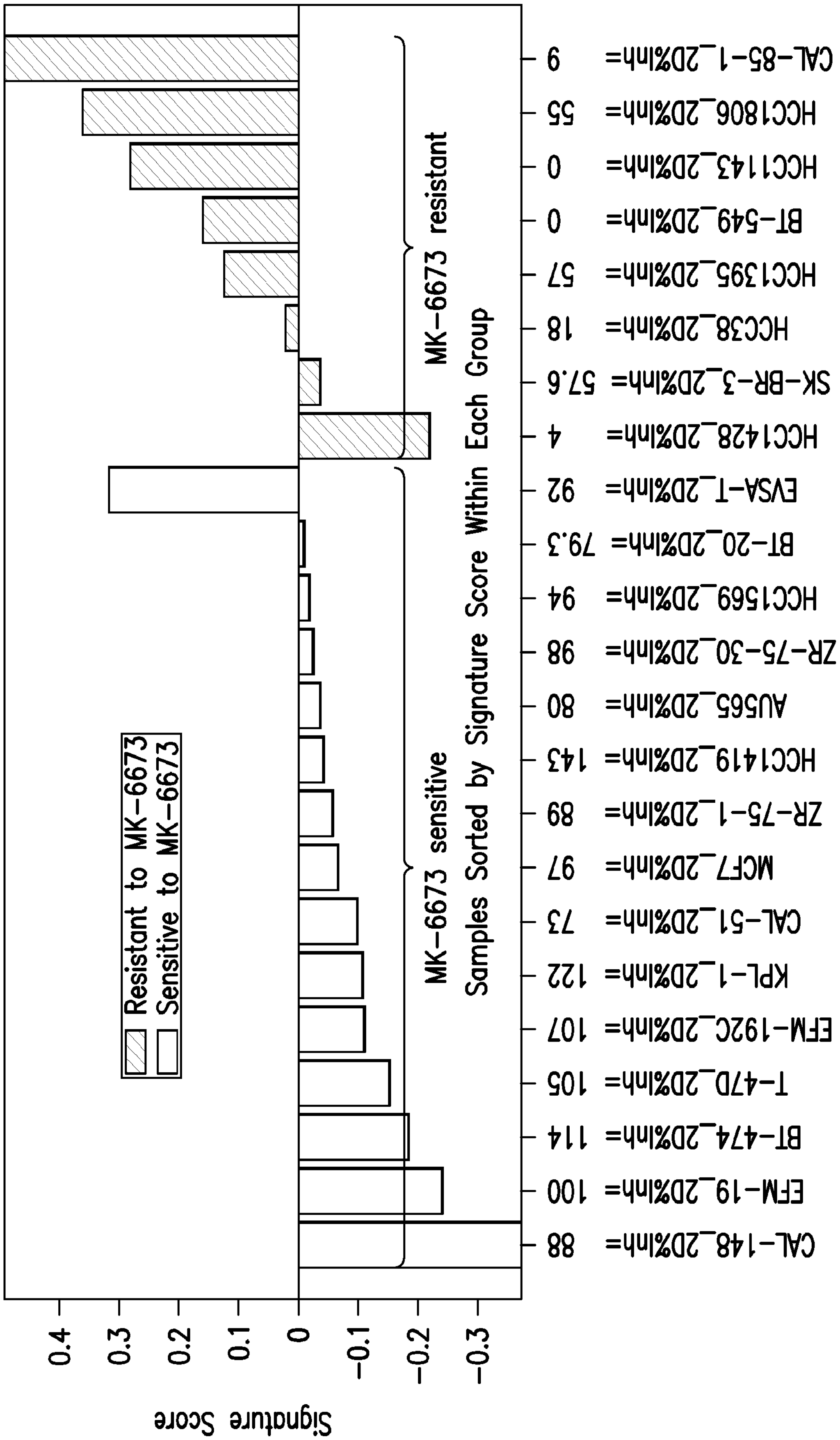
Signature Score
0.4
0.3
0.2
0.1
0
−0.1
−0.2
−0.3
Resistant to MK−6673
Sensitive to MK−6673
MK−6673 resistant
MK−6673 sensitive
Samples Sorted by Signature Score Within Each Group
CAL−85−1_2D%Inh= 9
HCC1806_2D%Inh= 55
HCC1143_2D%Inh= 0
BT−549_2D%Inh= 0
HCC1395_2D%Inh= 57
HCC38_2D%Inh= 18
SK−BR−3_2D%Inh= 57.6
HCC1428_2D%Inh= 4
EVSA−T_2D%Inh= 92
BT−20_2D%Inh= 79.3
HCC1569_2D%Inh= 94
ZR−75−30_2D%Inh= 98
AU565_2D%Inh= 80
HCC1419_2D%Inh= 143
ZR−75−1_2D%Inh= 89
MCF7_2D%Inh= 97
CAL−51_2D%Inh= 73
KPL−1_2D%Inh= 122
EFM−192C_2D%Inh= 107
T−47D_2D%Inh= 105
BT−474_2D%Inh= 114
EFM−19_2D%Inh= 100
CAL−148_2D%Inh= 88

FIG.13

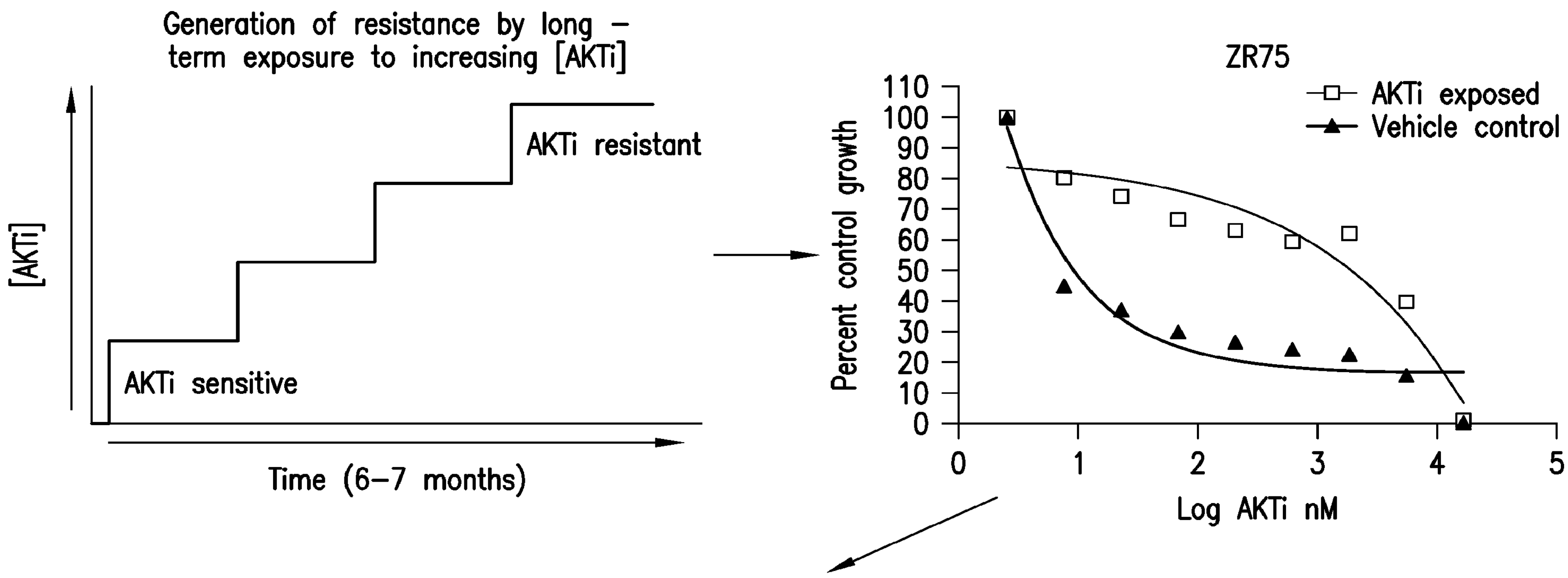


ID mechanisms of resistance:
• expression profiling

*–RAS signature significantly upregulated in both cell lines*

| AKTi Acquired Resistance Data | CAMA–1 | ZR–75 | CAMA–1 | ZR–15 |
|---|---|---|---|---|
| | K–S test | K–S test | Fold Change | Fold Change |
| | | | | |
| RAS signature | 2.E–09 | 2.E–07 | 1.4 | 1.4 |

FIG.14

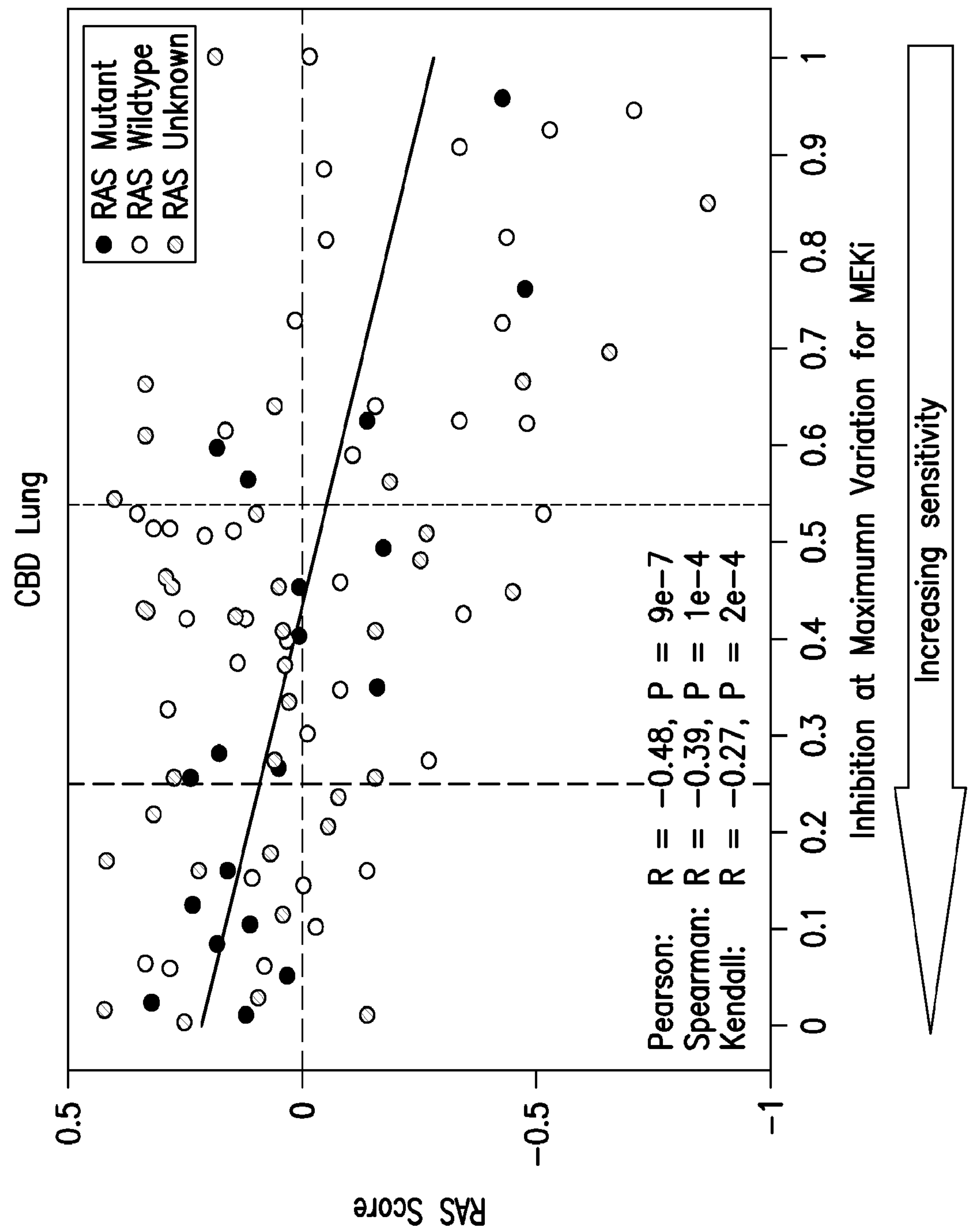
CBD Lung
RAS Mutant
RAS Wildtype
RAS Unknown
Pearson: R = −0.48, P = 9e−7
Spearman: R = −0.39, P = 1e−4
Kendall: R = −0.27, P = 2e−4
RAS Score
0.5
0
−0.5
−1
0
0.1
0.2
0.3
0.4
0.5
0.6
0.7
0.8
0.9
1
Inhibition at Maximumn Variation for MEKi
Increasing sensitivity

FIG.15

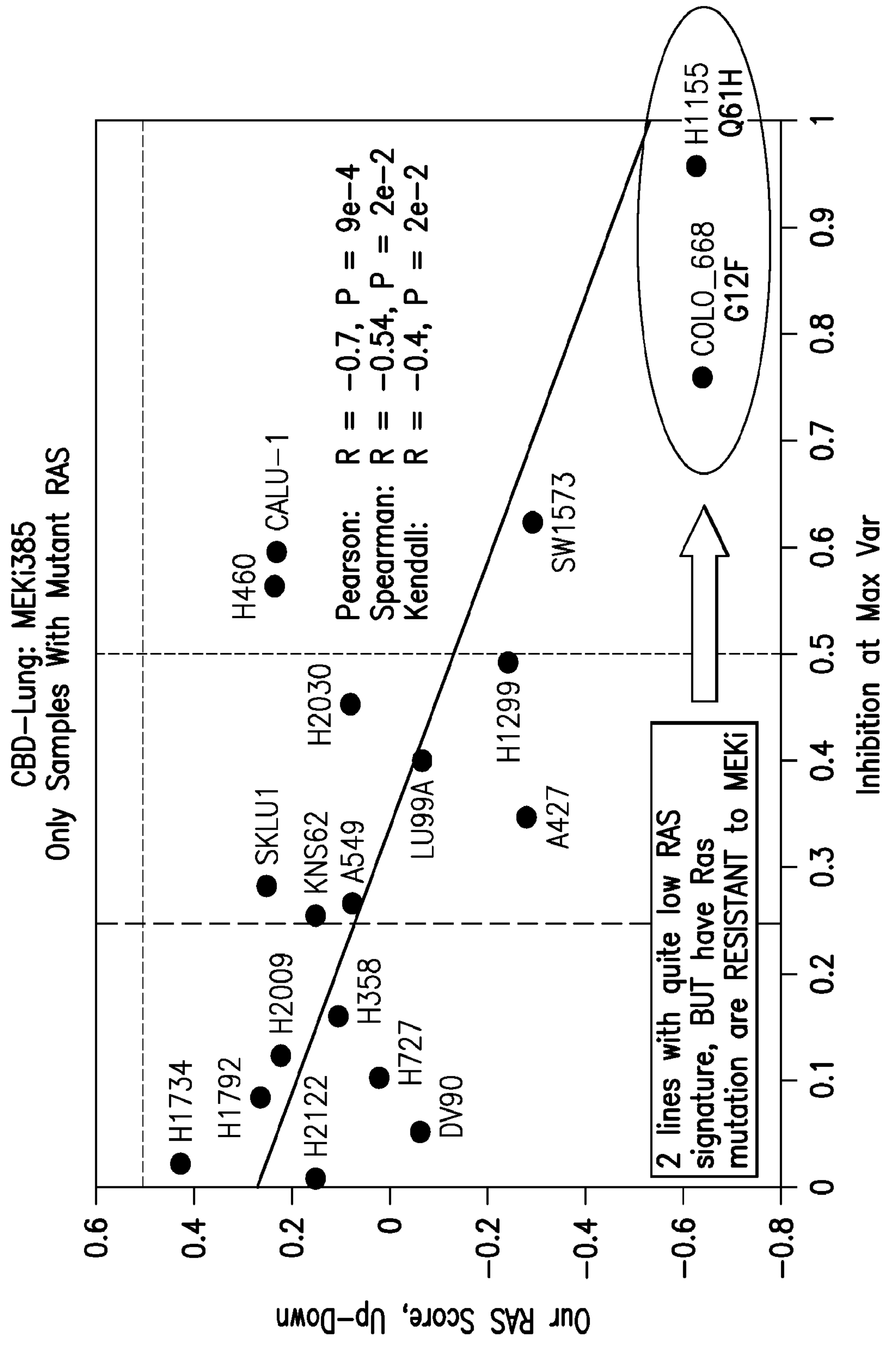
CBD-Lung: MEKi385
Only Samples With Mutant RAS
H1734
H1792
H2122
H2009
H727
DV90
H358
SKLU1
KNS62
A549
LU99A
H2030
A427
H1299
H460
CALU-1
SW1573
Pearson: R = -0.7, P = 9e-4
Spearman: R = -0.54, P = 2e-2
Kendall: R = -0.4, P = 2e-2
2 lines with quite low RAS signature, BUT have Ras mutation are RESISTANT to MEKi
COLO_668
G12F
H1155
Q61H
0
0.1
0.2
0.3
0.4
0.5
0.6
0.7
0.8
0.9
1
Inhibition at Max Var
0.6
0.4
0.2
0
-0.2
-0.4
-0.6
-0.8
Our RAS Score, Up-Down

FIG.16

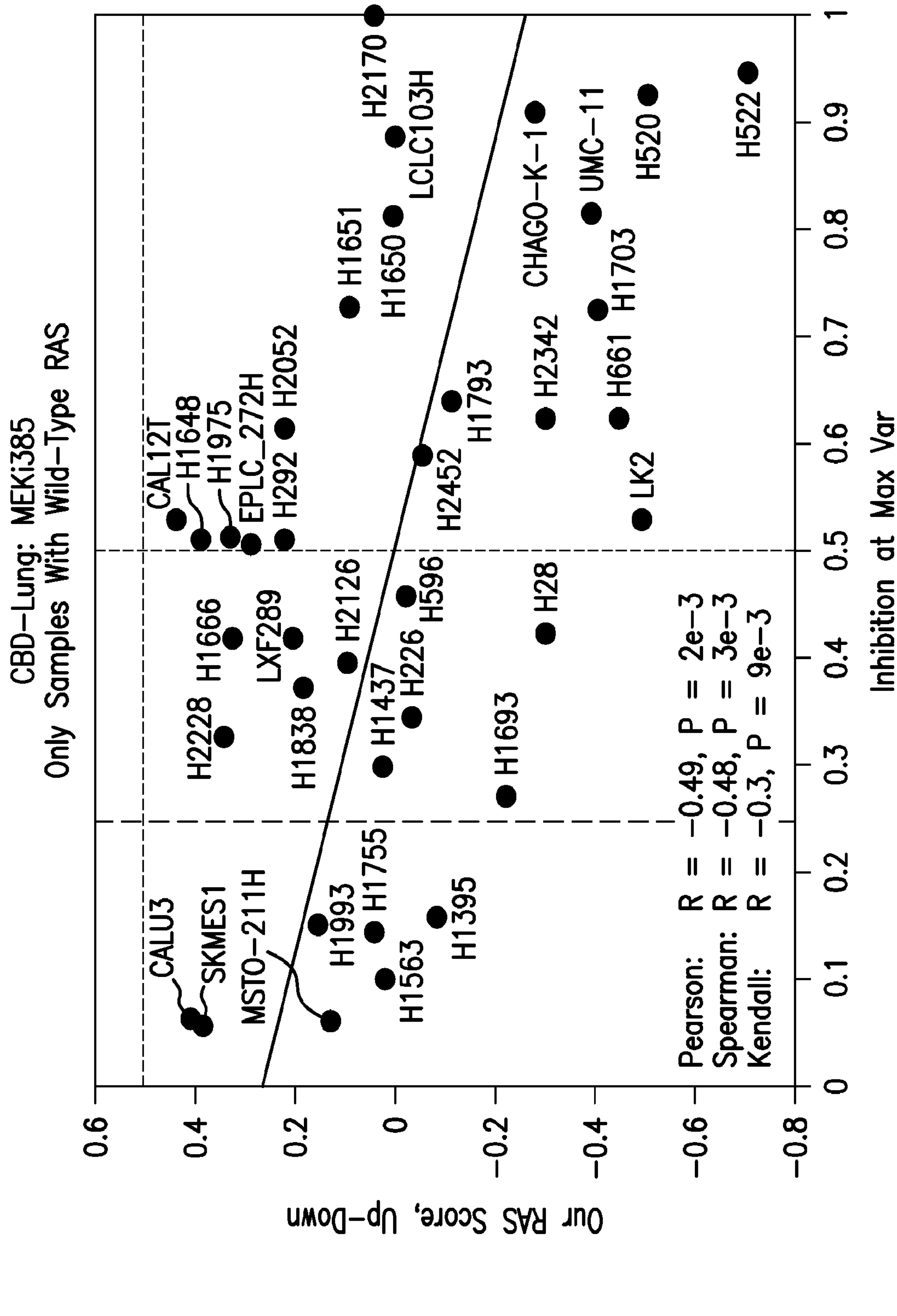
CBD-Lung: MEKi385
Only Samples With Wild-Type RAS
Our RAS Score, Up-Down
Inhibition at Max Var
0.6
0.4
0.2
0
-0.2
-0.4
-0.6
-0.8
0
0.1
0.2
0.3
0.4
0.5
0.6
0.7
0.8
0.9
1
CALU3
SKMES1
MSTO-211H
H1993
H1755
H1563
H1395
H2228
H1666
H1838
LXF289
H1437
H2126
H226
H1693
H596
H28
CAL12T
H1648
H1975
EPLC_272H
H292
H2052
H2452
H1793
H2342
LK2
H661
H1703
H1651
H1650
CHAGO-K-1
UMC-11
LCLC103H
H2170
H520
H522
Pearson: R = -0.49, P = 2e-3
Spearman: R = -0.48, P = 3e-3
Kendall: R = -0.3, P = 9e-3

FIG.17

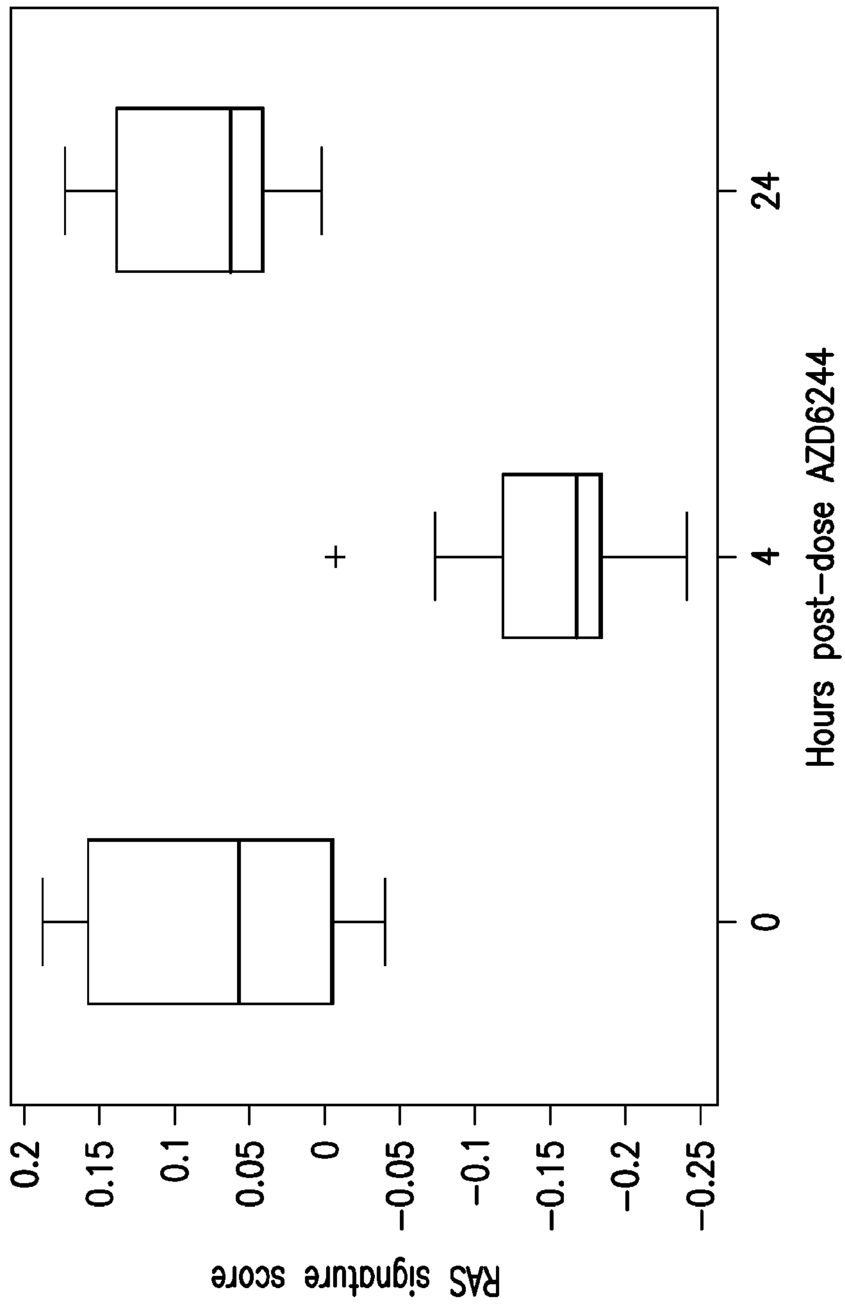
RAS signature score
0.2
0.15
0.1
0.05
0
-0.05
-0.1
-0.15
-0.2
-0.25
0
4
24
Hours post-dose AZD6244

FIG.18

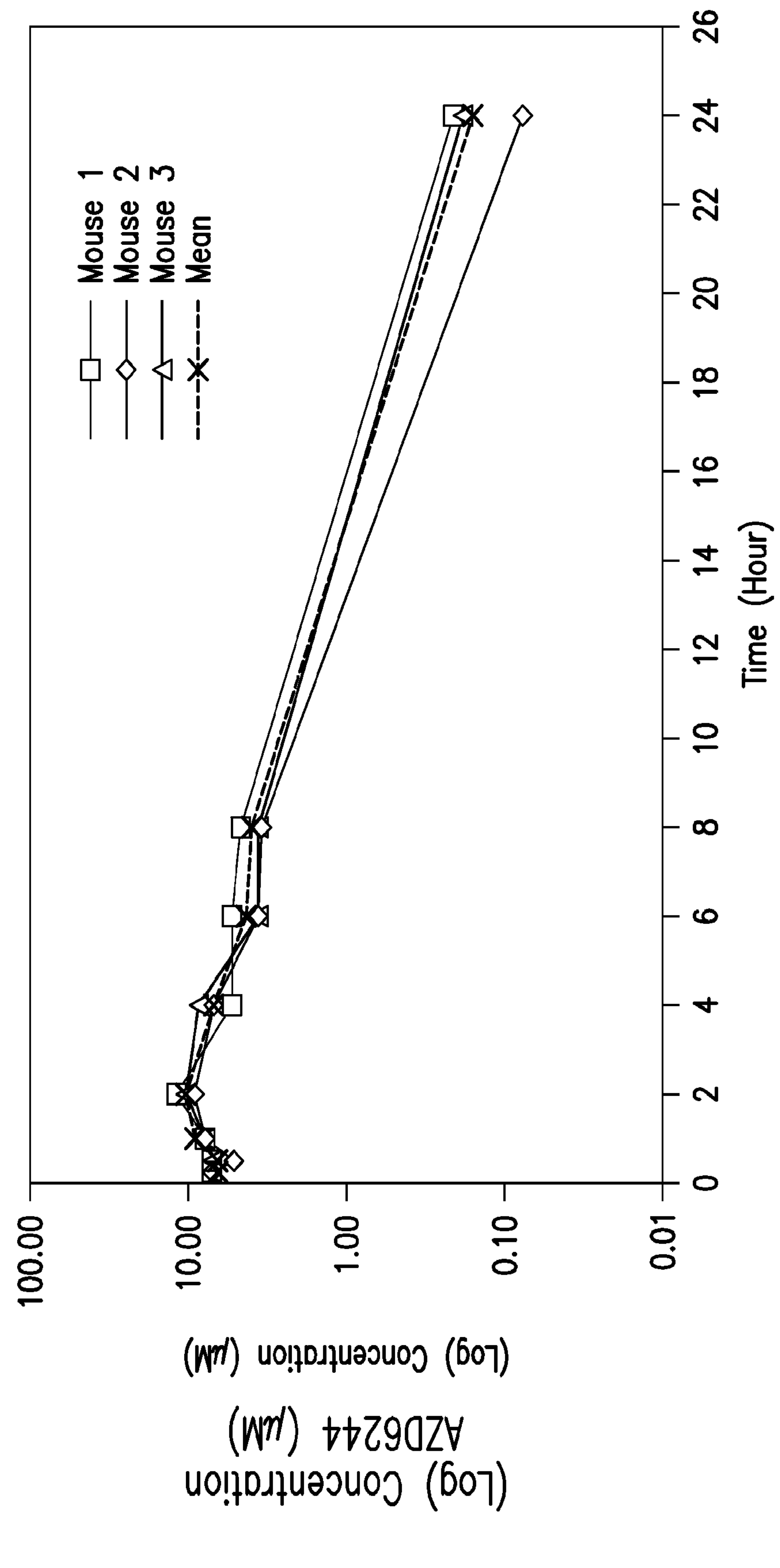
Mouse 1
Mouse 2
Mouse 3
Mean
Time (Hour)
0
2
4
6
8
10
12
14
16
18
20
22
24
26
100.00
10.00
1.00
0.10
0.01
(Log) Concentration (µM)
(Log) Concentration
AZD6244 (µM)

FIG. 19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5569588 A
- WO 0181346 A
- WO 01372557 A
- US 6403588 B
- WO 0143266 A
- US 7241444 B
- WO 2006065601 A
- US 7138400 B
- WO 0236555 A
- US 7122548 B
- WO 0039339 A
- WO 2004065545 PCT
- US 7171311 B
- WO 2002103320 A
- WO 2005086891 A
- WO 2006015312 A
- WO 2006084272 A
- US 20030104426 A, Similarly, Linsley
- US 20070154931 A, Radish
- WO 2007076128 A
- WO 2007076129 A
- WO 0218646 A, Altschuler
- WO 0216650 A, Scherer
- US 5539083 A
- WO 0105935 A
- US 5578832 A
- US 5556752 A
- US 5510270 A
- US 6028189 A
- US 41107499 A, Linsley & Schelter
- US 5545522 A
- US 5891636 A
- US 5716785 A
- WO 2006135627 A
- WO 2008070016 A


### Non-patent literature cited in the description


- **ROTHENBERG et al.** *Nat. Rev. Cancer,* 2003, vol. 3, 303-309
- **DRACOPOLI.** *Curr. Mol. Med.,* 2005, vol. 5, 103-110
- **FOLGUERIA et al.** *Clin. Cancer Res.,* 2005, vol. 11, 7434-7443
- **CHANG et al.** *Lancet,* 2003, vol. 362, 362-369
- **ROUZIER et al.** *Proc. Natl. Acad. Sci. USA,* 2005, vol. 102, 8315-8320
- **HAHN ; WEINBERG.** *Nat. Rev. Cancer,* 2002, vol. 2, 331-341
- **HANAHAN ; WEINBERG.** *Cell,* 2000, vol. 100, 57-70
- **TROSKO et al.** *Ann. N.Y. Acad. Sci.,* 2004, vol. 1028, 192-201
- **ADJEI ; HILDALGO.** *J. Clin. Oncol.,* 2005, vol. 23, 5386-5403
- **LYNCH et al.** *N.Engl. J. Med.,* 2004, vol. 350, 2129-2139
- **PAEZ et al.** *Science,* 2004, vol. 304, 1497-1500
- **WEINSTEIN.** *Science,* 2002, vol. 297, 63-64
- **JAIN et al.** *Science,* 2002, vol. 297, 102-104
- **GORRE et al.** *Science,* 2001, vol. 293, 876-880
- **DRUKER et al.** *N. Engl. J. Med.,* 2001, vol. 344, 1031-1037
- **DOWNWARD.** *Nature,* 2006, vol. 439, 274-275
- **DOWNWARD.** *Nat. Rev. Cancer,* 2002, vol. 3, 11-22
- **ADJEI.** *J. Nat. Cancer Instit.,* 2001, vol. 93, 1062-1073
- **COX ; DER.** *Curr. Opin. Pharmacol.,* 2002, vol. 2, 388-93
- **BLUM ; KLOOG.** *Drug Resist. Updat.,* 2005, vol. 8, 369-80
- **DANCEY.** *Curr. Pharm. Des.,* 2002, vol. 8, 2259-2267
- **MASSARELLI et al.** *Clin. Cancer Res.,* 2007, vol. 13, 2890-2896
- **RAPONI et al.** *Curr. Opin. Pharmacology,* 2008, vol. 8, 413-418
- **IHLE et al.** *Cancer Res.,* 2009, vol. 69, 143-150
- **WEISS et al.** *Am. J. Med. Genet.,* 1999, vol. 89, 14-22
- **MENDELSOHN ; BASELGA.** *Oncogene,* 2000, vol. 19, 6550-6565
- **BELLACOSA.** *Intl. J. Cancer,* 1995, vol. 64, 280-285
- **SIMPSON ; PARSONS.** *Exp. Cell Res.,* 2001, vol. 264, 29-41
- **BOS.** *Cancer Res.,* 1989, vol. 49, 4682-4689

- **HENSON ; GIBSON.** *Cellular Signalling,* 2006, vol. 18, 2089-2097
- **HENNESSY et al.** *Nat. Rev. Drug Disc.,* 2005, vol. 4, 988-1004
- **SEBOLT-LEOPOLD et al.** *Nat. Med,* 1999, vol. 5, 810-816
- **DOWNWARD.** *Nat. Rev. Cancer,* 2003, vol. 3, 11-22
- **HENSON ; GIBSON.** *Cellular Signaling,* 2006, vol. 18, 2089-2097
- **JAMES WATTERS et al.** *Methods and Gene Expression Signature for Assessing Growth Factor Signaling Pathway Regulation Status,* 19 March 2009
- **HENNESSY et al.** *Nat. Rev. Drug Discov.,* 2005, vol. 4, 988-1004
- **FELDMAN et al.** *J. Biol. Chem.,* 2005, vol. 280, 19867-19874
- **SATO et al.** *Oncogene,* 2002, vol. 21, 1727-1738
- **WYMANN et al.** *Mol. Cell. Biol.,* 1996, vol. 16, 1722-1733
- **VLAHOS et al.** *J. Biol. Chem.,* 1994, vol. 269, 5241-5248
- **WETZKER ; ROMMEL.** *Curr. Pharm. Des.,* 2004, vol. 10, 1915-1922
- **FINAN ; THOMAS.** *Biochem. Soc. Trans.,* 2004, vol. 32, 378-382
- **SHIN et al.** *Cancer Res.,* 2005, vol. 65, 2815-2824
- **CHENG et al.** *Oncogene,* 2005, vol. 24, 7482-7492
- **ANDREY LOBODA et al.** *Gene Expression Signature for Assessing p53 Pathway Functional Status,* 19 March 2009
- Life Testing. S-PLUS 2000 GUIDE TO STATISTICS. 2000, vol. 2, 368
- **SAMBROOK et al.** MOLECULAR CLONING--A LABORATORY MANUAL. Cold Spring Harbor Laboratory, 1989, vol. 1-3
- **AUSUBEL et al.** CURRENT PROTOCOLS IN MOLECULAR BIOLOGY. Current Protocols Publishing, 1994, vol. 2
- **CHIRGWIN et al.** *Biochemistry,* 1979, vol. 18, 5294-5299
- **BARRETT et al.** *Bioorg. Med. Chem. Lett.,* 2008, vol. 18, 6501-4
- **DUDA et al.** Pattern Classification. John Wiley & Sons, Inc, 2001
- **HASTIE et al.** The Elements of Statistical Learning. Springer-Verlag, 2001
- **ABDI.** A neural network primer. *J. Biol System.,* 1994, vol. 2, 247-283
- **CRISTIANINI ; SHAWE-TAYLOR.** An Introduction to Support Vector Machines. Cambridge University Press, 2000
- **BOSER et al.** A training algorithm for optimal margin classifiers, in Proceedings of the 5th Annual ACM Workshop on Computational Learning Theory. ACM Press, 1992, 142-152
- **VAPNIK.** Statistical Learning Theory. Wiley, 1998
- **DUDA.** Pattern Classification. John Wiley & Sons, Inc, 2001
- **HASTIE.** The Elements of Statistical Learning. Springer, 2001
- **FUREY et al.** *Bioinformatics,* 2000, vol. 16, 906-914
- **JAAKKOLA et al.** Proceedings of the 7th International Conference on Intelligent Systems for Molecular Biology. AAAI Press, 1999
- **BROWN et al.** *Proc. Natl. Acad. Sci.,* 2000, vol. 97 (1), 262-67
- **ZIEN et al.** *Bioinformatics,* 2000, vol. 16 (9), 799-807
- **FUREY et al.** *Bioinformatics,* 2000, vol. 16 (10), 906-914
- **AGRESTI.** An Introduction to Categorical Data Analysis. John Wiley & Sons, Inc, 1996
- **DUDA.** Pattern Classification. John Wiley & Sons, Inc, 2001
- **VENABLES ; RIPLEY.** Modern Applied Statistics with s-plus. Springer, 1997
- **DUDA ; HART.** Pattern Classification and Scene Analysis. John Wiley & Sons, Inc, 211-256
- **DUDA et al.** Pattern Classification. John Wiley & Sons, Inc,
- **KAUFMAN ; ROUSSEEUW.** Finding Groups in Data: An Introduction to Cluster Analysis. Wiley, 1990
- **EVERITT.** Cluster analysis. Wiley, 1993
- **BACKER.** Computer-Assisted Reasoning in Cluster Analysis. Prentice Hall, Upper Saddle River, 1995
- **JOLLIFFE.** Principal Component Analysis. Springer, 1986
- **KUBINYI.** 3D QSAR in drug design theory methods and applications. Pergamon Press, 1990, 589-638
- **ANDERSON ; 1973.** Cluster Analysis for applications. Academic Press, 1973
- **GORDON.** Classification. Chapman and Hall, CRC, 1999
- **BREIMAN.** *Machine Learning,* 1996, vol. 24, 123-140
- **EFRON ; TIBSHIRANI.** An Introduction to Bootstrap. Chapman & Hall, 1993
- **FREUND ; SCHAPIRE.** experiments with a new boosting algorithm. *Proceedings 13th International Conference on Machine Learning,* 1996, 148-156
- **FREUND ; SCHAPIRE.** *Journal of Computer and System Sciences,* 1997, vol. 55, 119-139

- **HASTI et al.** The Elements of Statistical Learning. Springer, 2001
- **PARK et al.** *Pac. Symp. Biocomput.,* 2002, vol. 6, 52-63
- **FRIEDMAN et al.** *Ann Stat,* 2000, vol. 28, 337-407
- **BEN-DOR et al.** *Journal of Computational Biology,* 2000, vol. 7, 559-583
- **HO.** The Random subspace method for constructing decision forests. *IEEE Trans Pattern Analysis and Machine Intelligence,* 1998, vol. 20 (8), 832-844
- **HAMES et al.** GEL ELECTROPHORESIS OF PROTEINS: A PRACTICAL APPROACH. IRL Press, 1990
- **SHEVCHENKO et al.** *Proc. Nat'l Acad. Sci. USA,* 1996, vol. 93, 1440-1445
- **SAGLIOCCO et al.** *Yeast,* 1996, vol. 12, 1519-1533
- **LANDER.** *Science,* 1996, vol. 274, 536-539
- **HARLOW ; LANE.** ANTIBODIES: A LABORATORY MANUAL. Cold Spring Harbor, 1988
- **KONONEN et al.** *Nat. Med,* 1998, vol. 4 (7), 844-7
- PCR PROTOCOLS: A GUIDE TO METHODS AND APPLICATIONS. Academic Press Inc, 1990
- **FROEHLER et al.** *Nucleic Acid Res.,* 1986, vol. 14, 5399-5407
- **MCBRIDE et al.** *Tetrahedron Lett.,* 1983, vol. 24, 246-248
- **EGHOLM et al.** *Nature,* 1993, vol. 363, 566-568
- **HUGHES et al.** *Nat. Biotech,* 2001, vol. 19, 342-7
- **SCHENA et al.** *Science,* 1995, vol. 270, 467-470
- **DERISI et al.** *Nature Genetics,* 1996, vol. 14, 457-460
- **SHALON et al.** *Genome Res.,* 1996, vol. 6, 639-645
- **SCHENA et al.** *Proc. Natl. Acad. Sci. U.S.A.,* 1995, vol. 93, 10539-11286
- **FODOR et al.** *Science,* 1991, vol. 251, 767-773
- **PEASE et al.** *Proc. Natl. Acad. Sci. U.S.A.,* 1994, vol. 91, 5022-5026
- **LOCKHART et al.** *Nature Biotechnology,* 1996, vol. 14, 1675
- **BLANCHARD et al.** *Biosensors & Bioelectronics,* vol. 11, 687-690
- **MASKOS ; SOUTHERN.** *Nuc. Acids. Res.,* 1992, vol. 20, 1679-1684
- **BLANCHARD et al.** *Biosensors and,* 1996, vol. 11, 687-690
- **BLANCHARD.** SYNTHETIC DNA ARRAYS IN GENETIC ENGINEERING. Plenum Press, 1998, vol. 20, 111-123
- CURRENT PROTOCOLS IN MOLECULAR BIOLOGY. Green Publishing Associates, Inc., John Wiley & Sons, Inc, 1989, vol. III, 13.12.1-13.12.5
- **BONALDO et al.** *Genome Res.,* 1996, vol. 6, 791-806
- **SCHENA et al.** *Proc. Natl. Acad. Sci. U.S.A.,* 1993, vol. 93, 10614
- **TIJESSEN.** HYBRIDIZATION WITH NUCLEIC ACID PROBES. Elsevier Science Publishers B. V, 1993
- **KRICKA.** NONISOTOPIC DNA PROBE TECHNIQUES. Academic Press, 1992
- **SHALON et al.** A DNA microarray system for analyzing complex DNA samples using two-color fluorescent probe hybridization. *Genome Research,* 1996, vol. 6, 639-645
- **SCHENA et al.** *Genome Res.,* 1996, vol. 6, 639-645
- **FERGUSON et al.** *Nature Biotech.,* 1996, vol. 14, 1681-1684
- **BILD et al.** *Nature,* 2006, vol. 439, 353-357
- **SWEET-CORDERO et al.** *Nat. Genet.,* 2005, vol. 37, 48-55
- **BLUM et al.** *Cancer Res.,* 2007, vol. 67, 3320-3328
- **SWEET-CARDER et al.** *Nat. Genet.,* 2005, vol. 37, 48-55
- **HUGHES et al.** *Nat. Biotechnol.,* 2001, vol. 19, 342-347
- **MARTON et al.** *Nat. Med.,* 1998, vol. 4, 1293-1301
- **OHREN et al.** *Nat. Struct. Mol. Biol.,* 2004, vol. 11, 119201197